# EUROPEAN PATENT SPECIFICATION

(11) **EP 4 103 559 B1**
(45) Date of publication and mention of the grant of the patent: **04.03.2026**
(21) Application number: 21709851.6
(22) Date of filing: 11.02.2021
(51) Int. Cl.: C07D 401/14, A61K 31/41, C07D 403/14, C07D 413/14, A61P 35/00, A61P 19/00, C07D 407/14, C07D 409/14

(54) **HETEROCYCLIC PAD4 INHIBITORS**
HETEROZYKLISCHE PAD4-INHIBITOREN
INHIBITEURS DE PAD4 HÉTÉROCYCLIQUES

(30) Priority: 12.02.2020 IN 202041006146
(43) Date of publication of application: 21.12.2022
(73) Proprietor: Bristol-Myers Squibb Company, Princeton, NJ 08543 (US)
(72) Inventor: SELVAKUMAR, Kumaravel, Bangalore Karnataka 560 099 (IN); PAIDI, Venkatram Reddy, Bangalore Karnataka 560 099 (IN); THANGATHIRUPATHY, Srinivasan, Bangalore Karnataka 560 099 (IN); KUMAR CM, Vijaya, Bangalore Karnataka 560 099 (IN); ALAJANGI, Tirupathi, Rao, Bangalore Karnataka 560 099 (IN); SURA, Mallikarjun, Reddy, Bangalore Karnataka 560 099 (IN); MAHADEVU, Krishna, Bangalore Karnataka 560 099 (IN); SISTLA, Ramesh Kumar, Bangalore Karnataka 560 099 (IN); AGARWAL, Piyush, Bangalore Karnataka 560 099 (IN); SUBBIAH KARUPPIAH, Arul Mozhi, Bangalore Karnataka 560 099 (IN); NAIR, Jalathi S., Bangalore Karnataka 560 099 (IN); MORAMPUDI, Ooha, Bangalore Karnataka 560 099 (IN); PANDA, Manoranjan, Bangalore Karnataka 560 099 (IN); TINO, Joseph A., Princeton, NJ 08543 (US); CHERNEY, Robert J., Princeton, NJ 08543 (US); DUNCIA, John V., Princeton, NJ 08543 (US); GARDNER, Daniel S., Princeton, NJ 08543 (US); DHAR, T. G. Murali, Princeton, NJ 08543 (US); ROSS, Audrey Graham, Cambridge, Massachusetts 02140 (US); GORMISKY, Paul E., Belmont, Massachusetts 02478 (US); ZHU, Xiao, Winchester, MA 01890 (US); SELETSKY, Boris M., Cambridge, MA 02140 (US); ANTROPOW, Alyssa H., Princeton, New Jersey 08543 (US); NIU, Deqiang, Lexington, MA 02420 (US); ZHU, Zhengdong, Westborough, MA 01581 (US); MIAO, Guobin, Cambridge, MA 02140 (US); CUERVO, Julio Hernán, Arlington, MA 02474 (US)
(74) Representative: Dehns
(86) International application number: PCT/US2021/017554
(87) International publication number: WO 2021/163254

(56) References cited:
- WO-A1-2014/015905
- WO-A1-2016/185279
- WO-A1-2017/100601
- WO-A1-2017/108282
- WO-A1-2019/077631
- US-A1- 2019 276 440
- GUO ZHENGWEI ET AL: "Synthesis of reversible PAD4 inhibitors via copper-catalyzed C-H arylation of benzimidazole", SCIENCE CHINA CHEMISTRY; THE FRONTIERS OF CHEMICAL BIOLOGY AND SYNTHESIS, SCIENCE CHINA PRESS, SIENCE CHINA PRESS, vol. 62, no. 5, 22 February 2019 (2019-02-22), pages 592 - 596, XP036777146, ISSN: 1674-7291, [retrieved on 20190222], DOI: 10.1007/S11426-018-9409-3
- LANGE SIGRUN ET AL: "Peptidylarginine Deiminases as Mediators of Microvesicular Release - Novel Therapeutic Interventions", 16 March 2017 (2017-03-16), XP055821033, Retrieved from the Internet <URL:doi:10.20944/preprints201703.0125.v1> [retrieved on 20210705], DOI: 10.20944/preprints201703.0125.v1

## Description

### FIELD OF THE INVENTION

The invention generally relates to substituted heterocyclic compounds, pharmaceutical compositions comprising these compounds, and these compounds for use in a method of the treatment of a disease or a disorder associated with PAD4 enzyme activity.

### BACKGROUND OF THE INVENTION

PAD4 is a member of the peptidylarginine deiminase (PAD) family of enzymes capable of catalysing the citrullination of arginine into citrulline within peptide sequences. PAD4 is responsible for the deimination or citrullination of a variety of proteins *in vitro* and *in vivo,* with consequences of diverse functional responses in a variety of diseases (Jones J.E. et al, Curr. Opin. Drug Discov. Devel., 12(5), (2009), 616-627). Examples of exemplar diseases or disorders include rheumatoid arthritis, diseases with neutrophilic contributions to pathogenesis (for example vasculitis, systemic lupus erythematosus, ulcerative colitis) in addition to oncology indications. PAD4 inhibitors also have wider applicability as tools and therapeutics for human diseases and disorders through epigenetic mechanisms.

Inhibitors of PAD4 have utility against Rheumatoid Arthritis (RA). RA is an autoimmune disease affecting approximately 1% of the population *(*Wegner N. et al, Immunol. Rev., 233(1), (2010), 34-54*).* It is characterized by inflammation of articular joints leading to debilitating destruction of bone and cartilage. A weak genetic association between PAD4 polymorphisms and susceptibility to RA has been suggested, albeit inconsistently, in a number of population studies (Kochi Y. et al, Ann. Rheum. Dis., 70, (2011), 512-515*).* PAD4 (along with family member PAD2) has been detected in synovial tissue where it is responsible for the deimination of a variety of joint proteins. This process is presumed to lead to a break of tolerance to, and initiation of immune responses to, citrullinated substrates such as fibrinogen, vimentin and collagen in RA joints. These anti-citrullinated protein antibodies (ACPA) contribute to disease pathogenesis and may also be used as a diagnostic test for RA (e.g. the commercially available CCP2 or cyclic citrullinated protein 2 test). In addition, increased citrullination may also offer additional direct contributions to disease pathogenesis through its ability to affect directly the function of several joint and inflammatory mediators (e.g. fibrinogen, anti-thrombin, and multiple chemokines). In a smaller subset of RA patients, anti-PAD4 antibodies can be measured and may correlate with a more erosive form of the disease.

PAD4 inhibitors are also useful for the reduction of pathological neutrophil activity in a variety of diseases. Studies suggest that the process of Neutrophil Extracellular Trap (NET) formation, an innate defense mechanism by which neutrophils are able to immobilize and kill pathogens, is associated with histone citrullination and is deficient in a PAD4 knockout mice (Neeli I. et al, J. Immunol., 180, (2008), 1895-1902*, and* Li P. et al, J. Exp. Med., 207(9), (2010), 1853-1862*).* PAD4 inhibitors may therefore have applicability for diseases where NET formation in tissues contributes to local injury and disease pathology. Such diseases include, but are not limited to, small vessel vasculitis (Kessenbrock K. et al, Nat. Med., 15(6), (2009), 623-625*),* systemic lupus erythematosus (Hakkim A. et al, Proc. Natl. Acad. Sci. USA, 107(21), (2010), 9813-9818*,* and Villanueva E. et al, J. Immunol., 187(1), (2011), 538-52*),* ulcerative colitis *(*Savchenko A. et al, Pathol. Int., 61(5), (2011), 290-7*),* cystic fibrosis, asthma *(*Dworski R. et al, J. Allergy Clin. Immunol., 127(5), (2011), 1260-6*),* deep vein thrombosis *(*Fuchs T. et al, Proc. Natl. Acad. Sci. USA, 107(36), (2010), 15880-5*),* periodontitis *(*Vitkov L. et al, Ultrastructural Pathol., 34(1), (2010), 25-30*),* sepsis *(*Clark S.R. et al, Nat. Med., 13(4), (2007), 463-9*),* appendicitis *(*Brinkmann V. et al, Science, 303, (2004), 1532-5*),* and stroke. In addition, there is evidence that NETs may contribute to pathology in diseases affecting the skin, e.g., in cutaneous lupus erythematosis *(*Villanueva E. et al, J. Immunol., 187(1), (2011), 538-52*)* and psoriasis *(*Lin A.M. et al., J. Immunol., 187(1), (2011), 490-500*),* so a PAD4 inhibitor may show benefit to tackle NET skin diseases, when administered by a systemic or cutaneous route. PAD4 inhibitors may affect additional functions within neutrophils and have wider applicability to neutrophilic diseases.

Studies have demonstrated efficacy of tool PAD inhibitors (for example, chloro-amidine) in a number of animal models of disease, including collagen-induced arthritis *(*Willis V C. et al, J. Immunol., 186(7), (2011), 4396-4404*),* dextran sulfate sodium (DSS)-induced experimental colitis (Chumanevich A.A. et al, Am. J. Physiol. Gastrointest. Liver Physiol., 300(6), (2011*), G929-G938*), spinal cord repair *(*Lange S. et al, Dev. Biol., 355(2), (2011), 205-14*),* and experimental autoimmune encephalomyelitis (EAE). The DSS colitis report also demonstrates that chloro-amidine drives apoptosis of inflammatory cells both *in vitro* and *in vivo,* suggesting that PAD4 inhibitors may be effective more generally in widespread inflammatory diseases.

PAD4 inhibitors are also useful in the treatment of cancers (Slack J.L. et al, Cell. Mol. Life Sci., 68(4), (2011), 709-720*).* Over-expression of PAD4 has been demonstrated in numerous cancers (Chang X. et al, BMC Cancer, 9, (2009*), 40).* An anti-proliferative role has been suggested for PAD4 inhibitors from the observation that PAD4 citrullinates arginine residues in histones at the promoters of p53-target genes such as p21, which are involved in cell cycle arrest and induction of apoptosis (Li P. et al, Mol. Cell Biol., 28(15), (2008), 4745-4758*).*

The aforementioned role of PAD4 in deiminating arginine residues in histones may be indicative of a role for PAD4 in epigenetic regulation of gene expression. PAD4 is the primary PAD family member observed to be resident in the nucleus as well as the cytoplasm. Early evidence that PAD4 may act as a histone demethyliminase as well as a deiminase is inconsistent and unproven. However, it may reduce histone arginine methylation (and hence epigenetic regulation associated with this mark) indirectly *via* depletion of available arginine residues by conversion to citrulline. PAD4 inhibitors are useful as epigenetic tools or therapeutics for affecting expression of varied target genes in additional disease settings. Through such mechanisms, PAD4 inhibitors may also be effective in controlling citrullination levels in stem cells and may therefore therapeutically affect the pluripotency status and differentiation potential of diverse stem cells including, but not limited to, embryonic stem cells, neural stem cells, haematopoietic stem cells and cancer stem cells. Accordingly, there remains an unmet need to identify and develop PAD4 inhibitors for the treatment of PAD4-mediated diseases or disorders.

WO 2014/015905 discloses 2-(azaindole-2-yl)benzimidazoles as PAD4 inhibitors useful in the treatment of various disorders, for example rheumatoid arthritis, vasculitis, systemic lupus erythematosus, ulcerative colitis, cancer, cystic fibrosis, asthma, cutaneous lupus erythematosis, and psoriasis.

### SUMMARY OF THE INVENTION

Accordingly, there are provided compounds of Formula (Ia): 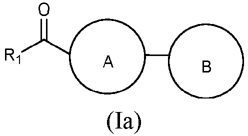 or a stereoisomer, an enantiomer, a diastereomer, a tautomer, or a pharmaceutically acceptable salt thereof.

In another general aspect, there are provided pharmaceutical compositions comprising at least one compound of Formula (Ia), or a stereoisomer, an enantiomer, a diastereomer, a tautomer, or a pharmaceutically acceptable salt thereof, and one or more pharmaceutically acceptable carriers.

In another general aspect, there is provided a compound for use in a method of treating a disease or a disorder associated with PAD4 enzyme activity, the method comprising administering to a subject in need of such treatment, a therapeutically effective amount of at least one compound of Formula (Ia), or a stereoisomer, an enantiomer, a diastereomer, a tautomer, or a pharmaceutically acceptable salt thereof.

The details of one or more embodiments of the invention are set forth in the description below. Other features, objects and advantages of the invention will be apparent from the following description including claims.

### DETAILED DESCRIPTION OF THE INVENTION

### 1. Definitions

The following are definitions of terms used in this specification. The initial definition provided for a group or term herein applies to that group or term throughout the specification, individually or as part of another group, unless otherwise indicated. Although the invention is described with reference to exemplary embodiments, it should nevertheless be understood that no limitation of the scope of the invention is thereby intended. Numerous alterations and further modifications of the inventive features of the invention can be envisaged by one skilled in the relevant art, and having possession of this disclosure. All such alterations and modifications are to be considered within the scope of the invention as defined in the appended claims.

The term "C₁₋₆ alkyl" as used herein refers to and includes straight and branched chain hydrocarbon groups containing 1 to 6 carbon atoms. Typical, non-limiting examples of C₁₋₆ alkyl include methyl, ethyl, propyl, isopropyl, butyl, t-butyl, isobutyl, pentyl, hexyl, and isohexyl.

The term "alkenyl" as used herein refers to and includes straight or branched chain hydrocarbon groups containing 2 to 20 carbons, which include 1 to 6 double bonds. Typical, non-limiting examples of alkenyl include vinyl, 2-propenyl, 3-butenyl, 2-butenyl, 4-pentenyl, 3-pentenyl, 2-hexenyl, 3-hexenyl, 2-heptenyl, 3-heptenyl, 4-heptenyl, 3-octenyl, 3-nonenyl, 4-decenyl, 3-undecenyl, and 4-dodecenyl.

The term "alkynyl" as used herein refers to and includes straight or branched chain hydrocarbon groups containing 2 to 12 carbon atoms, and at least one carbon to carbon triple bond. Typical, non-limiting examples of alkynyl include ethynyl, 2-propynyl, 3-butynyl, 2-butynyl, 4-pentynyl, 3-pentynyl, 2-hexynyl, 3-hexynyl, 2-heptynyl, 3-heptynyl, 4-heptynyl, 3-octynyl, 3-nonynyl, and 4-decynyl.

The term "cycloalkyl" as used herein refers to and includes a saturated or partially unsaturated (containing 1 or 2 double bonds) cyclic hydrocarbon group containing 1 to 3 rings (for example, monocyclic, bicyclic, or tricyclic), and containing a total of 3 to 20 carbon atoms forming the rings. The cycloalkyl may be optionally substituted. The rings of multi-ring cycloalkyls may exist as fused, bridged and/or joined through one or more spiro union to 1 or 2 aromatic cycloalkyl or heterocyclic rings. Typical, non-limiting examples of cycloalkyl groups include cyclopropyl, cyclobutyl, cyclopentyl, cyclohexyl, cycloheptyl, cyclooctyl, cyclodecyl, cyclododecyl, cyclopentenyl, cyclohexenyl, cycloheptenyl, cyclooctenyl, cyclohexadienyl, and cycloheptadienyl.

The terms "heterocyclic", "heterocyclyl" or "heterocyclo" as used herein refers to and includes optionally substituted, saturated, unsaturated or partially unsaturated 3 to 20 member cyclic groups (for example, 3 to 13 member monocyclic, 7 to 17 member bicyclic, or 10 to 20 member tricyclic ring systems), which have at least one heteroatom in at least one carbon atom-containing ring. The heteroatom is selected from nitrogen, oxygen and/or sulfur atoms. Each ring of the heterocyclic group may have 1, 2, 3, 4 or 5 heteroatoms, subject to a condition that that at least one ring contains at least one heteroatom. The nitrogen and sulfur heteroatoms may optionally be oxidized and the nitrogen heteroatoms may optionally be quaternized. The rings of multi-ring heterocycles may be fused, bridged and/or joined through one or more spiro unions. Typical, non-limiting examples of heterocyclic groups include azetidinyl, pyrrolidinyl, oxetanyl, imidazolinyl, oxazolidinyl, isoxazolinyl, thiazolidinyl, isothiazolidinyl, tetrahydrofuranyl, piperidinyl, piperazinyl, 2-oxopiperazinyl, 2-oxopiperidinyl, 2-oxopyrrolodinyl, 2-oxoazepinyl, azepinyl, 4-piperidonyl, tetrahydropyranyl, morpholinyl, thiamorpholinyl, thiamorpholinyl sulfoxide, thiamorpholinyl sulfone, 1,3-dioxolane and tetrahydro-1,1-dioxothienyl.

The term "aryl" as used herein refers to and includes aromatic homocyclic (i.e., hydrocarbon) monocyclic, bicyclic or tricyclic aromatic groups containing 6 to 14 carbons in the ring portion, and may optionally include one to three additional rings (either cycloalkyl, heterocyclo or heteroaryl) fused thereto. Typical, non-limiting examples of aryl groups includes phenyl, biphenyl, naphthyl (including 1-naphthyl, and 2-naphthyl), and anthracenyl.

The above-defined groups may optionally have one or more hydrogen atoms that are attached to a carbon atom substituted with any group known to one of skill in the art. Throughout the specification, groups and substituents thereof may be chosen to provide stable moieties and compounds.

The term "subject" as used herein refers to and includes any human or non-human organism that could potentially benefit from treatment with a PAD4 inhibitor. Exemplary subjects include humans and animals.

The terms "treating" or "treatment" as used herein refer to and include treatment of a disease-state in a subject, for example in a human or animal, and include: (a) inhibiting the disease-state, i.e., arresting it's development; (b) relieving the disease-state, i.e., causing regression of the disease state; and/or (c) preventing the disease-state from occurring in a subject.

The terms "preventing" or "prevention" as used herein refer to and include preventive treatment (i.e. prophylaxis and/or risk reduction) of a subclinical disease-state in a subject, for example in a human or animal, aimed at reducing the probability of the occurrence of a clinical disease-state. Subjects may be selected for preventative therapy based on factors that are known to increase risk of suffering a clinical disease state compared to the general population. "Prophylaxis" therapies can be divided into (a) primary prevention, and (b) secondary prevention. Primary prevention is defined as treatment in a subject that has not yet presented with a clinical disease state, whereas secondary prevention is defined as preventing a second occurrence of the same or similar clinical disease state.

The term "therapeutically effective amount" refers to and includes an amount of a compound or a composition according to the invention that is effective when administered alone or in combination to prevent or treat the disease or disorder associated with PAD4 enzyme activity. When applied to a combination, the term refers to combined amounts of the active ingredients that result in the preventive or therapeutic effect, whether administered in combination, serially, or simultaneously.

A "pharmaceutically acceptable carrier" refers to media generally accepted in the art for the delivery of biologically active agents to humans and/or animals. Pharmaceutically acceptable carriers are formulated according to a number of factors well within the purview of those of ordinary skill in the art. These include, without limitation, the type and nature of the active agent being formulated, the subject to which the agent-containing composition is to be administered, the intended route of administration of the compound or composition, and, the therapeutic indication being targeted. Pharmaceutically acceptable carriers include both aqueous and non-aqueous liquid media. Such carriers can include a number of different ingredients and additives in addition to the active agent, such additional ingredients being included in the formulation for a variety of reasons, e.g., stabilization of the active agent, binders, etc., well known to those of ordinary skill in the art. Typical, non-limiting examples of such carriers include diluents, preserving agents, fillers, flow regulating agents, disintegrating agents, wetting agents, emulsifying agents, suspending agents, sweetening agents, flavouring agents, perfuming agents, anti-bacterial agents, anti-fungal agents, lubricating agents, dispensing agents, and coating agents. Descriptions of suitable pharmaceutically acceptable carriers, and factors involved in their selection, are found in a variety of readily available sources such as, for example, Allen, L. V., Jr. et al., Remington: The Science and Practice of Pharmacy (2 Volumes), 22nd Edition, Pharmaceutical Press (2012*).*

Any tautomer, which may exist, is also contemplated herein as a part of the present invention. All stereoisomers of the compound of Formula (Ia), such as those, which may exist due to asymmetric carbons, including enantiomeric forms (which may exist even in the absence of asymmetric carbons, e.g., atropisomers) and diastereomeric forms are contemplated within the scope of this invention. Individual stereoisomers of the compound of Formula (Ia) may, for example, be substantially free of other isomers, or may be admixed, for example, as racemates or with all other, or other selected, stereoisomers. The chiral centres in these compounds may have the S or R configuration as defined by the IUPAC 1974 Recommendations. In general, the compounds according to the invention may be described herein without specific stereochemistry for convenience, however all stereoisomeric forms are included within the scope of the invention.

The present invention is intended to include all isotopes of atoms occurring in the present compounds. Isotopes include those atoms having the same atomic number but different mass numbers. By way of general example and without limitation, isotopes of hydrogen include deuterium (symbol D or ²H) and tritium (symbol T or ³H). For example, a methyl group may be represented by CH₃ or CD₃. Isotopes of carbon include ¹³C and ¹⁴C. Isotopically-labeled compounds of the invention can generally be prepared by conventional techniques known to those skilled in the art or by processes analogous to those described herein, using an appropriate isotopically-labeled reagent in place of the non-labeled reagent otherwise employed.

The compound of Formula (Ia) forms salts which are also within the scope of this invention. Reference to a compound of the Formula (Ia) herein is understood to include reference to salts thereof, unless otherwise indicated. The term "salt(s)", as employed herein, denotes acidic and/or basic salts formed with inorganic and/or organic acids and bases. In addition, when a compound of Formula (Ia) contains both a basic moiety and an acidic moiety, zwitterions ("inner salts") may be formed and are included within the term "salt(s)" as used herein. Pharmaceutically acceptable salts include those generally acceptable in the art of pharmaceutical sciences for administration in a subject, including humans and animals. In general, the pharmaceutically acceptable salts are non-toxic and physiologically acceptable salts. Salts of the compounds according to the invention may be formed, for example, by reacting the compound with an amount of acid or base, such as an equivalent amount, in a medium such as one in which the salt precipitates or in an aqueous medium followed by lyophilization.

The compounds of Formula (Ia) which contain a basic moiety may form salts with a variety of organic and inorganic acids. Exemplary acid addition salts include acetates (such as those formed with acetic acid or trihaloacetic acid, for example, trifluoroacetic acid), adipates, alginates, ascorbates, aspartates, benzoates, benzenesulfonates, bisulfates, borates, butyrates, citrates, camphorates, camphorsulfonates, cyclopentanepropionates, digluconates, dodecylsulfates, ethanesulfonates, fumarates, glucoheptanoates, glycerophosphates, hemisulfates, heptanoates, hexanoates, hydrochlorides (formed with hydrochloric acid), hydrobromides (formed with hydrogen bromide), hydroiodides, 2-hydroxyethanesulfonates, lactates, maleates (formed with maleic acid), methanesulfonates (formed with methanesulfonic acid), 2-naphthalenesulfonates, nicotinates, nitrates, oxalates, pectinates, persulfates, 3-phenylpropionates, phosphates, picrates, pivalates, propionates, salicylates, succinates, sulfates (such as those formed with sulfuric acid), sulfonates (such as those mentioned herein), tartrates, thiocyanates, toluenesulfonates such as tosylates, and undecanoates.

The compounds of Formula (Ia) which contain an acidic moiety may form salts with a variety of organic and inorganic bases. Exemplary basic salts include ammonium salts, alkali metal salts such as sodium, lithium, and potassium salts, alkaline earth metal salts such as calcium and magnesium salts, salts with organic bases (for example, organic amines) such as benzathines, dicyclohexylamines, hydrabamines (formed with N,N-bis(dehydroabietyl)-ethylenediamine), N-methyl-D-glucamines, N-methyl-D-glucamides, t-butyl amines, and salts with amino acids such as arginine, and lysine. Basic nitrogen-containing groups may be quaternized with agents such as lower alkyl halides (e.g. methyl, ethyl, propyl, and butyl chlorides, bromides and iodides), dialkyl sulfates (e.g. dimethyl, diethyl, dibutyl, and diamyl sulfates), long chain halides (e.g. decyl, lauryl, myristyl and stearyl chlorides, bromides and iodides), aralkyl halides (e.g. benzyl and phenethyl bromides), and others.

The invention encompasses compounds of Formula (Ia), including their stereoisomers, enantiomers, diastereomers, tautomers, and pharmaceutically acceptable salts, pharmaceutical compositions comprising these compounds, and these compounds for use in a method of the treatment of diseases or disorders associated with PAD4 enzyme activity.

### 2. Description of Certain Aspects of the Invention

In one aspect, the invention provides a compound of Formula (Ia): 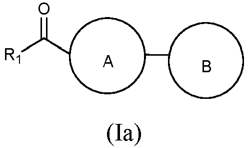 or a stereoisomer, an enantiomer, a diastereomer, a tautomer, or a pharmaceutically acceptable salt thereof, wherein: 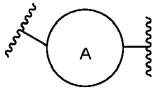 is 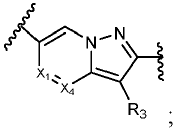 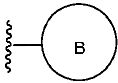 is selected from 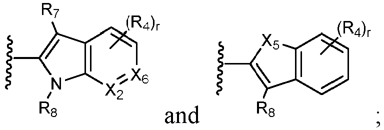
X₁ is independently selected from CR₂ and N;
X₂ is independently selected from CR₄ and N;
X₄ is independently selected from CR₂ and N; provided X₁ and X₄ are not both N;
X₅ is independently selected from O and S;
X₆ is independently selected from CR₄, and N; provided 1) X₂ and X₆ are not both N; 2) when X₂ and X₆ are both CR₄, one of R₄ is H;
R₁ is independently selected from 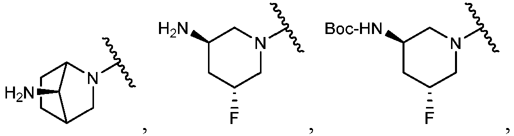 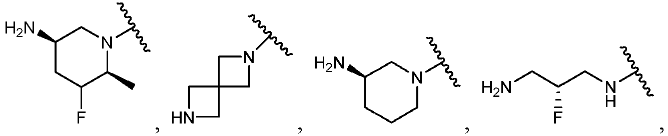 and 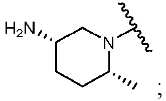
R₂ is independently selected from H, F, Cl, C₁₋₄ alkyl optionally substituted with one or more substituents selected from F, Cl, and OH, and -OC₁₋₄ alkyl;
R₃ is independently selected from H, F, Cl, CN, -C(=O)OR_{b}, and C₁₋₃ alkyl optionally substituted with one or more substituents selected from F, Cl, OH, NH₂, and N₃;
R₄ is independently selected from H, F, Cl, C₁₋₅ alkyl optionally substituted with one or more substituents selected from F, Cl, and OH, C₃₋₆ cycloalkyl, 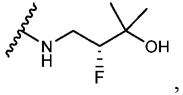 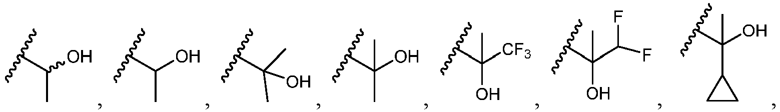 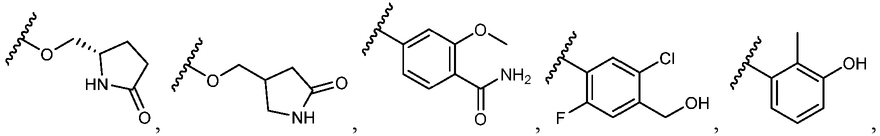 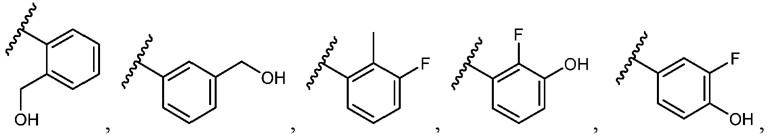 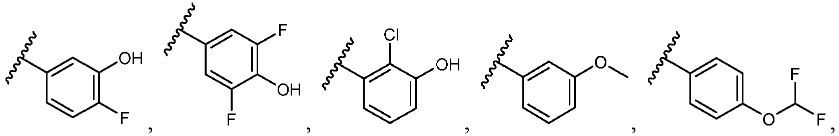 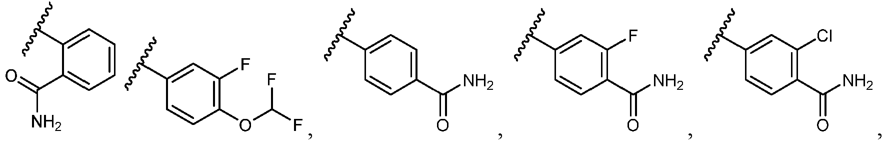 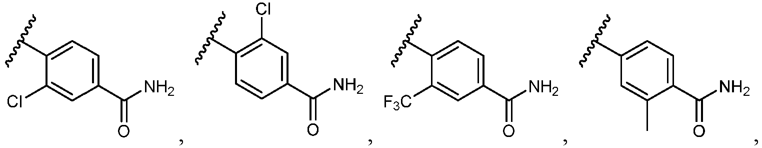 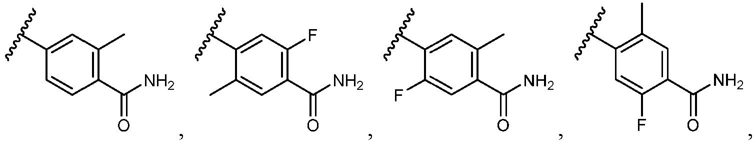 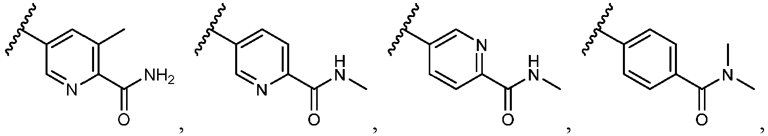 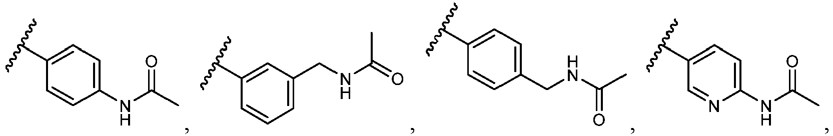 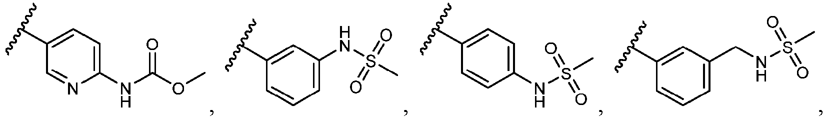 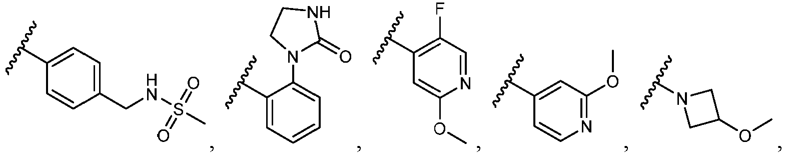 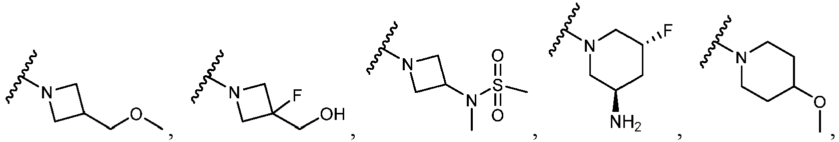 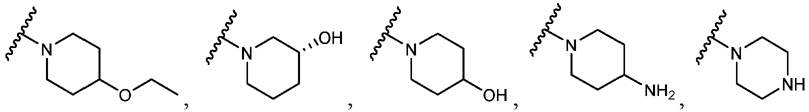 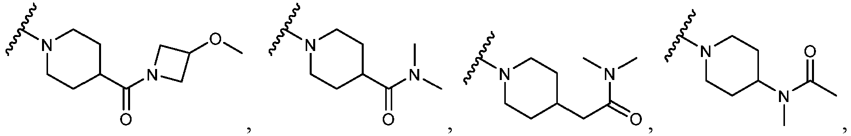 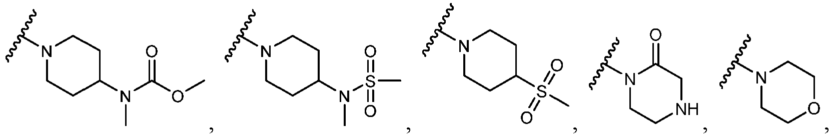 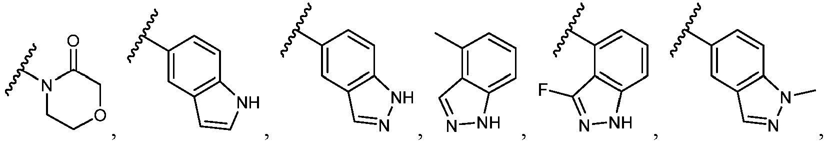 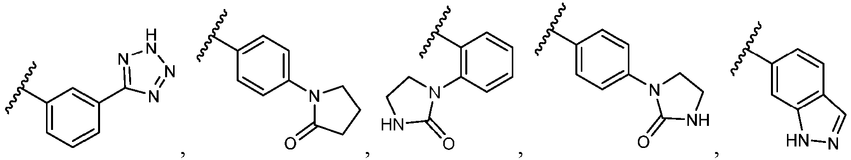 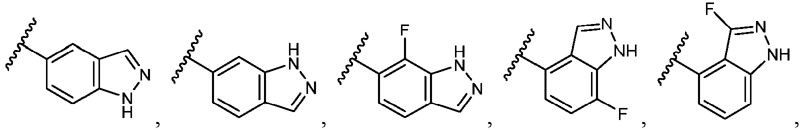 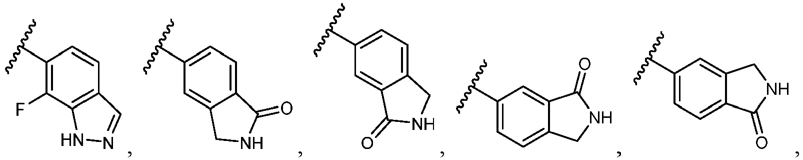 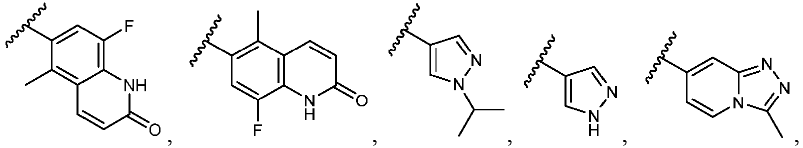 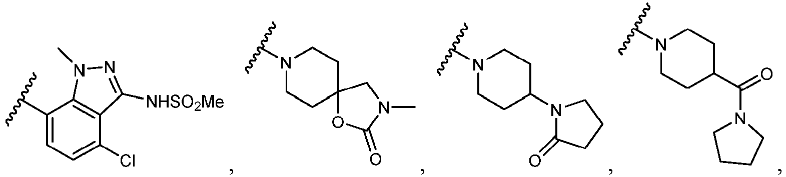 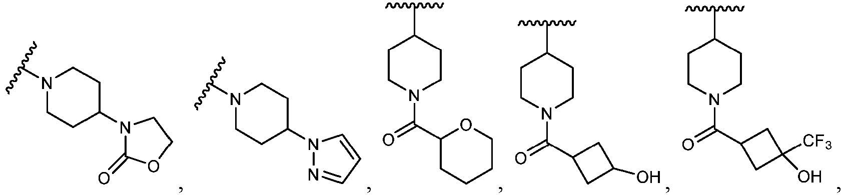 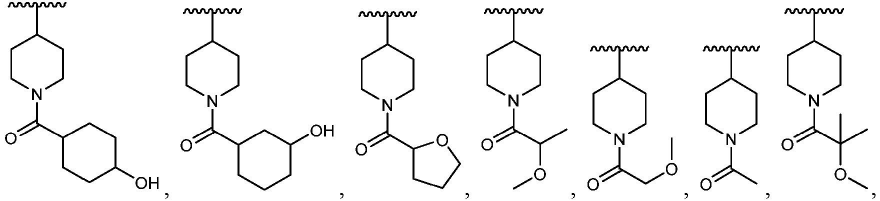 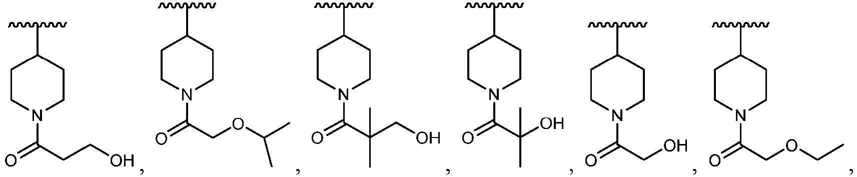 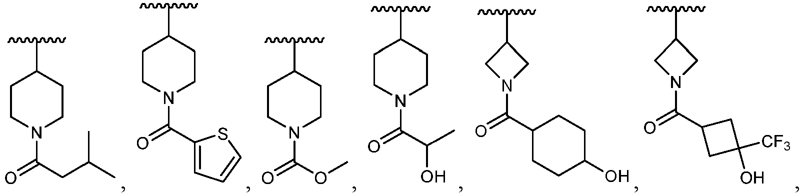 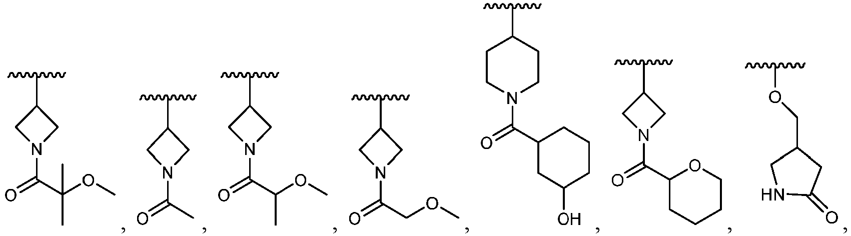 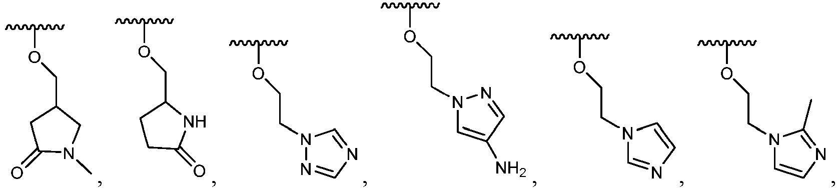 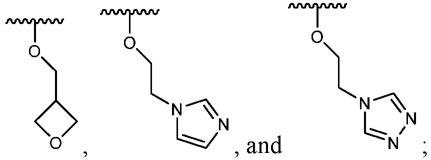
R₇ is independently selected from H, F, and Cl;
R₈ is independently selected from H, and C₁₋₆ alkyl optionally substituted with one or more substituents selected from F, Cl, and C₃₋₆ cycloalkyl;
R_{b} is independently selected from H, C₁₋₆ alkyl optionally substituted with one or more Rₑ, C₂₋₆ alkenyl optionally substituted with one or more Rₑ, C₂₋₆ alkynyl optionally substituted with one or more Rₑ, -(CH₂)ᵣ-C₃₋₁₀ carbocyclyl optionally substituted with one or more Rₑ, and -(CH₂)ᵣ-heterocyclyl optionally substituted with one or more Rₑ;
Rₑ is independently selected from F, Cl, Br, CN, NH₂, -NH-C₁₋₄ alkyl, -N(C₁₋₄ alkyl)₂, =O, OH, -OC₁₋₆ alkyl, -CO₂H, C₁₋₆ alkyl optionally substituted with one or more R_{f}, C₂₋₆ alkenyl, C₂₋₆ alkynyl, -(CH₂)ᵣ-C₃₋₆ cycloalkyl optionally substituted with one or more R_{f}, -(CH₂)ᵣ-aryl optionally substituted with one or more R_{f}, and -(CH₂)ᵣ-heterocyclyl optionally substituted with one or more R_{f};
R_{f} is independently selected from F, Cl, Br, CN, OH, OC₁₋₅ alkyl, C₁₋₅ alkyl optionally substituted with OH, C₂₋₅ alkenyl, C₂₋₅ alkynyl, C₃₋₆ cycloalkyl, and phenyl; and
r, at each occurrence, is independently selected from zero, 1, 2, 3, and 4.

In another aspect, the invention provides a compound of Formulae (IIa)-(XIII): 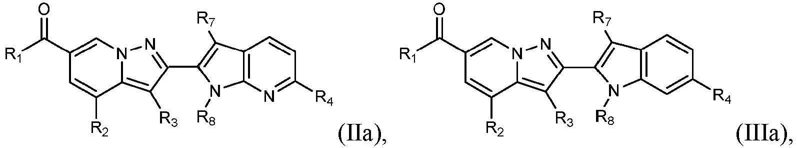 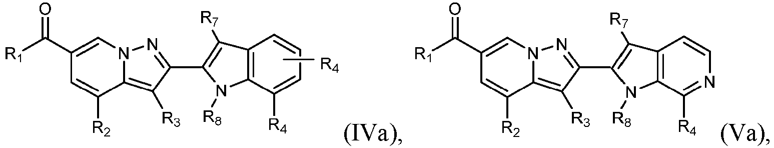 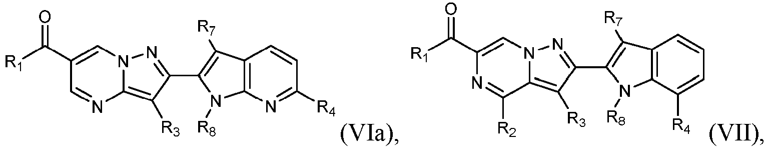 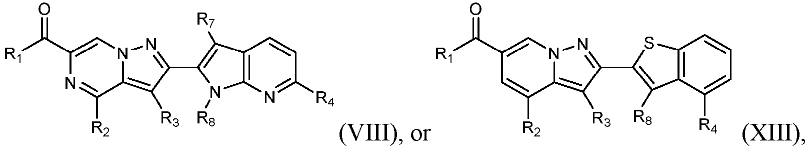 or a stereoisomer, an enantiomer, a diastereomer, a tautomer, or a pharmaceutically acceptable salt thereof. R₁, R₂, R₃, R₄, R₇, and R₈ are as defined in Formula (Ia).

In another aspect, the invention provides a compound having Formula (IIa): 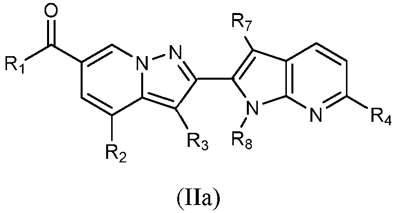 or a stereoisomer, an enantiomer, a diastereomer, a tautomer, or a pharmaceutically acceptable salt thereof, wherein:
R₁ is independently selected from 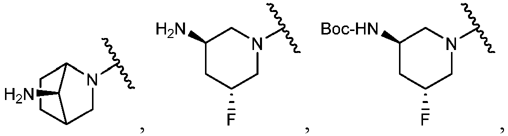 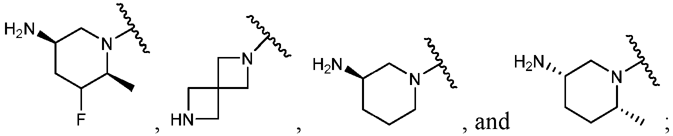
R₂ is independently selected from H, F, Cl, C₁₋₃ alkyl optionally substituted with one or more substituents selected from F, Cl, and OH, and OC₁₋₃ alkyl;
R₃ is independently selected from H, F, Cl, CH₃, and CH₂OH;
R₄ is as defined above for Formula (Ia);
R₇ is independently selected from H, F, and Cl; and
R₈ is independently selected from H, and C₁₋₆ alkyl optionally substituted with one or more substituents selected from F, Cl, and C₃₋₆ cycloalkyl.

In another aspect, the invention provides a compound having Formula (IIIa): 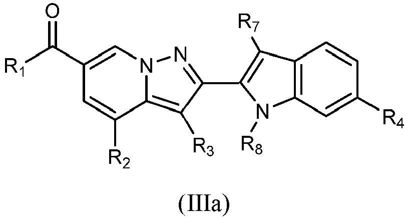 or a stereoisomer, an enantiomer, a diastereomer, a tautomer, or a pharmaceutically acceptable salt thereof, wherein:
R₁ is independently selected from 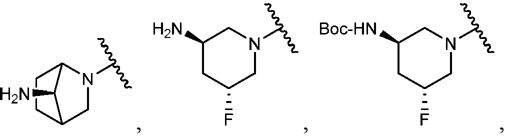 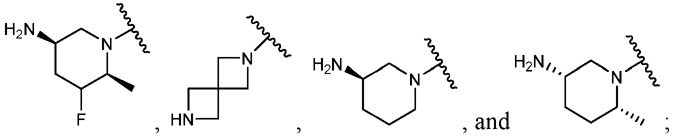
R₂ is independently selected from H, F, Cl, C₁₋₃ alkyl optionally substituted with one or more substituents selected from F, Cl, and OH, and OC₁₋₃ alkyl;
R₃ is independently selected from H, F, Cl, and C₁₋₃ alkyl optionally substituted with one or more substituents selected from F, Cl, and OH;
R₄ is as defined above for Formula (Ia);
R₇ is independently selected from H, F, and Cl; and
R₈ is independently selected from H, and C₁₋₆ alkyl optionally substituted with one or more substituents selected from F, Cl, and C₃₋₆ cycloalkyl.

In another aspect, the invention provides a compound having Formulae (IIa)-(IIIa), or a stereoisomer, an enantiomer, a diastereomer, a tautomer, or a pharmaceutically acceptable salt thereof, wherein:
R₁ is independently selected from 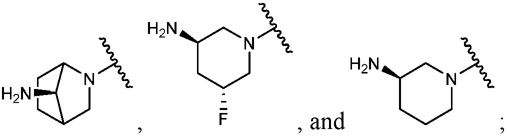
R₂ is independently selected from H, F, Cl, CH₃, and OCH₃;
R₃ is independently selected from H, F, Cl, CH₃, and CH₂OH;
R₄ is as defined above for Formula (Ia);
R₇ is H; and
R₈ is C₁₋₃ alkyl substituted with one C₃₋₆ cycloalkyl.

In another aspect, the invention provides a compound having Formula (IVa): 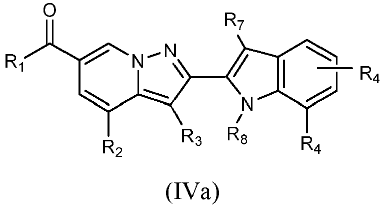 or a stereoisomer, an enantiomer, a diastereomer, a tautomer, or a pharmaceutically acceptable salt thereof, wherein:
R₁ is independently selected from 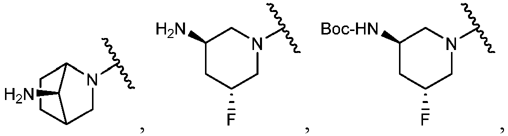 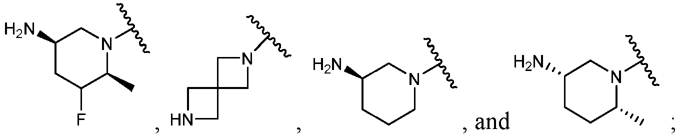
R₂ is independently selected from H, F, Cl, C₁₋₃ alkyl optionally substituted with one or more substituents selected from F, Cl, and OH, and OC₁₋₃ alkyl;
R₃ is independently selected from H, F, Cl, and C₁₋₃ alkyl optionally substituted with one
or more substituents selected from F, Cl, and OH;
R₄ is as defined above for Formula (Ia);
R₇ is independently selected from H, F, and Cl; and
R₈ is independently selected from H, and C₁₋₆ alkyl optionally substituted with one or more substituents selected from F, Cl, and C₃₋₆ cycloalkyl.

In another aspect, the invention provides a compound having Formulae (IIa)-(IIIa), or a stereoisomer, an enantiomer, a diastereomer, a tautomer, or a pharmaceutically acceptable salt thereof, wherein:
R₄ is independently selected from 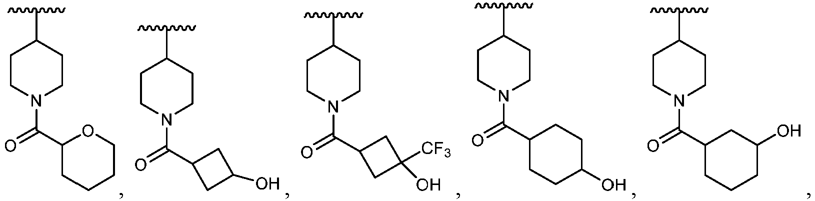 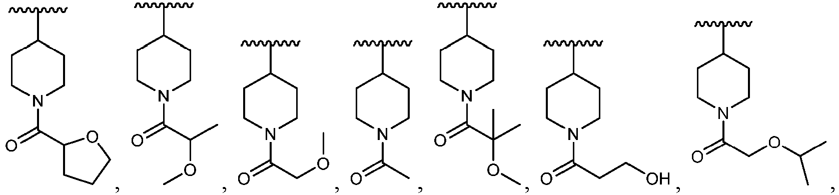 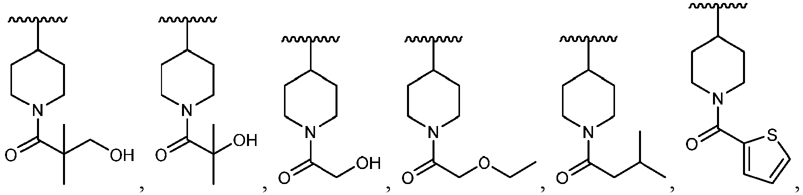 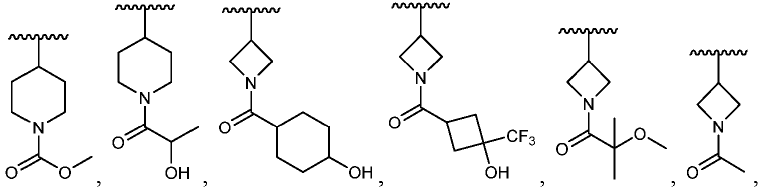 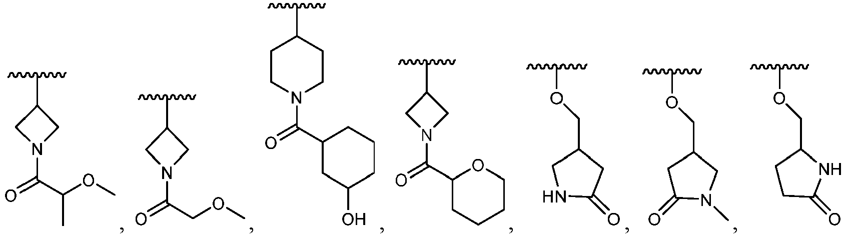 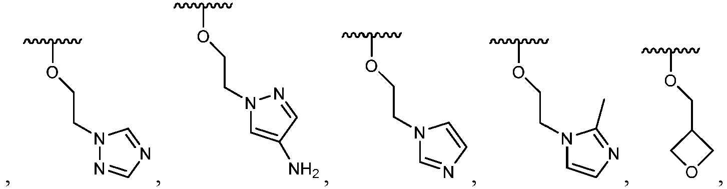 and 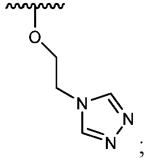 and other variables are as defined in Formulae (IIa)-(IIIa).

In another aspect, the invention provides a compound having Formula (Va): 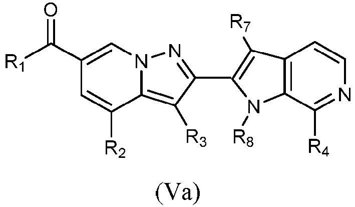 or a stereoisomer, an enantiomer, a diastereomer, a tautomer, or a pharmaceutically acceptable salt thereof, wherein:
R₁ is independently selected from 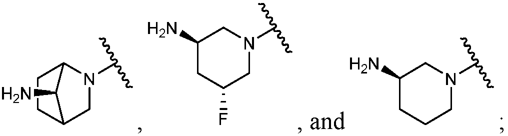
R₂ is independently selected from H, F, Cl, CH₃, and OCH₃;
R₃ is independently selected from H, F, Cl, CN, CH₃, and CH₂OH;
R₄ is as defined above for Formula (Ia);
R₇ is independently selected from H, F, and Cl; and
R₈ is independently selected from H, and C₁₋₆ alkyl optionally substituted with one or more substituents selected from F, Cl, and C₃₋₆ cycloalkyl.

In another aspect, the invention provides a compound having Formula (Va), or a stereoisomer, an enantiomer, a diastereomer, a tautomer, or a pharmaceutically acceptable salt thereof, wherein:
R₁ is independently selected from 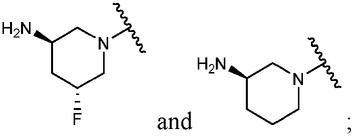
R₂ is CH₃;
R₃ is CH₃; and
R₄ is independently selected from, 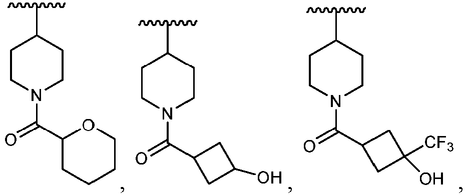 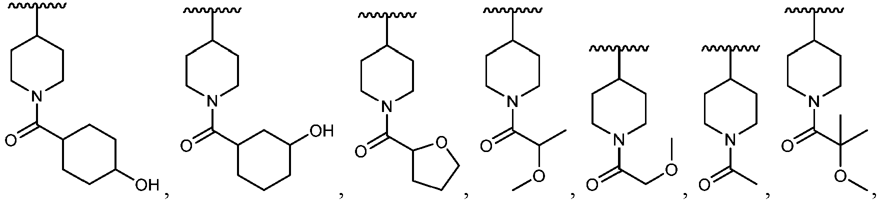 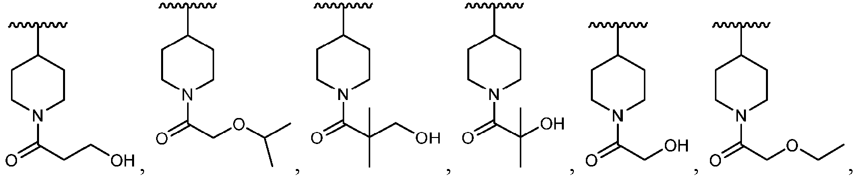 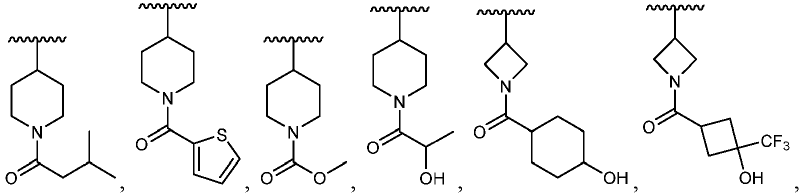 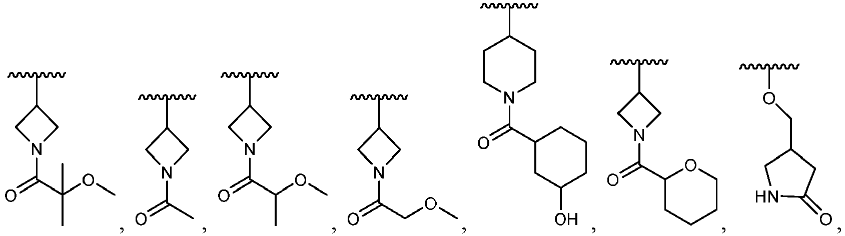 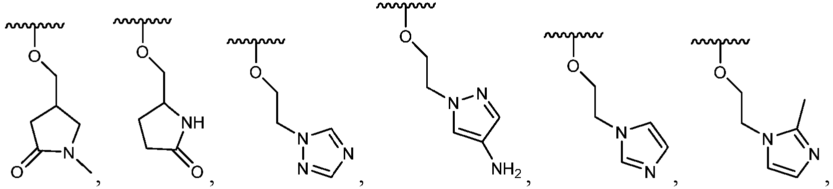 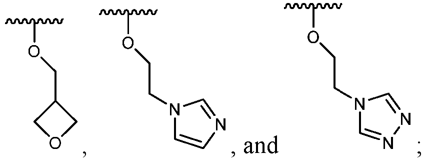
R₇ is H; and
R₈ is C₁₋₂ alkyl optionally substituted with cyclopropyl.

In another aspect, the invention provides a compound having Formula (VIa): 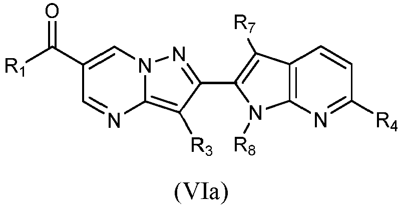 or a stereoisomer, an enantiomer, a diastereomer, a tautomer, or a pharmaceutically acceptable salt thereof, wherein:
R₁ is independently selected from 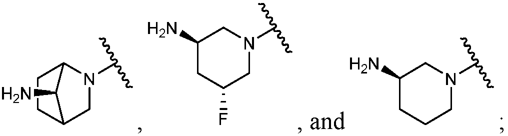
R₃ is independently selected from H, F, and CH₃;
R₄ is as defined above for Formula (Ia);
R₇ is independently selected from H, F, and Cl; and
R₈ is independently selected from H, and C₁₋₆ alkyl optionally substituted with one or more substituents selected from F, Cl, and C₃₋₆ cycloalkyl.

In another aspect, the invention provides a compound having Formula (VIa), or a stereoisomer, an enantiomer, a diastereomer, a tautomer, or a pharmaceutically acceptable salt thereof, wherein:
R₁ is independently selected from 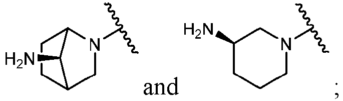
R₃ is CH₃;
R₄ is independently selected from F, Cl, 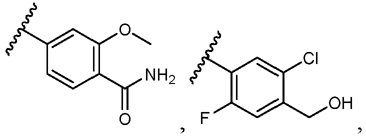 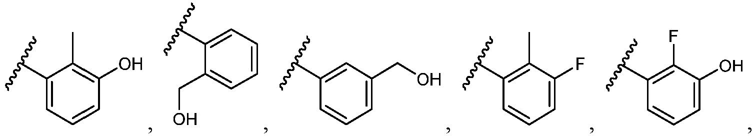 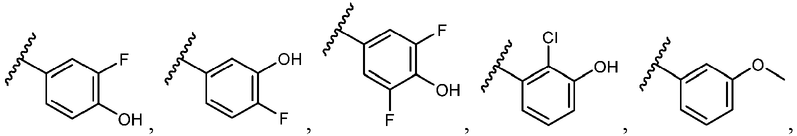 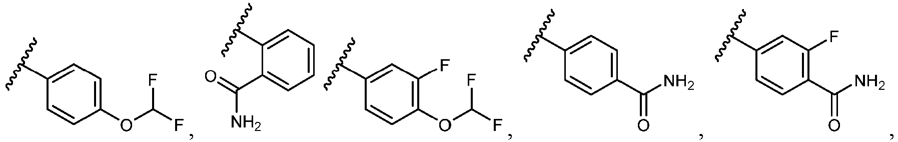 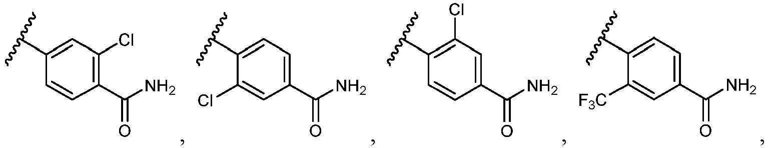 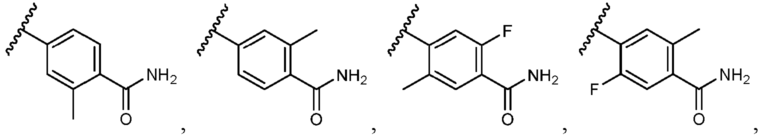 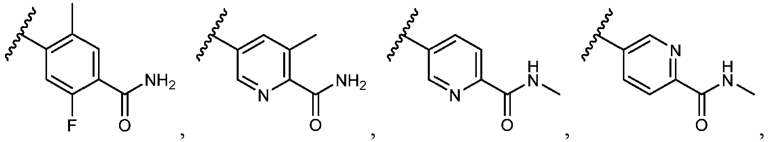 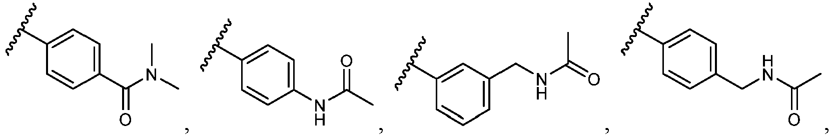 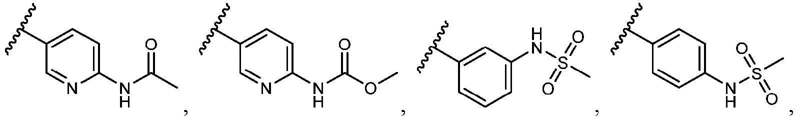 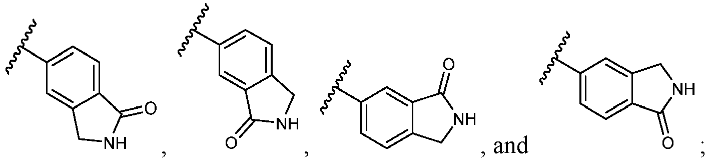
R₇ is H; and
R₈ is C₁₋₂ alkyl optionally substituted with cyclopropyl.

In another aspect, the invention provides a compound having Formula (VII): 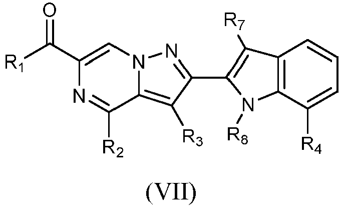 or a stereoisomer, an enantiomer, a diastereomer, a tautomer, or a pharmaceutically acceptable salt thereof, wherein:
R₁ is independently selected from 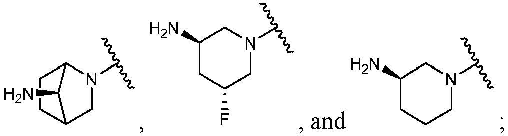
R₂ is independently selected from H, F, Cl, CH₃, and OCH₃;
R₃ is independently selected from H, F, and CH₃;R₄ is as defined above for Formula (Ia);
R₇ is independently selected from H, F, and Cl; and
R₈ is independently selected from H, and C₁₋₆ alkyl optionally substituted with one or more substituents selected from F, Cl, and C₃₋₆ cycloalkyl.

In another aspect, the invention provides a compound having Formula (VIII): 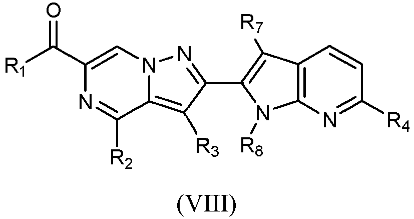 or a stereoisomer, an enantiomer, a diastereomer, a tautomer, or a pharmaceutically acceptable salt thereof, wherein:
R₁ is independently selected from 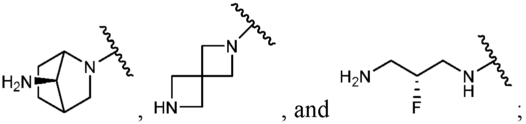
R₂ is independently selected from H, F, and OCH₃;
R₃ is independently selected from H, F, and CH₃;
R₄ is as defined above for Formula (Ia);
R₇ is independently selected from H, F, and Cl; and
R₈ is independently selected from H, and C₁₋₆ alkyl optionally substituted with one or more substituents selected from F, Cl, and C₃₋₆ cycloalkyl.

In another aspect, the invention provides a compound having Formula (XIII): 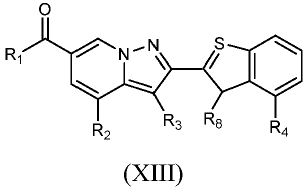 or a stereoisomer, an enantiomer, a diastereomer, a tautomer, or a pharmaceutically acceptable salt thereof, wherein:
R₁ is independently selected from 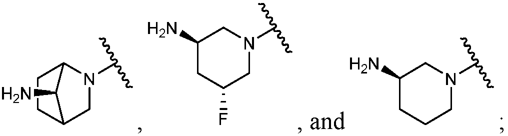
R₂ is independently selected from H, F, CH₃, and OCH₃;
R₃ is independently selected from H, F, and CH₃;R₄ is as defined above for Formula (Ia);
R₈ is independently selected from H, and C₁₋₆ alkyl optionally substituted with one or more substituents selected from F, Cl, and C₃₋₆ cycloalkyl.

Typical, non-limiting examples of the compounds according to the invention include:
((7R)-7-Amino-2-azabicyclo[2.2.1]heptan-2-yl)(2-(1-(cyclopropylmethyl)-1H-pyrrolo[2,3-b]pyridin-2-yl)-4-methoxy-3-methylpyrazolo[1,5-a]pyridin-6-yl)methanone (1);
((7R)-7-Amino-2-azabicyclo[2.2.1]heptan-2-yl)(2-(6-chloro-1-(cyclopropylmethyl)-1H-pyrrolo[2,3-b]pyridin-2-yl)-4-methoxy-3-methylpyrazolo[1,5-a]pyridin-6-yl)methanone (2);
6-(2-(6-((7R)-7-Amino-2-azabicyclo[2.2.1]heptane-2-carbonyl)-4-methoxy-3-methylpyrazolo[1,5-a]pyridin-2-yl)-1-(cyclopropylmethyl)-1H-pyrrolo[2,3-b]pyridin-6-yl)isoindolin-1-one (3);
((7R)-7-Amino-2-azabicyclo[2.2.1]heptan-2-yl)(2-(1-(cyclopropylmethyl)-6-methyl-1H-pyrrolo[2,3-b]pyridin-2-yl)-4-methoxy-3-methylpyrazolo[1,5-a]pyridin-6-yl)methanone (4);
5-(2-(6-((7R)-7-Amino-2-azabicyclo[2.2.1]- heptane-2-carbonyl)-4-methoxy-3-methylpyrazolo[1,5-a]pyridin-2-yl)-1-(cyclopropylmethyl)-1H-pyrrolo[2,3-b]pyridin-6-yl)isoindolin-1-one (5);
((7R)-7-Amino-2-azabicyclo[2.2.1]heptan-2-yl)(2-(6-cyclopropyl-1-(cyclopropylmethyl)-1H-pyrrolo[2,3-b]pyridin-2-yl)-4-methoxy-3-methylpyrazolo[1,5-a]pyridin-6-yl)methanone (6);
((7R)-7-Amino-2-azabicyclo[2.2.1]heptan-2-yl)(2-(1-(cyclopropylmethyl)-6-(1-isopropyl-1H-pyrazol-4-yl)-1H-pyrrolo[2,3-b]pyridin-2-yl)-4-methoxy-3-methylpyrazolo[1,5-a]pyridin-6-yl)methanone (7);
((7R)-7-Amino-2-azabicyclo[2.2.1]heptan-2-yl)(2-(1-(cyclopropylmethyl)-6-(3-methyl-[1,2,4]triazolo[4,3-a]pyridin-7-yl)-1H-pyrrolo[2,3-b]pyridin-2-yl)-4-methoxy-3-methylpyrazolo[1,5-a]pyridin-6-yl)methanone (8);
((7R)-7-Amino-2-azabicyclo[2.2.1]heptan-2-yl)(2-(1-(cyclopropylmethyl)-6-(2-fluoro-3-hydroxyphenyl)-1H-pyrrolo[2,3-b]pyridin-2-yl)-4-methoxy-3-methylpyrazolo[1,5-a]pyridin-6-yl)methanone (9);
((7R)-7-Amino-2-azabicyclo[2.2.1]heptan-2-yl)(2-(1-(cyclopropylmethyl)-6-(3-fluoro-4-hydroxyphenyl)-1H-pyrrolo[2,3-b]pyridin-2-yl)-4-methoxy-3-methylpyrazolo[1,5-a]pyridin-6-yl)methanone (10);
((7R)-7-Amino-2-azabicyclo[2.2.1]heptan-2-yl)(2-(1-(cyclopropylmethyl)-6-(4-(difluoromethoxy)phenyl)-1H-pyrrolo[2,3-b]pyridin-2-yl)-4-methoxy-3-methylpyrazolo[1,5-a]pyridin-6-yl)methanone (11);
((7R)-7-Amino-2-azabicyclo[2.2.1]heptan-2-yl)(2-(1-(cyclopropylmethyl)-6-(4-(difluoromethoxy)-3-fluorophenyl)-1H-pyrrolo[2,3-b]pyridin-2-yl)-4-methoxy-3-methylpyrazolo[1,5-a]pyridin-6-yl)methanone (12);
((7R)-7-Amino-2-azabicyclo[2.2.1]heptan-2-yl)(2-(1-(cyclopropylmethyl)-6-(3-methoxyazetidin-1-yl)-1H-pyrrolo[2,3-b]pyridin-2-yl)-4-methoxy-3-methylpyrazolo[1,5-a]pyridin-6-yl)methanone (13);
((3R,5R)-3-Amino-5-fluoropiperidin-1-yl)(2-(1-(cyclopropylmethyl)-1H-pyrrolo[2,3-b]pyridin-2-yl)-4-methoxy-3-methylpyrazolo[1,5-a]pyridin-6-yl)methanone (14);
((3R,5R)-3-Amino-5-fluoropiperidin-1-yl)(2-(6-chloro-1-(cyclopropylmethyl)-1H-pyrrolo[2,3-b]pyridin-2-yl)-4-methoxy-3-methylpyrazolo[1,5-a]pyridin-6-yl)methanone (15);
6-(2-(6-((3R,5R)-3-Amino-5-fluoropiperidine-1-carbonyl)-4-methoxy-3-methylpyrazolo[1,5-a]pyridin-2-yl)-1-(cyclopropylmethyl)-1H-pyrrolo[2,3-b]pyridin-6-yl)isoindolin-1-one (16);
((3R,5R)-3-Amino-5-fluoropiperidin-1-yl)(2-(1-(cyclopropylmethyl)-6-methyl-1H-pyrrolo[2,3-b]pyridin-2-yl)-4-methoxy-3-methylpyrazolo[1,5-a]pyridin-6-yl)-methanone (17);
((3R,5R)-3-Amino-5-fluoropiperidin-1-yl)(2-(1-(cyclopropylmethyl)-6-(1-isopropyl-1H-pyrazol-4-yl)-1H-indol-2-yl)-4-methoxy-3-methylpyrazolo[1,5-a]pyridin-6-yl)methanone (18);
((3R, SR)-3-Amino-5-fluoropiperidin-1-yl)(2-(6-cyclopropyl-1-(cyclopropylmethyl)-1H-pyrrolo[2,3-b]pyridin-2-yl)-4-methoxy-3-methylpyrazolo[1,5-a]pyridin-6-yl)methanone (19);
5-(2-(6-((3R,5R)-3-Amino-5-fluoropiperidine-1-carbonyl)-4-methoxy-3-methylpyrazolo[1,5-a]pyridin-2-yl)-1-(cyclopropylmethyl)-1H-pyrrolo[2,3-b]pyridin-6-yl)isoindolin-1-one (20);
((3R,5R)-3-Amino-5-fluoropiperidin-1-yl)(2-(1-(cyclopropylmethyl)-6-(3-methyl-[1,2,4]triazolo[4,3-a]pyridin-7-yl)-1H-pyrrolo[2,3-b]pyridin-2-yl)-4-methoxy-3-methylpyrazolo[1,5-a]pyridin-6-yl)methanone (21);
((7R)-7-Amino-2-azabicyclo[2.2.1]heptan-2-yl)(2-(1-(cyclopropylmethyl)-1H-indol-2-yl)-4-methoxy-3-methylpyrazolo[1,5-a]pyridin-6-yl)methanone (22);
6-(2-(6-((7R)-7-Amino-2-azabicyclo[2.2.1]heptane-2-carbonyl)-4-methoxy-3-methylpyrazolo[1,5-a]pyridin-2-yl)-1-(cyclopropylmethyl)-1H-indol-6-yl)isoindolin-1-one (23);
5-(2-(6-((7R)-7-Amino-2-azabicyclo[2.2.1]heptane-2-carbonyl)-4-methoxy-3-methylpyrazolo[1,5-a]pyridin-2-yl)-1-(cyclopropylmethyl)-1H-indol-6-yl)isoindolin-1-one (24);
((7R)-7-Amino-2-azabicyclo[2.2. 1]heptan-2-yl)(2-(1-(cyclopropylmethyl)-6-(3-methyl-[1,2,4]triazolo[4,3-a]pyridin-7-yl)-1H-indol-2-yl)-4-methoxy-3-methylpyrazolo[1,5-a]pyridin-6-yl)methanone (25);
((7R)-7-Amino-2-azabicyclo[2.2.1]heptan-2-yl)(2-(1-(cyclopropylmethyl)-6-(4-(difluoromethoxy)phenyl)-1H-indol-2-yl)-4-methoxy-3-methylpyrazolo[1,5-a]pyridin-6-yl)methanone (26);
((7R)-7-Amino-2-azabicyclo[2.2.1]heptan-2-yl)(2-(1-(cyclopropylmethyl)-6-methyl-1H-indol-2-yl)-4-methoxy-3-methylpyrazolo[1,5-a]pyridin-6-yl)methanone (27);
((7R)-7-Amino-2-azabicyclo[2.2.1]heptan-2-yl)(2-(6-cyclopropyl-1-(cyclopropylmethyl)-1H-indol-2-yl)-4-methoxy-3-methylpyrazolo[1,5-a]pyridin-6-yl)methanone (28);
6-(2-(6-((3R,5R)-3-Amino-5-fluoropiperidine-1-carbonyl)-4-methoxy-3-methylpyrazolo[1,5-a]pyridin-2-yl)-1-(cyclopropylmethyl)-1H-indol-6-yl)isoindolin-1-one (29);
5-(2-(6-((3R,5R)-3-Amino-5-fluoropiperidine-1-carbonyl)-4-methoxy-3-methylpyrazolo[1,5-a]pyridin-2-yl)-1-(cyclopropylmethyl)-1H-indol-6-yl)isoindolin-1-one (30);
((3R,5R)-3-Amino-5-fluoropiperidin-1-yl)(2-(1-(cyclopropylmethyl)-6-(3-methyl-[1,2,4]triazolo[4,3-a]pyridin-7-yl)-1H-indol-2-yl)-4-methoxy-3-methylpyrazolo[1,5-a]pyridin-6-yl)methanone (31);
((3R,5R)-3-Amino-5-fluoropiperidin-1-yl)(2-(1-(cyclopropylmethyl)-6-(2-fluoro-3-hydroxyphenyl)-1H-indol-2-yl)-4-methoxy-3-methylpyrazolo[1,5-a]pyridin-6-yl)methanone (32);
((3R,5R)-3-Amino-5-fluoropiperidin-1-yl)(2-(1-(cyclopropylmethyl)-6-(3-(methoxymethyl)- azetidin-1-yl)-1H-indol-2-yl)-4-methoxy-3-methylpyrazolo[1,5-a]pyridin-6-yl)methanone (33);
((3R,5R)-3-Amino-5-fluoropiperidin-1-yl)(2-(6-((3R,5R)-3-amino-5-fluoropiperidin-1-yl)-1-(cyclopropylmethyl)-1H-indol-2-yl)-4-methoxy-3-methylpyrazolo[1,5-a]pyridin-6-yl)methanone (34);
((3R,5R)-3-Amino-5-fluoropiperidin-1-yl)(2-(1-(cyclopropylmethyl)-6-(4-methoxypiperidin-1-yl)-1H-indol-2-yl)-4-methoxy-3-methylpyrazolo[1,5-a]pyridin-6-yl)methanone (35);
((3R, SR)-3-Amino-5-fluoropiperidin-1-yl)(2-(1-(cyclopropylmethyl)-6-(((R)-2-fluoro-3-hydroxy-3-methylbutyl)amino)-1H-indol-2-yl)-4-methoxy-3-methylpyrazolo[1,5-a]pyridin-6-yl)methanone (36);
((3R,5R)-3-Amino-5-fluoropiperidin-1-yl)(2-(1-(cyclopropylmethyl)-6-(3-fluoro-3-(hydroxymethyl)azetidin-1-yl)-1H-indol-2-yl)-4-methoxy-3-methylpyrazolo[1,5-a]pyridin-6-yl)methanone (37);
((7R)-7-Amino-2-azabicyclo[2.2.1]heptan-2-yl)(2-(1-(cyclopropylmethyl)-1H-pyrrolo[2,3-b]pyridin-2-yl)-4-fluoro-3-methylpyrazolo[1,5-a]pyridin-6-yl)methanone (38);
((7R)-7-Amino-2-azabicyclo[2.2.1]heptan-2-yl)(2-(6-chloro-1-(cyclopropylmethyl)-1H-pyrrolo[2,3-b]pyridin-2-yl)-4-fluoro-3-methylpyrazolo[1,5-a]pyridin-6-yl)methanone (39);
3((7R)-7-Amino-2-azabicyclo[2.2.1]heptan-2-yl)(2-(1-(cyclopropylmethyl)-6-methyl-1H-pyrrolo[2,3-b]pyridin-2-yl)-4-fluoro-3-methylpyrazolo[1,5-a]pyridin-6-yl)methanone (40);
((7R)-7-Amino-2-azabicyclo[2.2.1]heptan-2-yl)(2-(6-cyclopropyl-1-(cyclopropylmethyl)-1H-pyrrolo[2,3-b]pyridin-2-yl)-4-fluoro-3-methylpyrazolo[1,5-a]pyridin-6-yl)methanone (41);
5-(2-(6-((7R)-7-Amino-2-azabicyclo[2.2.1]heptane-2-carbonyl)-4-fluoro-3-methylpyrazolo[1,5-a]pyridin-2-yl)-1-(cyclopropylmethyl)-1H-pyrrolo[2,3-b]pyridin-6-yl)isoindolin-1-one (42);
((7R)-7-Amino-2-azabicyclo[2.2.1]heptan-2-yl)(2-(1-(cyclopropylmethyl)-6-(3-methyl-[1,2,4]triazolo[4,3-a]pyridin-7-yl)-1H-pyrrolo[2,3-b]pyridin-2-yl)-4-fluoro-3-methylpyrazolo[1,5-a]pyridin-6-yl)methanone (43);
((7R)-7-Amino-2-azabicyclo[2.2.1]heptan-2-yl)(2-(1-(cyclopropylmethyl)-6-(4-(difluoromethoxy)-3-fluorophenyl)-1H-pyrrolo[2,3-b]pyridin-2-yl)-4-fluoro-3-methylpyrazolo[1,5-a]pyridin-6-yl)methanone (44);
((7R)-7-Amino-2-azabicyclo[2.2.1]heptan-2-yl)(2-(1-(cyclopropylmethyl)-6-(5-fluoro-2-methoxypyridin-4-yl)-1H-pyrrolo[2,3-b]pyridin-2-yl)-4-fluoro-3-methylpyrazolo[1,5-a]pyridin-6-yl)methanone (45);
6-(2-(6-((7R)-7-Amino-2-azabicyclo[2.2.1]heptane-2-carbonyl)-4-fluoro-3-methylpyrazolo[1,5-a]pyridin-2-yl)-1-(cyclopropylmethyl)-1H-pyrrolo[2,3-b]pyridin-6-yl)isoindolin-1-one (46);
((7R)-7-Amino-2-azabicyclo[2.2.1]heptan-2-yl)(2-(1-(cyclopropylmethyl)-6-(4-(difluoromethoxy)phenyl)-1H-pyrrolo[2,3-b]pyridin-2-yl)-4-fluoro-3-methylpyrazolo[1,5-a]pyridin-6-yl)methanone (47);
((7R)-7-Amino-2-azabicyclo[2.2.1]heptan-2-yl)(2-(1-(cyclopropylmethyl)-6-(3-fluoro-4-hydroxyphenyl)-1H-pyrrolo[2,3-b]pyridin-2-yl)-4-fluoro-3-methylpyrazolo[1,5-a]pyridin-6-yl)methanone (48);
((7R)-7-Amino-2-azabicyclo[2.2.1]heptan-2-yl)(2-(1-(cyclopropylmethyl)-6-(2-fluoro-3-hydroxyphenyl)-1H-pyrrolo[2,3-b]pyridin-2-yl)-4-fluoro-3-methylpyrazolo[1,5-a]pyridin-6-yl)methanone (49);
((7R)-7-Amino-2-azabicyclo[2.2.1]heptan-2-yl)(2-(1-(cyclopropylmethyl)-6-(3-methoxyazetidin-1-yl)-1H-pyrrolo[2,3-b]pyridin-2-yl)-4-fluoro-3-methylpyrazolo[1,5-a]pyridin-6-yl)methanone (50);
((7R)-7-Amino-2-azabicyclo[2.2.1]heptan-2-yl)(2-(1-(cyclopropylmethyl)-6-((R)-3-hydroxypiperidin-1-yl)-1H-pyrrolo[2,3-b]pyridin-2-yl)-4-fluoro-3-methylpyrazolo[1,5-a]pyridin-6-yl)methanone (51);
((3R,5R)-3-Amino-5-fluoropiperidin-1-yl)(2-(1-(cyclopropylmethyl)-1H-pyrrolo[2,3-b]pyridin-2-yl)-4-fluoro-3-methylpyrazolo[1,5-a]pyridin-6-yl)methanone (52);
((3R,5R)-3-Amino-5-fluoropiperidin-1-yl)(2-(6-chloro-1-(cyclopropylmethyl)-1H-pyrrolo[2,3-b]pyridin-2-yl)-4-fluoro-3-methylpyrazolo[1,5-a]pyridin-6-yl)methanone (53);
((3R,5R)-3-Amino-5-fluoropiperidin-1-yl)(2-(1-(cyclopropylmethyl)-6-methyl-1H-pyrrolo[2,3-b]pyridin-2-yl)-4-fluoro-3-methylpyrazolo[1,5-a]pyridin-6-yl)methanone (54);
((3R,5R)-3-Amino-5-fluoropiperidin-1-yl)(2-(1-(cyclopropylmethyl)-6-(2-fluoro-3-hydroxyphenyl)-1H-pyrrolo[2,3-b]pyridin-2-yl)-4-fluoro-3-methylpyrazolo[1,5-a]pyridin-6-yl)methanone (55);
4-(2-(6-((3R,5R)-3-Amino-5-fluoropiperidine-1-carbonyl)-4-fluoro-3-methylpyrazolo[1,5-a]pyridin-2-yl)-1-(cyclopropylmethyl)-1H-pyrrolo[2,3-b]pyridin-6-yl)-2-fluorobenzamide (56);
((3R,5R)-3-Amino-5-fluoropiperidin-1 -yl)(2-(6-cyclopropyl-1-(cyclopropylmethyl)-1H-pyrrolo[2,3-b]pyridin-2-yl)-4-fluoro-3-methylpyrazolo[1,5-a]pyridin-6-yl)methanone (57);
N-(7-(2-(6-((7R)-7-Amino-2-azabicyclo[2.2.1]heptane-2-carbonyl)-4-fluoro-3-methylpyrazolo [1,5-a]pyridin -2-yl)-1-(cyclopropylmethyl)-1H-indol-6-yl)-4-chloro-1-methyl-1H-indazol-3-yl)methanesulfonamide (58);
((7R)-7-Amino-2-azabicyclo[2.2.1]heptan-2-yl)(2-(1-(cyclopropylmethyl)-6-(2-fluoro-3-hydroxyphenyl)-1H-indol-2-yl)-4-fluoro-3-methylpyrazolo[1,5-a]pyridin-6-yl)methanone (59);
((7R)-7-Amino-2-azabicyclo[2.2.1]heptan-2-yl)(2-(1-(cyclopropylmethyl)-6-(3-fluoro-4-hydroxyphenyl)-1H-indol-2-yl)-4-fluoro-3-methylpyrazolo[1,5-a]pyridin-6-yl)methanone (60);
((7R)-7-Amino-2-azabicyclo[2.2.1]heptan-2-yl)(2-(1-(cyclopropylmethyl)-6-(4-fluoro-3-hydroxyphenyl)-1H-indol-2-yl)-4-fluoro-3-methylpyrazolo[1,5-a]pyridin-6-yl)methanone (61);
((7R)-7-Amino-2-azabicyclo[2.2.1]heptan-2-yl)(2-(1-(cyclopropylmethyl)-6-(3-methyl-[1,2,4]triazolo[4,3-a]pyridin-7-yl)-1H-indol-2-yl)-4-fluoro-3-methylpyrazolo[1,5-a]pyridin-6-yl)methanone (62);
((7R)-7-Amino-2-azabicyclo[2.2.1]heptan-2-yl)(2-(1-(cyclopropylmethyl)-1H-pyrrolo[2,3-b]pyridin-2-yl)-3-methylpyrazolo[1,5-a]pyridin-6-yl)methanone (63);
5-(2-(6-((7R)-7-Amino-2-azabicyclo[2.2.1]heptane-2-carbonyl)-3-methylpyrazolo[1,5-a]pyridin-2-yl)-1-(cyclopropylmethyl)-1H-pyrrolo[2,3-b]pyridin-6-yl)isoindolin-1-one (64);
6-(2-(6-((7R)-7-Amino-2-azabicyclo[2.2.1]heptane-2-carbonyl)-3-methylpyrazolo[1,5-a]pyridin-2-yl)-1-(cyclopropylmethyl)-1H-pyrrolo[2,3-b]pyridin-6-yl)isoindolin-1-one (65);
((7R)-7-Amino-2-azabicyclo[2.2.1]heptan-2-yl)(2-(1-(cyclopropylmethyl)-6-methyl-1H-pyrrolo[2,3-b]pyridin-2-yl)-3-methylpyrazolo[1,5-a]pyridin-6-yl)methanone (66);
((7R)-7-Amino-2-azabicyclo[2.2.1]heptan-2-yl)(2-(1-(cyclopropylmethyl)-6-(3-fluoro-4-hydroxyphenyl)-1H-pyrrolo[2,3-b]pyridin-2-yl)-3-methylpyrazolo[1,5-a]pyridin-6-yl)methanone (67);
((7R)-7-Amino-2-azabicyclo[2.2.1]heptan-2-yl)(2-(1-(cyclopropylmethyl)-6-(2-fluoro-3-hydroxyphenyl)-1H-pyrrolo[2,3-b]pyridin-2-yl)-3-methylpyrazolo[1,5-a]pyridin-6-yl)methanone (68);
4-(2-(6-((7R)-7-Amino-2-azabicyclo[2.2.1]heptane-2-carbonyl)-3-methylpyrazolo[1,5-a]pyridin-2-yl)-1-(cyclopropylmethyl)-1H-pyrrolo[2,3-b]pyridin-6-yl)-2-fluorobenzamide (69);
((7R)-7-Amino-2-azabicyclo[2.2. 1]heptan-2-yl)(2-(1-(cyclopropylmethyl)-6-(3-methyl-[1,2,4]triazolo[4,3-a]pyridin-7-yl)-1H-pyrrolo[2,3-b]pyridin-2-yl)-3-methylpyrazolo[1,5-a]pyridin-6-yl)methanone (70);
((7R)-7-Amino-2-azabicyclo[2.2.1]heptan-2-yl)(2-(6-cyclopropyl-1-(cyclopropylmethyl)-1H-pyrrolo[2,3-b]pyridin-2-yl)-3-methylpyrazolo[1,5-a]pyridin-6-yl)methanone (71);
((7R)-7-Amino-2-azabicyclo[2.2.1]heptan-2-yl)(2-(1-(cyclopropylmethyl)-6-(5-fluoro-2-methoxypyridin-4-yl)-1H-pyrrolo[2,3-b]pyridin-2-yl)-3-methylpyrazolo[1,5-a]pyridin-6-yl)methanone (72);
((7R)-7-Amino-2-azabicyclo[2.2.1]heptan-2-yl)(2-(1-(cyclopropylmethyl)-6-(4-(difluoromethoxy)-3-fluorophenyl)-1H-pyrrolo[2,3-b]pyridin-2-yl)-3-methylpyrazolo[1,5-a]pyridin-6-yl)methanone (73);
((7R)-7-Amino-2-azabicyclo[2.2.1]heptan-2-yl)(2-(1-(cyclopropylmethyl)-6-(3,5-difluoro-4-hydroxyphenyl)-1H-pyrrolo[2,3-b]pyridin-2-yl)-3-methylpyrazolo[1,5-a]pyridin-6-yl)methanone (74);
((7R)-7-Amino-2-azabicyclo[2.2.1]heptan-2-yl)(2-(1-(cyclopropylmethyl)-6-(4-fluoro-3-hydroxyphenyl)-1H-pyrrolo[2,3-b]pyridin-2-yl)-3-methylpyrazolo[1,5-a]pyridin-6-yl)methanone (75);
((7R)-7-Amino-2-azabicyclo[2.2.1]heptan-2-yl)(2-(1-(cyclopropylmethyl)-6-(4-methoxypiperidin-1-yl)-1H-pyrrolo[2,3-b]pyridin-2-yl)-3-methylpyrazolo[1,5-a]pyridin-6-yl)methanone (76);
((7R)-7-Amino-2-azabicyclo[2.2.1]heptan-2-yl)(2-(1-(cyclopropylmethyl)-6-(4-hydroxypiperidin-1-yl)-1H-pyrrolo[2,3-b]pyridin-2-yl)-3-methylpyrazolo[1,5-a]pyridin-6-yl)methanone (77);
((7R)-7-Amino-2-azabicyclo[2.2.1]heptan-2-yl)(2-(1-(cyclopropylmethyl)-6-((R)-3-hydroxypiperidin-1-yl)-1H-pyrrolo[2,3-b]pyridin-2-yl)-3-methylpyrazolo[1,5-a]pyridin-6-yl)methanone (78);
((7R)-7-Amino-2-azabicyclo[2.2.1]heptan-2-yl)(2-(1-(cyclopropylmethyl)-6-(3-methoxyazetidin-1-yl)-1H-pyrrolo[2,3-b]pyridin-2-yl)-3-methylpyrazolo[1,5-a]pyridin-6-yl)methanone (79);
((3R,5R)-3-Amino-5-fluoropiperidin-1-yl)(2-(1-(cyclopropylmethyl)-1H-pyrrolo[2,3-b]pyridin-2-yl)-3-methylpyrazolo[1,5-a]pyridin-6-yl)methanone (80);
5-(2-(6-((3R,5R)-3-Amino-5-fluoropiperidine-1-carbonyl)-3-methylpyrazolo[1,5-a]pyridin-2-yl)-1-(cyclopropylmethyl)-1H-pyrrolo[2,3-b]pyridin-6-yl)isoindolin-1-one (81);
((3R,5R)-3-Amino-5-fluoropiperidin-1-yl)(2-(1-(cyclopropylmethyl)-6-methyl-1H-pyrrolo[2,3-b]pyridin-2-yl)-3-methylpyrazolo[1,5-a]pyridin-6-yl)methanone (82);
6-(2-(6-((3R,5R)-3-Amino-5-fluoropiperidine-1-carbonyl)-3-methylpyrazolo[1,5-a]pyridin-2-yl)-1-(cyclopropylmethyl)-1H-pyrrolo[2,3-b]pyridin-6-yl)isoindolin-1-one (83);
4-(2-(6-((3R,5R)-3-Amino-5-fluoropiperidine-1-carbonyl)-3-methylpyrazolo[1,5-a]pyridin-2-yl)-1-(cyclopropylmethyl)-1H-pyrrolo[2,3-b]pyridin-6-yl)-2-fluorobenzamide (84);
4-(2-(6-((3R,5R)-3-Amino-5-fluoropiperidine-1-carbonyl)-3-methylpyrazolo[1,5-a]pyridin-2-yl)-1-(cyclopropylmethyl)-1H-pyrrolo[2,3-b]pyridin-6-yl)-2-chlorobenzamide (85);
((3R,5R)-3-Amino-5-fluoropiperidin-1-yl)(2-(1-(cyclopropylmethyl)-6-(2-fluoro-3-hydroxyphenyl)-1H-pyrrolo[2,3-b]pyridin-2-yl)-3-methylpyrazolo[1,5-a]pyridin-6-yl)methanone (86);
((3R,5R)-3-Amino-5-fluoropiperidin-1-yl)(2-(1-(cyclopropylmethyl)-6-(4-methoxypiperidin-1-yl)-1H-pyrrolo[2,3-b]pyridin-2-yl)-3-methylpyrazolo[1,5-a]pyridin-6-yl)methanone (87);
((3R,5R)-3-Amino-5-fluoropiperidin-1-yl)(2-(1-(cyclopropylmethyl)-6-(4-hydroxypiperidin-1-yl)-1H-pyrrolo[2,3-b]pyridin-2-yl)-3-methylpyrazolo[1,5-a]pyridin-6-yl)methanone (88);
6-(2-(6-((7R)-7-Amino-2-azabicyclo[2.2.1]heptane-2-carbonyl)-3-methylpyrazolo[1,5-a]pyridin-2-yl)-1-(cyclopropylmethyl)-1H-indol-6-yl)isoindolin-1-one (89);
5-(2-(6-((7R)-7-Amino-2-azabicyclo[2.2.1]heptane-2-carbonyl)-3-methylpyrazolo[1,5-a]pyridin-2-yl)-1-(cyclopropylmethyl)-1H-indol-6-yl)isoindolin-1-one (90);
((7R)-7-Amino-2-azabicyclo[2.2. 1]heptan-2-yl)(2-(1-(cyclopropylmethyl)-6-(3-fluoro-3-(hydroxymethyl)azetidin-1-yl)-1H-indol-2-yl)-3-methylpyrazolo[1,5-a]pyridin-6-yl)methanone (91);
((3R,5R)-3-Amino-5-fluoropiperidin-1-yl)(2-(1-(cyclopropylmethyl)-6-(2-fluoro-3-hydroxyphenyl)-1H-indol-2-yl)-3-methylpyrazolo[1,5-a]pyridin-6-yl)methanone (92);
4-(2-(6-((3R,5R)-3-Amino-5-fluoropiperidine-1-carbonyl)-3-methylpyrazolo[1,5-a]pyridin-2-yl)-1-(cyclopropylmethyl)-1H-indol-6-yl)-2-fluorobenzamide (93);
((3R,5R)-3-Amino-5-fluoropiperidin-1-yl)(2-(1-(cyclopropylmethyl)-6-(3-fluoro-4-hydroxyphenyl)-1H-indol-2-yl)-3-methylpyrazolo[1,5-a]pyridin-6-yl)methanone (94);
5-(2-(6-((3R,5R)-3-Amino-5-fluoropiperidine-1-carbonyl)-3-methylpyrazolo[1,5-a]pyridin-2-yl)-1-(cyclopropylmethyl)-1H-indol-6-yl)isoindolin-1-one (95);
6-(2-(6-((3R,5R)-3-Amino-5-fluoropiperidine-1-carbonyl)-3-methylpyrazolo[1,5-a]pyridin-2-yl)-1-(cyclopropylmethyl)-1H-indol-6-yl)isoindolin-1-one (96);
((7R)-7-Amino-2-azabicyclo[2.2.1]heptan-2-yl)(2-(1-(cyclopropylmethyl)-6-(2-hydroxypropan-2-yl)-1H-indol-2-yl)-4-methoxy-3-methylpyrazolo[1,5-a]pyridin-6-yl)methanone (97);
((3R,5R)-3-Amino-5-fluoropiperidin-1-yl)(2-(1-(cyclopropylmethyl)-6-(2-hydroxypropan-2-yl)-1H-indol-2-yl)-4-methoxy-3-methylpyrazolo[1,5-a]pyridin-6-yl)methanone (98);
((3R,5R)-3-Amino-5-fluoropiperidin-1-yl)(2-(1-(cyclopropylmethyl)-6-(1,1,1-trifluoro-2-hydroxypropan-2-yl)-1H-indol-2-yl)-4-methoxy-3-methylpyrazolo[1,5-a]pyridin-6-yl)methanone (99);
((7R)-7-Amino-2-azabicyclo[2.2.1]heptan-2-yl)(2-(1-(cyclopropylmethyl)-6-(2-hydroxypropan-2-yl)-1H-indol-2-yl)-3-methylpyrazolo[1,5-a]pyridin-6-yl)methanone (100);
((7R)-7-Amino-2-azabicyclo[2.2.1]heptan-2-yl)(2-(1-(cyclopropylmethyl)-6-(1,1,1-trifluoro-2-hydroxypropan-2-yl)-1H-indol-2-yl)-3-methylpyrazolo[1,5-a]pyridin-6-yl)methanone (101);
((7R)-7-Amino-2-azabicyclo[2.2.1]heptan-2-yl)(2-(1-(cyclopropylmethyl)-6-(2-hydroxypropan-2-yl)-1H-pyrrolo[2,3-b]pyridin-2-yl)-3-methylpyrazolo[1,5-a]pyridin-6-yl)methanone (102);
((7R)-7-Amino-2-azabicyclo[2.2.1]heptan-2-yl)(2-(1-(cyclopropylmethyl)-6-(1,1,1-trifluoro-2-hydroxypropan-2-yl)-1H-pyrrolo[2,3-b]pyridin-2-yl)-3-methylpyrazolo[1,5-a]pyridin-6-yl)methanone (103);
((7R)-7-Amino-2-azabicyclo[2.2.1]heptan-2-yl)(2-(1-(cyclopropylmethyl)-6-(1-hydroxyethyl)-1H-pyrrolo[2,3-b]pyridin-2-yl)-3-methylpyrazolo[1,5-a]pyridin-6-yl)methanone (104);
((3R,5R)-3-Amino-5-fluoropiperidin-1-yl)(2-(1-(cyclopropylmethyl)-6-(2-hydroxypropan-2-yl)-1H-pyrrolo[2,3-b]pyridin-2-yl)-3-methylpyrazolo[1,5-a]pyridin-6-yl)methanone (105);
((3R,5R)-3-Amino-5-fluoropiperidin-1-yl)(2-(1-(cyclopropylmethyl)-6-(1,1,1-trifluoro-2-hydroxypropan-2-yl)-1H-pyrrolo[2,3-b]pyridin-2-yl)-3-methylpyrazolo[1,5-a]pyridin-6-yl)methanone (106, 107 as diastereomers);
((7R)-7-Amino-2-azabicyclo [2.2.1 ]heptan-2-yl)(2-(6-chloro-1-(cyclopropylmethyl)-1H-pyrrolo[2,3-b]pyridin-2-yl)-3-methylpyrazolo[1,5-a]pyrimidin-6-yl)methanone (108);
((7R)-7-Amino-2-azabicyclo[2.2.1]heptan-2-yl)(2-(1-(cyclopropylmethyl)-6-(2-fluoro-3-hydroxyphenyl)-1H-pyrrolo[2,3-b]pyridin-2-yl)-3-methylpyrazolo[1,5-a]pyrimidin-6-yl)methanone (109);
6-(2-(6-((7R)-7-Amino-2-azabicyclo[2.2.1]heptane-2-carbonyl)-3-methylpyrazolo[1,5-a]pyrimidin-2-yl)-1-(cyclopropylmethyl)-1H-pyrrolo[2,3-b]pyridin-6-yl)isoindolin-1-one (110);
((7R)-7-Amino-2-azabicyclo[2.2.1]heptan-2-yl)(2-(1-(cyclopropylmethyl)-6-(3-fluoro-4-hydroxyphenyl)-1H-pyrrolo[2,3-b]pyridin-2-yl)-3-methylpyrazolo[1,5-a]pyrimidin-6-yl)methanone (111);
4-(2-(6-((7R)-7-Amino-2-azabicyclo[2.2.1]heptane-2-carbonyl)-3-methylpyrazolo[1,5-a]pyrimidin-2-yl)-1-(cyclopropylmethyl)-1H-pyrrolo[2,3-b]pyridin-6-yl)-2-fluorobenzamide (112);
((7R)-7-Amino-2-azabicyclo[2.2.1]heptan-2-yl)(2-(1-(cyclopropylmethyl)-1H-indol-2-yl)-4-methoxy-3-methylpyrazolo[1,5-a]pyrazin-6-yl)methanone (113);
((3R,5R)-3-Amino-5-fluoropiperidin-1-yl)(2-(1-(cyclopropylmethyl)-1H-indol-2-yl)-4-methoxy-3-methylpyrazolo[1,5-a]pyrazin-6-yl)methanone (114);
((7R)-7-Amino-2-azabicyclo[2.2.1]heptan-2-yl)(2-(1-(cyclopropylmethyl)-1H-pyrrolo[2,3-b]pyridin-2-yl)-4-methoxy-3-methylpyrazolo[1,5-a]pyrazin-6-yl)methanone (115);
(2-(1-(Cyclopropylmethyl)-1H-pyrrolo[2,3-b]pyridin-2-yl)-4-methoxy-3-methylpyrazolo[1,5-a]pyrazin-6-yl)(2,6-diazaspiro[3.3]heptan-2-yl)methanone (116);
(R)-N-(3-Amino-2-fluoropropyl)-2-(1-(cyclopropylmethyl)-1H-pyrrolo[2,3-b]pyridin-2-yl)-4-methoxy-3-methylpyrazolo[1,5-a]pyrazine-6-carboxamide (117);
1-[2-(2-{6-[(7R)-7-Amino-2-azabicyclo[2.2.1]heptane-2-carbonyl]-4-methoxy-3-methylpyrazolo[1,5-a]pyridin-2-yl}-1-(cyclopropylmethyl)-1H-pyrrolo[2,3-b]pyridin-6-yl)phenyl]imidazolidin-2-one (205);
4-(2-{6-[(7R)-7-Amino-2-azabicyclo[2.2.1]heptane-2-carbonyl]-4-methoxy-3-methylpyrazolo[1,5-a]pyridin-2-yl}-1-(cyclopropylmethyl)-1H-pyrrolo[2,3-b]pyridin-6-yl)-2-methylbenzamide (206);
4-(2-{6-[(3R,5R)-3-Amino-5-fluoropiperidine-1-carbonyl]-4-methoxy-3-methylpyrazolo[1,5-a]pyridin-2-yl}-1-(cyclopropylmethyl)-1H-pyrrolo[2,3-b]pyridin-6-yl)-2-fluoro-5-methylbenzamide (207);
4-(2-{6-[(3R)-3-Aminopiperidine-1-carbonyl]-4-methoxy-3-methylpyrazolo[1,5-a]pyridin-2-yl}-1-(cyclopropylmethyl)-1H-pyrrolo[2,3-b]pyridin-6-yl)-2-fluoro-5-methylbenzamide (208);
[2-(2-{6-[(3R,5R)-3-Amino-5-fluoropiperidine-1-carbonyl]-4-methoxy-3-methylpyrazolo[1,5-a]pyridin-2-yl }-1-(cyclopropylmethyl)-1H-indol-6-yl)phenyl]methanol (209);
[3-(2-{6-[(3R,5R)-3-Amino-5-fluoropiperidine-1-carbonyl]-4-methoxy-3-methylpyrazolo[1,5-a]pyridin-2-yl }-1-(cyclopropylmethyl)-1H-indol-6-yl)phenyl]methanol (210);
N-{[3-(2-{6-[(3R,5R)-3-Amino-5-fluoropiperidine-1-carbonyl]-4-methoxy-3-methylpyrazolo[1,5-a]pyridin-2-yl }-1-(cyclopropylmethyl)-1H-indol-6-yl)phenyl]methyl}methanesulfonamide (211);
(3R,5R)-1-{2-[1-(Cyclopropylmethyl)-6-(3-methoxyphenyl)-1H-indol-2-yl]-4-methoxy-3-methylpyrazolo[1,5-a]pyridine-6-carbonyl}-5-fluoropiperidin-3-amine (212);
N-[3-(2-{6-[(3R,5R)-3-Amino-5-fluoropiperidine-1-carbonyl]-4-methoxy-3-methylpyrazolo[1,5-a]pyridin-2-yl}-1-(cyclopropylmethyl)-1H-indol-6-yl)phenyl]methanesulfonamide (213);
N-[4-(2-{6-[(3R,5R)-3-Amino-5-fluoropiperidine-1-carbonyl]-4-methoxy-3-methylpyrazolo[1,5-a]pyridin-2-yl}-1-(cyclopropylmethyl)-1H-indol-6-yl)phenyl]acetamide (214);
N-{[3-(2-{6-[(3R,5R)-3-Amino-5-fluoropiperidine-1-carbonyl]-4-methoxy-3-methylpyrazolo[1,5-a]pyridin-2-yl }-1-(cyclopropylmethyl)-1H-indol-6-yl)phenyl]methyl} acetamide (215);
N-[4-(2-{6-[(3R,5R)-3-Amino-5-fluoropiperidine-1-carbonyl]-4-methoxy-3-methylpyrazolo[1,5-a]pyridin-2-yl }-1-(cyclopropylmethyl)-1H-indol-6-yl)phenyl]methanesulfonamide (216);
4-(2-{6-[(3R,5R)-3-Amino-5-fluoropiperidine-1-carbonyl]-4-methoxy-3-methylpyrazolo[1,5-a]pyridin-2-yl}-1-(cyclopropylmethyl)-1H-indol-6-yl)-N,N-dimethylbenzamide (217);
(3R,5R)-1-{2-[1-(Cyclopropylmethyl)-6-(1H-indazol-5-yl)-1H-indol-2-yl]-4-methoxy-3-methylpyrazolo[1,5-a]pyridine-6-carbonyl}-5-fluoropiperidin-3-amine (218);
(3R,5R)-1-{2-[1-(Cyclopropylmethyl)-6-(1-methyl-1H-indazol-5-yl)-1H-indol-2-yl]-4-methoxy-3-methylpyrazolo[1,5-a]pyridine-6-carbonyl}-5-fluoropiperidin-3-amine (219);
(3R,5R)-1-{2-[1-(Cyclopropylmethyl)-6-[3-(2H-1,2,3,4-tetrazol-5-yl)phenyl]-1H-indol-2-yl]-4-methoxy-3-methylpyrazolo[1,5-a]pyridine-6-carbonyl}-5-fluoropiperidin-3-amine (220);
N-{[4-(2-{6-[(3R,5R)-3-Amino-5-fluoropiperidine-1-carbonyl]-4-methoxy-3-methylpyrazolo[1,5-a]pyridin-2-yl}-1-(cyclopropylmethyl)-1H-indol-6-yl)phenyl]methyl} acetamide (221);
(3R,5R)-1-{2-[1-(Cyclopropylmethyl)-6-(1H-indazol-4-yl)-1H-indol-2-yl]-4-methoxy-3-methylpyrazolo[1,5-a]pyridine-6-carbonyl}-5-fluoropiperidin-3-amine (222);
2-(2-{6-[(3R,5R)-3-Amino-5-fluoropiperidine-1-carbonyl]-4-methoxy-3-methylpyrazolo[1,5-a]pyridin-2-yl}-1-(cyclopropylmethyl)-1H-indol-6-yl)benzamide (223);
N-{[4-(2-{6-[(3R,5R)-3-Amino-5-fluoropiperidine-1-carbonyl]-4-methoxy-3-methylpyrazolo[1,5-a]pyridin-2-yl}-1-(cyclopropylmethyl)-1H-indol-6-yl)phenyl]methyl}methanesulfonamide (224);
6-(2-{6-[(3R,5R)-3-Amino-5-fluoropiperidine-1-carbonyl]-4-methoxy-3-methylpyrazolo[1,5-a]pyridin-2-yl}-1-(cyclopropylmethyl)-1H-indol-6-yl)-8-fluoro-5-methyl-1,2-dihydroquinolin-2-one (225);
N-[5-(2-{6-[(3R,5R)-3-Amino-5-fluoropiperidine-1-carbonyl]-4-methoxy-3-methylpyrazolo[1,5-a]pyridin-2-yl}-1-(cyclopropylmethyl)-1H-indol-6-yl)pyridin-2-yl]acetamide (226);
[3-(2-{6-[(3R)-3-Aminopiperidine-1-carbonyl]-4-methoxy-3-methylpyrazolo[1,5-a]pyridin-2-yl}-1-(cyclopropylmethyl)-1H-indol-6-yl)phenyl]methanol (227);
N-{[4-(2-{6-[(3R)-3-Aminopiperidine-1-carbonyl]-4-methoxy-3-methylpyrazolo[1,5-a]pyridin-2-yl}-1-(cyclopropylmethyl)-1H-indol-6-yl)phenyl]methyl}acetamide (228);
4-(2-{6-[(3R)-3-Aminopiperidine-1-carbonyl]-4-methoxy-3-methylpyrazolo[1,5-a]pyridin-2-yl}-1-(cyclopropylmethyl)-1H-indol-6-yl)benzamide (229);
4-(2-{6-[(3R)-3-Aminopiperidine-1-carbonyl]-4-methoxy-3-methylpyrazolo[1,5-a]pyridin-2-yl}-1-(cyclopropylmethyl)-1H-indol-6-yl)-N,N-dimethylbenzamide (230);
N-[4-(2-{6-[(3R)-3-Aminopiperidine-1-carbonyl]-4-methoxy-3-methylpyrazolo[1,5-a]pyridin-2-yl}-1-(cyclopropylmethyl)-1H-indol-6-yl)phenyl]methanesulfonamide (231);
N-{[3-(2-{6-[(3R)-3-Aminopiperidine-1-carbonyl]-4-methoxy-3-methylpyrazolo[1,5-a]pyridin-2-yl}-1-(cyclopropylmethyl)-1H-indol-6-yl)phenyl]methyl}acetamide (232);
[2-(2-{6-[(3R)-3-Aminopiperidine-1-carbonyl]-4-methoxy-3-methylpyrazolo[1,5-a]pyridin-2-yl}-1-(cyclopropylmethyl)-1H-indol-6-yl)phenyl]methanol (233);
N-{[4-(2-{6-[(3R)-3-Aminopiperidine-1-carbonyl]-4-methoxy-3-methylpyrazolo[1,5-a]pyridin-2-yl}-1-(cyclopropylmethyl)-1H-indol-6-yl)phenyl]methyl}methanesulfonamide (234);
N-{[3-(2-{6-[(3R)-3-Aminopiperidine-1-carbonyl]-4-methoxy-3-methylpyrazolo[1,5-a]pyridin-2-yl }-1-(cyclopropylmethyl)-1H-indol-6-yl)phenyl]methyl} methanesulfonamide (235);
[3-(2-{6-[(3R)-3-Aminopiperidine-1-carbonyl]-4-methoxy-3-methylpyrazolo[1,5-a]pyridin-2-yl}-1-(cyclopropylmethyl)-1H-indol-6-yl)phenyl]methanol (236);
2-(2-{6-[(3R)-3-Aminopiperidine-1-carbonyl]-4-methoxy-3-methylpyrazolo[1,5-a]pyridin-2-yl}-1-(cyclopropylmethyl)-1H-indol-6-yl)benzamide (237);
(3R)-1-{2-[1-(Cyclopropylmethyl)-6-(1H-indazol-4-yl)-1H-indol-2-yl]-4-methoxy-3-methylpyrazolo[1,5-a]pyridine-6-carbonyl}piperidin-3-amine (238);
6-(2-{6-[(3R)-3-Aminopiperidine-1-carbonyl]-4-methoxy-3-methylpyrazolo[1,5-a]pyridin-2-yl}-1-(cyclopropylmethyl)-1H-indol-6-yl)-8-fluoro-5-methyl-1,2-dihydroquinolin-2-one (239);
N-[5-(2-{6-[(3R)-3-Aminopiperidine-1-carbonyl]-4-methoxy-3-methylpyrazolo[1,5-a]pyridin-2-yl}-1-(cyclopropylmethyl)-1H-indol-6-yl)pyridin-2-yl]acetamide (240);
(3R,5R)-1-{2-[1-(Cyclopropylmethyl)-6-(4-methanesulfonylpiperidin-1-yl)-1H-indol-2-yl]-4-methoxy-3-methylpyrazolo[1,5-a]pyridine-6-carbonyl}-5-fluoropiperidin-3-amine (241);
1-(2-{6-[(3R,5R)-3-Amino-5-fluoropiperidine-1-carbonyl]-4-methoxy-3-methylpyrazolo[1,5-a]pyridin-2-yl}-1-(cyclopropylmethyl)-1H-indol-6-yl)-N,N-dimethylpiperidine-4-carboxamide (242);
N-[1-(2-{6-[(3R,5R)-3-Amino-5-fluoropiperidine-1-carbonyl]-4-methoxy-3-methylpyrazolo[1,5-a]pyridin-2-yl}-1-(cyclopropylmethyl)-1H-indol-6-yl)piperidin-4-yl]-N-methylacetamide (243);
N-[1-(2-{6-[(3R,5R)-3-Amino-5-fluoropiperidine-1-carbonyl]-4-methoxy-3-methylpyrazolo[1,5-a]pyridin-2-yl}-1-(cyclopropylmethyl)-1H-indol-6-yl)piperidin-4-yl]-N-methylacetamide (244);
Methyl N-[1-(2-{6-[(3R,5R)-3-amino-5-fluoropiperidine-1-carbonyl]-4-methoxy-3-methylpyrazolo[1,5-a]pyridin-2-yl}-1-(cyclopropylmethyl)-1H-indol-6-yl)piperidin-4-yl]-N-methylcarbamate (245);
N-[1-(2-{6-[(3R,5R)-3-Amino-5-fluoropiperidine-1-carbonyl]-4-methoxy-3-methylpyrazolo[1,5-a]pyridin-2-yl}-1-(cyclopropylmethyl)-1H-indol-6-yl)piperidin-4-yl]-N-methylmethanesulfonamide (246);
N-[1-(2-{6-[(3R,5R)-3-Amino-5-fluoropiperidine-1-carbonyl]-4-methoxy-3-methylpyrazolo[1,5-a]pyridin-2-yl}-1-(cyclopropylmethyl)-1H-indol-6-yl)azetidin-3-yl]-N-methylmethanesulfonamide (247);
(3R)-1-{2-[1-(Cyclopropylmethyl)-6-(4-methanesulfonylpiperidin-1-yl)-1H-indol-2-yl]-4-methoxy-3-methylpyrazolo[1,5-a]pyridine-6-carbonyl}piperidin-3-amine (248);
(3R)-1-{2-[1-(Cyclopropylmethyl)-6-(4-methoxypiperidin-1-yl)-1H-indol-2-yl]-4-methoxy-3-methylpyrazolo[1,5-a]pyridine-6-carbonyl}piperidin-3-amine (249);
2-[1-(2-{6-[(3R)-3-Aminopiperidine-1-carbonyl]-4-methoxy-3-methylpyrazolo[1,5-a]pyridin-2-yl}-1-(cyclopropylmethyl)-1H-indol-6-yl)piperidin-4-yl]-N,N-dimethylacetamide (250);
(3R)-1-{2-[1-(Cyclopropylmethyl)-6-[4-(3-methoxyazetidine-1-carbonyl)piperidin-1-yl]-1H-indol-2-yl]-4-methoxy-3-methylpyrazolo[1,5-a]pyridine-6-carbonyl }piperidin-3-amine (251);
N-[1-(2-{6-[(3R)-3-Aminopiperidine-1-carbonyl]-4-methoxy-3-methylpyrazolo[1,5-a]pyridin-2-yl}-1-(cyclopropylmethyl)-1H-indol-6-yl)piperidin-4-yl]-N-methylacetamide (252);
N-[1-(2-{6-[(3R)-3-Aminopiperidine-1-carbonyl]-4-methoxy-3-methylpyrazolo[1,5-a]pyridin-2-yl}-1-(cyclopropylmethyl)-1H-indol-6-yl)piperidin-4-yl]-N-methylacetamide (253);
N-[1-(2-{6-[(3R)-3-Aminopiperidine-1-carbonyl]-4-methoxy-3-methylpyrazolo[1,5-a]pyridin-2-yl}-1-(cyclopropylmethyl)-1H-indol-6-yl)piperidin-4-yl]-N-methylmethanesulfonamide (254);
Methyl N-[1-(2-{6-[(3R)-3-aminopiperidine-1-carbonyl]-4-methoxy-3-methylpyrazolo[1,5-a]pyridin-2-yl}-1-(cyclopropylmethyl)-1H-indol-6-yl)piperidin-4-yl]-N-methylcarbamate (255);
N-[1-(2-{6-[(3R)-3-Aminopiperidine-1-carbonyl]-4-methoxy-3-methylpyrazolo[1,5-a]pyridin-2-yl}-1-(cyclopropylmethyl)-1H-indol-6-yl)azetidin-3-yl]-N-methylmethanesulfonamide (256);
1-[2-(2-{6-[(7R)-7-Amino-2-azabicyclo[2.2.1]heptane-2-carbonyl]-4-fluoro-3-methylpyrazolo[1,5-a]pyridin-2-yl}-1-(cyclopropylmethyl)-1H-pyrrolo[2,3-b]pyridin-6-yl)phenyl]imidazolidin-2-one (257);
1-[4-(2-{6-[(7R)-7-Amino-2-azabicyclo[2.2.1]heptane-2-carbonyl]-4-fluoro-3-methylpyrazolo[1,5-a]pyridin-2-yl}-1-(cyclopropylmethyl)-1H-pyrrolo[2,3-b]pyridin-6-yl)phenyl]pyrrolidin-2-one (258);
3-(2-{6-[(7R)-7-Amino-2-azabicyclo[2.2.1]heptane-2-carbonyl]-4-fluoro-3-methylpyrazolo[1,5-a]pyridin-2-yl}-1-(cyclopropylmethyl)-1H-pyrrolo[2,3-b]pyridin-6-yl)-2-methylphenol (259);
4-(2-{6-[(7R)-7-Amino-2-azabicyclo[2.2.1]heptane-2-carbonyl]-4-fluoro-3-methylpyrazolo[1,5-a]pyridin-2-yl}-1-(cyclopropylmethyl)-1H-pyrrolo[2,3-b]pyridin-6-yl)-2-fluoro-5-methylbenzamide (260);
1-[4-(2-{6-[(7R)-7-Amino-2-azabicyclo[2.2.1]heptane-2-carbonyl]-4-fluoro-3-methylpyrazolo[1,5-a]pyridin-2-yl}-1-(cyclopropylmethyl)-1H-pyrrolo[2,3-b]pyridin-6-yl)phenyl]imidazolidin-2-one (261);
4-(2-{6-[(7R)-7-Amino-2-azabicyclo[2.2.1]heptane-2-carbonyl]-4-fluoro-3-methylpyrazolo[1,5-a]pyridin-2-yl}-1-(cyclopropylmethyl)-1H-pyrrolo[2,3-b]pyridin-6-yl)-2-methylbenzamide (262);
4-(2-{6-[(7R)-7-Amino-2-azabicyclo[2.2.1]heptane-2-carbonyl]-4-fluoro-3-methylpyrazolo[1,5-a]pyridin-2-yl}-1-(cyclopropylmethyl)-1H-pyrrolo[2,3-b]pyridin-6-yl)benzamide (263);
5-(2-{6-[(7R)-7-Amino-2-azabicyclo[2.2.1]heptane-2-carbonyl]-4-fluoro-3-methylpyrazolo[1,5-a]pyridin-2-yl}-1-(cyclopropylmethyl)-1H-pyrrolo[2,3-b]pyridin-6-yl)-N-methylpyridine-2-carboxamide (264);
((7R)-7-Amino-2-azabicyclo[2.2.1]heptan-2-yl)(2-(1-(cyclopropylmethyl)-6-((R)-3-hydroxypiperidin-1-yl)-1H-pyrrolo[2,3-b]pyridin-2-yl)-4-fluoro-3-methylpyrazolo[1,5-a]pyridin-6-yl)methanone (265);
(7R)-2-{2-[1-(Cyclopropylmethyl)-6-(morpholin-4-yl)-1H-pyrrolo[2,3-b]pyridin-2-yl]-4-fluoro-3-methylpyrazolo[1,5-a]pyridine-6-carbonyl}-2-azabicyclo[2.2.1]heptan-7-amine (266);
4-(2-{6-[(7R)-7-Amino-2-azabicyclo[2.2.1]heptane-2-carbonyl]-4-fluoro-3-methylpyrazolo[1,5-a]pyridin-2-yl }-1-(cyclopropylmethyl)-1H-pyrrolo[2,3-b]pyridin-6-yl)morpholin-3-one (267);
(7R)-2-{2-[1-(Cyclopropylmethyl)-6-(4-methanesulfonylpiperidin-1-yl)-1H-pyrrolo[2,3-b]pyridin-2-yl]-4-fluoro-3-methylpyrazolo[1,5-a]pyridine-6-carbonyl}-2-azabicyclo[2.2.1]heptan-7-amine (268);
8-(2-{6-[(3R)-3-Aminopiperidine-1-carbonyl]-4-fluoro-3-methylpyrazolo[1,5-a]pyridin-2-yl}-1-(cyclopropylmethyl)-1H-pyrrolo[2,3-b]pyridin-6-yl)-3-methyl-1-oxa-3,8-diazaspiro[4.5]decan-2-one (269);
(3R)-1-{2-[1-(Cyclopropylmethyl)-6-(4-methanesulfonylpiperidin-1-yl)-1H-pyrrolo[2,3-b]pyridin-2-yl]-4-fluoro-3-methylpyrazolo[1,5-a]pyridine-6-carbonyl }piperidin-3-amine (270);
N-[1-(2-{6-[(3R)-3-Aminopiperidine-1-carbonyl]-4-fluoro-3-methylpyrazolo[1,5-a]pyridin-2-yl}-1-(cyclopropylmethyl)-1H-pyrrolo[2,3-b]pyridin-6-yl)piperidin-4-yl]-N-methylacetamide (271);
Methyl N-[1-(2-{6-[(3R)-3-aminopiperidine-1-carbonyl]-4-fluoro-3-methylpyrazolo[1,5-a]pyridin-2-yl}-1-(cyclopropylmethyl)-1H-pyrrolo[2,3-b]pyridin-6-yl)piperidin-4-yl]-N-methylcarbamate (272);
1-[1-(2-{6-[(3R)-3-Aminopiperidine-1-carbonyl]-4-fluoro-3-methylpyrazolo[1,5-a]pyridin-2-yl}-1-(cyclopropylmethyl)-1H-pyrrolo[2,3-b]pyridin-6-yl)piperidin-4-yl]pyrrolidin-2-one (273);
(5S)-5-{[(2-{6-[(7R)-7-Amino-2-azabicyclo[2.2.1]heptane-2-carbonyl]-4-fluoro-3-methylpyrazolo[1,5-a]pyridin-2-yl}-1-(cyclopropylmethyl)-1H-pyrrolo[2,3-b]pyridin-6-yl)oxy]methyl}pyrrolidin-2-one (274);
4-{[(2-{6-[(7R)-7-Amino-2-azabicyclo[2.2.1]heptane-2-carbonyl]-4-fluoro-3-methylpyrazolo[1,5-a]pyridin-2-yl }-1-(cyclopropylmethyl)-1H-pyrrolo[2,3-b]pyridin-6-yl)oxy]methyl}pyrrolidin-2-one (275);
(3R)-1-{4-Chloro-2-[1-(cyclopropylmethyl)-6-(4-methoxypiperidin-1-yl)-1H-pyrrolo[2,3-b]pyridin-2-yl]-3-methylpyrazolo[1,5-a]pyridine-6-carbonyl}piperidin-3-amine (276);
4-(2-{6-[(3R)-3-Aminopiperidine-1-carbonyl]-4-fluoro-3-methylpyrazolo[1,5-a]pyridin-2-yl }-1-(cyclopropylmethyl)-1H-pyrrolo[2,3-b]pyridin-6-yl)-2-fluoro-5-methylbenzamide (277);
3-(2-{6-[(3R)-3-Aminopiperidine-1-carbonyl]-4-fluoro-3-methylpyrazolo[1,5-a]pyridin-2-yl}-1-(cyclopropylmethyl)-1H-pyrrolo[2,3-b]pyridin-6-yl)-2-chlorophenol (278);
4-(2-{6-[(3R)-3-Aminopiperidine-1-carbonyl]-4-fluoro-3-methylpyrazolo[1,5-a]pyridin-2-yl}-1-(cyclopropylmethyl)-1H-pyrrolo[2,3-b]pyridin-6-yl)-2-fluorobenzamide (279);
1-[4-(2-{6-[(3R)-3-Aminopiperidine-1-carbonyl]-4-fluoro-3-methylpyrazolo[1,5-a]pyridin-2-yl}-1-(cyclopropylmethyl)-1H-pyrrolo[2,3-b]pyridin-6-yl)phenyl]imidazolidin-2-one (280);
1-[2-(2-{6-[(3R)-3-Aminopiperidine-1-carbonyl]-4-fluoro-3-methylpyrazolo[1,5-a]pyridin-2-yl}-1-(cyclopropylmethyl)-1H-pyrrolo[2,3-b]pyridin-6-yl)phenyl]imidazolidin-2-one (281);
3-(2-{6-[(3R)-3-Aminopiperidine-1-carbonyl]-4-fluoro-3-methylpyrazolo[1,5-a]pyridin-2-yl}-1-(cyclopropylmethyl)-1H-pyrrolo[2,3-b]pyridin-6-yl)-2-methylphenol (282);
3-(2-{6-[(3R)-3-Aminopiperidine-1-carbonyl]-4-fluoro-3-methylpyrazolo[1,5-a]pyridin-2-yl}-1-(cyclopropylmethyl)-1H-pyrrolo[2,3-b]pyridin-6-yl)-2-fluorophenol (283);
5-(2-{6-[(3R)-3-Aminopiperidine-1-carbonyl]-4-fluoro-3-methylpyrazolo[1,5-a]pyridin-2-yl}-1-(cyclopropylmethyl)-1H-pyrrolo[2,3-b]pyridin-6-yl)-2,3-dihydro-1H-isoindol-1-one (284);
6-(2-{6-[(3R)-3-Aminopiperidine-1-carbonyl]-4-fluoro-3-methylpyrazolo[1,5-a]pyridin-2-yl}-1-(cyclopropylmethyl)-1H-pyrrolo[2,3-b]pyridin-6-yl)-2,3-dihydro-1H-isoindol-1-one (285);
4-(2-{6-[(3R)-3-Aminopiperidine-1-carbonyl]-4-fluoro-3-methylpyrazolo[1,5-a]pyridin-2-yl}-1-(cyclopropylmethyl)-1H-pyrrolo[2,3-b]pyridin-6-yl)-2-methylbenzamide (286);
5-(2-{6-[(3R)-3-Aminopiperidine-1-carbonyl]-4-fluoro-3-methylpyrazolo[1,5-a]pyridin-2-yl }-1-(cyclopropylmethyl)-1H-pyrrolo[2,3-b]pyridin-6-yl)-N-methylpyridine-2-carboxamide (287);
(3R)-1-{2-[1-(Cyclopropylmethyl)-6-{3-methyl-[1,2,4]triazolo[4,3-a]pyridin-7-yl}-1H-pyrrolo[2,3-b]pyridin-2-yl]-4-fluoro-3-methylpyrazolo[1,5-a]pyridine-6-carbonyl }piperidin-3-amine (288);
(3R,5R)-1-{4-Chloro-2-[6-chloro-1-(cyclopropylmethyl)-1H-pyrrolo[2,3-b]pyridin-2-yl]-3-methylpyrazolo[1,5-a]pyridine-6-carbonyl}-5-fluoropiperidin-3-amine (289);
(3R,5R)-1-{4-Chloro-2-[1-(cyclopropylmethyl)-6-(1H-indazol-5-yl)-1H-pyrrolo[2,3-b]pyridin-2-yl]-3-methylpyrazolo[1,5-a]pyridine-6-carbonyl}-5-fluoropiperidin-3-amine (290);
(3R)-1-{4-Chloro-2-[6-chloro-1-(cyclopropylmethyl)-1H-pyrrolo[2,3-b]pyridin-2-yl]-3-methylpyrazolo[1,5-a]pyridine-6-carbonyl } piperidin-3-amine (291);
(3R)-1-{4-chloro-2-[1-(Cyclopropylmethyl)-6-(1H-indazol-5-yl)-1H-pyrrolo[2,3-b]pyridin-2-yl]-3-methylpyrazolo[1,5-a]pyridine-6-carbonyl}piperidin-3-amine (292);
5-(2-{6-[(7R)-7-Amino-2-azabicyclo[2.2.1]heptane-2-carbonyl]-4-fluoro-3-methylpyrazolo[1,5-a]pyridin-2-yl}-1-(cyclopropylmethyl)-1H-indol-6-yl)-2,3-dihydro-1H-isoindol-1-one (293);
4-(2-{6-[(7R)-7-Amino-2-azabicyclo[2.2.1]heptane-2-carbonyl]-4-fluoro-3-methylpyrazolo[1,5-a]pyridin-2-yl}-1-(cyclopropylmethyl)-1H-indol-6-yl)-3-chlorobenzamide (294);
(7R)-2-{2-[1-(Cyclopropylmethyl)-6-{3-methyl-[1,2,4]triazolo[4,3-a]pyridin-7-yl}-1H-indol-2-yl]-4-fluoro-3-methylpyrazolo[1,5-a]pyridine-6-carbonyl}-2-azabicyclo[2.2.1]heptan-7-amine (295);
(7R)-2-{2-[1-(Cyclopropylmethyl)-6-(3-methoxyazetidin-1-yl)-1H-indol-2-yl]-4-fluoro-3-methylpyrazolo[1,5-a]pyridine-6-carbonyl}-2-azabicyclo[2.2.1]heptan-7-amine (296);
(7R)-2-{2-[1-(Cyclopropylmethyl)-6-(3-fluoro-1H-indazol-4-yl)-1H-pyrrolo[2,3-b]pyridin-2-yl]-3-methylpyrazolo[1,5-a]pyridine-6-carbonyl}-2-azabicyclo[2.2.1]heptan-7-amine (297);
(7R)-2-{2-[1-(Cyclopropylmethyl)-6-(1H-indazol-6-yl)-1H-pyrrolo[2,3-b]pyridin-2-yl]-3-methylpyrazolo[1,5-a]pyridine-6-carbonyl}-2-azabicyclo[2.2.1]heptan-7-amine (298);
(7R)-2-{2-[1-(Cyclopropylmethyl)-6-(1H-indazol-4-yl)-1H-pyrrolo[2,3-b]pyridin-2-yl]-3-methylpyrazolo[1,5-a]pyridine-6-carbonyl}-2-azabicyclo[2.2.1]heptan-7-amine (299);
(7R)-2-{2-[1-(Cyclopropylmethyl)-6-(1H-indazol-5-yl)-1H-pyrrolo[2,3-b]pyridin-2-yl]-3-methylpyrazolo[1,5-a]pyridine-6-carbonyl}-2-azabicyclo[2.2.1]heptan-7-amine (300);
3-(2-{6-[(7R)-7-Amino-2-azabicyclo[2.2.1]heptane-2-carbonyl]-3-methylpyrazolo[1,5-a]pyridin-2-yl}-1-(cyclopropylmethyl)-1H-pyrrolo[2,3-b]pyridin-6-yl)-2-methylphenol (301);
1-[2-(2-{6-[(7R)-7-Amino-2-azabicyclo[2.2.1]heptane-2-carbonyl]-3-methylpyrazolo[1,5-a]pyridin-2-yl}-1-(cyclopropylmethyl)-1H-pyrrolo[2,3-b]pyridin-6-yl)phenyl]imidazolidin-2-one (302);
4-(2-{6-[(7R)-7-Amino-2-azabicyclo[2.2.1]heptane-2-carbonyl]-3-methylpyrazolo[1,5-a]pyridin-2-yl}-1-(cyclopropylmethyl)-1H-pyrrolo[2,3-b]pyridin-6-yl)-2-methoxybenzamide (303);
1-[4-(2-{6-[(7R)-7-Amino-2-azabicyclo[2.2.1]heptane-2-carbonyl]-3-methylpyrazolo[1,5-a]pyridin-2-yl}-1-(cyclopropylmethyl)-1H-pyrrolo[2,3-b]pyridin-6-yl)phenyl]pyrrolidin-2-one (304);
4-(2-{6-[(7R)-7-Amino-2-azabicyclo[2.2.1]heptane-2-carbonyl]-3-methylpyrazolo[1,5-a]pyridin-2-yl}-1-(cyclopropylmethyl)-1H-pyrrolo[2,3-b]pyridin-6-yl)-2-fluoro-5-methylbenzamide (305);
(7R)-2-{2-[1-(Cyclopropylmethyl)-6-(7-fluoro-1H-indazol-6-yl)-1H-pyrrolo[2,3-b]pyridin-2-yl]-3-methylpyrazolo[1,5-a]pyridine-6-carbonyl}-2-azabicyclo[2.2.1]heptan-7-amine (306);
5-(2-{6-[(7R)-7-Amino-2-azabicyclo[2.2.1]heptane-2-carbonyl]-3-methylpyrazolo[1,5-a]pyridin-2-yl}-1-(cyclopropylmethyl)-1H-pyrrolo[2,3-b]pyridin-6-yl)-N-methylpyridine-2-carboxamide (307);
Methyl N-[5-(2-{6-[(7R)-7-amino-2-azabicyclo[2.2.1]heptane-2-carbonyl]-3-methylpyrazolo[1,5-a]pyridin-2-yl}-1-(cyclopropylmethyl)-1H-pyrrolo[2,3-b]pyridin-6-yl)pyridin-2-yl]carbamate (308);
(3R)-1-{2-[1-(Cyclopropylmethyl)-6-(1H-indazol-5-yl)-1H-pyrrolo[2,3-b]pyridin-2-yl]-3-methylpyrazolo[1,5-a]pyridine-6-carbonyl}piperidin-3-amine (309);
(3R)-1-{2-[1-(Cyclopropylmethyl)-6-(1H-indazol-6-yl)-1H-pyrrolo[2,3-b]pyridin-2-yl]-3-methylpyrazolo[1,5-a]pyridine-6-carbonyl }piperidin-3-amine (310);
(3R)-1-{2-[1-(Cyclopropylmethyl)-6-(1H-indazol-4-yl)-1H-pyrrolo[2,3-b]pyridin-2-yl]-3-methylpyrazolo[1,5-a]pyridine-6-carbonyl}piperidin-3-amine (311);
6-(2-{6-[(3R)-3-Aminopiperidine-1-carbonyl]-3-methylpyrazolo[1,5-a]pyridin-2-yl}-1-(cyclopropylmethyl)-1H-pyrrolo[2,3-b]pyridin-6-yl)-8-fluoro-5-methyl-1,2-dihydroquinolin-2-one (312);
3-(2-{6-[(3R)-3-Aminopiperidine-1-carbonyl]-3-methylpyrazolo[1,5-a]pyridin-2-yl}-1-(cyclopropylmethyl)-1H-pyrrolo[2,3-b]pyridin-6-yl)-2-chlorophenol (313);
3-(2-{6-[(3R)-3-Aminopiperidine-1-carbonyl]-3-methylpyrazolo[1,5-a]pyridin-2-yl}-1-(cyclopropylmethyl)-1H-pyrrolo[2,3-b]pyridin-6-yl)-2-fluorophenol (314);
6-(2-{6-[(3R)-3-Aminopiperidine-1-carbonyl]-3-methylpyrazolo[1,5-a]pyridin-2-yl}-1-(cyclopropylmethyl)-1H-pyrrolo[2,3-b]pyridin-6-yl)-2,3-dihydro-1H-isoindol-1-one (315);
4-(2-{6-[(3R)-3-Aminopiperidine-1-carbonyl]-3-methylpyrazolo[1,5-a]pyridin-2-yl}-1-(cyclopropylmethyl)-1H-pyrrolo[2,3-b]pyridin-6-yl)-2-methylbenzamide (316);
5-(2-{6-[(3R)-3-Aminopiperidine-1-carbonyl]-3-methylpyrazolo[1,5-a]pyridin-2-yl}-1-(cyclopropylmethyl)-1H-pyrrolo[2,3-b]pyridin-6-yl)-2,3-dihydro-1H-isoindol-1-one (317);
(7R)-2-{2-[1-(Cyclopropylmethyl)-6-(morpholin-4-yl)-1H-pyrrolo[2,3-b]pyridin-2-yl]-3-methylpyrazolo[1,5-a]pyridine-6-carbonyl}-2-azabicyclo[2.2.1]heptan-7-amine (318);
4-(2-{6-[(7R)-7-Amino-2-azabicyclo[2.2.1]heptane-2-carbonyl]-3-methylpyrazolo[1,5-a]pyridin-2-yl}-1-(cyclopropylmethyl)-1H-pyrrolo[2,3-b]pyridin-6-yl)morpholin-3-one (319);
(7R)-2-{2-[1-(Cyclopropylmethyl)-6-(4-methanesulfonylpiperidin-1-yl)-1H-pyrrolo[2,3-b]pyridin-2-yl]-3-methylpyrazolo[1,5-a]pyridine-6-carbonyl } -2-azabicyclo[2.2.1]heptan-7-amine (320);
(3R,5R)-1-{2-[1-(Cyclopropylmethyl)-6-(1H-indazol-6-yl)-1H-pyrrolo[2,3-b]pyridin-2-yl]-3-methylpyrazolo[1,5-a]pyridine-6-carbonyl}-5-fluoropiperidin-3-amine (321);
(3R,5R)-1-{2-[1-(Cyclopropylmethyl)-6-(1H-indazol-4-yl)-1H-pyrrolo[2,3-b]pyridin-2-yl]-3-methylpyrazolo[1,5-a]pyridine-6-carbonyl}-5-fluoropiperidin-3-amine (322);
(3R,5R)-1-{2-[1-(Cyclopropylmethyl)-6-(1H-indazol-5-yl)-1H-pyrrolo[2,3-b]pyridin-2-yl]-3-methylpyrazolo[1,5-a]pyridine-6-carbonyl}-5-fluoropiperidin-3-amine (323);
6-(2-{6-[(3R,5R)-3-Amino-5-fluoropiperidine-1-carbonyl]-3-methylpyrazolo[1,5-a]pyridin-2-yl }-1-(cyclopropylmethyl)-1H-pyrrolo[2,3-b]pyridin-6-yl)-8-fluoro-5-methyl-1,2-dihydroquinolin-2-one (324);
Methyl N-[1-(2-{6-[(3R,5R)-3-amino-5-fluoropiperidine-1-carbonyl]-3-methylpyrazolo[1,5-a]pyridin-2-yl }-1-(cyclopropylmethyl)-1H-pyrrolo[2,3-b]pyridin-6-yl)piperidin-4-yl]-N-methylcarbamate (325);
(7R)-2-{2-[1-(Cyclopropylmethyl)-6-(7-fluoro-1H-indazol-4-yl)-1H-indol-2-yl]-3-methylpyrazolo[1,5-a]pyridine-6-carbonyl }-2-azabicyclo[2.2.1]heptan-7-amine (326);
(7R)-2-{2-[1-(Cyclopropylmethyl)-6-(3-fluoro-1H-indazol-4-yl)-1H-indol-2-yl]-3-methylpyrazolo[1,5-a]pyridine-6-carbonyl}-2-azabicyclo[2.2.1]heptan-7-amine (327);
1-[4-(2-{6-[(7R)-7-Amino-2-azabicyclo[2.2.1]heptane-2-carbonyl]-3-methylpyrazolo[1,5-a]pyridin-2-yl }-1-(cyclopropylmethyl)-1H-indol-6-yl)phenyl]imidazolidin-2-one (328);
1-[4-(2-{6-[(7R)-7-Amino-2-azabicyclo[2.2.1]heptane-2-carbonyl]-3-methylpyrazolo[1,5-a]pyridin-2-yl }-1-(cyclopropylmethyl)-1H-indol-6-yl)phenyl]pyrrolidin-2-one (329);
(7R)-2-{2-[1-(Cyclopropylmethyl)-6-(7-fluoro-1H-indazol-6-yl)-1H-indol-2-yl]-3-methylpyrazolo[1,5-a]pyridine-6-carbonyl}-2-azabicyclo[2.2.1]heptan-7-amine (330);
4-(2-{6-[(7R)-7-Amino-2-azabicyclo[2.2.1]heptane-2-carbonyl]-3-methylpyrazolo[1,5-a]pyridin-2-yl}-1-(cyclopropylmethyl)-1H-indol-6-yl)-5-fluoro-2-methylbenzamide (331);
(7R)-2-{2-[1-(Cyclopropylmethyl)-6-(1H-indazol-6-yl)-1H-indol-2-yl]-3-methylpyrazolo[1,5-a]pyridine-6-carbonyl }-2-azabicyclo[2.2.1]heptan-7-amine (332);
(7R)-2-{2-[1-(Cyclopropylmethyl)-6-(1H-indazol-5-yl)-1H-indol-2-yl]-3-methylpyrazolo[1,5-a]pyridine-6-carbonyl }-2-azabicyclo[2.2.1]heptan-7-amine (333);
4-(2-{6-[(7R)-7-Amino-2-azabicyclo[2.2.1]heptane-2-carbonyl]-3-methylpyrazolo[1,5-a]pyridin-2-yl}-1-(cyclopropylmethyl)-1H-indol-6-yl)-2-methylbenzamide (334);
4-(2-{6-[(7R)-7-Amino-2-azabicyclo[2.2.1]heptane-2-carbonyl]-3-methylpyrazolo[1,5-a]pyridin-2-yl}-1-(cyclopropylmethyl)-1H-indol-6-yl)-3-(trifluoromethyl)benzamide (335);
(7R)-2-{2-[1-(Cyclopropylmethyl)-6-(7-fluoro-1H-indazol-6-yl)-1H-indol-2-yl]-3-methylpyrazolo[1,5-a]pyridine-6-carbonyl}-2-azabicyclo[2.2.1]heptan-7-amine (336);
N-[7-(2-{6-[(7R)-7-Amino-2-azabicyclo[2.2.1]heptane-2-carbonyl]-3-methylpyrazolo[1,5-a]pyridin-2-yl}-1-(cyclopropylmethyl)-1H-indol-6-yl)-4-chloro-1-methyl-1H-indazol-3-yl]methanesulfonamide (337);
1-[2-(2-{6-[(7R)-7-Amino-2-azabicyclo[2.2.1]heptane-2-carbonyl]-3-methylpyrazolo[1,5-a]pyridin-2-yl}-1-(cyclopropylmethyl)-1H-indol-6-yl)phenyl]imidazolidin-2-one (338);
4-(2-{6-[(7R)-7-Amino-2-azabicyclo[2.2.1]heptane-2-carbonyl]-3-methylpyrazolo[1,5-a]pyridin-2-yl}-1-(cyclopropylmethyl)-1H-indol-6-yl)-2-chlorobenzamide (339);
5-(2-{6-[(7R)-7-Amino-2-azabicyclo[2.2.1]heptane-2-carbonyl]-3-methylpyrazolo[1,5-a]pyridin-2-yl}-1-(cyclopropylmethyl)-1H-indol-6-yl)-N-methylpyridine-2-carboxamide (340);
(7R)-2-{2-[1-(Cyclopropylmethyl)-6-(1H-pyrazol-4-yl)-1H-indol-2-yl]-3-methylpyrazolo[1,5-a]pyridine-6-carbonyl} -2-azabicyclo[2.2.1]heptan-7-amine (341);
N-{[4-(2-{6-[(3R)-3-Aminopiperidine-1-carbonyl]-3-methylpyrazolo[1,5-a]pyridin-2-yl}-1-(cyclopropylmethyl)-1H-indol-6-yl)phenyl]methyl}methanesulfonamide (342);
N-{[3-(2-{6-[(3R)-3-Aminopiperidine-1-carbonyl]-3-methylpyrazolo[1,5-a]pyridin-2-yl}-1-(cyclopropylmethyl)-1H-indol-6-yl)phenyl]methyl}acetamide (343);
[3-(2-{6-[(3R)-3-Aminopiperidine-1-carbonyl]-3-methylpyrazolo[1,5-a]pyridin-2-yl}-1-(cyclopropylmethyl)-1H-indol-6-yl)phenyl]methanol (344);
(3R)-1-{2-[1'-(Cyclopropylmethyl)-1H,1'H-[5,6'-biindole]-2'-yl]-3-methylpyrazolo[1,5-a]pyridine-6-carbonyl}piperidin-3-amine (345);
6-(2-{6-[(3R)-3-Aminopiperidine-1-carbonyl]-3-methylpyrazolo[1,5-a]pyridin-2-yl }-1-(cyclopropylmethyl)-1H-indol-6-yl)-8-fluoro-5-methyl-1,2-dihydroquinolin-2-one (346);
N-[4-(2-{6-[(3R)-3-Aminopiperidine-1-carbonyl]-3-methylpyrazolo[1,5-a]pyridin-2-yl}-1-(cyclopropylmethyl)-1H-indol-6-yl)phenyl]methanesulfonamide (347);
N-[5-(2-{6-[(3R)-3-Aminopiperidine-1-carbonyl]-3-methylpyrazolo[1,5-a]pyridin-2-yl}-1-(cyclopropylmethyl)-1H-indol-6-yl)pyridin-2-yl]acetamide (348);
(R)-(3-Aminopiperidin-1-yl)(2-(1-(cyclopropylmethyl)-6-(3-methyl-[1,2,4]triazolo[4,3-a]pyridin-7-yl)-1H-indol-2-yl)-3-methylpyrazolo[1,5-a]pyridin-6-yl)methanone (349);
(7R)-2-{2-[1-(Cyclopropylmethyl)-6-(morpholin-4-yl)-1H-indol-2-yl]-3-methylpyrazolo[1,5-a]pyridine-6-carbonyl} -2-azabicyclo[2.2.1]heptan-7-amine (350);
(7R)-2-{2-[1-(Cyclopropylmethyl)-6-(4-methanesulfonylpiperidin-1-yl)-1H-indol-2-yl]-3-methylpyrazolo[1,5-a]pyridine-6-carbonyl}-2-azabicyclo[2.2.1]heptan-7-amine (351);
Methyl N-[1-(2-{6-[(3R,5R)-3-amino-5-fluoropiperidine-1-carbonyl]-3-methylpyrazolo[1,5-a]pyridin-2-yl }-1-(cyclopropylmethyl)-1H-indol-6-yl)piperidin-4-yl]-N-methylcarbamate (352);
N-[1-(2-{6-[(3R,5R)-3-Amino-5-fluoropiperidine-1-carbonyl]-3-methylpyrazolo[1,5-a]pyridin-2-yl}-1-(cyclopropylmethyl)-1H-indol-6-yl)piperidin-4-yl]-N-methylacetamide (353);
N-[1-(2-{6-[(3R,5R)-3-Amino-5-fluoropiperidine-1-carbonyl]-3-methylpyrazolo[1,5-a]pyridin-2-yl}-1-(cyclopropylmethyl)-1H-indol-6-yl)piperidin-4-yl]-N-methylmethanesulfonamide (354);
(3R,5R)-1-{2-[1-(Cyclopropylmethyl)-6-[4-(pyrrolidine-1-carbonyl)piperidin-1-yl]-1H-indol-2-yl]-3-methylpyrazolo[1,5-a]pyridine-6-carbonyl}-5-fluoropiperidin-3-amine (355);
1-[1-(2-{6-[(3R,5R)-3-Amino-5-fluoropiperidine-1-carbonyl]-3-methylpyrazolo[1,5-a]pyridin-2-yl }-1-(cyclopropylmethyl)-1H-indol-6-yl)piperidin-4-yl]pyrrolidin-2-one (356);
3-[1-(2-{6-[(3R,5R)-3-Amino-5-fluoropiperidine-1-carbonyl]-3-methylpyrazolo[1,5-a]pyridin-2-yl}-1-(cyclopropylmethyl)-1H-indol-6-yl)piperidin-4-yl]-1,3-oxazolidin-2-one (357);
Methyl N-[1-(2-{6-[(3R)-3-aminopiperidine-1-carbonyl]-3-methylpyrazolo[1,5-a]pyridin-2-yl}-1-(cyclopropylmethyl)-1H-indol-6-yl)piperidin-4-yl]-N-methylcarbamate (358);
N-[1-(2-{6-[(3R)-3-Aminopiperidine-1-carbonyl]-3-methylpyrazolo[1,5-a]pyridin-2-yl }-1-(cyclopropylmethyl)-1H-indol-6-yl)piperidin-4-yl]-N-methylacetamide (359);
N-[1-(2-{6-[(3R)-3-Aminopiperidine-1-carbonyl]-3-methylpyrazolo[1,5-a]pyridin-2-yl}-1-(cyclopropylmethyl)-1H-indol-6-yl)piperidin-4-yl]-N-methylmethanesulfonamide (360);
(3R)-1-{2-[1-(Cyclopropylmethyl)-6-[4-(pyrrolidine-1-carbonyl)piperidin-1-yl]-1H-indol-2-yl]-3-methylpyrazolo[1,5-a]pyridine-6-carbonyl}piperidin-3-amine (361);
N-[1-(2-{6-[(3R)-3-Aminopiperidine-1-carbonyl]-3-methylpyrazolo[1,5-a]pyridin-2-yl}-1-(cyclopropylmethyl)-1H-indol-6-yl)piperidin-4-yl]-N-methylmethanesulfonamide (362);
1-[1-(2-{6-[(3R)-3-Aminopiperidine-1-carbonyl]-3-methylpyrazolo[1,5-a]pyridin-2-yl}-1-(cyclopropylmethyl)-1H-indol-6-yl)piperidin-4-yl]pyrrolidin-2-one (363);
3-[1-(2-{6-[(3R)-3-Aminopiperidine-1-carbonyl]-3-methylpyrazolo[1,5-a]pyridin-2-yl}-1-(cyclopropylmethyl)-1H-indol-6-yl)piperidin-4-yl]-1,3-oxazolidin-2-one (364);
(3R)-1-{2-[1-(Cyclopropylmethyl)-6-[4-(1H-pyrazol-1-yl)piperidin-1-yl]-1H-indol-2-yl]-3-methylpyrazolo[1,5-a]pyridine-6-carbonyl}piperidin-3-amine (365);
N-[1-(2-{6-[(3R)-3-Aminopiperidine-1-carbonyl]-3-methylpyrazolo[1,5-a]pyridin-2-yl}-1-(cyclopropylmethyl)-1H-indol-6-yl)azetidin-3-yl]-N-methylmethanesulfonamide (366);
N-{[3-(2-{6-[(3R,5R)-3-Amino-5-fluoropiperidine-1-carbonyl]-3-methylpyrazolo[1,5-a]pyridin-2-yl }-1-(cyclopropylmethyl)-1H-indol-6-yl)phenyl]methyl}methanesulfonamide (367);
(3R,5R)-1-{2-[1-(Cyclopropylmethyl)-6-[3-(2H-1,2,3,4-tetrazol-5-yl)phenyl]-1H-indol-2-yl]-3-methylpyrazolo[1,5-a]pyridine-6-carbonyl}-5-fluoropiperidin-3-amine (368);
N-[4-(2-{ 6-[(3R, SR)-3-Amino-5-fluoropiperidine-1-carbonyl]-3-methylpyrazolo[1,5-a]pyridin-2-yl}-1-(cyclopropylmethyl)-1H-indol-6-yl)phenyl]methanesulfonamide (369);
N-{[4-(2-{6-[(3R,5R)-3-Amino-5-fluoropiperidine-1-carbonyl]-3-methylpyrazolo[1,5-a]pyridin-2-yl}-1-(cyclopropylmethyl)-1H-indol-6-yl)phenyl]methyl}methanesulfonamide (370);
[3-(2-{6-[(3R,5R)-3-Amino-5-fluoropiperidine-1-carbonyl]-3-methylpyrazolo[1,5-a]pyridin-2-yl}-1-(cyclopropylmethyl)-1H-indol-6-yl)phenyl]methanol (371);
2-(2-{6-[(3R,5R)-3-Amino-5-fluoropiperidine-1-carbonyl]-3-methylpyrazolo[1,5-a]pyridin-2-yl}-1-(cyclopropylmethyl)-1H-indol-6-yl)benzamide (372);
4-(2-{6-[(3R,5R)-3-Amino-5-fluoropiperidine-1-carbonyl]-3-methylpyrazolo[1,5-a]pyridin-2-yl}-1-(cyclopropylmethyl)-1H-indol-6-yl)benzamide (373);
(3R,5R)-1-{2-[1'-(Cyclopropylmethyl)-1H,1'H-[5,6'-biindole]-2'-yl]-3-methylpyrazolo[1,5-a]pyridine-6-carbonyl} -5-fluoropiperidin-3-amine (374);
[4-(2-{6-[(3R,5R)-3-Amino-5-fluoropiperidine-1-carbonyl]-3-methylpyrazolo[1,5-a]pyridin-2-yl}-1-(cyclopropylmethyl)-1H-indol-6-yl)-2-chloro-5-fluorophenyl]methanol (375);
6-(2-{6-[(3R,5R)-3-Amino-5-fluoropiperidine-1-carbonyl]-3-methylpyrazolo[1,5-a]pyridin-2-yl}-1-(cyclopropylmethyl)-1H-indol-6-yl)-8-fluoro-5-methyl-1,2-dihydroquinolin-2-one (376);
4-(2-{6-[(3R,5R)-3-Amino-5-fluoropiperidine-1-carbonyl]-3-methylpyrazolo[1,5-a]pyridin-2-yl}-1-(cyclopropylmethyl)-1H-indol-6-yl)-3-chlorobenzamide (377);
4-(2-{6-[(3R,5R)-3-Amino-5-fluoropiperidine-1-carbonyl]-3-methylpyrazolo[1,5-a]pyridin-2-yl}-1-(cyclopropylmethyl)-1H-indol-6-yl)-2-methylbenzamide (378);
(3R,5R)-1-{2-[1-(Cyclopropylmethyl)-6-{3-methyl-[1,2,4]triazolo[4,3-a]pyridin-7-yl}-1H-indol-2-yl]-3-methylpyrazolo[1,5-a]pyridine-6-carbonyl}-5-fluoropiperidin-3-amine (379);
2-(2-{6-[(3R)-3-Aminopiperidine-1-carbonyl]-3-methylpyrazolo[1,5-a]pyridin-2-yl}-1-(cyclopropylmethyl)-1H-pyrrolo[2,3-b]pyridin-6-yl)propan-2-ol (Homochiral) (380);
2-(2-{6-[(7R)-7-Amino-2-azabicyclo[2.2.1]heptane-2-carbonyl]-3-methylpyrazolo[1,5-a]pyridin-2-yl}-1-(cyclopropylmethyl)-1H-pyrrolo[2,3-b]pyridin-6-yl)-1,1-difluoropropan-2-ol (Diastereomeric mixture) (381);
2-(2-{6-[(3R)-3-Aminopiperidine-1-carbonyl]-3-methylpyrazolo[1,5-a]pyridin-2-yl}-1-(cyclopropylmethyl)-1H-pyrrolo[2,3-b]pyridin-6-yl)-1,1,1-trifluoropropan-2-ol (Diastereomeric mixture) (382);
2-(2-{6-[(7R)-7-Amino-2-azabicyclo[2.2.1]heptane-2-carbonyl]-4-fluoro-3-methylpyrazolo[1,5-a]pyridin-2-yl}-1-(cyclopropylmethyl)-1H-pyrrolo[2,3-b]pyridin-6-yl)-1,1,1-trifluoropropan-2-ol (383);
1-(2-{6-[(7R)-7-Amino-2-azabicyclo[2.2.1]heptane-2-carbonyl]-4-fluoro-3-methylpyrazolo[1,5-a]pyridin-2-yl}-1-(cyclopropylmethyl)-1H-pyrrolo[2,3-b]pyridin-6-yl)-1-cyclopropylethan-1-ol (384);
1-(2-{6-[(3R)-3-Aminopiperidine-1-carbonyl]-3-methylpyrazolo[1,5-a]pyridin-2-yl}-1-(cyclopropylmethyl)-1H-pyrrolo[2,3-b]pyridin-6-yl)ethan-1-ol (Diastereomeric mixture) (385);
((R)-3-Aminopiperidin-1-yl)(2-(1-(cyclopropylmethyl)-7-(1-(tetrahydro-2H-pyran-2-carbonyl)piperidin-4-yl)-1H-indol-2-yl)-4-methoxy-3-methylpyrazolo[1,5-a]pyridin-6-yl)methanone (386);
((R)-3-Aminopiperidin-1-yl)(2-(1-(cyclopropylmethyl)-7-(1-(tetrahydro-2H-pyran-2-carbonyl)piperidin-4-yl)-1H-indol-2-yl)-4-methoxy-3-methylpyrazolo[1,5-a]pyridin-6-yl)methanone (387);
((3R,5R)-3-Amino-5-fluoropiperidin-1-yl)(2-(1-(cyclopropylmethyl)-7-(1-(tetrahydro-2H-pyran-2-carbonyl)piperidin-4-yl)-1H-indol-2-yl)-4-methoxy-3-methylpyrazolo[1,5-a]pyridin-6-yl)methanone (388);
((3R,5R)-3-Amino-5-fluoropiperidin-1-yl)(2-(1-(cyclopropylmethyl)-7-(1-(tetrahydro-2H-pyran-2-carbonyl)piperidin-4-yl)-1H-indol-2-yl)-4-methoxy-3-methylpyrazolo[1,5-a]pyridin-6-yl)methanone (389);
((3R,5R)-3-Amino-5-fluoropiperidin-1-yl)(2-(1-(cyclopropylmethyl)-7-(1-(3-hydroxycyclobutane-1-carbonyl)piperidin-4-yl)-1H-indol-2-yl)-4-methoxy-3-methylpyrazolo[1,5-a]pyridin-6-yl)methanone (390);
((3R,5R)-3-Amino-5-fluoropiperidin-1-yl)(2-(1-(cyclopropylmethyl)-7-(1-((1r,4r)-4-hydroxycyclohexane-1-carbonyl)piperidin-4-yl)-1H-indol-2-yl)-4-methoxy-3-methylpyrazolo[1,5-a]pyridin-6-yl)methanone (391);
(R)-(3-Aminopiperidin-1-yl)(2-(1-(cyclopropylmethyl)-7-(1-(3-hydroxycyclobutane-1-carbonyl)piperidin-4-yl)-1H-indol-2-yl)-4-methoxy-3-methylpyrazolo[1,5-a]pyridin-6-yl)methanone (392);
((3R,5R)-3-Amino-5-fluoropiperidin-1-yl)(2-(1-(cyclopropylmethyl)-7-(1-(3-hydroxycyclobutane-1-carbonyl)piperidin-4-yl)-1H-indol-2-yl)-4-methoxy-3-methylpyrazolo[1,5-a]pyridin-6-yl)methanone (393);
((3R,5R)-3-Amino-5-fluoropiperidin-1-yl)(2-(1-(cyclopropylmethyl)-7-(1-(tetrahydrofuran-2-carbonyl)piperidin-4-yl)-1H-indol-2-yl)-4-methoxy-3-methylpyrazolo[1,5-a]pyridin-6-yl)methanone (394);
(R)-1-(4-(2-(6-((3R,5R)-3-Amino-5-fluoropiperidine-1-carbonyl)-4-methoxy-3-methylpyrazolo[1,5-a]pyridin-2-yl)-1-(cyclopropylmethyl)-1H-indol-7-yl)piperidin-1-yl)-2-methoxypropan-1-one (395);
(R)-(3-Aminopiperidin-1-yl)(2-(1-(cyclopropylmethyl)-7-(1-(3-hydroxycyclobutane-1-carbonyl)piperidin-4-yl)-1H-indol-2-yl)-4-methoxy-3-methylpyrazolo[1,5-a]pyridin-6-yl)methanone (396);
((3R,5R)-3-Amino-5-fluoropiperidin-1-yl)(2-(1-(cyclopropylmethyl)-7-(1-(tetrahydrofuran-2-carbonyl)piperidin-4-yl)-1H-indol-2-yl)-4-methoxy-3-methylpyrazolo[1,5-a]pyridin-6-yl)methanone (397);
(R)-(3-Aminopiperidin-1-yl)(2-(1-(cyclopropylmethyl)-7-(1-(3-hydroxy-3-(trifluoromethyl)cyclobutane-1-carbonyl)piperidin-4-yl)-1H-indol-2-yl)-4-methoxy-3-methylpyrazolo[1,5-a]pyridin-6-yl)methanone (398);
((3R,5R)-3-Amino-5-fluoropiperidin-1-yl)(2-(1-(cyclopropylmethyl)-7-(1-(3-hydroxy-3-(trifluoromethyl)cyclobutane-1-carbonyl)piperidin-4-yl)-1H-indol-2-yl)-4-methoxy-3-methylpyrazolo[1,5-a]pyridin-6-yl)methanone (399);
((R)-3-Aminopiperidin-1-yl)(2-(1-(cyclopropylmethyl)-7-(1-(tetrahydrofuran-2-carbonyl)piperidin-4-yl)-1H-indol-2-yl)-4-methoxy-3-methylpyrazolo[1,5-a]pyridin-6-yl)methanone (400);
(R)-1-(4-(2-(6-(3-Aminopiperidine-1-carbonyl)-4-methoxy-3-methylpyrazolo[1,5-a]pyridin-2-yl)-1-(cyclopropylmethyl)-1H-indol-7-yl)piperidin-1-yl)-2-methoxyethan-1-one (401);
(R)-1-(4-(2-(6-((R)-3-Aminopiperidine-1-carbonyl)-4-methoxy-3-methylpyrazolo[1,5-a]pyridin-2-yl)-1-(cyclopropylmethyl)-1H-indol-7-yl)piperidin-1-yl)-2-methoxypropan-1-one (402);
(S)-1-(4-(2-(6-((R)-3-Aminopiperidine-1-carbonyl)-4-methoxy-3-methylpyrazolo[1,5-a]pyridin-2-yl)-1-(cyclopropylmethyl)-1H-indol-7-yl)piperidin-1-yl)-2-methoxypropan-1-one (403);
(S)-1-(4-(2-(6-((3R,5R)-3-Amino-5-fluoropiperidine-1-carbonyl)-4-methoxy-3-methylpyrazolo[1,5-a]pyridin-2-yl)-1-(cyclopropylmethyl)-1H-indol-7-yl)piperidin-1-yl)-2-methoxypropan-1-one (404);
1-(4-(2-(6-((3R,5R)-3-Amino-5-fluoropiperidine-1-carbonyl)-4-methoxy-3-methylpyrazolo[1,5-a]pyridin-2-yl)-1-(cyclopropylmethyl)-1H-indol-7-yl)piperidin-1-yl)-2-methoxyethan-1-one (405);
((R)-3-Aminopiperidin-1-yl)(2-(1-(cyclopropylmethyl)-7-(1-((1s,4s)-4-hydroxycyclohexane-1-carbonyl)piperidin-4-yl)-1H-indol-2-yl)-4-methoxy-3-methylpyrazolo[1,5-a]pyridin-6-yl)methanone (406);
((3R,5R)-3-Amino-5-fluoropiperidin-1-yl)(2-(1-(cyclopropylmethyl)-7-(1-((1s,4s)-4-hydroxycyclohexane-1-carbonyl)piperidin-4-yl)-1H-indol-2-yl)-4-methoxy-3-methylpyrazolo[1,5-a]pyridin-6-yl)methanone (407);
((R)-3-Aminopiperidin-1-yl)(2-(1-(cyclopropylmethyl)-7-(1-((1r,4r)-4-hydroxycyclohexane-1-carbonyl)piperidin-4-yl)-1H-indol-2-yl)-4-methoxy-3-methylpyrazolo[1,5-a]pyridin-6-yl)methanone (408);
((3R,5R)-3-Amino-5-fluoropiperidin-1-yl)(2-(1-(cyclopropylmethyl)-7-(1-((1r,4r)-4-hydroxycyclohexane-1-carbonyl)piperidin-4-yl)-1H-indol-2-yl)-4-methoxy-3-methylpyrazolo[1,5-a]pyridin-6-yl)methanone (409);
(R)-1-(4-(2-(6-(3-Aminopiperidine-1-carbonyl)-4-methoxy-3-methylpyrazolo[1,5-a]pyridin-2-yl)-1-(cyclopropylmethyl)-1H-indol-7-yl)piperidin-1-yl)ethan-1-one (410);
1-(4-(2-(6-((3R,5R)-3-Amino-5-fluoropiperidine-1-carbonyl)-4-methoxy-3-methylpyrazolo[1,5-a]pyridin-2-yl)-1-(cyclopropylmethyl)-1H-indol-7-yl)piperidin-1-yl)ethan-1-one (411);
1-(4-(2-(6-((7R)-7-Amino-2-azabicyclo[2.2.1]heptane-2-carbonyl)-4-methoxy-3-methylpyrazolo[1,5-a]pyridin-2-yl)-1-(cyclopropylmethyl)-1H-indol-7-yl)piperidin-1-yl)ethan-1-one (412);
(R)-1-(4-(2-(6-(3-Aminopiperidine-1-carbonyl)-4-fluoro-3-methylpyrazolo[1,5-a]pyridin-2-yl)-1-(cyclopropylmethyl)-1H-indol-7-yl)piperidin-1-yl)ethan-1-one (413);
((3R,5R)-3-Amino-5-fluoropiperidin-1-yl)(2-(1-(cyclopropylmethyl)-7-(1-((1r,4r)-4-hydroxycyclohexane-1-carbonyl)piperidin-4-yl)-1H-indol-2-yl)-4-fluoro-3-methylpyrazolo[1,5-a]pyridin-6-yl)methanone (414);
((R)-3-Aminopiperidin-1-yl)(2-(1-(cyclopropylmethyl)-7-(1-((1r,4r)-4-hydroxycyclohexane-1-carbonyl)piperidin-4-yl)-1H-indol-2-yl)-4-fluoro-3-methylpyrazolo[1,5-a]pyridin-6-yl)methanone (415);
((3R,5R)-3-Amino-5-fluoropiperidin-1-yl)(2-(1-(cyclopropylmethyl)-7-(1-((1s,4s)-4-hydroxycyclohexane-1-carbonyl)piperidin-4-yl)-1H-indol-2-yl)-4-fluoro-3-methylpyrazolo[1,5-a]pyridin-6-yl)methanone (416);
1-(4-(2-(6-((3R,5R)-3-Amino-5-fluoropiperidine-1-carbonyl )-4-fluoro-3-methylpyrazolo[1,5-a]pyridin-2-yl)-1-(cyclopropylmethyl)-1H-indol-7-yl)piperidin-1-yl)-2-methoxy-2-methylpropan-1-one (417);
(R)-1-(4-(2-(6-(3-Aminopiperidine-1-carbonyl)-4-fluoro-3-methylpyrazolo[1,5-a]pyridin-2-yl)-1-(cyclopropylmethyl)-1H-indol-7-yl)piperidin-1-yl)-2-methoxy-2-methylpropan-1-one (418);
((3R,5R)-3-Amino-5-fluoropiperidin-1-yl)(2-(1-(cyclopropylmethyl)-7-(1-(3-hydroxycyclobutane-1-carbonyl)piperidin-4-yl)-1H-indol-2-yl)-4-fluoro-3-methylpyrazolo[1,5-a]pyridin-6-yl)methanone (419);
(R)-(3-Aminopiperidin-1-yl)(2-(1-(cyclopropylmethyl)-7-(1-(3-hydroxycyclobutane-1-carbonyl)piperidin-4-yl)-1H-indol-2-yl)-4-fluoro-3-methylpyrazolo[1,5-a]pyridin-6-yl)methanone (420);
((3R,5R)-3-Amino-5-fluoropiperidin-1-yl)(2-(1-(cyclopropylmethyl)-7-(1-(3-hydroxy-3-(trifluoromethyl)cyclobutane-1-carbonyl)piperidin-4-yl)-1H-indol-2-yl)-4-fluoro-3-methylpyrazolo[1,5-a]pyridin-6-yl)methanone (421);
(R)-(3-Aminopiperidin-1-yl)(2-(1-(cyclopropylmethyl)-7-(1-(3-hydroxy-3-(trifluoromethyl)cyclobutane-1-carbonyl)piperidin-4-yl)-1H-indol-2-yl)-4-fluoro-3-methylpyrazolo[1,5-a]pyridin-6-yl)methanone (422);
(S)-1-(4-(2-(6-((3R,5R)-3-Amino-5-fluoropiperidine-1-carbonyl)-4-fluoro-3-methylpyrazolo[1,5-a]pyridin-2-yl)-1-(cyclopropylmethyl)-1H-indol-7-yl)piperidin-1-yl)-2-methoxypropan-1-one (423);
(S)-1-(4-(2-(6-((R)-3-Aminopiperidine-1-carbonyl)-4-fluoro-3-methylpyrazolo[1,5-a]pyridin-2-yl)-1-(cyclopropylmethyl)-1H-indol-7-yl)piperidin-1-yl)-2-methoxypropan-1-one (424);
(R)-1-(4-(2-(6-((3R,5R)-3-Amino-5-fluoropiperidine-1-carbonyl)-4-fluoro-3-methylpyrazolo[1,5-a]pyridin-2-yl)-1-(cyclopropylmethyl)-1H-indol-7-yl)piperidin-1-yl)-2-methoxypropan-1-one (425);
(R)-1-(4-(2-(6-((R)-3-Aminopiperidine-1-carbonyl)-4-fluoro-3-methylpyrazolo[1,5-a]pyridin-2-yl)-1-(cyclopropylmethyl)-1H-indol-7-yl)piperidin-1-yl)-2-methoxypropan-1-one (426);
1-(4-(2-(6-((3R,5R)-3-Amino-5-fluoropiperidine-1-carbonyl)-4-fluoro-3-methylpyrazolo[1,5-a]pyridin-2-yl)-1-(cyclopropylmethyl)-1H-indol-7-yl)piperidin-1-yl)ethan-1-one (427);
1-(4-(2-(6-((3R,5R)-3-Amino-5-fluoropiperidine-1-carbonyl)-4-fluoro-3-methylpyrazolo[1,5-a]pyridin-2-yl)-1-(cyclopropylmethyl)-1H-indol-7-yl)piperidin-1-yl)-2-methoxyethan-1-one (428);
(R)-1-(4-(2-(6-(3-Aminopiperidine-1-carbonyl)-4-fluoro-3-methylpyrazolo[1,5-a]pyridin-2-yl)-1-(cyclopropylmethyl)-1H-indol-7-yl)piperidin-1-yl)-2-methoxyethan-1-one (429);
Ethyl 2-(7-(1-(tert-butoxycarbonyl)piperidin-4-yl)-1-(cyclopropylmethyl)-1H-indol-2-yl)-3-methylpyrazolo[1,5-a]pyridine-6-carboxylate (430);
1-(4-(2-(6-((3R,5R)-3-amino-5-fluoropiperidine-1-carbonyl)-3-methylpyrazolo[1,5-a]pyridin-2-yl)-1-(cyclopropylmethyl)-1H-indol-7-yl)piperidin-1-yl)-3-hydroxypropan-1-one (430);
1-(4-(2-(6-((3R,5R)-3-amino-5-fluoropiperidine-1-carbonyl)-3-methylpyrazolo[1,5-a]pyridin-2-yl)-1-(cyclopropylmethyl)-1H-indol-7-yl)piperidin-1-yl)-2-isopropoxyethan-1-one (431);
(R)-1-(4-(2-(6-(3-aminopiperidine-1-carbonyl)-3-methylpyrazolo[1,5-a]pyridin-2-yl)-1-(cyclopropylmethyl)-1H-indol-7-yl)piperidin-1-yl)-2-isopropoxyethan-1-one (432);
1-(4-(2-(6-((3R,SR)-3-amino-5-fluoropiperidine-1-carbonyl)-3-methylpyrazolo[1,5-a]pyridin-2-yl)-1-(cyclopropylmethyl)-1H-indol-7-yl)piperidin-1-yl)-3-hydroxy-2,2-dimethylpropan-1-one (433);
(R)-1-(4-(2-(6-(3-aminopiperidine-1-carbonyl)-3-methylpyrazolo[1,5-a]pyridin-2-yl)-1-(cyclopropylmethyl)-1H-indol-7-yl)piperidin-1-yl)-3-hydroxy-2,2-dimethylpropan-1-one (434);
1-(4-(2-(6-((3R,5R)-3-amino-5-fluoropiperidine-1-carbonyl)-3-methylpyrazolo[1,5-a]pyridin-2-yl)-1-(cyclopropylmethyl)-1H-indol-7-yl)piperidin-1-yl)-2-hydroxy-2-methylpropan-1-one (435);
(R)-1-(4-(2-(6-(3-aminopiperidine-1-carbonyl)-3-methylpyrazolo[1,5-a]pyridin-2-yl)-1-(cyclopropylmethyl)-1H-indol-7-yl)piperidin-1-yl)-2-hydroxy-2-methylpropan-1-one (436);
(R)-1-(4-(2-(6-(3-aminopiperidine-1-carbonyl)-3-methylpyrazolo[1,5-a]pyridin-2-yl)-1-(cyclopropylmethyl)-1H-indol-7-yl)piperidin-1-yl)-3-hydroxypropan-1-one (437);
1-(4-(2-(6-((3R,5R)-3-amino-5-fluoropiperidine-1-carbonyl)-3-methylpyrazolo[1,5-a]pyridin-2-yl)-1-(cyclopropylmethyl)-1H-indol-7-yl)piperidin-1-yl)-2-hydroxyethan-1-one (438);
(R)-1-(4-(2-(6-(3-aminopiperidine-1-carbonyl)-3-methylpyrazolo[1,5-a]pyridin-2-yl)-1-(cyclopropylmethyl)-1H-indol-7-yl)piperidin-1-yl)-2-hydroxyethan-1-one (439);
((R)-3-aminopiperidin-1-yl)(2-(1-(cyclopropylmethyl)-7-(1-((1R,3S)-3-hydroxycyclohexane-1-carbonyl)piperidin-4-yl)-1H-indol-2-yl)-3-methylpyrazolo[ 1,5-a]pyridin-6-yl)methanone (440);
(R)-1-(4-(2-(6-(3-aminopiperidine-1-carbonyl)-3-methylpyrazolo[1,5-a]pyridin-2-yl)-1-(cyclopropylmethyl)-1H-indol-7-yl)piperidin-1-yl)-2-ethoxyethan-1-one (441);
1-(4-(2-(6-((3R,5R)-3-amino-5-fluoropiperidine-1-carbonyl)-3-methylpyrazolo[1,5-a]pyridin-2-yl)-1-(cyclopropylmethyl)-1H-indol-7-yl)piperidin-1-yl)-2-ethoxyethan-1-one (442);
((R)-3-aminopiperidin-1-yl)(2-(1-(cyclopropylmethyl)-7-(1-((1r,4r)-4-hydroxycyclohexane-1-carbonyl)piperidin-4-yl)-1H-indol-2-yl)-3-methylpyrazolo[1,5-a]pyridin-6-yl)methanone (443);
((3R,5R)-3-amino-5-fluoropiperidin-1-yl)(2-(1-(cyclopropylmethyl)-7-(1-((1s,4s)-4-hydroxycyclohexane-1-carbonyl)piperidin-4-yl)-1H-indol-2-yl)-3-methylpyrazolo[1,5-a]pyridin-6-yl)methanone (444);
1-(4-(2-(6-((3R,5R)-3-amino-5-fluoropiperidine-1-carbonyl)-3-methylpyrazolo[1,5-a]pyridin-2-yl)-1-(cyclopropylmethyl)-1H-indol-7-yl)piperidin-1-yl)-3-methylbutan-1-one (445);
(R)-1-(4-(2-(6-(3-aminopiperidine-1-carbonyl)-3-methylpyrazolo[1,5-a]pyridin-2-yl)-1-(cyclopropylmethyl)-1H-indol-7-yl)piperidin-1-yl)-3-methylbutan-1-one (446);
((R)-3-aminopiperidin-1-yl)(2-(1-(cyclopropylmethyl)-7-(1-((1S,3R)-3-hydroxycyclohexane-1-carbonyl)piperidin-4-yl)-1H-indol-2-yl)-3-methylpyrazolo[1,5-a]pyridin-6-yl)methanone (447);
((3R,5R)-3-amino-5-fluoropiperidin-1-yl)(2-(1-(cyclopropylmethyl)-7-(1-((1S,3R)-3-hydroxycyclohexane-1-carbonyl)piperidin-4-yl)-1H-indol-2-yl)-3-methylpyrazolo[1,5-a]pyridin-6-yl)methanone (448);
((3R,5R)-3-amino-5-fluoropiperidin-1-yl)(2-(1-(cyclopropylmethyl)-7-(1-((1R,3 S)-3-hydroxycyclohexane-1-carbonyl)piperidin-4-yl)-1H-indol-2-yl)-3-methylpyrazolo[1,5-a]pyridin-6-yl)methanone (449);
((R)-3-aminopiperidin-1-yl)(2-(1-(cyclopropylmethyl)-7-(1-((1s,4s)-4-hydroxycyclohexane-1-carbonyl)piperidin-4-yl)-1H-indol-2-yl)-3-methylpyrazolo[1,5-a]pyridin-6-yl)methanone (450);
((3R,5R)-3-amino-5-fluoropiperidin-1-yl)(2-(1-(cyclopropylmethyl)-7-(1-((1r,4r)-4-hydroxycyclohexane-1-carbonyl)piperidin-4-yl)-1H-indol-2-yl)-3-methylpyrazolo[1,5-a]pyridin-6-yl)methanone (451);
1-(4-(2-(6-((3R,5R)-3-amino-5-fluoropiperidine-1-carbonyl)-3-methylpyrazolo[1,5-a]pyridin-2-yl)-1-(cyclopropylmethyl)-1H-indol-7-yl)piperidin-1-yl)ethan-1-one (452);
1-(4-(2-(6-((7R)-7-amino-2-azabicyclo[2.2.1]heptane-2-carbonyl)-3-methylpyrazolo[1,5-a]pyridin-2-yl)-1-(cyclopropylmethyl)-1H-indol-7-yl)piperidin-1-yl)-2-methoxyethan-1-one (453);
(S)-1-(4-(2-(6-((R)-3-aminopiperidine-1-carbonyl)-3-methylpyrazolo[1,5-a]pyridin-2-yl)-1-(cyclopropylmethyl)-1H-indol-7-yl)piperidin-1-yl)-2-methoxypropan-1-one (454);
(R)-1-(4-(2-(6-(3-aminopiperidine-1-carbonyl)-3-methylpyrazolo[1,5-a]pyridin-2-yl)-1-(cyclopropylmethyl)-1H-indol-7-yl)piperidin-1-yl)-2-methoxyethan-1-one (455);
(R)-(3-aminopiperidin-1-yl)(2-(1-(cyclopropylmethyl)-7-(1-(3-hydroxy-3-(trifluoromethyl)cyclobutane-1-carbonyl)piperidin-4-yl)-1H-indol-2-yl)-3-methylpyrazolo[1,5-a]pyridin-6-yl)methanone (456);
((R)-3-aminopiperidin-1-yl)(2-(1-(cyclopropylmethyl)-7-(1-(tetrahydro-2H-pyran-2-carbonyl)piperidin-4-yl)-1H-indol-2-yl)-3-methylpyrazolo[1,5-a]pyridin-6-yl)methanone (457);
((3R,5R)-3-amino-5-fluoropiperidin-1-yl)(2-(1-(cyclopropylmethyl)-7-(1-(tetrahydro-2H-pyran-2-carbonyl)piperidin-4-yl)-1H-indol-2-yl)-3-methylpyrazolo[1,5-a]pyridin-6-yl)methanone (458);
(S)-1-(4-(2-(6-((3R,5R)-3-amino-5-fluoropiperidine-1-carbonyl)-3-methylpyrazolo[1,5-a]pyridin-2-yl)-1-(cyclopropylmethyl)-1H-indol-7-yl)piperidin-1-yl)-2-methoxypropan-1-one (459);
(R)-1-(4-(2-(6-((R)-3-aminopiperidine-1-carbonyl)-3 -methylpyrazolo[1,5-a]pyridin-2-yl)-1-(cyclopropylmethyl)-1H-indol-7-yl)piperidin-1-yl)-2-methoxypropan-1-one (460);
(R)-1-(4-(2-(6-((3R,5R)-3-amino-5-fluoropiperidine-1-carbonyl)-3-methylpyrazolo[1,5-a]pyridin-2-yl)-1-(cyclopropylmethyl)-1H-indol-7-yl)piperidin-1-yl)-2-methoxypropan-1-one (461);
((R)-3-aminopiperidin-1-yl)(2-(1-(cyclopropylmethyl)-7-(1-(tetrahydrofuran-2-carbonyl)piperidin-4-yl)-1H-indol-2-yl)-3-methylpyrazolo[1,5-a]pyridin-6-yl)methanone (462);
((3R,5R)-3-amino-5-fluoropiperidin-1-yl)(2-(1-(cyclopropylmethyl)-7-(1-(tetrahydrofuran-2-carbonyl)piperidin-4-yl)-1H-indol-2-yl)-3-methylpyrazolo[1,5-a]pyridin-6-yl)methanone (463);
(R)-(3-aminopiperidin-1-yl)(2-(1-(cyclopropylmethyl)-7-(1-(thiophene-2-carbonyl)piperidin-4-yl)-1H-indol-2-yl)-3-methylpyrazolo[1,5-a]pyridin-6-yl)methanone (464);
(R)-(3-aminopiperidin-1-yl)(2-(1-(cyclopropylmethyl)-7-(1-(3-hydroxycyclobutane-1-carbonyl)piperidin-4-yl)-1H-indol-2-yl)-3-methylpyrazolo[1,5-a]pyridin-6-yl)methanone (465);
((3R,5R)-3-amino-5-fluoropiperidin-1-yl)(2-(1-(cyclopropylmethyl)-7-(1-(3-hydroxycyclobutane-1-carbonyl)piperidin-4-yl)-1H-indol-2-yl)-3-methylpyrazolo[1,5-a]pyridin-6-yl)methanone (466);
(4-(2-(6-((7R)-7-amino-2-azabicyclo[2.2.1]heptane-2-carbonyl)-3-methylpyrazolo[1,5-a]pyridin-2-yl)-1-(cyclopropylmethyl)-1H-indol-7-yl)piperidin-1-yl)(3-hydroxycyclobutyl)methanone (467);
Methyl (R)-4-(2-(6-(3-aminopiperidine-1-carbonyl)-3-methylpyrazolo[1,5-a]pyridin-2-yl)-1-(cyclopropylmethyl)-1H-indol-7-yl)piperidine-1-carboxylate (468);
Methyl 4-(2-(6-((3R,5R)-3-amino-5-fluoropiperidine-1-carbonyl)-3-methylpyrazolo[1,5-a]pyridin-2-yl)-1-(cyclopropylmethyl)-1H-indol-7-yl)piperidine-1-carboxylate (469);
Methyl 4-(2-(6-((7R)-7-amino-2-azabicyclo[2.2.1]heptane-2-carbonyl)-3-methylpyrazolo[1,5-a]pyridin-2-yl)-1-(cyclopropylmethyl)-1H-indol-7-yl)piperidine-1-carboxylate (470);
(R)-1-(4-(2-(6-((R)-3-aminopiperidine-1-carbonyl)-3-methylpyrazolo[1,5-a]pyridin-2-yl)-1-(cyclopropylmethyl)-1H-indol-7-yl)piperidin-1-yl)-2-hydroxypropan-1-one (471);
(R)-1-(4-(2-(6-((3R,5R)-3-amino-5-fluoropiperidine-1-carbonyl)-3-methylpyrazolo[1,5-a]pyridin-2-yl)-1-(cyclopropylmethyl)-1H-indol-7-yl)piperidin-1-yl)-2-hydroxypropan-1-one (472);
(2R)-1-(4-(2-(6-((7R)-7-Amino-2-azabicyclo[2.2.1]heptane-2-carbonyl)-3 - methylpyrazolo[1,5-a]pyridin-2-yl)-1-(cyclopropylmethyl)-1H-indol-7-yl)piperidin-1-yl)-2-hydroxypropan-1-one (473);
((3R,5R)-3-Amino-5-fluoropiperidin-1-yl)(2-(1-(cyclopropylmethyl)-7-(1-(thiophene-2-carbonyl)piperidin-4-yl)-1H-indol-2-yl)-3-methylpyrazolo[1,5-a]pyridin-6-yl)methanone (474);
(4-(2-(6-((7R)-7-Amino-2-azabicyclo[2.2.1]heptane-2-carbonyl)-3-methylpyrazolo[1,5-a]pyridin-2-yl)-1-(cyclopropylmethyl)-1H-indol-7-yl)piperidin-1-yl)(thiophen-2-yl)methanone (475);
1-(4-(2-(6-((3R,5R)-3-Amino-5-fluoropiperidine-1-carbonyl)-3-methylpyrazolo[1,5-a]pyridin-2-yl)-1-(cyclopropylmethyl)-1H-indol-7-yl)piperidin-1-yl)ethan-1-one (476);
1-(4-(2-(6-((7R)-7-Amino-2-azabicyclo[2.2.1]heptane-2-carbonyl)-3-methylpyrazolo[1,5-a]pyridin-2-yl)-1-(cyclopropylmethyl)-1H-indol-7-yl)piperidin-1-yl)ethan-1-one (477);
1-(4-(2-(6-((3R,5R)-3-Amino-5-fluoropiperidine-1-carbonyl)-3-methylpyrazolo[1,5-a]pyridin-2-yl)-1-(cyclopropylmethyl)-1H-indol-7-yl)piperidin-1-yl)-2-methoxyethan-1-one (478);
(R)-1-(4-(2-(6-(3-Aminopiperidine-1-carbonyl)-3-methylpyrazolo[1,5-a]pyridin-2-yl)-1-(cyclopropylmethyl)-1H-indol-7-yl)piperidin-1-yl)ethan-1-one (479);
(R)-1-(4-(2-(6-(3-aminopiperidine-1-carbonyl)-4-methoxy-3-methylpyrazolo[1,5-a]pyridin-2-yl)-1-(cyclopropylmethyl)-5-fluoro-1H-indol-7-yl)piperidin-1-yl)-2-methoxyethan-1-one (480);
1-(4-(2-(6-((3R,5R)-3-Amino-5-fluoropiperidine-1-carbonyl)-4-methoxy-3-methylpyrazolo[1,5-a]pyridin-2-yl)-1-(cyclopropylmethyl)-5-fluoro-1H-indol-7-yl)piperidin-1-yl)-2-methoxyethan-1-one (481);
1-(4-(2-(6-((3R,5R)-3-Amino-5-fluoropiperidine-1-carbonyl)-4-methoxy-3-methylpyrazolo[1,5-a]pyridin-2-yl)-1-(cyclopropylmethyl)-5-fluoro-1H-indol-7-yl)piperidin-1-yl)ethan-1-one (482);
(R)-1-(4-(2-(6-(3-Aminopiperidine-1-carbonyl)-4-methoxy-3-methylpyrazolo[1,5-a]pyridin-2-yl)-1-(cyclopropylmethyl)-5-fluoro-1H-indol-7-yl)piperidin-1-yl)ethan-1-one (483);
((R)-3-Aminopiperidin-1-yl)(2-(1-(cyclopropylmethyl)-5-fluoro-7-(1-((1r,4r)-4-hydroxycyclohexane-1-carbonyl)piperidin-4-yl)-1H-indol-2-yl)-3-methylpyrazolo[1,5-a]pyridin-6-yl)methanone (484);
((R)-3-Aminopiperidin-1-yl)(2-(1-(cyclopropylmethyl)-5-fluoro-7-(1-((1s,4s)-4-hydroxycyclohexane-1-carbonyl)piperidin-4-yl)-1H-indol-2-yl)-3-methylpyrazolo[1,5-a]pyridin-6-yl)methanone (485);
((3R,5R)-3-Amino-5-fluoropiperidin-1-yl)(2-(1-(cyclopropylmethyl)-5-fluoro-7-(1-((1s,4s)-4-hydroxycyclohexane-1-carbonyl)piperidin-4-yl)-1H-indol-2-yl)-3-methylpyrazolo[1,5-a]pyridin-6-yl)methanone (486);
((3R,5R)-3-Amino-5-fluoropiperidin-1-yl)(2-(1-(cyclopropylmethyl)-5-fluoro-7-(1-((1r,4r)-4-hydroxycyclohexane-1-carbonyl)piperidin-4-yl)-1H-indol-2-yl)-3-methylpyrazolo[1,5-a]pyridin-6-yl)methanone (487);
((R)-3-Aminopiperidin-1-yl)(2-(1-(cyclopropylmethyl)-5-fluoro-7-(1-(tetrahydrofuran-2-carbonyl)piperidin-4-yl)-1H-indol-2-yl)-3-methylpyrazolo[1,5-a]pyridin-6-yl)methanone (488);
((R)-3-Aminopiperidin-1-yl)(2-(1-(cyclopropylmethyl)-5-fluoro-7-(1-(tetrahydrofuran-2-carbonyl)piperidin-4-yl)-1H-indol-2-yl)-3-methylpyrazolo[1,5-a]pyridin-6-yl)methanone (489);
((3R,5R)-3-Amino-5-fluoropiperidin-1-yl)(2-(1-(cyclopropylmethyl)-5-fluoro-7-(1-(tetrahydrofuran-2-carbonyl)piperidin-4-yl)-1H-indol-2-yl)-3-methylpyrazolo[1,5-a]pyridin-6-yl)methanone (490);
((3R,5R)-3-Amino-5-fluoropiperidin-1-yl)(2-(1-(cyclopropylmethyl)-5-fluoro-7-(1-(tetrahydrofuran-2-carbonyl)piperidin-4-yl)-1H-indol-2-yl)-3-methylpyrazolo[1,5-a]pyridin-6-yl)methanone (491);
(R)-(3-Aminopiperidin-1-yl)(2-(1-(cyclopropylmethyl)-5-fluoro-7-(1-(3-hydroxycyclobutane-1-carbonyl)piperidin-4-yl)-1H-indol-2-yl)-3-methylpyrazolo[1,5-a]pyridin-6-yl)methanone (492);
(R)-1-(4-(2-(6-(3-Aminopiperidine-1-carbonyl)-3-methylpyrazolo[1,5-a]pyridin-2-yl)-1-(cyclopropylmethyl)-5-fluoro-1H-indol-7-yl)piperidin-1-yl)-2-methoxy-2-methylpropan-1-one (493);
1-(4-(2-(6-((3R,5R)-3-Amino-5-fluoropiperidine-1-carbonyl)-3-methylpyrazolo[1,5-a]pyridin-2-yl)-1-(cyclopropylmethyl)-5-fluoro-1H-indol-7-yl)piperidin-1-yl)-3-methylbutan-1-one (494);
1-(4-(2-(6-((3R,5R)-3-Amino-5-fluoropiperidine-1-carbonyl)-3-methylpyrazolo[1,5-a]pyridin-2-yl)-1-(cyclopropylmethyl)-5-fluoro-1H-indol-7-yl)piperidin-1-yl)-3-methylbutan-1-one (495);
((3R,5R)-3-Amino-5-fluoropiperidin-1-yl)(2-(1-(cyclopropylmethyl)-5-fluoro-7-(1-(3-hydroxycyclobutane-1-carbonyl)piperidin-4-yl)-1H-indol-2-yl)-3-methylpyrazolo[1,5-a]pyridin-6-yl)methanone (496);
(R)-1-(4-(2-(6-((R)-3-Aminopiperidine-1-carbonyl)-3-methylpyrazolo[1,5-a]pyridin-2-yl)-1-(cyclopropylmethyl)-5-fluoro-1H-indol-7-yl)piperidin-1-yl)-2-methoxypropan-1-one (497);
(R)-1-(4-(2-(6-((3R,5R)-3-Amino-5-fluoropiperidine-1-carbonyl)-3-methylpyrazolo[1,5-a]pyridin-2-yl)-1-(cyclopropylmethyl)-5-fluoro-1H-indol-7-yl)piperidin-1-yl)-2-methoxypropan-1-one (498);
(S)-1-(4-(2-(6-((R)-3-Aminopiperidine-1-carbonyl)-3-methylpyrazolo[1,5-a]pyridin-2-yl)-1-(cyclopropylmethyl)-5-fluoro-1H-indol-7-yl)piperidin-1-yl)-2-methoxypropan-1-one (499);
(S)-1-(4-(2-(6-((3R,5R)-3-Amino-5-fluoropiperidine-1-carb onyl)-3-methylpyrazolo[1,5-a]pyridin-2-yl)-1-(cyclopropylmethyl)-5-fluoro-1H-indol-7-yl)piperidin-1-yl)-2-methoxypropan-1-one (500);
1-(4-(2-(6-((3R,5R)-3-Amino-5-fluoropiperidine-1-carbonyl)-3-methylpyrazolo[1,5-a]pyridin-2-yl)-1-(cyclopropylmethyl)-5-fluoro-1H-indol-7-yl)piperidin-1-yl)-2-methoxy-2-methylpropan-1-one (501);
(R)-1-(4-(2-(6-(3-Aminopiperidine-1-carbonyl)-3-methylpyrazolo[1,5-a]pyridin-2-yl)-1-(cyclopropylmethyl)-5-fluoro-1H-indol-7-yl)piperidin-1-yl)-2-methoxyethan-1-one (502);
1-(4-(2-(6-((3R,5R)-3-Amino-5-fluoropiperidine-1-carbonyl)-3-methylpyrazolo[1,5-a]pyridin-2-yl)-1-(cyclopropylmethyl)-5-fluoro-1H-indol-7-yl)piperidin-1-yl)-2-methoxyethan-1-one (503);
(R)-(3-Aminopiperidin-1-yl)(2-(1-(cyclopropylmethyl)-5-fluoro-7-(1-(3-hydroxy-3-(trifluoromethyl)cyclobutane-1-carbonyl)piperidin-4-yl)-1H-indol-2-yl)-3-methylpyrazolo[1,5-a]pyridin-6-yl)methanone (504);
((3R,5R)-3-Amino-5-fluoropiperidin-1-yl)(2-(1-(cyclopropylmethyl)-5-fluoro-7-(1-(3-hydroxy-3-(trifluoromethyl)cyclobutane-1-carbonyl)piperidin-4-yl)-1H-indol-2-yl)-3-methylpyrazolo[1,5-a]pyridin-6-yl)methanone (505);
(R)-1-(4-(2-(6-(3-Aminopiperidine-1-carbonyl)-3-methylpyrazolo[1,5-a]pyridin-2-yl)-1-(cyclopropylmethyl)-5-fluoro-1H-indol-7-yl)piperidin-1-yl)ethan-1-one (506);
1-(4-(2-(6-((3R,5R)-3-Amino-5-fluoropiperidine-1-carbonyl)-3-methylpyrazolo[1,5-a]pyridin-2-yl)-1-(cyclopropylmethyl)-5-fluoro-1H-indol-7-yl)piperidin-1-yl)ethan-1-one (507);
((R)-3-Aminopiperidin-1-yl)(2-(1-(cyclopropylmethyl)-5-fluoro-7-(1-(tetrahydro-2H-pyran-2-carbonyl)piperidin-4-yl)-1H-indol-2-yl)-3-methylpyrazolo[1,5-a]pyridin-6-yl)methanone (508);
((3R,5R)-3-Amino-5-fluoropiperidin-1-yl)(2-(1-(cyclopropylmethyl)-5-fluoro-7-(1-(tetrahydro-2H-pyran-2-carbonyl)piperidin-4-yl)-1H-indol-2-yl)-3-methylpyrazolo[1,5-a]pyridin-6-yl)methanone (509);
(R)-1-(4-(2-(6-(3-Aminopiperidine-1-carbonyl)-4-methoxy-3-methylpyrazolo[1,5-a]pyridin-2-yl)-1-(cyclopropylmethyl)-1H-pyrrolo[2,3-c]pyridin-7-yl)piperidin-1-yl)-2-methoxyethan-1-one (510);
1-(4-(2-(6-((3R,5R)-3-Amino-5-fluoropiperidine-1-carbonyl)-4-methoxy-3-methylpyrazolo[1,5-a]pyridin-2-yl)-1-(cyclopropylmethyl)-1H-pyrrolo[2,3-c]pyridin-7-yl)piperidin-1-yl)-2-methoxyethan-1-one (511);
(R)-1-(4-(2-(6-(3-Aminopiperidine-1-carbonyl)-3-methylpyrazolo[1,5-a]pyridin-2-yl)-1-(cyclopropylmethyl)-1H-pyrrolo[2,3-c]pyridin-7-yl)piperidin-1-yl)-2-methoxyethan-1-one (512);
1-(4-(2-(6-((3R,5R)-3-Amino-5-fluoropiperidine-1-carbonyl)-3-methylpyrazolo[1,5-a]pyridin-2-yl)-1-(cyclopropylmethyl)-1H-pyrrolo[2,3-c]pyridin-7-yl)piperidin-1-yl)-2-methoxyethan-1-one (513);
((3R,5R)-3-Amino-5-fluoropiperidin-1-yl)(2-(1-(cyclopropylmethyl)-7-(1-((1r,4r)-4-hydroxycyclohexane-1-carbonyl)azetidin-3-yl)-1H-indol-2-yl)-4-methoxy-3-methylpyrazolo[1,5-a]pyridin-6-yl)methanone (514);
(R)-(3-Aminopiperidin-1-yl)(2-(1-(cyclopropylmethyl)-7-(1-(3-hydroxy-3-(trifluoromethyl)cyclobutane-1-carbonyl)azetidin-3-yl)-1H-indol-2-yl)-4-methoxy-3-methylpyrazolo[1,5-a]pyridin-6-yl)methanone (515);
(R)-1-(3-(2-(6-(3-Aminopiperidine-1-carbonyl)-4-methoxy-3-methylpyrazolo[1,5-a]pyridin-2-yl)-1-(cyclopropylmethyl)-1H-indol-7-yl)azetidin-1-yl)-2-methoxy-2-methylpropan-1-one (516);
((R)-3-Aminopiperidin-1-yl)(2-(1-(cyclopropylmethyl)-7-(1-((1r,4r)-4-hydroxycyclohexane-1-carbonyl)azetidin-3-yl)-1H-indol-2-yl)-4-methoxy-3-methylpyrazolo[1,5-a]pyridin-6-yl)methanone (517);
1-(3-(2-(6-((3R,5R)-3-Amino-5-fluoropiperidine-1-carbonyl)-4-methoxy-3-methylpyrazolo[1,5-a]pyridin-2-yl)-1-(cyclopropylmethyl)-1H-indol-7-yl)azetidin-1-yl)-2-methoxy-2-methylpropan-1-one (518);
((3R,5R)-3-Amino-5-fluoropiperidin-1-yl)(2-(1-(cyclopropylmethyl)-7-(1-((1s,4s)-4-hydroxycyclohexane-1-carbonyl)azetidin-3-yl)-1H-indol-2-yl)-4-methoxy-3-methylpyrazolo[1,5-a]pyridin-6-yl)methanone (519);
((R)-3-Aminopiperidin-1-yl)(2-(1-(cyclopropylmethyl)-7-(1-((1s,4s)-4-hydroxycyclohexane-1-carbonyl)azetidin-3-yl)-1H-indol-2-yl)-4-methoxy-3-methylpyrazolo[1,5-a]pyridin-6-yl)methanone (520);
(R)-1-(3-(2-(6-(3-Aminopiperidine-1-carbonyl)-4-methoxy-3-methylpyrazolo[1,5-a]pyridin-2-yl)-1-(cyclopropylmethyl)-1H-indol-7-yl)azetidin-1-yl)ethan-1-one (521);
1-(3-(2-(6-((3R,5R)-3-Amino-5-fluoropiperidine-1-carbonyl)-4-methoxy-3-methylpyrazolo[1,5-a]pyridin-2-yl)-1-(cyclopropylmethyl)-1H-indol-7-yl)azetidin-1-yl)ethan-1-one (522);
(S)-1-(3-(2-(6-((R)-3-Aminopiperidine-1-carbonyl)-4-methoxy-3-methylpyrazolo[1,5-a]pyridin-2-yl)-1-(cyclopropylmethyl)-1H-indol-7-yl)azetidin-1-yl)-2-methoxypropan-1-one (523);
(S)-1-(3-(2-( 6-((3R,5R)-3-Amino-5-fluoropiperidine-1-carbonyl)-4-methoxy-3-methylpyrazolo[1,5-a]pyridin-2-yl)-1-(cyclopropylmethyl)-1H-indol-7-yl)azetidin-1-yl)-2-methoxypropan-1-one (524);
(R)-1-(3-(2-(6-((R)-3-Aminopiperidine-1-carbonyl)-4-methoxy-3-methylpyrazolo[1,5-a]pyridin-2-yl)-1-(cyclopropylmethyl)-1H-indol-7-yl)azetidin-1-yl)-2-methoxypropan-1-one (525);
(R)-1-(3-(2-(6-((3R,5R)-3-Amino-5-fluoropiperidine-1-carbonyl)-4-methoxy-3-methylpyrazolo[1,5-a]pyridin-2-yl)-1-(cyclopropylmethyl)-1H-indol-7-yl)azetidin-1-yl)-2-methoxypropan-1-one (526);
1-(3-(2-(6-((3R,5R)-3-Amino-5-fluoropiperidine-1-carbonyl)-4-methoxy-3-methylpyrazolo[1,5-a]pyridin-2-yl)-1-(cyclopropylmethyl)-1H-indol-7-yl)azetidin-1-yl)-2-methoxyethan-1-one (527);
(R)-1-(3-(2-(6-(3-Aminopiperidine-1-carbonyl)-4-methoxy-3-methylpyrazolo[1,5-a]pyridin-2-yl)-1-(cyclopropylmethyl)-1H-indol-7-yl)azetidin-1-yl)-2-methoxyethan-1-one (528);
(R)-1-(3-(2-(6-(3-Aminopiperidine-1-carbonyl)-4-methoxy-3-methylpyrazolo[1,5-a]pyridin-2-yl)-1-(cyclopropylmethyl)-1H-indol-7-yl)azetidin-1-yl)-2-methoxyethan-1-one (529);
((R)-3-Aminopiperidin-1-yl)(2-(1-(cyclopropylmethyl)-7-(1-((1r,4r)-4-hydroxycyc1ohexane-1-carbonyl)azetidin-3-yl)-1H-indol-2-yl)-3-methylpyrazolo[1,5-a]pyridin-6-yl)methanone (530);
((3R,5R)-3-Amino-5-fluoropiperidin-1-yl)(2-(1-(cyclopropylmethyl)-7-(1-((1R,3S)-3-hydroxycyclohexane-1-carbonyl)azetidin-3-yl)-1H-indol-2-yl)-3-methylpyrazolo[1,5-a]pyridin-6-yl)methanone (531);
((R)-3-Aminopiperidin-1-yl)(2-(1-(cyclopropylmethyl)-7-(1-((1R,3S)-3-hydroxycyclohexane-1-carbonyl)azetidin-3-yl)-1H-indol-2-yl)-3-methylpyrazolo[1,5-a]pyridin-6-yl)methanone (532);
((3R,5R)-3-Amino-5-fluoropiperidin-1-yl)(2-(1-(cyclopropylmethyl)-7-(1-((1S,3R)-3-hydroxycyclohexane-1-carbonyl)azetidin-3-yl)-1H-indol-2-yl)-3-methylpyrazolo[1,5-a]pyridin-6-yl)methanone (533);
((R)-3-Aminopiperidin-1-yl)(2-(1-(cyclopropylmethyl)-7-(1-((1S,3R)-3-hydroxycyclohexane-1-carbonyl)azetidin-3-yl)-1H-indol-2-yl)-3-methylpyrazolo[1,5-a]pyridin-6-yl)methanone (534);
(R)-(3-Aminopiperidin-1-yl)(2-(1-(cyclopropylmethyl)-7-(1-(3-hydroxy-3-(trifluoromethyl)cyclobutane-1-carbonyl)azetidin-3-yl)-1H-indol-2-yl)-3-methylpyrazolo[1,5-a]pyridin-6-yl)methanone (535);
(R)-1-(3-(2-(6-(3-Aminopiperidine-1-carbonyl)-3-methylpyrazolo[1,5-a]pyridin-2-yl)-1-(cyclopropylmethyl)-1H-indol-7-yl)azetidin-1-yl)-2-methoxy-2-methylpropan-1-one (536);
((3R,5R)-3-Amino-5-fluoropiperidin-1-yl)(2-(1-(cyclopropylmethyl)-7-(1-((1r,4r)-4-hydroxycyclohexane-1-carbonyl)azetidin-3-yl)-1H-indol-2-yl)-3-methylpyrazolo[1,5-a]pyridin-6-yl)methanone (537);
((3R,5R)-3-Amino-5-fluoropiperidin-1-yl)(2-(1-(cyclopropylmethyl)-7-(1-((1s,4s)-4-hydroxycyclohexane-1-carbonyl)azetidin-3-yl)-1H-indol-2-yl)-3-methylpyrazolo[1,5-a]pyridin-6-yl)methanone (538);
((R)-3-Aminopiperidin-1-yl)(2-(1-(cyclopropylmethyl)-7-(1-((1s,4s)-4-hydroxycyclohexane-1-carbonyl)azetidin-3-yl)-1H-indol-2-yl)-3-methylpyrazolo[1,5-a]pyridin-6-yl)methanone (539);
(R)-1-(3-(2-(6-((3R,5R)-3-Amino-5-fluoropiperidine-1-carbonyl)-3-methylpyrazolo[1,5-a]pyridin-2-yl)-1-(cyclopropylmethyl)-1H-indol-7-yl)azetidin-1-yl)-2-methoxypropan-1-one (540);
(R)-1-(3-(2-(6-((R)-3-Aminopiperidine-1-carbonyl)-3-methylpyrazolo[1,5-a]pyridin-2-yl)-1-(cyclopropylmethyl)-1H-indol-7-yl)azetidin-1-yl)-2-methoxypropan-1-one (541);
(S)-1-(3-(2-(6-((3R,5R)-3-Amino-5-fluoropiperidine-1-carbonyl)-3-methylpyrazolo[1,5-a]pyridin-2-yl)-1-(cyclopropylmethyl)-1H-indol-7-yl)azetidin-1-yl)-2-methoxypropan-1-one (542);
(S)-1-(3-(2-(6-((R)-3-aminopiperidine-1-carbonyl)-3-methylpyrazolo[1,5-a]pyridin-2-yl)-1-(cyclopropylmethyl)-1H-indol-7-yl)azetidin-1-yl)-2-methoxypropan-1-one (543);
((3R,5R)-3-Amino-5-fluoropiperidin-1-yl)(2-(1-(cyclopropylmethyl)-7-(1-(tetrahydro-2H-pyran-2-carbonyl)azetidin-3-yl)-1H-indol-2-yl)-3-methylpyrazolo[1,5-a]pyridin-6-yl)methanone (544);
((R)-3-Aminopiperidin-1-yl)(2-(1-(cyclopropylmethyl)-7-(1-(tetrahydro-2H-pyran-2-carbonyl)azetidin-3-yl)-1H-indol-2-yl)-3-methylpyrazolo[1,5-a]pyridin-6-yl)methanone (545);
(R)-1-(3-(2-(6-(3-Aminopiperidine-1-carbonyl)-3-methylpyrazolo[1,5-a]pyridin-2-yl)-1-(cyclopropylmethyl)-1H-indol-7-yl)azetidin-1-yl)-2-methoxyethan-1-one (546);
1-(3-(2-(6-((3R,5R)-3-Amino-5-fluoropiperidine-1-carbonyl)-3-methylpyrazolo[1,5-a]pyridin-2-yl)-1-(cyclopropylmethyl)-1H-indol-7-yl)azetidin-1-yl)-2-methoxyethan-1-one (547);
1-(3-(2-(6-((3R,5R)-3-Amino-5-fluoropiperidine-1-carbonyl)-3-methylpyrazolo[1,5-a]pyridin-2-yl)-1-(cyclopropylmethyl)-1H-indol-7-yl)azetidin-1-yl)ethan-1-one (548);
1-(3-(2-(6-((7R)-7-Amino-2-azabicyclo[2.2.1]heptane-2-carbonyl)-3-methylpyrazolo[1,5-a]pyridin-2-yl)-1-(cyclopropylmethyl)-1H-indol-7-yl)azetidin-1-yl)ethan-1-one (549);
4-(((2-(6-((R)-3-Aminopiperidine-1-carbonyl)-4-methoxy-3-methylpyrazolo[1,5-a]pyridin-2-yl)-1-(cyclopropylmethyl)-1H-indol-7-yl)oxy)methyl)pyrrolidin-2-one (550);
4-(((2-(6-((R)-3-Aminopiperidine-1-carbonyl)-4-methoxy-3-methylpyrazolo[1,5-a]pyridin-2-yl)-1-(cyclopropylmethyl)-1H-indol-7-yl)oxy)methyl)-1-methylpyrrolidin-2-one (551);
4-(((2-(6-((R)-3-Aminopiperidine-1-carbonyl)-4-methoxy-3-methylpyrazolo[1,5-a]pyridin-2-yl)-1-(cyclopropylmethyl)-1H-indol-7-yl)oxy)methyl)-1-methylpyrrolidin-2-one (552);
4-(((2-(6-((3R,5R)-3-Amino-5-fluoropiperidine-1-carbonyl)-4-methoxy-3-methylpyrazolo[1,5-a]pyridin-2-yl)-1-(cyclopropylmethyl)-1H-indol-7-yl)oxy)methyl)-1-methylpyrrolidin-2-one (553);
4-(((2-(6-((3R,5R)-3-Amino-5-fluoropiperidine-1-carbonyl)-4-methoxy-3-methylpyrazolo[1,5-a]pyridin-2-yl)-1-(cyclopropylmethyl)-1H-indol-7-yl)oxy)methyl)-1-methylpyrrolidin-2-one (554);
(R)-(2-(7-(2-(1H-1,2,4-Triazol-1-yl)ethoxy)-1-(cyclopropylmethyl)-1H-indol-2-yl)-4-methoxy-3-methylpyrazolo[1,5-a]pyridin-6-yl)(3-aminopiperidin-1-yl)methanone (555);
(2-(7-(2-(1H-1,2,4-Triazol-1-yl)ethoxy)-1-(cyclopropylmethyl)-1H-indol-2-yl)-4-methoxy-3-methylpyrazolo[1,5-a]pyridin-6-yl)((3R,5R)-3-amino-5-fluoropiperidin-1-yl)methanone (556);
(2-(7-(2-(4-Amino-1H-pyrazol-1-yl)ethoxy)-1-(cyclopropylmethyl)-1H-indol-2-yl)-4-methoxy-3-methylpyrazolo[1,5-a]pyridin-6-yl)((3R,5R)-3-amino-5-fluoropiperidin-1-yl)methanone (557);
(R)-(2-(7-(2-(4-Amino-1H-pyrazol-1-yl)ethoxy)-1-(cyclopropylmethyl)-1H-indol-2-yl)-4-methoxy-3-methylpyrazolo[1,5-a]pyridin-6-yl)(3-aminopiperidin-1-yl)methanone (558);
(S)-5-(((2-(6-((R)-3-Aminopiperidine-1-carbonyl)-4-methoxy-3-methylpyrazolo[1,5-a]pyridin-2-yl)-1-(cyclopropylmethyl)-1H-indol-7-yl)oxy)methyl)pyrrolidin-2-one (559);
(S)-5-(((2-(6-((3R,5R)-3-Amino-5-fluoropiperidine-1-carbonyl)-4-methoxy-3-methylpyrazolo[1,5-a]pyridin-2-yl)-1-(cyclopropylmethyl)-1H-indol-7-yl)oxy)methyl)pyrrolidin-2-one (560);
(R)-5-(((2-(6-((R)-3-Aminopiperidine-1-carbonyl)-4-methoxy-3-methylpyrazolo[1,5-a]pyridin-2-yl)-1-(cyclopropylmethyl)-1H-indol-7-yl)oxy)methyl)pyrrolidin-2-one (561);
4-(((2-(6-((R)-3-Aminopiperidine-1-carbonyl)-4-methoxy-3-methylpyrazolo[1,5-a]pyridin-2-yl)-1-(cyclopropylmethyl)-1H-indol-7-yl)oxy)methyl)pyrrolidin-2-one (562);
4-(((2-(6-((3R,5R)-3-Amino-5-fluoropiperidine-1-carbonyl)-4-methoxy-3-methylpyrazolo[1,5-a]pyridin-2-yl)-1-(cyclopropylmethyl)-1H-indol-7-yl)oxy)methyl)pyrrolidin-2-one (563);
4-(((2-(6-((R)-3-Aminopiperidine-1-carbonyl)-4-methoxy-3-methylpyrazolo[1,5-a]pyridin-2-yl)-1-(cyclopropylmethyl)-1H-indol-7-yl)oxy)methyl)pyrrolidin-2-one (564);
4-(((2-(6-((3R,5R)-3-Amino-5-fluoropiperidine-1-carbonyl)-4-methoxy-3-methylpyrazolo[1,5-a]pyridin-2-yl)-1-(cyclopropylmethyl)-1H-indol-7-yl)oxy)methyl)pyrrolidin-2-one (565);
4-(((2-(6-((R)-3-Aminopiperidine-1-carbonyl)-4-fluoro-3-methylpyrazolo[1,5-a]pyridin-2-yl)-1-(cyclopropylmethyl)-1H-indol-7-yl)oxy)methyl)pyrrolidin-2-one (566);
4-(((2-(6-((3R,5R)-3-Amino-5-fluoropiperidine-1-carbonyl)-4-fluoro-3-methylpyrazolo[1,5-a]pyridin-2-yl)-1-(cyclopropylmethyl)-1H-indol-7-yl)oxy)methyl)pyrrolidin-2-one (567);
(S)-5-(((2-(6-((3R,5R)-3-Amino-5-fluoropiperidine-1-carbonyl)-4-fluoro-3-methylpyrazolo[1,5-a]pyridin-2-yl)-1-(cyclopropylmethyl)-1H-indol-7-yl)oxy)methyl)pyrrolidin-2-one (568);
(S)-5-(((2-(6-((R)-3-Aminopiperidine-1-carbonyl)-4-fluoro-3-methylpyrazolo[1,5-a]pyridin-2-yl)-1-(cyclopropylmethyl)-1H-indol-7-yl)oxy)methyl)pyrrolidin-2-one (569);
(R)-5-(((2-(6-((R)-3-Aminopiperidine-1-carbonyl)-4-fluoro-3-methylpyrazolo[1,5-a]pyridin-2-yl)-1-(cyclopropylmethyl)-1H-indol-7-yl)oxy)methyl)pyrrolidin-2-one (570);
4-(((2-(6-((R)-3-Aminopiperidine-1-carbonyl)-4-fluoro-3-methylpyrazolo[1,5-a]pyridin-2-yl)-1-(cyclopropylmethyl)-1H-indol-7-yl)oxy)methyl)pyrrolidin-2-one (571);
4-(((2-(6-((3R,5R)-3-Amino-5-fluoropiperidine-1-carbonyl)-4-fluoro-3-methylpyrazolo[1,5-a]pyridin-2-yl)-1-(cyclopropylmethyl)-1H-indol-7-yl)oxy)methyl)pyrrolidin-2-one (572);
4-(((2-(6-((3R,5R)-3-Amino-5-fluoropiperidine-1-carbonyl)-3-methylpyrazolo[1,5-a]pyridin-2-yl)-1-(cyclopropylmethyl)-1H-indol-7-yl)oxy)methyl)pyrrolidin-2-one (573);
(2-(7-(2-(1H-Imidazol-1-yl)ethoxy)-1-(cyclopropylmethyl)-1H-indol-2-yl)-3-methylpyrazolo[1,5-a]pyridin-6-yl)((3R,5R)-3-amino-5-fluoropiperidin-1-yl)methanone (574);
(R)-(2-(7-(2-(1H-Imidazol-1-yl)ethoxy)-1-(cyclopropylmethyl)-1H-indol-2-yl)-3-methylpyrazolo[1,5-a]pyridin-6-yl)(3-aminopiperidin-1-yl)methanone (575);
4-(((2-(6-((R)-3-Aminopiperidine-1-carbonyl)-3-methylpyrazolo[1,5-a]pyridin-2-yl)-1-(cyclopropylmethyl)-1H-indol-7-yl)oxy)methyl)-1-methylpyrrolidin-2-one (576);
4-(((2-(6-((3R,5R)-3-Amino-5-fluoropiperidine-1-carbonyl)-3-methylpyrazolo[1,5-a]pyridin-2-yl)-1-(cyclopropylmethyl)-1H-indol-7-yl)oxy)methyl)-1-methylpyrrolidin-2-one (577);
4-(((2-(6-((R)-3-Aminopiperidine-1-carbonyl)-3-methylpyrazolo[1,5-a]pyridin-2-yl)-1-(cyclopropylmethyl)-1H-indol-7-yl)oxy)methyl)-1-methylpyrrolidin-2-one (578);
4-(((2-(6-((3R,5R)-3-Amino-5-fluoropiperidine-1-carbonyl)-3-methylpyrazolo[1,5-a]pyridin-2-yl)-1-(cyclopropylmethyl)-1H-indol-7-yl)oxy)methyl)-1-methylpyrrolidin-2-one (579);
(R)-(2-(7-(2-(1H-1,2,4-Triazol-1-yl)ethoxy)-1-(cyclopropylmethyl)-1H-indol-2-yl)-3-methylpyrazolo[1,5-a]pyridin-6-yl)(3-aminopiperidin-1-yl)methanone (580);
(2-(7-(2-(1H-1,2,4-Triazol-1-yl)ethoxy)-1-(cyclopropylmethyl)-1H-indol-2-yl)-3-methylpyrazolo[1,5-a]pyridin-6-yl)((3R,5R)-3-amino-5-fluoropiperidin-1-yl)methanone (581);
(R)-(2-(7-(2-(4-Amino-1H-pyrazol-1-yl)ethoxy)-1-(cyclopropylmethyl)-1H-indol-2-yl)-3-methylpyrazolo[1,5-a]pyridin-6-yl)(3-aminopiperidin-1-yl)methanone (582);
(2-(7-(2-(4-Amino-1H-pyrazol-1-yl)ethoxy)-1-(cyclopropylmethyl)-1H-indol-2-yl)-3-methylpyrazolo[1,5-a]pyridin-6-yl)((3R,5R)-3-amino-5-fluoropiperidin-1-yl)methanone (583);
(S)-5-(((2-(6-((R)-3-Aminopiperidine-1-carbonyl)-3-methylpyrazolo[1,5-a]pyridin-2-yl)-1-(cyclopropylmethyl)-1H-indol-7-yl)oxy)methyl)pyrrolidin-2-one (584);
(S)-5-(((2-(6-((3R,5R)-3-Amino-5-fluoropiperidine-1-carbonyl)-3-methylpyrazolo[1,5-a]pyridin-2-yl)-1-(cyclopropylmethyl)-1H-indol-7-yl)oxy)methyl)pyrrolidin-2-one (585);
(R)-5-(((2-(6-((R)-3-Aminopiperidine-1-carbonyl)-3-methylpyrazolo[1,5-a]pyridin-2-yl)-1-(cyclopropylmethyl)-1H-indol-7-yl)oxy)methyl)pyrrolidin-2-one (586);
(R)-5-(((2-(6-((3R,5R)-3-Amino-5-fluoropiperidine-1-carbonyl)-3-methylpyrazolo[1,5-a]pyridin-2-yl)-1-(cyclopropylmethyl)-1H-indol-7-yl)oxy)methyl)pyrrolidin-2-one (587);
4-(((2-(6-((R)-3-Aminopiperidine-1-carbonyl)-3-methylpyrazolo[1,5-a]pyridin-2-yl)-1-(cyclopropylmethyl)-1H-indol-7-yl)oxy)methyl)pyrrolidin-2-one (588);
4-(((2-(6-((R)-3-Aminopiperidine-1-carbonyl)-3-methylpyrazolo[1,5-a]pyridin-2-yl)-1-(cyclopropylmethyl)-1H-indol-7-yl)oxy)methyl)pyrrolidin-2-one (589);
4-(((2-(6-((3R,5R)-3-Amino-5-fluoropiperidine-1-carbonyl)-3-methylpyrazolo[1,5-a]pyridin-2-yl)-1-(cyclopropylmethyl)-1H-indol-7-yl)oxy)methyl)pyrrolidin-2-one (590);
4-(((2-(6-((R)-3-Aminopiperidine-1-carbonyl)-4-methoxy-3-methylpyrazolo[1,5-a]pyridin-2-yl)-1-(cyclopropylmethyl)-1H-pyrrolo[2,3-c]pyridin-7-yl)oxy)methyl)pyrrolidin-2-one (591);
(2-(7-(2-(1H-1,2,4-Triazol-1-yl)ethoxy)-1-(cyclopropylmethyl)-1H-pyrrolo[2,3-c]pyridin-2-yl)-4-methoxy-3-methylpyrazolo[1,5-a]pyridin-6-yl)((3R,5R)-3-amino-5-fluoropiperidin-1-yl)methanone (592);
(R)-(2-(7-(2-(1H-1,2,4-Triazol-1-yl)ethoxy)-1-(cyclopropylmethyl)-1H-pyrrolo[2,3-c]pyridin-2-yl)-4-methoxy-3-methylpyrazolo[1,5-a]pyridin-6-yl)(3-aminopiperidin-1-yl)methanone (593);
((3R,5R)-3-Amino-5-fluoropiperidin-1-yl)(2-(1-(cyclopropylmethyl)-7-(2-(2-methyl-1H-imidazol-1-yl)ethoxy)-1H-pyrrolo[2,3-c]pyridin-2-yl)-4-methoxy-3-methylpyrazolo[1,5-a]pyridin-6-yl)methanone (594);
(R)-(3-Aminopiperidin-1-yl)(2-(1-(cyclopropylmethyl)-7-(2-(2-methyl-1H-imidazol-1-yl)ethoxy)-1H-pyrrolo[2,3-c]pyridin-2-yl)-4-methoxy-3-methylpyrazolo[1,5-a]pyridin-6-yl)methanone (595);
(R)-(3-Aminopiperidin-1-yl)(2-(1-(cyclopropylmethyl)-7-(oxetan-3-ylmethoxy)-1H-pyrrolo[2,3-c]pyridin-2-yl)-4-methoxy-3-methylpyrazolo[1,5-a]pyridin-6-yl)methanone (596);
(2-(7-(2-(1H-Imidazol-1-yl)ethoxy)-1-(cyclopropylmethyl)-1H-pyrrolo[2,3-c]pyridin-2-yl)-4-methoxy-3-methylpyrazolo[1,5-a]pyridin-6-yl)((3R,5R)-3-amino-5-fluoropiperidin-1-yl)methanone (597);
(R)-(2-(7-(2-(1H-Imidazol-1-yl)ethoxy)-1-(cyclopropylmethyl)-1H-pyrrolo[2,3-c]pyridin-2-yl)-4-methoxy-3-methylpyrazolo[1,5-a]pyridin-6-yl)(3-aminopiperidin-1-yl)methanone (598);
(R)-(2-(7-(2-(4H-1,2,4-Triazol-4-yl)ethoxy)-1-(cyclopropylmethyl)-1H-pyrrolo[2,3-c]pyridin-2-yl)-4-methoxy-3-methylpyrazolo[1,5-a]pyridin-6-yl)(3-aminopiperidin-1-yl)methanone (599);
(2-(7-(2-(4H-1,2,4-Triazol-4-yl)ethoxy)-1-(cyclopropylmethyl)-1H-pyrrolo[2,3-c]pyridin-2-yl)-4-methoxy-3-methylpyrazolo[1,5-a]pyridin-6-yl)((3R,5R)-3-amino-5-fluoropiperidin-1-yl)methanone (600);
4-(((2-(6-((3R,5R)-3-Amino-5-fluoropiperidine-1-carbonyl)-4-methoxy-3-methylpyrazolo[1,5-a]pyridin-2-yl)-1-(cyclopropylmethyl)-1H-pyrrolo[2,3-c]pyridin-7-yl)oxy)methyl)pyrrolidin-2-one (601);
4-(((2-(6-((3R,5R)-3-Amino-5-fluoropiperidine-1-carbonyl)-4-methoxy-3-methylpyrazolo[1,5-a]pyridin-2-yl)-1-(cyclopropylmethyl)-1H-pyrrolo[2,3-c]pyridin-7-yl)oxy)methyl)pyrrolidin-2-one (602);
4-(((2-(6-((R)-3-Aminopiperidine-1-carbonyl)-4-methoxy-3-methylpyrazolo[1,5-a]pyridin-2-yl)-1-(cyclopropylmethyl)-1H-pyrrolo[2,3-c]pyridin-7-yl)oxy)methyl)pyrrolidin-2-one (603);
(5R)-5-(((2-(6-((7R)-7-Amino-2-azabicyclo[2.2.1]heptane-2-carbonyl)-4-methoxy-3-methylpyrazolo[1,5-a]pyridin-2-yl)-1-(cyclopropylmethyl)-1H-pyrrolo[2,3-c]pyridin-7-yl)oxy)methyl)pyrrolidin-2-one (604);
4-(((2-(6-((7R)-7-Amino-2-azabicyclo[2.2.1]heptane-2-carbonyl)-4-methoxy-3-methylpyrazolo[1,5-a]pyridin-2-yl)-1-(cyclopropylmethyl)-1H-pyrrolo[2,3-c]pyridin-7-yl)oxy)methyl)pyrrolidin-2-one (605);
4-(((2-(6-((7R)-7-Amino-2-azabicyclo[2.2.1]heptane-2-carbonyl)-4-methoxy-3-methylpyrazolo[1,5-a]pyridin-2-yl)-1-(cyclopropylmethyl)-1H-pyrrolo[2,3-c]pyridin-7-yl)oxy)methyl)pyrrolidin-2-one (606);
(SR)-5-(((2-(6-((7S)-7-Amino-2-azabicyclo[2.2.1]heptane-2-carbonyl)-4-methoxy-3-methylpyrazolo[1,5-a]pyridin-2-yl)-1-(cyclopropylmethyl)-1H-pyrrolo[2,3-c]pyridin-7-yl)oxy)methyl)pyrrolidin-2-one (607);
4-(((2-(6-((7R)-7-Amino-2-azabicyclo[2.2.1]heptane-2-carbonyl)-4-methoxy-3-methylpyrazolo[1,5-a]pyridin-2-yl)-1-(cyclopropylmethyl)-1H-pyrrolo[2,3-c]pyridin-7-yl)oxy)methyl)pyrrolidin-2-one (608);
(R)-(2-(7-(2-(4H-1,2,4-Triazol-4-yl)ethoxy)-1-(cyclopropylmethyl)-1H-pyrrolo[2,3-c]pyridin-2-yl)-3-methylpyrazolo[1,5-a]pyridin-6-yl)(3-aminopiperidin-1-yl)methanone (609);
(2-(7-(2-(1H-1,2,4-Triazol-1-yl)ethoxy)-1-(cyclopropylmethyl)-1H-pyrrolo[2,3-c]pyridin-2-yl)-3-methylpyrazolo[1,5-a]pyridin-6-yl)((3R,5R)-3-amino-5-fluoropiperidin-1-yl)methanone (610);
((3R,5R)-3-Amino-5-fluoropiperidin-1-yl)(2-(1-(cyclopropylmethyl)-7-(2-(2-methyl-1H-imidazol-1-yl)ethoxy)-1H-pyrrolo[2,3-c]pyridin-2-yl)-3-methylpyrazolo[1,5-a]pyridin-6-yl)methanone (611);
(R)-(3-Aminopiperidin-1-yl)(2-(1-(cyclopropylmethyl)-7-(2-(2-methyl-1H-imidazol-1-yl)ethoxy)-1H-pyrrolo[2,3-c]pyridin-2-yl)-3-methylpyrazolo[1,5-a]pyridin-6-yl)methanone (612);
(2-(7-(2-(4H-1,2,4-Triazol-4-yl)ethoxy)-1-(cyclopropylmethyl)-1H-pyrrolo[2,3-c]pyridin-2-yl)-3-methylpyrazolo[1,5-a]pyridin-6-yl)((3R,5R)-3-amino-5-fluoropiperidin-1-yl)methanone (613);
4-(((2-(6-((3R,5R)-3-Amino-5-fluoropiperidine-1-carbonyl)-3-methylpyrazolo[1,5-a]pyridin-2-yl)-1-(cyclopropylmethyl)-1H-pyrrolo[2,3-c]pyridin-7-yl)oxy)methyl)pyrrolidin-2-one (614);
4-(((2-(6-((3R,5R)-3-Amino-5-fluoropiperidine-1-carbonyl)-3-methylpyrazolo[1,5-a]pyridin-2-yl)-1-(cyclopropylmethyl)-1H-pyrrolo[2,3-c]pyridin-7-yl)oxy)methyl)pyrrolidin-2-one (615);
(2-(7-(2-(1H-Imidazol-1-yl)ethoxy)-1-(cyclopropylmethyl)-1H-pyrrolo[2,3-c]pyridin-2-yl)-3-methylpyrazolo[1,5-a]pyridin-6-yl)((3R,5R)-3-amino-5-fluoropiperidin-1-yl)methanone (616);
(R)-(2-(7-(2-(1H-Imidazol-1-yl)ethoxy)-1-(cyclopropylmethyl)-1H-pyrrolo[2,3-c]pyridin-2-yl)-3-methylpyrazolo[1,5-a]pyridin-6-yl)(3-aminopiperidin-1-yl)methanone (617);
4-(((2-(6-((R)-3-Aminopiperidine-1-carbonyl)-3-methylpyrazolo[1,5-a]pyridin-2-yl)-1-(cyclopropylmethyl)-1H-pyrrolo[2,3-c]pyridin-7-yl)oxy)methyl)pyrrolidin-2-one (618);
4-(((2-(6-((R)-3-Aminopiperidine-1-carbonyl)-3-methylpyrazolo[1,5-a]pyridin-2-yl)-1-(cyclopropylmethyl)-1H-pyrrolo[2,3-c]pyridin-7-yl)oxy)methyl)pyrrolidin-2-one (619);
(2-(7-(2-(4H-1,2,4-Triazol-4-yl)ethoxy)-1-methyl-1H-pyrrolo[2,3-c]pyridin-2-yl)-4-methoxy-3-methylpyrazolo[1,5-a]pyridin-6-yl)((3R,5R)-3-amino-5-fluoropiperidin-1-yl)methanone (620); and
((3R,5R)-3-Amino-5-fluoropiperidin-1-yl) (2-(3-(cyclopropylmethyl)-4-(1-(2-hydroxyethyl)-1H-pyrazol-4-yl)benzo[b]thiophen-2-yl)-4-methoxy-3-methylpyrazolo[1,5-a]pyridin-6-yl)methanone (621);
or a stereoisomer, an enantiomer, a diastereomer, a tautomer, or a pharmaceutically acceptable salt thereof.

In one embodiment, the present invention may provide compounds with IC₅₀ values ≤ 4.000 µM, using the RFMS PAD4 functional assay disclosed herein, preferably, IC₅₀ values ≤ 1.000 µM, preferably, IC₅₀ values ≤ 0.500 µM, preferably, IC₅₀ values ≤ 0.100 µM, more preferably, IC₅₀ values ≤ 0.050 µM, more preferably, IC₅₀ values ≤ 0.03 µM, more preferably, IC₅₀ values ≤ 0.02 µM, even more preferably, IC₅₀ values ≤ 0.01 µM.

As defined above and described herein, X₂ is selected from N and CR₄. In some embodiments, X₂ is N. In some embodiments, X₂ is CR₄. In certain embodiments, X₂ is selected from those functional groups depicted in the examples below.

In some embodiments, R₁ is 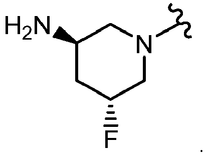 In some embodiments, R₁ is 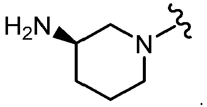 R₁ is selected from

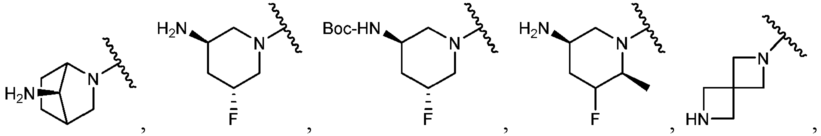

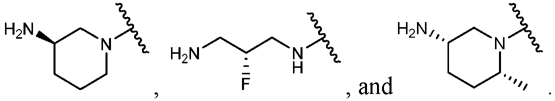

In some embodiments, R₂ is H. In some embodiments, R₂ is F. In some embodiments, R₂ is C₁₋₃ alkyl. In some embodiments, R₂ is methyl. In some embodiments, R₂ is ethyl. In some embodiments, R₂ is propyl. In some embodiments, R₂ is -OCH₃. In some embodiments, R₂ is -OCH₂CH₃. In some embodiments, R₂ is -OCH₂CH₂CH₃. In certain embodiments, R₂ is selected from those functional groups depicted in the examples below. R₂ is preferably selected from H, F, Cl, CH₃, and OCH₃.

In some embodiments, R₃ is CH₃.R₃ is preferably selected from H, F, Cl, CH₃, and CH₂OH.
In some embodiments, R₄ is H.
In some embodiments, R₄ is C₁₋₃ alkyl. In some embodiments, R₄ is CH₃.
In some embodiments, R₄ is selected from 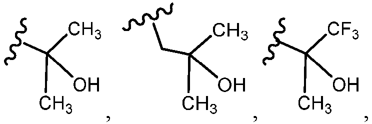 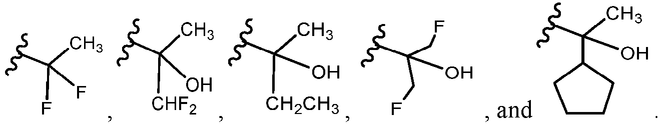 In some embodiments, R₄ is 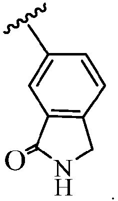 In some embodiments, R₄ is

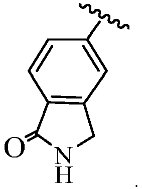

R₄is selected from H, F, Cl, C₁₋₅ alkyl optionally substituted with one or more substituents selected from F, Cl, and OH, C₃₋₆ cycloalkyl,

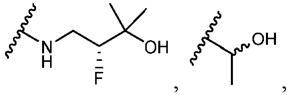

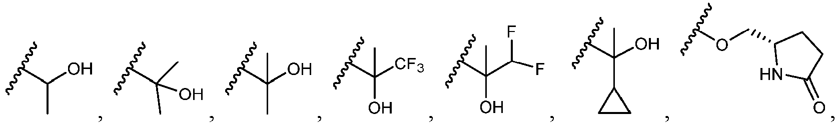

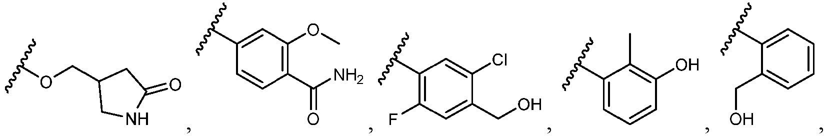

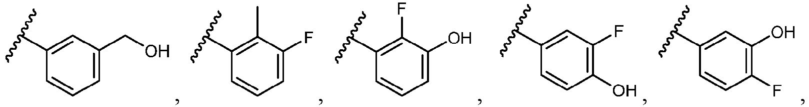

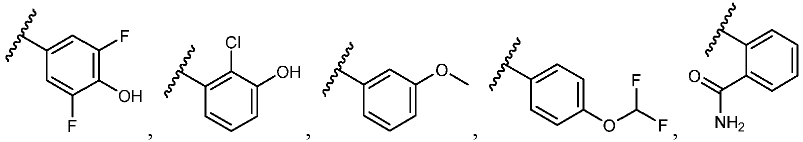

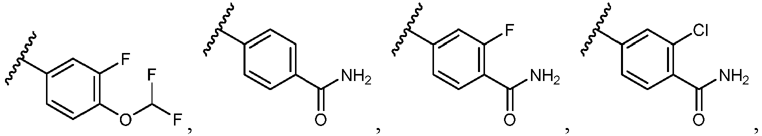

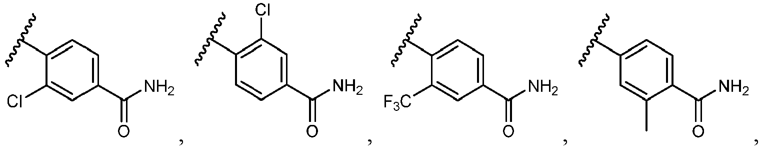

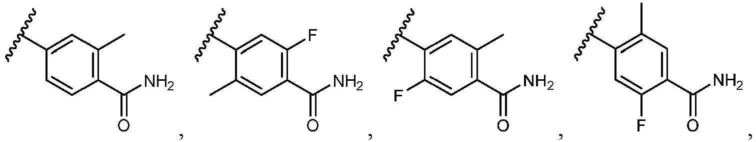

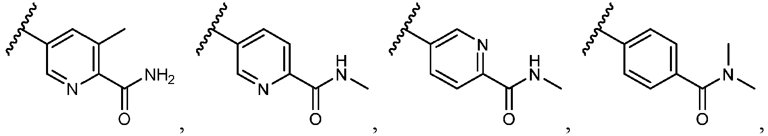

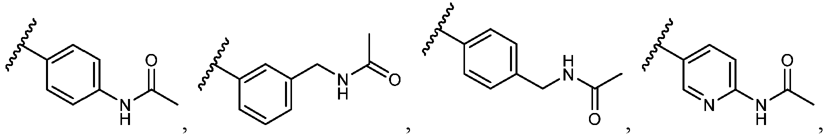

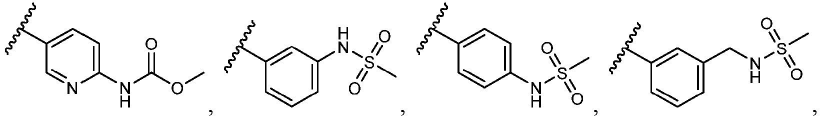

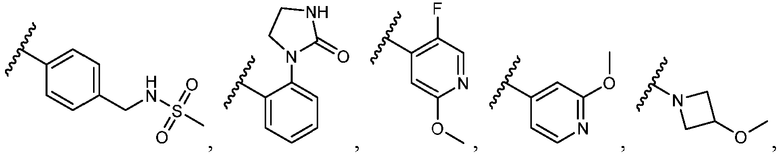

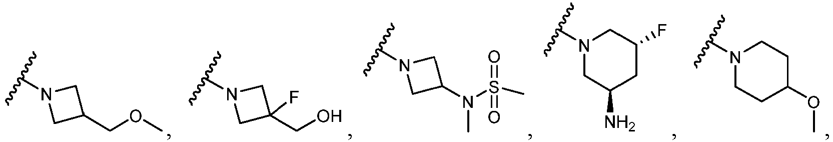

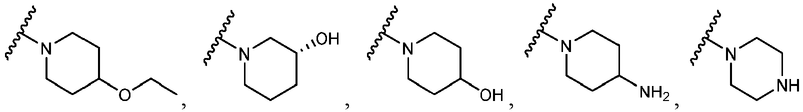

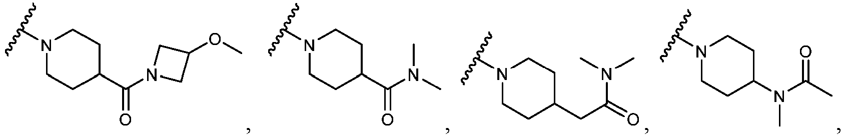

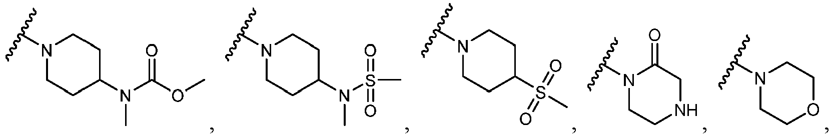

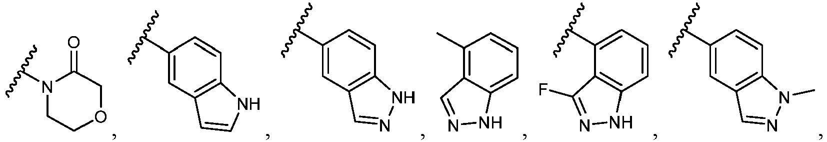

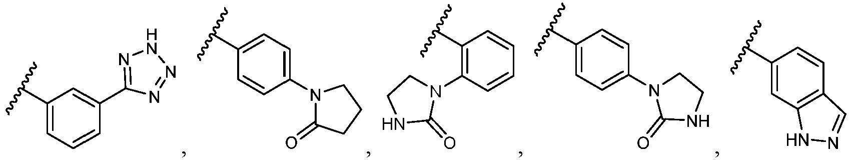

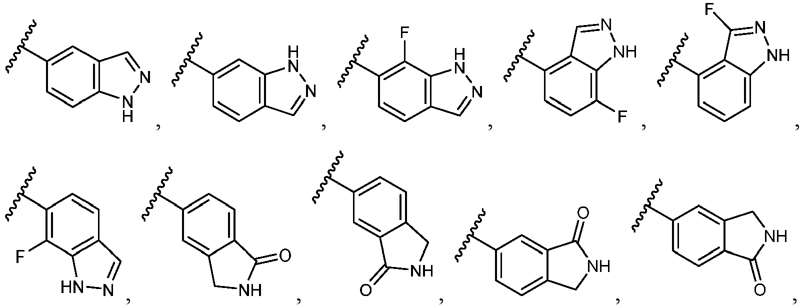

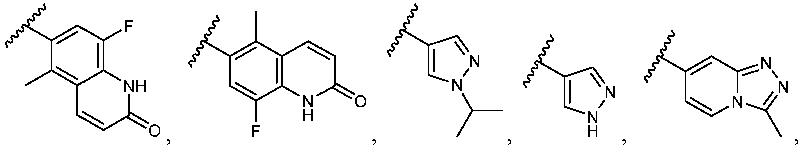

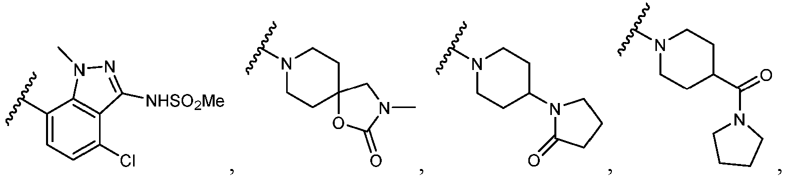

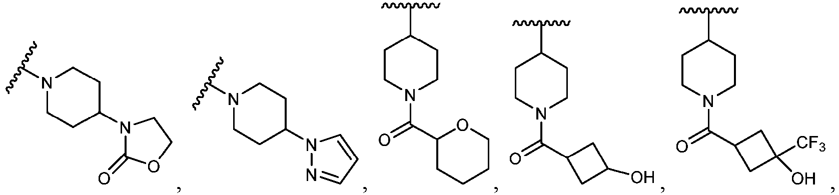

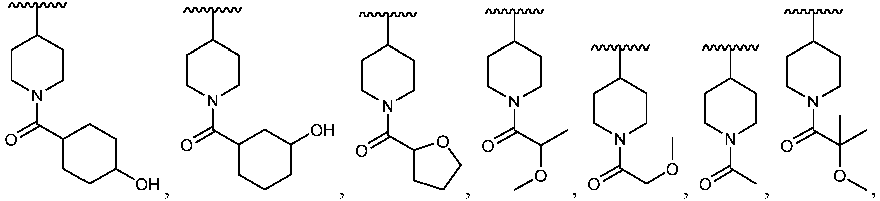

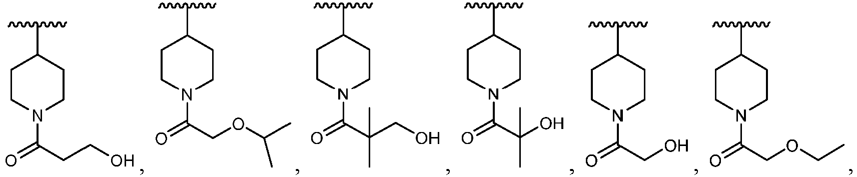

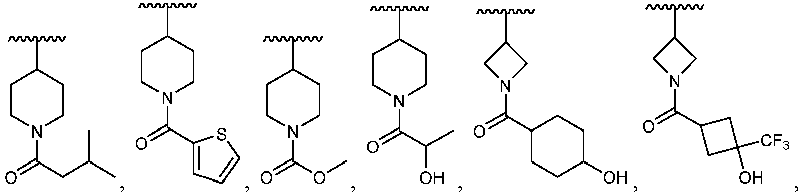

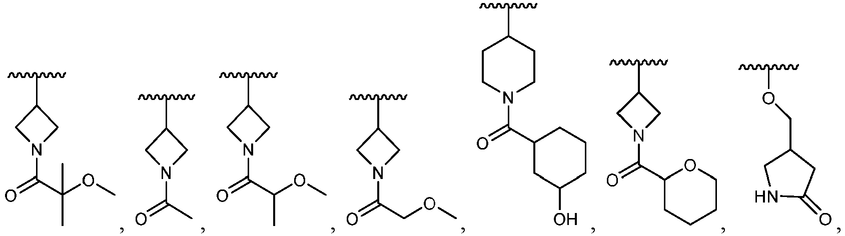

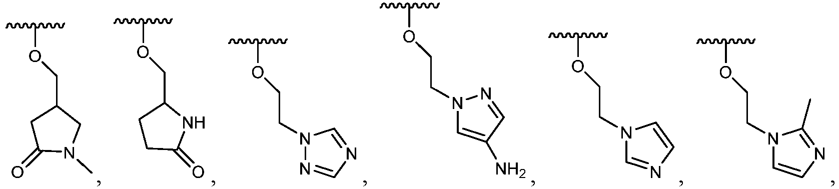

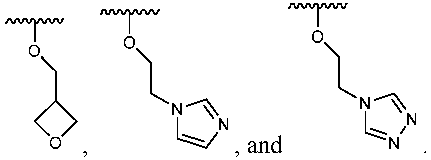

In some embodiments, R₇ is H. R₇ is selected from H, F, and Cl.

As defined above and described herein, R₈ is selected from H and C₁₋₆ alkyl optionally substituted with one or more substitutents selected from F, Cl, and C₃₋₆ cycloalkyl. In some embodiments, R₈ is hydrogen. In some embodiments, R₈ is C₁₋₂ alkyl substituted with C₃₋₆ cycloalkyl. In some embodiments, R₈ is methyl. In some embodiments, R₈ is ethyl. In some embodiments, R₈ is cyclopropyl. Preferably, R₈ is - CH₂-cyclopropyl or -CH₂-cyclobutyl. In certain embodiments, R₈ is selected from those functional groups depicted in the examples below.

As defined above and described herein, R_{b} is independently selected from H, C₁₋₆ alkyl optionally substituted with one or more Rₑ, C₂₋₆ alkenyl optionally substituted with one or more Rₑ, C₂₋₆ alkynyl optionally substituted with one or more Rₑ, -(CH₂)ᵣ-C₃₋₁₀ carbocyclyl optionally substituted with one or more Rₑ, and -(CH₂)ᵣ-heterocyclyl optionally substituted with one or more Rₑ.

As defined above and described herein, Rₑ is independently selected from F, Cl, Br, CN, NH₂, -NH-C₁₋₄ alkyl, -N(C₁₋₄ alkyl)₂, =O, OH, -OC₁₋₆ alkyl, -CO₂H, C₁₋₆ alkyl optionally substituted with one or more R_{f}, C₂₋₆ alkenyl, C₂₋₆ alkynyl, -(CH₂)ᵣ-C₃₋₆ cycloalkyl optionally substituted with one or more R_{f}, -(CH₂)ᵣ-aryl optionally substituted with one or more R_{f}, and -(CH₂)ᵣ-heterocyclyl optionally substituted with one or more R_{f}.

As defined above and described herein, R_{f} is independently selected from F, Cl, Br, CN, OH, C₁₋₅ alkyl optionally substituted with OH, C₂₋₅ alkenyl, C₂₋₅ alkynyl, C₃₋₆ cycloalkyl, and phenyl.

As defined above and described herein, r is 0-4. In some embodiments, r is 0. In some embodiments, r is 1. In some embodiments, r is 2. In some embodiments, r is 3. In some embodiments, r is 4.

In some embodiments, the compound of Formula (Ia) is selected from examples depicted below. In certain embodiments, the present invention provides any compound described above and herein, or a pharmaceutically acceptable salt thereof or a composition for use in therapy. In some embodiments, the present invention provides any compound described above and herein in isolated form. In some embodiments, the present invention provides the compounds according to any one of claims presented below.

### 3. Pharmaceutical Compositions

**In** another aspect, the invention provides a composition comprising a compound of this invention or a stereoisomer, an enantiomer, a diastereomer, a tautomer, or a pharmaceutically acceptable salt thereof, and a pharmaceutically acceptable carrier, adjuvant, or vehicle. The amount of a compound in the compositions of this invention is such that it is effective to measurably inhibit PAD4 in a biological sample or in a patient. A composition of this invention may be formulated for administration to a patient in need of such composition. A composition of this invention may be formulated for oral administration to a patient.

The term "subject," as used herein, is used interchangeably with the term "patient" and means an animal, preferably a mammal.A subject or patient may be a human. A subject (or patient) may be a veterinary subject (or patient).A veterinary subject (or patient) may be a canine, a feline, or an equine subject.

The term "pharmaceutically acceptable carrier, adjuvant, or vehicle" refers to a non-toxic carrier, adjuvant, or vehicle that does not destroy the pharmacological activity of the compound with which it is formulated. Pharmaceutically acceptable carriers, adjuvants or vehicles that may be used in the compositions of this invention include, but are not limited to, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol and wool fat.

Compositions of the present invention may be administered orally, parenterally, by inhalation spray, topically, rectally, nasally, buccally, vaginally or via an implanted reservoir. The term "parenteral" as used herein includes subcutaneous, intravenous, intramuscular, intra-articular, intra-synovial, intrasternal, intrathecal, intrahepatic, intralesional and intracranial injection or infusion techniques. The compositions may be administered orally, intraperitoneally or intravenously. Sterile injectable forms of the compositions of this invention may be aqueous or oleaginous suspension. These suspensions may be formulated according to techniques known in the art using suitable dispersing or wetting agents and suspending agents. The sterile injectable preparation may also be a sterile injectable solution or suspension in a non-toxic parenterally acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium.

For this purpose, any bland fixed oil may be employed including synthetic mono- or di-glycerides. Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions may also contain a long-chain alcohol diluent or dispersant, such as carboxymethyl cellulose or similar dispersing agents that are commonly used in the formulation of pharmaceutically acceptable dosage forms including emulsions and suspensions. Other commonly used surfactants, such as Tweens, Spans and other emulsifying agents or bioavailability enhancers which are commonly used in the manufacture of pharmaceutically acceptable solid, liquid, or other dosage forms may also be used for the purposes of formulation.

Pharmaceutically acceptable compositions of this invention may be orally administered in any orally acceptable dosage form including, but not limited to, capsules, tablets, aqueous suspensions or solutions. In the case of tablets for oral use, carriers commonly used include lactose and corn starch. Lubricating agents, such as magnesium stearate, are also typically added. For oral administration in a capsule form, useful diluents include lactose and dried cornstarch. When aqueous suspensions are required for oral use, the active ingredient is combined with emulsifying and suspending agents. If desired, certain sweetening, flavoring or coloring agents may also be added.

Alternatively, pharmaceutically acceptable compositions of this invention may be administered in the form of suppositories for rectal administration. These can be prepared by mixing the agent with a suitable non-irritating excipient that is solid at room temperature but liquid at rectal temperature and therefore will melt in the rectum to release the drug. Such materials include cocoa butter, beeswax and polyethylene glycols.

Pharmaceutically acceptable compositions of this invention may also be administered topically, especially when the target of treatment includes areas or organs readily accessible by topical application, including diseases of the eye, the skin, or the lower intestinal tract. Suitable topical formulations are readily prepared for each of these areas or organs. Topical application for the lower intestinal tract can be effected in a rectal suppository formulation (see above) or in a suitable enema formulation. Topically-transdermal patches may also be used. For topical applications, provided pharmaceutically acceptable compositions may be formulated in a suitable ointment containing the active component suspended or dissolved in one or more carriers. Carriers for topical administration of compounds of this invention include, but are not limited to, mineral oil, liquid petrolatum, white petrolatum, propylene glycol, polyoxyethylene, polyoxypropylene compound, emulsifying wax and water.

Alternatively, provided pharmaceutically acceptable compositions can be formulated in a suitable lotion or cream containing the active components suspended or dissolved in one or more pharmaceutically acceptable carriers. Suitable carriers include, but are not limited to, mineral oil, sorbitan monostearate, polysorbate 60, cetyl esters wax, cetearyl alcohol, 2-octyldodecanol, benzyl alcohol and water.

For ophthalmic use, provided pharmaceutically acceptable compositions may be formulated as micronized suspensions in isotonic, pH adjusted sterile saline, or, preferably, as solutions in isotonic, pH adjusted sterile saline, either with or without a preservative such as benzylalkonium chloride. Alternatively, for ophthalmic uses, the pharmaceutically acceptable compositions may be formulated in an ointment such as petrolatum.

Pharmaceutically acceptable compositions of this invention may also be administered by nasal aerosol or inhalation. Such compositions are prepared according to techniques well-known in the art of pharmaceutical formulation and may be prepared as solutions in saline, employing benzyl alcohol or other suitable preservatives, absorption promoters to enhance bioavailability, fluorocarbons, and/or other conventional solubilizing or dispersing agents.

Preferably, pharmaceutically acceptable compositions of this invention are formulated for oral administration. Such formulations may be administered with or without food. Pharmaceutically acceptable compositions of this invention may be administered without food.Pharmaceutically acceptable compositions of this invention can also be administered with food.

Pharmaceutically acceptable compositions of this invention can be administered to humans and other animals orally, rectally, parenterally, intracisternally, intravaginally, intraperitoneally, topically (as by powders, ointments, or drops), bucally, as an oral or nasal spray, or the like, as required. The compounds of the invention may be administered orally or parenterally at dosage levels of about 0.01 mg/kg to about 50 mg/kg and preferably from about 1 mg/kg to about 25 mg/kg, of subject body weight per day, one or more times a day, to obtain the desired therapeutic effect.

Liquid dosage forms for oral administration include, but are not limited to, pharmaceutically acceptable emulsions, microemulsions, solutions, suspensions, syrups and elixirs. In addition to the active compounds, the liquid dosage forms may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethylformamide, oils (in particular, cottonseed, groundnut, corn, germ, olive, castor, and sesame oils), glycerol, tetrahydrofurfuryl alcohol, polyethylene glycols and fatty acid esters of sorbitan, and mixtures thereof. Besides inert diluents, the oral compositions can also include adjuvants such as wetting agents, emulsifying and suspending agents, sweetening, flavoring, and perfuming agents.

Injectable preparations, for example, sterile injectable aqueous or oleaginous suspensions may be formulated according to the known art using suitable dispersing or wetting agents and suspending agents. The sterile injectable preparation may also be a sterile injectable solution, suspension or emulsion in a nontoxic parenterally acceptable diluent or solvent, for example, as a solution in 1,3-butanediol. Among the acceptable vehicles and solvents that may be employed are water, Ringer's solution, U.S.P. and isotonic sodium chloride solution. In addition, sterile, fixed oils are conventionally employed as a solvent or suspending medium. For this purpose any bland fixed oil can be employed including synthetic mono- or diglycerides. In addition, fatty acids such as oleic acid may also be used in the preparation of injectables.

Injectable formulations can be sterilized, for example, by filtration through a bacterial-retaining filter, or by incorporating sterilizing agents in the form of sterile solid compositions which can be dissolved or dispersed in sterile water or other sterile injectable medium prior to use.

In order to prolong the effect of a compound of the present invention, it is often desirable to slow the absorption of the compound from subcutaneous or intramuscular injection. This may be accomplished by the use of a liquid suspension of crystalline or amorphous material with poor water solubility. The rate of absorption of the compound then depends upon its rate of dissolution that, in turn, may depend upon crystal size and crystalline form. Alternatively, delayed absorption of a parenterally administered compound form is accomplished by dissolving or suspending the compound in an oil vehicle. Injectable depot forms are made by forming microencapsule matrices of the compound in biodegradable polymers such as polylactide-polyglycolide. Depending upon the ratio of compound to polymer and the nature of the particular polymer employed, the rate of compound release can be controlled. Examples of other biodegradable polymers include poly(orthoesters) and poly(anhydrides). Depot injectable formulations are also prepared by entrapping the compound in liposomes or microemulsions that are compatible with body tissues.

Compositions for rectal or vaginal administration are preferably suppositories which can be prepared by mixing the compounds of this invention with suitable non-irritating excipients or carriers such as cocoa butter, polyethylene glycol or a suppository wax which are solid at ambient temperature but liquid at body temperature, and therefore melt in the rectum or vaginal cavity and release the active compound.

Solid dosage forms for oral administration include capsules, tablets, pills, powders, and granules. In such solid dosage forms, the active compound is mixed with at least one inert, pharmaceutically acceptable excipient or carrier such as sodium citrate or dicalcium phosphate and/or (a) fillers or extenders such as starches, lactose, sucrose, glucose, mannitol, and silicic acid, (b) binders such as, for example, carboxymethylcellulose, alginates, gelatin, polyvinylpyrrolidinone, sucrose, and acacia, (c) humectants such as glycerol, (d) disintegrating agents such as agar-agar, calcium carbonate, potato or tapioca starch, alginic acid, certain silicates, and sodium carbonate, (e) solution retarding agents such as paraffin, (f) absorption accelerators such as quaternary ammonium compounds, (g) wetting agents such as, for example, cetyl alcohol and glycerol monostearate, (h) absorbents such as kaolin and bentonite clay, and (i) lubricants such as talc, calcium stearate, magnesium stearate, solid polyethylene glycols, sodium lauryl sulfate, and mixtures thereof. In the case of capsules, tablets and pills, the dosage form may also comprise buffering agents.

Solid compositions of a similar type may also be employed as fillers in soft and hard-filled gelatin capsules using such excipients as lactose or milk sugar as well as high molecular weight polyethylene glycols. The solid dosage forms of tablets, dragees, capsules, pills, and granules can be prepared with coatings and shells such as enteric coatings and other coatings well known in the pharmaceutical formulating art. They may optionally contain opacifying agents and can also be of a composition that they release the active ingredient(s) only, or preferentially, in a certain part of the intestinal tract, optionally, in a delayed manner. Examples of embedding compositions that can be used include polymeric substances and waxes. Solid compositions of a similar type may also be employed as fillers in soft and hard-filled gelatin capsules using such excipients as lactose or milk sugar as well as high molecular weight polethylene glycols.

The active compounds can also be in micro-encapsulated form with one or more excipients as noted above. The solid dosage forms of tablets, dragees, capsules, pills, and granules can be prepared with coatings and shells such as enteric coatings, release controlling coatings and other coatings well known in the pharmaceutical formulating art. In such solid dosage forms the active compound may be admixed with at least one inert diluent such as sucrose, lactose or starch. Such dosage forms may also comprise, as is normal practice, additional substances other than inert diluents, e.g., tableting lubricants and other tableting aids such a magnesium stearate and microcrystalline cellulose. In the case of capsules, tablets and pills, the dosage forms may also comprise buffering agents.

Dosage forms for topical or transdermal administration of a compound of this invention include ointments, pastes, creams, lotions, gels, powders, solutions, sprays, inhalants or patches. The active component is admixed under sterile conditions with a pharmaceutically acceptable carrier and any needed preservatives or buffers as may be required. Ophthalmic formulation, ear drops, and eye drops are also contemplated as being within the scope of this invention. Additionally, the present invention contemplates the use of transdermal patches, which have the added advantage of providing controlled delivery of a compound to the body. Such dosage forms can be made by dissolving or dispensing the compound in the proper medium. Absorption enhancers can also be used to increase the flux of the compound across the skin. The rate can be controlled by either providing a rate controlling membrane or by dispersing the compound in a polymer matrix or gel.

The amount of compounds of the present invention that may be combined with the carrier materials to produce a composition in a single dosage form will vary depending upon the host treated, the particular mode of administration. Preferably, provided compositions should be formulated so that a dosage of between 0.01 - 100 mg/kg body weight/day of the inhibitor can be administered to a patient receiving these compositions.

A compound of the current invention can be administered alone or in combination with one or more other therapeutic compounds, possible combination therapy taking the form of fixed combinations or the administration of a compound of the invention and one or more other therapeutic compounds being staggered or given independently of one another, or the combined administration of fixed combinations and one or more other therapeutic compounds. Exemplary of such other therapeutic agents include corticosteroids, rolipram, calphostin, cytokine-suppressive anti-inflammatory drugs (CSAIDs), Interleukin-10, glucocorticoids, salicylates, nitric oxide, and other immunosuppressants; nuclear translocation inhibitors, such as deoxyspergualin (DSG); non-steroidal antiinflammatory drugs (NSAIDs) such as ibuprofen, celecoxib and rofecoxib; steroids such as prednisone or dexamethasone; antiviral agents such as abacavir; antiproliferative agents such as methotrexate, leflunomide, FK506 (tacrolimus, Prograf); cytotoxic drugs such as azathiprine and cyclophosphamide; TNF-α inhibitors such as tenidap, anti-TNF antibodies or soluble TNF receptor, and rapamycin (sirolimus or Rapamune) or derivatives thereof. A compound of the current invention can besides or in addition be administered especially for tumor therapy in combination with chemotherapy, radiotherapy, immunotherapy, phototherapy, surgical intervention, or a combination of these. Long-term therapy is equally possible as is adjuvant therapy in the context of other treatment strategies, as described above. Other possible treatments are therapy to maintain the patient's status after tumor regression, or even chemopreventive therapy, for example in patients at risk.

Those additional agents may be administered separately from an inventive compound-containing composition, as part of a multiple dosage regimen. Alternatively, those agents may be part of a single dosage form, mixed together with a compound of this invention in a single composition. If administered as part of a multiple dosage regime, the two active agents may be submitted simultaneously, sequentially or within a period of time from one another normally within five hours from one another.

As used herein, the term "combination," "combined," and related terms refer to the simultaneous or sequential administration of therapeutic agents in accordance with this invention. For example, a compound of the present invention may be administered with another therapeutic agent simultaneously or sequentially in separate unit dosage forms or together in a single unit dosage form. Accordingly, the present invention may provide a single unit dosage form comprising a compound of the current invention, an additional therapeutic agent, and a pharmaceutically acceptable carrier, adjuvant, or vehicle.

The amount of both an inventive compound and additional therapeutic agent (in those compositions which comprise an additional therapeutic agent as described above) that may be combined with the carrier materials to produce a single dosage form will vary depending upon the host treated and the particular mode of administration. Preferably, compositions of this invention should be formulated so that a dosage of between 0.01 - 100 mg/kg body weight/day of an inventive compound can be administered.

In those compositions which comprise an additional therapeutic agent, that additional therapeutic agent and the compound of this invention may act synergistically. Therefore, the amount of additional therapeutic agent in such compositions will be less than that required in a monotherapy utilizing only that therapeutic agent.

The amount of additional therapeutic agent present in the compositions of this invention will be no more than the amount that would normally be administered in a composition comprising that therapeutic agent as the only active agent. Preferably, the amount of additional therapeutic agent in the presently disclosed compositions will range from about 50% to 100% of the amount normally present in a composition comprising that agent as the only therapeutically active agent.

It should also be understood that a specific dosage and treatment regimen for any particular patient will depend upon a variety of factors, including the activity of the specific compound employed, the age, body weight, general health, sex, diet, time of administration, rate of excretion, drug combination, and the judgment of the treating physician and the severity of the particular disease or disorder being treated. The amount of a compound of the present invention in the composition will also depend upon the particular compound in the composition.

### 4. Uses of Compounds

Compounds and compositions described herein are generally useful for the inhibition of PAD4.

The activity of a compound utilized in this invention as an inhibitor of PAD4, may be assayed *in vitro, in vivo* or in a cell line. *In vitro* assays include assays that determine the inhibition of PAD4. Detailed conditions for assaying a compound utilized in this invention as an inhibitor of PAD4 are set forth in the Examples below. A provided compound may inhibit PAD4 selectively as compared to PAD2.

As used herein, the terms "treatment," "treat," and "treating" refer to reversing, alleviating, delaying the onset of, or inhibiting the progress of a disease or disorder, or one or more symptoms thereof, as described herein. In some embodiments, treatment may be administered after one or more symptoms have developed. In other embodiments, treatment may be administered in the absence of symptoms. For example, treatment may be administered to a susceptible individual prior to the onset of symptoms (e.g., in light of a history of symptoms and/or in light of genetic or other susceptibility factors). Treatment may also be continued after symptoms have resolved, for example to prevent or delay their recurrence.

Provided compounds are inhibitors of PAD4 and are therefore useful for treating one or more diseases or disorders associated with PAD4 enzyme activity. Thus, the compounds of the present invention may be used in a method for treating a disease or a disorder associated with PAD4 enzyme activity, comprising the step of administering to a patient in need thereof a compound of the present invention, or a pharmaceutically acceptable composition thereof.

A disease or a disorder associated with PAD4 enzyme activity may be a disease, condition, or disorder mediated by inappropriate PAD4 activity. A disease or a disorder associated with PAD4 enzyme activity may be selected from the group consisting of rheumatoid arthritis, vasculitis, systemic lupus erythematosus, ulcerative colitis, cancer, cystic fibrosis, asthma, cutaneous lupus erythematosus, and psoriasis. The disease or a disorder associated with PAD4 enzyme activity may be rheumatoid arthritis. The disease or a disorder associated with PAD4 enzyme activity may be systemic lupus.The disease or a disorder associated with PAD4 enzyme activity may be vasculitis. The disease or a disorder associated with PAD4 enzyme activity may be cutaneous lupus erythematosus. The disease or a disorder associated with PAD4 enzyme activity may be psoriasis.

Compounds of the present invention may be used in a method of treatment of rheumatoid arthritis, vasculitis, systemic lupus erythematosus, ulcerative colitis, cancer, cystic fibrosis, asthma, cutaneous lupus erythematosus, or psoriasis, which method comprises administering to a human subject in need thereof, a therapeutically effective amount of a provided compound or a stereoisomer, an enantiomer, a diastereomer, a tautomer, or a pharmaceutically acceptable salt thereof.

A compound of the present invention may be used in a method of treatment of rheumatoid arthritis, which method comprises administering to a human subject in need thereof, a therapeutically effective amount of a provided compound, a stereoisomer, an enantiomer, a diastereomer, a tautomer, or a pharmaceutically acceptable salt thereof. A compound of the present invention may be used in a method of treatment of systemic lupus, which method comprises administering to a human subject in need thereof, a therapeutically effective amount of a provided compound, a stereoisomer, an enantiomer, a diastereomer, a tautomer, or a pharmaceutically acceptable salt thereof. A compound of the present invention may be used in a method of treatment of vasculitis, which method comprises administering to a human subject in need thereof, a therapeutically effective amount of a provided compound, a stereoisomer, an enantiomer, a diastereomer, a tautomer, or a pharmaceutically acceptable salt thereof. A compound of the present invention may be used in a method of treatment of cutaneous lupus erythematosus, which method comprises administering to a human subject in need thereof, a therapeutically effective amount of a provided compound, a stereoisomer, an enantiomer, a diastereomer, a tautomer, or a pharmaceutically acceptable salt thereof. A compound of the present inventoion may be used in a method of treatment of psoriasis, which method comprises administering to a human subject in need thereof, a therapeutically effective amount of a provided compound, a stereoisomer, an enantiomer, a diastereomer, a tautomer, or a pharmaceutically acceptable salt thereof.

A disease or a disorder associated with PAD4 enzyme activity may be selected from the group consisting of acid-induced lung injury, acne (PAPA), acute lymphocytic leukemia, acute respiratory distress syndrome, Addison's disease, adrenal hyperplasia, adrenocortical insufficiency, ageing, AIDS, alcoholic hepatitis, alcoholic liver disease, allergen induced asthma, allergic bronchopulmonary, aspergillosis, allergic conjunctivitis, alopecia, Alzheimer's disease, amyloidosis, amyotropic lateral sclerosis, weight loss, angina pectoris, angioedema, anhidrotic ecodermal dysplasia-ID, ankylosing spondylitis, anterior segment, inflammation, antiphospholipid syndrome, aphthous stomatitis, appendicitis, arthritis, asthma, atherosclerosis, atopic dermatitis, autoimmune diseases, autoimmune hepatitis, bee sting-induced inflammation, Bechet's disease, Bechet's syndrome, Bells Palsey, berylliosis, Blau syndrome, bone pain, bronchiolitis, burns, bursitis, cancer, cardiac hypertrophy, carpal tunnel syndrome, catabolic disorders, cataracts, cerebral aneurysm, chemical irritant-induced inflammation, chorioretinitis, chronic heart failure, chronic lung disease of prematurity, chronic lymphocytic leukemia, chronic obstructive pulmonary disease, colitis, complex regional pain syndrome, connective tissue disease, corneal ulcer, crohn's disease, cryopyrin-associated periodic syndromes, cyrptococcosis, cystic fibrosis, deficiency of the interleukin-1-receptor antagonist (DIRA), dermatitis, dermatitis endotoxemia, dermatomyositis, diffuse intrinsic pontine glioma, endometriosis, endotoxemia, epicondylitis, erythroblastopenia, familial amyloidotic polyneuropathy, familial cold urticarial, familial Mediterranean fever, fetal growth retardation, glaucoma, glomerular disease, glomerular nephritis, gout, gouty arthritis, graft-versus-host disease, gut diseases, head injury, headache, hearing loss, heart disease, hemolytic anemia, Henoch-Scholein purpura, hepatitis, hereditary periodic fever syndrome, herpes zoster and simplex, HIV-1, Hodgkin's disease, Huntington's disease, hyaline membrane disease, hyperammonemia, hypercalcemia, hypercholesterolemia, hyperimmunoglobulinemia D with recurrent fever (HIDS), hypoplastic and other anemias, hypoplastic anemia, idiopathic thrombocytopenic purpura, incontinentia pigmenti, infectious mononucleosis, inflammatory bowel disease, inflammatory lung disease, inflammatory neuropathy, inflammatory pain, insect bite-induced inflammation, iritis, irritant-induced inflammation, ischemia/reperfusion, juvenile rheumatoid arthritis, keratitis, kidney disease, kidney injury caused by parasitic infections, kidney injury caused by parasitic infections, kidney transplant rejection prophylaxis, leptospiriosis, leukemia, Loeffler's syndrome, lung injury, lupus, lupus nephritis, lymphoma, meningitis, mesothelioma, mixed connective tissue disease, Muckle-Wells syndrome (urticaria deafness amyloidosis), multiple sclerosis, muscle wasting, muscular dystrophy, myasthenia gravis, myocarditis, mycosis fungoides, myelodysplastic syndrome, myositis, nasal sinusitis, necrotizing enterocolitis, neonatal onset multisystem inflammatory disease (NOMID), nephrotic syndrome, neuritis, neuropathological diseases, non-allergen induced asthma, obesity, ocular allergy, optic neuritis, organ transplant, osteoarthritis, otitis media, Paget's disease, pain, pancreatitis, Parkinson's disease, pemphigus, pericarditis, periodic fever, periodontitis, peritoneal endometriosis, pertussis, pharyngitis and adenitis (PFAPA syndrome), plant irritant-induced inflammation, pneumonia, pneumonitis, pneumosysts infection, poison ivy/ urushiol oil-induced inflammation, polyarteritis nodosa, polychondritis, polycystic kidney disease, polymyositis, psoriasis, psychosocial stress diseases, pulmonary disease, pulmonary hypertension, pulmonary fibrosis, pyoderma gangrenosum, pyogenic sterile arthritis, renal disease, retinal disease, rheumatic carditis, rheumatic disease, rheumatoid arthritis, sarcoidosis, seborrhea, sepsis, severe pain, sickle cell, sickle cell anemia, silica-induced disease, Sjogren's syndrome, skin diseases, sleep apnea, solid tumors, spinal cord injury, Stevens-Johnson syndrome, stroke, subarachnoid hemorrhage, sunburn, temporal arteritis, tenosynovitis, thrombocytopenia, thyroiditis, tissue transplant, TNF receptor associated periodic syndrome (TRAPS), toxoplasmosis, transplant, traumatic brain injury, tuberculosis, type 1 diabetes, type 2 diabetes, ulcerative colitis, urticarial, uveitis, Wegener's granulomatosis, interstitial lung disease, psoriatic arthritis, juvenile idiopathic arthritis, Sjögren's syndrome, antineutrophil cytoplasmic antibody (ANCA)-associated vasculitis, antiphospholipid antibody syndrome, sepsis, deep vein thrombosis, fibrosis, Alzheimer's, scleroderma and CREST syndrome.

In one embodiment, the invention provides a compound, or a pharmaceutically acceptable salt thereof, for use in therapy.A compound of the present invention, or a pharmaceutically acceptable salt thereof, may be used in a method of treatment of a disease or a disorder mediated by inappropriate PAD4 activity. A compound of the present invention, or a stereoisomer, an enantiomer, a diastereomer, a tautomer, or a pharmaceutically acceptable salt thereof, may be used in a method of treatment of rheumatoid arthritis, vasculitis, systemic lupus erythematosus, ulcerative colitis, cancer, cystic fibrosis, asthma, cutaneous lupus erythematosus, or psoriasis. A compound of the present invention, the invention provides a compound, or a stereoisomer, an enantiomer, a diastereomer, a tautomer, or a pharmaceutically acceptable salt thereof, may be used in a method of treatment of rheumatoid arthritis. A compound of the present invention, or a stereoisomer, an enantiomer, a diastereomer, a tautomer, or a pharmaceutically acceptable salt thereof, may be used in a method of treatment of systemic lupus.A compound of the present invention, or a stereoisomer, an enantiomer, a diastereomer, a tautomer, or a pharmaceutically acceptable salt thereof, may be used in a method of treatment of vasculitis. A compound of the present invention, or a stereoisomer, an enantiomer, a diastereomer, a tautomer, or a pharmaceutically acceptable salt thereof, may be used in a method of treatment of cutaneous lupus erythematosus.A compound of the present invention, or a stereoisomer, an enantiomer, a diastereomer, a tautomer, or a pharmaceutically acceptable salt thereof, may be used in a method of treatment of psoriasis. A compound of the present invention, or a stereoisomer, an enantiomer, a diastereomer, a tautomer, or a pharmaceutically acceptable salt thereof, may be used in the manufacture of a medicament for use in the treatment of a disorder mediated by inappropriate PAD4 activity.A compound of the present invention, or a stereoisomer, an enantiomer, a diastereomer, a tautomer, or a pharmaceutically acceptable salt thereof, may be used in the manufacture of a medicament for use in the treatment of rheumatoid arthritis, vasculitis, systemic lupus erythematosus, ulcerative colitis, cancer, cystic fibrosis, asthma, cutaneous lupus erythematosus, or psoriasis. A compound of the present invention, or a stereoisomer, an enantiomer, a diastereomer, a tautomer, or a pharmaceutically acceptable salt thereof, may be used in the manufacture of a medicament for use in the treatment of rheumatoid arthritis. A compound of the present invention, or a stereoisomer, an enantiomer, a diastereomer, a tautomer, or a pharmaceutically acceptable salt thereof, may be used in the manufacture of a medicament for use in the treatment of systemic lupus.A compound of the present invention, or a stereoisomer, an enantiomer, a diastereomer, a tautomer, or a pharmaceutically acceptable salt thereof, may be used in the manufacture of a medicament for use in the treatment of vasculitis. A compound of the present invention, or a stereoisomer, an enantiomer, a diastereomer, a tautomer, or a pharmaceutically acceptable salt thereof, may be used in the manufacture of a medicament for use in the treatment of cutaneous lupus erythematosus.A compound of the present invention, or a stereoisomer, an enantiomer, a diastereomer, a tautomer, or a pharmaceutically acceptable salt thereof, may be used in the manufacture of a medicament for use in the treatment of psoriasis. A pharmaceutical composition of the pesent invention may be used for the treatment or prophylaxis of a disease or a disorder mediated by inappropriate PAD4 activity comprising a provided compound, or a stereoisomer, an enantiomer, a diastereomer, a tautomer, or a pharmaceutically acceptable salt thereof. A pharmaceutical composition of the pesent invention may be used for the treatment or prophylaxis of rheumatoid arthritis, vasculitis, systemic lupus erythematosus, ulcerative colitis, cancer, cystic fibrosis, asthma, cutaneous lupus erythematosus, or psoriasis, comprising a provided compound, or a stereoisomer, an enantiomer, a diastereomer, a tautomer, or a pharmaceutically acceptable salt thereof. A pharmaceutical composition of the pesent invention may be used for the treatment or prophylaxis of rheumatoid arthritis comprising a provided compound, or a stereoisomer, an enantiomer, a diastereomer, a tautomer, or a pharmaceutically acceptable salt thereof. A pharmaceutical composition of the pesent invention may be used for the treatment or prophylaxis of systemic lupus comprising a provided compound, or a stereoisomer, an enantiomer, a diastereomer, a tautomer, or a pharmaceutically acceptable salt thereof. A pharmaceutical composition of the pesent invention may be used for the treatment or prophylaxis of vasculitis comprising a provided compound, or a stereoisomer, an enantiomer, a diastereomer, a tautomer, or a pharmaceutically acceptable salt thereof. or the treatment or prophylaxis of cutaneous lupus erythematosus comprising a provided compound, or a stereoisomer, an enantiomer, a diastereomer, a tautomer, or a pharmaceutically acceptable salt thereof. A pharmaceutical composition of the pesent invention may be used for the treatment or prophylaxis of psoriasis comprising a provided compound, or a stereoisomer, an enantiomer, a diastereomer, a tautomer, or a pharmaceutically acceptable salt thereof.

All features of each of the aspects of the invention apply to all other aspects mutatis mutandis. In order that the invention described herein may be more fully understood, the following examples are set forth. It should be understood that these examples are for illustrative purposes only and are not to be construed as limiting this invention in any manner.

### 5. Examples

The following Examples have been prepared, isolated and characterized using the methods disclosed herein. The following examples demonstrate a partial scope of the invention and are not meant to be limiting of the scope of the invention.

Details of various HPLC/LC-MS methods used in the analysis of compounds and intermediates is provided below.

Method A: SUNFIRE C18 (4.6×150) mm, 3.5 µm column; flow rate 1 ml/min; gradient time 15 min; 10-100% solvent B; monitoring at 254 nm and 220 nm (solvent A: 5% acetonitrile (ACN), 95% water, 0.05% trifluoroacetic acid (TFA); solvent B: 95% ACN, 5% water, 0.05% TFA).

Method B: X-Bridge Phenyl (4.6×150) mm, 3.5 µm column; flow rate 1 ml/min; gradient time 15 min;% 10-100% solvent B; monitoring at 254 nm and 220 nm (solvent A: 5% ACN, 95% water, 0.05% TFA; solvent B: 95% ACN, 5% water, 0.05% TFA).

Method C: Kinetex EVO C18 (4.6×100) mm, 2.6 µm, buffer: 0.05% TFA in water, mobile phase C: buffer: ACN (95:5), mobile phase D: ACN: buffer (95:5).

Method D: Kinetex Biphenyl (4.6× 100) mm, 2.6 µm, buffer: 0.05% TFA in water, mobile phase C: buffer: ACN (95:5), mobile phase D: ACN: buffer (95:5).

Method E: XBridge BEH XP C18 (50×2.1) mm, 2.5µm, Time (min): 0-3% B: 0-100 buffer A: 95% water: 5% ACN; 10 mM ammonium acetate (NH₄OAc) B: 5% water: 95% ACN; 10 mM NH₄OAc, flow: 1.1 ml/min, temperature: 50°C.

Method F: XBridge BEH XP C18 (50×2.1) mm, 2.5µm, time (min): 0-3%, B: 0-100, A: 95% water: 5% ACN; 0.1% TFA, B: 5% water: 95% ACN; 0.1%TFA, flow: 1.1 ml/min, temperature: 50°C.

Method G: ACQUITY UPLC^{®} BEH C18 (3×50) mm, 1.7µm, buffer: 10 mM NH₄OAc, mobile phase A: buffer: ACN (95:5), mobile phase B: buffer: ACN (5:95), flow:0.7ml/min, method: 0 min-20% B, 20% to 100% B 2 min, 2 to 2.3min-100%.

Method H: Kinetex XB C18 (75×3) mm, 2.6µm, mobile phase A: 10 mM NH₄OAc in water, water: ACN (98:02), mobile phase B: 10 mM NH₄OAc in water: ACN (02:98), gradient: 20-100%B over 4 min, flow: 1.0ml/min.

Method I: ACQUITY UPLC^{®} BEH C18 (3×50) mm, 1.7µm, buffer: 0.05% TFA in water, mobile phase A: buffer: ACN (95:5), mobile phase B: ACN: buffer (95:5).

Method J: Column: HALO C18, 3x30 mm, 2.7 µm; Mobile Phase A: water+0.05%TFA, Mobile Phase B: acetonitrile+0.05%TFA; Flow rate: 1.5 mL/min; Gradient: 5% B to 95% B in 2.5 min, hold at 95% for 1 min, 95% B to 5% B in 0.05 min; Detection: MS and UV (254 nm).

Method K: Column: Shim-pack XR-ODS, 3×50 mm, 2.2 µm; Mobile Phase A: water/0.05%TFA, Mobile Phase B: acetonitrile/0.05%TFA; Flow Rate: 1.2000 mL/min; Gradient: 5% B to 95% B in 3.3 min, hold at 95% for 0.7 min, 95% B to 5% B in 0.1 min; Detection: MS and UV (254 nm).

General procedure for purification using Reverse Phase HPLC (RP-HPLC) is as follows. Typically, the sample to be purified was dissolved in DCM:MeOH (2:1), 20% by weight of poly(4-vinylpyridine) (Aldrich # 226963) was added to this solution, and the container containing the solution was placed on a shaker for 2 hours. Thereafter, the contents were filtered through a 25 mm syringe filter fitted with a 0.45 µm nylon membrane (VWR # 28145-489) into a clean vial, and dried using centrifugal evaporation to afford the purified sample.

### General synthetic procedures:

### Intermediate 1

### 1-Amino-3-methoxy-5-(methoxycarbonyl)pyridin-1-ium 2,4-dinitrophenolate

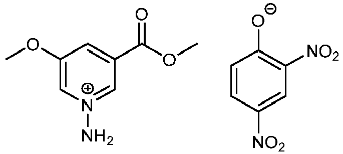

O-(2,4-dinitrophenyl)hydroxylamine (10.01 g, 50.3 mmol) was added to a stirred solution of methyl 5-methoxynicotinate (7 g, 41.9 mmol) in tetrahydrofuran (THF, 50 ml) and water (50 ml). The reaction mixture was heated to 40°C, stirred for 12 hours, and filtered to afford 1-amino-3-methoxy-5-(methoxycarbonyl)pyridin-1-ium (10 g, 130%) as yellowish brown solid, which was used in the next step without further purification. Liquid chromatography-mass spectrometry (LC-MS) m/z: 183.1 [M+H]⁺.

### Intermediate 2

### tert-Butyl-3-(1-(cyclopropylmethyl)-1H-pyrrolo[2,3-b]pyridin-2-yl)propiolate

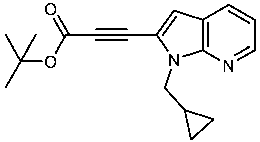

tert-Butyl propiolate (0.605 g, 4.80 mmol) and bis(triphenylphosphine)palladium(II) dichloride (0.153 g, 0.218 mmol) were added to a stirred solution of 1-(cyclopropylmethyl)-2-iodo-1H-pyrrolo[2,3-b]pyridine (1.3 g, 4.36 mmol) in a mixture of triethylamine (TEA, 5 ml) and THF (5 ml). The reaction mixture was heated to 65°C, stirred for 1 hour, and filtered through a celite bed. The filtrate was concentrated under reduced pressure to obtain a crude product, which was purified using a silica gel column (40 g, Redisep^{®} SiO₂ column, eluting with 20% ethyl acetate (EtOAc) in hexane) to afford the title compound (1.2 g, 93%) as a brown liquid. LC-MS m/z: 297.1 [M+H]⁺. ¹H NMR (300 MHz, chloroform-d) δ ppm: 8.55-8.38 (m, 1H), 7.99-7.87 (m, 1H), 7.26-6.99 (m, 1H), 7.04-6.99 (m, 1H), 4.43-4.06 (m, 2H), 3.26-3.16 (m, 1H), 1.65-1.38 (m, 10H), 1.34-1.07 (m, 1H), 0.59-0.39 (m, 4H).

### Intermediate 3

### 6-Chloro-1-(cyclopropylmethyl)-2-iodo-1H-pyrrolo[2,3-b]pyridine

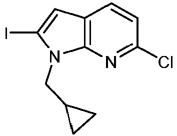

Sodium hydride (2.87 g, 71.8 mmol) was added to a stirred solution of 6-chloro-2-iodo-1H-pyrrolo[2,3-b]pyridine (10 g, 35.9 mmol) in dimethylformamide (DMF, 200 ml) at 0°C and the reaction mixture was stirred for 0.5 hours, followed by addition of (bromomethyl)cyclopropane (4.50 ml, 46.7 mmol). The reaction mixture was stirred for 1 hour, poured into ice water (200 ml), and extracted with ethyl acetate (3×200 ml). The combined organic fractions were washed with water (2×300 ml) and brine (2×200 ml), dried over sodium sulfate (Na₂SO₄), filtered, and concentrated to obtain a crude product, which was purified using a silica gel column (80 g, Redisep^{®} Silica column, 0-50% EtOAc in hexane as an eluent) to afford the title compound (8 g, 67%) as yellow solid. LC-MS m/z: 332.9 [M+H]⁺. ¹H NMR (400 MHz, chloroform-d) δ ppm: 7.75 (d, J=8.1 Hz, 1H), 7.04 (d, J=8.3 Hz, 1H), 6.79 (s, 1H), 1.46-1.26 (m, 1H), 0.61-0.44 (m, 4H).

### Intermediate 4

### tert-Butyl-3-(6-chloro-1-(cyclopropylmethyl)-1H-pyrrolo[2,3-b]pyridin-2-yl)propiolate

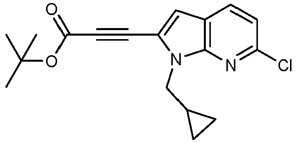

tert-Butyl propiolate (1.669 g, 13.23 mmol) and bis(triphenylphosphine)palladium(II) dichloride (0.422 g, 0.601 mmol) were added to a stirred solution of 6-chloro-1-(cyclopropyl-methyl)-2-iodo-1H-pyrrolo[2,3-b]pyridine (Intermediate 3, 4 g, 12.03 mmol) in a mixture of TEA (10 ml) and THF (10 ml). The reaction mixture was stirred at 50°C for 1 hour, and filtered through a celite bed. The filtrate was concentrated under reduced pressure to obtain a crude compound, which was purified using a silica gel column (40 g, Redisep^{®} SiO₂ column, eluting with 20% EtOAc in hexane) to afford the title compound (2.5 g, 63%) as yellow liquid. LC-MS m/z: 331.1 [M+H]⁺.

### Intermediate 5

### 6-Bromo-1-(cyclopropylmethyl)-2-ethynyl-1H-indole

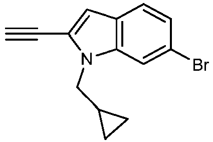

Potassium carbonate (K₂CO₃, 11.18 g, 81 mmol) was added to a stirred solution of dimethyl (1-diazo-2-oxopropyl)phosphonate (51.8 g, 27.0 mmol, 10% solution in acetonitrile) in methanol (MeOH, 50 ml), followed by the addition of 6-bromo-1-(cyclopropylmethyl)-1H-indole-2-carbaldehyde (7.5g, 27.0 mmol) at 0°C. The reaction mixture was allowed to warm to a room temperature, stirred for 6 hours, and concentrated under vacuum to afford a crude residue, which was diluted with water and extracted with EtOAc (2×100 ml). The combined organic layers were dried over Na₂SO₄, filtered, and the filtrate was concentrated under reduced pressure to obtain a crude product, which was purified using a silica gel column (40 g, Redisep^{®} SiO₂ column, eluting with 0-5% EtOAc in hexane) to afford the title compound (6.3g, 85%) as a brown liquid. LC-MS m/z: 276.1 [M+2H]⁺.

### Intermediate 6

### tert-Butyl-3-(6-bromo-1-(cyclopropylmethyl)-1H-indol-2-yl)propiolate

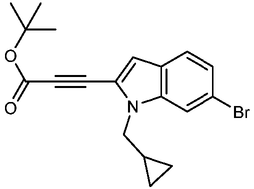

Lithium diisopropylamide (LDA, 17.23 ml, 34.5 mmol) was added to a stirred solution of 6-bromo-1-(cyclopropylmethyl)-2-ethynyl-1H-indole (Intermediate 5, 6.3g, 22.98 mmol) in THF (10 ml) at 0°C, and the reaction mixture was stirred for 30 min, followed by the addition of Boc₂O (5.34 ml, 22.98 mmol) slowly. The reaction mixture was stirred for 1 hour, quenched with water, and extracted with EtOAc (2×100 ml). The combined organic layers were washed with water (2×300 ml) and brine (2×200 ml), dried over Na₂SO₄, filtered, and the filtrate was concentrated under reduced pressure. The crude product obtained was purified using a silica gel column (80 g, Redisep^{®} Silica column, 0-5% EtOAc in hexane as an eluent) to afford the title compound (6.2 g, 72%). LC-MS m/z: 318.0 [M-isobutene+H]⁺. ¹H NMR (400 MHz, DMSO-*d₆*) δ ppm: 7.94 (s, 1H), 7.58 (d, J=8.5 Hz, 1H), 7.26 (dd, J=1.5, 8.5 Hz, 1H), 7.21 (s, 1H), 4.17 (d, J=7.0 Hz, 2H), 1.51 (s, 9H), 1.27-1.18 (m, 1H), 0.52-0.40 (m, 4H).

### Intermediate 7

### 1-(Cyclopropylmethyl)-2-(trimethylstannyl)-1H-pyrrolo[2,3-b]pyridine

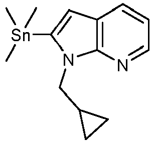

Hexamethylditin (0.278 ml, 1.342 mmol) was added to a stirred solution of 1-(cyclopropyl -methyl)-2-iodo-1*H*-pyrrolo[2,3-*b*]pyridine (0.2 g, 0.671 mmol) in 1,4-dioxane (1.5 ml), and the reaction mixture was degassed with argon for 5 min, followed by the addition of [1,1'-bis(di-tert-butylphosphino)-ferrocene]-dichloropalladium(II) (0.031 g, 0.047 mmol). The reaction mixture was stirred at 70°C for 2 hours, filtered through a celite bed, and the filtrate was concentrated to afford a crude product (250 mg), which was used in the next steps without further purification. LC-MS m/z: 337 [M+H]⁺.

### Intermediate 8

### 1-Amino-3-(ethoxycarbonyl)pyridin-1-ium 2,4-dinitrophenolate

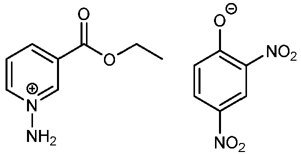

Intermediate 8 was prepared following a procedure similar to the preparation of Intermediate 1 (10 g, 28.5mmol, 100%). LC-MS m/z: 168 [M+H]⁺.

### Intermediate 9

### 1-Amino-3-fluoro-5-(methoxycarbonyl)pyridin-1-ium 2,4,6-trimethylbenzenesulfonate

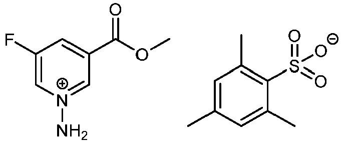

O-(mesitylsulfonyl)hydroxylamine (8.33 g, 38.7 mmol) was added to a stirred solution of methyl 5-fluoronicotinate (5 g, 32.2 mmol) in dichloromethane (DCM, 20 ml) at 0°C. The reaction mixture was stirred for 2 hours, and concentrated to afford 1-amino-3-fluoro-5-(methoxycarbonyl)pyridin-1-ium (5 g, 91%) as a brown solid, which was used in the next step without further purification. LC-MS m/z: 171.0 [M+H]⁺.

### Intermediate 10

### 1-(Cyclopropylmethyl)-2-(4,4,5,5-tetramethyl-1,3,2-dioxaborolan-2-yl)-1H-indole

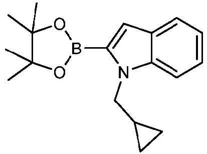

sec-Butyllithium (16.68 ml, 23.36 mmol) was added drop wise to a stirred solution of 1-(cyclopropylmethyl)-1H-indole (2 g, 11.68 mmol) in THF (20 ml) under argon atmosphere at -78°C. The reaction mixture was stirred further for 30 min, followed by the addition of 2-isopropoxy-4,4,5,5-tetramethyl-1,3,2-dioxaborolane (2.173 g, 11.68 mmol). The reaction mixture was stirred at a room temperature for 2 hours, quenched with drop wise addition of water (50 ml), and extracted with EtOAc (2×50 ml). The combined organic extracts were washed with water (100 ml) and brine (100 ml), dried over Na₂SO₄, filtered, and the filtrate was evaporated to dryness under reduced pressure. The crude product thus obtained was purified using a silica gel column column (EtOAc in hexane), and the purified product was isolated at 15% EtOAc in hexane. Required fractions were collected and volatiles were evaporated to dryness under reduced pressure to afford the title compound (2 g, 58%). ¹H NMR (400 MHz, DMSO-*d₆*) δ ppm: 0.32-0.44 (m, 4 H), 1.16-1.34 (m, 13 H), 4.32 (d, J=6.85 Hz, 2 H), 6.94-7.05 (m, 2 H), 7.16-7.25 (m, 1 H), 7.51-7.60 (m, 2 H).

### Intermediate 11

### 1-(Cyclopropylmethyl)-2-(4,4,5,5-tetramethyl-1,3,2-dioxaborolan-2-yl)-1H-pyrrolo[2,3-b]pyridine

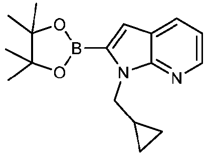

Intermediate 11 was prepared following a procedure similar to the preparation of Intermediate 10 (1.3 g, 31%). LC-MS (ESI) m/z: 300.3 [M+H]⁺.

### Intermediate 12

### 6-Bromo-1-(cyclopropylmethyl)-2-iodo-1H-indole

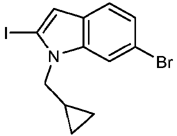

Sodium hydride (NaH, 1.372 g, 34.3 mmol) was added lot wise to a stirred solution of 6-bromo-2-iodo-1H-indole (5.52 g, 17.15 mmol, Ref: *Bioorg. Med. Chem.* 22(5), **2014,** 1708) in DMF (20 ml) at 0°C, and the reaction mixture was stirred for 10 minutes followed by drop wise the addition of (bromomethyl)-cyclopropane (2.498 ml, 25.7 mmol). The reaction mixture was stirred at a room temperature for 16 hours, diluted with cold water (50 ml), and extracted with EtOAc (3×50 ml). The combined organic layers were dried over anhydrous sodium sulphate, filtered, and the filtrate was evaporated under reduced pressure. The crude product thus obtained was purified using a silica gel column (24g, Redisep^{®} SiO₂ column, eluting with 10% EtOAc in n-hexane) to afford the title compound (5g, 78%). LC-MS m/z: 377 [M+H]⁺. ¹H NMR (400 MHz, DMSO-*d₆*) δ ppm: 7.83 (s, 1H), 7.43 (d, J=8.3 Hz, 1H), 7.14 (dd, J=1.8, 8.5 Hz, 1H), 6.82 (s, 1H), 4.13 (d, J=6.8 Hz, 2H), 1.31-1.13 (m, 1H), 0.51-0.41 (m, 4H).

### Intermediate 13

### 1-Amino-3-chloro-5-(methoxycarbonyl)pyridin-1-ium-2,4,6-trimethylbenzenesulfonate

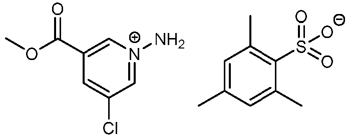

O-(mesitylsulfonyl)hydroxylamine (2.6 g, 11.66 mmol) was added to a stirred solution of methyl 5-chloronicotinate (2 g, 11.66 mmol) in DCM (40 ml) at 0°C. The reaction mixture was stirred at a room temperature for 2 hours, and evaporated under reduced pressure to afford crude 1-amino-3-chloro-5-(methoxycarbonyl)pyridin-1-ium (2.1 g, 96%) as a white solid, which was used in the next step without further purification. LC-MS m/z: 187.2 [M+H]⁺.

### Intermediate 14

### Ethyl 6-bromo-1-(cyclopropylmethyl)-1H-indole-2-carboxylate

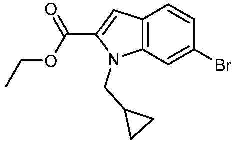

Potassium carbonate (10.3 g, 74.6 mmol) was added to a stirred solution of ethyl 6-bromo-1H-indole-2-carboxylate (10g, 37.3 mmol) in DMF (60 ml), followed by the addition of (bromomethyl)cyclopropane (5.0 g, 37.3 mmol). The reaction mixture was stirred for 16 hours, and potassium carbonate (5 g) and (bromomethyl)cyclopropane (2.5 g) were added. The resulting reaction mixture was heated to 50°C for 2 hours, diluted with water (150 ml), and extracted with ethyl acetate (3×150 ml). The combined organic layers were concentrated in vacuo to afford ethyl 6-bromo-1-(cyclopropylmethyl)-1H-indole-2-carboxylate (10 g, 83%) as a brown solid, which was used further without purification. LC-MS m/z: 322.0 [M+H]⁺.

### Intermediate 15

### (6-Bromo-1-(cyclopropylmethyl)-1H-indol-2-yl)methanol

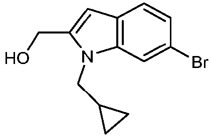

LiBH₄ (15.52 ml, 62.1 mmol, 4 M in THF) was added to a stirred solution of Intermediate 14 (10g, 31 mmol) in THF (50 ml) at 0°C. The reaction mixture was allowed to warm to a room temperature over 1 hour, stirred for 16 hours, cooled to 0°C and additional amount of LiBH₄ (15.52 ml, 62.1 mmol, 4 M in THF) was added dropwise. The reaction mixture was stirred for 6 hours at a room temperature, cooled to 0°C, quenched by slow addition of saturated ammonium chloride solution (need to add carefully, sudden evolution of gas was noticed), stirred for ~ 45 minutes, and extracted with ethyl acetate (4×100 ml). The organic layers were separated, dried over Na₂SO₄, filtered and concentrated to obtain a crude product, which was further purified using a silica gel column (0-30% ethyl acetate / petether) to afford 6-bromo-1-(cyclopropylmethyl)-1H-indol-2-yl)methanol (8.5g, 98%). LC-MS m/z: 280.1 [M+H]⁺.

### Intermediate 16

### 6-Bromo-1-(cyclopropylmethyl)-1H-indole-2-carbaldehyde

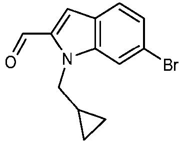

Manganese dioxide (10.3 g, 119 mmol) was added to a stirred solution of Intermediate 15 (8.3g, 29.6 mmol) in THF (50 ml). The reaction mixture was stirred at 45°C for 2 hours, and filtered through a celite bed. The filtrate was concentrated in vacuo to obtain 6-bromo-1-(cyclopropylmethyl)-1H-indole-2-carbaldehyde (7.6g, 92%) as a brown syrup, which was used further without purification. LC-MS m/z: 278.1 [M+H]⁺.

### Intermediate 17

### (E)-6-Bromo-1-(cyclopropylmethyl)-2-(2-nitroprop-1-en-1-yl)-1H-indole

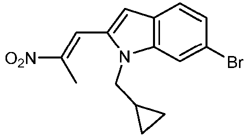

Ammonium acetate (122 mg, 1.582 mmol) was added to a stirred solution of Intermediate 16 (400 mg, 1.438 mmol) in nitroethane (2 ml, 28.0 mmol). The reaction mixture was stirred at 90°C for 2 hours, diluted with water (5ml), and extracted with ethyl acetate (2×10 ml). The combined organic layers were washed with brine, dried over sodium sulfate, filtered, and concentrated in vacuum to afford (E)-6-bromo-1-(cyclopropylmethyl)-2-(2-nitroprop-1-en-1-yl)-1H-indole as yellow liquid (420 mg), which was used in the next step without purification. LC-MS retention time: 1.26 min., m/z =335.1 [M+H]⁺

### Intermediate 18

### Ethyl 2-(6-bromo-1-(cyclopropylmethyl)-1H-indol-2-yl)-3-methylpyrazolo[1,5-a]pyridine-6-carboxylate

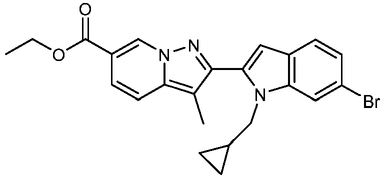

Intermediate 8 (75 mg, 0.447 mmol) and copper (I) iodide (6 mg, 0.03 mmol) were added to a stirred solution of Intermediate 17 (50 mg, 0.149 mmol) in DMF (1 ml). The reaction mixture was stirred at 80°C for 30 hours, concentrated to remove DMF, and water (10 ml) was added, and the contents were extracted with ethyl acetate (3×50 ml). The combined organic layers were washed with brine, dried over sodium sulfate, filtered, and concentrated in vacuum to obtain a crude product, which was purified using a silica gel column (0-5% methanol in dichloromethane) to afford ethyl 2-(6-bromo-1-(cyclopropylmethyl)-1H-indol-2-yl)-3-methylpyrazolo[1,5-a]pyridine-6-carboxylate (20 mg, 30%). LC-MS (ES): m/z = 452.1 [M+H]⁺. ¹H NMR (400 MHz, DMSO-*d₆*) δ ppm: 9.19 (s, 1H), 7.90 (s, 1H), 7.83 (d, J=9.5 Hz, 1H), 7.62-7.58 (m, 2H), 7.24-7.20 (m, 1H), 6.91 (s, 1H), 4.50 (d, J=7.0 Hz, 2H), 4.36 (q, J=7.0 Hz, 2H), 2.42 (s, 3H), 1.36 (t, J=7.0 Hz, 3H), 1.11 (m, 1H), 0.32-0.26 (m, 2H), 0.18-0.13 (m, 2H).

### Intermediate 19

### Ethyl 2-(6-bromo-1-(cyclopropylmethyl)-1H-indol-2-yl)-4-methoxy-3-methylpyrazolo[1,5-a]pyridine-6-carboxylate

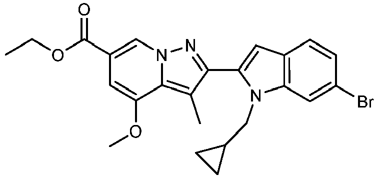

Intermediate 19 was prepared following a procedure similar to the preparation of Intermediate 18 using appropriate reactants. LC-MS retention time: 1.76 min, m/z: 468.2 [M+H] (Method G). ¹H NMR (400 MHz, chloroform-*d*) δ ppm: 8.91 (s, 1H), 8.50 (s, 1H), 7.80 (dd, J=3.01, 1.51 Hz, 1H), 7.43-7.60 (m, 1H), 6.85-6.91 (m, 1H), 6.75 (s, 1H), 4.37 (d, J = 6.5 Hz, 2H), 4.02 (s, 3H), 3.93 (s, 3H), 2.56-2.62 (m, 3H), 1.15-1.34 (m, 1H), 0.33-0.46 (m, 2H), 0.13-0.18 (m, 2H).

### Intermediate 20

### 6-Chloro-1-(cyclopropylmethyl)-1H-pyrrolo[2,3-b]pyridine

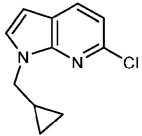

Sodium hydride in mineral oil (60% w/w, 1.31 g, 32.8 mmol) was added portion wise to a stirred solution of 6-chloro-1H-pyrrolo[2,3-b]pyridine (5g, 32.8 mmol) in DMF (25 ml) at 0°C, the reaction mixture was stirred for 15 minutes, followed by the slow addition of (bromomethyl)cyclopropane (4.42 g, 32.8 mmol) over 5 minutes. The reaction mixture was allowed to warm to a room temperature, stirred for 16 hours, quenched with water (50 ml), and extracted with ethyl acetate (3×50 ml). The combined organic layers were concentrated in vacuo to obtain a crude product, which was purified using a silica gel column (0-5% ethyl acetate / pet ether) to afford 6-chloro-1-(cyclopropylmethyl)-1H-pyrrolo[2,3-b]pyridine (6.5g, 96%). LC-MS retention time: 1.85 min, m/z: 207.0 [M+H] (Method G).

### Intermediate 21

### 6-Chloro-1-(cyclopropylmethyl)-1H-pyrrolo[2,3-b]pyridine-2-carbaldehyde

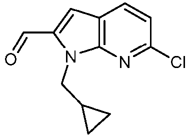

sec-Butyllithium (10.37 ml, 14.52 mmol, 1.4 M in cyclohexane) was added to a stirred solution of Intermediate 20 (2 g, 9.68 mmol) in THF (20 ml) at -78°C, the contents were stirred for 30 minutes, followed by the addition of DMF (2 ml, 25.8 mmol). The reaction mixture was allowed to warm to -20°C, then to a room temperature, stirred for 12 hours, cooled to -10°C, and quenched by addition of saturated ammonium chloride solution. The reaction mixture was diluted with water (100 ml) and extracted with ethylacetate (3×100 ml). The combined organic layers were dried over sodium sulphate, filtered and the filtrate was concentrated in vacuo to obtain a crude product, which was purified using a silica gel column (0 to 15% ethyl acetate/pet ether) to afford 6-chloro-1-(cyclopropylmethyl)-1H-pyrrolo[2,3-b]pyridine-2-carbaldehyde (1.45 g, 64%) as an off-white crystalline solid. LC-MS retention time: 1.85 min., m/z: 235.0 [M+H] (Method G).

### Intermediate 22

### (E)-6-Chloro-1-(cyclopropylmethyl)-2-(2-nitroprop-1-en-1-yl)-1H-pyrrolo[2,3-b]pyridine

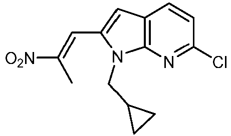

Intermediate 22 was prepared following a procedure similar to the preparation of Intermediate 17 using Intermediate 21. LC-MS retention time: 1.17 min, m/z: 292.2 [M+H] (Method G).

### Intermediate 23

### Ethyl 2-(6-chloro-1-(cyclopropylmethyl)-1H-pyrrolo[2,3-b]pyridin-2-yl)-3-methylpyrazolo[1,5-a]pyridine-6-carboxylate

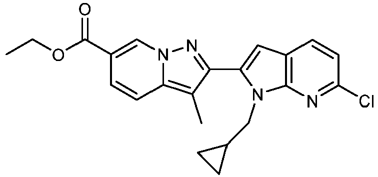

Intermediate 23 was prepared following a procedure similar to the preparation of Intermediate 18 using Intermediate 22 and Intermediate 8. LC-MS retention time: 1.66 min, m/z: 409.1 [M+H] (Method G).

### Intermediate 24

### Methyl 2-(6-chloro-1-(cyclopropylmethyl)-1H-pyrrolo[2,3-b]pyridin-2-yl)-4-chloro-3-methylpyrazolo[1,5-a]pyridine-6-carboxylate

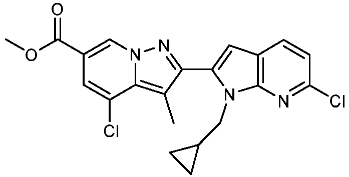

Intermediate 24 was prepared as a gummy liquid following a procedure similar to the preparation of Intermediate 18 using Intermediate 13 and Intermediate 22. LC-MS (ES): m/z : 429.1 [M+H]⁺.

### Intermediate 25

### Ethyl 7-(1-(tert-butoxycarbonyl)-1,2,3,6-tetrahydropyridin-4-yl)-1H-indole-2-carboxylate

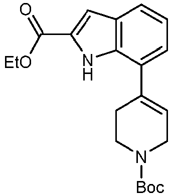

K₂CO₃ (15.46 g, 112 mmol) and PdCl₂(dppf).CH₂Cl₂ (3.05 g, 3.73 mmol) were added to a stirred solution of tert-butyl 4-(4,4,5,5-tetramethyl-1,3,2-dioxaborolan-2-yl)-3,6-dihydropyridine-1(2H)-carboxylate (11.53 g, 37.3 mmol) and ethyl 7-bromo-1H-indole-2-carboxylate (10 g, 37.3 mmol) in dioxane (200 ml). The reaction mixture was purged with nitrogen for 2 minutes, stirred at 100°C for 3 hours thereafter, filtered through a celite bed, and the celite bed was thoroughly washed with ethyl acetate. The combined filtrate was concentrated under reduced pressure to obtain a crude product, which was purified using a silica gel column (0-30% ethyl acetate in pet ether) to afford the title compound (11.3 g, 82%) as a gummy liquid. LC-MS m/z: 369.4 [M-H]⁺. 1H NMR (300 MHz, DMSO-*d₆*) δ ppm: 11.43 (s, 1H), 7.57 (d, J=7.2 Hz, 1H), 7.21 (d, J=4.0 Hz, 1H), 7.24-7.04 (m, 2H), 5.96 (br, s, 1H), 4.34 (q, J=7.1 Hz, 2H), 4.08-3.99 (m, 2H), 3.62 (t, J=5.7 Hz, 2H), 1.51-1.45 (m, 2H), 1.46 (s, 9H), 1.34 (t, J=7.2 Hz, 3H).

### Intermediate 26

### Ethyl 7-(1-(tert-butoxycarbonyl)piperidin-4-yl)-1H-indole-2-carboxylate

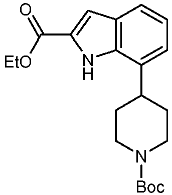

Pd/C (7.18 g, 6.75 mmol, 50% wet) was added to a stirred solution of Intermediate 25 (5.0 g, 13.50 mmol) in methanol (100 ml) purged with nitrogen, and the reaction mixture was stirred under H₂ atmosphere for 4 hours. The reaction mixture was filtered through a celite bed, and the filtrate was concentrated under reduced pressure to obtain ethyl 7-(1-(tert-butoxycarbonyl)piperidin-4-yl)-1H-indole-2-carboxylate (4.95 g, 98%). LC-MS m/z: 371.4 [M-H]⁺. ¹H NMR (300 MHz, DMSO-*d₆*) δ ppm: 11.84 (s, 1H), 7.49 (d, J=7.6 Hz, 1H), 7.21-7.01 (m, 3H), 4.36 (q, J=7.1 Hz, 2H), 4.16-4.04 (m, 4H), 3.61-3.42 (m, 1H), 1.90-1.74 (m, 2H), 1.59-1.47 (m, 2H), 1.43 (s, 9H), 1.35 (t, J=7.2 Hz, 3H).

### Intermediate 27

### Ethyl 7-(1-(tert-butoxycarbonyl)piperidin-4-yl)-1-(cyclopropylmethyl)-1H-indole-2-carboxylate

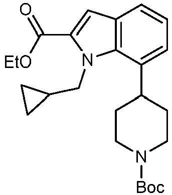

K₂CO₃ (14.47 g, 105 mmol) and (bromomethyl)cyclopropane (14.14 g, 105 mmol) were added to a stirred solution of Intermediate 26 (13 g, 34.9 mmol) in DMF (100 ml). The reaction mixture was stirred at 50°C for 12 hours, allowed to cool to a room temperature, diluted with water, and extracted with ethyl acetate (3×150 ml). The combined organic layers were washed with water and brine solution, dried over anhydrous sodium sulphate, and the volatiles were evaporated under reduced pressure to obtain a crude product, which was purified using a silica gel column (0-20% ethyl acetate in pet ether) to afford ethyl 7-(1-(tert-butoxycarbonyl)piperidin-4-yl)-1-(cyclopropylmethyl)-1H-indole-2-carboxylate (4.8 g, 32%) as a gummy liquid. LC-MS m/z: 427.5 [M+H]⁺.

### Intermediate 28

### tert-Butyl 4-(1-(cyclopropylmethyl)-2-(hydroxymethyl)-1H-indol-7-yl)piperidine-1-carboxylate

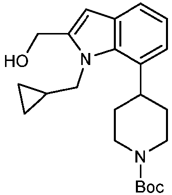

LiBH₄ in tetrahydrofuran (10.08 ml, 40.3 mmol) was added to a stirred solution of Intermediate 27 (4.3 g, 10.08 mmol) in tetrahydrofuran (80 ml) at 0°C. The reaction mixture was allowed to warm to a room temperature, stirred for 48 hours, quenched with saturated ammonium chloride solution, and extracted with ethyl acetate (4×50 ml). The combined organic layers were washed with water and brine solution, dried over anhydrous sodium sulphate, and evaporated under reduced pressure to obtain a crude product, which was purified using a silica gel column (0-70% ethyl acetate in pet ether) to afford tert-butyl 4-(1-(cyclopropylmethyl)-2-(hydroxymethyl)-1H-indol-7-yl)piperidine-1-carboxylate (3.7 g, 95%) as a gummy liquid. LC-MS m/z: 385.4 [M+H]⁺. ¹H NMR (300 MHz, DMSO-*d₆*) δ ppm: 7.33 (d, J=6.8 Hz, 1H), 7.03-6.90 (m, 2H), 6.38 (s, 1H), 5.24 (br, t, 1H), 4.60 (s, 2H), 4.30 (d, J=5.3 Hz, 2H), 4.22-3.98 (m, 2H), 3.59-3.38 (m, 1H), 3.02-2.74 (m, 2H), 1.88-1.80 (m, 2H), 1.74-1.50 (m, 2H), 1.43 (s, 9 H), 1.19-1.01 (m, 1H), 0.55-0.37 (m, 2H), 0.36-0.19 (m, 2H).

### Intermediate 29

### tert-Butyl 4-(1-(cyclopropylmethyl)-2-formyl-1H-indol-7-yl)piperidine-1-carboxylate

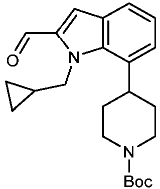

Manganese dioxide (9.04 g, 104 mmol) was added to a stirred solution of Intermediate 28 (4 g, 10.40 mmol) in tetrahydrofuran (60 ml). The reaction mixture was heated to 40°C, stirred for 2 hours, filtered through a celite bed, and washed with ethyl acetate. The combined filtrate was concentrated under reduced pressure to obtain tert-butyl 4-(1-(cyclopropylmethyl)-2-formyl-1H-indol-7-yl)piperidine-1-carboxylate (3.8 g, 96%). LC-MS m/z: 383.4 [M+H]⁺. ¹H NMR (400 MHz, chloroform-d) δ ppm: 9.84 (s, 1H), 7.63 (dd, J=1.3, 7.8 Hz, 1H), 7.42-7.33 (m, 1H), 7.30 (s, 1H), 7.32 (s, 1H), 7.18 (t, J=7.6 Hz, 1H), 5.32 (s, 1H), 4.83 (d, J=6.0 Hz, 2H), 4.47-4.23 (m, 2H), 3.81-3.72 (m, 1H), 3.64-3.48 (m, 1H), 2.93 (br, t, J=12.3 Hz, 2H), 2.01-1.95 (m, 2H), 1.84-1.78 (m, 2H), 1.52 (s, 9H), 1.18-1.08 (m, 1H), 0.50-0.42 (m, 2H), 0.42-0.35 (m, 2H).

### Intermediate 30

### tert-Butyl (E)-4-(1-(cyclopropylmethyl)-2-(2-nitroprop-1-en-1-yl)-1H-indol-7-yl)piperidine-1-carboxylate

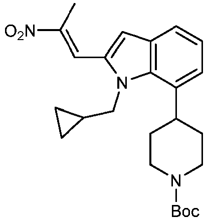

Ammonium acetate (0.937 g, 12.16 mmol) was added to a stirred solution of Intermediate 29 (3.1 g, 8.10 mmol) in nitroethane (20 ml, 280 mmol). The reaction mixture was heated to 80°C, stirred for 2 hours, diluted with water and extracted with ethyl acetate (2×50 ml). The combined organic layers were washed with water and brine solution, dried over anhydrous sodium sulphate, and evaporated under reduced pressure to obtain tert-butyl (E)-4-(1-(cyclopropylmethyl)-2-(2-nitroprop-1-en-1-yl)-1H-indol-7-yl)piperidine-1-carboxylate (3.5 g, 98%). LC-MS m/z: 440.4 [M+H]⁺. ¹H NMR (300 MHz, chloroform-d) δ ppm: 8.23 (s, 1H), 7.53 (d, J=6.80 Hz, 1H), 7.07-7.24 (m, 2H), 6.89 (s, 1H), 4.37 (br, s, 1H), 4.33 (br, d, J=5.3 Hz, 2H), 3.46-3.59 (m, 1H), 2.78-2.98 (m, 2H), 2.59 (s, 3H), 2.00-1.90 (m, 2H), 1.86-1.77 (m, 1H), 1.53-1.63 (m, 9H), 1.22-1.32 (m, 1H), 1.07-1.21 (m, 1H), 0.83 (br, d, J=2.27 Hz, 1H), 0.53-0.62 (m, 2H), 0.31 (q, J=5.16 Hz, 2H).

### Intermediate 31

### Ethyl 7-(1-(tert-butoxycarbonyl)-1,2,3,6-tetrahydropyridin-4-yl)-5-fluoro-1H-indole-2-carboxylate

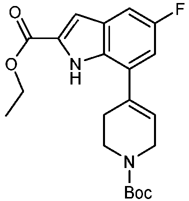

tert-Butyl 4-(4,4,5,5-tetramethyl-1,3,2-dioxaborolan-2-yl)-3,6-dihydropyridine-1(2H)-carboxylate (5.40 g, 17.48 mmol) and potassium carbonate (6.04 g, 43.7 mmol) were added to a stirred solution of ethyl 7-bromo-5-fluoro-1H-indole-2-carboxylate (5 g, 17.48 mmol) in 1,4-dioxane (45 ml) and water (5 ml). The reaction mixture was degassed with nitrogen for 2 min, followed by addition of PdCl₂(dppf) (1.279 g, 1.748 mmol). The reaction mixture was degassed for additional 2 min, heated to 100°C for 3 hours, filtered through a celite bed, and the celite bed was washed with ethyl acetate (300 ml). The combined filtrates were concentrated under reduced pressure to obtain a crude product, which was purified using a silica gel column (0-30% ethyl acetate in hexane) to afford ethyl 7-(1-(tert-butoxycarbonyl)-1,2,3,6-tetrahydropyridin-4-yl)-5-fluoro-1H-indole-2-carboxylate (5.7 g, 84%). LC-MS m/z: 387.5 [M-H] ⁺. ¹H NMR (300 MHz, chloroform-d) δ ppm: 8.92 (br, s, 1H), 7.25-7.20 (m, 1H), 7.18 (d, J=2.2 Hz, 1H), 6.95 (dd, J=2.3, 10.2 Hz, 1H), 6.12 (br, s, 1H), 4.42 (q, J=7.2 Hz, 2H), 4.16 (br, d, J=3.0 Hz, 2H), 3.71 (t, J=5.5 Hz, 2H), 2.63-2.42 (m, 2H), 1.53 (s, 9H), 1.42 (t, J=7.2 Hz, 3H).

### Intermediate 32

### Ethyl 7-(1-(tert-butoxycarbonyl)piperidin-4-yl)-5-fluoro-1H-indole-2-carboxylate

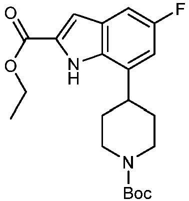

Pd/C (3.29 g, 15.45 mmol) was added to a solution of Intermediate 31 (6.0 g, 15.45 mmol) in methanol (60 ml) purged with nitrogen for 2 min. The reaction mixture was stirred under hydrogen atmosphere at 1 atmospheric pressure for 3 hours, filtered through a celite bed, and washed with ethyl acetate (300 ml). The combined organic layers were concentrated under reduced pressure to obtain ethyl 7-(1-(tert-butoxycarbonyl)piperidin-4-yl)-5-fluoro-1H-indole-2-carboxylate (5.5 g, 91%). LC-MS m/z: 389.2 [M-H] ⁺. ¹H NMR (300 MHz, DMSO-*d₆*) δ ppm: 12.00 (s, 1H), 7.25 (dd, *J*=2.3, 9.1 Hz, 1H), 7.15 (d, *J*=1.9 Hz, 1H), 7.00 (dd, *J*=2.5, 10.8 Hz, 1H), 4.36 (q, *J*=7.2 Hz, 2H), 4.11 (br d, *J*=12.5 Hz, 2H), 3.52 (br s, 1H), 3.34 (s, 1H), 3.07-2.74 (m, 2H), 1.81 (br d, *J*=11.7 Hz, 2H), 1.62-1.50 (m, 1H), 1.43 (s, 9H), 1.34 (t, *J*=7.0 Hz, 3H).

### Intermediate 33

### Ethyl 7-(1-(tert-butoxycarbonyl)piperidin-4-yl)-1-(cyclopropylmethyl)-5-fluoro-1H-indole-2-carboxylate

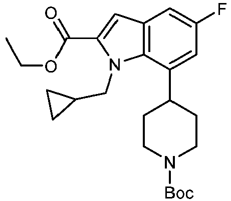

Cesium carbonate (10.43 g, 32.0 mmol), TBAI (4.73 g, 12.81 mmol) and (bromomethyl)cyclopropane (3.46 g, 25.6 mmol) were added to a stirred solution of Intermediate 32 (5 g, 12.81 mmol) in DMF (50 ml). The reaction mixture was heated to 55°C for 16 hours, quenched with water (100 ml), and extracted with ethyl acetate (2×100 ml). The combined organic layers were dried over sodium sulphate, filtered, and concentrated under reduced pressure to obtain a crude product, which was purified using a silica gel column (0-20% ethyl acetate in hexane) to afford ethyl 7-(1-(tert-butoxycarbonyl)piperidin-4-yl)-1-(cyclopropylmethyl)-5-fluoro-1H-indole-2-carboxylate (4.8 g, 84%). LC-MS m/z: 389.3 [M+H]⁺. ¹H NMR (400 MHz, chloroform-*d*) δ ppm: 7.32 (s, 1H), 7.18 (dd, J=2.5, 8.5 Hz, 1H), 6.99 (dd, J=2.8, 10.8 Hz, 1H), 4.79 (d, J=6.0 Hz, 2H), 4.44-4.34 (m, 2H), 3.55 (br, s, 1H), 2.92 (br, s, 2H), 2.00 (br, d, J=13.0 Hz, 2H), 1.76 (br, dd, J=4.0, 12.5 Hz, 2H), 1.52 (s, 9H), 1.45-1.40 (m, 3H), 1.05 (s, 1H), 0.48-0.39 (m, 2H), 0.38-0.30 (m, 2H).

### Intermediate 34

### tert-Butyl 4-(1-(cyclopropylmethyl)-5-fluoro-2-(hydroxymethyl)-1H-indol-7-yl)piperidine-1-carboxylate

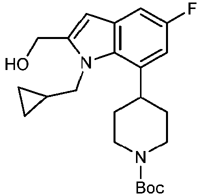

LiBH₄ (9.84 ml, 39.4 mmol) was added to a stirred solution of Intermediate 33 (3.5 g, 7.87 mmol) in THF (35 ml) at 0°C. The reaction mixture was stirred for 16 hours at a room temperature, filtered through a celite bed, and washed with ethyl acetate (70 ml). The combined organic layers were concentrated under reduced pressure to obtain crude tert-butyl 4-(1-(cyclopropylmethyl)-5-fluoro-2-(hydroxymethyl)-1H-indol-7-yl)piperidine-1-carboxylate (2.5 g, 79%). LC-MS m/z: 403.4 [M+H]⁺. ¹H NMR (300 MHz, DMSO-*d₆*) δ ppm: 7.10 (dd, J=9.07, 2.64 Hz, 1H), 6.74-6.92 (m, 1H), 6.38 (s, 1H), 5.29 (t, J=5.48 Hz, 1H), 4.59 (d, J=5.67 Hz, 1H), 4.28 (br d, J=5.29 Hz, 2H), 4.13 (br d, J=12.46 Hz, 1H), 3.51 (br t, J=10.58 Hz, 1H), 3.13-3.32 (m, 1H), 2.35 (s, 2H), 2.27 (d, J=5.29 Hz, 1H), 1.84 (br d, J=12.84 Hz, 2H), 1.51-1.74 (m, 2H), 1.35-1.51 (m, 9H), 0.74-1.04 (m, 1H), 0.21-0.51 (m, 4H).

### Intermediate 35

### tert-Butyl 4-(1-(cyclopropylmethyl)-5-fluoro-2-formyl-1H-indol-7-yl)piperidine-1-carboxylate

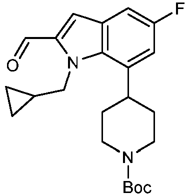

Manganese dioxide (0.691 g, 7.95 mmol) was added to a stirred solution of Intermediate 34 (3.2 g, 7.95 mmol) in tetrahydrofuran (32 ml). The reaction mixture was stirred for 16 hours, filtered through a celite bed, and washed with ethyl acetate (70 ml). The organic layers were concentrated under reduced pressure to obtain tert-butyl 4-(1-(cyclopropylmethyl)-5-fluoro-2-formyl-1H-indol-7-yl)piperidine-1-carboxylate (2.7 g, 85%). LC-MS m/z: 345.1 [M-tBu]⁺. ¹H NMR (300 MHz, chloroform-*d*) δ ppm: 9.81 (s, 1H), 7.25-7.20 (m, 2H), 7.05 (dd, J=2.3, 11.0 Hz, 1H), 4.78 (d, J=6.0 Hz, 2H), 4.48-4.22 (m, 2H), 3.52 (br, t, J=11.5 Hz, 1H), 3.02-2.77 (m, 2H), 2.05-1.89 (m, 2H), 1.86-1.64 (m, 2H), 1.50 (s, 9H), 1.15-1.01 (m, 1H), 0.49-0.31 (m, 4H).

### Intermediate 36

### tert-Butyl (E)-4-(1-(cyclopropylmethyl)-5-fluoro-2-(2-nitroprop-1-en-1-yl)-1H-indol-7-yl)piperidine-1-carboxylate

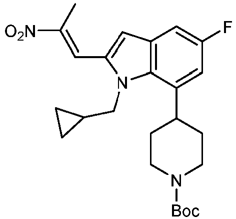

Intermediate 36 was prepared following a procedure similar to the preparation of Intermediate 30, using appropriate reactants. LC-MS m/z: 402.3 [M+H]⁺. ¹H NMR (300 MHz, chloroform-*d*) δ ppm: 8.18 (s, 1H), 7.15 (dd, J=2.5, 8.1 Hz, 1H), 6.94 (dd, J=2.3, 11.0 Hz, 1H), 6.82 (s, 1H), 4.47-4.26 (m, 4 H), 3.60-3.44 (m, 1H), 3.01-2.79 (m, 2H), 2.58 (s, 3H), 2.01-1.90 (m, 2H), 1.84-1.70 (m, 2H), 1.50 (s, 9H), 1.15-1.05 (m, 1H), 0.62-0.52 (m, 2H), 0.29 (q, J=5.3 Hz, 2H).

### Intermediate 37

### Ethyl 3-(2-chloro-3-nitropyridin-4-yl)-2-hydroxyacrylate

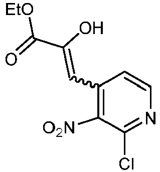

Sodium ethoxide in ethanol (28.2 g, 87 mmol) was added to diethyl oxalate (12.70 g, 87 mmol) in toluene (50 ml). The reaction mixture was stirred for 10 min at a room temperature, followed by the addition of 2-chloro-4-methyl-3-nitropyridine (10 g, 57.9 mmol). The reaction mixture was stirred at a room temperature for 16 hours, concentrated, the residue thus obtained was diluted with water (20 ml), and acidified with acetic acid (10 ml) to bring pH to about 4. The solids thus obtained were filtered and dried to afford ethyl 3-(2-chloro-3-nitropyridin-4-yl)-2-hydroxyacrylate (15 g, 95%) as an off-white solid. LC-MS m/z: 273.0 [M+H]⁻.¹H NMR (300 MHz, DMSO-*d₆*) δ ppm: 8.61 (d, J=5.7 Hz, 1H), 8.31 (d, J=5.7 Hz, 1H), 5.92 (s, 1H), 4.30 (q, J=7.1 Hz, 2H), 1.28 (t, J=7.0 Hz, 3H).

### Intermediate 38

### Ethyl 7-chloro-1H-pyrrolo[2,3-c]pyridine-2-carboxylate

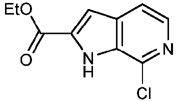

Iron (15.36 g, 275 mmol) and ammonium chloride (23.54 g, 440 mmol) in water (30 ml) were added to a stirred solution of Intermediate 37 (15.0 g, 55.0 mmol) in ethanol (50 ml) and THF (100 ml). The reaction mixture was stirred at 65°C for 3 hours, filtered through a celite bed, the filtrate obtained was diluted with water (50 ml), and quenched with saturated NaHCO₃ solution, followed by extraction with ethyl acetate (2×100 ml). The combined organic layers were dried over sodium sulphate, filtered, and concentrated under reduced pressure to obtain a crude product, which was purified using a silica gel column (0-50% ethyl acetate in hexane) to afford ethyl 7-chloro-1H-pyrrolo[2,3-c]pyridine-2-carboxylate (6.5 g, 53%). LC-MS m/z: 225.0 [M+H]⁺. ¹H NMR (400 MHz, DMSO-*d₆*) δ ppm: 12.73 (br, s, 1H), 7.99 (d, J=5.5 Hz, 1H), 7.68 (d, J=5.5 Hz, 1H), 7.30 (s, 1H), 4.39 (q, J=7.0 Hz, 2H), 1.36 (t, J=7.0 Hz, 3H).

### Intermediate 39

### Ethyl 7-(1-(tert-butoxycarbonyl)-1,2,3,6-tetrahydropyridin-4-yl)-1H-pyrrolo[2,3-c]pyridine-2-carboxylate

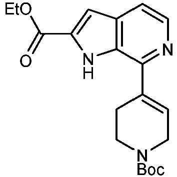

tert-Butyl 4-(4,4,5,5-tetramethyl-1,3,2-dioxaborolan-2-yl)-3,6-dihydropyridine-1(2H)-carboxylate (11.63 g, 37.6 mmol) and K₂CO₃ in water (36.2 ml, 72.3 mmol) were added to a stirred solution of Intermediate 38 (6.5 g, 28.9 mmol) in 1,4-dioxane (65 ml). The reaction mixture was degassed with argon for 10 min, followed by the addition of PdCl₂(dppf)-CH₂Cl₂ adduct (1.418 g, 1.736 mmol), and stirred at 110°C for 16 hours. The reaction mixture was filtered through a celite bed, the filtrate was diluted with water (50 ml) and extracted with ethyl acetate (2×100 ml). The combined organic layers were dried over sodium sulphate, filtered, and concentrated under reduced pressure to obtain a crude product, which was purified using a silica gel column (0-60% ethyl acetate in hexane) to afford ethyl 7-(1-(tert-butoxycarbonyl)-1,2,3,6-tetrahydropyridin-4-yl)-1H-pyrrolo[2,3-c]pyridine-2-carboxylate (10.2 g, 95%). LC-MS m/z: 372.2 [M+H]⁺. ¹H NMR (300 MHz, DMSO-*d₆*) δ ppm: 11.95 (br, s, 1H), 8.16 (d, J=5.3 Hz, 1H), 7.53 (d, J=5.7 Hz, 1H), 7.23 (s, 1H), 6.36 (br s, 1H), 4.38 (q, J=7.1 Hz, 2H), 4.15-4.08 (m, 2H), 4.03 (d, J=7.2 Hz, 1H), 3.94 (s, 2H), 3.59 (br t, J=5.3 Hz, 2H), 2.72-2.62 (m, 2H), 1.46 (s, 9H), 1.35 (t, J=7.2 Hz, 3H).

### Intermediate 40

### Ethyl 7-(1-(tert-butoxycarbonyl)piperidin-4-yl)-1H-pyrrolo[2,3-c]pyridine-2-carboxylate

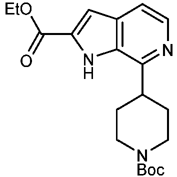

Pd/C (50% wet, 2.92 g, 2.75 mmol) was added to a stirred solution of Intermediate 39 (10.2 g, 27.5 mmol) in ethanol (10 ml). The reaction mixture was stirred under hydrogen atmosphere for 16 hours, filtered through a celite bed, and the filtrate was concentrated to obtain ethyl 7-(1-(tert-butoxycarbonyl)piperidin-4-yl)-1H-pyrrolo[2,3-c]pyridine-2-carboxylate (8.1 g, 79%). LC-MS m/z: 374.1 [M+H] ⁺. ¹H NMR (300 MHz, DMSO-*d₆*) δ ppm: 12.38 (s, 1H), 8.09 (d, J=5.3 Hz, 1H), 7.46 (d, J=5.7 Hz, 1H), 7.18 (d, J=1.5 Hz, 1H), 4.43-4.34 (m, 2H), 4.16-4.05 (m, 2H), 3.94 (s, 2H), 3.80-3.62 (m, 2H), 3.44-3.42 (m, 2H), 1.76 (br, s, 1H), 1.43 (s, 9H), 1.39-1.23 (m, 3H).

### Intermediate 41

### Ethyl 7-(1-(tert-butoxycarbonyl)piperidin-4-yl)-1-(cyclopropylmethyl)-1H-pyrrolo[2,3-c]pyridine-2-carboxylate

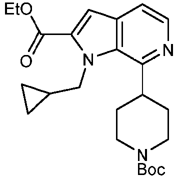

Cesium carbonate (4.45 g, 13.66 mmol) and TBAI (0.841 g, 2.276 mmol) were added to a stirred solution of Intermediate 40 (1.7 g, 4.55 mmol) in DMF (10 ml), followed by the addition of (bromomethyl)cyclopropane (1.229 g, 9.10 mmol). The reaction mixture was stirred at 60°C for 3 hours, diluted with water (20 ml), and extracted with ethyl acetate (3×20 ml). The combined organic layers were washed with brine, dried over sodium sulfate, filtered, and concentrated in vacuum to obtain a crude product, which was purified using a silica gel column (0-20% ethyl acetate in hexane) to afford ethyl 7-(1-(tert-butoxycarbonyl)piperidin-4-yl)-1-(cyclopropylmethyl)-1H-pyrrolo[2,3-c]pyridine-2-carboxylate (1.3 g, 67%). LC-MS m/z: 428.2 [M+H]⁺. ¹H NMR (300 MHz, DMSO-*d₆*) δ ppm: 8.18 (d, J=5.3 Hz, 1H), 7.53 (d, J=5.3 Hz, 1H), 7.33 (s, 1H), 4.80 (br, d, J=6.4 Hz, 2H), 4.35 (q, J=7.2 Hz, 2H), 4.19-3.98 (m, 2H), 3.68-3.52 (m, 1H), 2.94 (br, s, 2H), 1.85 (br, s, 4 H), 1.43 (s, 9H), 1.36-1.32 (m, 3H), 1.27-0.99 (m, 1H), 0.53-0.35 (m, 2H), 0.33-0.17 (m, 2H).

### Intermediate 42

### tert-Butyl 4-(1-(cyclopropylmethyl)-2-(hydroxymethyl)-1H-pyrrolo[2,3-c]pyridin-7-yl)piperidine-1-carboxylate

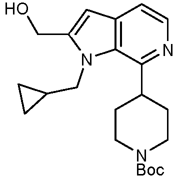

LiBH₄ (4.56 ml, 18.24 mmol) was added to a stirred solution of Intermediate 41 (1.3 g, 3.04 mmol) in THF (30 ml). The reaction mixture was stirred at a room temperature for 36 hours, quenched with saturated ammonium chloride solution (10 ml), diluted with water (20 ml), and extracted with ethyl acetate (3×20 ml). The combined organic layers were washed with brine, dried over sodium sulfate, filtered, and concentrated in vacuum to obtain a crude product, which was purified using a silica gel column (0-20% ethyl acetate in hexane) to afford tert-butyl 4-(1-(cyclopropylmethyl)-2-(hydroxymethyl)-1H-pyrrolo[2,3-c]pyridin-7-yl)piperidine-1-carboxylate (1.02 g, 87%). LC-MS m/z: 386.2 [M+H]⁺. ¹H NMR (300 MHz, DMSO-*d₆*) δ ppm: 8.04 (d, J=5.3 Hz, 1H), 7.32 (d, J=5.3 Hz, 1H), 6.45 (s, 1H), 5.42 (t, J=5.3 Hz, 1H), 4.65 (d, J=5.7 Hz, 2H), 4.34 (br d, J=6.0 Hz, 2H), 4.11 (br d, J=10.6 Hz, 2H), 3.57 (br d, J=11.0 Hz, 1H), 2.90 (br s, 2H), 1.88 (br d, J=9.8 Hz, 2H), 1.83-1.64 (m, 2H), 1.43 (s, 9H), 1.24 (br s, 1H), 0.53-0.42 (m, 2H), 0.31 (q, J=4.9 Hz, 2H).

### Intermediate 43

### tert-Butyl 4-(1-(cyclopropylmethyl)-2-formyl-1H-pyrrolo[2,3-c]pyridin-7-yl)piperidine-1-carboxylate

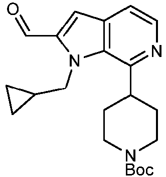

Manganese dioxide (2.255 g, 25.9 mmol) was added to a stirred solution of Intermediate 42 (1 g, 2.59 mmol) in THF (10 ml). The reaction mixture was stirred at 40°C for 3 hours, filtered through a celite bed, and the filtrate was concentrated to obtain tert-butyl 4-(1-(cyclopropylmethyl)-2-formyl-1H-pyrrolo[2,3-c]pyridin-7-yl)piperidine-1-carboxylate (0.9 g, 90%). LC-MS m/z: 384.2[M+H]⁺. ¹H NMR (300 MHz, DMSO-*d₆*) δ ppm: 10.03 (s, 1H), 8.22 (d, J=5.3 Hz, 1H), 7.61 (d, J=5.3 Hz, 1H), 7.56 (s, 1H), 4.80 (d, J=6.0 Hz, 2H), 4.18-4.00 (m, 2H), 3.67-3.55 (m, 1H), 3.13-2.81 (m, 2H), 1.94-1.75 (m, 4H), 1.47-1.35 (m, 9H), 1.29-1.12 (m, 1H), 0.51-0.37 (m, 2H), 0.36-0.23 (m, 2H).

### Intermediate 44

### tert-Butyl (E)-4-(1-(cyclopropylmethyl)-2-(2-nitroprop-1-en-1-yl)-1H-pyrrolo[2,3-c]pyridin-7-yl)piperidine-1-carboxylate

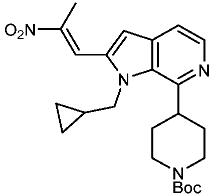

Ammonium acetate (0.271 g, 3.52 mmol) was added to a stirred solution of Intermediate 43 (0.9 g, 2.347 mmol) in nitroethane (1.678 ml, 23.47 mmol). The reaction mixture was stirred at 90°C for 2 hours, diluted with water (50 ml), and extracted with ethyl acetate (2×100 ml). The combined organic layers were dried over sodium sulphate, filtered, and concentrated under reduced pressure to obtain tert-butyl (E)-4-(1-(cyclopropylmethyl)-2-(2-nitroprop-1-en-1-yl)-1H-pyrrolo[2,3-c]pyridin-7-yl)piperidine-1-carboxylate (0.85 g, 82%). LC-MS m/z: 441.2 [M+H]⁺.

### Intermediate 45

### Ethyl 7-bromo-1-(cyclopropylmethyl)-1H-indole-2-carboxylate

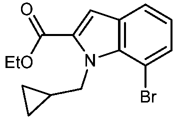

Potassium carbonate (41.2 g, 298 mmol) was added to a stirred solution of ethyl 7-bromo-1H-indole-2-carboxylate (20 g, 74.6 mmol) in DMF (80 ml), followed by addition of (bromomethyl)cyclopropane (14.47 ml, 149 mmol). The reaction mixture was stirred at 50°C for 16 hours, diluted with water (100 ml), and extracted with ethyl acetate (2×200 ml). The combined organic layers were washed with brine, dried over sodium sulfate, filtered, and concentrated in vacuum to obtain a crude product, which was purified using a silica gel column (0-10% ethyl acetate in hexane) to afford (22 g, 92%) of ethyl 7-bromo-1-(cyclopropylmethyl)-1H-indole-2-carboxylate as off white solid. LC-MS m/z: 322.0 [M+H]⁺. ¹H NMR (400 MHz, DMSO-*d₆*) δ ppm: 7.76 (d, J=8.0 Hz, 1H), 7.59 (d, J=8.0 Hz, 1H), 7.41 (s, 1H), 7.07 (t, J=8.0 Hz, 1H), 5.01 (d, J=8.0 Hz, 2H), 4.34 (q, J=8.0 Hz, 2H), 2.33 (br, s, 1H), 1.34 (t, J=8.00 Hz, 3H), 1.27-1.15 (m, 1H), 0.42-0.29 (m, 4H).

### Intermediate 46

### Ethyl 7-(1-(tert-butoxycarbonyl)azetidin-3-yl)-1-(cyclopropylmethyl)-1H-indole-2-carboxylate

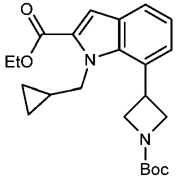

Tris(trimethylsilyl)silane (5.67 g, 22.81 mmol) and sodium carbonate (4.61 g, 43.5 mmol) were added to a stirred solution of Intermediate 45 (9.84 g, 34.8 mmol) in 1,2-dimethoxyethane (35 ml) in a first vial, and the reaction mixture was purged with argon followed by the addition of Ir[DF(CF₃)PPY]₂(DTBBPY)PF₆ (0.244 g, 0.217 mmol). A mixture of 4,4'-di-tert-butyl-2,2'-dipyridyl (0.292 g, 1.086 mmol) and Nickel(II) chloride ethylene glycol dimethyl ether complex (0.239 g, 1.086 mmol) in 1,2-dimethoxy ethane (0.5 ml) was prepared in another vial and the contents were sonicated for 10 min to obtain a catalyst (green suspension). The catalyst was added into the reaction mixture in the first vial, and the vial containing the reaction mixture was placed under Blue LED light (34 W) for 16 hours. The reaction mixture was diluted with water (100 ml), and extracted with ethyl acetate (2×200 ml). The combined organic layers were washed with brine, dried over sodium sulfate, filtered, and concentrated in vacuum to obtain a crude product, which was purified using a silica gel column (0-20% ethyl acetate in hexane) to afford (5.6 g, 64%) of ethyl 7-(1-(tert-butoxycarbonyl)azetidin-3-yl)-1-(cyclopropylmethyl)-1H-indole-2-carboxylate as pale yellow liquid. LC-MS m/z: 343.3 [M-56] ⁺. ¹H NMR (400 MHz, DMSO-*d₆*) δ ppm: 7.63 (d, *J*=8.0 Hz, 1H), 7.49 (d, *J*=8.0 Hz, 1H), 7.36 (s, 1H), 7.19 (t, *J*=8.0 Hz, 1H), 4.63 (d, *J*=8.0 Hz, 2H), 4.57-4.52 (m, 1H), 4.41-4.29 (m, 2H), 4.12-3.94 (m, 2H), 3.82-3.80 (m, 2H), 2.12 (quin, *J*=7.5 Hz, 1H), 1.41 (s, 9H), 1.33(t, *J*=8.0 Hz, 3H), 0.94-0.84 (m, 1H), 0.39-0.27 (m, 2H), 0.21-0.02 (m, 2H).

### Intermediate 47

### tert-Butyl 3-(1-(cyclopropylmethyl)-2-(hydroxymethyl)-1H-indol-7-yl)azetidine-1-carboxylate

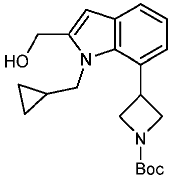

LiBH₄ (14.05 ml, 56.2 mmol) was added to a stirred solution of Intermediate 46 (5.6 g, 14.05 mmol) in THF (50 ml). The reaction mixture was stirred at a room temperature for 4 days, quenched with saturated ammonium chloride solution (10 ml), diluted with water (20 ml), and extracted with ethyl acetate (3×50 ml). The combined organic layers were washed with brine, dried over sodium sulfate, filtered, and concentrated in vacuum to obtain a crude product, which was purified using a silica gel column (0-20% ethyl acetate in hexane) to afford (2.0 g, 39%) of tert-butyl 3-(1-(cyclopropylmethyl)-2-(hydroxymethyl)-1H-indol-7-yl)azetidine-1-carboxylate. LC-MS m/z: 357.0 [M+H] ⁺. ¹H NMR (400 MHz, DMSO-*d₆*) δ ppm: 7.41 (d, J=8.0 Hz, 1H), 7.29 (d, J=8.0 Hz, 1H), 7.06 (t, J=8.0 Hz, 1H), 6.41 (s, 1H), 5.23 (t, J=4.0 Hz, 1H), 4.66-4.55 (m, 2H), 4.36 (br, t, J=8.5 Hz, 2H), 4.24 (d, J=6.0 Hz, 2H), 3.96 (br, t, J=8.0 Hz, 2H), 1.99 (s, 1H), 1.46-1.38 (m, 9H), 1.09-1.00 (m, 1H), 0.47-0.37 (m, 2H), 0.24 (q, J=5.0 Hz, 2H).

### Intermediate 48

### tert-Butyl 3-(1-(cyclopropylmethyl)-2-formyl-1H-indol-7-yl)azetidine-1-carboxylate

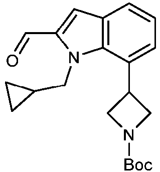

Manganese dioxide (4.88 g, 56.1 mmol) was added to a stirred solution of Intermediate 47 (2 g, 5.61 mmol) in THF (10 ml). The reaction mixture was stirred at 40°C for 3 hours, filtered through a celite bed, and the filtrate was concentrated to obtain (1.9 g, 96%) of tert-butyl 3-(1-(cyclopropylmethyl)-2-formyl-1H-indol-7-yl)azetidine-1-carboxylate. LC-MS m/z: 299 [M-56]⁺. ¹H NMR (400 MHz, DMSO-*d₆*) δ ppm: 9.86 (s, 1H), 7.72 (d, J=8.0 Hz, 1H), 7.60 (d, J=8.0 Hz, 1H), 7.56 (s, 1H), 7.23 (t, J=8.0 Hz, 1H), 4.65 (d, J=8.0 Hz, 2H), 4.38 (br t, J=8.0 Hz, 2H), 4.06-3.96 (m, 2H), 1.77 (td, J=3.4, 6.3 Hz, 1H), 1.41 (s, 9H), 1.02-0.99 (m, 1H), 0.41-0.30 (m, 2H), 0.26-0.15 (m, 2H).

### Intermediate 49

### tert-Butyl (E)-3-(1-(cyclopropylmethyl)-2-(2-nitroprop-1-en-1-yl)-1H-indol-7-yl)azetidine-1-carboxylate

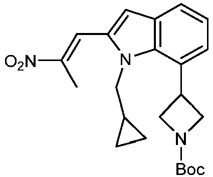

Ammonium acetate (0.620 g, 8.04 mmol) was added to a stirred solution of Intermediate 48 (1.9 g, 5.36 mmol) in nitroethane (3.83 ml, 53.6 mmol). The reaction mixture was stirred at 90°C for 2 hours, diluted with water (20 ml), and extracted with ethyl acetate (3×20 ml). The combined organic layers were washed with brine, dried over sodium sulfate, filtered, and concentrated in vacuum to afford (2.2 g, 100%) of tert-butyl (E)-3-(1-(cyclopropylmethyl)-2-(2-nitroprop-1-en-1-yl)-1H-indol-7-yl)azetidine-1-carboxylate. LC-MS m/z: 412.3 [M+H]⁺. ¹H NMR (400 MHz, DMSO-*d₆*) δ ppm: 8.21 (s, 1H), 7.60 (d, J=8.0 Hz, 1H), 7.45 (d, J=8.0 Hz, 1H), 7.18 (t, J=8.0 Hz, 1H), 7.09 (s, 1H), 4.58-4.53 (m, 1H), 4.43-4.33 (m, 2H), 4.30 (d, J=6.0 Hz, 2H), 4.09-3.95 (m, 2H), 2.48-2.38 (m, 1H), 1.47-1.37 (m, 9H), 1.06-0.96 (m, 1H), 0.49-0.39 (m, 2H), 0.29-0.15 (m, 2H).

### Intermediate 50

### (E)-7-(benzyloxy)-1-(cyclopropylmethyl)-2-(2-nitroprop-1-en-1-yl)-1H-indole

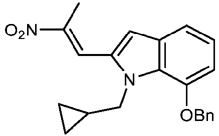

Ammonium acetate (0.606 g, 7.86 mmol) was added to a stirred solution of 7-(benzyloxy)-1-(cyclopropylmethyl)-1H-indole-2-carbaldehyde (2 g, 6.55 mmol) in nitroethane (18.73 ml, 262 mmol). The reaction mixture was heated to 90°C for 2 hours, allowed to cool to a room temperature, diluted with water (100 ml), and extracted with ethyl acetate (2×100 ml). The combined organic layers were dried over sodium sulphate, filtered, and concentrated under reduced pressure to obtain crude (E)-7-(benzyloxy)-1-(cyclopropylmethyl)-2-(2-nitroprop-1-en-1-yl)-1H-indole (1.9 g, 80%) LC-MS m/z: 361.7 [M+H]⁺.

### Intermediate 51

### 7-Chloro-1H-pyrrolo[2,3-c]pyridine

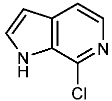

Vinyl magnesium bromide (189 ml, 189 mmol) was added to a stirred solution of 2-chloro-3-nitropyridine (10 g, 63.1 mmol) in THF (80 ml) at -78°C. The reaction mixture was stirred for 1 hour, warmed to -20°C, and the resulting solution was stirred at -20°C for 12 hours. The reaction mixture was quenched with saturated ammonium chloride solution (20 ml), diluted with water (2×50 ml), and extracted with ethyl acetate (3×50 ml). The combined organic layers were washed with brine, dried over sodium sulfate, filtered, and concentrated in vacuum to obtain a crude product, which was purified using a silica gel column (0-50% ethyl acetate in hexane) to afford 7-chloro-1H-pyrrolo[2,3-c]pyridine (3.4 g, 35%) as brown solid. LC-MS m/z: 153.1 [M+H]⁺. ¹H NMR (300 MHz, DMSO-*d₆*) δ ppm: 8.35-8.30 (m, 1H), 8.29 (s, 1H), 7.88-7.78 (m, 1H), 7.78-7.70 (m, 1H), 7.66 (s, 1H).

### Intermediate 52

### 7-Chloro-1-(cyclopropylmethyl)-1H-pyrrolo[2,3-c]pyridine

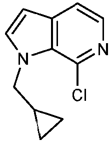

Sodium hydride (1.049 g, 26.2 mmol) was added to a stirred solution of Intermediate 51 (2 g, 13.11 mmol) in DMF (20 ml) at 0°C, the contents were stirred for 20 minutes, followed by the addition of (bromomethyl)cyclopropane (1.910 ml, 19.66 mmol). The resulting reaction mixture was stirred for 16 hours, quenched with ice-flakes and cold water (20 ml), and extracted with ethyl acetate (3×30 ml). The combined organic layers were washed with brine, dried over sodium sulfate, filtered, and concentrated in vacuum to obtain a crude product, which was purified using a silica gel column (0-20% ethyl acetate in hexane) to afford 7-chloro-1-(cyclopropylmethyl)-1H-pyrrolo[2,3-c]pyridine (2.2 g, 81%) of. LC-MS m/z: 207.1 [M+H]⁺. ¹H NMR (300 MHz, DMSO-*d₆*) δ ppm: 7.90 (d, J=5.3 Hz, 1H), 7.76 (d, J=3.0 Hz, 1H), 7.58 (d, J=5.3 Hz, 1H), 6.63 (d, J=3.4 Hz, 1H), 4.40 (d, J=6.8 Hz, 2H), 1.40-1.22 (m, 1H), 0.53-0.39 (m, 4H).

### Intermediate 53

### 7-Chloro-1-(cyclopropylmethyl)-1H-pyrrolo[2,3-c]pyridine-2-carbaldehyde

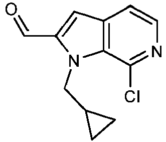

sec-Butyllithium (11.41 ml, 15.97 mmol) was added to a stirred solution of Intermediate 52 (2.2 g, 10.64 mmol) in tetrahydrofuran (30 ml) at -78°C, the contents were stirred for 1 hour, followed by the addition of DMF (1.236 ml, 15.97 mmol). The resulting reaction mixture was stirred at the same temperature for 1 hour, quenched with saturated ammonium chloride solution (10 ml), diluted with water (20 ml), and extracted with ethyl acetate (3×20 ml). The combined organic layers were washed with brine, dried over sodium sulfate, filtered, and concentrated in vacuum to obtain a crude product, which was purified using a silica gel column (eluting with 0-20% ethyl acetate in hexane) to afford 7-chloro-1-(cyclopropylmethyl)-1H-pyrrolo[2,3-c]pyridine-2-carbaldehyde (1.2 g, 78%) as an off white solid. LC-MS m/z: 235.0 [M+H]⁺. ¹H NMR (400 MHz, DMSO-*d₆*) δ ppm: 10.10 (s, 1H), 8.08 (d, J=5.0 Hz, 1H), 7.84 (d, J=5.5 Hz, 1H), 7.67 (s, 1H), 4.92 (d, J=7.0 Hz, 2H), 1.35-1.24 (m, 1H), 0.47-0.41 (m, 2H), 0.41-0.33 (m, 2H).

### Intermediate 54

### (E)-7-Chloro-1-(cyclopropylmethyl)-2-(2-nitroprop-1-en-1-yl)-1H-pyrrolo[2,3-c]pyridine

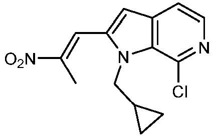

(E)-7-chloro-1-(cyclopropylmethyl)-2-(2-nitroprop-1-en-1-yl)-1H-pyrrolo[2,3-c]pyridine (1.2g, 97%) was prepared following a procedure similar to the preparation of Intermediate 57. LC-MS m/z 292.2 [M+H]⁺. ¹H NMR (400 MHz, DMSO-*d₆*) δ ppm: 8.18 (s, 1H), 8.01 (d, J=5.5 Hz, 1H), 7.69 (d, J=5.5 Hz, 1H), 7.14 (s, 1H), 4.64 (d, J=7.0 Hz, 2H), 2.54-2.52 (m, 3H), 1.33-1.09 (m, 1H), 0.43-0.23 (m, 4H).

### Intermediate 55

### 7-Chloro-1-methyl-1H-pyrrolo[2,3-c]pyridine

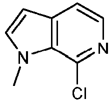

Sodium hydride (1.311 g, 32.8 mmol) was added to a stirred solution of Intermediate 51 (5 g, 32.8 mmol) in DMF (100 ml) at 0°C, the contents were stirred for 5 min, followed by the addition of iodomethane (2.049 ml, 32.8 mmol). The resulting reaction mixture was stirred at a room temperature for 16 hours, quenched with ice water, and extracted with ethyl acetate (3×100 ml). The combined organic layers were dried over Na₂SO₄, and concentrated to obtain a crude product, which was purified using a silica gel column (0-50% ethyl acetate in hexane) to afford 7-chloro-1-methyl-1H-pyrrolo[2,3-c]pyridine (4 g, 73%) as off-white solid. LC-MS m/z: 167.0 [M+H]⁺.

### Intermediate 56

### 7-Chloro-1-methyl-1H-pyrrolo[2,3-c]pyridine-2-carbaldehyde

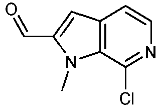

*sec*-Butyl lithium (6.43 ml, 9 mmol) was added to a solution of Intermediate 55 (1 g, 6 mmol) in tetrahydrofuran (10 ml) at -78°C, the contents were stirred for 0.5 hours, followed by the addition of DMF (0.697 ml, 9 mmol). The resulting reaction mixture was stirred for 1 hour, quenched with saturated NH₄Cl solution (50 ml), extracted with ethyl acetate (3×50 ml), dried over Na₂SO₄, and concentrated to obtain a crude product, which was purified using a silica gel column (0-50% ethyl acetate in hexane) to afford 7-chloro-1-methyl-1H-pyrrolo[2,3-c]pyridine-2-carbaldehyde (1.0 g, 86%) as a brown oil. LC-MS m/z: 195.0 [M+H]⁺.

### Intermediate 57

### (E)-7-Chloro-1-methyl-2-(2-nitroprop-1-en-1-yl)-1H-pyrrolo[2,3-c]pyridine

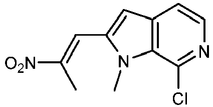

Ammonium acetate (0.594 g, 7.71 mmol) was added to a solution of Intermediate 56 (1 g, 5.14 mmol) in nitroethane (10 ml). The reaction mixture was heated to 90°C, stirred for 2 hours, diluted with ethyl acetate (50 ml), washed with saturated NH₄Cl, dried over Na₂SO₄, and concentrated to obtain a crude product, which was purified using a silica gel column (0-50% ethyl acetate in hexane) to afford (E)-7-chloro-1-methyl-2-(2-nitroprop-1-en-1-yl)-1H-pyrrolo[2,3-c]pyridine (1.1 g, 85%) as a yellow solid. LC-MS m/z: 252.3 [M+H] ⁺.

### Intermediate 58

### 4-Bromobenzo[b]thiophene-3-carbaldehyde

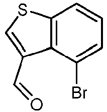

A solution of 4-bromobenzo[b]thiophene (10 g, 46.9 mmol) and dichloro(methoxy)methane (5.39 g, 46.9 mmol) in DCM (500 ml) was stirred and cooled to 0°C, followed by the addition of TiCl₄ (65.17 ml, 591 mmol) dropwise at a room temperature. The resulting reaction mixture was stirred for 16 hours at a room temperature. Thereafter, saturated ammonium chloride (500 ml) was added dropwise to the reaction mixture, and the contents were extracted with DCM (2×500 ml). The combined organic layers were washed with water (200 ml) and brine solution (200 ml), followed by drying over sodium sulphate. The organic layers were filtered, and volatiles were evaporated to dryness under reduced pressure to obtain a crude product, which was purified using a silica gel column (0-20% ethyl acetate in hexane) to afford 4-bromobenzo[b]thiophene-3-carbaldehyde (6 g, 53%). ¹H NMR (300 MHz, DMSO-*d*₆) δ ppm: 10.80-10.95 (m, 1H), 8.80 (s, 1H), 8.21 (dd, *J*=8.31, 0.76 Hz, 1H), 7.79-7.85 (m, 1H), 7.41 (t, *J*=7.93 Hz, 1H).

### Intermediate 59

### (4-Bromobenzo[b]thiophen-3-yl)(cyclopropyl)methanol

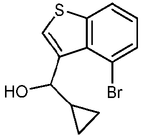

Cyclopropylmagnesium bromide (12.44 ml, 12.44 mmol) was added dropwise under nitrogen atmosphere to a stirred solution of Intermediate 58 (2 g, 8.30 mmol) in THF (30 ml) cooled to -20°C. The reaction mixture stirred for 2 hours, diluted with water (50 ml), and extracted with ethyl acetate (2×50 ml). The combined organic layers were washed with water (100 ml) and brine solution (100 ml), followed by drying over sodium sulphate. The organic layers were filtered, and volatiles were evaporated to dryness under reduced pressure to afford (4-bromobenzo[b]thiophen-3-yl) (cyclopropyl)methanol (2.2 g, 94%). ¹H NMR (300 MHz, DMSO-*d*₆) δ ppm: 7.99-8.08 (m, 1H), 7.84-7.90 (m, 1H), 7.60-7.68 (m, 1H), 7.19-7.28 (m, 1H), 5.52-5.65 (m, 1H), 5.11-5.23 (m, 1H), 1.33-1.48 (m, 1H), 0.27-0.49 (m, 4H).

### Intermediate 60

### 4-Bromo-3-(cyclopropylmethyl)benzo[b]thiophene

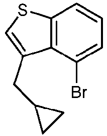

Triethylsilane (14 ml, 49.4 mmol) was added to a stirred solution of Intermediate 50 (14 g, 49.4 mmol) in DCM (200 ml), followed by the addition of TFA (14 ml, 182 mmol) after 5 minutes. The resulting reaction mixture was stirred for 1 hour at a room temperature. Thereafter, the volatiles were evaporated to dryness under reduced pressure, saturated NaHCO₃ (100 ml) was added, and the contents were extracted with DCM (2×150 ml). The combined organic extracts were washed with water (100 ml) and brine solution (100 ml), dried over sodium sulphate, filtered, and volatiles were evaporated to dryness under reduced pressure to obtain a crude product, which was purified using a silica gel column (0-10% ethyl acetate in hexane) to afford 4-bromo-3-(cyclopropylmethyl)benzo[b]thiophene (6 g, 45%). ¹H NMR (300 MHz, DMSO-*d*₆) δ ppm: 8.02 (dd, J=7.93, 0.76 Hz, 1H), 7.70 (s, 1H), 7.63 (dd, J=7.55, 1.13 Hz, 1H), 7.24 (t, J=7.74 Hz, 1H), 3.05 (d, J=6.80 Hz, 2H), 1.24 (br, d, J=3.78 Hz, 1H), 0.54-0.44 (m, 2H), 0.31-0.17 (m, 2H).

### Intermediate 61

### 4-Bromo-3-(cyclopropylmethyl)benzo[b]thiophene-2-carbaldehyde

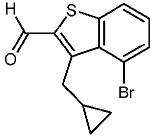

LDA (2M in THF, 11.23 ml, 22.46 mmol) was added dropwise to a stirred solution of Intermediate 60 (3 g, 11.23 mmol) in THF (30 ml) cooled to -78°C, the contents were stirred for 1 hour at -78°C, followed by the addition of DMF (1.739 ml, 22.46 mmol). The resulting reaction mixture was stirred for 3 hours, water (100 ml) was added, and the contents were extracted with ethyl acetate (2×100 ml). The combined organic layers were washed with water (100 ml) and brine solution (100 ml), dried over sodium sulphate, filtered, and volatiles were evaporated to dryness under reduced pressure to afford 4-bromo-3-(cyclopropylmethyl)benzo[b]thiophene-2-carbaldehyde (3g, 91%). ¹H NMR (300 MHz, DMSO-*d₆*) δ ppm: 10.32 (s, 1H), 8.15 (dd, J=8.3, 0.70 Hz, 1H), 8.07-7.92 (m, 1H), 7.45 (t, , J=8.0Hz, (1H), 3.57 (d, J=6.4 Hz, 2H), 1.24-1.03 (m, 1H), 0.54-0.23 (m, 4H). LC-MS m/z: 295.31 [M+H]⁺.

### Intermediate 62

### (E)-4-Bromo-3-(cyclopropylmethyl)-2-(2-nitroprop-1-en-1-yl)benzo[b]thiophene

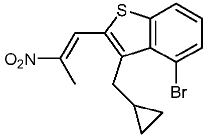

Ammonium acetate (0.261 g, 3.39 mmol) was added to a stirred solution of Intermediate 61 (1 g, 3.39 mmol) in nitroethane (0.242 ml, 3.39 mmol). The reaction mixture was stirred for 2 hours at 90°C, water (100 ml) was added to the reaction mixture, and the contents were extracted with ethyl acetate (2×100 ml). The combined organic layers were washed with water (100 ml), dried over sodium sulphate, filtered, and volatiles were evaporated to dryness under reduced pressure to afford (E)-4-bromo-3-(cyclopropylmethyl)-2-(2-nitroprop-1-en-1-yl)benzo[b]thiophene (1 g, 84%) as a yellow solid. ¹H NMR (300 MHz, DMSO-*d₆*) δ ppm: 8.39 (s, 1H), 8.14 (d, J=8.3 Hz, 1H), 7.76 (d, J=7.6 Hz, 1H), 7.44-7.34 (m, 1H), 3.34 (m, 2 H), 1.29-1.2 (m, 1H), 0.47-0.42 (m, 2H), 0.27 ( d, J=4.5 Hz, 2H).

### Example 1

### ((7R)-7-Amino-2-azabicyclo[2.2.1]heptan-2-yl)(2-(1-(cyclopropylmethyl)-1H-pyrrolo[2,3-b]pyridin-2-yl)-4-methoxy-3-methylpyrazolo[1,5-a]pyridin-6-yl)methanone

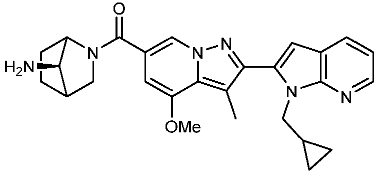

### Intermediate 1A 3-(tert-Butyl)-6-methyl 2-(1-(cyclopropylmethyl)-1H-pyrrolo[2,3-b]pyridin-2-yl)-4-methoxypyrazolo[1,5-a]pyridine-3,6-dicarboxylate

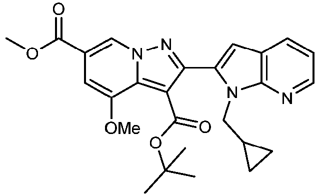

1-Amino-3-methoxy-5-(methoxycarbonyl)pyridin-1-ium (Intermediate 1, 0.618 g, 3.37 mmol) and K₂CO₃ (0.933 g, 6.75 mmol) were added to a stirred solution of tert-butyl 3-(1-(cyclopropylmethyl)-1H-pyrrolo[2,3-b]pyridin-2-yl)propiolate (Intermediate 2, 1 g, 3.37 mmol) in DMF (20 ml). The reaction mixture was stirred at a room temperature for 16 hours, diluted with EtOAc (50 ml), washed with water (3×50 ml) and brine (3×50 ml), dried over Na₂SO₄, and concentrated under reduced pressure to obtain a crude product. The crude product was purified using a silica gel column (24 g, Redisep^{®} SiO₂ column, eluting with 30% EtOAc in hexane) to afford the title compound (650 mg, 40%) as a yellow solid. LC-MS m/z: 477.2 [M+H]⁺. ¹H NMR (400 MHz, chloroform-d) δ ppm: 8.92 (d, J=1.0 Hz, 1H), 8.39 (d, J=4.8 Hz, 1H), 7.98-7.93 (m, 1H), 7.16-7.08 (m, 2H), 6.82 (s, 1H), 4.48 (d, J=7.0 Hz, 2H), 4.04 (s, 3H), 3.99 (s, 3H), 1.43 (m, 9 H), 1.37-1.21 (m, 5H), 0.38-0.25 (m, 4H).

### Intermediate 1B

### 2-(1-(Cyclopropylmethyl)-1H-pyrrolo[2,3-b]pyridin-2-yl)-4-methoxy-6-(methoxycarbonyl)pyrazolo[1,5-a]pyridine-3-carboxylic acid

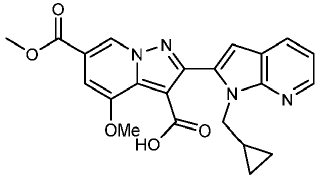

TFA (0.210 ml, 2.73 mmol) was added to a stirred solution of Intermediate 1A (650 mg, 1.364 mmol) in DCM (5 ml). The reaction mixture was stirred at a room temperature for 1 hour, and concentrated to afford the title compound (600 mg, 73%) as a brown solid. The crude compound was used in the next steps without further purification. LC-MS m/z: 421.1 [M+H]⁺.

### Intermediate 1C

### Methyl 2-(1-(cyclopropylmethyl)-1H-pyrrolo-[2,3-b]pyridin-2-yl)-4-methoxy-3-methylpyrazolo[1,5-a]pyridine-6-carboxylate

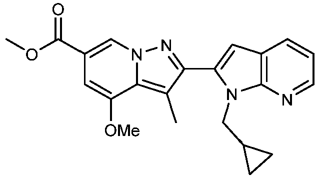

Borane dimethyl sulfide complex (3.57 ml, 7.14 mmol) was added to a stirred solution of Intermediate 1B (600 mg, 1.427 mmol) in THF (10 ml) at 0°C. The reaction mixture was stirred for 30 min, followed by heating to 65°C and stirring for 5 hours, quenched with methanol (5 ml) at 0°C, and concentrated to afford the title compound (520 mg, 93%) as a brown solid. The crude compound was used in the next steps without further purification. LC-MS m/z: 391.2 [M+H]⁺. ¹H NMR (400 MHz, chloroform-d) δ ppm: 8.88-8.76 (m, 1H), 8.44-8.27 (m, 1H), 7.99-7.85 (m, 1H), 7.13-6.90 (m, 1H), 6.89-6.85 (m, 1H), 6.76-6.69 (m, 1H), 4.62-4.44 (m, 2H), 4.07-3.96 (m, 6H), 2.58 (s, 3H), 1.05-0.78 (m, 1H), 0.45-0.11 (m, 4H).

### Intermediate 1D

### 2-(1-(Cyclopropylmethyl)-1H-pyrrolo[2,3-b]pyridin-2-yl)-4-methoxy-3-methylpyrazolo[1,5-a]pyridine-6-carboxylic acid

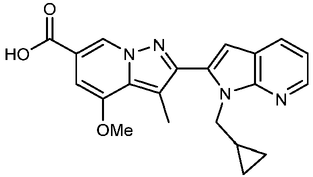

Lithium hydroxide (LiOH, 153 mg, 6.40 mmol) was added to a stirred solution of Intermediate 1C (500 mg, 1.281 mmol) in a mixture of THF (3 ml), methanol (3 ml) and water (3 ml). The reaction mixture was stirred at a room temperature for 30 min, concentrated, and the crude residue was acidified with 1.5 N HCl. The solid product obtained was filtered, and dried under vacuum to afford the title compound (360 mg, 75%) as an off-white solid. LC-MS m/z: 377.1 [M+H]⁺.

### Intermediate 1E

### tert-Butyl-((7R)-2-(2-(1-(cyclopropylmethyl)-1H-pyrrolo[2,3-b]pyridin-2-yl)-4-methoxy-3-methylpyrazolo[1,5-a]pyridine-6-carbonyl)-2-azabicyclo[2.2.1]heptan-7-yl)carbamate

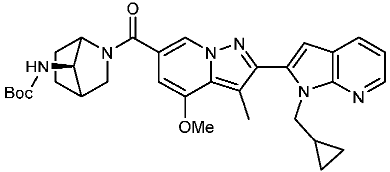

(1-[Bis(dimethylamino)methylene]-1H-1,2,3-triazolo[4,5-b]pyridinium 3-oxide hexafluorophosphate (HATU, 707 mg, 1.860 mmol) and N,N-diisopropylethylamine (DIPEA, 0.487 ml, 2.79 mmol) were added to a stirred solution of Intermediate 1D (350 mg, 0.930 mmol) and tert-butyl ((7R)-2-azabicyclo[2.2.1]- heptan-7-yl)carbamate (197 mg, 0.930 mmol) in DMF (5 ml). The reaction mixture was stirred at a room temperature for 12 hours, poured into ice water (10 ml), and the solid product separated was filtered and dried under vacuum to obtain a crude product, which was purified using a silica gel column (24 g, Redisep^{®} SiO₂ column, eluting with 50% EtOAc in hexane) to afford the title compound (300 mg, 57%) as an off-white solid. LC-MS m/z: 571.2 [M+H]⁺.

### Example 1

TFA (0.364 ml, 4.73 mmol) was added to a stirred solution of Intermediate 1E (270 mg, 0.473 mmol) in DCM (5 ml). T he reaction mixture was stirred at a room temperature for 1 hour, concentrated, and the crude compound obtained was purified using Prep-HPLC. The purified sample was further purified using RP-HPLC to afford the title compound (76 mg, 34%). LC-MS m/z: 471.2 [M+H]⁺. LC-MS retention time: 1.76 min (Method E). ¹H NMR (400 MHz, DMSO-*d*₆) δ ppm: 8.55 (s, 0.5 H), 8.39 (s, 0.5H), 8.31-8.33 (m, 1H), 8.03-8.05 (m, 1H), 7.14-7.17(m, 1H), 6.81 (s, 1H), 6.59-6.65 (m, 1H), 4.47 (br, d, J=7.03 Hz, 2H), 4.09 (br, s, 0.5H), 3.94-4.04 (m, 3H), 3.82 (br s, 0.5H), 3.62-3.77 (m, 0.5 H), 3.03-3.29 (m, 3.5H), 2.52-2.55 (m, 3H), 2.13-2.32 (m, 2H), 1.88-2.08 (m, 3H), 1.58-1.70 (m, 1H), 1.33-1.47 (m, 1H), 1.09-1.19 (m, 1H), 0.17-0.30 (m, 4H).

### Example 2

### ((7R)-7-Amino-2-azabicyclo[2.2.1]heptan-2-yl)(2-(6-chloro-1-(cyclopropylmethyl)-1H-pyrrolo[2,3-b]pyridin-2-yl)-4-methoxy-3-methylpyrazolo[1,5-a]pyridin-6-yl)methanone

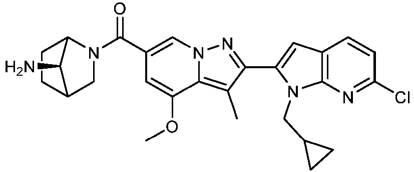

### Intermediate 2A

### 3-(tert-Butyl)-6-methyl 2-(6-chloro-1-(cyclopropylmethyl)-1H-pyrrolo[2,3-b]pyridin-2-yl)-4-methoxypyrazolo[1,5-a]pyridine-3,6-dicarboxylate

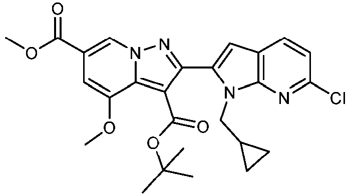

1-Amino-3-methoxy-5-(methoxycarbonyl)pyridin-1-ium (Intermediate 1, 1.384 g, 7.56 mmol) and K₂CO₃ (2.089 g, 15.11 mmol) were added to a stirred solution of tert-butyl 3-(6-chloro-1-(cyclopropylmethyl)-1H-pyrrolo[2,3-b]pyridin-2-yl)propiolate (Intermediate 4, 2.5 g, 7.56 mmol) in DMF (5 ml). The reaction mixture was stirred at a room temperature for 16 hours, diluted with EtOAc (50 ml), washed with water (3×50 ml), dried over Na₂SO₄, and concentrated to obtain a crude product, which was purified using a silica gel column (40 g, Redisep^{®} SiO₂ column, eluting with 30% EtOAc in hexane) to afford the title compound (1.5 g, 39%) as a yellow liquid. LC-MS m/z: 511.2 [M+H]⁺.

### Intermediate 2B

### 2-(6-Chloro-1-(cyclopropylmethyl)-1H-pyrrolo[2,3-b]pyridin-2-yl)-4-methoxy-6-(methoxycarbonyl)pyrazolo[1,5-a]pyridine-3-carboxylic acid

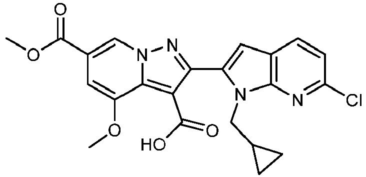

2,6-Lutidine (0.684 ml, 5.87 mmol) and trimethylsilyl trifluoromethanesulfonate (0.530 ml, 2.94 mmol) were added to a solution of Intermediate 2A (1.5 g, 2.94 mmol) in DCM (20 ml). The reaction mixture was stirred at a room temperature for 30 min, diluted with DCM (30 ml), washed with 10% NaHCO₃ solution (3×50 ml), dried over Na₂SO₄, and concentrated to afford the title compound (1.5 g, 79%) as a brown solid. LC-MS m/z: 455.1 [M+H]⁺.

### Intermediate 2C

### Methyl-2-(6-chloro-1-(cyclopropylmethyl)-1H-pyrrolo[2,3-b]pyridin-2-yl)-4-methoxy-3-methylpyrazolo[1,5-a]pyridine-6-carboxylate

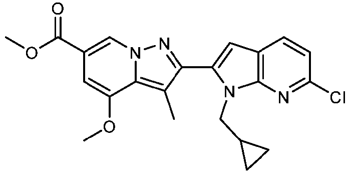

Borane dimethyl sulphide complex (3.30 ml, 6.60 mmol) was added to a stirred solution of Intermediate 2B (1 g, 2.198 mmol) in THF (10 ml) at 0°C. The reaction mixture was stirred at the same temperature for 30 min, then heated to 65°C and stirred for 5 hours, quenched with methanol (5 ml), and the reaction contents were concentrated to afford the title compound as an off-white solid (200 mg, 22%). The crude compound was used in the next steps without further purification. LC-MS m/z: 425.1 [M+H]⁺.

### Intermediate 2D

### 2-(6-Chloro-1-(cyclopropylmethyl)-1H-pyrrolo[2,3-b]pyridin-2-yl)-4-methoxy-3-methylpyrazolo[1,5-a]pyridine-6-carboxylic acid

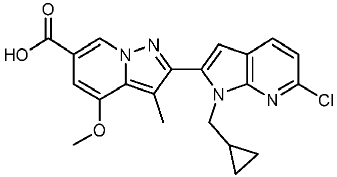

LiOH (141 mg, 5.88 mmol) was added to a stirred solution of Intermediate 2C (500 mg, 1.177 mmol) in a mixture of THF (3 ml), methanol (3 ml) and water (3 ml), and the reaction mixture was stirred at a room temperature for 30 min. The reaction mixture was concentrated under reduced pressure and the residue obtained was acidified with 1.5 N HCl. The separated solids were filtered, and dried under vacuum to afford the title compound (400 mg, 83%) as an off-white solid. LC-MS m/z: 411.1 [M+H]⁺.

### Intermediate 2E

### tert-Butyl-((7R)-2-(2-(6-chloro-1-(cyclopropylmethyl)-1H-pyrrolo[2,3-b]pyridin-2-yl)-4-methoxy-3-methylpyrazolo[1,5-a]pyridine-6-carbonyl)-2-azabicyclo[2.2.1]heptan-7-yl)carbamate

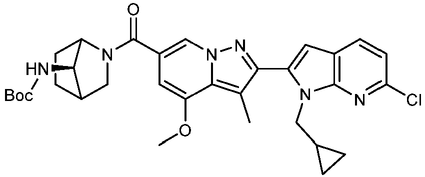

HATU (555 mg, 1.460 mmol) and DIPEA (0.383 ml, 2.191 mmol) were added to a stirred solution of Intermediate 2D (300 mg, 0.730 mmol) and tert-butyl ((7R)-2-azabicyclo[2.2.1]- heptan-7-yl)carbamate (155 mg, 0.730 mmol) in DMF (5 ml). The reaction mixture was stirred at a room temperature for 12 hours, poured into ice water (10 ml), the solid product thus obtained was filtered, and dried under vacuum to afford the title compound (400 mg, 91%). LC-MS m/z: 605.4 [M+H]⁺.

### Example 2

TFA (0.127 ml, 1.653 mmol) was added to a solution of Intermediate 2E (100 mg, 0.165 mmol) in DCM (5 ml). The reaction mixture was stirred at a room temperature for 1 hour, concentrated under reduced pressure, and the crude product obtained was purified using Prep-HPLC. The product was further purified using RP-HPLC to afford the title compound (12 mg, 14%). LC-MS m/z: 505.3 [M+H]⁺. LC-MS retention time: 1.79 min ((Method E). ¹H NMR (400 MHz, DMSO-*d*₆) δ ppm: 8.55 (s, 0.5H), 8.39 (s, 0.5 H), 8.11 (d, J=8.03 Hz, 1H), 7.22 (d, J=8.53 Hz, 1H), 6.87 (s, 1H), 6.60-6.66 (m, 1H), 4.42 (br, d, J=7.03 Hz, 2H), 4.09 (s, 0.5H),3.99 (s, 3H), 3.81 (br, s, 0.5H), 3.48- 61 (m, 2H), 3.12-3.29 (m, 2H), 3.06-3.25 (m, 2H), 2.57-2.66 (m, 1H), 2.53 (br, s, 3H), 1.60-2.23 (m, 4H), 1.39-1.41 (m, 1H), 1.13-1.15 (m, 1H), 0.31 (br, d, J=8.03 Hz, 2H), 0.20 (br, d, J=4.02 Hz, 2H).

### Example 3

### 6-(2-(6-((7R)-7-Amino-2-azabicyclo[2.2.1]heptane-2-carbonyl)-4-methoxy-3-methylpyrazolo[1,5-a]pyridin-2-yl)-1-(cyclopropylmethyl)-1H-pyrrolo[2,3-b]pyridin-6-yl)isoindolin-1-one

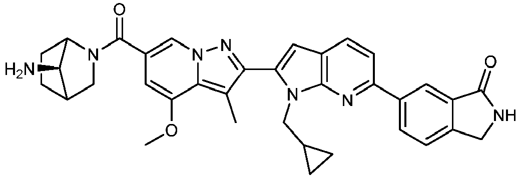

### Intermediate 3A

### tert-Butyl-((7R)-2-(2-(1-(cyclopropylmethyl)-6-(3-oxoisoindolin-5-yl)-1H-pyrrolo[2,3-b]pyridin-2-yl)-4-methoxy-3-methylpyrazolo[1,5-a]pyridine-6-carbonyl)-2-azabicyclo[2.2.1]heptan-7-yl)carbamate

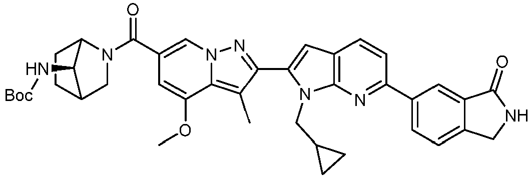

PdCl₂(dppf).CH₂Cl₂ (13.50 mg, 0.017 mmol) was added to a degasified solution of Intermediate 2E (100 mg, 0.165 mmol), (3-oxoisoindolin-5-yl)boronic acid (43.9 mg, 0.248 mmol) and K₂CO₃ (68.5 mg, 0.496 mmol) in dioxane (5 ml) and water (0.556 ml). The reaction mixture was heated to 100°C and stirred for 12 hours, filtered through a celite bed, and the filtrate was concentrated to afford a crude product, which was purified using Prep-HPLC to afford the title compound (16 mg, 14%). LC-MS m/z: 702.4 [M+H]⁺. ¹H NMR (400 MHz, chloroform-d) δ ppm: 8.60 (s, 1H), 8.49-8.46 (m, 1H), 8.28-8.20 (m, 1H), 8.07-7.95 (m, 1H), 7.73-7.64 (m, 1H), 7.62-7.53 (m, 1H), 6.78 (s, 1H), 6.53 (s, 1H), 6.20-6.10 (m, 1H), 4.64-4.41 (m, 5H), 4.01 (s, 3H), 3.89-3.72 (m, 2H), 3.33-3.18 (m, 1H), 2.64-2.50 (m, 4H), 1.95 (br s, 2H), 1.41 (s, 9H), 1.34-1.14 (m, 2H), 0.38-0.25 (m, 4H).

### Example 3

TFA (0.016 ml, 0.214 mmol) was added to a solution of Intermediate 3A (15 mg, 0.021 mmol) in DCM (5 ml). The reaction mixture was stirred for 1 hour, concentrated, and the crude product obtained was purified using Prep-HPLC. The purified product was further purified using RP-HPLC to afford the title compound (7.42mg, 57%). LC-MS m/z: 602.2 [M+H]⁺. HPLC retention time: 6.364 min (Method C). ¹H NMR (400 MHz, DMSO-*d*₆) δ ppm: 8.70-8.57 (m, 1H), 8.46-8.32 (m, 3H), 8.18-8.08 (m, 1H), 7.88-7.77 (m, 1H), 7.72-7.58 (m, 1H), 6.89-6.76 (m, 1H), 6.66-6.52 (m, 1H), 4.71-4.48 (m, 2H), 4.47-4.35 (m, 2H), 4.10-3.88 (m, 5H), 3.87-3.42 (m, 1H), 3.06-3.22 (m, 1H), 2.54-2.41 (m, 3H), 1.76-2.26 (m, 4H), 1.48-1.31 (m, 1H), 1.27-1.15 (m, 1H), 0.44-0.20 (m, 4H).

Compounds of Examples 4 to 12 in Table 1 were prepared following a procedure similar to the preparation of a compound of Example 3, using Suzuki coupling reaction of Intermediate 2E with appropriate boronic acid.

**Table 1**

| Example | Structure, |
|---|---|
| | Name, |
| | Analytical Data: LC-MS m/z [M+H]⁺; HPLC retention time (HPLC method) |
| 4 | 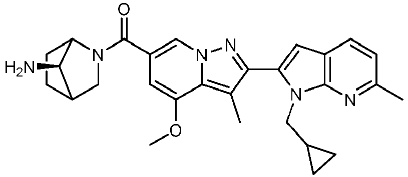 ((7R)-7-Amino-2-azabicyclo[2.2.1]heptan-2-yl)(2-(1-(cyclopropylmethyl)-6-methyl-1H-pyrrolo[2,3-b]pyridin-2-yl)-4-methoxy-3-methylpyrazolo[1,5-a]pyridin-6-yl)methanone. |
| | Analytical data: 485.2, 4.452 min (Method C) |
| 5 | 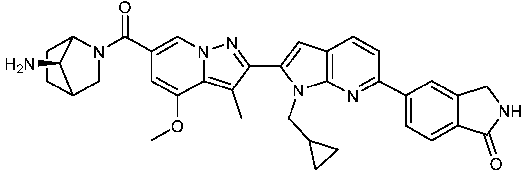 5-(2-(6-((7R)-7-Amino-2-azabicyclo[2.2.1]- heptane-2-carbonyl)-4-methoxy-3-methylpyrazolo[1,5-a]pyridin-2-yl)-1-(cyclopropylmethyl)-1H-pyrrolo[2,3-b]pyridin-6-yl)isoindolin-1-one. |
| | Analytical data: 602.2, 6.281 min (Method C) |
| 6 | 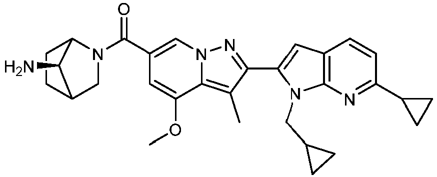 ((7R)-7-Amino-2-azabicyclo[2.2.1]heptan-2-yl)(2-(6-cyclopropyl-1-(cyclopropylmethyl)-1H-pyrrolo[2,3-b]pyridin-2-yl)-4-methoxy-3-methylpyrazolo[1,5-a]pyridin-6-yl)methanone |
| | Analytical data: 511.4, 7.977 min (Method C) |
| 7 | 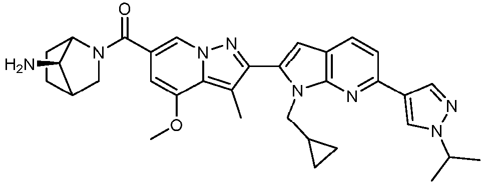 ((7R)-7-Amino-2-azabicyclo[2.2.1]heptan-2-yl)(2-(1-(cyclopropylmethyl)-6-(1-isopropyl-1H-pyrazol-4-yl)-1H-pyrrolo[2,3-b]pyridin-2-yl)-4-methoxy-3-methylpyrazolo[1,5-a]pyridin-6-yl)methanone |
| | Analytical data: 579.4, 6.603 min (Method C) |
| 8 | 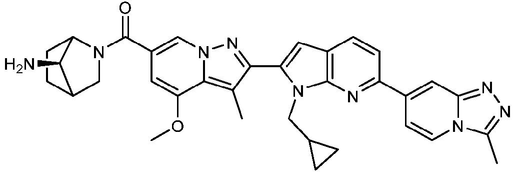 ((7R)-7-Amino-2-azabicyclo[2.2.1]heptan-2-yl)(2-(1-(cyclopropylmethyl)-6-(3-methyl-[1,2,4]triazolo[4,3-a]pyridin-7-yl)-1H-pyrrolo[2,3-b]pyridin-2-yl)-4-methoxy-3-methylpyrazolo[1,5-a]pyridin-6-yl)methanone. |
| | Analytical data: 602.3, 4.761 min (Method C) |
| 9 | 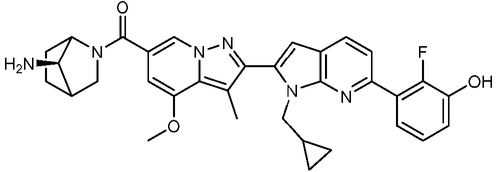 ((7R)-7-Amino-2-azabicyclo[2.2.1]heptan-2-yl)(2-(1-(cyclopropylmethyl)-6-(2-fluoro-3-hydroxyphenyl)-1H-pyrrolo[2,3-b]pyridin-2-yl)-4-methoxy-3-methylpyrazolo[1,5-a]pyridin-6-yl)methanone |
| | Analytical data: 581.3, 7.259 min (Method C) |
| 10 | 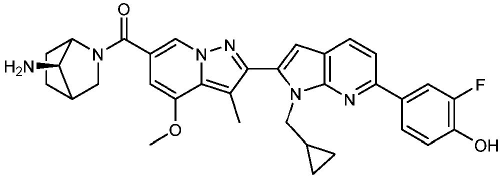 ((7R)-7-Amino-2-azabicyclo[2.2.1]heptan-2-yl)(2-(1-(cyclopropylmethyl)-6-(3-fluoro-4-hydroxyphenyl)-1H-pyrrolo[2,3-b]pyridin-2-yl)-4-methoxy-3-methylpyrazolo[1,5-a]pyridin-6-yl)methanone. |
| | Analytical data: 581.4, 4.619 min (Method C) |
| 11 | 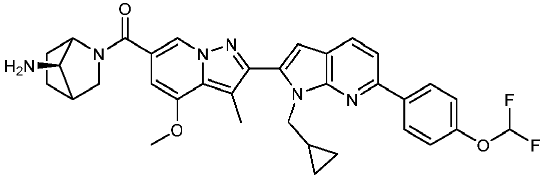 ((7R)-7-Amino-2-azabicyclo[2.2.1]heptan-2-yl)(2-(1-(cyclopropylmethyl)-6-(4-(difluoromethoxy)phenyl)-1H-pyrrolo[2,3-b]pyridin-2-yl)-4-methoxy-3-methylpyrazolo[1, 5-a]pyridin-6-yl)methanone |
| | Analytical data: 613.2, 5.878 min (Method C) |
| 12 | 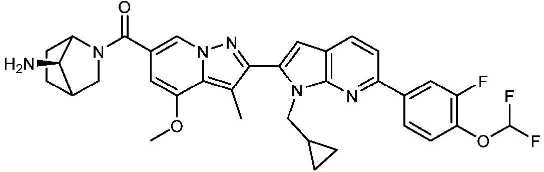 ((7R)-7-Amino-2-azabicyclo[2.2.1]heptan-2-yl)(2-(1-(cyclopropylmethyl)-6-(4-(difluoromethoxy)-3-fluorophenyl)-1H-pyrrolo[2,3-b]pyridin-2-yl)-4-methoxy-3-methylpyrazolo[1,5-a]pyridin-6-yl)methanone. |
| | Analytical data: 631.3, 2.417 min (Method E) |

### Example 13

### ((7R)-7-Amino-2-azabicyclo[2.2.1]heptan-2-yl)(2-(1-(cyclopropylmethyl)-6-(3-methoxyazetidin-1-yl)-1H-pyrrolo[2,3-b]pyridin-2-yl)-4-methoxy-3-methylpyrazolo[1,5-a]pyridin-6-yl)methanone

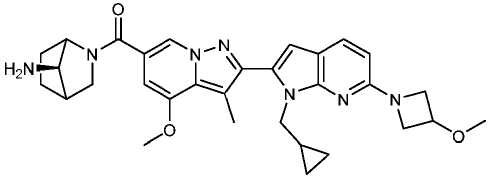

### Intermediate 13A

### tert-Butyl-((7R)-2-(2-(1-(cyclopropylmethyl)-6-(3-methoxyazetidin-1-yl)-1H-pyrrolo[2,3-b]pyridin-2-yl)-4-methoxy-3-methylpyrazolo[1,5-a]pyridine-6-carbonyl)-2-azabicyclo[2.2.1]heptan-7-yl)carbamate

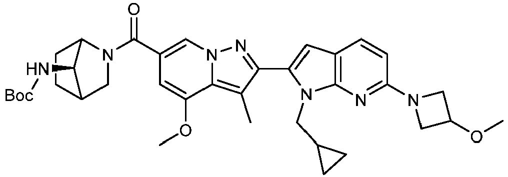

Pd₂(dba)₃ (5.67 mg, 6.20 µmol) and 2-Dicyclohexylphosphino-2',6'-diisopropoxybiphenyl (RUPHOS, 15.42 mg, 0.033 mmol) were added to a degassed solution of Intermediate 2E (100 mg, 0.165 mmol), 3-methoxyazetidine (28.8 mg, 0.331 mmol) and caesium carbonate (162 mg, 0.496 mmol) in dioxane (5 ml). The reaction mixture was heated to 85°C and stirred for 12 hours, filtered, and the filtrate was concentrated under reduced pressure to afford the crude product, which was used in the next step without further purification. LC-MS m/z: 656.5 [M+H]⁺.

### Example 13

TFA (0.117 ml, 1.525 mmol) was added to a stirred solution of Intermediate 13A (100 mg, 0.152 mmol) in DCM (5 ml). The reaction mixture was stirred for 1 hour, concentrated under reduced pressure, and the crude product obtained was purified using Prep-HPLC. The purified sample was further purified using RP-HPLC to afford the title compound (8.5mg, 10%). LC-MS m/z: 556.4 [M+H]⁺. HPLC retention time: 7.703 min (Method C). ¹H NMR (400 MHz, DMSO-*d*₆) δ ppm: 8.56-8.34 (m, 1H), 7.78 (d, J=8.5 Hz, 1H), 6.62-6.57 (m, 2H), 6.27 (d, J=8.5 Hz, 1H), 4.35 (br, d, J=6.0 Hz, 4H), 4.27-4.13 (m, 2H), 3.98 (s, 6H), 3.84-3.58 (m, 5H), 3.29 3.06 (m, 1H),2.67-2.57 (m, 3H), 2.20-1.40 (m, 5H), 1.24-1.12 (m, 1H), 0.32-0.22 (m, 4H).

### Example 14

### ((3R,5R)-3-Amino-5-fluoropiperidin-1-yl)(2-(1-(cyclopropylmethyl)-1H-pyrrolo[2,3-b]pyridin-2-yl)-4-methoxy-3-methylpyrazolo[1,5-a]pyridin-6-yl)methanone

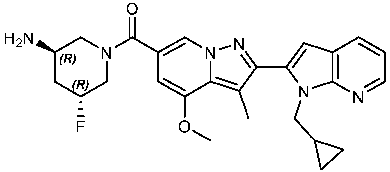

### Intermediate 14A

### tert-Butyl-((3R,5R)-1-(2-(1-(cyclopropylmethyl)-1H-pyrrolo[2,3-b]pyridin-2-yl)-4-methoxy-3-methylpyrazolo[1,5-a]pyridine-6-carbonyl)-5-fluoropiperidin-3-yl)carbamate

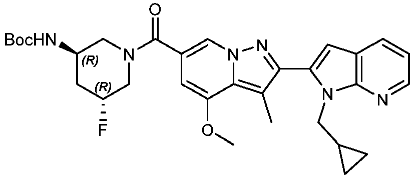

Intermediate 14A was prepared following a procedure similar to the preparation of Intermediate 2E (20 mg, 52%). LC-MS m/z: 577.1 [M+H]⁺.

### Example 14

TFA (0.027 ml, 0.347 mmol) was added to a stirred solution of Intermediate 14A (20 mg, 0.035 mmol) in DCM (1 ml) at a room temperature. The reaction mixture was stirred for 1 hour, concentrated under reduced pressure, and the crude product obtained was purified using Prep-HPLC. The purified product was further purified using RP-HPLC to afford the title compound (6 mg, 36%). LC-MS m/z: 477.1 [M+H]⁺. LC-MS retention time: 1.374 min (method E). ¹H NMR (400 MHz, DMSO-*d*₆) δ ppm: 8.39 (s, 1H), 8.32 (dd, J=1.5, 4.6 Hz, 1H), 8.04 (dd, J=1.7, 7.8 Hz, 1H), 7.16 (dd, J=4.6, 7.8 Hz, 1H), 6.81 (s, 1H), 6.56 (s,1H), 4.89 (br, s, 1H), 4.47 (d, J=7.1 Hz, 2H), 3.98 (s, 4H), 3.03-2.98 (m, 2H), 2.55 (s, 3H), 2.21-2.11 (m, 1H), 1.35-1.09, (m, 3H), 0.32-0.14 (m, 4H).

### Example 15

### ((3R,5R)-3-Amino-5-fluoropiperidin-1-yl)(2-(6-chloro-1-(cyclopropylmethyl)-1H-pyrrolo[2,3-b]pyridin-2-yl)-4-methoxy-3-methylpyrazolo[1,5-a]pyridin-6-yl)methanone

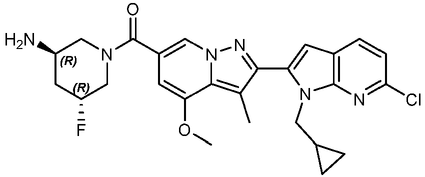

### Intermediate 15A

### tert-Butyl-((3R,5R)-1-(2-(6-chloro-1-(cyclopropylmethyl)-1H-pyrrolo[2,3-b]pyridin-2-yl)-4-methoxy-3-methylpyrazolo[1,5-a]pyridine-6-carbonyl)-5-fluoropiperidin-3-yl)carbamate

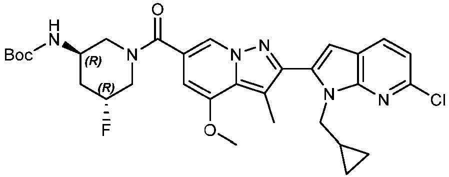

HATU (1296 mg, 3.41 mmol) and DIPEA (0.893 ml, 5.11 mmol) were added to a stirred solution of Intermediate 2D (700 mg, 1.704 mmol) and tert-butyl ((3R,5R)-5-fluoropiperidin-3-yl)carbamate (372 mg, 1.704 mmol) in DMF (5 ml). The reaction mixture was stirred at a room temperature for 12 hours, poured into ice water (40 ml), and the solids obtained were filtered, dried under vacuum to afford the title compound (1g, 67%) as an off-white solid. LC-MS m/z: 611.4 [M+H]⁺.

### Example 15

TFA (0.252 ml, 3.27 mmol) was added to a stirred solution of Intermediate 15A (200 mg, 0.327 mmol) in DCM (5 ml). The reaction mixture was stirred at a room temperature for 1 hour, concentrated under reduced pressure, and the crude product obtained was purified using Prep-HPLC. The purified product was further purified using RP-HPLC to afford the title compound (21 mg, 12%). LC-MS m/z: 608.4 [M+H]⁺. LC-MS retention time: 2.054 min (Method E). ¹H NMR (400 MHz, DMSO-*d*₆) δ ppm: 8.38 (s, 1H), 8.10 (d, J=8.07 Hz, 1H), 7.22 (d, J=8.07 Hz, 1H), 6.87 (s, 1H), 6.56 (s, 1H), 4.96-4.78 (m, 1H), 4.41 (d, J=7.09 Hz, 3H), 3.98 (s, 4H), 2.97 (br, s, 2H), 2.52-2.55 (m, 3H), 2.14 (br, s, 1H), 1.41-1.60 (m, 1H), 1.12-1.20 (m, 1H), 0.27-0.34 (m, 2H), 0.20-0.26 (m, 2H).

### Example 16

### 6-(2-(6-((3R,5R)-3-Amino-5-fluoropiperidine-1-carbonyl)-4-methoxy-3-methylpyrazolo[1,5-a]pyridin-2-yl)-1-(cyclopropylmethyl)-1H-pyrrolo[2,3-b]pyridin-6-yl)isoindolin-1-one

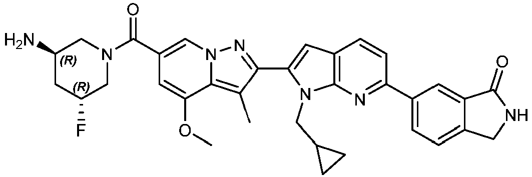

### Intermediate 16A

### tert-Butyl-((3R,5R)-1-(2-(1-(cyclopropylmethyl)-6-(3-oxoisoindolin-5-yl)-1H-pyrrolo[2,3-b]pyridin-2-yl)-4-methoxy-3-methylpyrazolo[1,5-a]pyridine-6-carbonyl)-5-fluoropiperidin-3-yl)carbamate

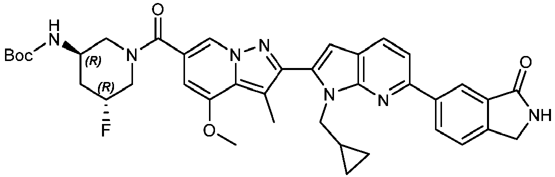

PdCl₂(dppf).CH₂Cl₂ (9.35 mg, 0.011 mmol) was added to a degassed solution of Intermediate 15A (70 mg, 0.115 mmol), (3-oxoisoindolin-5-yl)boronic acid (30.4 mg, 0.172 mmol) and K₂CO₃ (47.5 mg, 0.344 mmol) in dioxane (5 ml) and water (0.556 ml). The reaction mixture was stirred at 100°C for 12 hours, filtered, and the filtrate was concentrated under reduced pressure to afford a crude product. The crude product was used in next the step without further purification. LC-MS m/z: 708.5 [M+H]⁺.

### Example 16

TFA (0.076 ml, 0.989 mmol) was added to a solution of Intermediate 16A (70 mg, 0.099 mmol) in DCM (5 ml). The reaction mixture was stirred for 1 hour, concentrated under reduced pressure, and the crude product obtained was purified using Prep-HPLC. The purified product was further purified using RP-HPLC to afford the title compound (12 mg, 19%). LC-MS m/z: 608.4 [M+H]⁺. HPLC retention time: 6.559 min (method E). ¹H NMR (400 MHz, DMSO-*d*₆) δ ppm: 8.65 (s, 1H), 8.48-8.41 (m, 3H), 8.16 (d, J=8.4 Hz, 1H), 8.10-7.98 (m, 1H), 7.99 (brs, 1H), 7.88 (d, J=8.0 Hz, 1H), 7.73 (d, J=8.0 Hz, 1H), 6.95 (s, 1H), 6.59 (s, 1H), 5.08-4.55 (m, 1H), 4.59 (d, J=6.8 Hz, 3H), 4.46 (s, 3H), 4.00 (s, 3H), 3.20 (br d, J=10.5 Hz, 1H), 2.57-2.53 (m, 3H), 2.49-2.27 (m, 2H), 1.78-1.58 (m, 1H), 1.34-1.11 (m, 2H), 0.34-0.26 (m, 4H).

Compounds of Examples 17 to 21 in Table 2 were prepared following a procedure similar to the preparation of a compound of Example 16, using the Intermediate 15A and the appropriate boronic acid.

**Table 2**

| Example | Structure |
|---|---|
| | Name |
| | Analytical Data: LC-MS m/z [M+H]⁺; HPLC retention time (HPLC method) |
| 17 | 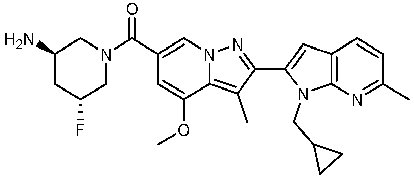 ((3R,5R)-3-Amino-5-fluoropiperidin-1-yl)(2-(1-(cyclopropylmethyl)-6-methyl-1H-pyrrolo[2,3-b]pyridin-2-yl)-4-methoxy-3-methylpyrazolo[1,5-a]pyridin-6-yl)-methanone. |
| | Analytical Data: 491.4, 4.563 min (Method C) |
| 18 | 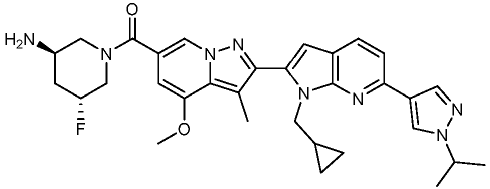 ((3R,5R)-3-Amino-5-fluoropiperidin-1-yl)(2-(1-(cyclopropylmethyl)-6-(1-isopropyl-1H-pyrazol-4-yl)-1H-indol-2-yl)-4-methoxy-3-methylpyrazolo[1,5-a]pyridin-6-yl)methanone |
| | Analytical Data: 585.5, 7.566 min (Method C) |
| 19 | 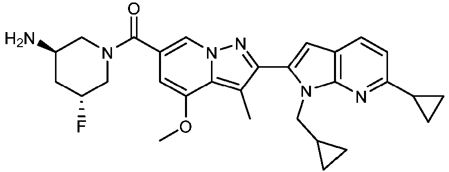 ((3R,5R)-3-Amino-5-fluoropiperidin-1-yl)(2-(6-cyclopropyl-1-(cyclopropylmethyl)-1H-pyrrolo[2,3-b]pyridin-2-yl)-4-methoxy-3-methylpyrazolo[1,5-a]pyridin-6-yl)methanone |
| | Analytical Data: 585.5, 7.566 min (Method C) |
| 20 | 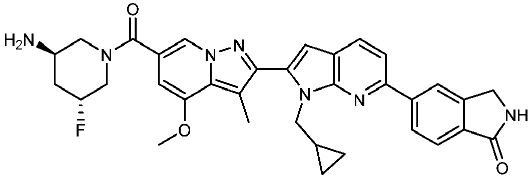 5-(2-(6-((3R,5R)-3-Amino-5-fluoropiperidine-1-carbonyl)-4-methoxy-3-methylpyrazolo[1,5-a]pyridin-2-yl)-1-(cyclopropylmethyl)-1H-pyrrolo[2,3-b]pyridin-6-yl)isoindolin-1-one |
| | Analytical Data: 608.3, 5.958 min (Method C) |
| 21 | 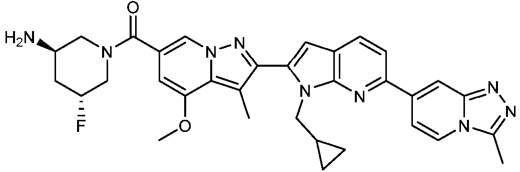 ((3R,5R)-3-Amino-5-fluoropiperidin-1-yl)(2-(1-(cyclopropylmethyl)-6-(3-methyl-[1,2,4]triazolo[4,3-a]pyridin-7-yl)-1H-pyrrolo[2,3-b]pyridin-2-yl)-4-methoxy-3-methylpyrazolo[1,5-a]pyridin-6-yl)methanone |
| | Analytical Data: 608.4, 5.179 min (Method C) |

### Example 22

### ((7R)-7-Amino-2-azabicyclo[2.2.1]heptan-2-yl)(2-(1-(cyclopropylmethyl)-1H-indol-2-yl)-4-methoxy-3-methylpyrazolo[1,5-a]pyridin-6-yl)methanone

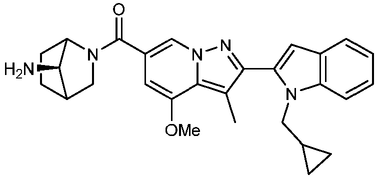

### Intermediate 22A

### 3-(tert-Butyl)-6-methyl 2-(1-(cyclopropylmethyl)-1H-indol-2-yl)-4-methoxypyrazolo[1,5-a]pyridine-3,6-dicarboxylate

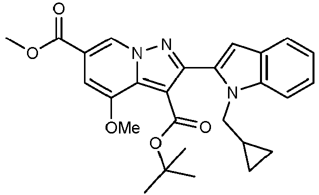

tert-Butyl-3-(1-(cyclopropylmethyl)-1H-indol-2-yl)propiolate (1g, 3.39 mmol) and K₂CO₃ (0.468 g, 3.39 mmol) were added to a stirred solution of 1-amino-3-methoxy-5-(methoxy-carbonyl)pyridin-1-ium (Intermediate 1, 0.620 g, 3.39 mmol) in DMF (10 ml). The reaction mixture was stirred at a room temperature for 48 hours, quenched with water (100 ml), and extracted with EtOAc (2×100 ml). The combined organic layers were dried over Na₂SO₄, filtered, and concentrated under reduced pressure to obtain a crude product, which was purified using a silica gel column (EtOAc in hexane). The product was isolated at 10-20% EtOAc in hexane, required fractions were collected and volatiles were evaporated to dryness under reduced pressure to afford the title compound (480 mg, 30%). LC-MS m/z: 476.2 [M+H]⁺.

### Intermediate 22B

### 2-(1-(Cyclopropylmethyl)-1H-indol-2-yl)-4-methoxy-6-(methoxycarbonyl)pyrazolo[1,5-a]pyridine-3-carboxylic acid

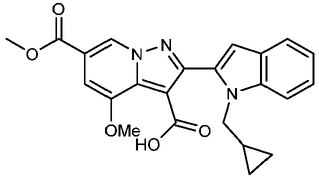

Formic acid (0.028 ml, 0.736 mmol) was added to a solution of Intermediate 22A (350 mg, 0.736 mmol) in DCM (1 ml). The reaction mixture was stirred at a room temperature for 4 hours, quenched with saturated NaHCO₃ solution, and extracted with DCM (2×100 ml). The combined the organic layers were dried over Na₂SO₄, filtered, and concentrated under reduced pressure to afford the crude title compound (150 mg, 49%) LC-MS m/z: 420.2 [M+H]⁺.

### Intermediate 22C

### Methyl-2-(1-(cyclopropylmethyl)-1H-indol-2-yl)-4-methoxy-3-methylpyrazolo[1,5-a]pyridine-6-carboxylate

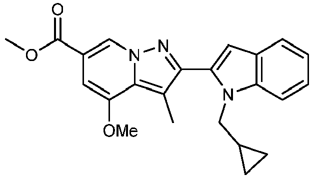

Borane dimethyl sulfide complex (0.834 ml, 1.669 mmol) was added to a stirred solution of Intermediate 22B (350 mg, 0.834 mmol) in THF (2 ml). The reaction mixture was stirred at 50°C for 5 hours, cooled to a room temperature, quenched with methanol, and concentrated under reduced pressure to afford a crude product, which was purified using a silica gel column (EtOAc in hexane (10-20%)) to afford the title compound (100 mg, 31%) LC-MS /z: 390.2 [M+H]⁺.

### Intermediate 22D

### 2-(1-(Cyclopropylmethyl)-1H-indol-2-yl)-4-methoxy-3-methylpyrazolo[1,5-a]pyridine-6-carboxylic acid

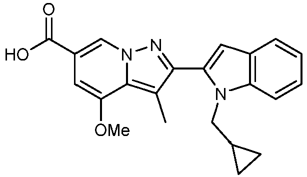

LiOH (9.39 mg, 0.392 mmol) was added to a stirred solution of Intermediate 22C (100 mg, 0.131 mmol) in THF (6 ml) and water (6 ml). The reaction mixture was stirred at a room temperature for 16 hours, concentrated under reduced pressure to evaporate THF, and acidified with 1N HCl. The solid product separated was filtered and dried under vacuum to afford the title compound (80 mg, 163%); LC-MS m/z: 376.2 [M+H]⁺.

### Intermediate 22E

### tert-Butyl-((7R)-2-(2-(1-(cyclopropylmethyl)-1H-indol-2-yl)-4-methoxy-3-methylpyrazolo[1,5-a]pyridine-6-carbonyl)-2-azabicyclo[2.2.1]heptan-7-yl)carbamate

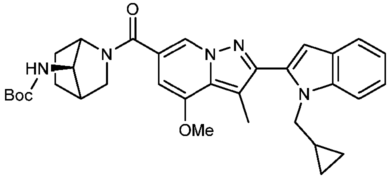

HATU (161 mg, 0.424 mmol), DIPEA (0.185 ml, 1.060 mmol) and tert-butyl ((7R)-2-azabicyclo[2.2.1]heptan-7-yl)carbamate (90 mg, 0.424 mmol) were added to a stirred solution of Intermediate 22D (260 mg, 0.353 mmol) in DMF (2 ml) at 0°C. The reaction mixture was stirred at a room temperature for 2 hours, quenched with water (100 ml), and extracted with EtOAc (2×100 ml). The combined the organic layers were dried over sodium sulphate, filtered, and concentrated under reduced pressure to afford the crude product (100 mg, 49%). LC-MS m/z: 570.3 [M+H]⁺.

### Example 22

TFA (0.022 ml, 0.281 mmol) was added to a stirred solution of Intermediate 24E (80 mg, 0.140 mmol) in DCM (2 ml). The reaction mixture was stirred at a room temperature for 16 hours, evaporated completely under reduced pressure, and the crude product obtained was purified using Prep-HPLC. The purified product was further purified using RP-HPLC to afford the title compound (13.4 mg, 20%). LC-MS m/z: 470.3 [M+H]⁺. LC-MS retention time: 2.0 min (Method E). ¹H NMR (400 MHz, DMSO-*d*₆) δ ppm: 8.63-8.40 (m, 1H), 8.32-8.01 (m, 1H), 7.62 (t, J=7.3 Hz, 2H), 7.33-6.93 (m, 2H), 6.76 (s, 1H), 6.67-6.50 (m, 1H), 4.58-4.25 (m, 3H), 4.08-3.96 (m, 3H), 3.90 (s, 1H), 3.87-3.80 (m, 1H), 3.67-3.54 (m, 2H), 3.30-3.14 (m, 1H), 2.97-2.60 (m, 2H), 2.05-1.58 (m, 2H), 1.29-1.13 (m, 2H), 1.13-1.03 (m, 1H), 0.34-0.21 (m, 2H), 0.12 (q, J=4.8 Hz, 2H).

### Example 23

### 6-(2-(6-((7R)-7-Amino-2-azabicyclo[2.2.1]heptane-2-carbonyl)-4-methoxy-3-methylpyrazolo[1,5-a]pyridin-2-yl)-1-(cyclopropylmethyl)-1H-indol-6-yl)isoindolin-1-one

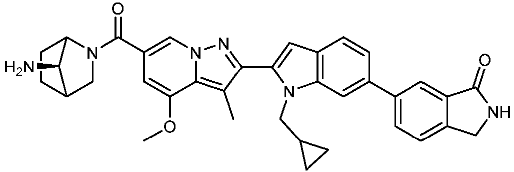

### Intermediate 23A

### 3-(tert-Butyl)-6-methyl 2-(6-bromo-1-(cyclopropylmethyl)-1H-indol-2-yl) -4-methoxypyrazolo[1,5-a]pyridine-3,6-dicarboxylate

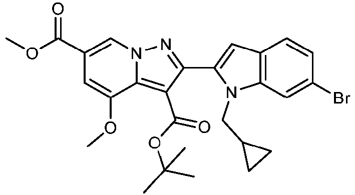

tert-Butyl 3-(6-bromo-1-(cyclopropylmethyl)-1H-indol-2-yl)propiolate (Intermediate 6, 5.5g, 14.69 mmol) and K₂CO₃ (6.09 g, 44.1 mmol) were added to a solution of 1-amino-3-methoxy-5-(methoxycarbonyl)pyridin-1-ium 2,4-dinitrophenolate (Intermediate 1, 8.07 g, 22.04 mmol) in DMF (50 ml). The reaction mixture was stirred overnight, concentrated under a vacuum at ~45°C over a period of 1 hour, diluted with EtOAc, and filtered through a celite bed. The filtrate was further washed with water, dried over Na₂SO₄, filtered, and the organic layer was concentrated under vacuum to obtain a crude compound, which was purified using a silica gel column (40 g, Redisep^{®} SiO₂ column, eluting with 30% EtOAc in hexane) to afford the title compound (5.3g, 65%). LC-MS m/z: 500.1 [M+H]⁺. ¹H NMR (400 MHz, DMSO-*d*₆) δ ppm: 9.04-8.91 (m, 1H), 8.56-7.96-7.84 (m, 1H), 7.63-7.53 (m, 1H), 7.30-7.06 (m, 2H), 6.80-6.70 (m, 1H), 4.44-4.30 (m, 2H), 3.96-3.86 (m, 6H), 1.43-1.36 (m, 8H), 1.12-1.02 (m, 1H), 0.34-0.24 (m, 2H), 0.18-0.12 (m, 2H).

### Intermediate 23B

### 2-(6-Bromo-1-(cyclopropylmethyl)-1H-indol-2-yl)-4-methoxy-6-(methoxycarbonyl)pyrazolo[1,5-a]pyridine-3-carboxylic acid

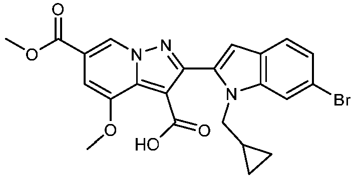

2,6-Lutidine (8.91 ml, 76 mmol) was added to a stirred solution of Intermediate 23A (5.3g, 9.56 mmol) in DCM (50 ml), followed by the addition of trimethylsilyl trifluoromethanesulfonate (6.91 ml, 38.2 mmol) at 0-5°C. The reaction mixture was stirred for 2 hours at the same temperature, quenched with 10% NaHCO₃ solution, stirred for 10 min, and diluted with EtOAc (50 ml). The organic layer was separated, and aqueous layer was extracted with EtOAc (3×25 ml). The combined organic layers were dried over Na₂SO₄, and concentrated under vacuum at a room temperature to obtain the title compound as a pale yellow syrup (5.5 g, 102%). LC-MS m/z: 500 [M+H]⁺.

### Intermediate 23C

### Methyl-2-(6-bromo-1-(cyclopropylmethyl)-1H-indol-2-yl)-4-methoxy-3-methylpyrazolo[1,5-a]pyridine-6-carboxylate

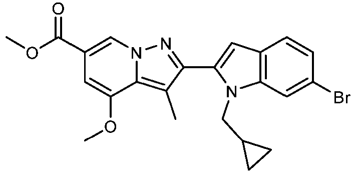

Borane-methyl sulfide complex (2.52 ml, 26.6 mmol) was added to a stirred solution of Intermediate 23B (5.3 g, 10.64 mmol) in THF (50 ml) at 0°C. The reaction mixture was stirred for 30 min, slowly heated to 50°C and stirred for 4 hours, followed by stirring at a room temperature for 12 hours. The reaction mixture was cooled to 0°C, quenched by careful addition of methanol, and concentrated under vacuum to obtain a crude residue. The crude product was triturated with water (3×25 ml), and the sticky off-white residue was purified using a silica gel column (40 g, Redisep^{®} SiO₂ column, eluting with 0 to 50% EtOAc in hexane) to afford the title compound (1.1g, 22%). LC-MS m/z: 470.1 [M+H]⁺.

### Intermediate 23D

### 2-(6-Bromo-1-(cyclopropylmethyl)-1H-indol-2-yl)-4-methoxy-3-methylpyrazolo[1,5-a]pyridine-6-carboxylic acid

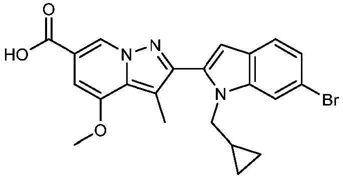

LiOH (0.169 g, 7.05 mmol) in water (5 ml) was added to a stirred suspension of Intermediate 23C (1.1 g, 2.349 mmol) in a mixture of methanol (20 ml) and THF (5 ml). The reaction mixture was stirred for 4 hours, concentrated under vacuum to obtain a crude salt, which was neutralized using 1.5 N HCl. The solids obtained were filtered and dried under vacuum to afford the title compound (930 mg, 87%) as an off-white solid. LC-MS m/z: 455 [M+H]⁺.

### Intermediate 23E tert-Butyl-((7R)-2-(2-(6-bromo-1-(cyclopropylmethyl)-1H-indol-2-yl)-4-methoxy-3-methylpyrazolo[1,5-a]pyridine-6-carbonyl)-2-azabicyclo[2.2.1]heptan-7-yl)carbamate

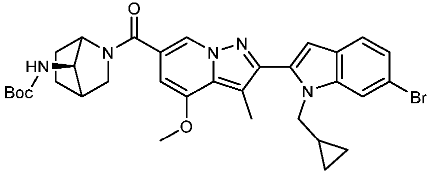

HATU (250 mg, 0.658 mmol), DIPEA (0.265 ml, 1.519 mmol) and tert-butyl ((7R)-2-azabicyclo[2.2.1]heptan-7-yl)carbamate (107 mg, 0.506 mmol) were successively added to a stirred solution of Intermediate 23D (230 mg, 0.506 mmol) in DMF (2 ml). The reaction mixture was stirred for 3 hours, cooled, and treated with ice-cold water and stirred for 10 min. The solids obtained were filtered, and dried in vacuum to afford the title compound (300 mg, 91%) as an off-white solid. LC-MS m/z: 650.3 [M+H]. ¹H NMR (400 MHz, DMSO-*d*₆) δ ppm: 8.59-8.50 (m, 1H), 8.42-8.31 (m, 1H), 7.89 (s, 1H), 7.59 (d, J=8.3 Hz, 1H), 7.22 (d, J=8.8 Hz, 1H), 7.14 -7.01 (m, 1H), 6.80 (s, 1H), 6.68-6.60 (m, 1H), 4.40 (br, d, J=6.4 Hz, 2H), 4.25-4.12 (m, 1H), 4.00 (br, s, 3H), 3.84-3.64 (m, 1H), 3.58-3.43 (m,1H), 3.21-2.86 (m, 2H), 2.44 (br, s, 3H), 2.33 (br, s, 1H), 2.08-1.74 (m, 3H), 1.35 (br, s, 10H), 0.29 (br d,J=7.6 Hz, 2H), 0.11 (br, s, 2H).

### Intermediate 23F

### tert-Butyl-((7R)-2-(2-(1-(cyclopropylmethyl)-6-(3-oxoisoindolin-5-yl)-1H-indol-2-yl)-4-methoxy-3-methylpyrazolo[1,5-a]pyridine-6-carbonyl)-2-azabicyclo[2.2.1]heptan-7-yl)carbamate

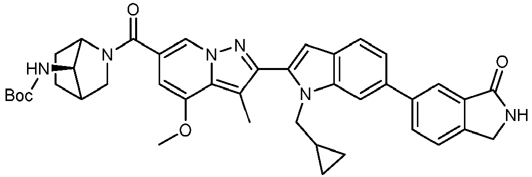

K₂CO₃ (29.8 mg, 0.216 mmol) and PdCl₂(dppf) (7.90 mg, 10.79 µmol) were added to a solution of Intermediate 23E (70 mg, 0.108 mmol) and 6-(4,4,5,5-tetramethyl-1,3,2-dioxaborolan-2-yl)isoindolin-1-one (28.0 mg, 0.108 mmol) in dioxane (3 ml) in a 8 ml chemglass vial. The reaction mixture was degassed using argon, heated to 85°C and stirred for 8 hours, filtered, and concentrated under vacuum to afford a crude product, which was purified using Prep-HPLC to obtain the title compound (40 mg, 53%) as a white solid. LC-MS retention time: 0.94 min. LC-MS m/z: 701.5 [M+H]⁺.

### Example 23

TFA (0.1ml, 1.298 mmol) was added to a solution of Intermediate 23F (40 mg, 0.057 mmol) in DCM (1 ml). The reaction mixture was stirred for 2 hours, concentrated under vacuum, the residue obtained was treated with 28% ammonia solution, and kept on standby for 30 min. The aqueous layer was decanted, and solids left in the flask were dried under vacuum. The crude product was further purified using RP-HPLC to afford the title compound (25 mg, 0.040 mmol, 70%), which was lyophilized to afford a white solid. LC-MS m/z: 601.4 [M+H]⁺. HPLC retention time: 6.958 min (Method C). ¹H NMR (400 MHz, DMSO-*d*₆) δ ppm: 8.63-8.48 (m, 1H), 8.78-8.43 (m, 1H), 8.43-8.35 (m, 1H), 8.05-7.94 (m, 3H), 7.80-7.65 (m, 2H), 7.50-7.40 (m, 1H), 6.85-6.75 (m, 1H), 6.68-6.57 (m, 1H), 4.56-4.46 (m, 2H), 4.46-4.40 (m, 2H), 4.24-4.18 (m, 1H), 4.03-3.85 (m, 3H), 3.82-3.46 (m, 1H), 3.15-3.06 (m, 1H), 2.53 (s, 3H), 2.30-2.20 (m, 1H), 2.10-1.78 (m, 4H), 1.52-1.37 (m, 1H), 1.19-1.10 (m, 1H), 0.36-0.26 (m, 2H), 0.19-0.13 (m, 2H).

Compounds of Examples 24 to 28 in Table 3 were prepared following a procedure similar to the preparation of a compound of Example 23, using Suzuki coupling of Intermediate 23E with appropriate boronic acid.

**Table 3**

| Example | Structure |
|---|---|
| | Name |
| | Analytical Data: LC-MS m/z [M+H]⁺; HPLC retention time (HPLC method ) |
| 24 | 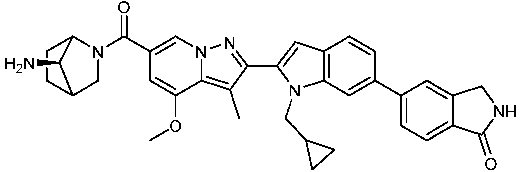 5-(2-(6-((7R)-7-Amino-2-azabicyclo[2.2.1]heptane-2-carbonyl)-4-methoxy-3-methylpyrazolo[1,5-a]pyridin-2-yl)-1-(cyclopropylmethyl)-1H-indol-6-yl)isoindolin-1-one |
| | Analytical Data: 601.4, 6.779 min (Method C) |
| 25 | 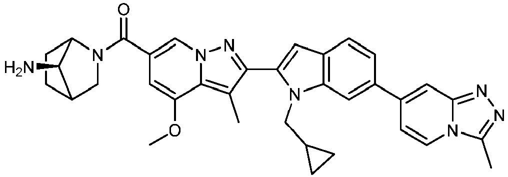 ((7R)-7-Amino-2-azabicyclo[2.2.1]heptan-2-yl)(2-(1-(cyclopropylmethyl)-6-(3-methyl-[1,2,4]triazolo[4,3-a]pyridin-7-yl)-1H-indol-2-yl)-4-methoxy-3-methylpyrazolo[1,5-a]pyridin-6-yl)methanone |
| | Analytical Data: 601.4, 5.325 min (Method C) |
| 26 | 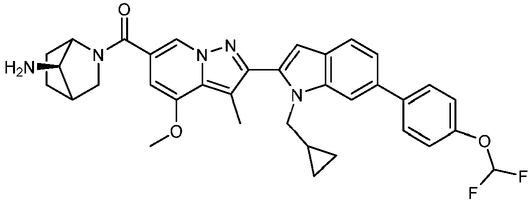 ((7R)-7-Amino-2-azabicyclo[2.2.1]heptan-2-yl)(2-(1-(cyclopropylmethyl)-6-(4-(difluoromethoxy)phenyl)-1H-indol-2-yl)-4-methoxy-3-methylpyrazolo[1,5-a]pyridin-6-yl)methanone |
| | Analytical Data: 612.4, 5.954 min (Method C) |
| 27 | 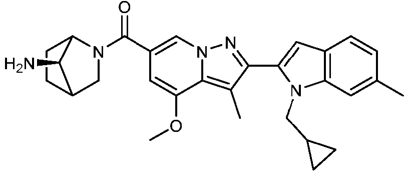 ((7R)-7-Amino-2-azabicyclo[2.2.1]heptan-2-yl)(2-(1-(cyclopropylmethyl)-6-methyl-1H-indol-2-yl)-4-methoxy-3-methylpyrazolo[1,5-a]pyridin-6-yl)methanone |
| | Analytical Data: 484.3, 5.698 min ( Method C) |
| 28 | 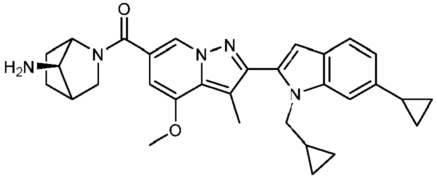 ((7R)-7-Amino-2-azabicyclo[2.2.1]heptan-2-yl)(2-(6-cyclopropyl-1-(cyclopropylmethyl)-1H-indol-2-yl)-4-methoxy-3-methylpyrazolo[1,5-a]pyridin-6-yl)methanone |
| | Analytical Data: 511.3, 4.777 min (Method C) |

### Example 29

### 6-(2-(6-((3R,5R)-3-Amino-5-fluoropiperidine-1-carbonyl)-4-methoxy-3-methylpyrazolo[1,5-a]pyridin-2-yl)-1-(cyclopropylmethyl)-1H-indol-6-yl)isoindolin-1-one

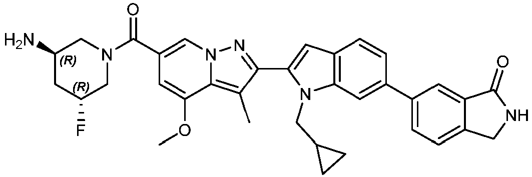

### Intermediated 29A

### tert-Butyl-((3R,5R)-1-(2-(6-bromo-1-(cyclopropylmethyl)-1H-indol-2-yl)-4-methoxy-3-methylpyrazolo[1,5-a]pyridine-6-carbonyl)-5-fluoropiperidin-3-yl)carbamate

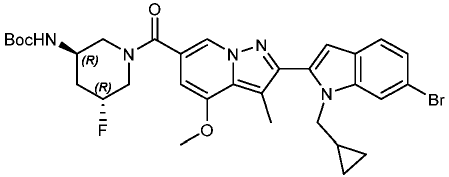

HATU (1742 mg, 4.58 mmol) followed by DIPEA (2.401 ml, 13.74 mmol) were added to a stirred solution of Intermediate 23D (2081 mg, 4.58 mmol) and tert-butyl ((3R,5R)-5-fluoropiperidin-3-yl)carbamate (1000 mg, 4.58 mmol) in DMF (3 ml). The reaction mixture was stirred at a room temperature overnight, cooled to 0°C, and ice-cold water was added. The solids obtained were filtered, washed with water, and purified using a silica gel column (0-1% methanol/chloroform) to afford the title compound (1.4g, 47%) as an off-white solid. LC-MS m/z: 655.4 [M+H]⁺.

### Intermediate 29B

### tert-Butyl-((3R,5R)-1-(2-(1-(cyclopropylmethyl)-6-(3-oxoisoindolin-5-yl)-1H-indol-2-yl)-4-methoxy-3-methylpyrazolo[1,5-a]pyridine-6-carbonyl)-5-fluoropiperidin-3-yl)carbamate

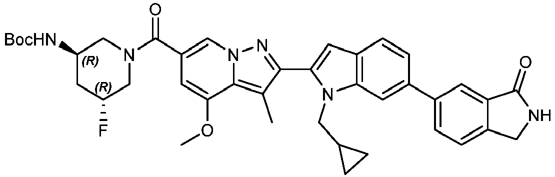

K₂CO₃ (29.6 mg, 0.214 mmol) and PdCl₂(dppf) (7.82 mg, 10.69 µmol) were added to a solution of Intermediate 29A (70 mg, 0.107 mmol) and 6-(4,4,5,5-tetramethyl-1,3,2-dioxaborolan-2-yl)isoindolin-1-one (27.7 mg, 0.107 mmol) in dioxane (3 ml). The reaction mixture was degassed using argon, heated to 85°C and stirred for 8 hours, filtered, and the filtrate was concentrated under vacuum to obtain a crude compound, which was purified using Prep-HPLC to afford the title compound (40 mg, 53%) as a white solid. LC-MS m/z: 707.5 [M+H]⁺.

### Example 29

TFA (0.1ml, 1.298 mmol) was added to a solution of Intermediate 29B (40 mg, 0.057 mmol) in DCM (1 ml). The reaction mixture was stirred for 2 hours, concentrated under vacuum, the crude compound obtained was treated with 28% ammonia solution, and kept on standby for 30 minutes. The aqueous layer was decanted, and the solids left in the flask were dried under vacuum. The product was further purified using RP-HPLC to afford the title compound (18 mg, 51%), which was lyophilized to afford white solid. LC-MS m/z: 607.4 [M+H]⁺. HPLC retention time: 7.167 min (Method C). ¹H NMR (400 MHz, DMSO-*d*₆) δ ppm: 8.54 (s, 1H), 8.38 (s, 1H), 7.98 (d, J=5.5 Hz, 2H), 7.89 (d, J=7.5 Hz, 1H), 7.78-7.65 (m, 2H), 7.49 (d, J=8.3Hz, 1H), 6.81 (s, 1H), 6.56 (s, 1H), 5.02-4.92 (m, 1H), 4.55-4.41 (m, 5H), 4.00 (s, 4H), 3.50-3.36 (m, 1H), 3.28-3.05 (m, 1H), 2.47-2.35 (m, 3H), 1.92-1.89 (m, 1H), 1.69 (br d, J=1.0 Hz, 1H), 1.24-1.20 (m, 1H), 0.40-0.29(m, 2H), 0.21-0.11 (m, 2H).

Compounds of Examples 30 to 33 in Table 4 were prepared following a procedure similar to the preparation of a compound of Example 29, by Suzuki coupling of Intermediate 29A with appropriate boronic acid.

**Table 4**

| Example | Structure |
|---|---|
| | Name |
| | Analytical Data: LC-MS m/z [M+H]⁺; HPLC retention time (HPLC method 1) |
| 30 | 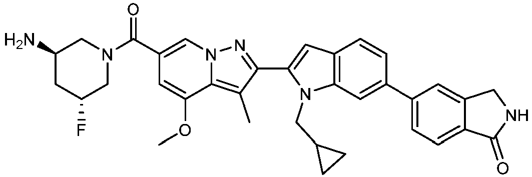 5-(2-(6-((3R,5R)-3-Amino-5-fluoropiperidine-1-carbonyl)-4-methoxy-3-methylpyrazolo[1,5-a]pyridin-2-yl)-1-(cyclopropylmethyl)-1H-indol-6-yl)isoindolin-1-one |
| | Analytical Data: 607.2, 6.951 min (Method C) |
| 31 | 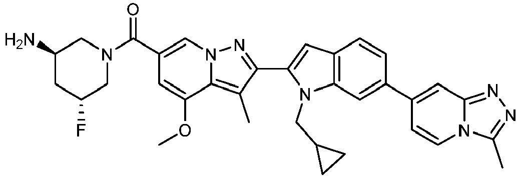 ((3R,5R)-3-Amino-5-fluoropiperidin-1-yl)(2-(1-(cyclopropylmethyl)-6-(3-methyl-[1,2,4]triazolo[4,3-a]pyridin-7-yl)-1H-indol-2-yl)-4-methoxy-3-methylpyrazolo[1,5-a]pyridin-6-yl)methanone |
| | Analytical Data: 674, 5.471 min (Method C) |
| 32 | 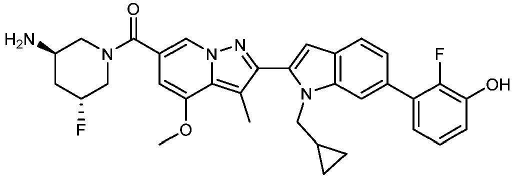 ((3R,5R)-3-Amino-5-fluoropiperidin-1-yl)(2-(1-(cyclopropylmethyl)-6-(2-fluoro-3-hydroxyphenyl)-1H-indol-2-yl)-4-methoxy-3-methylpyrazolo[1,5-a]pyridin-6-yl)methanone |
| | Analytical Data: 586.4, 1.964 min (Method E) |

### Example 33

### ((3R,5R)-3-Amino-5-fluoropiperidin-1-yl)(2-(1-(cyclopropylmethyl)-6-(3-(methoxymethyl)- azetidin-1-yl)-1H-indol-2-yl)-4-methoxy-3-methylpyrazolo[1,5-a]pyridin-6-yl)methanone

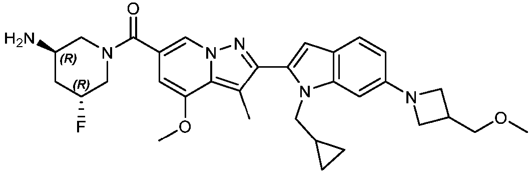

### Intermediate 33A

### tert-Butyl-((3R,5R)-1-(2-(1-(cyclopropylmethyl)-6-(3-(methoxymethyl)azetidin-1-yl)-1H-indol-2-yl)-4-methoxy-3-methylpyrazolo[1,5-a]pyridine-6-carbonyl)-5-fluoropiperidin-3-yl)carbamate

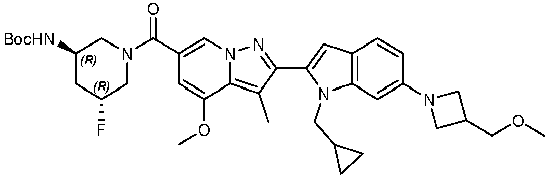

Pd₂(dba)₃ (12 mg, 0.013 mmol), Cs₂CO₃ (100 mg, 0.307 mmol), and RUPHOS (13 mg, 0.028 mmol) were added to a solution of Intermediate 29A (65 mg, 0.099 mmol) and 3-(methoxymethyl)- azetidine (10.04 mg, 0.099 mmol) in dioxane (3 ml) in a 8 ml chemglass vial. The reaction mixture was degassed using argon, heated to 110°C and stirred for 14 hours, filtered, and concentrated under vacuum to afford a crude compound (70 mg, 104%), which was used in the next step without further purification. LC-MS m/z: 675.5 [M+H]⁺.

### Example 33

TFA (0.3ml, 3.89 mmol) was added to a solution of Intermediate 33A (70 mg, 0.104 mmol) in DCM (1 ml). The reaction mixture was stirred for 2 hours, concentrated under vacuum, and the crude product obtained was purified using prep-HPLC. The product was further purified using RP-HPLC to afford the title compound (13 mg, 22%). LC-MS m/z: 575 [M+H]⁺. LC-MS retention time: 1.963 min (Method E). ¹H NMR (400 MHz, methanol-*d*₄) δ ppm: 8.27 (d, *J*=1.0 Hz, 1H), 7.46 (d, *J*=8.5 Hz, 1H), 6.59 (s, 1H), 6.55 (s, 2H), 6.45 (dd, *J*=2.0, 8.5 Hz, 1H), 5.01 -4.96 (m, 1H), 4.27 (d, *J*=6.5 Hz, 2H), 4.09-3.96 (m, 6H), 3.72-3.64 (m, 4H), 3.51-3.35 (m, 1H), 3.31-3.14 (m, 2H), 3.06-2.88(m, 1H), 2.88-2.68 (m, 1H), 2.53 (s, 3H), 2.45-2.19 (m, 1H), 1.75-1.54 (m, 1H), 1.42-1.23 (m, 1H), 1.19-0.99 (m, 1H), 0.35-0.28 (m, 2H), 0.13 -0.01 (m, 2H),

Compounds of Examples 34 to 37 in Table 5 were prepared following a procedure similar to the preparation of a compound of Example 33, by Buchwald coupling of Intermediate 29A with appropriate amine.

**Table 5**

| Example | Structure |
|---|---|
| | Name |
| | Analytical Data: LC-MS m/z [M+H]⁺; HPLC retention time (HPLC method) |
| 34 | 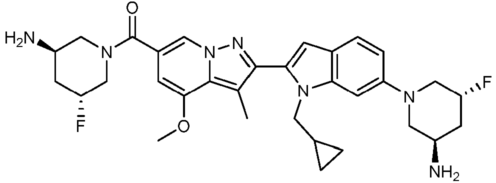 ((3R,5R)-3-Amino-5-fluoropiperidin-1-yl)(2-(6-((3R,5R)-3-amino-5-fluoropiperidin-1-yl)-1-(cyclopropylmethyl)-1H-indol-2-yl)-4-methoxy-3-methylpyrazolo[1,5-a]pyridin-6-yl)methanone |
| | Analytical Data: 592.4, 1.216 min (Method E) |
| 35 | 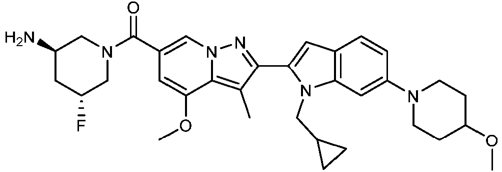 ((3R,5R)-3-Amino-5-fluoropiperidin-1-yl)(2-(1-(cyclopropylmethyl)-6-(4-methoxypiperidin-1-yl)-1H-indol-2-yl)-4-methoxy-3-methylpyrazolo[1,5-a]pyridin-6-yl)methanone |
| | Analytical Data: 589.4, 1.970 min (Method E) |
| 36 | 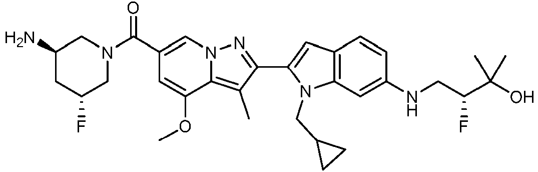 ((3R,5R)-3-Amino-5-fluoropiperidin-1-yl)(2-(1-(cyclopropylmethyl)-6-(((R)-2-fluoro-3-hydroxy-3-methylbutyl)amino)-1H-indol-2-yl)-4-methoxy-3-methylpyrazolo[1,5-a]pyridin-6-yl)methanone |
| | Analytical Data: 595.4, 1.712 min (Method E) |
| 37 | 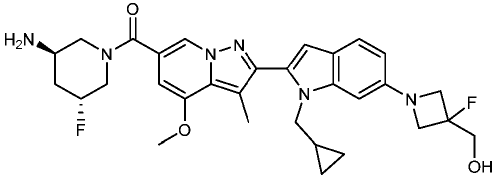 ((3R,5R)-3-Amino-5-fluoropiperidin-1-yl)(2-(1-(cyclopropylmethyl)-6-(3-fluoro-3-(hydroxymethyl)azetidin-1-yl)-1H-indol-2-yl)-4-methoxy-3-methylpyrazolo[1,5-a]pyridin-6-yl)methanone |
| | Analytical Data: 579.4, 1.428 min (Method F) |

### Example 38

### ((7R)-7-Amino-2-azabicyclo[2.2.1]heptan-2-yl)(2-(1-(cyclopropylmethyl)-1H-pyrrolo[2,3-b]pyridin-2-yl)-4-fluoro-3-methylpyrazolo[1,5-a]pyridin-6-yl)methanone

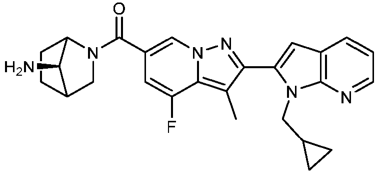

### Intermediate 38A

### 3-(tert-Butyl)-6-methyl 2-(1-(cyclopropylmethyl)-1H-pyrrolo[2,3-b]pyridin-2-yl)-4-fluoropyrazolo[1,5-a]pyridine-3,6-dicarboxylate

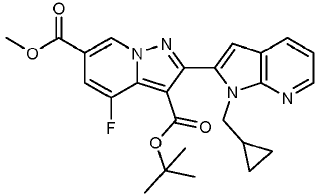

Intermediate 38A was prepared following a procedure similar to the preparation of Intermediate 1A, and using 1-amino-3-fluoro-5-(methoxycarbonyl)pyridin-1-ium 2,4,6-trimethylbenzenesulfonate (Intermediate 9) and tert-butyl 3-(1-(cyclopropylmethyl)-1H-pyrrolo[2,3-b]pyridin-2-yl)propiolate (Intermediate 2), as a pale yellow solid (200 mg, 43%). LC-MS m/z: 465.1 [M+H]⁺.

### Intermediate 38B

### 2-(1-(Cyclopropylmethyl)-1H-pyrrolo[2,3-b]pyridin-2-yl)-4-fluoro-6-(methoxycarbonyl)pyrazolo[1,5-a]pyridine-3-carboxylic acid

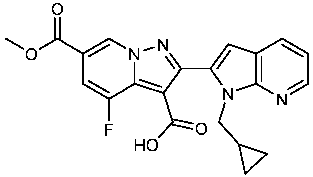

Intermediate 38B was prepared following a procedure similar to the preparation of Intermediate 1B, as a pale yellow solid (170 mg, 56%). LC-MS m/z: 409.2 [M+H]⁺.

### Intermediate 38C

### Methyl-2-(1-(cyclopropylmethyl)-1H-pyrrolo[2,3-b]pyridin-2-yl)-4-fluoro-3-methylpyrazolo[1,5-a]pyridine-6-carboxylate

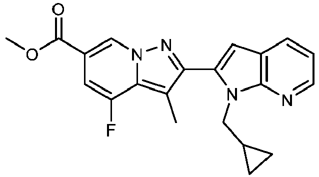

Intermediate 38C was prepared following a procedure similar to the preparation of Intermediate 1C, as a brown solid (142 mg, 90%). LC-MS m/z: 379.2 [M+H]⁺.

### Intermediate 38D

### 2-(1-(Cyclopropylmethyl)-1H-pyrrolo[2,3-b]pyridin-2-yl)-4-fluoro-3-methylpyrazolo[1,5-a]pyridine-6-carboxylic acid

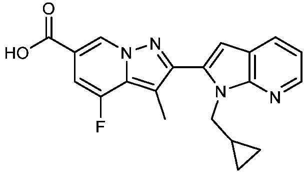

Intermediate 38D was prepared following a procedure similar to the preparation of Intermediate, as an off white solid (125 mg, 93%). LC-MS m/z: 365.2 [M+H]⁺.

### Intermediate 38E

### tert-Butyl-((7R)-2-(2-(1-(cyclopropylmethyl)-1H-pyrrolo[2,3-b]pyridin-2-yl)-4-fluoro-3-methylpyrazolo[1,5-a]pyridine-6-carbonyl)-2-azabicyclo[2.2.1]heptan-7-yl)carbamate

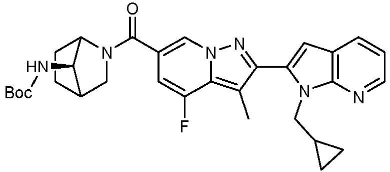

Intermediate 38E was prepared following a procedure similar to preparation of Intermediate 1E, as an off white solid (60 mg, 66%). LC-MS m/z: 559.3 [M+H]⁺.

### Example 38

Compound of Example 38 was prepared following a procedure similar to the preparation of a compound of Example 1 (8.07 mg, 19%). LC-MS m/z: 459.3 [M+H]⁺. HPLC retention time: 5.349 min (Method C). ¹H NMR (400 MHz, DMSO-*d*₆) δ ppm: 8.87-8.72 (m, 1H), 8.34 (dd, J=4.52, 1.51 Hz, 1H), 8.07 (dd, J=7.78, 1.76 Hz, 1H), 7.13-7.19 (m, 2H), 6.88 (s, 1H), 4.50 (br, d, J=6.53 Hz, 2H), 4.08-3.81 (m, 1H), 3.75-3.45 (m, 2H), 3.10-3.29 (m, 3H), 2.53 (s, 3H), 2.09-2.32 (m, 2H), 1.96 (br, s, 3H), 1.87-1.65 (m, 1H), 1.36-1.46 (m, 1H), 1.24 (s, 1H), 0.21-0.45 (m, 4H).

### Example 39

### ((7R)-7-Amino-2-azabicyclo[2.2.1]heptan-2-yl)(2-(6-chloro-1-(cyclopropylmethyl)-1H-pyrrolo[2,3-b]pyridin-2-yl)-4-fluoro-3-methylpyrazolo[1,5-a]pyridin-6-yl)methanone

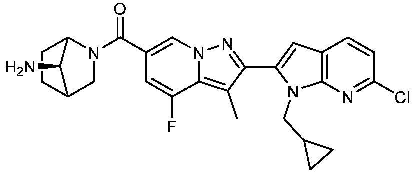

### Intermediate 39A

### 3-(tert-Butyl)-6-methyl 2-(6-chloro-1-(cyclopropylmethyl)-1H-pyrrolo[2,3-b]pyridin-2-yl)-4-fluoropyrazolo[1,5-a]pyridine-3,6-dicarboxylate:

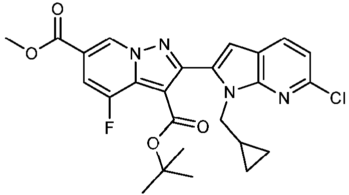

Intermediate 39A was preparedfollowing a procedure similar to the preparation of Intermediate 2A, as a yellow solid (1.5 g, 33%). LC-MS m/z: 499.1 [M+H]⁺.

### Intermediate 39B

### 2-(6-Chloro-1-(cyclopropylmethyl)-1H-pyrrolo[2,3-b]pyridin-2-yl)-4-fluoro-6-(methoxycarbonyl)pyrazolo[1,5-a]pyridine-3-carboxylic acid:

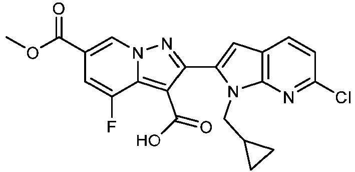

Intermediate 39B was prepared following a procedure similar to preparation of Intermediate 2B, as a brown solid (1.3 g, 98%). LC-MS m/z: 443.1 [M+H]⁺.

### Intermediate 39C

### Methyl-2-(6-chloro-1-(cyclopropylmethyl)-1H-pyrrolo[2,3-b]pyridin-2-yl)-4-fluoro-3-methylpyrazolo[1,5-a]pyridine-6-carboxylate:

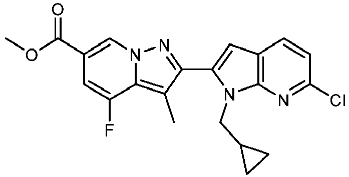

Intermediate 39B was prepared following a procedure similar to the preparation of Intermediate 2C, as a brown solid (1.21 g, 50%). LC-MS m/z: 413.2 [M+H]⁺.

### Intermediate 39D

### 2-(6-Chloro-1-(cyclopropylmethyl)-1H-pyrrolo[2,3-b]pyridin-2-yl)-4-fluoro-3-methylpyrazolo[1,5-a]pyridine-6-carboxylic acid:

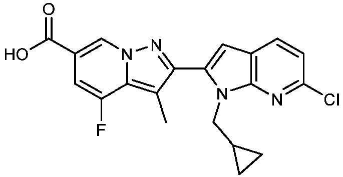

Intermediate 39D was prepared following a procedure similar to the preparation of Intermediate 2D, as a brown solid (1.2 g, 67%) as a brown solid. LC-MS m/z: 399.2 [M+H]⁺.

### Intermediate 39E

### tert-Butyl-((7R)-2-(2-(6-chloro-1-(cyclopropylmethyl)-1H-pyrrolo[2,3-b]pyridin-2-yl)-4-fluoro-3-methylpyrazolo[1,5-a]pyridine-6-carbonyl)-2-azabicyclo[2.2.1]heptan-7-yl)carbamate:

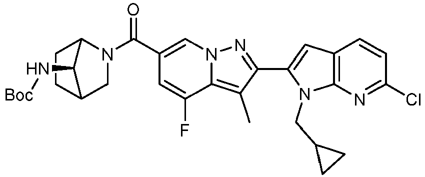

Intermediate 39E was prepared following a procedure similar to the preparation of Intermediate 2E, as a brown solid (700 mg, 78%) as a brown solid. LC-MS m/z: 593.3 [M+H]⁺.

### Example 39

Compound of Example 39 was prepared following a procedure similar to the preparation of a compound of Example 2 (7.2 mg, 8%). LC-MS m/z: 493.3 [M+H]⁺. LC-MS retention time: 2.105 min (Method E). ¹H NMR (400 MHz, DMSO-*d*₆) δ ppm: 8.95-8.71 (m, 1H), 8.13 (d, J=8.3 Hz, 1H), 7.29-7.11 (m, 2H), 6.95 (s, 1H), 4.56-4.18 (m, 2.5H), 4.28-4.21 (s, 0.5H), 3.84-3.75 (m, 0.5 H), 3.57 (br s, 1.5H), 3.19 (br d, J=10.8 Hz, 0.5H), 2.69-2.58 (m, 1.5H), 2.03-1.81 (m, 3H), 1.69-1.58 (m, 1H), 1.28 -1.10 (m, 2H), 0.40-0.14 (m, 4H).

### Example 40

### 3((7R)-7-Amino-2-azabicyclo[2.2.1]heptan-2-yl)(2-(1-(cyclopropylmethyl)-6-methyl-1H-pyrrolo[2,3-b]pyridin-2-yl)-4-fluoro-3-methylpyrazolo[1,5-a]pyridin-6-yl)methanone:

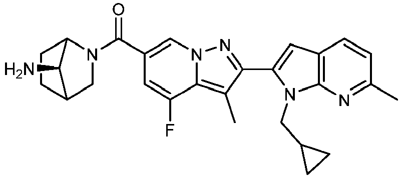

### Intermediate 40A

### tert-Butyl-((7R)-2-(2-(1-(cyclopropylmethyl)-6-methyl-1H-pyrrolo[2,3-b]pyridin-2-yl)-4-fluoro-3-methylpyrazolo[1,5-a]pyridine-6-carbonyl)-2-azabicyclo[2.2.1]heptan-7-yl)carbamate:

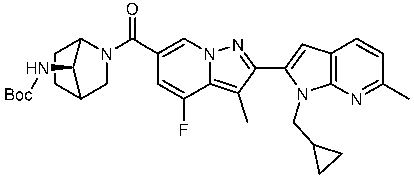

PdCl₂(dppf). DCM complex (13.77 mg, 0.017 mmol) was added to a degassed and stirred solution of Intermediate 39E (100 mg, 0.169 mmol), trimethylboraxine (31.7 mg, 0.253 mmol), and K₂CO₃ (69.9 mg, 0.506 mmol) in dioxane (5 ml) and water (0.556 ml). The reaction mixture was stirred at 100°C for 12 hours, filtered, and concentrated under reduced pressure to afford a crude compound. The crude compound was used in the next step without further purification. LC-MS m/z: 573.3 [M+H]⁺.

### Example 40

Compound of Example 40 was prepared following a procedure similar to the preparation of Example 3 to obtain the tittle compound as a brown solid (15 mg, 18%). LC-MS m/z: 473.2 [M+H]⁺. HPLC retention time: 4.76 min (Method C). ¹H NMR (400 MHz, DMSO-*d*₆) δ ppm: 8.90-8.77 (m, 1H), 8.04-7.93 (m, 1H), 7.23 (s, 1H), 7.08-7.04 (m, 2H), 6.96-6.81(m, 1H), 4.63-4.47 (m, 2.5H), 4.27 (brs, 0.5H), 3.80 (brs, 1H), 3.58 (brs, 2H), 2.62 (s, 3H), 2.53 (s, 3H), 2.02-1.85 (m, 2H), 1.70-1.66 (m, 1H), 1.28-1.02 (m, 3H), 0.41-0.19 (m, 4H).

Compounds of Examples 41 to 49 in Table 6 were prepared following a procedure similar to the preparation of a compound of Example 40, using Suzuki coupling of Intermediate 39E with appropriate boronic acid.

**Table 6**

| Example | Structure |
|---|---|
| | Name |
| | Analytical Data: LC-MS m/z [M+H]⁺; HPLC retention time (HPLC method) |
| 41 | 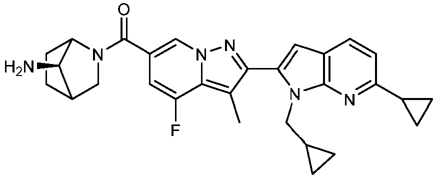 ((7R)-7-Amino-2-azabicyclo[2.2.1]heptan-2-yl)(2-(6-cyclopropyl-1-(cyclopropylmethyl)-1H-pyrrolo[2,3-b]pyridin-2-yl)-4-fluoro-3-methylpyrazolo[1,5-a]pyridin-6-yl)methanone |
| | Analytical Data: 499.4, 6.953 min (Method C) |
| 42 | 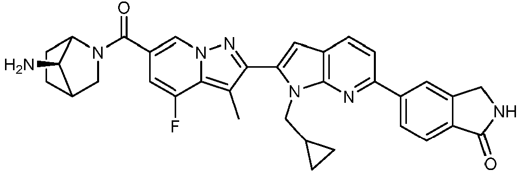 5-(2-(6-((7R)-7-Amino-2-azabicyclo[2.2.1]heptane-2-carbonyl)-4-fluoro-3-methylpyrazolo[1,5-a]pyridin-2-yl)-1-(cyclopropylmethyl)-1H-pyrrolo[2,3-b]pyridin-6-yl)isoindolin-1-one |
| | Analytical Data: 590.2, 12.831 min (Method G) |
| 43 | 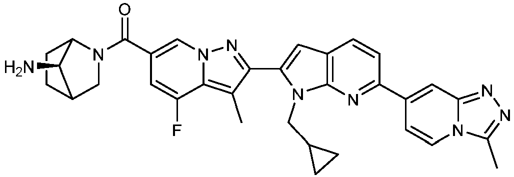 ((7R)-7-Amino-2-azabicyclo[2.2.1]heptan-2-yl)(2-(1-(cyclopropylmethyl)-6-(3-methyl-[1,2,4]triazolo[4,3-a]pyridin-7-yl)-1H-pyrrolo[2,3-b]pyridin-2-yl)-4-fluoro-3-methylpyrazolo[1,5-a]pyridin-6-yl)methanone |
| | Analytical Data: 587.2, 5.439 min (Method C) |
| 44 | 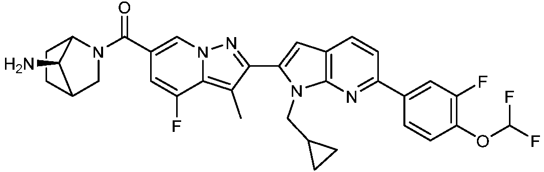 ((7R)-7-Amino-2-azabicyclo[2.2.1]heptan-2-yl)(2-(1-(cyclopropylmethyl)-6-(4-(difluoromethoxy)-3-fluorophenyl)-1H-pyrrolo[2,3-b]pyridin-2-yl)-4-fluoro-3-methylpyrazolo[1,5-a]pyridin-6-yl)methanone |
| | Analytical Data: 619.4, 6.063 min (Method C) |
| 45 | 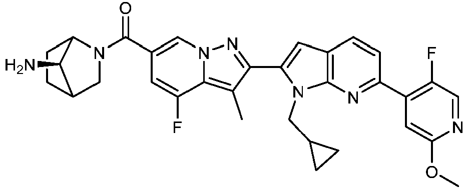 ((7R)-7-Amino-2-azabicyclo[2.2.1]heptan-2-yl)(2-(1-(cyclopropylmethyl)-6-(5-fluoro-2-methoxypyridin-4-yl)-1H-pyrrolo[2,3-b]pyridin-2-yl)-4-fluoro-3-methylpyrazolo[1,5-a]pyridin-6-yl)methanone |
| | Analytical Data: 584.2, 5.467 min (Method C) |
| 46 | 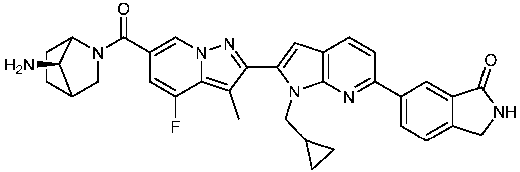 6-(2-(6-((7R)-7-Amino-2-azabicyclo[2.2.1]heptane-2-carbonyl)-4-fluoro-3-methylpyrazolo[1,5-a]pyridin-2-yl)-1-(cyclopropylmethyl)-1H-pyrrolo[2,3-b]pyridin-6-yl)isoindolin-1-one |
| | Analytical Data: 590.3, 6.629 min (Method C) |
| 47 | 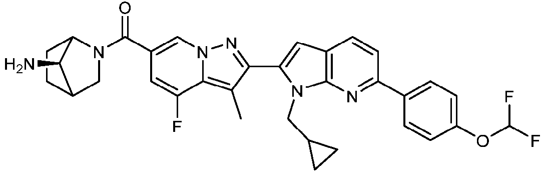 ((7R)-7-Amino-2-azabicyclo[2.2.1]heptan-2-yl)(2-(1-(cyclopropylmethyl)-6-(4-(difluoromethoxy)phenyl)-1H-pyrrolo[2,3-b]pyridin-2-yl)-4-fluoro-3-methylpyrazolo[1,5-a]pyridin-6-yl)methanone |
| | Analytical Data: 601.4, 5.976 min (Method C) |
| 48 | 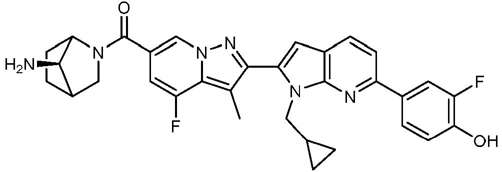 ((7R)-7-Amino-2-azabicyclo[2.2.1]heptan-2-yl)(2-(1-(cyclopropylmethyl)-6-(3-fluoro-4-hydroxyphenyl)-1H-pyrrolo[2,3-b]pyridin-2-yl)-4-fluoro-3-methylpyrazolo[1,5-a]pyridin-6-yl)methanone |
| | Analytical Data: 567.2, 7.351 min (Method C) |
| 49 | 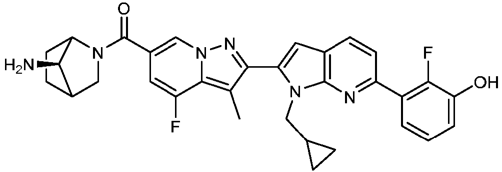 ((7R)-7-Amino-2-azabicyclo[2.2.1]heptan-2-yl)(2-(1-(cyclopropylmethyl)-6-(2-fluoro-3-hydroxyphenyl)-1H-pyrrolo[2,3-b]pyridin-2-yl)-4-fluoro-3-methylpyrazolo[1,5-a]pyridin-6-yl)methanone |
| | Analytical Data: 569.3, 7.115 min (Method C) |

### Example 50

### ((7R)-7-Amino-2-azabicyclo[2.2.1]heptan-2-yl)(2-(1-(cyclopropylmethyl)-6-(3-methoxyazetidin-1-yl)-1H-pyrrolo[2,3-b]pyridin-2-yl)-4-fluoro-3-methylpyrazolo[1,5-a]pyridin-6-yl)methanone

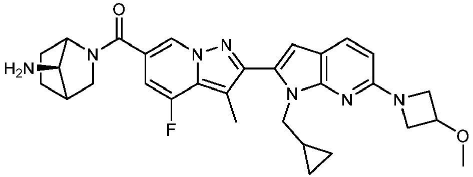

### Intermediate 50A

### tert-Butyl-((7R)-2-(2-(1-(cyclopropylmethyl)-6-(3-methoxyazetidin-1-yl)-1H-pyrrolo[2,3-b]pyridin-2-yl)-4-fluoro-3-methylpyrazolo[1,5-a]pyridine-6-carbonyl)-2-azabicyclo[2.2.1]heptan-7-yl)carbamate

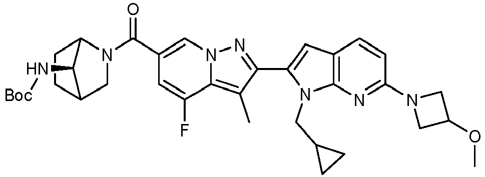

Intermediate 50A was prepared following a procedure similar to the preparation of Intermediate 13A as a brown solid (crude 100 mg, 92%). LC-MS m/z: 644.5 [M+H]⁺.

### Example 50

Compound of Example 50 was prepared following a procedure similar to the preparation of a compound of Example 13, as a brown solid (23 mg, 26%). LC-MS m/z: 544.4 [M+H]⁺. HPLC retention time: 7.768 min (Method C). ¹H NMR (400 MHz, DMSO-*d*₆) δ ppm: 8.82 (s, 0.5H), 8.66 (s, 0.5 H), 7.80 (d, J=8.5 Hz, 1H), 7.13 (s, 1H), 7.10 (s, 1H), 6.68 (s, 1H), 6.29 (d, J=8.0 Hz, 1H), 4.53-4.28 (m, 4H), 4.27-4.14 (m, 2.5 H), 4.08 (br s, 0.5 H), 3.80 (dd, J=8.8, 4.3 Hz, 4H), 3.62-3.36 (m, 2H), 3.25-3.13 (m, 2H), 2.53 (m, 3H), 2.32-1.83 (m, 5H), 1.40-1.38 (m,1H), 1.22-1.20 (m 2H), 0.34-0.24 (m, 4H).

### Example 51

### ((7R)-7-Amino-2-azabicyclo[2.2.1]heptan-2-yl)(2-(1-(cyclopropylmethyl)-6-((R)-3-hydroxypiperidin-1-yl)-1H-pyrrolo[2,3-b]pyridin-2-yl)-4-fluoro-3-methylpyrazolo[1,5-a]pyridin-6-yl)methanone

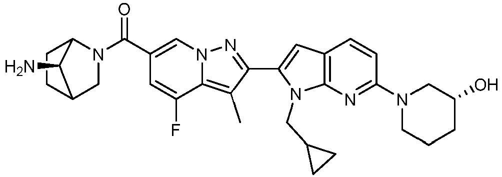

Compound of Example 51 was prepared following a procedure similar to the preparation of a compound of Example 50, by Buchwald coupling of Intermediate 39E with appropriate amine. LC-MS m/z: 558.4 [M+H]⁺, HPLC retention time: 5.795 min (method C).

### Example 52

### ((3R,5R)-3-Amino-5-fluoropiperidin-1-yl)(2-(1-(cyclopropylmethyl)-1H-pyrrolo[2,3-b]pyridin-2-yl)-4-fluoro-3-methylpyrazolo[1,5-a]pyridin-6-yl)methanone

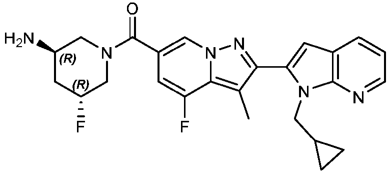

### Intermediate 52A

### tert-Butyl-((3R,5R)-1-(2-(1-(cyclopropylmethyl)-1H-pyrrolo[2,3-b]pyridin-2-yl)-4-fluoro-3-methylpyrazolo[1,5-a]pyridine-6-carbonyl)-5-fluoropiperidin-3-yl)carbamate

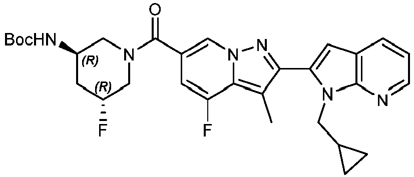

Intermediate 52A was prepared following a procedure similar to the preparation of Intermediate 14A as off-white solid (40 mg, 43%). LC-MS m/z: 565.3 [M+H]⁺, LC-MS retention time: 2.01 min.

### Example 52

Compound of Example 52 was prepared following a procedure similar to the preparation of a compound of Example 14 as off-white solid (7.73 mg, 23%). LC-MS m/z: 465.2 [M+H]⁺. LC-MS retention time: 7.132 min (Method C). ¹H NMR (400 MHz, DMSO-*d*₆) δ ppm: 8.65-8.74 (m, 1H), 8.23-8.35 (m, 1H), 7.96-8.06 (m, 1H), 7.00-7.20 (m, 2H), 6.84 (s, 1H), 4.80-5.04 (m, 1H), 4.37-4.56 (m, 4H), 2.84-3.03 (m, 4H), 2.57 (s, 3H), 2.04-2.23 (m, 1H), 1.40-1.64 (m, 1H), 1.12-1.24 (m, 2H), 0.14-0.42 (m, 4H).

### Example 53

### ((3R,5R)-3-Amino-5-fluoropiperidin-1-yl)(2-(6-chloro-1-(cyclopropylmethyl)-1H-pyrrolo[2,3-b]pyridin-2-yl)-4-fluoro-3-methylpyrazolo[1,5-a]pyridin-6-yl)methanone

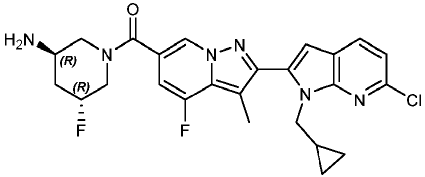

### Intermediate 53A

### tert-Butyl-((3R,5R)-1-(2-(6-chloro-1-(cyclopropylmethyl)-1H-pyrrolo[2,3-b]pyridin-2-yl)-4-fluoro-3-methylpyrazolo[1,5-a]pyridine-6-carbonyl)-5-fluoropiperidin-3-yl)carbamate

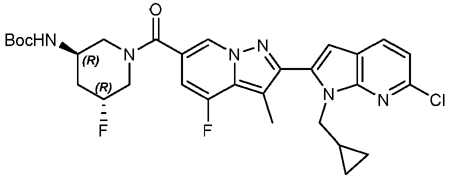

Intermediate 53A was prepared following a procedure similar to the preparation of Intermediate 15A, as off-white solid (130 mg, 87%). LC-MS m/z: 599.2 [M+H]⁺.

### Example 53

Compound of Example 53 was prepared following a procedure similar to the preparation of a compound of Example 15, as off-white solid (10.1 mg, 9%). LC-MS m/z: 499.2 [M+H]⁺. LC-MS retention time: 2.058 min (Method E). ¹H NMR (400 MHz, DMSO-*d*₆) δ ppm: 8.79 (br s, 1H), 8.19-7.95 (m, 1H), 7.24 (d, J=8.3 Hz, 1H), 7.20-7.09 (m, 1H), 6.95 (s, 1H), 5.11-4.90 (m, 1H), 4.44 (d,J=7.1 Hz, 2H), 3.46-3.41 (m, 1H), 3.08-2.77 (m, 2H), 2.53 (s, 3H), 2.41-2.34 (m, 1H), 1.96-1.75 (m, 1H), 1.27-1.11 (m, 1H), 0.38-0.19 (m, 4H).

### Example 54

### ((3R,5R)-3-Amino-5-fluoropiperidin-1-yl)(2-(1-(cyclopropylmethyl)-6-methyl-1H-pyrrolo[2,3-b]pyridin-2-yl)-4-fluoro-3-methylpyrazolo[1,5-a]pyridin-6-yl)methanone

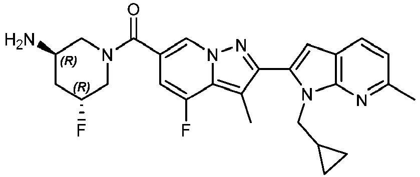

### Intermediate 54A

### tert-Butyl-((3R,5R)-1-(2-(1-(cyclopropylmethyl)-6-methyl-1H-pyrrolo[2,3-b]pyridin-2-yl)-4-fluoro-3-methylpyrazolo[1,5-a]pyridine-6-carbonyl)-5-fluoropiperidin-3-yl)carbamate

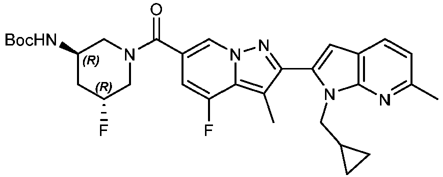

Intermediate 54A was prepared following a procedure similar to the preparation of Intermediate 16A, as off-white solid (70 mg, 91%). LC-MS m/z: 579.3 [M+H]⁺.

### Example 54

Compound of Example 54 was prepared following a procedure similar to the preparation of a compound of Example 16, as off-white solid (9.8 mg, 15%). LC-MS m/z: 479.3 [M+H]⁺. LC-MS retention time: 1.19 min (Method E). ¹H NMR (400 MHz, DMSO-*d*₆) δ ppm: 8.76-8.67 (m, 1H), 7.93 (d, J=7.8 Hz, 1H), 7.15-6.98 (m, 2H), 6.80 (s, 1H),4.94-4.79 (m, 1H); 4.49-4.39 (m, 3H), 4.10 (ddd, J=6.4, 5.0, 2.6 Hz, 1H), 3.04-2.94 (m, 3H), 2.58 (s, 3H), 2.19-2.06 (m, 1H), 1.64-1.45 (m, 1H), 1.27-1.11 (m, 3H), 0.33-0.18 (m, 4H).

Compounds of Examples 55 to 57 in Table 7 were prepared following a procedure similar to the preparation of a compound of Example 54, by Suzuki coupling of Intermediate 53A with appropriate boronic acid.

**Table 7**

| Example | Structure |
|---|---|
| | Name |
| | Analytical Data: LC-MS m/z [M+H]⁺; HPLC retention time (HPLC method) |
| 55 | 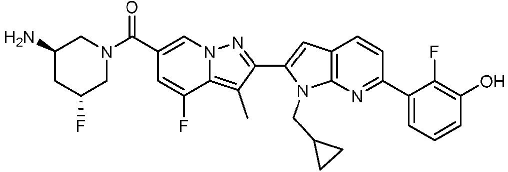 ((3R,5R)-3-Amino-5-fluoropiperidin-1-yl)(2-(1-(cyclopropylmethyl)-6-(2-fluoro-3-hydroxyphenyl)-1H-pyrrolo[2,3-b]pyridin-2-yl)-4-fluoro-3-methylpyrazolo[1,5-a]pyridin-6-yl)methanone |
| | Analytical Data: 575.2, 7.279 min (Method C) |
| 56 | 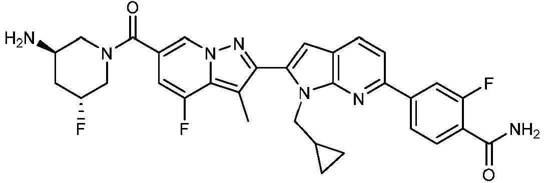 4-(2-(6-((3R,5R)-3-Amino-5-fluoropiperidine-1-carbonyl)-4-fluoro-3-methylpyrazolo[1,5-a]pyridin-2-yl)-1-(cyclopropylmethyl)-1H-pyrrolo[2,3-b]pyridin-6-yl)-2-fluorobenzamide |
| | Analytical Data: 602.3, 6.922 min (Method C) |
| 57 | 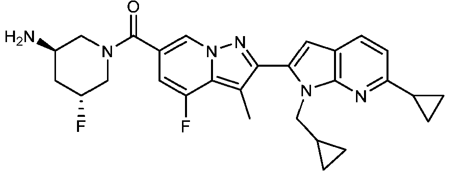 ((3R,5R)-3-Amino-5-fluoropiperidin-1-yl)(2-(6-cyclopropyl-1-(cyclopropylmethyl)-1H-pyrrolo[2,3-b]pyridin-2-yl)-4-fluoro-3-methylpyrazolo[1,5-a]pyridin-6-yl)methanone |
| | Analytical Data: 505.3, 2.265 min (Method E) |

### Example 58

### N-(7-(2-(6-((7R)-7-Amino-2-azabicyclo[2.2.1]heptane-2-carbonyl)-4-fluoro-3-methylpyrazolo [1,5-a]pyridin -2-yl)-1-(cyclopropylmethyl)-1H-indol-6-yl)-4-chloro-1-methyl-1H-indazol-3-yl)methanesulfonamide

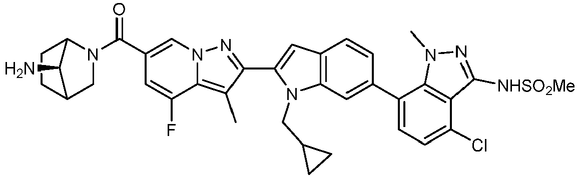

### Intermediate 58A

### 3-(tert-Butyl)-6-methyl 2-(6-bromo-1-(cyclopropylmethyl)-1H-indol-2-yl)-4-fluoropyrazolo[1,5-a]pyridine-3,6-dicarboxylate

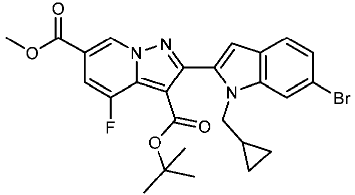

Intermediate 58A was prepared following a procedure similar to the preparation of Intermediate 23A, as a pale yellow solid (2.0 g, 27%). LC-MS m/z: 543.9 [M+H]⁺.

### Intermediate 58B

### 2-(6-Bromo-1-(cyclopropylmethyl)-1H-indol-2-yl)-4-fluoro-6-(methoxycarbonyl)pyrazolo[1,5-a]pyridine-3-carboxylic acid

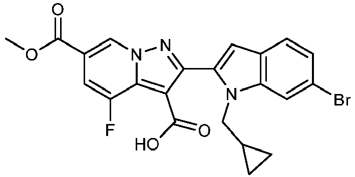

Intermediate 58B was prepared following a procedure similar to the preparation of Intermediate 23B, as a pale yellow solid (2 g, 3.07 mmol, 89%). LC-MS m/z: 488.0 [M+H]⁺.

### Intermediate 58C

### Methyl-2-(6-bromo-1-(cyclopropylmethyl)-1H-indol-2-yl)-4-fluoro-3-methylpyrazolo[1,5-a]pyridine-6-carboxylate

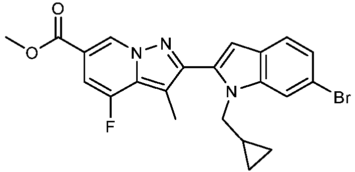

Intermediate 58C was prepared following a procedure similar to the preparation of 23C as a pale yellow solid (crude 2.0g, 75%). LC-MS m/z: 458.1 [M+H]⁺.

### Intermediate 58D

### 2-(6-Bromo-1-(cyclopropylmethyl)-1H-indol-2-yl)-4-fluoro-3-methylpyrazolo[1,5-a]pyridine-6-carboxylic acid

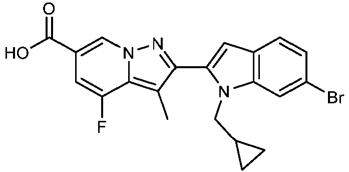

Intermediate 58D was prepared following a procedure similar to the preparation of Intermediate 23D, as a pale yellow solid (1.6 g, 83%). LC-MS m/z: 444.1 [M+H]⁺.

### Intermediate 58E

### tert-Butyl-((7R)-2-(2-(6-bromo-1-(cyclopropylmethyl)-1H-indol-2-yl)-4-fluoro-3-methylpyrazolo[1,5-a]pyridine-6-carbonyl)-2-azabicyclo[2.2.1]heptan-7-yl)carbamate

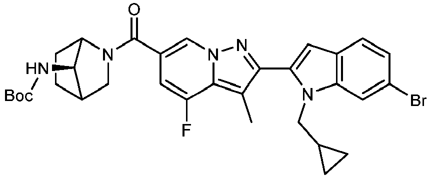

Intermediate 58E was prepared following a procedure similar to the preparation of Intermediate 23E, as a pale yellow solid (700 mg, 97%). LC-MS: m/z: 638.2 [M+H]⁺.

### Intermediate 58F

### tert-Butyl-((7R)-2-(2-(6-(4-chloro-1-methyl-3-(methylsulfonamido)-1H-indazol-7-yl)-1-(cyclopropylmethyl)-1H-indol-2-yl)-4-fluoro-3-methylpyrazolo[1,5-a]pyridine-6-carbonyl)-2-azabicyclo[2.2.1]heptan-7-yl)carbamate

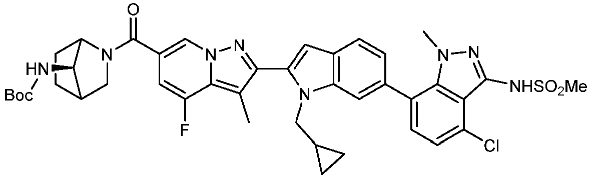

Intermediate 58F was prepared following a procedure similar to the preparation of Intermediate 23F as a pale yellow solid (70 mg, 95%). LC-MS m/z: 668.3 [M+H]⁺.

### Example 58

Compound of Example 58 was prepared following a procedure similar to the preparation of a compound of Example 23, as a pale yellow solid (5 mg, 0.007 mmol, 8%). LC-MS m/z: 715.3 [M+H]⁺. LC-MS retention time: 0.85 min (Method G).

Compounds of Examples 59 to 62 in Table 8 were prepared following a procedure similar to the preparation of a compound of Example 58, by Suzuki coupling of Intermediate 58E with appropriate boronic acid.

**Table 8**

| Example | Structure |
|---|---|
| | Name |
| | Analytical Data: LC-MS m/z [M+H]⁺; HPLC retention time (HPLC method) |
| 59 | 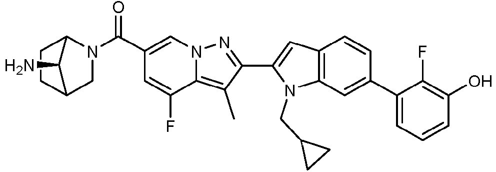 ((7R)-7-Amino-2-azabicyclo[2.2.1]heptan-2-yl)(2-(1-(cyclopropylmethyl)-6-(2-fluoro-3-hydroxyphenyl)-1H-indol-2-yl)-4-fluoro-3-methylpyrazolo[1,5-a]pyridin-6-yl)methanone |
| | Analytical Data: 568.3, 3.8 min (Method C) |
| 60 | 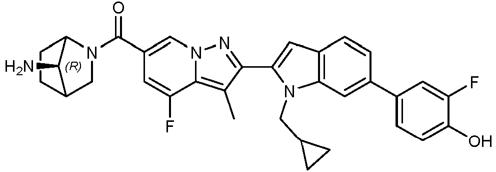 ((7R)-7-Amino-2-azabicyclo[2.2.1]heptan-2-yl)(2-(1-(cyclopropylmethyl)-6-(3-fluoro-4-hydroxyphenyl)-1H-indol-2-yl)-4-fluoro-3-methylpyrazolo[1,5-a]pyridin-6-yl)methanone |
| | Analytical Data: 568.3, 10.01 min (Method A) |
| 61 | 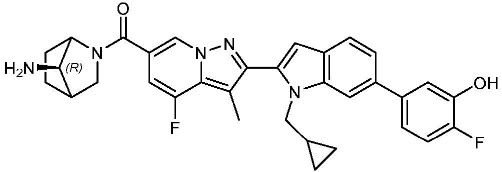 ((7R)-7-Amino-2-azabicyclo[2.2.1]heptan-2-yl)(2-(1-(cyclopropylmethyl)-6-(4-fluoro-3-hydroxyphenyl)-1H-indol-2-yl)-4-fluoro-3-methylpyrazolo[1,5-a]pyridin-6-yl)methanone |
| | Analytical Data: 568.3, 3.5 min (Method C) |
| 62 | 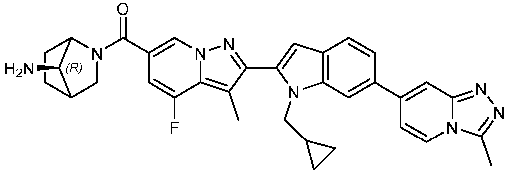 ((7R)-7-Amino-2-azabicyclo[2.2.1]heptan-2-yl)(2-(1-(cyclopropylmethyl)-6-(3-methyl-[1,2,4]triazolo[4,3-a]pyridin-7-yl)-1H-indol-2-yl)-4-fluoro-3-methylpyrazolo[1,5-a]pyridin-6-yl)methanone |
| | Analytical Data: 589.4, 1.16 min (Method C) |

### Example 63

### ((7R)-7-Amino-2-azabicyclo[2.2.1]heptan-2-yl)(2-(1-(cyclopropylmethyl)-1H-pyrrolo[2,3-b]pyridin-2-yl)-3-methylpyrazolo[1,5-a]pyridin-6-yl)methanone

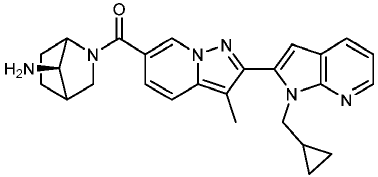

### Intermediate 63A

### 3-(tert-Butyl)-6-ethyl 2-(1-(cyclopropylmethyl)-1H-pyrrolo[2,3-b]pyridin-2-yl)pyrazolo[1,5-a]pyridine-3,6-dicarboxylate

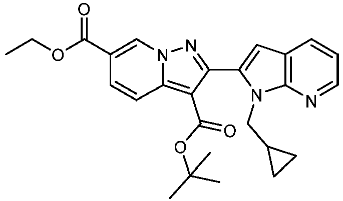

K₂CO₃ (839 mg, 6.07 mmol) and tert-butyl 3-(1-(cyclopropylmethyl)-1H-pyrrolo[2,3-b]pyridin-2-yl)propiolate (Intermediate 2, 600 mg, 2.024 mmol) were added to a stirred solution of 1-amino-3-(ethoxycarbonyl)pyridin-1-ium salt (Intermediate 8, 338 mg, 2.024 mmol) in acetone (25 ml). The reaction mixture was bubbled with air for 5 minutes, stirred overnight, filtered, and concentrated in vacuum to afford a crude compound, which was dissolved in EtOAc and washed with water. The organic layer was concentrated under vacuum to afford a crude compound, which was purified using a silica gel column to afford the title compound as a pale yellow solid (300 mg, 32%). LC-MS m/z: 461.2 [M+H]⁺. ¹H NMR (400 MHz, DMSO-*d*₆) δ ppm: 8.73 (s, 1H), 8.15 (d, J=8.0 Hz, 1H), 7.69-7.62 (m, 1H), 7.59-7.50 (m, 4H), 7.36 (t, J=6.8 Hz, 1H), 7.13 (br, t, J=7.3 Hz, 2H), 7.04-7.00 (m, 1H), 6.93 (s, 1H), 4.58-4.47 (m, 4H), 4.41 (d, J=1.5 Hz, 2H), 4.10 (s, 3H), 3.42 (s, 3H), 3.39-3.35 (m, 1H), 3.27 (br, s, 1H), 2.61-1.24 (s, 3H), 0.36-0.32 (m, 5H), 0.15 (s, 2H).

### Intermediate 63B

### 2-(1-(Cyclopropylmethyl)-1H-pyrrolo[2,3-b]pyridin-2-yl)-6-(ethoxycarbonyl)pyrazolo[1,5-a]pyridine-3-carboxylic acid

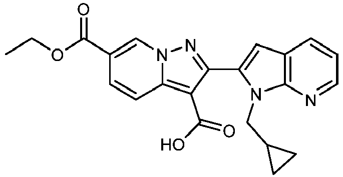

A solution of Intermediate 63A (300 mg, 0.651 mmol) in DCM (3 ml) and TFA (0.5 m) was stirred for 2 hours, and concentrated under reduced pressure at a room temperature to obtain a pale pink compound, which was dried over high vacuum to afford the title compound (200 mg, 76%). The crude compound was used in the next steps without further purification. LC-MS m/z: 405.2 [M+H]⁺.

### Intermediate 63C

### Ethyl-2-(1-(cyclopropylmethyl)-1H-pyrrolo[2,3-b]pyridin-2-yl)-3-methylpyrazolo[1,5-a]pyridine-6-carboxylate

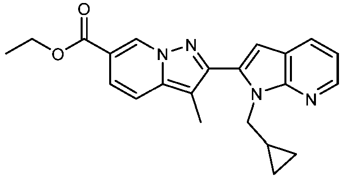

Intermediate 63C was prepared following a procedure similar to the preparation of Intermediate 1C, as a pale yellow solid (120 mg, 65%). LC-MS m/z: 375.1 [M+H]⁺. ¹H NMR (400 MHz, DMSO-*d*₆) δ ppm: 9.27-9.22 (m, 1H), 8.35-8.31 (m, 1H), 8.08-8.03 (m, 1H), 7.87-7.81 (m, 1H), 7.62 (d, J=9.3 Hz, 1H), 7.19-7.13 (m,1H), 6.94 (s, 1H), 4.59 (d, J=7.0 Hz, 2H), 4.40-4.34 (m, 2H), 2.48-2.45 (m, 3H), 1.37 (t, J=7.0 Hz, 3H), 1.29 -1.19 (m, 1H), 0.35-0.24 (m, 4H).

### Intermediate 63D

### 2-(1-(Cyclopropylmethyl)-1H-pyrrolo[2,3-b]pyridin-2-yl)-3-methylpyrazolo[1,5-a]pyridine-6-carboxylic acid

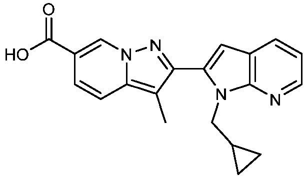

Intermediate 63D was prepared following a procedure similar to the preparation of Intermediate 1D, as off-white solid (110 mg, 99%). LC-MS m/z: 347 [M+H]⁺.

### Intermediate 63E

### tert-Butyl-((7R)-2-(2-(1-(cyclopropylmethyl)-1H-pyrrolo[2,3-b]pyridin-2-yl)-3-methylpyrazolo[1,5-a]pyridine-6-carbonyl)-2-azabicyclo[2.2.1]heptan-7-yl)carbamate

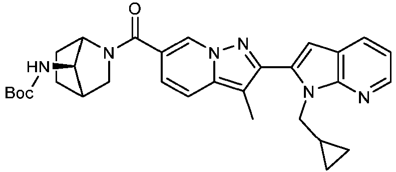

Intermediate 63C was prepared following a procedure similar to the preparation of Intermediate 1E (70 mg, 90%). LC-MS m/z: 541.3 [M+H]⁺.

### Example 63

Compound of Example 63 was prepared following a procedure similar to preparation of Example 1 (9.0 mg, 15%). LC-MS m/z: 441.3 [M+H]⁺. LC-MS retention time: 1.570 min (Method E). ¹H NMR (400 MHz, DMSO-*d*₆) δ ppm: 8.94-8.78 (m, 1H), 8.32 (d, J=4.6 Hz, 1H), 8.05 (dd, J=1.1, 7.7 Hz, 1H), 7.85-7.75 (m, 1H), 7.39-7.24 (m, 1H), 7.16 (dd, J=4.5, 7.7 Hz, 1H), 6.87 (s, 1H), 4.56 (br d, J=7.1 Hz, 2H), 3.93-3.90 (m, 1H), 3.77-3.68 (m, 1H), 3.65-3.46 (m, 2H), 3.32-3.06 (m, 2H), 2.63-2.52 (m, 1H), 2.48-2.36 (m, 3H), 2.02-1.84 (m, 2H), 1.80-1.65 (m, 1H), 1.54-1.37 (m, 1H), 1.30-1.13 (m, 1H), 0.31-0.19 (m, 4H).

### Example 64

### 5-(2-(6-((7R)-7-Amino-2-azabicyclo[2.2.1]heptane-2-carbonyl)-3-methylpyrazolo[1,5-alpyridin-2-yl)-1-(cyclopropylmethyl)-1H-pyrrolo[2,3-b]pyridin-6-yl)isoindolin-1-one

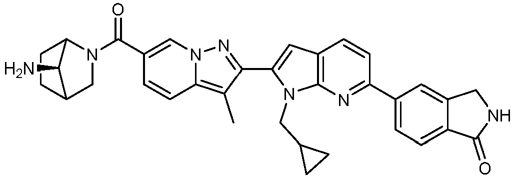

### Intermediate 64A

### 3-(tert-Butyl)-6-ethyl 2-(6-chloro-1-(cyclopropylmethyl)-1H-pyrrolo[2,3-b]pyridin-2-yl)pyrazolo[1,5-a]pyridine-3,6-dicarboxylate

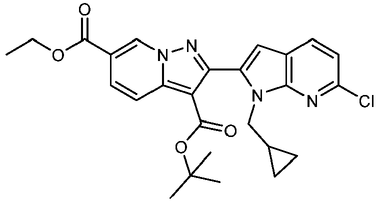

Intermediate 64A was prepared following a procedure similar to the preparation of Intermediate 2A (2 g, 30%). LC-MS m/z: 495.2 [M+H]⁺.

### Intermediate 64B

### 2-(6-Chloro-1-(cyclopropylmethyl)-1H-pyrrolo[2,3-b]pyridin-2-yl)-6-(ethoxycarbonyl)pyrazolo[1,5-a]pyridine-3-carboxylic acid

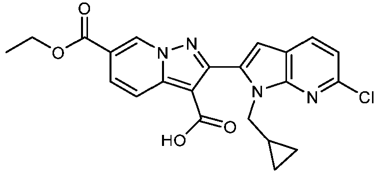

Intermediate 64B was prepared following a procedure similar to the preparation of Intermediate 2B (1.5 g, 85%). LC-MS m/z: 441.3 [M+H]⁺.

### Intermediate 64C

### Ethyl-2-(6-chloro-1-(cyclopropylmethyl)-1H-pyrrolo[2,3-b]pyridin-2-yl)-3-methylpyrazolo[1,5-a]pyridine-6-carboxylate

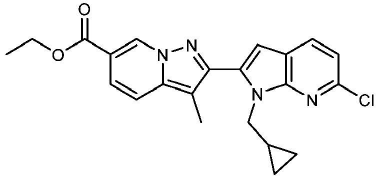

Intermediate 64C was prepared following a procedure similar to the preparation of Intermediate 2C (240 mg, 0.05 mmol, 86%). LC-MS m/z: 409.2 [M+H]⁺.

### Intermediate 64D

### 2-(6-Chloro-1-(cyclopropylmethyl)-1H-pyrrolo[2,3-b]pyridin-2-yl)-3-methylpyrazolo[1,5-a]pyridine-6-carboxylic acid

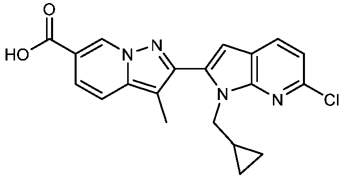

Intermediate 63D was prepared following a procedure similar to the preparation of Intermediate 2D (900 mg, 88%). LC-MS m/z: 381.1 [M+H]⁺.

### Intermediate 64E

### tert-Butyl-((7R)-2-(2-(6-chloro-1-(cyclopropylmethyl)-1H-pyrrolo[2,3-b]pyridin-2-yl)-3-methylpyrazolo[1,5-a]pyridine-6-carbonyl)-2-azabicyclo[2.2.1]heptan-7-yl)carbamate

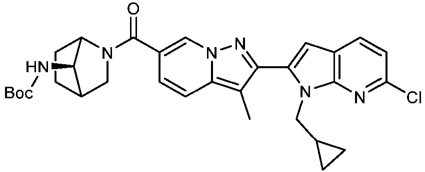

Intermediate 64E was prepared following a procedure similar to the preparation of Intermediate 2E (1.2 g, 88%). LC-MS m/z: 575.3 [M+H]⁺.

### Intermediate 64F

### tert-Butyl-((7R)-2-(2-(1-(cyclopropylmethyl)-6-(1-oxoisoindolin-5-yl)-1H-pyrrolo[2,3-b]pyridin-2-yl)-3-methylpyrazolo[1,5-a]pyridine-6-carbonyl)-2-azabicyclo[2.2.1]heptan-7-yl)carbamate

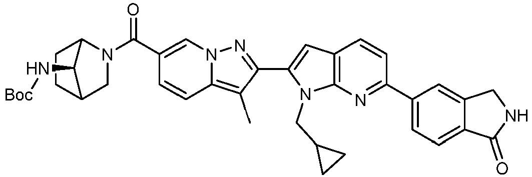

Intermediate 63F was prepared following a procedure similar to the preparation of Intermediate 3A (50 mg, 61%). LC-MS m/z: 672.4 [M+H]⁺.

### Example 64

Compound of Example 64 was prepared following a procedure similar to the preparation of compound of Example 3 (8.88 mg, 17%). LC-MS m/z: 672.4 [M+H]⁺. HPLC retention time: 5.956 min (Method C). ¹H NMR (400 MHz, DMSO-*d*₆) δ ppm: 9.01 -8.88 (m, 1H), 8.61 (s, 1H), 8.37 (s, 1H), 8.32 (d, J=7.8 Hz, 1H), 8.18 (d, J=8.0 Hz, 1H), 8.12-7.91 (m, 3H), 7.90-7.77 (m, 2H), 7.40-7.28 (m, 1H), 6.95 (s, 1H), 4.69 (br d, J=6.0 Hz, 2H), 4.50 (s, 2H), 4.25 (br s, 1H), 3.82 (br dd, J=3.0, 9.5 Hz, 1H), 3.71-3.51 (m, 2H), 3.48-3.36 (m, 1H), 3.19-3.07 (m, 1H), 2.70-2.53 (m, 1H), 2.46-2.43 (m, 3H), 2.06-1.80 (m, 3H), 1.77-1.60 (m, 1H), 1.34-1.15 (m, 1H), 0.38-0.30 (m, 4H).

Compounds of Examples 65 to 75 in Table 9 were prepared following a procedure similar to the preparation of a compound of Example 64, by Suzuki coupling of Intermediate 64E with appropriate boronic acid.

**Table 9**

| Example | Structure |
|---|---|
| | Name |
| | Analytical Data: LC-MS m/z [M+H]⁺; HPLC retention time (HPLC method) |
| 65 | 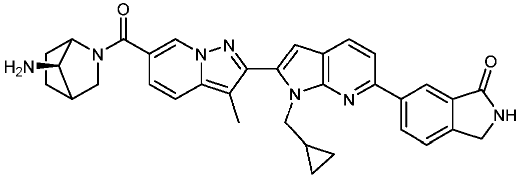 6-(2-(6-((7R)-7-Amino-2-azabicyclo[2.2.1]heptane-2-carbonyl)-3-methylpyrazolo[1,5-a]pyridin-2-yl)-1-(cyclopropylmethyl)-1H-pyrrolo[2,3-b]pyridin-6-yl)isoindolin-1-one |
| | Analytical Data: 572.3, 6.015 min (Method C) |
| 66 | 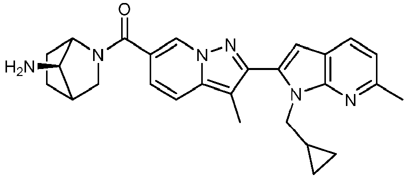 ((7R)-7-Amino-2-azabicyclo[2.2.1]heptan-2-yl)(2-(1-(cyclopropylmethyl)-6-methyl-1H-pyrrolo[2,3-b]pyridin-2-yl)-3-methylpyrazolo[1,5-a]pyridin-6-yl)methanone |
| | Analytical Data: 455.3, 5.696 min (Method C) |
| 67 | 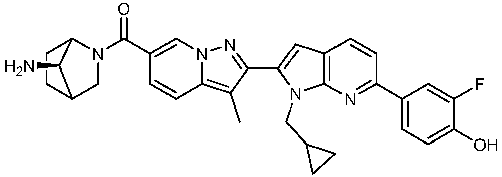 ((7R)-7-Amino-2-azabicyclo[2.2.1]heptan-2-yl)(2-(1-(cyclopropylmethyl)-6-(3-fluoro-4-hydroxyphenyl)-1H-pyrrolo[2,3-b]pyridin-2-yl)-3-methylpyrazolo[1,5-a]pyridin-6-yl)methanone |
| | Analytical Data: 551.3, 6.268 min (Method C) |
| 68 | 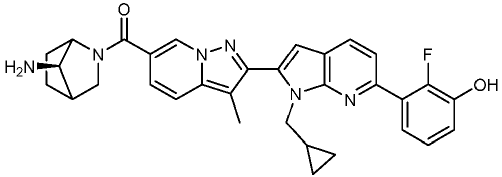 ((7R)-7-Amino-2-azabicyclo[2.2.1]heptan-2-yl)(2-(1-(cyclopropylmethyl)-6-(2-fluoro-3-hydroxyphenyl)-1H-pyrrolo[2,3-b]pyridin-2-yl)-3-methylpyrazolo[1,5-a]pyridin-6-yl)methanone |
| | Analytical Data: 551.4, 6.502 min (Method C) |
| 69 | 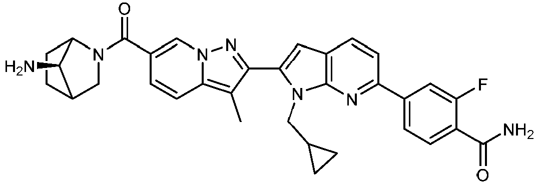 4-(2-(6-((7R)-7-Amino-2-azabicyclo[2.2.1]heptane-2-carbonyl)-3-methylpyrazolo[1,5-a]pyridin-2-yl)-1-(cyclopropylmethyl)-1H-pyrrolo[2,3-b]pyridin-6-yl)-2-fluorobenzamide |
| | Analytical Data: 578.4, 7.954 min (Method C) |
| 70 | 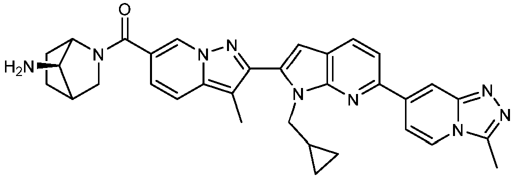 ((7R)-7-Amino-2-azabicyclo[2.2.1]heptan-2-yl)(2-(1-(cyclopropylmethyl)-6-(3-methyl-[1,2,4]triazolo[4,3-a]pyridin-7-yl)-1H-pyrrolo[2,3-b]pyridin-2-yl)-3-methylpyrazolo[1,5-a]pyridin-6-yl)methanone |
| | Analytical Data: 572.4, 5.613 min (Method C) |
| 71 | 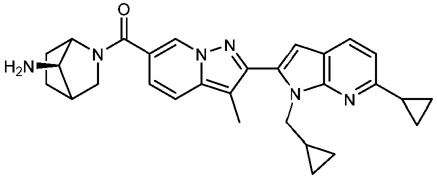 ((7R)-7-Amino-2-azabicyclo[2.2.1]heptan-2-yl)(2-(6-cyclopropyl-1-(cyclopropylmethyl)-1H-pyrrolo[2,3-b]pyridin-2-yl)-3-methylpyrazolo[1,5-a]pyridin-6-yl)methanone |
| | Analytical Data: 481.3, 1.588 min (Method F) |
| 72 | 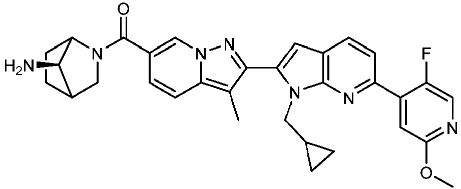 ((7R)-7-Amino-2-azabicyclo[2.2.1]heptan-2-yl)(2-(1-(cyclopropylmethyl)-6-(5-fluoro-2-methoxypyridin-4-yl)-1H-pyrrolo[2,3-b]pyridin-2-yl)-3-methylpyrazolo[1,5-a]pyridin-6-yl)methanone |
| | Analytical Data: 566.3, 1.893 min (Method F) |
| 73 | 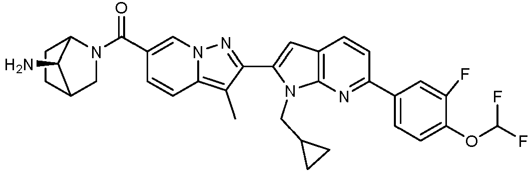 ((7R)-7-Amino-2-azabicyclo[2.2.1]heptan-2-yl)(2-(1-(cyclopropylmethyl)-6-(4-(difluoromethoxy)-3-fluorophenyl)-1H-pyrrolo[2,3-b]pyridin-2-yl)-3-methylpyrazolo[1,5-a]pyridin-6-yl)methanone |
| | Analytical Data: 601.3, 1.987 min (Method F) |
| 74 | 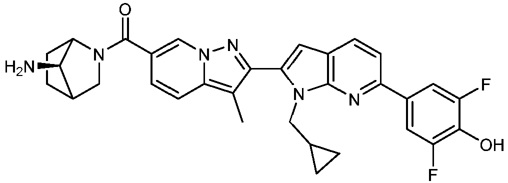 ((7R)-7-Amino-2-azabicyclo[2.2.1]heptan-2-yl)(2-(1-(cyclopropylmethyl)-6-(3,5-difluoro-4-hydroxyphenyl)-1H-pyrrolo[2,3-b]pyridin-2-yl)-3-methylpyrazolo[1,5-a]pyridin-6-yl)methanone |
| | Analytical Data: 569.3, 1.648 min (Method F) |
| 75 | 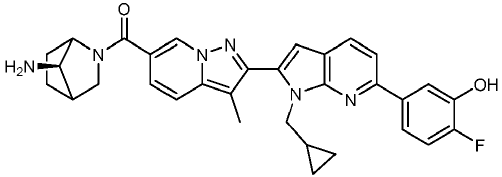 ((7R)-7-Amino-2-azabicyclo[2.2.1]heptan-2-yl)(2-(1-(cyclopropylmethyl)-6-(4-fluoro-3-hydroxyphenyl)-1H-pyrrolo[2,3-b]pyridin-2-yl)-3-methylpyrazolo[1,5-a]pyridin-6-yl)methanone |
| | Analytical Data: 551.3, 1.639 min (Method F) |

### Example 76

### ((7R)-7-Amino-2-azabicyclo[2.2.1]heptan-2-yl)(2-(1-(cyclopropylmethyl)-6-(4-methoxypiperidin-1-yl)-1H-pyrrolo[2,3-b]pyridin-2-yl)-3-methylpyrazolo[1,5-a]pyridin-6-yl)methanone

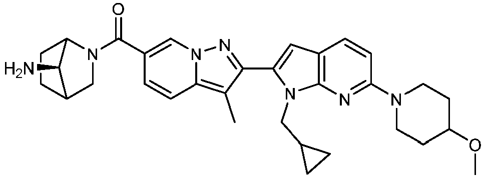

### Intermediate 76A

### tert-Butyl-((7R)-2-(2-(1-(cyclopropylmethyl)-6-(4-methoxypiperidin-1-yl)-1H-pyrrolo[2,3-b]pyridin-2-yl)-3-methylpyrazolo[1,5-a]pyridine-6-carbonyl)-2-azabicyclo[2.2.1]heptan-7-yl)carbamate

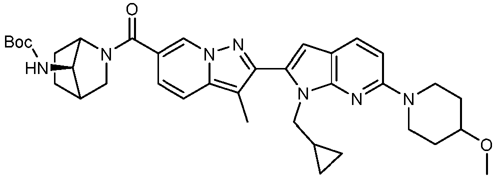

Intermediate 76A was prepared following a procedure similar to the preparation of Intermediate 13A (50 mg, 63%). LC-MS m/z: 654.5 [M+H]⁺.

### Example 76

Compound of Example 76 was prepared following a procedure similar to the preparation of compound of Example 13 (9.1 mg, 16%). LC-MS m/z: 554.4 [M+H]⁺. LC-MS retention time: 1.994 min (Method E). ¹H NMR (400 MHz, DMSO-*d*₆) δ ppm: 8.99-8.71 (m, 1H), 8.11-7.93 (m, 2H), 7.87-7.67 (m, 1H), 7.56-7.20 (m, 2H), 6.82-6.72 (m, 1H), 6.66 (s, 1H), 4.48-4.41 (m, 2H), 4.27-4.20 (m, 1H), 4.09-3.98 (m, 3H), 3.65-3.54 (m, 2H), 3.49-3.41 (m, 2H), 3.26-3.22 (m, 3H), 3.24-3.16 (m, 1H), 2.72-2.59 (m, 2H),2.42 (s, 3H), 2.02-1.84 (m, 5H), 1.76-1.61 (m, 1H), 1.54-1.41 (m, 2 H), 1.27-1.24 (m, 1H), 0.39-0.22 (m, 4H).

Compounds of Examples 77 to 79 in Table 10 were prepared following a procedure similar to the preparation of a compound of Example 76, by Buchwald coupling of Intermediate 64E with appropriate amines.

**Table 10**

| Example | Structure |
|---|---|
| | Name |
| | Analytical Data: LC-MS m/z [M+H]⁺; HPLC retention time (HPLC method) |
| 77 | 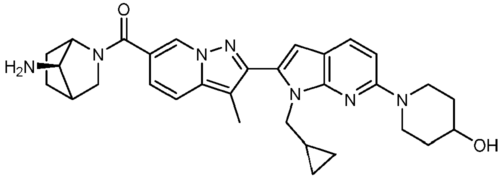 ((7R)-7-Amino-2-azabicyclo[2.2.1]heptan-2-yl)(2-(1-(cyclopropylmethyl)-6-(4-hydroxypiperidin-1-yl)-1H-pyrrolo[2,3-b]pyridin-2-yl)-3-methylpyrazolo[1,5-a]pyridin-6-yl)methanone |
| | Analytical Data: 540.4, 1.078 min (Method F) |
| 78 | 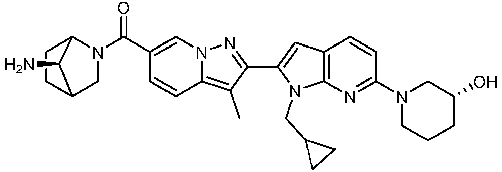 ((7R)-7-Amino-2-azabicyclo[2.2.1]heptan-2-yl)(2-(1-(cyclopropylmethyl)-6-((R)-3-hydroxypiperidin-1-yl)-1H-pyrrolo[2,3-b]pyridin-2-yl)-3-methylpyrazolo[1, 5-a]pyridin-6-yl)methanone |
| | Analytical Data: 540.4, 1.235 min (Method F) |
| 79 | 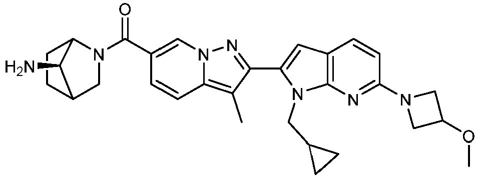 ((7R)-7-Amino-2-azabicyclo[2.2.1]heptan-2-yl)(2-(1-(cyclopropylmethyl)-6-(3-methoxyazetidin-1-yl)-1H-pyrrolo[2,3-b]pyridin-2-yl)-3-methylpyrazolo[1, 5-a]pyridin-6-yl)methanone |
| | Analytical Data: 526.4, 1.393 min (Method F) |

### Example 80

### ((3R,5R)-3-Amino-5-fluoropiperidin-1-yl)(2-(1-(cyclopropylmethyl)-1H-pyrrolo[2,3-b]pyridin-2-yl)-3-methylpyrazolo[1,5-a]pyridin-6-yl)methanone

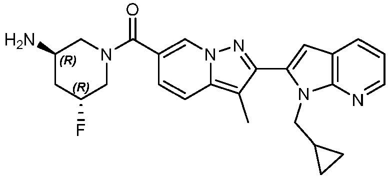

### Intermediate 80A

### tert-Butyl-((3R,5R)-1-(2-(1-(cyclopropylmethyl)-1H-pyrrolo[2,3-b]pyridin-2-yl)-3-methylpyrazolo[1,5-a]pyridine-6-carbonyl)-5-fluoropiperidin-3-yl)carbamate

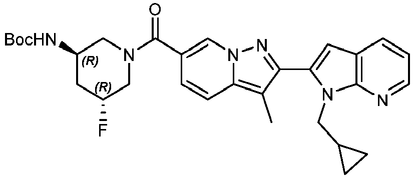

Intermediate 80A was prepared following a procedure similar to the preparation of Intermediate 14A (50 mg, 63%). LC-MS m/z: 547.3 [M+H]⁺.

### Example 80

Compound of Example 80 was prepared following a procedure similar to the preparation of Example 14 (21.2 mg, 43%). LC-MS m/z: 447.3 [M+H]⁺. ¹H NMR (400 MHz, DMSO-*d*₆) δ ppm: 8.79 (s, 1H), 8.33 (dd, J=1.6, 4.8 Hz, 1H), 8.05 (dd, J=1.5, 7.8 Hz, 1H), 7.82 (d, J=9.3 Hz, 1H), 7.23 (dd, J=1.2, 9.3Hz, 1H), 7.16 (dd, J=4.6, 7.8 Hz, 1H), 6.87 (s, 1H), 5.04-4.80 (m, 1H), 4.55 (d, J=7.1 Hz, 3H), 3.91 (s, 2H), 3.03-2.95 (m, 1H), 2.45 (s, 3H), 2.22-2.09 (m, 1H), 1.65-1.41 (m, 1H), 1.29-1.15 (m, 1H), 0.34-0.21 (m, 4H).

### Example 81

### 5-(2-(6-((3R,5R)-3-Amino-5-fluoropiperidine-1-carbonyl)-3-methylpyrazolo[1,5-a]pyridin-2-yl)-1-(cyclopropylmethyl)-1H-pyrrolo[2,3-b]pyridin-6-yl)isoindolin-1-one

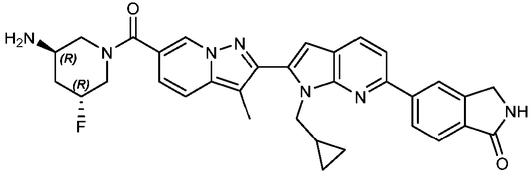

### Intermediate 81A

### tert-Butyl-((3R,5R)-1-(2-(6-chloro-1-(cyclopropylmethyl)-1H-pyrrolo[2,3-b]pyridin-2-yl)-3-methylpyrazolo[1,5-a]pyridine-6-carbonyl)-5-fluoropiperidin-3-yl)carbamate

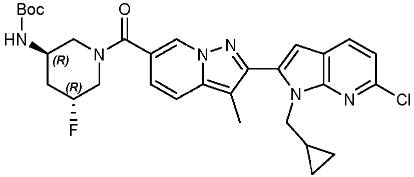

Intermediate 81A was prepared following a procedure similar to the preparation of Intermediate 15A (600 mg, 79%). LC-MS m/z: 581.3 [M+H]⁺.

### Intermediate 81B

### tert-Butyl-((3R,5R)-1-(2-(1-(cyclopropylmethyl)-6-(1-oxoisoindolin-5-yl)-1H-pyrrolo[2,3-b]pyridin-2-yl)-3-methylpyrazolo[1,5-a]pyridine-6-carbonyl)-5-fluoropiperidin-3-yl)carbamate

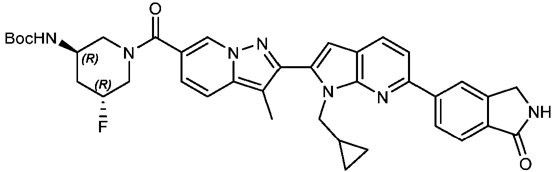

Intermediate 81B was prepared following a procedure similar to the preparation of Intermediate 16A (50 mg, 61%). LC-MS m/z: 678.4 [M+H]⁺.

### Example 81

Compound of Example 81 was prepared following a procedure similar to the preparation of Example 16 (7.1 mg). LC-MS m/z: 578.4 [M+H]⁺. LC-MS retention time: 1.576 min (Method E). ¹H NMR (400 MHz, DMSO-*d*₆) δ ppm: 8.86-8.77 (m, 1H), 8.66-8.56 (m, 1H), 8.42-8.28 (m, 2H), 8.17 (d, J=8.1 Hz, 1H), 7.88-7.76 (m, 3H), 7.24 (d, J=8.4 Hz, 1H), 6.93 (s, 1H), 5.08-4.82 (m, 1H), 4.73-4.59 (m, 2H), 4.56-4.38 (m, 2H), 4.22-4.12 (m, 1H), 4.12-3.85 (m, 1H), 3.09-2.95 (m, 2H), 2.48-2.38 (m, 3H), 2.24-2.11 (m, 1H), 1.98-1.82 (m, 1H), 1.68-1.42 (m, 1H), 1.39-1.14 (m, 1H), 0.37-0.30 (m, 4H).

Compounds of Examples 82 to 86 in Table 11 were prepared following a procedure similar to the preparation of a compound of Example 81, by Suzuki coupling of Intermediate 81A with appropriate boronic acid.

**Table 11**

| Example | Structure |
|---|---|
| | Name |
| | Analytical Data: LC-MS m/z [M+H]⁺; HPLC retention time (HPLC method) |
| 82 | 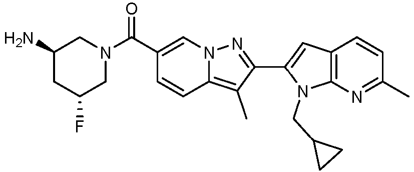 ((3R,5R)-3-Amino-5-fluoropiperidin-1-yl)(2-(1-(cyclopropylmethyl)-6-methyl-1H-pyrrolo[2,3-b]pyridin-2-yl)-3-methylpyrazolo[1,5-a]pyridin-6-yl)methanone |
| | Analytical Data: 461.4, 1.129 min (Method F) |
| 83 | 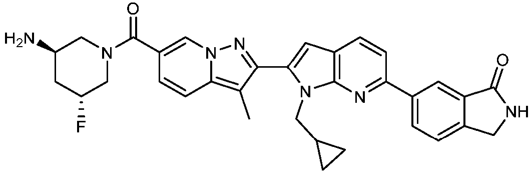 6-(2-(6-((3R,5R)-3-Amino-5-fluoropiperidine-1-carbonyl)-3-methylpyrazolo[1,5-a]pyridin-2-yl)-1-(cyclopropylmethyl)-1H-pyrrolo[2,3-b]pyridin-6-yl)isoindolin-1-one |
| | Analytical Data: 578.3, 1.440 min (Method F) |
| 84 | 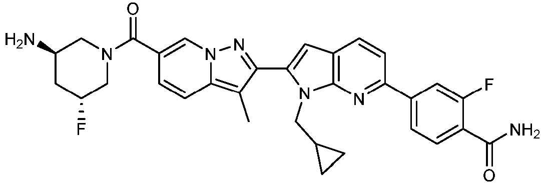 4-(2-(6-((3R,5R)-3-Amino-5-fluoropiperidine-1-carbonyl)-3-methylpyrazolo[1,5-a]pyridin-2-yl)-1-(cyclopropylmethyl)-1H-pyrrolo[2,3-b]pyridin-6-yl)-2-fluorobenzamide |
| | Analytical Data: 584.3, 1.623 min (Method F) |
| 85 | 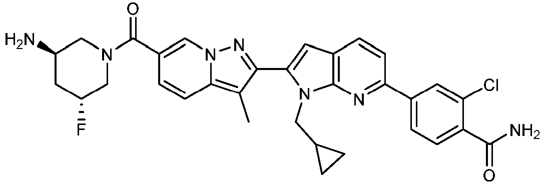 4-(2-(6-((3R,5R)-3-Amino-5-fluoropiperidine-1-carbonyl)-3-methylpyrazolo[1,5-a]pyridin-2-yl)-1-(cyclopropylmethyl)-1H-pyrrolo[2,3-b]pyridin-6-yl)-2-chlorobenzamide |
| | Analytical Data: 600.3, 1.454 min (Method F) |
| 86 | 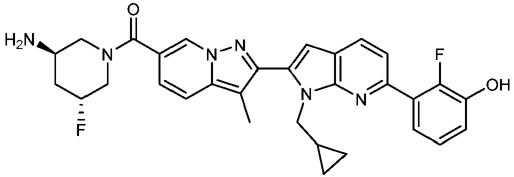 ((3R,5R)-3-Amino-5-fluoropiperidin-1-yl)(2-(1-(cyclopropylmethyl)-6-(2-fluoro-3-hydroxyphenyl)-1H-pyrrolo[2,3-b]pyridin-2-yl)-3-methylpyrazolo[1,5-a]pyridin-6-yl)methanone |
| | Analytical Data: 557.4, 1.603 min (Method F) |

### Example 87

### ((3R,5R)-3-Amino-5-fluoropiperidin-1-yl)(2-(1-(cyclopropylmethyl)-6-(4-methoxypiperidin-1-yl)-1H-pyrrolo[2,3-b]pyridin-2-yl)-3-methylpyrazolo[1,5-a]pyridin-6-yl)methanone

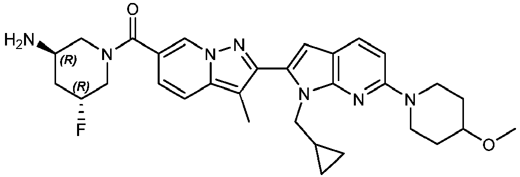

### Intermediate 87A

### tert-Butyl-((3R,5R)-1-(2-(1-(cyclopropylmethyl)-6-(4-methoxypiperidin-1-yl)-1H-pyrrolo[2,3-b]pyridin-2-yl)-3-methylpyrazolo[1,5-a]pyridine-6-carbonyl)-5-fluoropiperidin-3-yl)carbamate

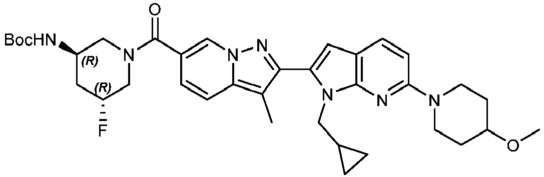

Intermediate 87A was prepared following a procedure similar to the preparation of Intermediate 13A (60 mg, 75%). LC-MS m/z: 660.5 [M+H]⁺.

### Example 87

Compound of Example 87 was prepared following a procedure similar to the preparation of Example 13 (5 mg, 9%). LC-MS m/z: 560.4 [M+H]⁺. ¹H NMR (400 MHz, DMSO-*d*₆) δ ppm: 8.72 (s, 1H), 7.79-7.69 (m, 2H), 7.22-7.14 (m, 1H), 6.75 (d, J=8.6 Hz, 1H), 6.65 (s, 1H), 5.10-4.80 (m, 1H), 4.46-4.38 (m, 3H), 4.06-3.97 (m, 4H), 3.28-3.30 (m, 5H), 3.22-3.18 (m, 2H), 2.98-2.93 (m, 1H), 2.41 (s, 3H), 1.99-1.89 (m, 3H), 1.51-1.42 (m, 3H), 1.32 -1.09 (m, 1H), 0.96-0.84 (m, 1H), 0.31 (d, J=6.6 Hz, 4H).

### Example 88

### ((3R,5R)-3-Amino-5-fluoropiperidin-1-yl)(2-(1-(cyclopropylmethyl)-6-(4-hydroxypiperidin-1-yl)-1H-pyrrolo[2,3-b]pyridin-2-yl)-3-methylpyrazolo[1,5-a]pyridin-6-yl)methanone

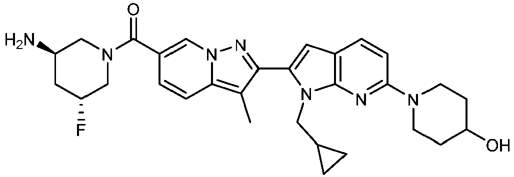

Compound of Example 88 was prepared following a procedure similar to the preparation of Example 87, by Buchwald coupling of Intermediate 81A with appropriate amine. LC-MS m/z: 546.3 [M+H]⁺. LC-MS retention time: 1.573 min (Method F).

### Example 89

### 6-(2-(6-((7R)-7-Amino-2-azabicyclo[2.2.1]heptane-2-carbonyl)-3-methylpyrazolo[1,5-a]pyridin-2-yl)-1-(cyclopropylmethyl)-1H-indol-6-yl)isoindolin-1-one

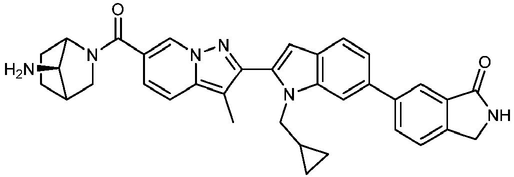

### Intermediate 89A

### 3-(tert-Butyl)-6-ethyl 2-(6-bromo-1-(cyclopropylmethyl)-1H-indol-2-yl)pyrazolo[1,5-a]pyridine-3,6-dicarboxylate

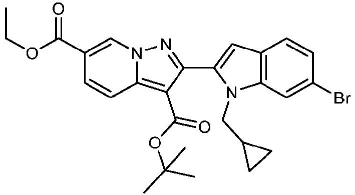

Intermediate 89A was prepared following a procedure similar to the preparation of Intermediate 23A, as a pale brown glassy solid (300 mg, 26%). LC-MS m/z: 538.2 [M+H]⁺.

### Intermediate 89B

### 2-(6-Bromo-1-(cyclopropylmethyl)-1H-indol-2-yl)-6-(ethoxycarbonyl)pyrazolo[1,5-a]pyridine-3-carboxylic acid

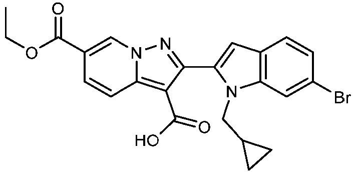

Intermediate 89B was prepared following a procedure similar to the preparation of Intermediate 23B, as pale yellow syrup (300 mg, 99%). LC-MS m/z: 484.0 [M+2H]⁺.

### Intermediate 89C

### Ethyl-2-(6-bromo-1-(cyclopropylmethyl)-1H-indol-2-yl)-3-methylpyrazolo[1,5-a]pyridine-6-carboxylate

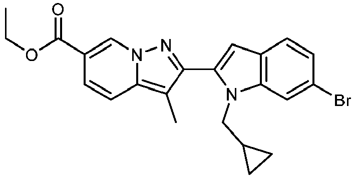

Intermediate 89C was prepared following a procedure similar to the preparation of Intermediate 23C (250 mg, 89%). LC-MS m/z: 454.1 [M+2H]⁺.

### Intermediate 89D

### 2-(6-Bromo-1-(cyclopropylmethyl)-1H-indol-2-yl)-3-methylpyrazolo[1,5-a]pyridine-6-carboxylic acid

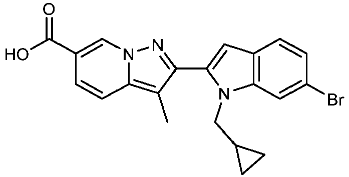

Intermediate 89D was prepared following a procedure similar to the preparation of Intermediate 23D, as brown syrup (230 mg, 98%). LC-MS m/z: 426.3 [M+2H]⁺.

### Intermediate 89E

### tert-Butyl-((7R)-2-(2-(6-bromo-1-(cyclopropylmethyl)-1H-indol-2-yl)-3-methylpyrazolo[1,5-a]pyridine-6-carbonyl)-2-azabicyclo[2.2.1]heptan-7-yl)carbamate

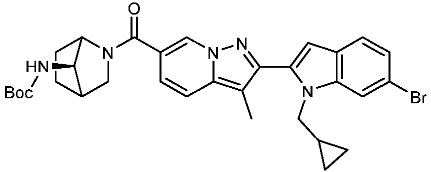

Intermediate 89E was prepared following a procedure similar to the preparation of Intermediate 23E, as a glassy solid (180 mg, 54%). LC-MS m/z: 620.3 [M+2H]⁺.

### Intermediate 89F

### tert-Butyl-((7R)-2-(2-(1-(cyclopropylmethyl)-6-(3-oxoisoindolin-5-yl)-1H-indol-2-yl)-3-methylpyrazolo[1,5-a]pyridine-6-carbonyl)-2-azabicyclo[2.2.1]heptan-7-yl)carbamate

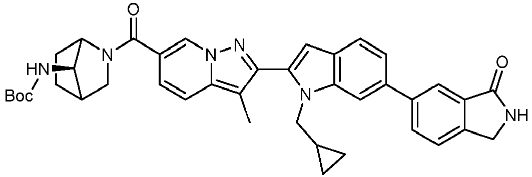

Intermediate 89F was prepared following a procedure similar to the preparation of Intermediate 23F, as a white solid (40 mg, 53%). LC-MS m/z: 671.5 [M+H]⁺.

### Example 89

Compound of Example 89 was prepared following a procedure similar to the preparation of the compound of Example 23, as a white solid (4 mg, 11%). LC-MS m/z: 571.3 [M+H]⁺. ¹H NMR (400 MHz, methanol-*d*₄) δ ppm: 8.82 (s, 1H), 8.75 (s, 1H), 8.14 (s, 1H), 8.02 (dd, J=1.8, 7.8 Hz, 1H), 7.83 (s, 1H), 7.75-7.64 (m, 3H),7.57-7.42 (m, 1H), 7.40-7.22 (m, 1H), 6.82 (s, 1H), 5.07-4.88 (m, 1H), 4.83-4.80 (m, 1H), 4.59 (s, 1H), 4.56-4.50 (m, 4H), 4.35 (s, 1H), 4.02(s, 1H), 3.85-3.58 (m, 1H), 3.57-3.39 (m, 1H), 3.34 (br, d, J=6.0 Hz, 1H), 3.29-3.22 (m, 1H), 2.50-2.43 (m, 3H), 2.40 (br, s, 1H), 2.34 (br, s, 1H), 2.13-1.89 (m, 3H), 1.71-1.48 (m, 1H), 1.42-1.24 (m, 1H), 1.24-1.11 (m, 1H), 0.41-0.30 (m, 2H), 0.18-0.08 (m, 2H).

### Example 90

### 5-(2-(6-((7R)-7-Amino-2-azabicyclo[2.2.1]heptane-2-carbonyl)-3-methylpyrazolo[1,5-a]pyridin-2-yl)-1-(cyclopropylmethyl)-1H-indol-6-yl)isoindolin-1-one

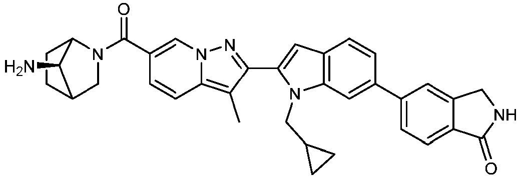

Compound of Example 90 was prepared following a procedure similar to the preparation of the compound of Example 89, by Suzuki coupling of Intermediate 89E with appropriate boronic acid. LC-MS m/z: 571.3 [M+H]⁺, LC-MS retention time: 7.662 min (Method C).

### Example 91

### ((7R)-7-Amino-2-azabicyclo[2.2.1]heptan-2-yl)(2-(1-(cyclopropylmethyl)-6-(3-fluoro-3-(hydroxymethyl)azetidin-1-yl)-1H-indol-2-yl)-3-methylpyrazolo[1,5-a]pyridin-6-yl)methanone

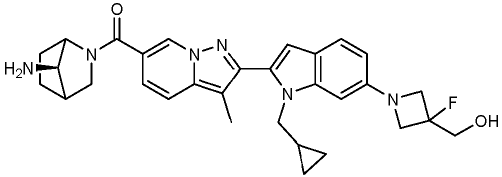

### Intermediate 91A

### tert-Butyl-((7R)-2-(2-(1-(cyclopropylmethyl)-6-(3-fluoro-3-(hydroxymethyl)azetidin-1-yl)-1H-indol-2-yl)-3-methylpyrazolo[1,5-a]pyridine-6-carbonyl)-2-azabicyclo[2.2.1]heptan-7-yl)carbamate

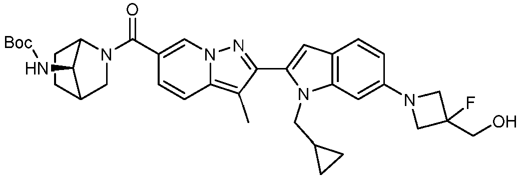

Intermediate 91A was prepared following a procedure similar to the preparation of Intermediate 33A (45 mg, 62%). LC-MS m/z: 643.5 [M+H]⁺.

### Example 91

Compound of Example 91 was prepared following a procedure similar to the preparation of the compound of 33 (9 mg, 7%). LC-MS m/z: 543.4 [M+H]⁺. LC-MS retention time: 1.285 min (Method E). ¹H NMR (400 MHz, DMSO-*d*₆) δ ppm: 8.74 (br, d, J=2.0 Hz, 1H), 7.77-7.66 (m, 1H), 7.45 (d, J=8.6 Hz, 1H), 7.34-7.14 (m, 2H), 6.68 (s, 1H), 6.59 (s,1H), 6.45-6.35 (m, 1H), 5.37-5.27 (m, 1H), 4.48-4.36 (m, 2H), 4.11-3.98 (m, 3H), 3.90-3.73 (m, 5H), 2.39 (s, 3H), 2.03-1.80 (m, 4H), 1.58 -1.45 (m, 2H), 1.38-1.07 (m, 3H), 0.34-0.23 (m, 2H), 0.20-0.05 (m, 2H).

### Example 92

### ((3R,5R)-3-Amino-5-fluoropiperidin-1-yl)(2-(1-(cyclopropylmethyl)-6-(2-fluoro-3-hydroxyphenyl)-1H-indol-2-yl)-3-methylpyrazolo[1,5-a]pyridin-6-yl)methanone

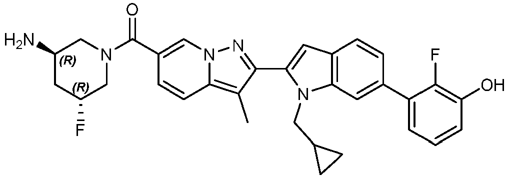

### Intermediate 92A

### tert-Butyl-((3R,5R)-1-(2-(6-bromo-1-(cyclopropylmethyl)-1H-indol-2-yl)-3-methylpyrazolo[1,5-a]pyridine-6-carbonyl)-5-fluoropiperidin-3-yl)carbamate

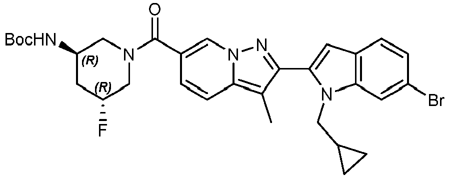

Intermediate 92A was prepared following a procedure similar to the preparation of Intermediate 29A (300 mg, 41%). LC-MS m/z: 657.3 [M+H]⁺.

### Intermediate 92B

### ((3R,5R)-3-Amino-5-fluoropiperidin-1-yl)(2-(1-(cyclopropylmethyl)-6-(2-fluoro-3-hydroxyphenyl)-1H-indol-2-yl)-3-methylpyrazolo[1,5-a]pyridin-6-yl)methanone

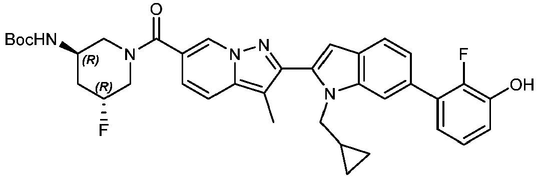

Intermediate 92B was prepared following a procedure similar to the preparation of Intermediate 29B (50 mg, 68%). LC-MS m/z: 557.4 [M+H]⁺.

### Example 92

Compound of Example 92 was prepared following a procedure similar to the preparation of the compound of Example 29 (16.3 mg). LC-MS m/z: 556.3 [M+H]⁺. LC-MS retention time: 1.843 min (method E). ¹H NMR (400 MHz, DMSO-*d*₆) δ ppm: 9.93-9.83 (m, 1H), 8.82 (s, 1H), 8.11-7.89 (m, 1H), 7.83 (d, J=9.3 Hz, 1H), 7.76-7.65 (m, 2H), 7.30-7.18 (m,2H), 7.11-7.04 (m, 1H), 7.01-6.92 (m, 2H), 6.87 (s, 1H), 5.11-4.93 (m, 1H), 4.54-4.40 (m, 3H), 3.28-3.20 (m, 2H), 2.43 (s, 3H), 2.42- 2.35 (m, 2H), 1.97-1.75 (m, 1H), 1.19 -1.07 (m, 1H), 0.36-0.27 (m, 2H), 0.25-0.13 (m, 2H).

Compounds of Examples 93 to 96 in Table 12 were prepared following a procedure similar to the preparation of a compound of Example 92, by Suzuki coupling of Intermediate 92A with appropriate boronic acid.

**Table 12**

| Example | Structure |
|---|---|
| | Name |
| | Analytical Data: LC-MS m/z [M+H]⁺; HPLC retention time (HPLC method) |
| 93 | 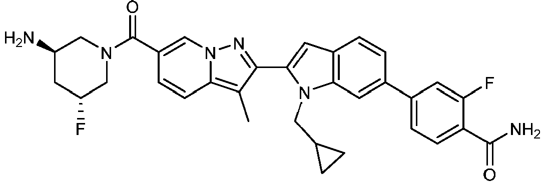 4-(2-(6-((3R,5R)-3-Amino-5-fluoropiperidine-1-carbonyl)-3-methylpyrazolo[1,5-a]pyridin-2-yl)-1-(cyclopropylmethyl)-1H-indol-6-yl)-2-fluorobenzamide |
| | Analytical Data: 583.3, 1.530 min (Method F) |
| 94 | 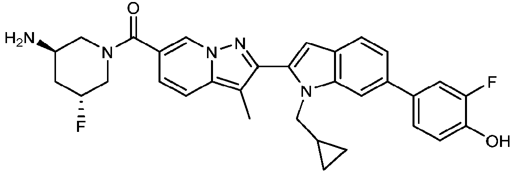 ((3R,5R)-3-Amino-5-fluoropiperidin-1-yl)(2-(1-(cyclopropylmethyl)-6-(3-fluoro-4-hydroxyphenyl)-1H-indol-2-yl)-3-methylpyrazolo[1,5-a]pyridin-6-yl)methanone |
| | Analytical Data: 556.3, 1.668 min (Method F) |
| 95 | 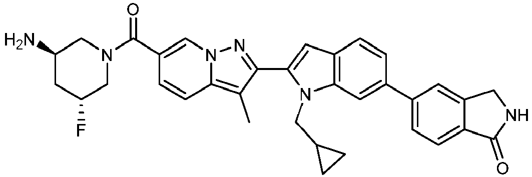 5-(2-(6-((3R,5R)-3-Amino-5-fluoropiperidine-1-carbonyl)-3-methylpyrazolo[1,5-a]pyridin-2-yl)-1-(cyclopropylmethyl)-1H-indol-6-yl)isoindolin-1-one |
| | Analytical Data: 577.4, 1.460 min (Method F) |
| 96 | 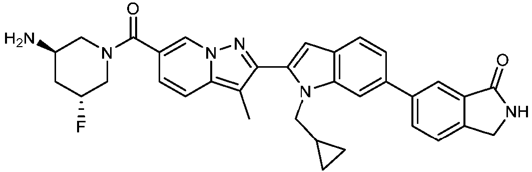 6-(2-(6-((3R,5R)-3-Amino-5-fluoropiperidine-1-carbonyl)-3-methylpyrazolo[1,5-a]pyridin-2-yl)-1-(cyclopropylmethyl)-1H-indol-6-yl)isoindolin-1-one |
| | Analytical Data: 577.3, 7.422 min (Method F) |

### Example 97

### ((7R)-7-Amino-2-azabicyclo[2.2.1]heptan-2-yl)(2-(1-(cyclopropylmethyl)-6-(2-hydroxypropan-2-yl)-1H-indol-2-yl)-4-methoxy-3-methylpyrazolo[1,5-a]pyridin-6-yl)methanone

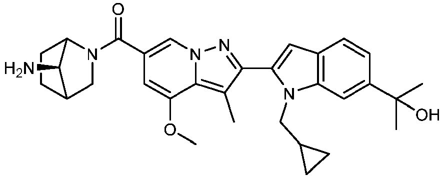

### Intermediate 97A

### ((7R)-7-Amino-2-azabicyclo[2.2.1]heptan-2-yl)(2-(6-bromo-1-(cyclopropylmethyl)-1H-indol-2-yl)-4-methoxy-3-methylpyrazolo[1,5-a]pyridin-6-yl)methanone

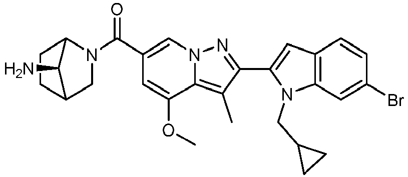

TFA (0.3ml, 3.89 mmol) was added to a solution of Intermediate 23E (200 mg, 0.308 mmol) in DCM (1 ml). The reaction mixture was stirred for 2 hours, and concentrated under vacuum to afford the crude title compound (180 mg, 100%), which was used in next step without further purification. LC-MS m/z: 548 [M+H]⁺.

### Intermediate 97B

### (9H-Fluoren-9-yl)methyl-((7R)-2-(2-(6-bromo-1-(cyclopropylmethyl)-1H-indol-2-yl)-4-methoxy-3-methylpyrazolo[1,5-a]pyridine-6-carbonyl)-2-azabicyclo[2.2.1]heptan-7-yl)carbamate

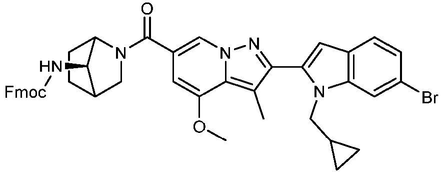

Sodium bicarbonate (115 mg, 1.367 mmol) was added to a stirred solution of Intermediate 97A (250 mg, 0.456 mmol) in a mixture of solvents dioxane (1 ml) and water (1 ml), followed by the addition of (9H-fluoren-9-yl)methyl carbonochloridate (118 mg, 0.456 mmol). The reaction mixture was diluted with DCM and stirred for about 5 min. The organic layer was separated and concentrated under reduced pressure to afford crude compound, which was purified using a silica gel column (eluting with 0-60% EtOAc in hexane) to afford the title compound (70 mg, 20%) as off-white solid. LC-MS m/z: 772.3 [M+H]⁺.

### Intermediate 97C

### (9H-Fluoren-9-yl)methyl-((7R)-2-(2-(6-acetyl-1-(cyclopropylmethyl)-1H-indol-2-yl)-4-methoxy-3-methylpyrazolo[1,5-a]pyridine-6-carbonyl)-2-azabicyclo[2.2.1]heptan-7-yl)carbamate

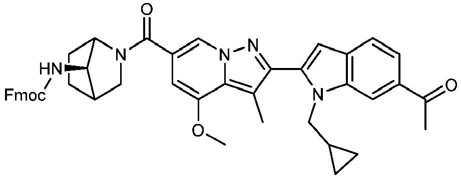

Tributyl(1-ethoxyvinyl)stannane (46.9 mg, 0.130 mmol) and Pd(PPh₃)₂Cl₂ (9.11 mg, 0.013 mmol) were added to a solution of Intermediate 97B (100 mg, 0.130 mmol) in toluene (3 m) in a vial. The reaction mixture was degassed with argon for 2 min, heated to 85°C and stirred for 16 hours, diluted in minimum amount of DCM and loaded onto a silica gel column for purification. Eluting with 0-100% EtOAc / petether afforded the ether cleaved title compound (70 mg, 74%) as a colourless syrup. LC-MS m/z: 734.4 [M+H]⁺.

### Intermediate 97D

### (9H-Fluoren-9-yl)methyl-((7R)-2-(2-(1-(cyclopropylmethyl)-6-(2-hydroxypropan-2-yl)-1H-indol-2-yl)-4-methoxy-3-methylpyrazolo[1,5-a]pyridine-6-carbonyl)-2-azabicyclo[2.2.1]heptan-7-yl)carbamate

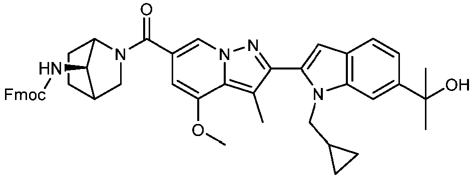

Methyl magnesium bromide (0.127 ml, 0.382 mmol, 3 M in THF) was added to a stirred solution of Intermediate 97C (70 mg, 0.095 mmol) in THF (3 ml) at 0°C. The reaction mixture was stirred for 1 hour, quenched with saturated NH₄Cl solution at 0°C, extracted with EtOAc, dried over Na₂SO₄, and concentrated under reduced pressure to afford a crude compound. Purification by prep-HPLC of the crude compound afforded the title compound (40 mg, 56%) as a white solid. LC-MS m/z: 750.4 [M+H]⁺.

### Example 97

Piperidine (10.56 µl, 0.107 mmol) was added drop wise to a stirred solution of Intermediate 97D (40 mg, 0.053 mmol) in DCM. The reaction mixture was stirred for 2 hours, and concentrated to afford a crude compound as off-white solid. Purification by Prep-HPLC afforded the title compound. The purified compound was further purified using RP-HPLC to afford the title compound (3 mg, 10%) as off-white solid. LC-MS m/z: 534.4 [M+H]⁺. HPLC retention time: 6.983 (Method C). ¹H NMR (400 MHz, methanol-*d*₄) δ ppm: 8.33 (s, 1H), 7.69 (s, 1H), 7.58 (d, J=8.0 Hz, 1H), 7.24 (dd, J=1.5, 8.5 Hz, 1H), 6.68 (s, 1H), 6.65-6.61 (m, 1H), 4.60 (s, 2H), 4.43-4.27 (m, 2H), 4.16-4.00 (m, 4H), 3.76-3.62 (m, 1H), 3.62-3.54 (m, 1H), 3.53-3.41 (m, 1H), 3.30-3.06 (m, 1H), 2.54 (s, 3H), 2.48-2.34 (m, 1H), 2.18-1.90 (m, 5H), 1.23-1.03 (m, 2H), 1.01-0.81 (m, 1H), 0.37-0.30 (m, 2H), 0.14-0.06 (m, 2H).

### Example 98

### ((3R,5R)-3-Amino-5-fluoropiperidin-1-yl)(2-(1-(cyclopropylmethyl)-6-(2-hydroxypropan-2-yl)-1H-indol-2-yl)-4-methoxy-3-methylpyrazolo[1,5-a]pyridin-6-yl)methanone

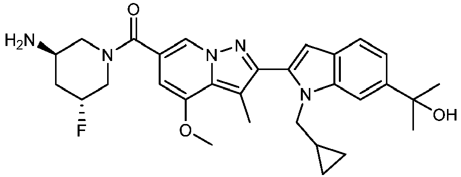

### Intermediate 98A

### ((3R,5R)-3-Amino-5-fluoropiperidin-1-yl)(2-(6-bromo-1-(cyclopropylmethyl)-1H-indol-2-yl)-4-methoxy-3-methylpyrazolo[1,5-a]pyridin-6-yl)methanone

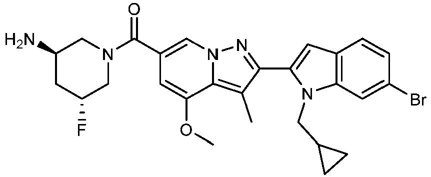

Intermediate 98A was prepared following a procedure similar to the preparation of Intermediate 97A (70 mg, 100%). LCMS m/z: 778.3 [M+H]⁺.

### Intermediate 98B

### (9H-Fluoren-9-yl)methyl-((3R,5R)-1-(2-(6-bromo-1-(cyclopropylmethyl)-1H-indol-2-yl)-4-methoxy-3-methylpyrazolo[1,5-a]pyridine-6-carbonyl)-5-fluoropiperidin-3-yl)carbamate

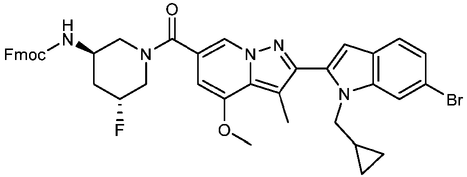

Intermediate 98B was prepared following a procedure similar to the preparation of Intermediate 97B (65 mg, 66%) as an off-white solid. LC-MS m/z: 778.3 [M+H]⁺.

### Intermediate 98C

### (9H-Fluoren-9-yl)methyl-((3R,5R)-1-(2-(6-acetyl-1-(cyclopropylmethyl)-1H-indol-2-yl)-4-methoxy-3-methylpyrazolo[1,5-a]pyridine-6-carbonyl)-5-fluoropiperidin-3-yl)carbamate

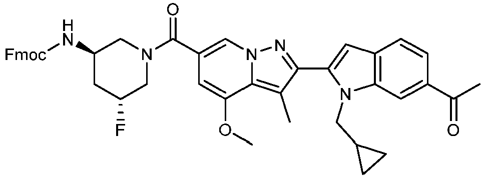

Intermediate 98C was prepared following a procedure similar to the preparation of Intermediate 97C (crude 70 mg) as black syrup. LC-MS m/z: 740.4 [M+H]⁺.

### Intermediate 98D

### (9H-Fluoren-9-yl)methyl-((3R,5R)-1-(2-(1-(cyclopropylmethyl)-6-(2-hydroxypropan-2-yl)-1H-indol-2-yl)-4-methoxy-3-methylpyrazolo[1,5-a]pyridine-6-carbonyl)-5-fluoropiperidin-3-yl)carbamate

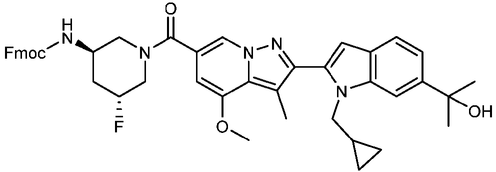

Intermediate 98D was prepared following a procedure similar to the preparation of Intermediate 97D (30 mg, 42%) as a pale yellow solid. LC-MS m/z: 756.2 [M+H]⁺.

### Example 98

Compound of Example 98 was prepared following a procedure similar to the preparation of a compound of Example 97 (1.5 mg, 6%), as a pale yellow solid. LC-MS m/z: 534.2 [M+H]⁺. HPLC retention time: 6.983 min (Method C). ¹H NMR (400 MHz, DMSO-*d*₆) δ ppm: 8.34 (s, 1H), 7.65 (s, 1H), 7.52 (d, J=8.5 Hz, 1H), 7.19 (dd, J=1.3, 8.3 Hz, 1H), 6.68 (s, 1H), 6.53 (s, 1H), 5.01-4.93 (m, 2H), 4.35 (br d, J=6.5 Hz, 3H), 3.97 (s, 4H), 3.26-3.09 (m, 1H), 3.08-2.82 (m, 2H), 2.62 (m, 3H), 2.43-2.25 (m, 2H), 2.24-1.99 (m, 1H), 1.51 (s, 6H), 1.17-1.05 (m, 1H), 0.33-0.28 (m, 2H), 0.17-0.12 (m, 2H).

### Example 99

### ((3R,5R)-3-Amino-5-fluoropiperidin-1-yl)(2-(1-(cyclopropylmethyl)-6-(1,1,1-trifluoro-2-hydroxypropan-2-yl)-1H-indol-2-yl)-4-methoxy-3-methylpyrazolo[1,5-a]pyridin-6-yl)methanone

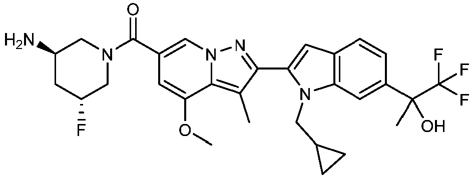

### Intermediate 99A

### tert-Butyl-((3R,5R)-1-(2-(6-acetyl-1-(cyclopropylmethyl)-1H-indol-2-yl)-4-methoxy-3-methylpyrazolo[1,5-a]pyridine-6-carbonyl)-5-fluoropiperidin-3-yl)carbamate

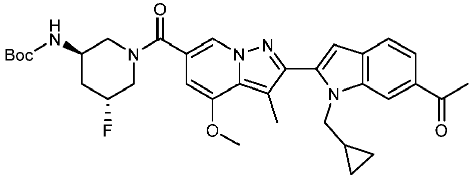

Intermediate 99A was prepared following a procedure similar to the preparation of Intermediate 97C (200 mg, 66%) as a glassy solid. LC-MS m/z: 618.4 [M+H]⁺.

### Intermediate 99B

### tert-Butyl-((3R,5R)-1-(2-(1-(cyclopropylmethyl)-6-(1,1,1-trifluoro-2-hydroxypropan-2-yl)-1H-indol-2-yl)-4-methoxy-3-methylpyrazolo[1,5-a]pyridine-6-carbonyl)-5-fluoropiperidin-3-yl)carbamate

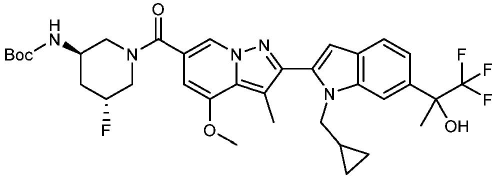

Trimethyl(trifluoromethyl)silane (0.142 ml, 0.283 mmol, 2 M in THF) was added to a solution of Intermediate 99A (35 mg, 0.057 mmol) in DME (0.5 ml) in a 8 ml chemglass vial, followed by the addition of caesium fluoride (1 mg, 6.58 µmol) on the walls of the vial. The vial was closed and the contents were mixed, stirred at a room temperature for 16 hours, and concentrated under vacuum to afford a crude title compound (30 mg, 70%), which was used in the next step without further purification. LC-MS m/z: 760 [M+H]⁺.

### Example 99

2,6-Lutidine (0.018 ml, 0.158 mmol) was added to a stirred solution of Intermediate 99B (30 mg, 0.039 mmol) in DCM (1 ml), and the reaction mixture was cooled to 0°C, followed by the addition of TMS-OTf (0.014 ml, 0.079 mmol). The reaction mixture was stirred at 0°C for 30 minutes, and additional 28 µl of TMS-OTf was added. The contents were allowed to warm to a room temperature, stirred for 30 minutes, quenched by the addition of 10% NaHCO₃ solution, and kept on standby over the weekend. The mixture was extracted with EtOAc (4×10 ml), dried over Na₂SO₄, concentrated under vacuum and the crude compound was purified using prep-HPLC. The purified compound was further purified using RP-HPLC to afford the title compound (2.2 mg, 10%). LC-MS m/z: 588 [M+H]⁺. LC-MS retention time: 1.889 min (Method E). ¹H NMR (400 MHz, DMSO-*d*₆) δ ppm: 8.39 (s, 1H), 7.80 (s, 1H), 7.61 (d, J=8.3 Hz, 1H), 7.30 (br, d, J=8.3 Hz, 1H), 6.75 (s, 1H), 6.55 (s, 1H), 5.10-4.92 (m,1H), 4.41-4.36 (m, 2H), 3.98 (s, 3H),3.80- 3.17 (m, 1H),2.54 (s, 3H), 2.30-2.21 (m, 2H), 1.79 (br, s, 3H), 1.31-1.07 (m, 1H), 0.91-0.77 (m, 2H), 0.36-0.24 (m, 2H),0.16 (br, d, J=5.1 Hz, 2H).

### Example 100

### ((7R)-7-Amino-2-azabicyclo[2.2.1]heptan-2-yl)(2-(1-(cyclopropylmethyl)-6-(2-hydroxypropan-2-yl)-1H-indol-2-yl)-3-methylpyrazolo[1,5-a]pyridin-6-yl)methanone

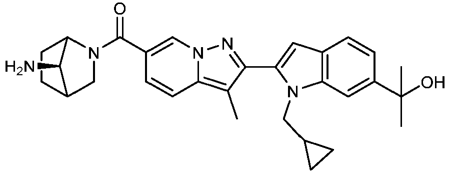

### Intermediate 100A

### tert-Butyl-((7R)-2-(2-(6-acetyl-1-(cyclopropylmethyl)-1H-indol-2-yl)-3-methylpyrazolo[1,5-a]pyridine-6-carbonyl)-2-azabicyclo[2.2.1]heptan-7-yl)carbamate

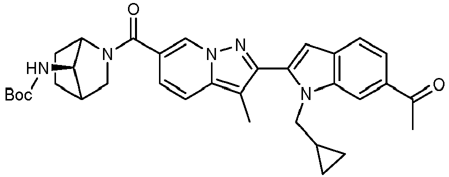

Intermediate 100A was prepared following a procedure similar to the preparation of Intermediate 97C (70 mg, 37%). LC-MS m/z: 582.4 [M+H]⁺.

### Intermediate 100B

### 1-(2-(6-((7R)-7-Amino-2-azabicyclo[2.2.1]heptane-2-carbonyl)-3-methylpyrazolo[1,5-a]pyridin-2-yl)-1-(cyclopropylmethyl)-1H-indol-6-yl)ethan-1-one

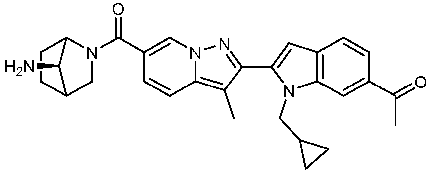

TFA (9.27 µl, 0.120 mmol) was added to a stirred solution of Intermediate 100A (70 mg, 0.120 mmol) in DCM (0.5 ml). The reaction mixture was stirred at a room temperature for 2 hours, and concentrated under reduced pressure to afford a crude product (30 mg, 52%). LC-MS m/z: 482.3 [M+H]⁺.

### Example 100

Methyl magnesium bromide (0.415 ml, 0.415 mmol) was added to a stirred solution of Intermediate 100B (100 mg, 0.208 mmol) in THF (3 ml) at 0°C. The reaction mixture was stirred for 3 hours, allowed to warm to a room temperature, stirred for 3 hours, quenched with saturated ammonium chloride (50 ml), and extracted with EtOAc (2×100 ml). The combined organic layers were dried over sodium sulphate, filtered, and the filtrate was concentrated under reduced pressure to afford the title compound. The crude compound was purified by prep-HPLC. The purified compound was further purified using RP-HPLC to afford the title compound (1.6 mg, 1.5%). LC-MS m/z: 498.4 [M+H]. HPLC retention time: 5.115 min (Method C). ¹H NMR (400 MHz, methanol-*d*₄) δ ppm: 8.73 (s, 1H), 7.74-7.64 (m, 2H), 7.57 (d, *J*=8.0 Hz, 1H), 7.31 (dd, *J*=1.0, 9.0 Hz, 1H), 7.24 (d, *J*=8.6 Hz, 1H), 6.72 (s, 1H), 4.42 (d, *J*=6.5 Hz, 2H), 4.33 (br s, 1H), 4.01 (s, 1H), 3.84-3.58 (m, 1H), 3.49 (s, 1H), 3.42 (s, 1H), 3.28-3.20 (m, 2H), 3.14 (d, *J*=1.5 Hz, 1H), 2.43 (s, 3H), 2.40-2.30 (m, 1H), 2.21 (s, 1H), 2.14-2.01 (m, 2H), 1.99-1.91 (m, 1H), 1.90 (s, 1H), 1.64 (s, 7H), 1.29 (s, 6H), 1.18-1.09 (m, 1H), 0.36-0.28 (m, 2H), 0.10 (s, 2H).

### Example 101

### ((7R)-7-Amino-2-azabicyclo[2.2.1]heptan-2-yl)(2-(1-(cyclopropylmethyl)-6-(1,1,1-trifluoro-2-hydroxypropan-2-yl)-1H-indol-2-yl)-3-methylpyrazolo[1,5-a]pyridin-6-yl)methanone

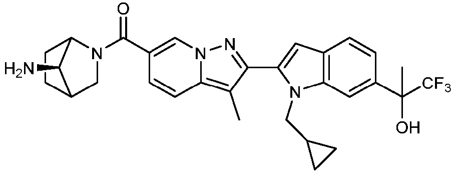

### Intermediate 101A

### tert-Butyl-((7R)-2-(2-(1-(cyclopropylmethyl)-6-(1,1,1-trifluoro-2-hydroxypropan-2-yl)-1H-indol-2-yl)-3-methylpyrazolo[1,5-a]pyridine-6-carbonyl)-2-azabicyclo[2.2.1]heptan-7-yl)carbamate

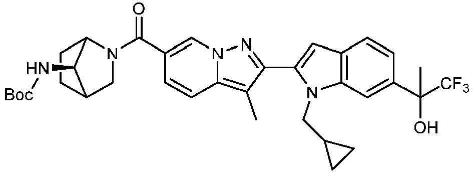

Me₃SiCF₃ (430 µl, 0.860 mmol) was added to a stirred solution of Intermediate 100A (50 mg, 0.086 mmol) in THF (1 ml) at -78°C, followed by slow addition of TBAF (215 µl, 0.430 mmol). The reaction mixture was allowed to warm to a room temperature, stirred for 3 hours, quenched with water (100 ml), and extracted with EtOAc (2×100 ml). The combined organic layers were dried over sodium sulphate, filtered, and the filtrate was concentrated under reduced pressure to afford a crude title compound (30 mg, 54%). LC-MS m/z: 652.3 [M+H]⁺.

### Example 101

HCl (1 ml, 4 M in dioxane) was added to a stirred solution of Intermediate 101A (40 mg, 0.061 mmol) in dioxane (0.5 ml) at a room temperature. The reaction mixture was stirred for 2 hours, concentrated under reduced pressure, and the crude product obtained was purified using prep-HPLC. The purified product was further purified using RP-HPLC to afford the title compound (8.6 mg, 23%). LC-MS m/z: 552.3 [M+H]. HPLC retention time: 6.316 min (Method C). ¹H NMR (400 MHz, methanol-*d₄*) δ ppm: 8.80 (s, 1H), 8.73 (s, 1H), 7.82 (s, 1H), 7.73-7.59 (m, 2H), 7.36-7.29 (m, 2H), 6.76 (s, 1H), 5.05-4.88 (m, 1H), 4.43 (d, *J*=6.5 Hz, 2H), 4.33 (s, 1H), 4.00 (s, 1H), 3.72-3.63 (m, 1H), 3.29-3.20 (m, 4H), 2.43 (s, 3H), 2.31 (br s, 1H), 2.14-1.87 (m, 3H), 1.83 (s, 3H), 1.74-1.52 (m, 3H), 1.46-1.24 (m, 3H), 1.14-1.07 (m, 1H), 1.02 (t, *J*=7.5 Hz, 3H), 0.90 (s, 1H), 0.36-0.28 (m, 2H), 0.15-0.03 (m, 2H).

### Example 102

### ((7R)-7-Amino-2-azabicyclo[2.2.1]heptan-2-yl)(2-(1-(cyclopropylmethyl)-6-(2-hydroxypropan-2-yl)-1H-pyrrolo[2,3-b]pyridin-2-yl)-3-methylpyrazolo[1,5-a]pyridin-6-yl)methanone

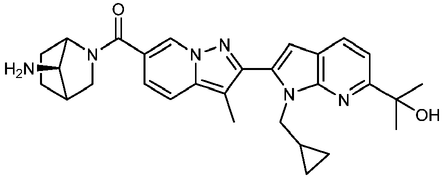

### Intermediate 102A

### tert-Butyl-((7R)-2-(2-(6-acetyl-1-(cyclopropylmethyl)-1H-pyrrolo[2,3-b]pyridin-2-yl)-3-methylpyrazolo[1,5-a]pyridine-6-carbonyl)-2-azabicyclo[2.2.1]heptan-7-yl)carbamate

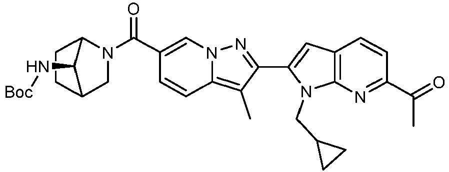

Intermediate 102A was prepared following a procedure similar to the preparation of Intermediate 97C (150 mg, 74%). LC-MS m/z: 583.34 [M+H]⁺.

### Intermediate 102B

### tert-Butyl-((7R)-2-(2-(1-(cyclopropylmethyl)-6-(2-hydroxypropan-2-yl)-1H-pyrrolo[2,3-b]pyridin-2-yl)-3-methylpyrazolo[1,5-a]pyridine-6-carbonyl)-2-azabicyclo[2.2.1]heptan-7-yl)carbamate

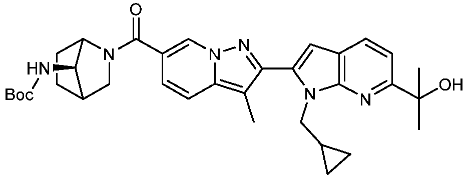

Intermediate 102B was prepared following a procedure similar to the preparation of Intermediate 97D (70 mg, 68%) LC-MS m/z: 599.4 [M+H]⁺.

### Example 102

Compound of Example 102 was prepared following a procedure similar to the preparation of a compound of Example 1 (12.7 mg, 22%) LC-MS m/z: 499.4 [M+H]⁺. HPLC retention time: 5.846 min (Method C). ¹H NMR (400 MHz, DMSO-*d*₆) δ ppm: 9.14-8.85 (m, 1H), 8.06 (d, J=8.5 Hz, 1H), 7.93-7.79 (m, 1H), 7.54 (d, J=8.5 Hz, 1H), 7.48-7.27 (m, 1H), 6.88 (s, 1H), 4.71-4.47 (m, 2H), 4.36-4.21 (m, 1H), 3.92-3.78 (m, 1H), 3.69-3.56 (m, 2H), 2.73 (s, 2H), 2.54 (s, 3H), 2.52-2.31 (m, 6H), 2.15-1.83 (m, 4H), 1.78-1.70 (m, 1H), 1.59 (s, 6H), 1.39-1.14 (m, 1H), 0.43-0.34 (m, 4H).

### Example 103

### ((7R)-7-Amino-2-azabicyclo[2.2.1]heptan-2-yl)(2-(1-(cyclopropylmethyl)-6-(1,1,1-trifluoro-2-hydroxypropan-2-yl)-1H-pyrrolo[2,3-b]pyridin-2-yl)-3-methylpyrazolo[1,5-a]pyridin-6-yl)methanone

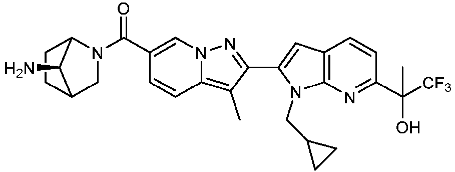

### Intermediate 103A

### tert-Butyl-((7R)-2-(2-(1-(cyclopropylmethyl)-6-(1,1,1-trifluoro-2-hydroxypropan-2-yl)-1H-pyrrolo[2,3-b]pyridin-2-yl)-3-methylpyrazolo[1,5-a]pyridine-6-carbonyl)-2-azabicyclo[2.2.1]heptan-7-yl)carbamate

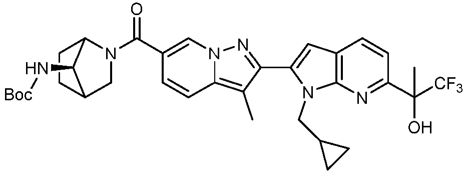

Intermediate 103A was prepared following a procedure similar to the preparation of Intermediate 101A (50 mg, 45%). LC-MS m/z: 653.4 [M+H]⁺.

### Example 103

Compound of Example 103 was prepared following a procedure similar to the preparation of Example 101 (1.9 mg, 3%) LC-MS m/z: 553.4 [M+H]⁺. HPLC retention time: 6.865 min (Method C). ¹H NMR (400 MHz, methanol-*d*₄) δ ppm: 9.00-8.80 (m, 1H), 8.10 (d, J=8.5 Hz, 1H), 7.76 (br s, 1H), 7.52 (d, J=8.0 Hz, 1H), 7.39 (br s, 1H), 6.87 (s, 1H), 4.96-4.91 (m, 1H), 4.61 (dd, J=7.0, 3.5 Hz, 2H), 4.48-4.33 (m, 1H), 3.93 (s, 1H), 3.77 (br s, 1H), 2.72 - 2.84 (m, 2H), 2.49 (s, 3H), 2.23 (s, 1H), 2.01 (br s, 2H), 1.86 (s, 3H), 1.49-1.22 (m, 2H), 1.18 (br s, 1H), 0.35-0.12 (m, 4H).

### Example 104

### ((7R)-7-Amino-2-azabicyclo[2.2.1]heptan-2-yl)(2-(1-(cyclopropylmethyl)-6-(1-hydroxyethyl)-1H-pyrrolo[2,3-b]pyridin-2-yl)-3-methylpyrazolo[1,5-a]pyridin-6-yl)methanone

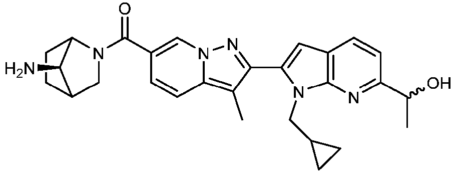

### Intermediate 104A

### tert-Butyl-((7R)-2-(2-(1-(cyclopropylmethyl)-6-(1-hydroxyethyl)-1H-pyrrolo[2,3-b]pyridin-2-yl)-3-methylpyrazolo[1,5-a]pyridine-6-carbonyl)-2-azabicyclo[2.2.1]heptan-7-yl)carbamate

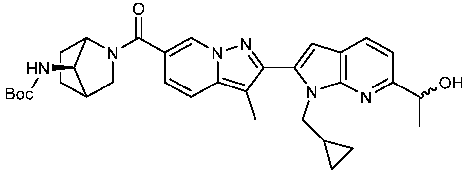

Sodium borohydride (4.54 mg, 0.120 mmol) was added to a stirred solution of Intermediate 102A (70 mg, 0.120 mmol) in methanol. The reaction mixture was stirred at a room temperature for 3 hours, quenched with saturated ammonium chloride (100 ml), and extracted with EtOAc (2×100 ml). The combined organic layers were dried over sodium sulphate, filtered, and the filtrate was concentrated under reduced pressure to afford a crude title compound (40 mg, 57%). LC-MS m/z: 585.4 [M+H]⁺.

### Example 104

Compound of Example 104 was prepared following a procedure similar to the preparation of Example 1 (5.6 mg, 10%). LC-MS m/z: 485.4 [M+H]⁺; HPLC retention time: 7.898 min (Method C). ¹H NMR (400 MHz, methanol-*d*₄) δ ppm: 8.89-8.83 (br, s, 1H), 8.03 (d, J=8.5 Hz, 1H), 7.75 (br, s, 1H), 7.37 (br, s, 1H), 7.31 (d, J=8.0 Hz, 1H), 6.80 (s, 1H), 5.10-4.92 (m, 1H), 4.74 (br, s, 1H), 4.60 (d, J=7.0 Hz, 1H), 4.44 (br, s, 1H), 3.92 (br, d, J=6.5 Hz, 1H), 3.77 (br, s, 1H), 3.67 (br, s, 1H), 3.58-3.38 (m, 1H), 2.87-2.63 (m, 1H), 2.48 (s, 3H), 2.21 (br, s, 1H), 1.87-2.02 (br ,s, 2H), 1.64-1.53 (m, 3H), 1.42-1.24 (m, 2H), 1.24-1.06 (m, 1H), 0.34-0.18 (m, 4H).

### Example 105

### ((3R,5R)-3-Amino-5-fluoropiperidin-1-yl)(2-(1-(cyclopropylmethyl)-6-(2-hydroxypropan-2-yl)-1H-pyrrolo[2,3-b]pyridin-2-yl)-3-methylpyrazolo[1,5-a]pyridin-6-yl)methanone

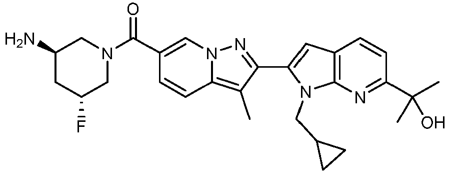

### Intermediate 105A

### tert-Butyl-((3R,5R)-1-(2-(6-acetyl-1-(cyclopropylmethyl)-1H-pyrrolo[2,3-b]pyridin-2-yl)-3-methylpyrazolo[1,5-a]pyridine-6-carbonyl)-5-fluoropiperidin-3-yl)carbamate

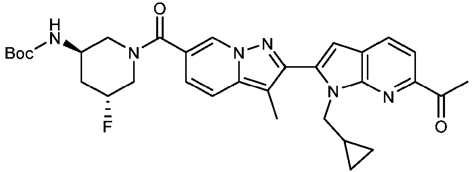

Intermediate 105A was prepared following a procedure similar to the preparation of Intermediate 97C (400 mg, 79%). LC-MS: 589.3 [M+H]⁺.

### Intermediate 105B

### tert-Butyl-((3R,5R)-1-(2-(1-(cyclopropylmethyl)-6-(2-hydroxypropan-2-yl)-1H-pyrrolo[2,3-b]pyridin-2-yl)-3-methylpyrazolo[1,5-a]pyridine-6-carbonyl)-5-fluoropiperidin-3-yl)carbamate

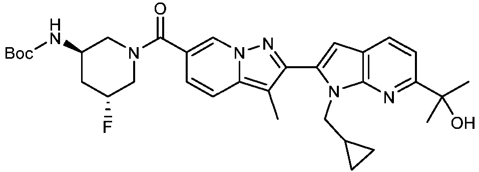

Intermediate 105B was prepared following a procedure similar to the preparation of Intermediate 97D (30 mg, 29%). LC-MS m/z: 605.3 [M+H]⁺.

### Example 105

Compound of Example 105 was prepared following a procedure similar to the preparation of the compound of Example 1 (14 mg, 20%). LC-MS m/z: 505.4 [M+H]⁺; HPLC retention time: 7.101 min (Method C). ¹H NMR (400 MHz, methanol-*d*₄) δ ppm: 8.74 (t, J=1.3 Hz, 1H), 8.01 (d, J=8.0 Hz, 1H), 7.75 (d, J=9.4 Hz, 1H), 7.42 (d, J=8.0 Hz, 1H), 7.28 (dd, J=1.5, 9.0Hz, 1H), 6.78 (s, 1H), 5.06-5.01 (m, 1H), 4.98-4.88 (m, 1H), 4.84-4.73 (m, 2H), 4.57 (d, J=6.5 Hz, 3H), 4.51 (br d, J=3.5 Hz, 1H), 4.47-4.20 (m, 1H),3.63 (s, 1H), 3.55-3.36 (m, 3H), 3.31-3.28 (m, 1H), 3.18-3.14 (m, 1H), 2.99-2.83 (m, 1H), 2.53-2.41 (m, 4H), 1.97 (s, 2H), 1.83-1.64 (m, 8H), 1.39 -1.28 (m, 1H), 1.25-1.14 (m, 1H), 0.93 (d, J=6.5 Hz, 1H), 0.36-0.25 (m, 4H)

### Examples 106 and 107

### Intermediates 106A and 107A

### tert-Butyl-((3R,5R)-1-(2-(1-(cyclopropylmethyl)-6-(1,1,1-trifluoro-2-hydroxypropan-2-yl)-1H-pyrrolo[2,3-b]pyridin-2-yl)-3-methylpyrazolo[1,5-a]pyridine-6-carbonyl)-5-fluoropiperidin-3-yl)carbamate

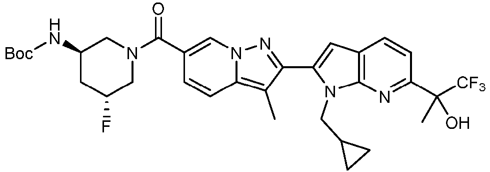

Intermediates 106A and 107A were obtained following a procedure similar to the preparation of Intermediate 101A. Both the diastereomers were isolated by chiral SFC method. Diastereomer-1: (35 mg, 8%). LC-MS m/z: 659.4 [M+H]⁺. Diastereomer-2: (35 mg, 8%). LC-MS m/z: 659.4 [M+H]⁺.

### Examples 106 and 107

### ((3R,5R)-3-Amino-5-fluoropiperidin-1-yl)(2-(1-(cyclopropylmethyl)-6-(1,1,1-trifluoro-2-hydroxypropan-2-yl)-1H-pyrrolo[2,3-b]pyridin-2-yl)-3-methylpyrazolo[1,5-a]pyridin-6-yl)methanone

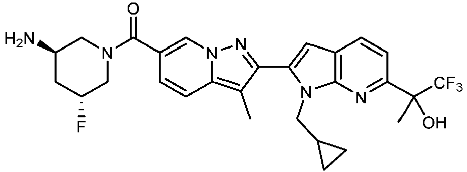

TFA was added to the diastereomer 106A (35mg) in DCM, and the reaction mixture was stirred at a room temperature for 2 hours The volatiles were removed under vacuum and the crude product was purified by HPLC. The purified product was further purified using RP-HPLC to afford a compound of Example 106 (30 mg, 94%). LC-MS m/z: 559.27 [M+H]⁺; HPLC retention time: 7.046 min (Method C). ¹H NMR (400 MHz, methanol-*d*₄) δ ppm: 8.79 (s, 1H), 8.10 (d, J=8.0 Hz, 1H), 7.78 (d, J=9.1 Hz, 1H), 7.52 (d, J=8.0 Hz, 1H), 7.30 (dd, J=9.0, 1.5 Hz, 1H), 6.87 (s, 1H), 4.80-4.88 (m, 1H), 4.64-4.50 (m, 2H), 3.72-3.42 (m, 2H), 3.11-2.95 (m, 1H), 2.50 (s, 3H), 1.96 (s, 1H), 1.86 (s, 4H), 1.31 (s, 3H), 1.39-1.15 (m, 1H), 1.24-1.12 (m, 1H), 0.36-0.26 (m, 4H).

Compound of Example 107 was prepared analogous to the compound of Example 106 (21 mg, 66%). LC-MS m/z: 559.27 [M+H]⁺; HPLC retention time: 7.048 min (Method C). ¹H NMR (400 MHz, methanol-*d*₄) δ ppm: 8.79 (s, 1H), 8.10 (d, J=8.0 Hz, 1H), 7.78 (d, J=9.1 Hz, 1H), 7.52 (d, J=8.0 Hz, 1H), 7.30 (dd, J=9.0, 1.5 Hz, 1H), 6.87 (s, 1H), 4.80-4.88 (m, 1H), 4.64-4.50 (m, 2H), 3.72-3.42 (m, 2H), 3.11-2.95 (m, 1H), 2.50 (s, 3H), 1.96 (s, 1H), 1.86 (s, 4H), 1.31 (s, 3H), 1.39-1.15 (m, 1H), 1.24-1.12 (m, 1H), 0.36-0.26 (m, 4H).

### Example 108

### ((7R)-7-Amino-2-azabicyclo[2.2.1]heptan-2-yl)(2-(6-chloro-1-(cyclopropylmethyl)-1H-pyrrolo[2,3-b]pyridin-2-yl)-3-methylpyrazolo[1,5-a]pyrimidin-6-yl)methanone

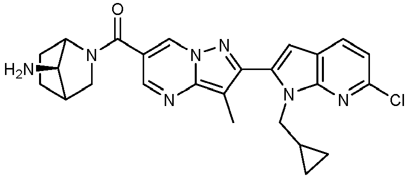

### Intermediate 108A

### 5-Bromo-4-methyl-1H-pyrazol-3-amine

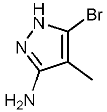

Borane dimethyl sulfide complex (3.04 ml, 32.0 mmol) was added to a stirred solution of ethyl 3-amino-5-bromo-1H-pyrazole-4-carboxylate (3.0 g, 12.82 mmol) in THF (30 ml) at 0°C. The reaction mixture was stirred for 1 hour, heated to 65°C and stirred further for 16 hours, slowly quenched with methanol (5 ml) at a room temperature and stirred at 65°C for 1 hour, the volatiles were removed under vacuum, and the residue obtained was washed with saturated brine solution and extracted with DCM (2×50 ml). The combined organic extracts were dried over sodium sulphate, filtered, and the filtrate was evaporated under reduced pressure to afford the title compound (1.55g, 68%) as a white solid. LC-MS m/z: 176.0 [M+H]⁺.

### Intermediate 108B

### Ethyl-2-bromo-3-methylpyrazolo[1,5-a]pyrimidine-6-carboxylate

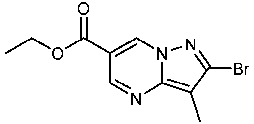

Acetic acid (5 ml) was added to a stirred solution of Intermediate 108A (0.8g, 4.55 mmol) and Ethyl 2-formyl-3-oxopropanoate (0.655 g, 4.55 mmol) in ethanol (25 ml) at 30°C. The reaction mixture was stirred at 80°C for 16 hours, evaporated to dryness, quenched with brine solution and extracted with DCM (2×50 ml). The combined organic extracts were dried over sodium sulphate, filtered, and the filtrate was evaporated under reduced pressure to afford the crude product, which was loaded onto a silica gel column (24g) and purified using EtOAc in hexane (0-100%) in a gradient manner to afford the title compound (460 mg, 36%). LC-MS m/z: 285.9 [M+H]⁺. ¹H NMR (400 MHz, CDCl₃) δ ppm: 9.2- 9.1 (m, 1H), 8.9- 8.8 (m, 1H), 4.5- 4.4 (m, 2H), 2.3 (s, 3H), 1.5-1.4 (m, 3H).

### Intermediate 108C

### 2-Bromo-3-methylpyrazolo[1,5-a]pyrimidine-6-carboxylic acid

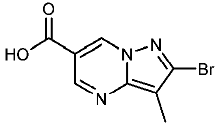

LiOH (388 mg, 16.19 mmol) in water (1 ml) was added to a stirred solution of Intermediate 108B (460 mg, 1.619 mmol) in THF (6.00 ml) and methanol (3 ml). The reaction mixture was stirred at a room temperature for 16 hours, evaporated to dryness, acidified with citric acid solution, and extracted with DCM (2×50 ml). The combined organic extracts were dried over sodium sulphate, filtered, and the filtrate was evaporated under reduced pressure to afford the title compound as a crude product (380 mg, 92%). LC-MS m/z: 257.0 [M+H]⁺. ¹H NMR (400 MHz, MeOD) δ ppm: 9.21- 9.20 (d, 1H, *J*=2.4Hz), 8.95- 8.94 (d, 1H, *J*=2.8 Hz), 2.4 (s, 3H).

### Intermediate 108D

### tert-Butyl-((7R)-2-(2-bromo-3-methylpyrazolo[1,5-a] pyrimidine-6-carbonyl)-2-azabicyclo[2.2.1]heptan-7-yl)carbamate

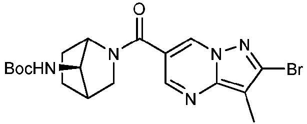

HATU (80 mg, 0.211 mmol) and DIPEA (0.092 ml, 0.527 mmol) were added to a stirred solution of Intermediate 108C (45 mg, 0.176 mmol), tert-butyl ((7R)-2-azabicyclo[2.2.1]heptan-7-yl)carbamate (44.8 mg, 0.211 mmol) in DMF (3 ml). The reaction mixture was stirred at a room temperature for 16 hours, evaporated to dryness, quenched with brine solution, and extracted with DCM (2×50 ml). The combined organic extracts were dried over sodium sulphate, filtered, and the filtrate was evaporated under reduced pressure to afford the crude material. The crude material was loaded onto a silica gel column (12g) and purified using EtOAc in hexane (0-100%) in a gradient manner to afford the title compound (55 mg, 70%). LC-MS m/z: 396.1 [M-54]. ¹H NMR (400 MHz, MeOD) δ ppm: 8.73-8.72 (d, 1H, *J*=2.4Hz), 8.70-8.69 (d, 1H, *J*=2.8 Hz), 3.80-3.76 (m, 1H), 3.27-3.23 (m, 1H), 3.15-2.96 (m, 2H), 2.53 (m, 1H), 2.35-2.34 (s, 3H), 1.91-1.61 (m, 4H), 1.40 (s, 9H).

### Intermediate 108E

### tert-Butyl-((7R)-2-(2-(6-chloro-1-(cyclopropylmethyl)-1H-pyrrolo[2,3-b]pyridin-2-yl)-3-methylpyrazolo[1,5-a]pyrimidine-6-carbonyl)-2-azabicyclo[2.2.1]heptan-7-yl)carbamate

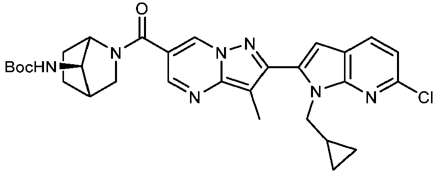

Pd(PPh₃)₄ (10.78 mg, 9.33 µmol) was added to a stirred solution of Intermediate 108D (210 mg, 0.466 mmol), 6-chloro-1-(cyclopropylmethyl)-2-(trimethylstannyl)-1H-pyrrolo[2,3-b]pyridine (Intermediate-7, 215 mg, 0.583 mmol) and CuI (17.76 mg, 0.093 mmol) in dioxane (5 ml), and the reaction mixture was heated to 110°C and stirred for 3 hours The reaction mixture was evaporated to dryness to afford the crude material. The crude material was loaded onto a silica gel column (12g) and purified using EtOAc in hexane (0-100%) in a gradient manner to afford the title compound (220 mg, 82%). LC-MS m/z: 576.3 [M+H]⁺. ¹H NMR (400 MHz, MeOD) δ ppm 7.7 - 7.6 (m, 1H), 7.5 - 7.3 (m, 1H), 7.2 - 7.1 (m, 1H), 7.0 (m, 1H), 6.6 - 6.4 (m, 1H), 4.02 - 4.00 (m, 2H), 3.96 - 3.94 (m, 2H), 3.69 - 3.66 (m, 2H), 2.16 - 2.06 (s, 3H), 1.92 (m, 1H), 1.8 - 1.5 (m, 4H), 0.90 - 0.85 (m, 1H), 0.65 - 0.61 (m, 2H), 0.40 - 0.37 (m, 2H).

### Example 108

TFA (0.033 ml, 0.434 mmol) was added to a stirred solution of Intermediate 108E (25 mg, 0.043 mmol) in DCM (5ml). The reaction mixture was stirred at a room temperature for 16 hours, evaporated to dryness and the crude material was purified using prep-HPLC. The purified product was further purified using RP-HPLC to afford the title compound (5.5 mg, 27%). LC-MS m/z: 476.3 [M+H]⁺. LC-MS retention time: 2.76 min, (Method H). ¹H NMR (400 MHz, DMSO-*d*₆) δ ppm: 9.3-9.1 (m, 1H), 8.7-8.6 (m, 1H), 8.1-8.0 (m, 1H), 7.2-7.1 (m, 1H), 6.9 (s, 1H), 4.7-4.6 (m, 2H), 4.5 (m, 1H), 4.4-4.1 (m, 1H), 3.9-3.5 (m, 2H), 2.6 (s, 3H), 2.55-2.45 (m, 1H), 2.2-2.0 (m, 2H), 1.2-1.1 (m, 1H), 1.0-0.8 (m, 2H), 0.4-0.3 (m, 2H).

### Example 109

### ((7R)-7-Amino-2-azabicyclo[2.2.1]heptan-2-yl)(2-(1-(cyclopropylmethyl)-6-(2-fluoro-3-hydroxyphenyl)-1H-pyrrolo[2,3-b]pyridin-2-yl)-3-methylpyrazolo[1,5-a]pyrimidin-6-yl)methanone

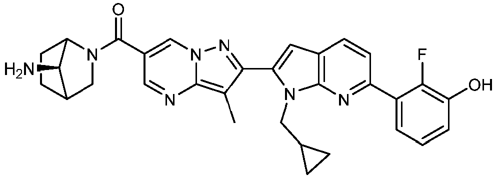

### Intermediate 109A

### tert-Butyl-((7R)-2-(2-(1-(cyclopropylmethyl)-6-(2-fluoro-3-hydroxyphenyl)-1H-pyrrolo[2,3-b]pyridin-2-yl)-3-methylpyrazolo[1,5-a]pyrimidine-6-carbonyl)-2-azabicyclo[2.2.1]heptan-7-yl)carbamate

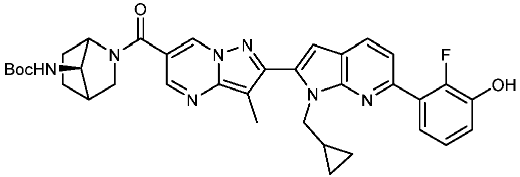

Pd(PPh₃)₄ (3.51 mg, 3.04 µmol) was added to a stirred solution of Intermediate 108E (35 mg, 0.061 mmol), 2-fluoro-3-(4,4,5,5-tetramethyl-1,3,2-dioxaborolan-2-yl)phenol (17.36 mg, 0.073 mmol) and K₂CO₃ (20.99 mg, 0.152 mmol) in 1,4-dioxane (4 ml). The reaction mixture was stirred at 85°C for 3 hours, filtered through a celite bed, and the filtrate was evaporated to dryness to afford a crude material. The crude compound was used in the next step without further purification. LC-MS m/z: 652.4 [M+H]⁺.

### Example 109

TFA (0.047 ml, 0.614 mmol) was added to a stirred solution of Intermediate 109A (40 mg, 0.061 mmol) in DCM (5 ml). The reaction mixture was stirred at a room temperature for 16 hours, evaporated to dryness, and the crude material obtained was purified using RP-HPLC to afford the title compound (1.2 mg, 22%). LC-MS m/z: 552.3 [M+H]⁺. LC-MS retention time: 1.70 min /1.48 min. (Methods E / F). ¹H NMR (400 MHz, DMSO-*d*₆) δ ppm: 9.8 (s, 1H), 9.4-9.2 (m, 1H), 8.7-8.6 (m, 1H), 8.2-8.1 (m, 1H), 7.6-7.5 (m, 1H), 7.4-7.3 (m, 1H), 7.2-7.1 (m, 1H), 7.1-7.0 (m, 2H), 4.7-4.5 (m, 2H), 4.4-4.3 (m, 2H), 3.9-3.8 (m, 1H), 2.3 (s, 3H), 1.9-1.8 (m, 2H), 1.7-1.6 (m, 2H), 1.5 (m, 1H), 1.2-1.1 (m, 1H), 0.9-0.8 (m, 2H), 0.4- 0.3 (m, 2H).

Compounds of Examples 110 to 112 in Table 13 were prepared following a procedure similar to the preparation of a compound of Example 109, by Suzuki coupling of Intermediate 108E with appropriate boronate acid.

**Table 13**

| Example | Structure |
|---|---|
| | Name |
| | Analytical Data: LC-MS m/z [M+H]⁺; HPLC retention time 1 (HPLC method 1), HPLC retention time 2 (HPLC method 2), |
| 110 | 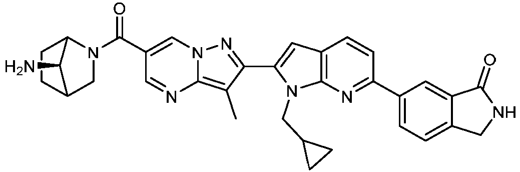 6-(2-(6-((7R)-7-Amino-2-azabicyclo[2.2.1]heptane-2-carbonyl)-3-methylpyrazolo[1,5-a]pyrimidin-2-yl)-1-(cyclopropylmethyl)-1H-pyrrolo[2,3-b]pyridin-6-yl)isoindolin-1-one |
| | Analytical Data: 573.3, 1.346 min (Method A), 1.341 min (Method B) |
| 111 | 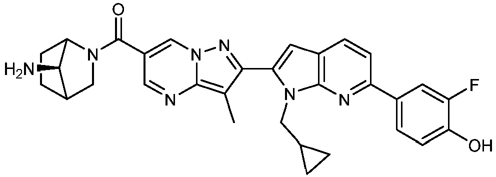 ((7R)-7-Amino-2-azabicyclo[2.2.1]heptan-2-yl)(2-(1-(cyclopropylmethyl)-6-(3-fluoro-4-hydroxyphenyl)-1H-pyrrolo[2,3-b]pyridin-2-yl)-3-methylpyrazolo[1,5-a]pyrimidin-6-yl)methanone |
| | Analytical Data: 552.3, 1.626 min (Method A), 1.481min (Method B) |
| 112 | 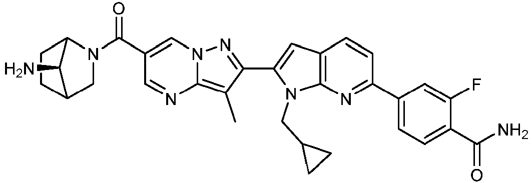 4-(2-(6-((7R)-7-Amino-2-azabicyclo[2.2.1]heptane-2-carbonyl)-3-methylpyrazolo[1,5-a]pyrimidin-2-yl)-1-(cyclopropylmethyl)-1H-pyrrolo[2,3-b]pyridin-6-yl)-2-fluorobenzamide |
| | Analytical Data: 579.3, 1.567 min (Method A), 1.366 min (Method B) |

### Example 113

### ((7R)-7-Amino-2-azabicyclo[2.2.1]heptan-2-yl)(2-(1-(cyclopropylmethyl)-1H-indol-2-yl)-4-methoxy-3-methylpyrazolo[1,5-a]pyrazin-6-yl)methanone

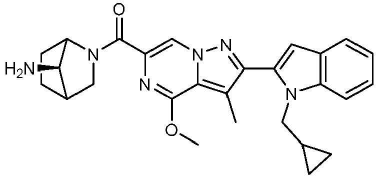

### Intermediate 113A

### Ethyl-5-bromo-4-methyl-1H-pyrazole-3-carboxylate:

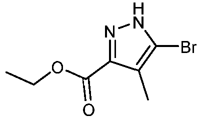

Bromine (3.34 ml, 64.9 mmol) was added to a stirred suspension of ethyl 4-methyl-1*H*-pyrazole-3-carboxylate (10.0 g, 64.9 mmol) in water (50 ml). The reaction mixture was stirred at a room temperature for 2 hours, diluted with EtOAc (50 ml), washed with saturated NaHCO₃ solution (20 ml), brine, dried over sodium sulfate, filtered, and the filtrate was concentrated in vacuum. The crude product thus obtained was purified using a silica gel column (120 g, Redisep^{®} SiO₂ column, eluting with 10% EtOAc in n-hexane) to obtain (9.0 g, 60%) of title compound as a white solid. LC-MS m/z: 231.0 [M-H]⁺. ¹H NMR (400 MHz, DMSO-*d*₆) δ ppm: 14.08 (br s, 1 H), 4.32 (q, *J*=7.03 Hz, 2 H), 2.16 (s, 3 H), 1.31 (t, *J*=7.03 Hz, 3 H).

### Intermediate 113B

### Ethyl-3-bromo-4-methyl-1-(2-oxopropyl)-1H-pyrazole-5-carboxylate (A2115-423)

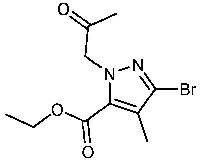

K₂CO₃ (6.40 g, 46.3 mmol) was added to a stirred solution of Intermediate 113A (9.0 g, 38.6 mmol) in DMF (30 ml), followed by the addition of 1-chloropropan-2-one (3.25 ml, 38.6 mmol). The resulting reaction mixture was stirred at a room temperature for 1 hour, diluted with water (20 ml), and extracted with EtOAc (3×30 ml). The combined organic layers were washed with brine, dried over sodium sulfate, filtered, and the filtrate was concentrated under reduced pressure. The crude product thus obtained was purified using a silica gel column (120 g, Redisep^{®} SiO₂ column, eluting with 14% EtOAc in n-hexane) to afford the title compound 6.5 g (58%) as off-white solid. LC-MS m/z: 288.9 [M+H]⁺. ¹H NMR (400 MHz, DMSO-*d*₆) δ ppm: 5.38 (s, 2H), 4.26 (q, J=7.03 Hz, 2H), 2.18 (d, J=1.00 Hz, 6H), 1.28 (t, J=7.03 Hz, 3H).

### Intermediate 113C

### 2-Bromo-3,6-dimethylpyrazolo[1,5-a]pyrazin-4(5H)-one

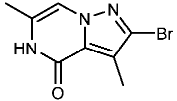

Ammonium acetate (34.7 g, 450 mmol) was added to a stirred solution of Intermediate 113B (6.5 g, 22.48 mmol) in acetic acid (15.0 m). The reaction mixture was stirred at 120°C for 16 hours, diluted with water (100 ml), and the resulting solids were filtered off and dried under vacuum to obtain 4.2 g (77%) of the title compound as off-white solid. LC-MS m/z: 240.1 [M-H]⁺. ¹H NMR (400 MHz, DMSO-*d*₆) δ ppm: 11.28 (br, s, 1H), 7.42 (s, 1H), 2.34 (s, 3H), 2.07 (s, 3H).

### Intermediate 113D

### 2-Bromo-4-methoxy-3,6-dimethylpyrazolo[1,5-a]pyrazine (A2115-429-1)

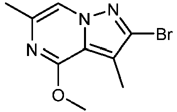

POCl₃ (2.310 ml, 24.79 mmol) was added to the Intermediate 113C (2.0 g, 8.26 mmol). The reaction mixture solution was stirred at 115°C for 2 hours, concentrated to dryness to obtain Intermediate 2-bromo-4-chloro-3,6-dimethylpyrazolo[1,5-a]pyrazine as a brown solid. LC-MS retention time: 1.72 min.; m/z: 259.9 [M+H]⁺. Sodium methoxide in methanol (5.4 M, 7.65 ml, 41.3 mmol) was added to the crude product obtained above in methanol (5 ml) at 0°C, and the reaction mixture was stirred at a room temperature for 16 hours, diluted with water (20 ml) and extracted with EtOAc (2×20 ml). The combined organic layers were washed with brine, dried over sodium sulfate, filtered, and concentrated in vacuum. The crude product was purified using a silica gel column (24 g, Redisep^{®} SiO₂ column, eluting with 14% EtOAc in n-hexane) to afford 1.8 g (85%) of the title compound as a brown solid. LC-MS m/z: 256.0 [M+H]⁺. ¹H NMR (400 MHz, DMSO-*d*₆) δ ppm: 8.12 (s, 1H), 4.01 (s, 3H), 2.43-2.33 (m, 6 H).

### Intermediate 113E

### 2-Bromo-4-methoxy-3-methylpyrazolo[1,5-a]pyrazine-6-carbaldehyde

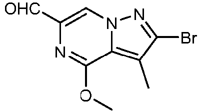

Selenium dioxide (1.430 g, 12.89 mmol) was added to a stirred solution of Intermediate 113D (3.0 g, 11.71 mmol) in 1,4-dioxane (10 ml). The reaction mixture was stirred at 110°C for 16 hours, filtered through a celite bed, and the filtrate was concentrated to obtain 2 g of the crude title compound, which was used in the next step without further purification. LC-MS m/z: 270.0 [M+H]⁺.

### Intermediate 113F

### Methyl-2-bromo-4-methoxy-3-methylpyrazolo [1,5-a]pyrazine-6-carboxylate

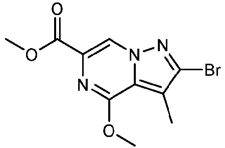

Oxone (5.92 g, 9.63 mmol) was added to a stirred solution of Intermediate 113E (2.0 g, 7.41 mmol) in MeOH (8 ml). The reaction mixture was stirred at 70°C for 6 hours, concentrated under reduced pressure. The residue thus obtained was diluted with water (10 ml), and extracted with EtOAc (2× 20 ml). The combined organic layers were washed with brine, dried over sodium sulfate, filtered, and the filtrate was concentrated in vacuum. The crude product thus obtained was purified using a silica gel column (24 g Redisep^{®} SiO₂ column, eluting with 14% EtOAc in n-hexane) to afford 0.32 g (14%) of the title compound as off-white solid. LC-MS m/z: 300 [M+H]⁺. ¹H NMR (400 MHz, DMSO-*d*₆) δ ppm: 8.90 (s, 1 H), 4.09 (s, 3 H), 3.88 (s, 3 H), 2.34 (s, 3H).

### Intermediate 113G

### Methyl-2-(1-(tert-butoxycarbonyl)-1H-indol-2-yl)-4-methoxy-3-methylpyrazolo[1,5-a]pyrazine-6-carboxylate

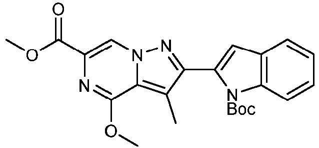

(1-(*tert*-Butoxycarbonyl)-1H-indol-2-yl)boronic acid (239 mg, 0.916 mmol) and potassium phosphate tribasic in water (2M, 1.041 ml, 2.083 mmol) were added to a stirred solution of Intermediate 113F (250 mg, 0.833 mmol) in 1,4-dioxane (4 ml). The solution was degassed with argon and Pd(Ph₃)₄ (67.4 mg, 0.058 mmol) was added to this solution. The reaction mixture was stirred at 100°C for 10 hours, diluted with water (10 ml), and extracted with 10% MeOH in DCM (2×30 ml). The combined organic layers were washed with brine, dried over sodium sulfate, filtered, and concentrated in vacuum to afford the title compound (280 mg, crude). The crude product was used in the next step without further purification. LC-MS m/z: 437.4 [M+H]⁺. ¹H NMR (400 MHz, DMSO-*d*₆) δ ppm: 9.01(s, 1 H), 7.65-7.01 (m, 5H), 4.13 (s, 3H), 3.90 (s, 3 H), 2.70 (s, 3 H), 1.24 (s, 9H).

### Intermediate 113H

### Methyl-2-(1H-indol-2-yl)-4-methoxy-3-methylpyrazolo[1,5-a]pyrazine-6-carboxylate

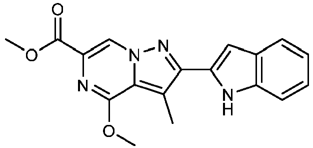

TFA (0.494 ml, 6.42 mmol) was added to a stirred suspension of Intermediate 113G (280mg, 0.642 mmol) in DCM (5 ml). The reaction mixture was stirred at a room temperature for 16 hours, concentrated to remove the excess TFA, and the residue thus obtained was diluted with water (5 ml) and extracted with DCM (2×15 ml). The combined organic layers were washed with saturated NaHCO₃ solution, brine, dried over sodium sulfate, filtered, and the filtrate was concentrated in vacuum to afford 160 mg (56%) of the title compound as a brown solid. LC-MS m/z: 337.2 [M+H]⁺. ¹H NMR (400 MHz, methanol-*d*₄) δ ppm: 8.82 (s, 1H), 7.68-7.53 (m, 4H), 6.92 (s, 1H), 4.21 (s, 3H), 3.98 (s, 3H), 2.72 (s, 3H).

### Intermediate 113I

### 2-(1-(Cyclopropylmethyl)-1H-indol-2-yl)-4-methoxy-3-methylpyrazolo[1,5-a]pyrazine-6-carboxylic acid

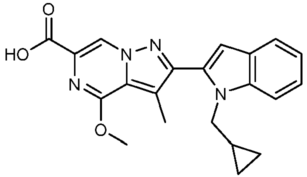

Sodium hydride (23.19 mg, 0.580 mmol) was added to a stirred solution of Intermediate 113H (130 mg, 0.387 mmol) in DMF (1 ml). The reaction mixture was stirred at 0°C for 5 min, followed by the addition of (Bromomethyl)cyclopropane (62.6 mg, 0.464 mmol). The reaction mixture was stirred at a room temperature for 16 hours, diluted with water (5 ml), and extracted with EtOAc (2×10 ml). The combined organic layers were washed with 1N HCl, brine, dried over sodium sulfate, filtered, and the filtrate was concentrated in vacuum to afford the crude title compound (140 mg) as brown solid. LC-MS m/z: 377 [M+H]⁺. ¹H NMR (400 MHz, DMSO-*d*₆) δ ppm: 11.66 (s, 1 H), 8.84 (s, 1 H), 7.71- 7.27 (m, 4 H), 6.85 (s, 1 H), 4.40 (d, J=6.53 Hz, 2 H), 4.14 (s, 3 H), 2.73 (s, 3 H), 0.86 (s, 1 H), 0.25-0.39 (m, 2 H), 0.15 (q, J=4.69 Hz, 2 H).

### Intermediate 113J

### tert-Butyl-((7R)-2-(2-(1-(cyclopropylmethyl)-1H-indol-2-yl)-4-methoxy-3-methylpyrazolo[1,5-a]pyrazine-6-carbonyl)-2-azabicyclo[2.2.1]heptan-7-yl)carbamate

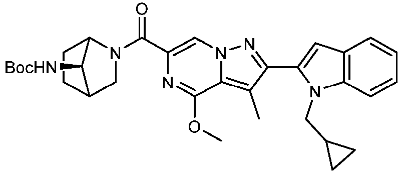

HATU (106 mg, 0.279 mmol), *tert*-butyl ((7R)-2-azabicyclo[2.2.1]heptan-7-yl)carbamate (47.4 mg, 0.223 mmol) and DIPEA (0.065 ml, 0.372 mmol) were added to a stirred solution of Intermediate 113I (70 mg, 0.186 mmol) in DMF (1 ml). The reaction mixture was stirred at a room temperature for 2 hours, diluted with water (5 ml), and extracted with EtOAc (2×10 ml). The combined organic layers were washed with brine, dried over sodium sulfate, filtered, and the filtrate was concentrated in vacuum to afford the crude title compound (70 mg). LC-MS m/z: 571.3 [M+H]⁺. ¹H NMR (400 MHz, DMSO-*d*₆) δ ppm: 8.82 (dd, J=4.27, 1.25 Hz, 1H), 8.67-8.60(m, 1H), 7.61-7.57 (m, 2H), 7.21-7.06 (m, 2H), 6.87 (s, 1H), 4.46 -4.36 (m, 3H), 4.16-4.08 (m, 3 H), 3.58-3.48 (m, 1H), 3.39-3.35 (m, 1 H), 3.29-3.21 (m, 2 H), 2.72-2.68 (m, 1H), 2.58-2.55 (m, 2H), 2.46-2.42 (m, 2H), 1.98-1.82 (m, 1H), 1.31-1.48 (m, 9H), 0.92-0.86 (m, 1H), 0.50 (dd, J=8.03, 1.51 Hz, 2H), 0.13-0.33 (m, 2H).

### Example 113

TFA (0.094 ml, 1.227 mmol) was added to a stirred solution of Intermediate 113J (70 mg, 0.123 mmol) in DCM (3 ml). The resulting solution was stirred at a room temperature for 16 hours, concentrated, and the crude material thus obtained was purified using preparative LC-MS. The purified product was further purified using RP-HPLC to afford 14.7 mg (25%) of the title compound. LC-MS m/z: 471.3 [M+H]⁺. LC-MS retention time: 2.067 min (Method G), 1.780 min (Method F). ¹H NMR (400 MHz, DMSO-*d*₆) δ ppm: 8.72-8.56 (m, 1H), 7.62 (t, J=7.5 Hz, 2H), 7.22 (t, J=7.6 Hz, 1H), 7.13-7.04 (m, 1H), 6.83 (s, 1H), 4.57 (br, s, 1H), 4.39 (br d, J=6.6 Hz, 2H), 4.16-4.04 (m, 3H), 3.90 (s, 2H), 3.63-3.51 (m, 1H), 3.19 (br, s, 1H), 3.10 (br, d, J = 11.7 Hz, 1H), 2.54 (s, 3H), 2.26-2.04 (m,2H), 2.01-1.84 (m, 2H), 1.46-1.34 (m, 1H), 1.15-1.02 (m, 1H), 0.29 (br, d, J = 8.1 Hz, 2H), 0.14 (br, d, J = 4.6 Hz, 2H).

### Example 114

### ((3R,5R)-3-Amino-5-fluoropiperidin-1-yl)(2-(1-(cyclopropylmethyl)-1H-indol-2-yl)-4-methoxy-3-methylpyrazolo[1,5-a]pyrazin-6-yl)methanone

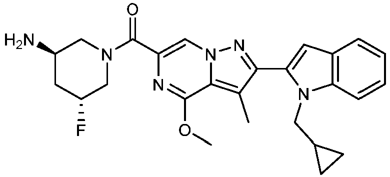

Compound of Example 114 was prepared following a procedure similar to the preparation of a compound of Example 113, by coupling Intermediate 113I with the appropriate amine. LC-MS m/z: 477.3 [M+H]⁺, LC-MS retention time: 1.7 min (UPLC).

### Example 115

### ((7R)-7-Amino-2-azabicyclo[2.2.1]heptan-2-yl)(2-(1-(cyclopropylmethyl)-1H-pyrrolo[2,3-b]pyridin-2-yl)-4-methoxy-3-methylpyrazolo[1,5-a]pyrazin-6-yl)methanone

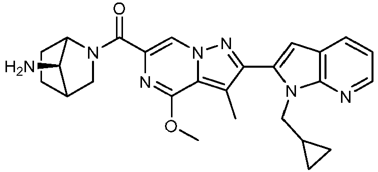

### Intermediate 115A

### Methyl-2-(1-(cyclopropylmethyl)-1H-pyrrolo[2,3-b]pyridin-2-yl)-4-methoxy-3-methylpyrazolo[1,5-a]pyrazine-6-carboxylate

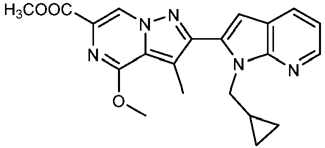

1-(Cyclopropylmethyl)-2-(trimethylstannyl)-1H-pyrrolo[2,3-b]pyridine (Intermediate 7, 167 mg, 0.500 mmol) was added to a stirred solution of Intermediate 113F (150 mg, 0.500 mmol) in 1,4-dioxane (3 ml). The reaction mixture was degassed with argon, followed by the addition of Pd(Ph₃)₄ (57.8 mg, 0.050 mmol). The reaction mixture was stirred at 110°C for 16 hours, diluted with water (5 ml), and extracted with EtOAc (2×10 ml). The combined organic layers were washed with brine, dried over sodium sulfate, filtered and concentrated in vacuum to obtain the crude title compound (200 mg). LC-MS m/z: 392.2 [M+H]⁺.

### Intermediate 115B

### 2-(1-(Cyclopropylmethyl)-1H-pyrrolo[2,3-b]pyridin-2-yl)-4-methoxy-3-methylpyrazolo[1,5-a]pyrazine-6-carboxylic acid

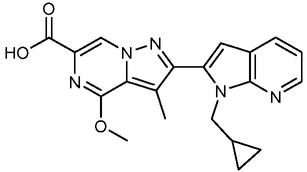

Sodium hydroxide in water (1.277 ml, 2.55 mmol) was added to a stirred solution of Intermediate 115A (200 mg, 0.511 mmol) in THF (3 ml). The resulting solution was stirred at a room temperature for 1 hour, acidified with 1N HCl (3.0 ml), and extracted with EtOAc (2×10 ml). The combined organic layers were washed with brine, dried over sodium sulfate, filtered, and the filtrate was concentrated in vacuum. The compound thus obtained was washed with diethyl ether (5 ml) to remove impurities and dried to afford (110 mg) of the title compound. LC-MS m/z: 378.2 [M+H]⁺ .¹H NMR (400 MHz, DMSO-*d*₆) δ ppm: 13.31-13.14 (m, 1H), 8.90 (s, 1H), 8.40(s, 1H), 7.72-7.14 (m, 2H), 6.89 (s, 1H), 4.62-4.48 (m, 2H), 4.36-4.08 (m,3H), 2.43-2.22 (m, 3H), 1.05-0.97 (m, 1H), 0.57-0.32 (m, 2H), 0.30-0.12 (m, 2H).

### Intermediate 115C

### tert-Butyl-((7R)-2-(2-(1-(cyclopropylmethyl)-1H-pyrrolo[2,3-b]pyridin-2-yl)-4-methoxy-3-methylpyrazolo[1,5-a]pyrazine-6-carbonyl)-2-azabicyclo[2.2.1]heptan-7-yl)carbamate

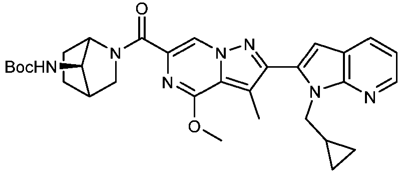

HATU (76 mg, 0.199 mmol), tert-butyl ((7R)-2-azabicyclo[2.2.1]heptan-7-yl)carbamate (33.8 mg, 0.159 mmol) and DIPEA (0.046 ml, 0.265 mmol) were added to a stirred solution of Intermediate 115B (50 mg, 0.132 mmol) in DMF (1 ml). The reaction mixture was stirred at a room temperature for 2 hours, diluted with water (5 ml), and extracted with EtOAc (2×10 ml). The combined organic layers were washed with brine, dried over sodium sulfate, filtered, and the filtrate was concentrated in vacuum. The crude product was purified using a silica gel column (24 g, Redisep^{®} SiO₂ column, eluting with 4% methanol in DCM to afford the title compound (0.050 g, 66%). LC-MS m/z: 572.3 [M+H]⁺. ¹H NMR (400 MHz, DMSO-*d*₆) δ ppm: 8.68 (s, 1H), 8.35-8.33 (m, 1H), 8.09-8.04 (m, 1H), 7.68-7.51 (m, 1H), 7.20-7.14 (m, 1H), 6.89 (s, 1H), 4.53-4.43 (m, 2H), 4.12 (s, 3H), 4.10-4.06 (m, 1H), 3.72 -3.47 (m, 3H), 2.69 (s, 3H), 1.97-1.82 (m, 3H), 1.40-1.29 (m, 9H), 1.26-1.21 (m, 2H), 1.20-1.10 (m, 1H), 0.34-0.16 (m, 4H).

### Example 115

TFA (0.067 ml, 0.875 mmol) was added to a stirred solution of Intermediate 115C (50 mg, 0.087 mmol) in DCM (3 ml). The resulting solution was stirred at a room temperature for 16 hours, concentrated, and the crude product thus obtained was purified using preparative LC-MS. The purified product was further purified using RP-HPLC to afford 17.5 mg (42%) of the title compound. LC-MS m/z: 472.3 [M+H]⁺, retention time: 1.78 min (Method G), 1.338 min (Method F). ¹H NMR (400 MHz, DMSO-*d*₆) δ ppm: 8.65 (s, 1H), 8.34 (dd, *J=*1.5, 4.6 Hz, 1H), 8.06 (dd, *J=*1.5, 7.8 Hz, 1H), 7.17 (dd, *J*=4.6, 7.8 Hz, 1H),6.89 (s, 1H), 4.54-4.44 (m, 3H), 4.20 (s, 1H), 4.16-4.05 (m, 3H), 3.60 (d, *J=*10.3 Hz, 1H), 3.56-3.51 (m, 1H), 3.17 (s, 1H), 3.09 (d, *J*=11.2 Hz, 1H), 2.61-2.55 (m, 3H), 2.24-2.06 (m, 2H), 2.01-1.83 (m, 2H), 1.64-1.34 (m, 1H), 1.25-1.12 (m, 1H), 0.33-0.16 (m, 4H).

Compounds of Examples 116 and 117 in Table 14 were prepared following a procedure similar to the preparation of a compound of Example 115, by coupling of Intermediate 115B with appropriate amine.

**Table 14**

| Example | Structure |
|---|---|
| | Name |
| | Analytical Data: LC-MS m/z [M+H]⁺; HPLC retention time (HPLC method) |
| 116 | 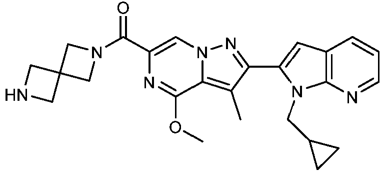 (2-(1-(Cyclopropylmethyl)-1H-pyrrolo[2,3-b]pyridin-2-yl)-4-methoxy-3-methylpyrazolo[1,5-a]pyrazin-6-yl)(2,6-diazaspiro[3.3]heptan-2-yl)methanone |
| | Analytical Data: 472.3, 1.4 min (Method F) |
| 117 | 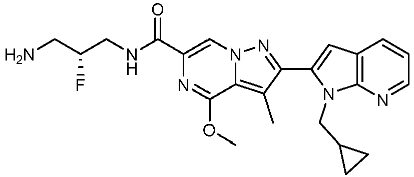 (R)-N-(3-Amino-2-fluoropropyl)-2-(1-(cyclopropylmethyl)-1H-pyrrolo[2,3-b]pyridin-2-yl)-4-methoxy-3 -methylpyrazolo[1, 5-a]pyrazine-6-carboxamide |
| | Analytical Data: 452.3, 1.6 min (Method G) |

Compounds of Examples 205 and 206 in Table 25 were prepared following a procedure similar to the preparation of a compound of Example 3, using Suzuki coupling reaction of Intermediate 2E with appropriate boronic acid or boronate esters.

**Table 25**

| Example | Structure |
|---|---|
| | Name |
| | Analytical Data: LC-MS m/z [M+H]⁺; HPLC retention time (HPLC method) |
| 205 | 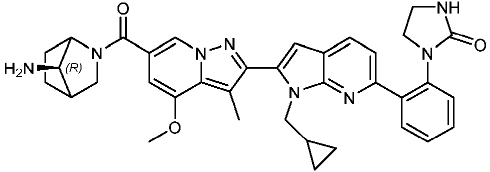 1-[2-(2-{6-[(7R)-7-Amino-2-azabicyclo[2.2.1]heptane-2-carbonyl]-4-methoxy-3-methylpyrazolo[1,5-a]pyridin-2-yl}-1-(cyclopropylmethyl)-1H-pyrrolo[2,3-b]pyridin-6-yl)phenyl]imidazolidin-2-one |
| | Analytical Data: 631.4; 1.568 min (Method E) |
| 206 | 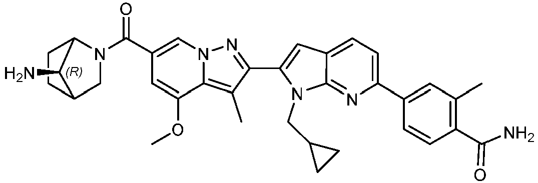 4-(2-{6-[(7R)-7-Amino-2-azabicyclo[2.2.1]heptane-2-carbonyl]-4-methoxy-3-methylpyrazolo[1,5-a]pyridin-2-yl}-1-(cyclopropylmethyl)-1H-pyrrolo[2,3-b]pyridin-6-yl)-2-methylbenzamide |
| | Analytical Data: 604.4; 1.781 min (Method E) |

### Example 207

### 4-(2-{6-[(3R,5R)-3-Amino-5-fluoropiperidine-1-carbonyl]-4-methoxy-3-methylpyrazolo[1,5-a]pyridin-2-yl}-1-(cyclopropylmethyl)-1H-pyrrolo[2,3-b]pyridin-6-yl)-2-fluoro-5-methylbenzamide

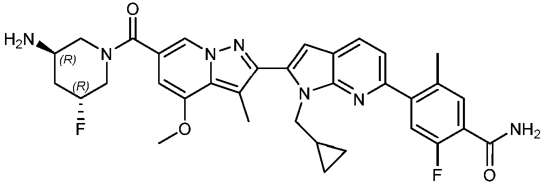

Compound of Example 207 was prepared following a procedure similar to the preparation compound of Example 16, using Intermediate 15A and appropriate boronic acid or boronate ester. LC-MS m/z: 627.3 [M+H]⁺; HPLC retention time: 1.762 min (HPLC method E).

### Example 208

### 4-(2-{6-[(3R)-3-Aminopiperidine-1-carbonyl]-4-methoxy-3-methylpyrazolo[1,5-a]pyridin-2-yl }-1-(cyclopropylmethyl)- 1H-pyrrolo[2,3-b]pyridin-6-yl)-2-fluoro-5-methylbenzamide

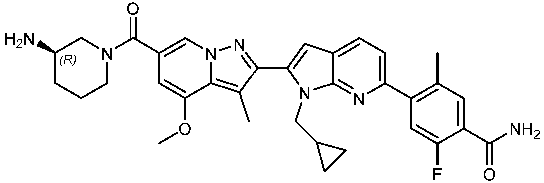

Compound of Example 208 was prepared following a procedure similar to the preparation of compound of Example 3, using Intermediate 2D and tert-butyl (R)-piperidin-3-ylcarbamate in amide formation reaction and the appropriate boronic acid or boronate ester in Suzuki coupling reaction. LC-MS m/z: 609.3 [M+H]⁺; HPLC retention time: 1.710 min (HPLC Method E).

Compounds of Examples 209 to 226 in Table 26 were prepared following a procedure similar to the preparation of a compound of Example 29, by Suzuki coupling of Intermediate 29A with appropriate boronic acid or boronate ester.

**Table 26**

| Example | Structure |
|---|---|
| | Name |
| | Analytical Data: LC-MS m/z [M+H]⁺; HPLC retention time (HPLC method 1) |
| 209 | 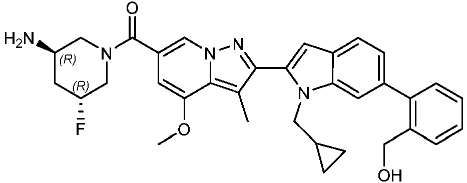 [2-(2-{6-[(3R,5R)-3-Amino-5-fluoropiperidine-1-carbonyl]-4-methoxy-3-methylpyrazolo[1,5-a]pyridin-2-yl}-1-(cyclopropylmethyl)-1H-indol-6-yl)phenyl]methanol |
| | Analytical Data: 582.3; 1.913 min (Method E) |
| 210 | 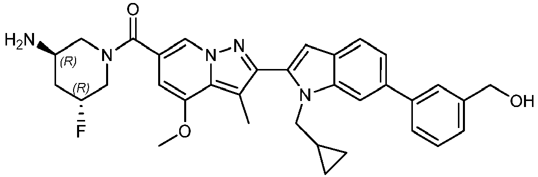 [3-(2-{6-[(3R,5R)-3-Amino-5-fluoropiperidine-1-carbonyl]-4-methoxy-3-methylpyrazolo[1,5-a]pyridin-2-yl}-1-(cyclopropylmethyl)-1H-indol-6-yl)phenyl]methanol |
| | Analytical Data: 582.3; 1.856 min (Method E) |
| 211 | 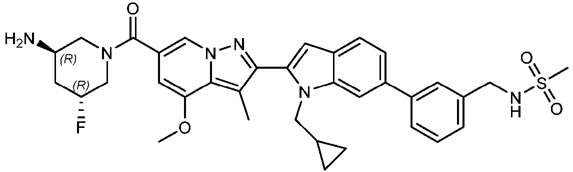 N-{[3-(2-{6-[(3R,5R)-3-Amino-5-fluoropiperidine-1-carbonyl]-4-methoxy-3-methylpyrazolo[1,5-a]pyridin-2-yl}-1-(cyclopropylmethyl)-1H-indol-6-yl)phenyl]methyl}methanesulfonamide |
| | Analytical Data: 659.3; 1.878 min (Method E) |
| 212 | 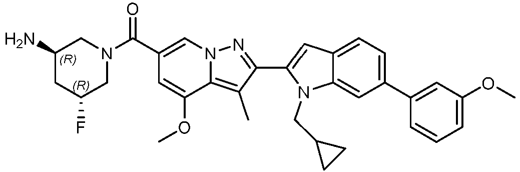 (3R,5R)-1-{2-[1-(Cyclopropylmethyl)-6-(3-methoxyphenyl)-1H-indol-2-yl]-4-methoxy-3-methylpyrazolo[1,5-a]pyridine-6-carbonyl}-5-fluoropiperidin-3-amine |
| | Analytical Data: 582.3; 2.275 min (Method E) |
| 213 | 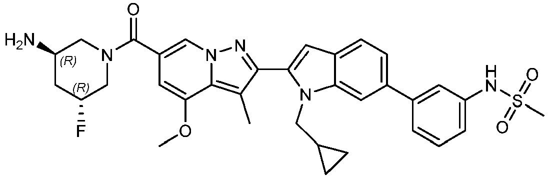 N-[3-(2-{6-[(3R,5R)-3-Amino-5-fluoropiperidine-1-carbonyl]-4-methoxy-3-methylpyrazolo[1,5-a]pyridin-2-yl}-1-(cyclopropylmethyl)-1H-indol-6-yl)phenyl]methanesulfonamide |
| | Analytical Data: 645.3, 1.871 min (Method E) |
| 214 | 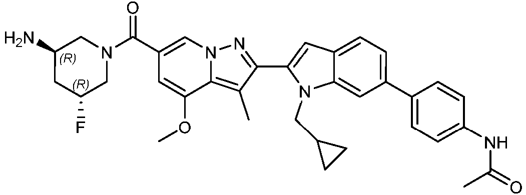 N-[4-(2-{6-[(3R,5R)-3-Amino-5-fluoropiperidine-1-carbonyl]-4-methoxy-3-methylpyrazolo[1,5-a]pyridin-2-yl}-1-(cyclopropylmethyl)-1H-indol-6-yl)phenyl]acetamide |
| | Analytical Data: 609.3; 1.772 min (Method E) |
| 215 | 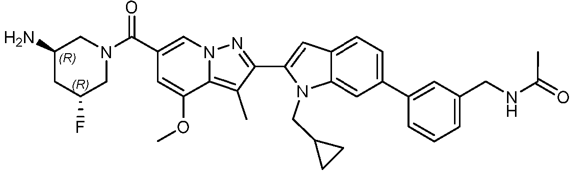 N-{[3-(2-{6-[(3R,5R)-3-Amino-5-fluoropiperidine-1-carbonyl]-4-methoxy-3-methylpyrazolo[1,5-a]pyridin-2-yl}-1-(cyclopropylmethyl)-1H-indol-6-yl)phenyl]methyl}acetamide |
| | Analytical Data: 623.2.3; 1.782 min (Method E) |
| 216 | 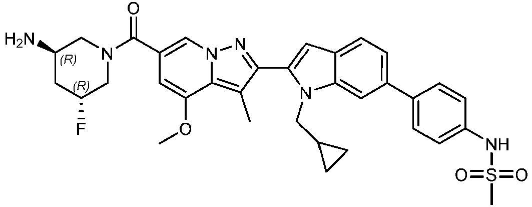 N-[4-(2-{6-[(3R,5R)-3-Amino-5-fluoropiperidine-1-carbonyl]-4-methoxy-3-methylpyrazolo[1,5-a]pyridin-2-yl}-1-(cyclopropylmethyl)-1H-indol-6-yl)phenyl]methanesulfonamide |
| | Analytical Data: 645.2; 1.828 min (Method E) |
| 217 | 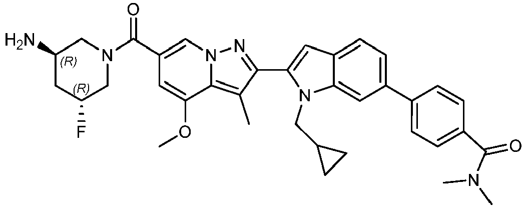 4-(2-{6-[(3R,5R)-3-Amino-5-fluoropiperidine-1-carbonyl]-4-methoxy-3-methylpyrazolo[1,5-a]pyridin-2-yl}-1-(cyclopropylmethyl)-1H-indol-6-yl)-N,N-dimethylbenzamide |
| | Analytical Data: 623.3; 1.864 min (Method E) |
| 218 | 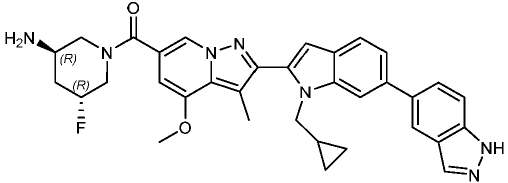 (3R,5R)-1-{2-[1-(Cyclopropylmethyl)-6-(1H-indazol-5-yl)-1H-indol-2-yl]-4-methoxy-3-methylpyrazolo[1,5-a]pyridine-6-carbonyl}-5-fluoropiperidin-3-amine |
| | Analytical Data: 592.3; 1.819 min (Method E) |
| 219 | 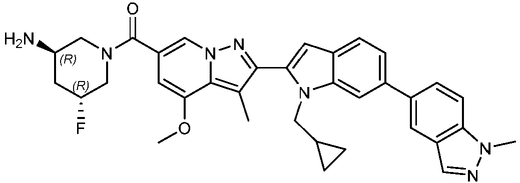 (3R,5R)-1-{2-[1-(Cyclopropylmethyl)-6-(1-methyl-1H-indazol-5-yl)-1H-indol-2-yl]-4-methoxy-3-methylpyrazolo[1,5-a]pyridine-6-carbonyl}-5-fluoropiperidin-3-amine |
| | Analytical Data: 606.3; 2.054 min (Method E) |
| 220 | 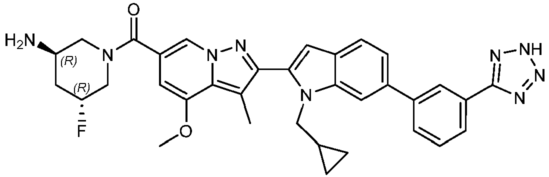 |
| | (3R,5R)-1-{2-[1-(Cyclopropylmethyl)-6-[3-(2H-1,2,3,4-tetrazol-5-yl)phenyl]-1H-indol-2-yl]-4-methoxy-3-methylpyrazolo[1,5-a]pyridine-6-carbonyl}-5-fluoropiperidin-3-amine |
| | Analytical Data: 620.3; 1.455 min (Method E) |
| 221 | 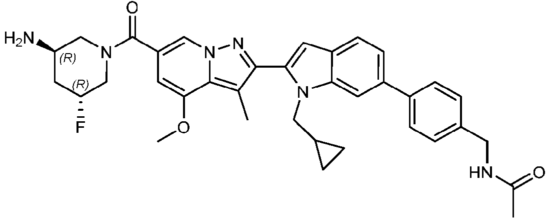 N-{[4-(2-{6-[(3R,5R)-3-Amino-5-fluoropiperidine-1-carbonyl]-4-methoxy-3-methylpyrazolo[1,5-a]pyridin-2-yl}-1-(cyclopropylmethyl)-1H-indol-6-yl)phenyl]methyl}acetamide |
| | Analytical Data: 623.3; 1.742 min (Method E) |
| 222 | 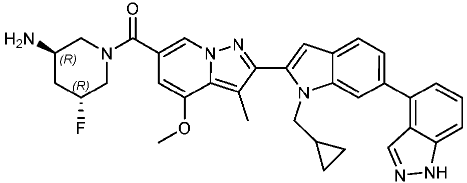 (3R,5R)-1-{2-[1-(Cyclopropylmethyl)-6-(1H-indazol-4-yl)-1H-indol-2-yl]-4-methoxy-3-methylpyrazolo[1,5-a]pyridine-6-carbonyl}-5-fluoropiperidin-3-amine |
| | Analytical Data: 592.3; 1.876 min (Method E) |
| 223 | 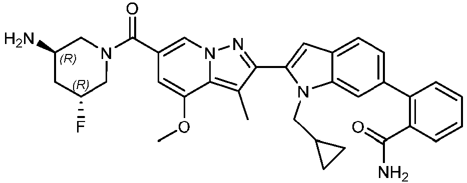 2-(2-{6-[(3R,5R)-3-Amino-5-fluoropiperidine-1-carbonyl]-4-methoxy-3-methylpyrazolo[1,5-a]pyridin-2-yl}-1-(cyclopropylmethyl)-1H-indol-6-yl)benzamide |
| | Analytical Data: 595.3; 1.653 min (Method E) |
| 224 | 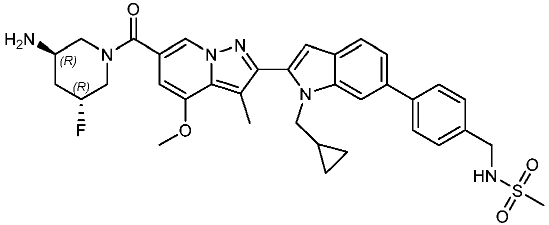 N-{[4-(2-{6-[(3R,5R)-3-Amino-5-fluoropiperidine-1-carbonyl]-4-methoxy-3-methylpyrazolo[1,5-a]pyridin-2-yl}-1-(cyclopropylmethyl)-1H-indol-6-yl)phenyl]methyl}methanesulfonamide |
| | Analytical Data: 659.2; 1.865 min (Method E) |
| 225 | 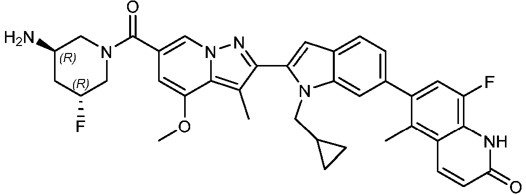 6-(2-{6-[(3R,5R)-3-Amino-5-fluoropiperidine-1-carbonyl]-4-methoxy-3-methylpyrazolo[1,5-a]pyridin-2-yl}-1-(cyclopropylmethyl)-1H-indol-6-yl)-8-fluoro-5-methyl-1,2-dihydroquinolin-2-one |
| | Analytical Data: 651.3, 1.807 min (Method E) |
| 226 | 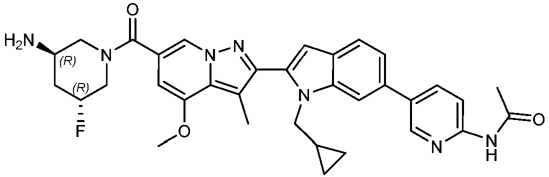 N-[5-(2-{6-[(3R,5R)-3-Amino-5-fluoropiperidine-1-carbonyl]-4-methoxy-3-methylpyrazolo[1,5-a]pyridin-2-yl}-1-(cyclopropylmethyl)-1H-indol-6-yl)pyridin-2-yl]acetamide |
| | Analytical Data: 610.3; 1.721 min (Method E) |

Compounds of Examples 227 to 240 in Table 27 were prepared following a procedure similar to the preparation of a compound of Example 29, using the Intermediate 23D and *tert*-butyl (R)-piperidin-3-ylcarbamate in amide formation and the appropriate boronic acid/boronate ester in Suzuki coupling reaction.

**Table 27**

| Example | Structure |
|---|---|
| | Name |
| | Analytical Data: LC-MS m/z [M+H]⁺; HPLC retention time (HPLC method 1) |
| 227 | 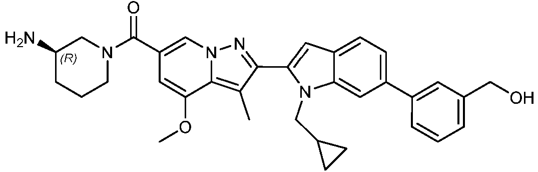 [3-(2-{6-[(3R)-3-Aminopiperidine-1-carbonyl]-4-methoxy-3-methylpyrazolo[1,5-a]pyridin-2-yl}-1-(cyclopropylmethyl)-1H-indol-6-yl)phenyl]methanol |
| | Analytical Data: 564.3; 1.777 min (Method E) |
| 228 | 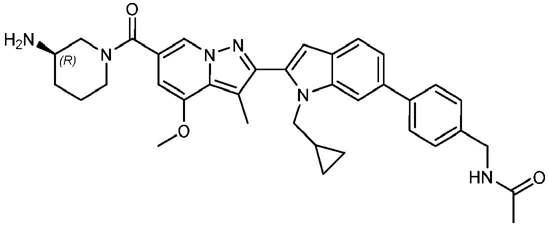 N-{[4-(2-{6-[(3R)-3-Aminopiperidine-1-carbonyl]-4-methoxy-3-methylpyrazolo[1,5-a]pyridin-2-yl}-1-(cyclopropylmethyl)-1H-indol-6-yl)phenyl]methyl}acetamide |
| | Analytical Data: 605.3; 1.602 min (Method E) |
| 229 | 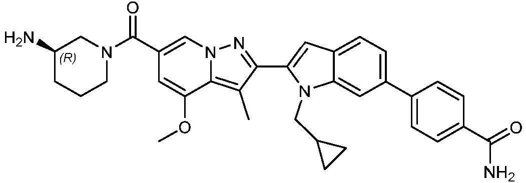 4-(2-{6-[(3R)-3-Aminopiperidine-1-carbonyl]-4-methoxy-3-methylpyrazolo[1,5-a]pyridin-2-yl}-1-(cyclopropylmethyl)-1H-indol-6-yl)benzamide |
| | Analytical Data: 577.3; 1.557 min (Method E) |
| 230 | 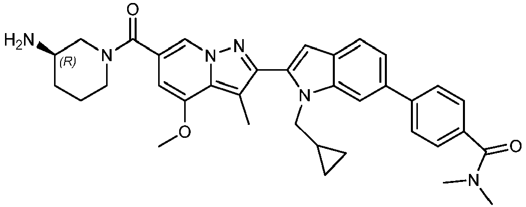 4-(2-{6-[(3R)-3-Aminopiperidine-1-carbonyl]-4-methoxy-3-methylpyrazolo[1,5-a]pyridin-2-yl}-1-(cyclopropylmethyl)-1H-indol-6-yl)-N,N-dimethylbenzamide |
| | Analytical Data: 605.3; 1.771 min (Method E) |
| 231 | 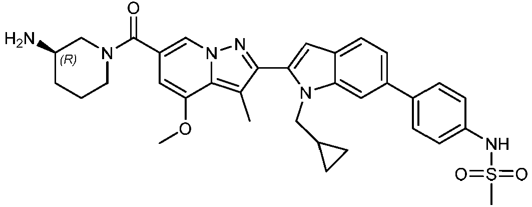 N-[4-(2-{6-[(3R)-3-Aminopiperidine-1-carbonyl]-4-methoxy-3-methylpyrazolo[1,5-a]pyridin-2-yl}-1-(cyclopropylmethyl)-1H-indol-6-yl)phenyl]methanesulfonamide |
| | Analytical Data: 625.3; 1.743min (Method E) |
| 232 | 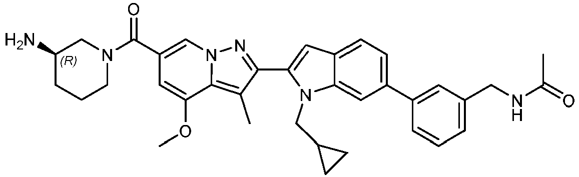 N-{[3-(2-{6-[(3R)-3-Aminopiperidine-1-carbonyl]-4-methoxy-3-methylpyrazolo[1,5-a]pyridin-2-yl}-1-(cyclopropylmethyl)-1H-indol-6-yl)phenyl]methyl}acetamide |
| | Analytical Data: 605.3, 1.699 min (Method E) |
| 233 | 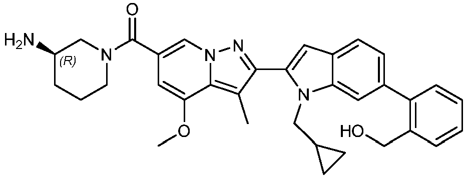 |
| | [2-(2-{6-[(3R)-3-Aminopiperidine-1-carbonyl]-4-methoxy-3-methylpyrazolo[1,5-a]pyridin-2-yl}-1-(cyclopropylmethyl)-1H-indol-6-yl)phenyl]methanol |
| | Analytical Data: 564.3; 1.831 min (Method E) |
| 234 | 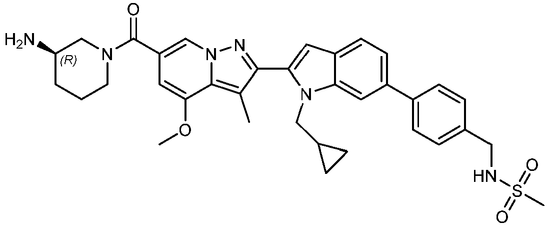 N-{[4-(2-{6-[(3R)-3-Aminopiperidine-1-carbonyl]-4-methoxy-3-methylpyrazolo[1,5-a]pyridin-2-yl}-1-(cyclopropylmethyl)-1H-indol-6-yl)phenyl]methyl}methanesulfonamide |
| | Analytical Data: 641.2; 1.763 min (Method E) |
| 235 | 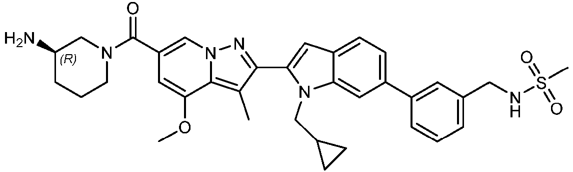 N-{[3-(2-{6-[(3R)-3-Aminopiperidine-1-carbonyl]-4-methoxy-3-methylpyrazolo[1,5-a]pyridin-2-yl}-1-(cyclopropylmethyl)-1H-indol-6-yl)phenyl]methyl} methanesulfonamide |
| | Analytical Data: 641.3; 1.802 min (Method E) |
| 236 | 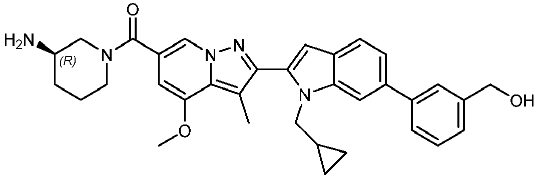 [3-(2-{6-[(3R)-3-Aminopiperidine-1-carbonyl]-4-methoxy-3-methylpyrazolo[1,5-a]pyridin-2-yl}-1-(cyclopropylmethyl)-1H-indol-6-yl)phenyl]methanol |
| | Analytical Data: 564.2; 1.738 min (Method E) |
| 237 | 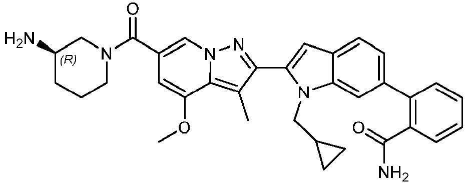 2-(2-{6-[(3R)-3-Aminopiperidine-1-carbonyl]-4-methoxy-3-methylpyrazolo[1,5-a]pyridin-2-yl}-1-(cyclopropylmethyl)-1H-indol-6-yl)benzamide |
| | Analytical Data: 577.3; 1.573 min (Method E) |
| 238 | 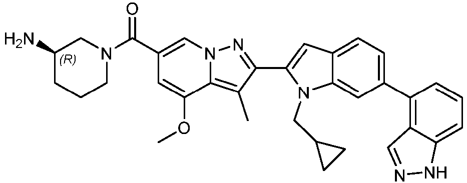 (3R)-1-{2-[1-(Cyclopropylmethyl)-6-(1H-indazol-4-yl)-1H-indol-2-yl]-4-methoxy-3-methylpyrazolo[1,5-a]pyridine-6-carbonyl}piperidin-3-amine |
| | Analytical Data: 574.3; 1.703 min (Method E) |
| 239 | 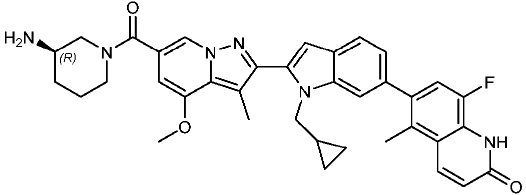 6-(2-{6-[(3R)-3-Aminopiperidine-1-carbonyl]-4-methoxy-3-methylpyrazolo[1,5-a]pyridin-2-yl}-1-(cyclopropylmethyl)-1H-indol-6-yl)-8-fluoro-5-methyl-1,2-dihydroquinolin-2-one |
| | Analytical Data: 633.3; 1.753 min (Method E) |
| 240 | 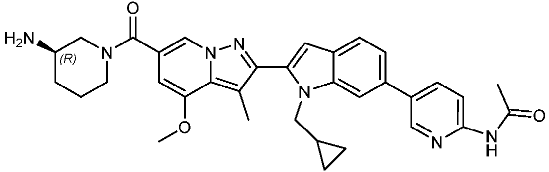 |
| | N-[5-(2-{6-[(3R)-3-Aminopiperidine-1-carbonyl]-4-methoxy-3-methylpyrazolo[1,5-a]pyridin-2-yl}-1-(cyclopropylmethyl)-1H-indol-6-yl)pyridin-2-yl]acetamide |
| | Analytical Data: 592.3; 1.640 min (Method E) |

Compounds of Examples 241 to 247 in Table 28 were prepared following a procedure similar to the preparation of a compound of Example 33, by Buchwald coupling of Intermediate 29A with appropriate amine.

**Table 28**

| Example | Structure |
|---|---|
| | Name |
| | Analytical Data: LC-MS m/z [M+H]⁺; HPLC retention time (HPLC method) |
| 241 | 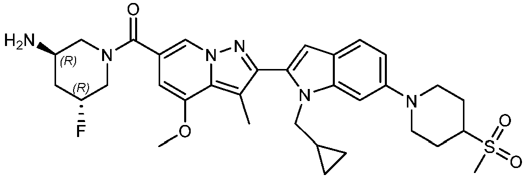 (3R,5R)-1-{2-[1-(Cyclopropylmethyl)-6-(4-methanesulfonylpiperidin-1-yl)-1H-indol-2-yl]-4-methoxy-3-methylpyrazolo[1,5-a]pyridine-6-carbonyl}-5-fluoropiperidin-3-amine |
| | Analytical Data: 637.2; 1.588 min (Method E) |
| 242 | 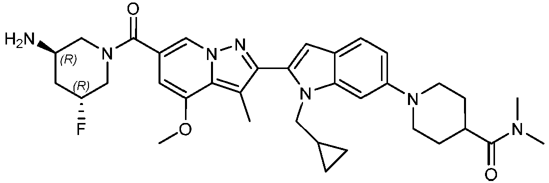 1-(2-{6-[(3R,5R)-3-Amino-5-fluoropiperidine-1-carbonyl]-4-methoxy-3-methylpyrazolo[1,5-a]pyridin-2-yl}-1-(cyclopropylmethyl)-1H-indol-6-yl)-N,N-dimethylpiperidine-4-carboxamide |
| | Analytical Data: 630.4; 1.634 min (Method E) |
| 243 | 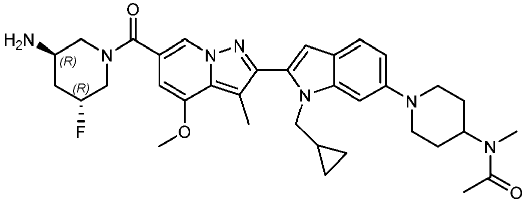 N-[1-(2-{6-[(3R,5R)-3-Amino-5-fluoropiperidine-1-carbonyl]-4-methoxy-3-methylpyrazolo[1,5-a]pyridin-2-yl}-1-(cyclopropylmethyl)-1H-indol-6-yl)piperidin-4-yl]-N-methylacetamide |
| | Analytical Data: 630.4; 1.423min (Method E) |
| 244 | 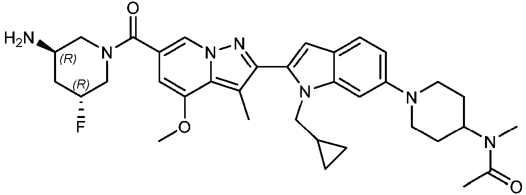 N-[1-(2-{6-[(3R,5R)-3-Amino-5-fluoropiperidine-1-carbonyl]-4-methoxy-3-methylpyrazolo[1,5-a]pyridin-2-yl}-1-(cyclopropylmethyl)-1H-indol-6-yl)piperidin-4-yl]-N-methylacetamide |
| | Analytical Data: 630.4; 1.617 min (Method E) |
| 245 | 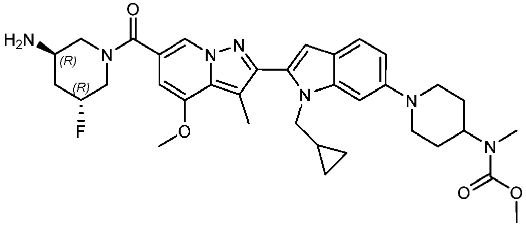 Methyl N-[1-(2-{6-[(3R,5R)-3-amino-5-fluoropiperidine-1-carbonyl]-4-methoxy-3-methylpyrazolo[1,5-a]pyridin-2-yl }-1-(cyclopropylmethyl)-1H-indol-6-yl)piperidin-4-yl]-N-methylcarbamate |
| | Analytical Data: 646.3; 1.895min (Method E) |
| 246 | 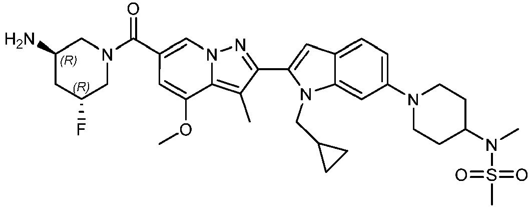 N-[1-(2-{6-[(3R,5R)-3-Amino-5-fluoropiperidine-1-carbonyl]-4-methoxy-3-methylpyrazolo[1,5-a]pyridin-2-yl}-1-(cyclopropylmethyl)-1H-indol-6-yl)piperidin-4-yl]-N-methylmethanesulfonamide |
| | Analytical Data: 666.3; 1.733 min (Method E) |
| 247 | 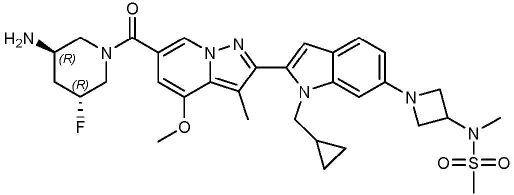 N-[1-(2-{6-[(3R,5R)-3-Amino-5-fluoropiperidine-1-carbonyl]-4-methoxy-3-methylpyrazolo[1,5-a]pyridin-2-yl}-1-(cyclopropylmethyl)-1H-indol-6-yl)azetidin-3-yl]-N-methylmethanesulfonamide |
| | Analytical Data: 638.3; 1.670 min (Method E) |

Compounds of Examples 248 to 256 in Table 29 were prepared following a procedure similar to the preparation of a compound of Example 33, by using Intermediate 23D and *tert*-butyl (R)-piperidin-3-ylcarbamate for amide formation and appropriate secondary amine in the Buchwald coupling reaction.

**Table 29**

| Example | Structure |
|---|---|
| | Name |
| | Analytical Data: LC-MS m/z [M+H]⁺; HPLC retention time (HPLC method) |
| 248 | 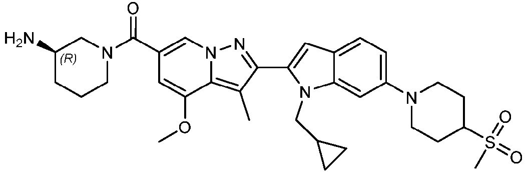 |
| | (3R)-1-{2-[1-(Cyclopropylmethyl)-6-(4-methanesulfonylpiperidin-1-yl)-1H-indol-2-yl]-4-methoxy-3-methylpyrazolo[1,5-a]pyridine-6-carbonyl }piperidin-3-amine |
| | Analytical Data: 619.3; 1.530 min (Method E) |
| 249 | 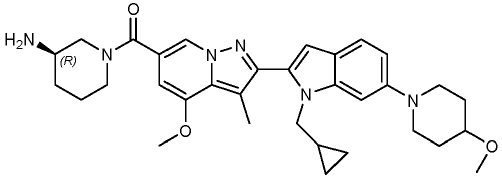 |
| | (3R)-1-{2-[1-(Cyclopropylmethyl)-6-(4-methoxypiperidin-1-yl)-1H-indol-2-yl]-4-methoxy-3-methylpyrazolo[1,5-a]pyridine-6-carbonyl}piperidin-3-amine |
| | Analytical Data: 571.3, 1.837 min (Method E) |
| 250 | 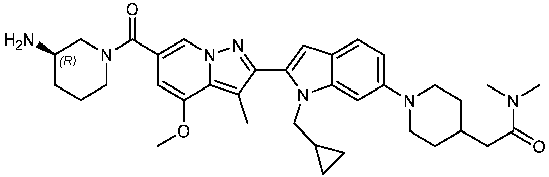 |
| | 2-[1-(2-{6-[(3R)-3-Aminopiperidine-1-carbonyl]-4-methoxy-3-methylpyrazolo[1,5-a]pyridin-2-yl}-1-(cyclopropylmethyl)-1H-indol-6-yl)piperidin-4-yl]-N,N-dimethylacetamide |
| | Analytical Data: 626.3; 1.647 min (Method E) |
| 251 | 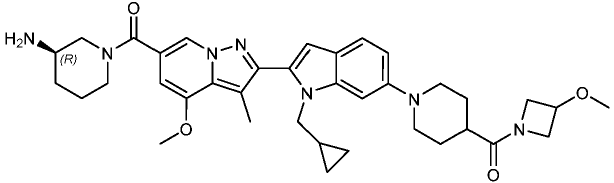 |
| | (3R)-1-{2-[1-(Cyclopropylmethyl)-6-[4-(3-methoxyazetidine-1-carbonyl)piperidin-1-yl]-1H-indol-2-yl]-4-methoxy-3-methylpyrazolo[1,5-a]pyridine-6-carbonyl}piperidin-3-amine |
| | Analytical Data: 654.4; 1.565 min (Method E) |
| 252 | 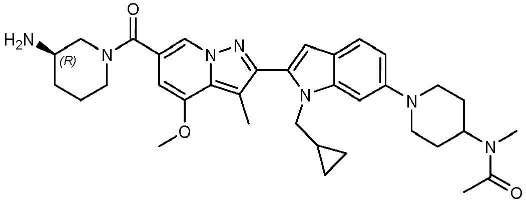 |
| | N-[1-(2-{6-[(3R)-3-Aminopiperidine-1-carbonyl]-4-methoxy-3-methylpyrazolo[1,5-a]pyridin-2-yl}-1-(cyclopropylmethyl)-1H-indol-6-yl)piperidin-4-yl]-N-methylacetamide |
| | Analytical Data: 612.4; 1.571 min (Method E) |
| 253 | 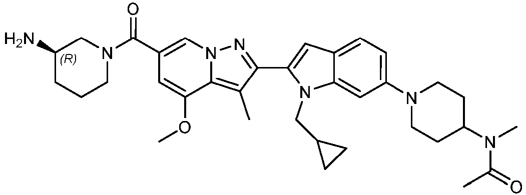 |
| | N-[1-(2-{6-[(3R)-3-Aminopiperidine-1-carbonyl]-4-methoxy-3-methylpyrazolo[1,5-a]pyridin-2-yl}-1-(cyclopropylmethyl)-1H-indol-6-yl)piperidin-4-yl]-N-methylacetamide |
| | Analytical Data: 612.4; 1.566 min (Method E) |
| 254 | 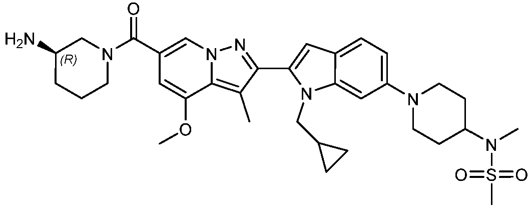 |
| | N-[1-(2-{6-[(3R)-3-Aminopiperidine-1-carbonyl]-4-methoxy-3-methylpyrazolo[1,5-a]pyridin-2-yl}-1-(cyclopropylmethyl)-1H-indol-6-yl)piperidin-4-yl]-N-methylmethanesulfonamide |
| | Analytical Data: 648.3; 1.677 min (Method E) |
| 255 | 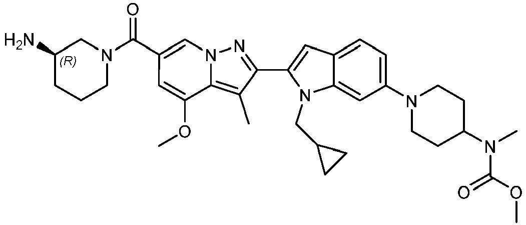 |
| | Methyl N-[1-(2-{6-[(3R)-3-aminopiperidine-1-carbonyl]-4-methoxy-3-methylpyrazolo[1,5-a]pyridin-2-yl }-1-(cyclopropylmethyl)-1H-indol-6-yl)piperidin-4-yl]-N-methylcarbamate |
| | Analytical Data: 628.4; 1.840 min (Method E) |
| 256 | 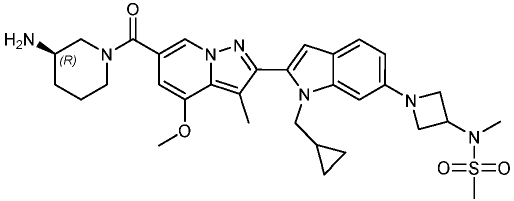 |
| | N-[1-(2-{6-[(3R)-3-Aminopiperidine-1-carbonyl]-4-methoxy-3-methylpyrazolo[1,5-a]pyridin-2-yl}-1-(cyclopropylmethyl)-1H-indol-6-yl)azetidin-3-yl]-N-methylmethanesulfonamide |
| | Analytical Data: 620.3; 1.631 min (Method E) |

Compounds of Examples 257 to 264 in Table 30 were prepared following a procedure similar to the preparation of a compound of Example 40, using Suzuki coupling of Intermediate 39E with appropriate boronic acid or boronate ester.

**Table 30**

| Example | Structure |
|---|---|
| | Name |
| | Analytical Data: LC-MS m/z [M+H]⁺; HPLC retention time (HPLC method) |
| 257 | 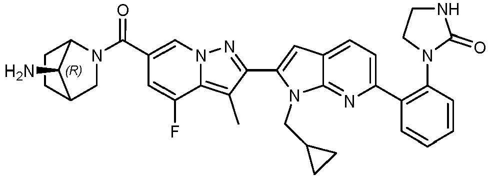 |
| | 1-[2-(2-{6-[(7R)-7-Amino-2-azabicyclo[2.2.1]heptane-2-carbonyl]-4-fluoro-3-methylpyrazolo[1,5-a]pyridin-2-yl}-1-(cyclopropylmethyl)-1H-pyrrolo[2,3-b]pyridin-6-yl)phenyl]imidazolidin-2-one |
| | Analytical Data: 619.2; 1.678 min (Method E) |
| 258 | 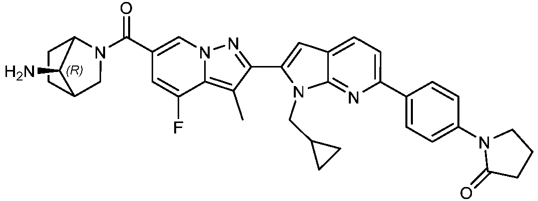 |
| | 1-[4-(2-{6-[(7R)-7-Amino-2-azabicyclo[2.2.1]heptane-2-carbonyl]-4-fluoro-3-methylpyrazolo[1,5-a]pyridin-2-yl}-1-(cyclopropylmethyl)-1H-pyrrolo[2,3-b]pyridin-6-yl)phenyl]pyrrolidin-2-one |
| | Analytical Data: 618.3; 1.984 min (Method E) |
| 259 | 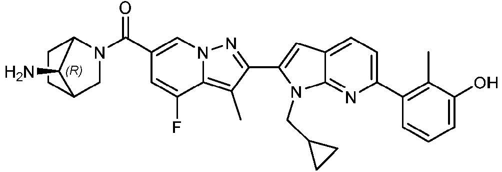 |
| | 3-(2-{6-[(7R)-7-Amino-2-azabicyclo[2.2.1]heptane-2-carbonyl]-4-fluoro-3-methylpyrazolo[1,5-a]pyridin-2-yl}-1-(cyclopropylmethyl)-1H-pyrrolo[2,3-b]pyridin-6-yl)-2-methylphenol |
| | Analytical Data: 565.3; 1.900 min (Method E) |
| 260 | 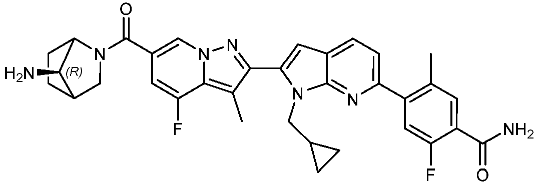 |
| | 4-(2-{6-[(7R)-7-Amino-2-azabicyclo[2.2.1]heptane-2-carbonyl]-4-fluoro-3-methylpyrazolo[1,5-a]pyridin-2-yl}-1-(cyclopropylmethyl)-1H-pyrrolo[2,3-b]pyridin-6-yl)-2-fluoro-5-methylbenzamide |
| | Analytical Data: 610.3; 1.760 min (Method E) |
| 261 | 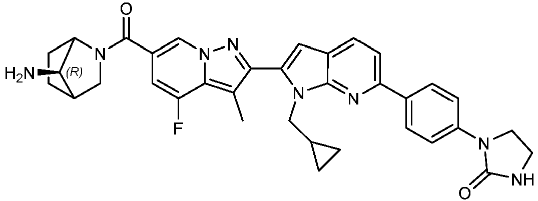 |
| | 1-[4-(2-{6-[(7R)-7-Amino-2-azabicyclo[2.2.1]heptane-2-carbonyl]-4-fluoro-3-methylpyrazolo[1,5-a]pyridin-2-yl}-1-(cyclopropylmethyl)-1H-pyrrolo[2,3-b]pyridin-6-yl)phenyl]imidazolidin-2-one |
| | Analytical Data: 619.4; 1.844 min (Method E) |
| 262 | 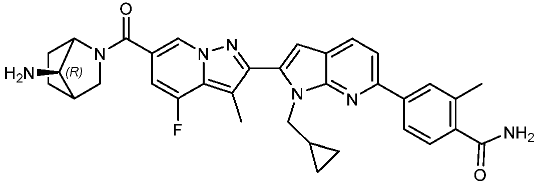 |
| | 4-(2-{6-[(7R)-7-Amino-2-azabicyclo[2.2.1]heptane-2-carbonyl]-4-fluoro-3-methylpyrazolo[1,5-a]pyridin-2-yl}-1-(cyclopropylmethyl)-1H-pyrrolo[2,3-b]pyridin-6-yl)-2-methylbenzamide |
| | Analytical Data: 592.4; 1.734 min (Method E) |
| 263 | 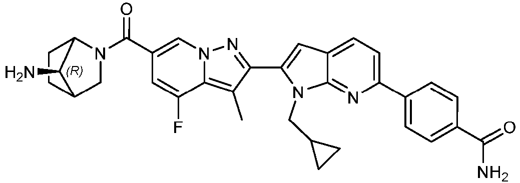 |
| | 4-(2-{6-[(7R)-7-Amino-2-azabicyclo[2.2.1]heptane-2-carbonyl]-4-fluoro-3-methylpyrazolo[1,5-a]pyridin-2-yl}-1-(cyclopropylmethyl)-1H-pyrrolo[2,3-b]pyridin-6-yl)benzamide |
| | Analytical Data: 578.3; 1.682 min (Method E) |
| 264 | 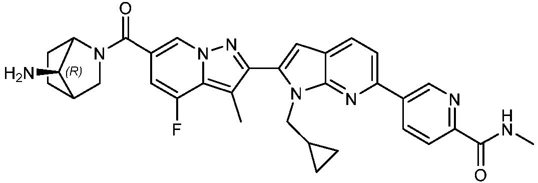 |
| | 5-(2-{6-[(7R)-7-Amino-2-azabicyclo[2.2.1]heptane-2-carbonyl]-4-fluoro-3-methylpyrazolo[1,5-a]pyridin-2-yl}-1-(cyclopropylmethyl)-1H-pyrrolo[2,3-b]pyridin-6-yl)-N-methylpyridine-2-carboxamide |
| | Analytical Data: 593.3; 1.783 min (Method E) |

Compounds of Examples 265 to 268 in Table 31 were prepared following a procedure similar to the preparation of a compound of Example 50, using Intermediate 39E and appropriate secondary amine in Buchwald coupling reaction.

**Table 31**

| Example | Structure |
|---|---|
| | Name |
| | Analytical Data: LC-MS m/z [M+H]⁺; HPLC retention time (HPLC method) |
| 265 | 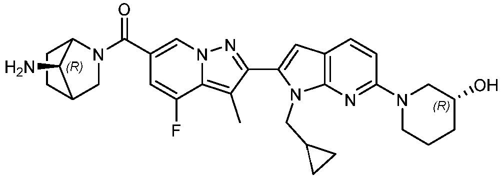 |
| | ((7R)-7-Amino-2-azabicyclo[2.2.1]heptan-2-yl)(2-(1-(cyclopropylmethyl)-6-((R)-3-hydroxypiperidin-1-yl)-1H-pyrrolo[2,3-b]pyridin-2-yl)-4-fluoro-3-methylpyrazolo[1,5-a]pyridin-6-yl)methanone |
| | Analytical Data: 558.4; 5.795 min (Method C) |
| 266 | 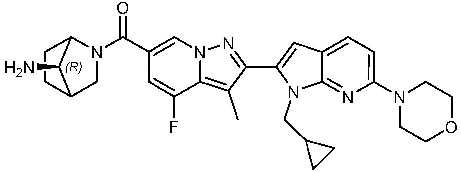 |
| | (7R)-2-{2-[1-(Cyclopropylmethyl)-6-(morpholin-4-yl)-1H-pyrrolo[2,3-b]pyridin-2-yl]-4-fluoro-3-methylpyrazolo[1,5-a]pyridine-6-carbonyl}-2-azabicyclo[2.2.1]heptan-7-amine |
| | Analytical Data: 544.3; 1.668 min (Method E) |
| 267 | 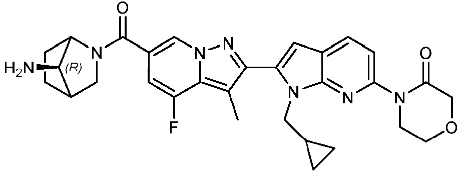 |
| | 4-(2-{6-[(7R)-7-Amino-2-azabicyclo[2.2.1]heptane-2-carbonyl]-4-fluoro-3-methylpyrazolo[1,5-a]pyridin-2-yl}-1-(cyclopropylmethyl)-1H-pyrrolo[2,3-b]pyridin-6-yl)morpholin-3-one |
| | Analytical Data: 558.3; 1.595 min (Method E) |
| 268 | 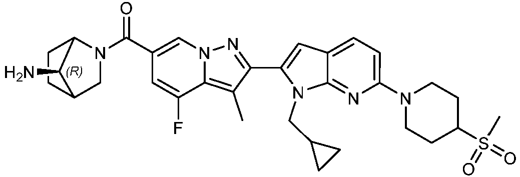 |
| | (7R)-2-{2-[1-(Cyclopropylmethyl)-6-(4-methanesulfonylpiperidin-1-yl)-1H-pyrrolo[2,3-b]pyridin-2-yl]-4-fluoro-3-methylpyrazolo[1,5-a]pyridine-6-carbonyl}-2-azabicyclo[2.2.1]heptan-7-amine |
| | Analytical Data: 620.2; 1.713 min (Method E) |

Compounds of Examples 269 to 273 in Table 32 were prepared following a procedure similar to the preparation of a compound of Example 50, using Intermediate 39D and *tert*-butyl (R)-piperidin-3-ylcarbamate in amide formation and with appropriate secondary amine in Buchwald coupling reaction.

**Table 32**

| Example | Structure |
|---|---|
| | Name |
| | Analytical Data: LC-MS m/z [M+H]⁺; HPLC retention time (HPLC method) |
| 269 | 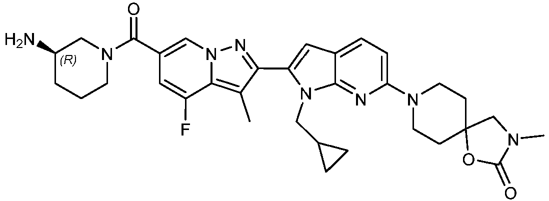 8-(2-{6-[(3R)-3-Aminopiperidine-1-carbonyl]-4-fluoro-3-methylpyrazolo[1,5-a]pyridin-2-yl}-1-(cyclopropylmethyl)-1H-pyrrolo[2,3-b]pyridin-6-yl)-3-methyl-1-oxa-3,8-diazaspiro[4.5]decan-2-one |
| | Analytical Data: 615.3; 1.703 min (Method E) |
| 270 | 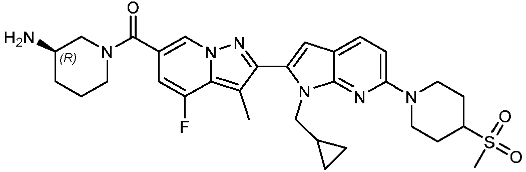 (3R)-1-{2-[1-(Cyclopropylmethyl)-6-(4-methanesulfonylpiperidin-1-yl)-1H-pyrrolo[2,3-b]pyridin-2-yl]-4-fluoro-3-methylpyrazolo[1,5-a]pyridine-6-carbonyl }piperidin-3-amine |
| | Analytical Data: 608.3; 1.558 min (Method E) |
| 271 | 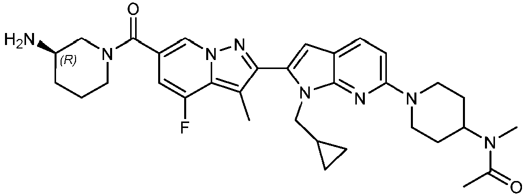 N-[1-(2-{6-[(3R)-3-Aminopiperidine-1-carbonyl]-4-fluoro-3-methylpyrazolo[1,5-a]pyridin-2-yl}-1-(cyclopropylmethyl)-1H-pyrrolo[2,3-b]pyridin-6-yl)piperidin-4-yl]-N-methylacetamide |
| | Analytical Data: 601.3; 1.618 min (Method E) |
| 272 | 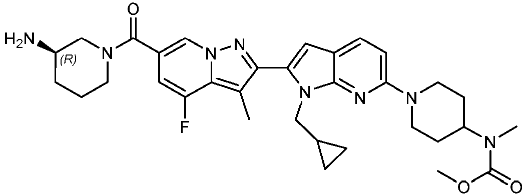 Methyl N-[1-(2-{6-[(3R)-3-aminopiperidine-1-carbonyl]-4-fluoro-3-methylpyrazolo[1,5-a]pyridin-2-yl}-1-(cyclopropylmethyl)-1H-pyrrolo[2,3-b]pyridin-6-yl)piperidin-4-yl]-N-methylcarbamate |
| | Analytical Data: 617.3; 1.905min (Method E) |
| 273 | 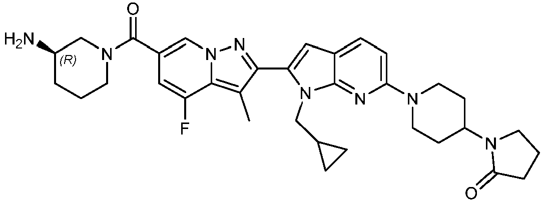 1-[1-(2-{6-[(3R)-3-Aminopiperidine-1-carbonyl]-4-fluoro-3-methylpyrazolo[1,5-a]pyridin-2-yl}-1-(cyclopropylmethyl)-1H-pyrrolo[2,3-b]pyridin-6-yl)piperidin-4-yl]pyrrolidin-2-one |
| | Analytical Data: 613.3; 1.640 min (Method E) |

Compounds of Examples 274 and 275 in Table 33 were prepared following a procedure similar to the preparation of a compound of Example 50, using Intermediate 39E with appropriate primary alcohol in Buchwald coupling reaction.

**Table 33**

| Example | Structure |
|---|---|
| | Name |
| | Analytical Data: LC-MS m/z [M+H]⁺; HPLC retention time (HPLC method) |
| 274 | 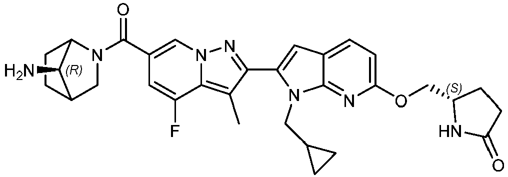 (5S)-5-{[(2-{6-[(7R)-7-Amino-2-azabicyclo[2.2.1]heptane-2-carbonyl]-4-fluoro-3-methylpyrazolo[1,5-a]pyridin-2-yl}-1-(cyclopropylmethyl)-1H-pyrrolo[2,3-b]pyridin-6-yl)oxy]methyl } pyrrolidin-2-one |
| | Analytical Data: 572.2; 1.453 min (Method E) |
| 275 | 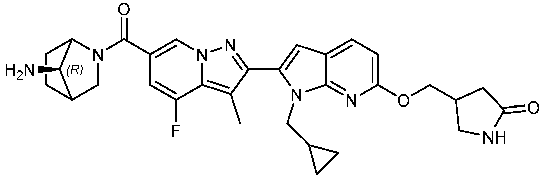 4-{[(2-{6-[(7R)-7-Amino-2-azabicyclo[2.2.1]heptane-2-carbonyl]-4-fluoro-3-methylpyrazolo[1,5-a]pyridin-2-yl}-1-(cyclopropylmethyl)-1H-pyrrolo[2,3-b]pyridin-6-yl)oxy]methyl }pyrrolidin-2-one (mixture of diastereomers) |
| | Analytical Data: 572.4; 1.519 min (Method E) |

### Example 276

### (3R)-1-{4-Chloro-2-[1-(cyclopropylmethyl)-6-(4-methoxypiperidin-1-yl)-1H-pyrrolo[2,3-b]pyridin-2-yl]-3-methylpyrazolo[1,5-a]pyridine-6-carbonyl}piperidin-3-amine

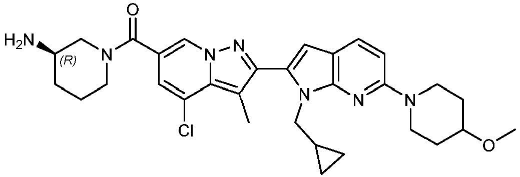

Compound of Example 276 was prepared following a procedure similar to the preparation of a compound of Example 50, using Intermediate 24 to carboxylic acid and tert-butyl (R)-piperidin-3-ylcarbamate in amide formation and with appropriate secondary amine in Buchwald coupling reaction. LC-MS m/z: 576.3 [M+H]⁺; HPLC retention time: 1.649 min (method F)

Compounds of Examples 277 to 288 in Table 34 were prepared following a procedure similar to the preparation of a compound of Example 54, by using Intermediate 39D and *tert-butyl* (R)-piperidin-3-ylcarbamate in amide formation and appropriate boronic acid or boronate ester in Suzuki coupling reaction.

**Table 34**

| Example | Structure |
|---|---|
| | Name |
| | Analytical Data: LC-MS m/z [M+H]⁺; HPLC retention time (HPLC method) |
| 277 | 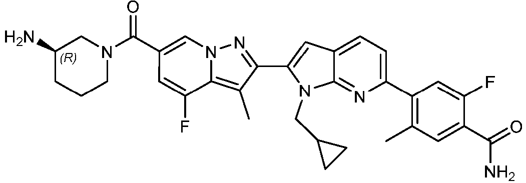 4-(2-{6-[(3R)-3-Aminopiperidine-1-carbonyl]-4-fluoro-3-methylpyrazolo[1,5-a]pyridin-2-yl}-1-(cyclopropylmethyl)-1H-pyrrolo[2,3-b]pyridin-6-yl)-2-fluoro-5-methylbenzamide |
| | Analytical Data: 598.3; 1.689 min (Method E) |
| 278 | 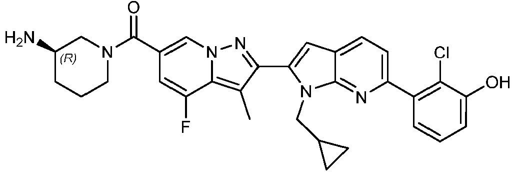 3-(2-{6-[(3R)-3-Aminopiperidine-1-carbonyl]-4-fluoro-3-methylpyrazolo[1,5-a]pyridin-2-yl}-1-(cyclopropylmethyl)-1H-pyrrolo[2,3-b]pyridin-6-yl)-2-chlorophenol |
| | Analytical Data: 573.2; 1.777 min (Method E) |
| 279 | 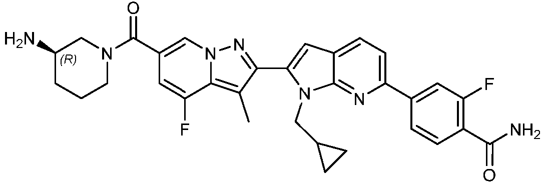 4-(2-{6-[(3R)-3-Aminopiperidine-1-carbonyl]-4-fluoro-3-methylpyrazolo[1,5-a]pyridin-2-yl}-1-(cyclopropylmethyl)-1H-pyrrolo[2,3-b]pyridin-6-yl)-2-fluorobenzamide |
| | Analytical Data: 584.3; 1.603 min (Method E) |
| 280 | 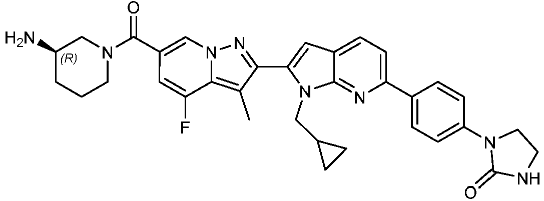 1-[4-(2-{6-[(3R)-3-Aminopiperidine-1-carbonyl]-4-fluoro-3-methylpyrazolo[1,5-a]pyridin-2-yl}-1-(cyclopropylmethyl)-1H-pyrrolo[2,3-b]pyridin-6-yl)phenyl]imidazolidin-2-one |
| | Analytical Data: 607.3; 1.710 min (Method E) |
| 281 | 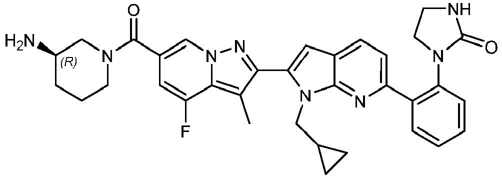 1-[2-(2-{6-[(3R)-3-Aminopiperidine-1-carbonyl]-4-fluoro-3-methylpyrazolo[1,5-a]pyridin-2-yl }-1-(cyclopropylmethyl)-1H-pyrrolo[2,3-b]pyridin-6-yl)phenyl]imidazolidin-2-one |
| | Analytical Data: 607.3; 1.554 min (Method E) |
| 282 | 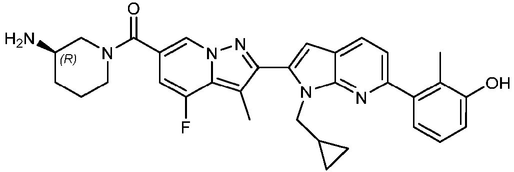 3-(2- f 6-[(3R)-3-Aminopiperidine-1-carbonyl]-4-fluoro-3-methylpyrazolo[1,5-a]pyridin-2-yl}-1-(cyclopropylmethyl)-1H-pyrrolo[2,3-b]pyridin-6-yl)-2-methylphenol |
| | Analytical Data: 555.3; 1.782 min (Method E) |
| 283 | 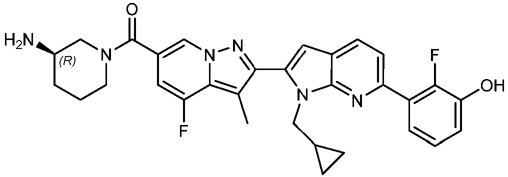 3-(2-{6-[(3R)-3-Aminopiperidine-1-carbonyl]-4-fluoro-3-methylpyrazolo[1,5-a]pyridin-2-yl }-1-(cyclopropylmethyl)-1H-pyrrolo[2,3-b]pyridin-6-yl)-2-fluorophenol |
| | Analytical Data: 557.3; 1.735 min (Method E) |
| 284 | 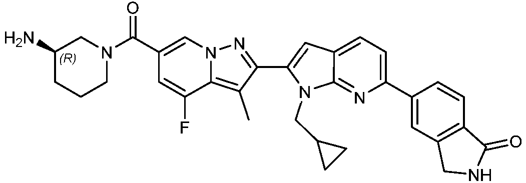 5-(2-{6-[(3R)-3-Aminopiperidine-1-carbonyl]-4-fluoro-3-methylpyrazolo[1,5-a]pyridin-2-yl}-1-(cyclopropylmethyl)-1H-pyrrolo[2,3-b]pyridin-6-yl)-2,3-dihydro-1H-isoindol-1-one |
| | Analytical Data: 578.3; 1.533 min (Method E) |
| 285 | 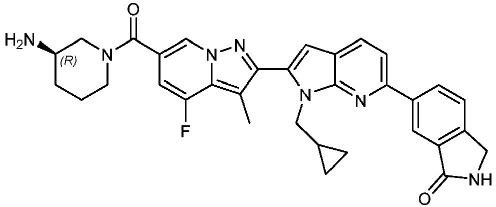 6-(2-{6-[(3R)-3-Aminopiperidine-1-carbonyl]-4-fluoro-3-methylpyrazolo[1,5-a]pyridin-2-yl}-1-(cyclopropylmethyl)-1H-pyrrolo[2,3-b]pyridin-6-yl)-2,3-dihydro-1H-isoindol-1-one |
| | Analytical Data: 578.3, 1.625 min (Method E) |
| 286 | 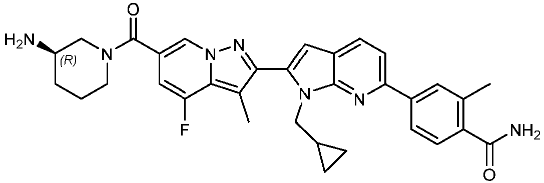 4-(2-{6-[(3R)-3-Aminopiperidine-1-carbonyl]-4-fluoro-3-methylpyrazolo[1,5-a]pyridin-2-yl}-1-(cyclopropylmethyl)-1H-pyrrolo[2,3-b]pyridin-6-yl)-2-methylbenzamide |
| | Analytical Data: 580.3; 1.618 min (Method E) |
| 287 | 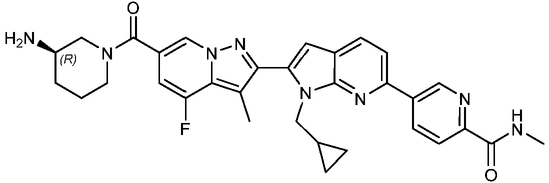 5-(2-{6-[(3R)-3-Aminopiperidine-1-carbonyl]-4-fluoro-3-methylpyrazolo[1,5-a]pyridin-2-yl}-1-(cyclopropylmethyl)-1H-pyrrolo[2,3-b]pyridin-6-yl)-N-methylpyridine-2-carboxamide |
| | Analytical Data: 581.2; 1.693 min (Method E) |
| 288 | 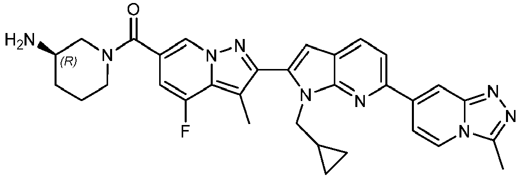 (3R)-1-{2-[1-(Cyclopropylmethyl)-6-{3-methyl-[1,2,4]triazolo[4,3-a]pyridin-7-yl}-1H-pyrrolo[2,3-b]pyridin-2-yl]-4-fluoro-3-methylpyrazolo[1,5-a]pyridine-6-carbonyl}piperidin-3-amine |
| | Analytical Data: 578.3; 1.441 min (Method E) |

### Example 289

### (3R,5R)-1-{4-Chloro-2-[6-chloro-1-(cyclopropylmethyl)-1H-pyrrolo[2,3-b]pyridin-2-yl]-3-methylpyrazolo[1,5-a]pyridine-6-carbonyl}-5-fluoropiperidin-3-amine

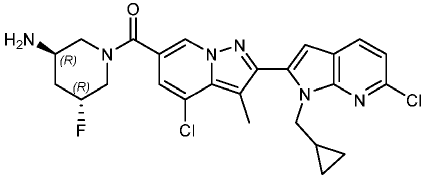

Compound of Example 289 was prepared following a procedure similar to the preparation of a compound of Example 53 by using Intermediate 24 to carboxylic acid and tert-butyl ((3R,5R)-5-fluoropiperidin-3-yl)carbamate in amide formation. LC-MS m/z: 515.1 [M+H]⁺; HPLC retention time: 2.048 min (Method E).

### Example 290

### (3R,5R)-1-{4-Chloro-2-[1-(cyclopropylmethyl)-6-(1H-indazol-5-yl)-1H-pyrrolo[2,3-b]pyridin-2-yl]-3-methylpyrazolo[1,5-a]pyridine-6-carbonyl}-5-fluoropiperidin-3-amine

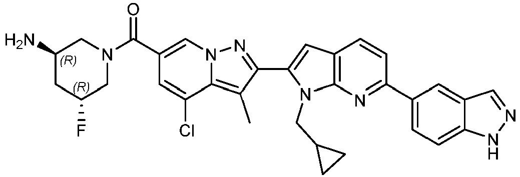

Compound of Example 290 was prepared following a procedure similar to the preparation of a compound of Example 54 by using Intermediate 24 to carboxylic acid and tert-butyl ((3R,5R)-5-fluoropiperidin-3-yl)carbamate in amide formation and appropriate boronic acid or boronate ester in Suzuki coupling reaction. LC-MS m/z: 597.2 [M+H]⁺; HPLC retention time: 1.878 min (Method E).

### Example 291

### (3R)-1-{4-Chloro-2-[6-chloro-1-(cyclopropylmethyl)-1H-pyrrolo[2,3-b]pyridin-2-yl]-3-methylpyrazolo[1,5-a]pyridine-6-carbonyl}piperidin-3-amine

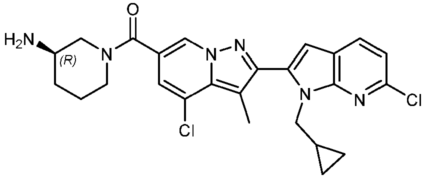

Compound of Example 291 was prepared following a procedure similar to the preparation of a compound of Example 53, by using Intermediate 24 to carboxylic acid and tert-butyl (R)-piperidin-3-ylcarbamate in amide formation. LC-MS m/z: 497.1 [M+H]⁺; HPLC retention time: 1.992 min (Method E).

### Example 292

### (3R)-1-{4-chloro-2-[1-(Cyclopropylmethyl)-6-(1H-indazol-5-yl)-1H-pyrrolo[2,3-b]pyridin-2-yl]-3-methylpyrazolo[1,5-a]pyridine-6-carbonyl}piperidin-3-amine

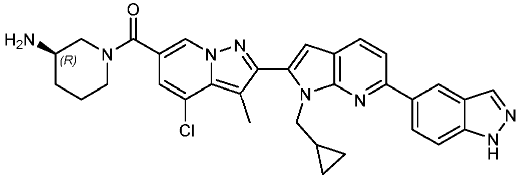

Compound of Example 292 was prepared following a procedure similar to the preparation of a compound of Example 54 by using Intermediate 24 to carboxylic acid and tert-butyl (R)-piperidin-3-ylcarbamate in amide formation and appropriate boronic acid or boronate ester in Suzuki coupling reaction. LC-MS m/z: 579.2 [M+H]⁺; HPLC retention time: 1.787 min (Method E)

Compounds of Examples 293 to 295 in Table 35 were prepared following a procedure similar to the preparation of a compound of Example 58, by Suzuki coupling of Intermediate 58E with appropriate boronic acid.

**Table 35**

| Example | Structure |
|---|---|
| | Name |
| | Analytical Data: LC-MS m/z [M+H]⁺; HPLC retention time (HPLC method) |
| 293 | 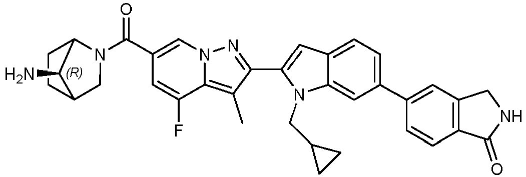 5-(2-{6-[(7R)-7-Amino-2-azabicyclo[2.2.1]heptane-2-carbonyl]-4-fluoro-3-methylpyrazolo[1,5-a]pyridin-2-yl}-1-(cyclopropylmethyl)-1H-indol-6-yl)-2,3-dihydro-1H-isoindol-1-one |
| | Analytical Data: 589.3; 1.808 min (Method E) |
| 294 | 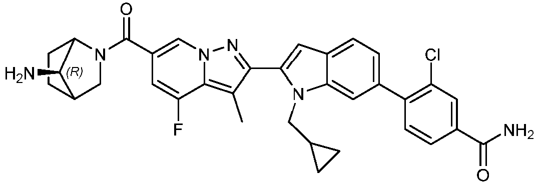 4-(2-{6-[(7R)-7-Amino-2-azabicyclo[2.2.1]heptane-2-carbonyl]-4-fluoro-3-methylpyrazolo[1,5-a]pyridin-2-yl}-1-(cyclopropylmethyl)-1H-indol-6-yl)-3-chlorobenzamide |
| | Analytical Data: 611.3; 1.880 min (Method E) |
| 295 | 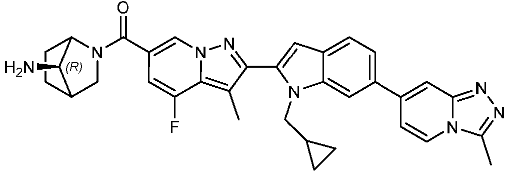 (7R)-2-{2-[1-(Cyclopropylmethyl)-6-{3-methyl-[1,2,4]triazolo[4,3-a]pyridin-7-yl}-1H-indol-2-yl]-4-fluoro-3-methylpyrazolo[1,5-a]pyridine-6-carbonyl}-2-azabicyclo[2.2.1]heptan-7-amine |
| | Analytical Data: 589.4; 1.784 min (Method D) |

### Example 296

### (7R)-2-{2-[1-(Cyclopropylmethyl)-6-(3-methoxyazetidin-1-yl)-1H-indol-2-yl]-4-fluoro-3-methylpyrazolo[1,5-a]pyridine-6-carbonyl}-2-azabicyclo[2.2.1]heptan-7-amine

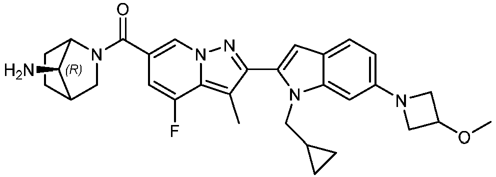

Compound of Example 296 was prepared following a procedure similar to the preparation of a compound of Example 50, by using Intermediate 58E and appropriate secondary amine in Buchwald coupling reaction. LC-MS m/z: 543.3 [M+H]⁺; HPLC retention time: 2.028 min (Method E).

Compounds of Examples 297 to 308 in Table 36 were prepared following a procedure similar to the preparation of a compound of Example 64, by Suzuki coupling of Intermediate 64E with appropriate boronic acid.

**Table 36**

| Example | Structure |
|---|---|
| | Name |
| | Analytical Data: LC-MS m/z [M+H]⁺; HPLC retention time (HPLC method) |
| 297 | 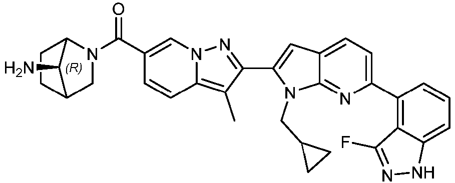 (7R)-2-{2-[1-(Cyclopropylmethyl)-6-(3-fluoro-1H-indazol-4-yl)-1H-pyrrolo[2,3-b]pyridin-2-yl]-3-methylpyrazolo[1,5-a]pyridine-6-carbonyl}-2-azabicyclo[2.2.1]heptan-7-amine |
| | Analytical Data: 575.3;1.56min (Method E) |
| 298 | 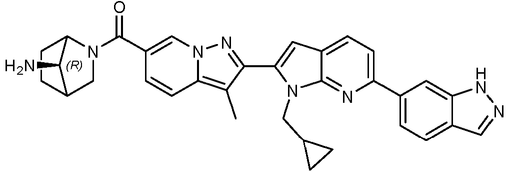 (7R)-2-{2-[1-(Cyclopropylmethyl)-6-(1H-indazol-6-yl)-1H-pyrrolo[2,3-b]pyridin-2-yl]-3-methylpyrazolo[1,5-a]pyridine-6-carbonyl}-2-azabicyclo[2.2.1]heptan-7-amine |
| | Analytical Data: 557.2, 1.754min (Method E) |
| 299 | 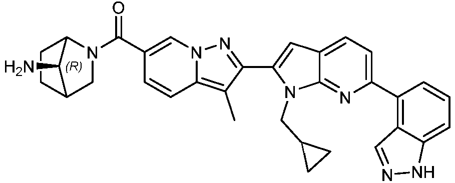 (7R)-2-{2-[1-(Cyclopropylmethyl)-6-(1H-indazol-4-yl)-1H-pyrrolo[2,3-b]pyridin-2-yl]-3-methylpyrazolo[1,5-a]pyridine-6-carbonyl}-2-azabicyclo[2.2.1]heptan-7-amine |
| | Analytical Data: 557.2; 1.749min (Method E) |
| 300 | 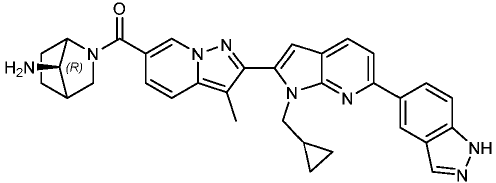 (7R)-2-{2-[1-(Cyclopropylmethyl)-6-(1H-indazol-5-yl)-1H-pyrrolo[2,3-b]pyridin-2-yl]-3-methylpyrazolo[1,5-a]pyridine-6-carbonyl}-2-azabicyclo[2.2.1]heptan-7-amine |
| | Analytical Data: 557.2; 1.702 min (Method E) |
| 301 | 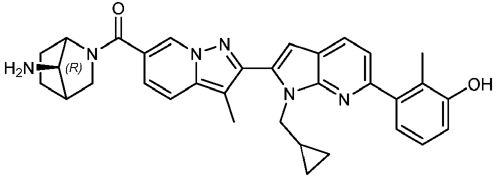 3-(2-{6-[(7R)-7-Amino-2-azabicyclo[2.2.1]heptane-2-carbonyl]-3-methylpyrazolo[1,5-a]pyridin-2-yl}-1-(cyclopropylmethyl)-1H-pyrrolo[2,3-b]pyridin-6-yl)-2-methylphenol |
| | Analytical Data: 547.3; 1.491min (Method E) |
| 302 | 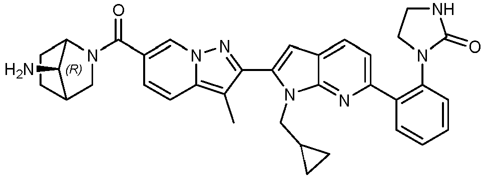 1-[2-(2-{6-[(7R)-7-Amino-2-azabicyclo[2.2.1]heptane-2-carbonyl]-3-methylpyrazolo[1,5-a]pyridin-2-yl}-1-(cyclopropylmethyl)-1H-pyrrolo[2,3-b]pyridin-6-yl)phenyl]imidazolidin-2-one |
| | Analytical Data: 601.3; 1.395 min (Method E) |
| 303 | 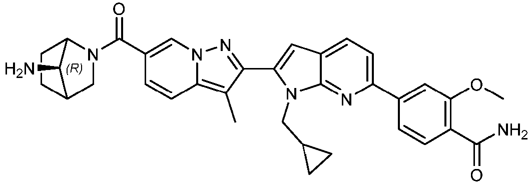 4-(2-{6-[(7R)-7-Amino-2-azabicyclo[2.2.1]heptane-2-carbonyl]-3-methylpyrazolo[1,5-a]pyridin-2-yl}-1-(cyclopropylmethyl)-1H-pyrrolo[2,3-b]pyridin-6-yl)-2-methoxybenzamide |
| | Analytical Data: 590.3; 1.752min (Method E) |
| 304 | 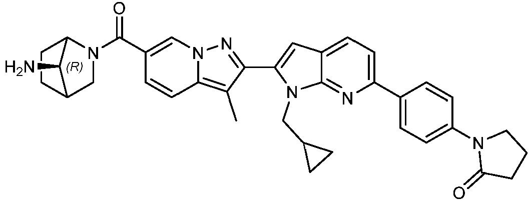 1-[4-(2-{6-[(7R)-7-Amino-2-azabicyclo[2.2.1]heptane-2-carbonyl]-3-methylpyrazolo[1,5-a]pyridin-2-yl}-1-(cyclopropylmethyl)-1H-pyrrolo[2,3-b]pyridin-6-yl)phenyl]pyrrolidin-2-one |
| | Analytical Data: 600.4; 1.875min (Method E) |
| 305 | 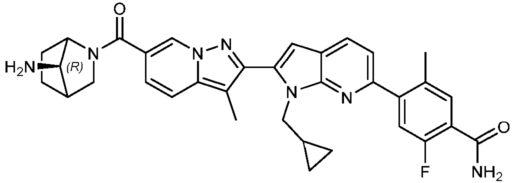 4-(2-{6-[(7R)-7-Amino-2-azabicyclo[2.2.1]heptane-2-carbonyl]-3-methylpyrazolo[1,5-a]pyridin-2-yl}-1-(cyclopropylmethyl)-1H-pyrrolo[2,3-b]pyridin-6-yl)-2-fluoro-5-methylbenzamide |
| | Analytical Data: 592.3; 1.433min (Method E) |
| 306 | 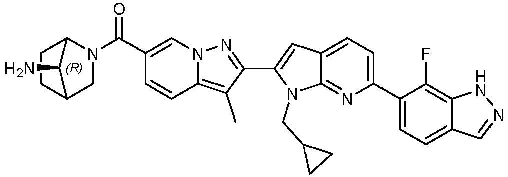 (7R)-2-{2-[1-(Cyclopropylmethyl)-6-(7-fluoro-1H-indazol-6-yl)-1H-pyrrolo[2,3-b]pyridin-2-yl]-3-methylpyrazolo[1,5-a]pyridine-6-carbonyl}-2-azabicyclo[2.2.1]heptan-7-amine |
| | Analytical Data: 575.3, 2.036 min (Method E) |
| 307 | 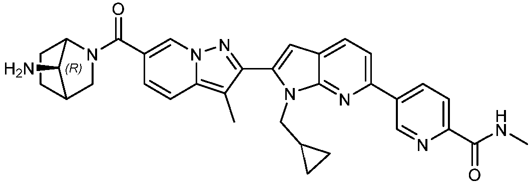 5-(2-{6-[(7R)-7-Amino-2-azabicyclo[2.2.1]heptane-2-carbonyl]-3-methylpyrazolo[1,5-a]pyridin-2-yl}-1-(cyclopropylmethyl)-1H-pyrrolo[2,3-b]pyridin-6-yl)-N-methylpyridine-2-carboxamide |
| | Analytical Data: 575.4; 1.508 min (Method E) |
| 308 | 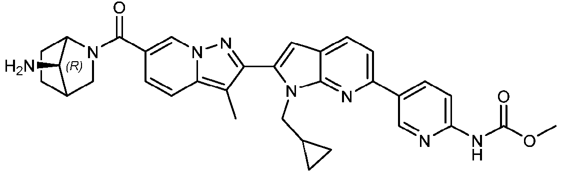 Methyl N-[5-(2-{6-[(7R)-7-amino-2-azabicyclo[2.2.1]heptane-2-carbonyl]-3-methylpyrazolo[1,5-a]pyridin-2-yl}-1-(cyclopropylmethyl)-1H-pyrrolo[2,3-b]pyridin-6-yl)pyridin-2-yl]carbamate |
| | Analytical Data: 591.4; 1.748min (Method E) |

Compounds of Examples 309 to 317 in Table 37 were prepared following a procedure similar to the preparation of a compound of Example 64, by using Intermediate 64D and tert-butyl (R)-piperidin-3-ylcarbamate in amide formation and appropriate boronic acid / boronate ester in Suzuki coupling.

**Table 37**

| Example | Structure |
|---|---|
| | Name |
| | Analytical Data: LC-MS m/z [M+H]⁺; HPLC retention time (HPLC method) |
| 309 | 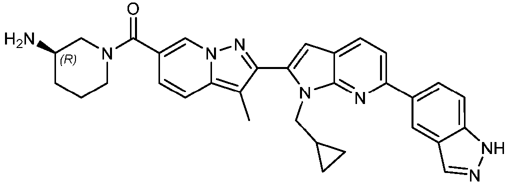 (3R)-1-{2-[1-(Cyclopropylmethyl)-6-(1H-indazol-5-yl)-1H-pyrrolo[2,3-b]pyridin-2-yl]-3-methylpyrazolo[1,5-a]pyridine-6-carbonyl}piperidin-3-amine |
| | Analytical Data: 545.3; 1.523 min (Method E) |
| 310 | 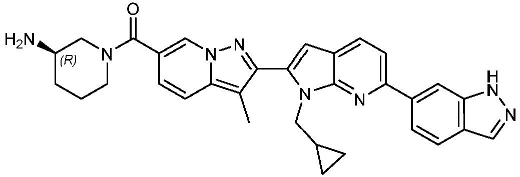 (3R)-1-{2-[1-(Cyclopropylmethyl)-6-(1H-indazol-6-yl)-1H-pyrrolo[2,3-b]pyridin-2-yl]-3-methylpyrazolo[1,5-a]pyridine-6-carbonyl}piperidin-3-amine |
| | Analytical Data: 545.3; 1.608min (Method E) |
| 311 | 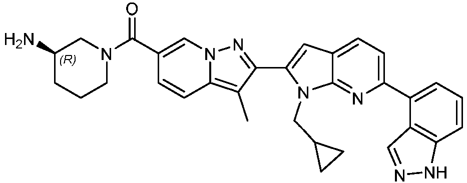 (3R)-1-{2-[1-(Cyclopropylmethyl)-6-(1H-indazol-4-yl)-1H-pyrrolo[2,3-b]pyridin-2-yl]-3-methylpyrazolo[1,5-a]pyridine-6-carbonyl}piperidin-3-amine |
| | Analytical Data: 545.3; 1.581 min (Method E) |
| 312 | 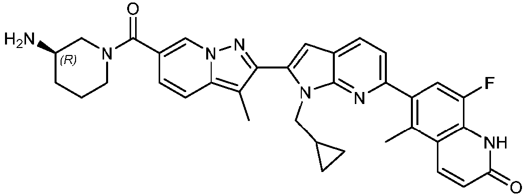 6-(2-{6-[(3R)-3-Aminopiperidine-1-carbonyl]-3-methylpyrazolo[1,5-a]pyridin-2-yl}-1-(cyclopropylmethyl)-1H-pyrrolo[2,3-b]pyridin-6-yl)-8-fluoro-5-methyl-1,2-dihydroquinolin-2-one |
| | Analytical Data: 604.4; 1.454min (Method E) |
| 313 | 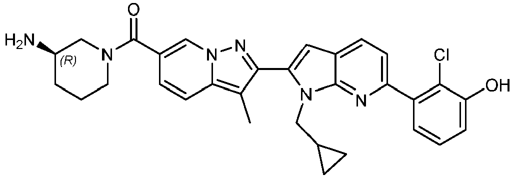 3-(2-{6-[(3R)-3-Aminopiperidine-1-carbonyl]-3-methylpyrazolo[1,5-a]pyridin-2-yl}-1-(cyclopropylmethyl)-1H-pyrrolo[2,3-b]pyridin-6-yl)-2-chlorophenol |
| | Analytical Data: 555.3; 1.606 min (Method E) |
| 314 | 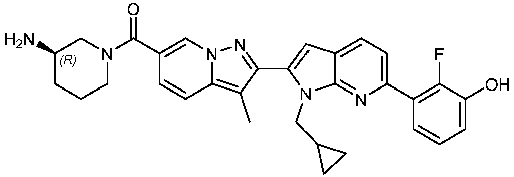 3-(2-{6-[(3R)-3-Aminopiperidine-1-carbonyl]-3-methylpyrazolo[1,5-a]pyridin-2-yl}-1-(cyclopropylmethyl)-1H-pyrrolo[2,3-b]pyridin-6-yl)-2-fluorophenol |
| | Analytical Data: 539.3; 1.579 min (Method E) |
| 315 | 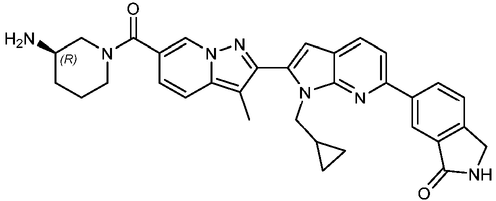 6-(2-{6-[(3R)-3-Aminopiperidine-1-carbonyl]-3-methylpyrazolo[1,5-a]pyridin-2-yl}-1-(cyclopropylmethyl)-1H-pyrrolo[2,3-b]pyridin-6-yl)-2,3-dihydro-1H-isoindol-1-one |
| | Analytical Data: 560.4; 1.507 min (Method E) |
| 316 | 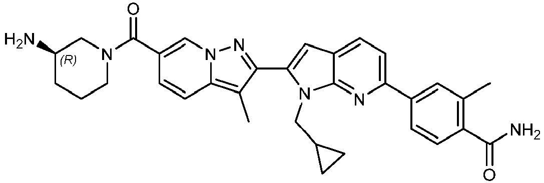 4-(2-{6-[(3R)-3-Aminopiperidine-1-carbonyl]-3-methylpyrazolo[1,5-a]pyridin-2-yl}-1-(cyclopropylmethyl)-1H-pyrrolo[2,3-b]pyridin-6-yl)-2-methylbenzamide |
| | Analytical Data: 562.4; 1.415min (Method E) |
| 317 | 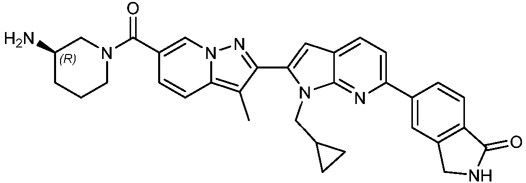 5-(2-{6-[(3R)-3-Aminopiperidine-1-carbonyl]-3-methylpyrazolo[1,5-a]pyridin-2-yl}-1-(cyclopropylmethyl)-1H-pyrrolo[2,3-b]pyridin-6-yl)-2,3-dihydro-1H-isoindol-1-one |
| | Analytical Data: 560.4; 1.391 min (Method E) |

Compounds of Examples 318 to 320 in Table 38 were prepared following a procedure similar to the preparation of a compound of Example 76, by Buchwald coupling of Intermediate 64E with appropriate amines.

**Table 38**

| Example | Structure |
|---|---|
| | Name |
| | Analytical Data: LC-MS m/z [M+H]⁺; HPLC retention time (HPLC method) |
| 318 | 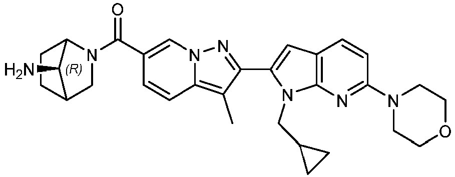 (7R)-2-{2-[1-(Cyclopropylmethyl)-6-(morpholin-4-yl)-1H-pyrrolo[2,3-b]pyridin-2-yl]-3-methylpyrazolo[1,5-a]pyridine-6-carbonyl}-2-azabicyclo[2.2.1]heptan-7-amine |
| | Analytical Data: 526.3, 1.782 min (Method E) |
| 319 | 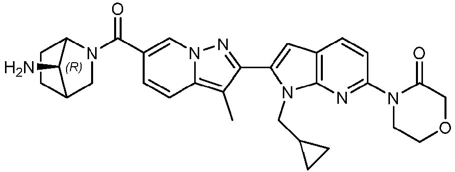 4-(2-{6-[(7R)-7-Amino-2-azabicyclo[2.2.1]heptane-2-carbonyl]-3-methylpyrazolo[1,5-a]pyridin-2-yl}-1-(cyclopropylmethyl)-1H-pyrrolo[2,3-b]pyridin-6-yl)morpholin-3-one |
| | Analytical Data: 540.4; 1.458 min (Method E) |
| 320 | 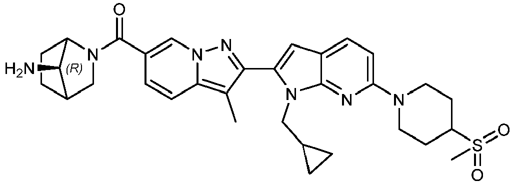 (7R)-2-{2-[1-(Cyclopropylmethyl)-6-(4-methanesulfonylpiperidin-1-yl)-1H-pyrrolo[2,3-b]pyridin-2-yl]-3-methylpyrazolo[1,5-a]pyridine-6-carbonyl}-2-azabicyclo[2.2.1]heptan-7-amine |
| | Analytical Data: 602.2; 1.326min (Method E) |

Compounds of Examples 321 to 324 in Table 39 were prepared following a procedure similar to the preparation of a compound of Example 81, by Suzuki coupling of Intermediate 81A with appropriate boronic acid.

**Table 39**

| Example | Structure |
|---|---|
| | Name |
| | Analytical Data: LC-MS m/z [M+H]⁺; HPLC retention time (HPLC method) |
| 321 | 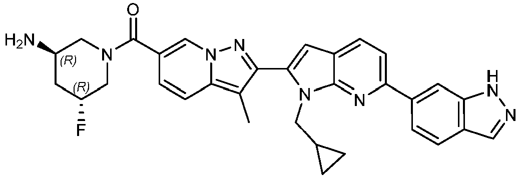 (3R,5R)-1-{2-[1-(Cyclopropylmethyl)-6-(1H-indazol-6-yl)-1H-pyrrolo[2,3-b]pyridin-2-yl]-3-methylpyrazolo[1,5-a]pyridine-6-carbonyl}-5-fluoropiperidin-3-amine |
| | Analytical Data: 563.3; 2.191 min (Method E) |
| 322 | 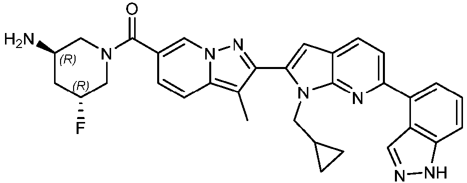 (3R,5R)-1-{2-[1-(Cyclopropylmethyl)-6-(1H-indazol-4-yl)-1H-pyrrolo[2,3-b]pyridin-2-yl]-3-methylpyrazolo[1,5-a]pyridine-6-carbonyl}-5-fluoropiperidin-3-amine |
| | Analytical Data: 563.2; 1.744min (Method E) |
| 323 | 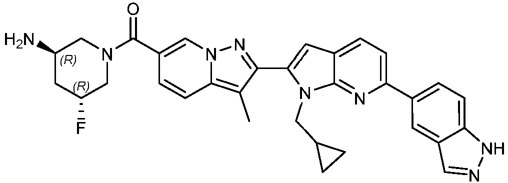 (3R,5R)-1-{2-[1-(Cyclopropylmethyl)-6-(1H-indazol-5-yl)-1H-pyrrolo[2,3-b]pyridin-2-yl]-3-methylpyrazolo[1,5-a]pyridine-6-carbonyl}-5-fluoropiperidin-3-amine |
| | Analytical Data: 563.3; 1.670min (Method E) |
| 324 | 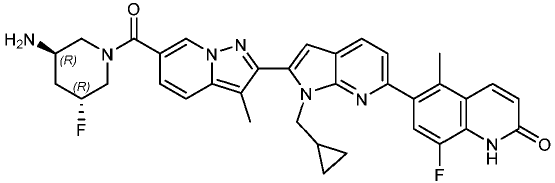 6-(2-{6-[(3R,5R)-3-Amino-5-fluoropiperidine-1-carbonyl]-3-methylpyrazolo[1,5-a]pyridin-2-yl}-1-(cyclopropylmethyl)-1H-pyrrolo[2,3-b]pyridin-6-yl)-8-fluoro-5-methyl-1,2-dihydroquinolin-2-one |
| | Analytical Data: 622.3; 1.461min (Method E) |

### Example 325

### Methyl N-[1-(2-{6-[(3R,5R)-3-amino-5-fluoropiperidine-1-carbonyl]-3-methylpyrazolo[1,5-a]pyridin-2-yl}-1-(cyclopropylmethyl)-1H-pyrrolo[2,3-b]pyridin-6-yl)piperidin-4-yl]-N-methylcarbamate

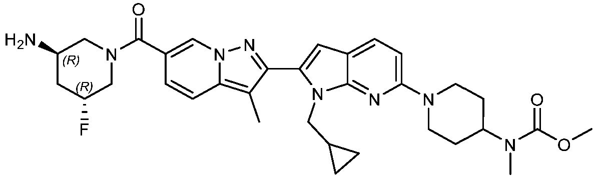

Compound of Example 325 was prepared following a procedure similar to the preparation of a compound of Example 87, by Buchwald coupling of Intermediate 81A with appropriate secondary amine. LC-MS m/z: 617 [M+H]⁺; HPLC retention time: 1.530min (Method E).

Compounds of Examples 326 to 341 in Table 40 were prepared following a procedure similar to the preparation of a compound of Example 89, by Suzuki coupling of Intermediate 89E with appropriate boronic acid or boronate ester.

**Table 40**

| Example | Structure |
|---|---|
| | Name |
| | Analytical Data: LC-MS m/z [M+H]⁺; HPLC retention time (HPLC method) |
| 326 | 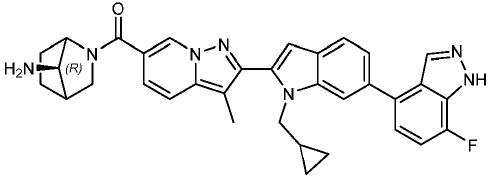 (7R)-2-{2-[1-(Cyclopropylmethyl)-6-(7-fluoro-1H-indazol-4-yl)-1H-indol-2-yl]-3-methylpyrazolo[1,5-a]pyridine-6-carbonyl}-2-azabicyclo[2.2.1]heptan-7-amine |
| | Analytical Data: 574.3; 2.144 min (Method E) |
| 327 | 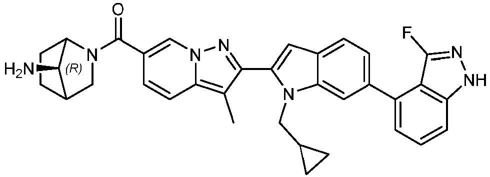 (7R)-2-{2-[1-(Cyclopropylmethyl)-6-(3-fluoro-1H-indazol-4-yl)-1H-indol-2-yl]-3-methylpyrazolo[1,5-a]pyridine-6-carbonyl}-2-azabicyclo[2.2.1]heptan-7-amine |
| | Analytical Data:574.3; 2.173min (Method E) |
| 328 | 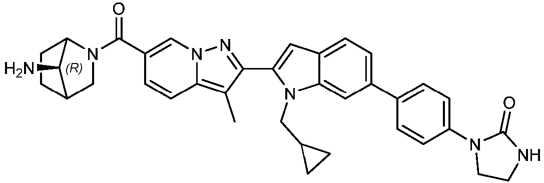 1-[4-(2-{6-[(7R)-7-Amino-2-azabicyclo[2.2.1]heptane-2-carbonyl]-3-methylpyrazolo[1,5-a]pyridin-2-yl}-1-(cyclopropylmethyl)-1H-indol-6-yl)phenyl]imidazolidin-2-one |
| | Analytical Data: 600.4; 1.762min (Method E) |
| 329 | 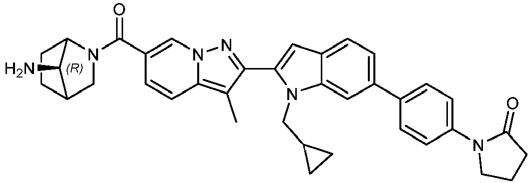 1-[4-(2-{6-[(7R)-7-Amino-2-azabicyclo[2.2.1]heptane-2-carbonyl]-3-methylpyrazolo[1,5-a]pyridin-2-yl}-1-(cyclopropylmethyl)-1H-indol-6-yl)phenyl]pyrrolidin-2-one |
| | Analytical Data: 599.4; 1.883 min (Method E) |
| 330 | 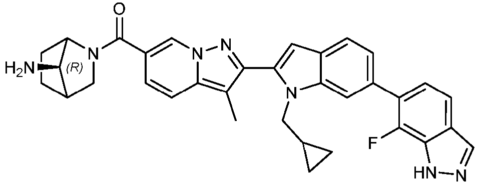 (7R)-2-{2-[1-(Cyclopropylmethyl)-6-(7-fluoro-1H-indazol-6-yl)-1H-indol-2-yl]-3-methylpyrazolo[1,5-a]pyridine-6-carbonyl}-2-azabicyclo[2.2.1]heptan-7-amine |
| | Analytical Data:574.4; 2.053min (Method E) |
| 331 | 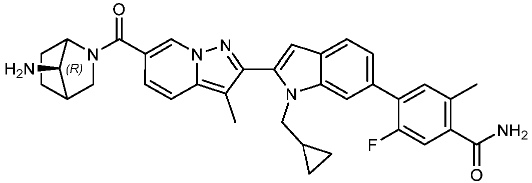 4-(2-{6-[(7R)-7-Amino-2-azabicyclo[2.2.1]heptane-2-carbonyl]-3-methylpyrazolo[1,5-a]pyridin-2-yl}-1-(cyclopropylmethyl)-1H-indol-6-yl)-5-fluoro-2-methylbenzamide |
| | Analytical Data: 591.2; 1.481 min (Method E) |
| 332 | 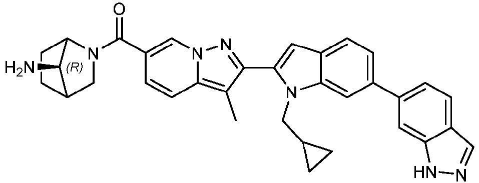 (7R)-2-{2-[1-(Cyclopropylmethyl)-6-(1H-indazol-6-yl)-1H-indol-2-yl]-3-methylpyrazolo[1,5-a]pyridine-6-carbonyl}-2-azabicyclo[2.2.1]heptan-7-amine |
| | Analytical Data: 556.2; 1.772min (Method E) |
| 333 | 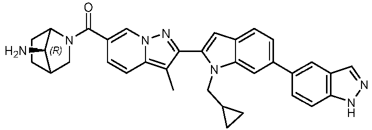 (7R)-2-{2-[1-(Cyclopropylmethyl)-6-(1H-indazol-5-yl)-1H-indol-2-yl]-3-methylpyrazolo[1,5-a]pyridine-6-carbonyl}-2-azabicyclo[2.2.1]heptan-7-amine |
| | Analytical Data: 556.2; 1.703 min (Method E) |
| 334 | 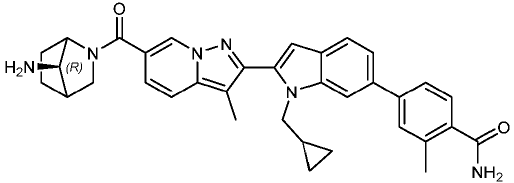 4-(2-{6-[(7R)-7-Amino-2-azabicyclo[2.2.1]heptane-2-carbonyl]-3-methylpyrazolo[1,5-a]pyridin-2-yl}-1-(cyclopropylmethyl)-1H-indol-6-yl)-2-methylbenzamide |
| | Analytical Data: 573.2; 1.383 min (Method E) |
| 335 | 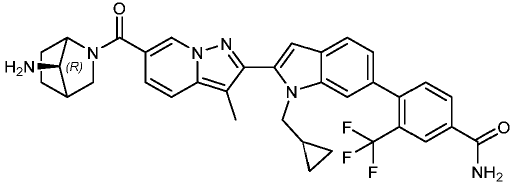 4-(2-{6-[(7R)-7-Amino-2-azabicyclo[2.2.1]heptane-2-carbonyl]-3-methylpyrazolo[1,5-a]pyridin-2-yl}-1-(cyclopropylmethyl)-1H-indol-6-yl)-3-(trifluoromethyl)benzamide |
| | Analytical Data: 627.3; 1.493min (Method E) |
| 336 | 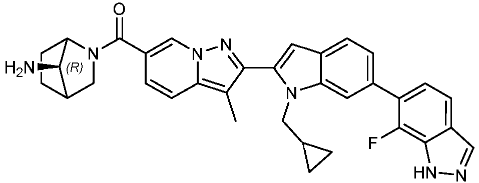 (7R)-2-{2-[1-(Cyclopropylmethyl)-6-(7-fluoro-1H-indazol-6-yl)-1H-indol-2-yl]-3-methylpyrazolo[1,5-a]pyridine-6-carbonyl}-2-azabicyclo[2.2.1]heptan-7-amine |
| | Analytical Data:574.3; 2.128min (Method E) |
| 337 | 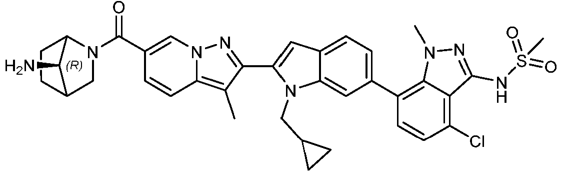 N-[7-(2-{6-[(7R)-7-Amino-2-azabicyclo[2.2.1]heptane-2-carbonyl]-3-methylpyrazolo[1,5-a]pyridin-2-yl}-1-(cyclopropylmethyl)-1H-indol-6-yl)-4-chloro-1-methyl-1H-indazol-3-yl]methanesulfonamide |
| | Analytical Data: 698.3; 2.217min (Method E) |
| 338 | 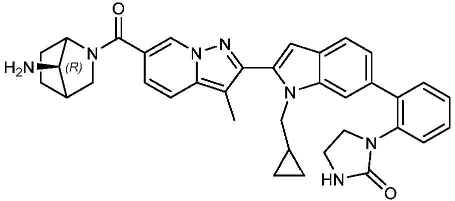 1-[2-(2-{6-[(7R)-7-Amino-2-azabicyclo[2.2.1]heptane-2-carbonyl]-3-methylpyrazolo[1,5-a]pyridin-2-yl}-1-(cyclopropylmethyl)-1H-indol-6-yl)phenyl]imidazolidin-2-one |
| | Analytical Data: 601.4; 1.825min (Method E) |
| 339 | 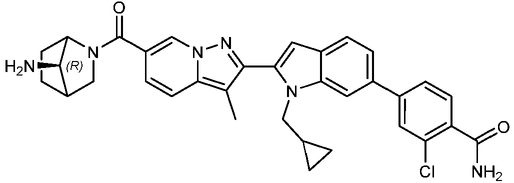 4-(2-{6-[(7R)-7-Amino-2-azabicyclo[2.2.1]heptane-2-carbonyl]-3-methylpyrazolo[1,5-a]pyridin-2-yl}-1-(cyclopropylmethyl)-1H-indol-6-yl)-2-chlorobenzamide |
| | Analytical Data: 594.2; 1.755 min (Method E) |
| 340 | 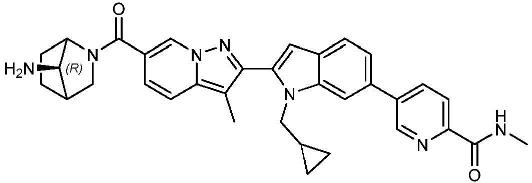 5-(2-{6-[(7R)-7-Amino-2-azabicyclo[2.2.1]heptane-2-carbonyl]-3-methylpyrazolo[1,5-a]pyridin-2-yl}-1-(cyclopropylmethyl)-1H-indol-6-yl)-N-methylpyridine-2-carboxamide |
| | Analytical Data: 574.2; 1.441min (Method E) |
| 341 | 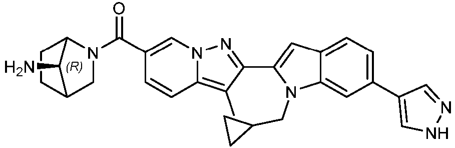 (7R)-2-{2-[1-(Cyclopropylmethyl)-6-(1H-pyrazol-4-yl)-1H-indol-2-yl]-3-methylpyrazolo[1,5-a]pyridine-6-carbonyl}-2-azabicyclo[2.2.1]heptan-7-amine |
| | Analytical Data: 506.2; 1.554 min (Method E) |

Compounds of Examples 342 to 349 in Table 41 were prepared following a procedure similar to the preparation of a compound of Example 92, by using Intermediate 89D and tert-butyl (R)-piperidin-3-ylcarbamate in amide formation and with appropriate boronic acid or boronate ester in Suzuki coupling reaction.

**Table 41**

| Example | Structure |
|---|---|
| | Name |
| | Analytical Data: LC-MS m/z [M+H]⁺; HPLC retention time (HPLC method) |
| 342 | 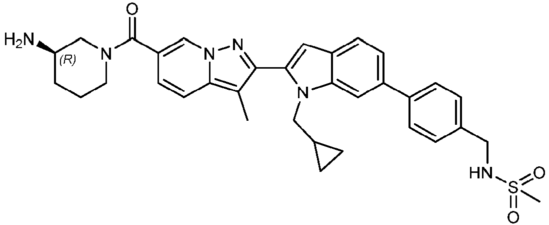 N-{[4-(2-{6-[(3R)-3-Aminopiperidine-1-carbonyl]-3-methylpyrazolo[1,5-a]pyridin-2-yl}-1-(cyclopropylmethyl)-1H-indol-6-yl)phenyl]methyl}methanesulfonamide |
| | Analytical Data: 611.2; 1.571min (Method E) |
| 343 | 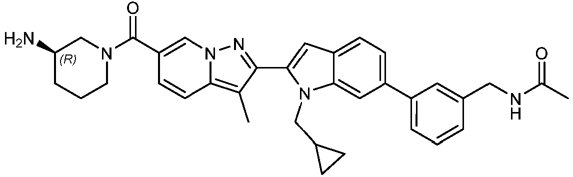 |
| | N-{[3-(2-{6-[(3R)-3-Aminopiperidine-1-carbonyl]-3-methylpyrazolo[1,5-a]pyridin-2-yl}-1-(cyclopropylmethyl)-1H-indol-6-yl)phenyl]methyl}acetamide |
| | Analytical Data: 575.3; 1.584 min (Method E) |
| 344 | 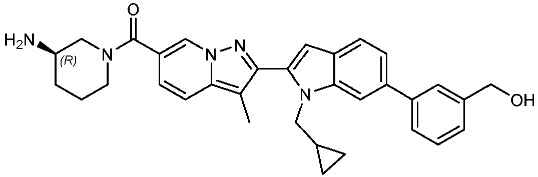 |
| | [3-(2-{6-[(3R)-3-Aminopiperidine-1-carbonyl]-3-methylpyrazolo[1,5-a]pyridin-2-yl}-1-(cyclopropylmethyl)-1H-indol-6-yl)phenyl]methanol |
| | Analytical Data: 534.3; 1.556 min (Method E) |
| 345 | 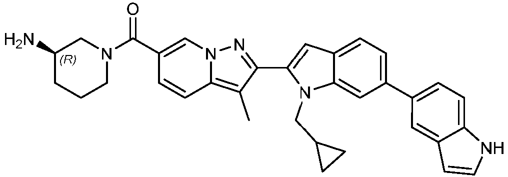 |
| | (3R)-1-{2-[1'-(Cyclopropylmethyl)-1H,1'H-[5,6'-biindole]-2'-yl]-3-methylpyrazolo[1,5-a]pyridine-6-carbonyl}piperidin-3-amine |
| | Analytical Data: 543.2; 1.869min (Method E) |
| 346 | 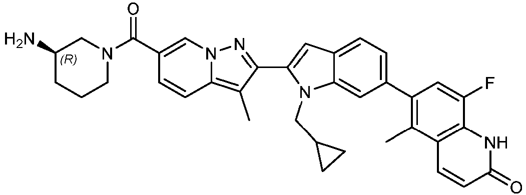 6-(2-{6-[(3R)-3-Aminopiperidine-1-carbonyl]-3-methylpyrazolo[1,5-a]pyridin-2-yl}-1-(cyclopropylmethyl)-1H-indol-6-yl)-8-fluoro-5-methyl-1,2-dihydroquinolin-2-one |
| | Analytical Data: 603.3; 1.535 min (Method E) |
| 347 | 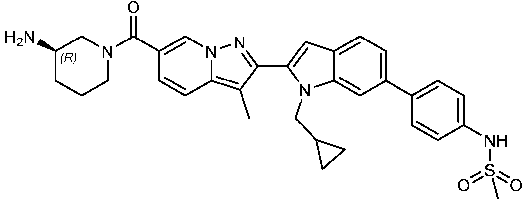 |
| | N-[4-(2-{6-[(3R)-3-Aminopiperidine-1-carbonyl]-3-methylpyrazolo[1,5-a]pyridin-2-yl}-1-(cyclopropylmethyl)-1H-indol-6-yl)phenyl]methanesulfonamide |
| | Analytical Data: 597.3; 1.625min (Method E) |
| 348 | 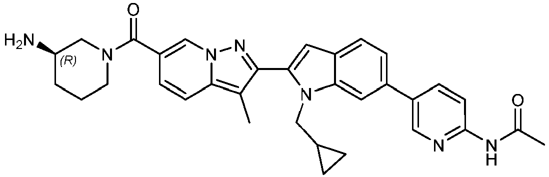 |
| | N-[5-(2-{6-[(3R)-3-Aminopiperidine-1-carbonyl]-3-methylpyrazolo[1,5-a]pyridin-2-yl}-1-(cyclopropylmethyl)-1H-indol-6-yl)pyridin-2-yl]acetamide |
| | Analytical Data: 562.3; 1.625min (Method E) |
| 349 | 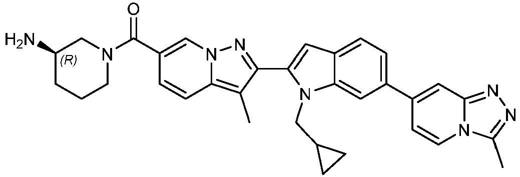 |
| | (R)-(3-Aminopiperidin-1-yl)(2-(1-(cyclopropylmethyl)-6-(3-methyl-[1,2,4]triazolo[4,3-a]pyridin-7-yl)-1H-indol-2-yl)-3-methylpyrazolo[1,5-a]pyridin-6-yl)methanone |
| | Analytical Data: 559.3; 1.452min (Method E) |

Compounds of Examples 350 and 351 in Table 42 were prepared following a procedure similar to the preparation of a compound of Example 91, by Buchwald coupling of Intermediate 89E with appropriate secondary amine.

**Table 42**

| Example | Structure |
|---|---|
| | Name |
| | Analytical Data: LC-MS m/z [M+H]⁺; HPLC retention time (HPLC method) |
| 350 | 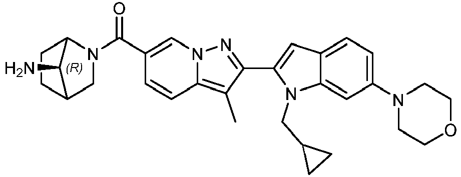 |
| | (7R)-2-{2-[1-(Cyclopropylmethyl)-6-(morpholin-4-yl)-1H-indol-2-yl]-3-methylpyrazolo[1,5-a]pyridine-6-carbonyl}-2-azabicyclo[2.2.1]heptan-7-amine |
| | Analytical Data: 525.3; 1.755 min (Method E) |
| 351 | 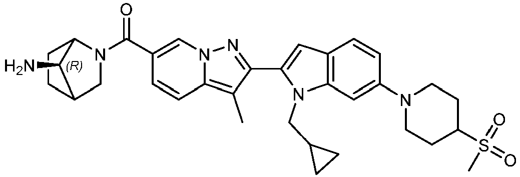 |
| | (7R)-2-{2-[1-(Cyclopropylmethyl)-6-(4-methanesulfonylpiperidin-1-yl)-1H-indol-2-yl]-3-methylpyrazolo[1,5-a]pyridine-6-carbonyl}-2-azabicyclo[2.2.1]heptan-7-amine |
| | Analytical Data: 601.3; 1.500min (Method E) |

Compounds of Examples 352 to 357 in Table 43 were prepared following a procedure similar to the preparation of a compound of Example 91, by using Intermediate 89D and tert-butyl ((3R,5R)-5-fluoropiperidin-3-yl)carbamate in amide formation and with appropriate secondary amine in Buchwald coupling reaction.

**Table 43**

| Example | Structure |
|---|---|
| | Name |
| | Analytical Data: LC-MS m/z [M+H]⁺; HPLC retention time (HPLC method) |
| 352 | 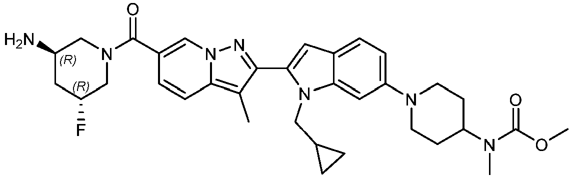 |
| | Methyl N-[1-(2-{6-[(3R,5R)-3-amino-5-fluoropiperidine-1-carbonyl]-3-methylpyrazolo[1,5-a]pyridin-2-yl}-1-(cyclopropylmethyl)-1H-indol-6-yl)piperidin-4-yl]-N-methylcarbamate |
| | Analytical Data: 616.3; 1.830min (Method E) |
| 353 | 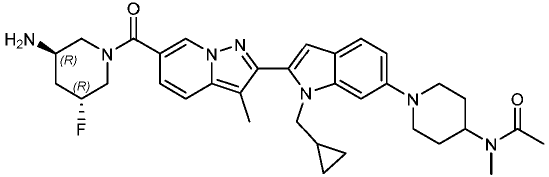 |
| | N-[1-(2-{6-[(3R,5R)-3-Amino-5-fluoropiperidine-1-carbonyl]-3-methylpyrazolo[1,5-a]pyridin-2-yl}-1-(cyclopropylmethyl)-1H-indol-6-yl)piperidin-4-yl]-N-methylacetamide |
| | Analytical Data: 600.4, 1.059min (Method E) |
| 354 | 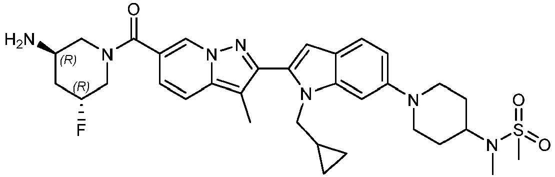 |
| | N-[1-(2-{6-[(3R,5R)-3-Amino-5-fluoropiperidine-1-carbonyl]-3-methylpyrazolo[1,5-a]pyridin-2-yl}-1-(cyclopropylmethyl)-1H-indol-6-yl)piperidin-4-yl]-N-methylmethanesulfonamide |
| | Analytical Data: 636.2; 1.656 min (Method E) |
| 355 | 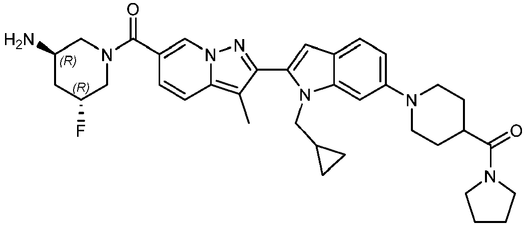 |
| | (3R,5R)-1-{2-[1-(Cyclopropylmethyl)-6-[4-(pyrrolidine-1-carbonyl)piperidin-1-yl]-1H-indol-2-yl]-3-methylpyrazolo[1,5-a]pyridine-6-carbonyl }-5-fluoropiperidin-3-amine |
| | Analytical Data: 626.4; 1.636 min (Method E) |
| 356 | 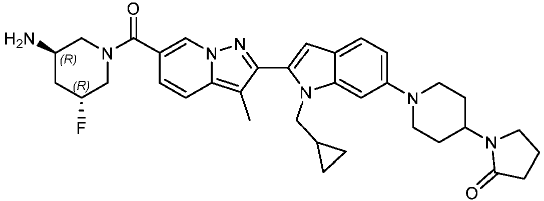 |
| | 1-[1-(2-{6-[(3R,5R)-3-Amino-5-fluoropiperidine-1-carbonyl]-3-methylpyrazolo[1,5-a]pyridin-2-yl}-1-(cyclopropylmethyl)-1H-indol-6-yl)piperidin-4-yl]pyrrolidin-2-one |
| | Analytical Data: 612.3; 1.512min (Method E) |
| 357 | 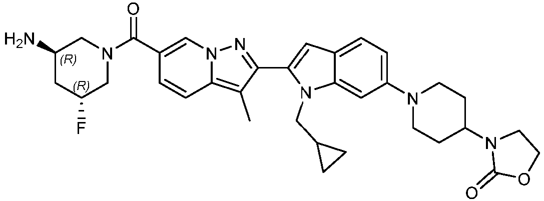 |
| | 3-[1-(2-{6-[(3R,5R)-3-Amino-5-fluoropiperidine-1-carbonyl]-3-methylpyrazolo[1,5-a]pyridin-2-yl}-1-(cyclopropylmethyl)-1H-indol-6-yl)piperidin-4-yl]-1,3-oxazolidin-2-one |
| | Analytical Data: 614.3; 1.479 min (Method E) |

Compounds of Examples 358 to 366 in Table 44 were prepared following a procedure similar to the preparation of a compound of Example 91, by using Intermediate 89D and tert-butyl (R)-piperidin-3-ylcarbamate in amide formation and with appropriate secondary amine in Buchwald coupling reaction.

**Table 44**

| Example | Structure |
|---|---|
| | Name |
| | Analytical Data: LC-MS m/z [M+H]⁺; HPLC retention time (HPLC method) |
| 358 | 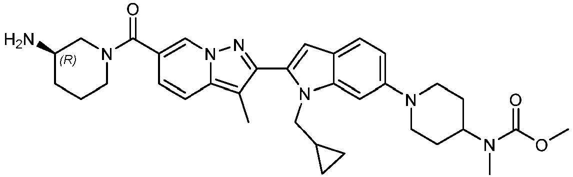 |
| | Methyl N-[1-(2-{6-[(3R)-3-aminopiperidine-1-carbonyl]-3-methylpyrazolo[1,5-a]pyridin-2-yl}-1-(cyclopropylmethyl)-1H-indol-6-yl)piperidin-4-yl]-N-methylcarbamate |
| | Analytical Data: 598.4; 1.170 min (Method E) |
| 359 | 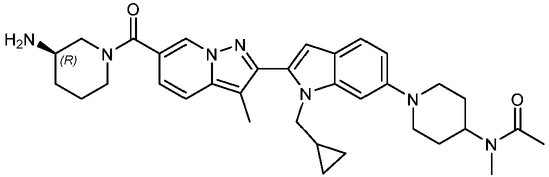 |
| | N-[1-(2-{6-[(3R)-3-Aminopiperidine-1-carbonyl]-3-methylpyrazolo[1,5-a]pyridin-2-yl}-1-(cyclopropylmethyl)-1H-indol-6-yl)piperidin-4-yl]-N-methylacetamide |
| | Analytical Data: 582.4; 1.488 min (Method E) |
| 360 | 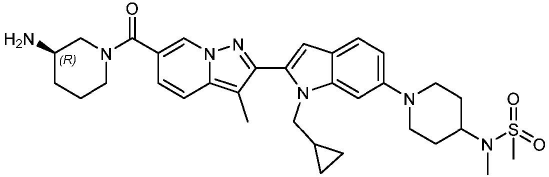 |
| | N-[1-(2-{6-[(3R)-3-Aminopiperidine-1-carbonyl]-3-methylpyrazolo[1,5-a]pyridin-2-yl}-1-(cyclopropylmethyl)-1H-indol-6-yl)piperidin-4-yl]-N-methylmethanesulfonamide |
| | Analytical Data: 618.4; 1.063min (Method E) |
| 361 | 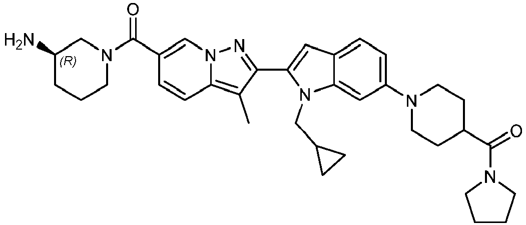 |
| | (3R)-1-{2-[1-(Cyclopropylmethyl)-6-[4-(pyrrolidine-1-carbonyl)piperidin-1-yl]-1H-indol-2-yl]-3-methylpyrazolo[1,5-a]pyridine-6-carbonyl}piperidin-3-amine |
| | Analytical Data: 608.3; 1.591min (Method E) |
| 362 | 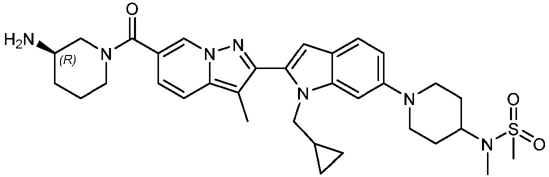 |
| | N-[1-(2-{6-[(3R)-3-Aminopiperidine-1-carbonyl]-3-methylpyrazolo[1,5-a]pyridin-2-yl}-1-(cyclopropylmethyl)-1H-indol-6-yl)piperidin-4-yl]-N-methylmethanesulfonamide |
| | Analytical Data: 618.4; 1.063min (Method E) |
| 363 | 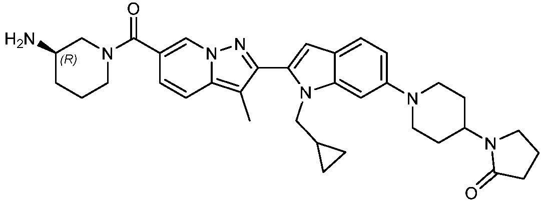 |
| | 1-[1-(2-{6-[(3R)-3-Aminopiperidine-1-carbonyl]-3-methylpyrazolo[1,5-a]pyridin-2-yl}-1-(cyclopropylmethyl)-1H-indol-6-yl)piperidin-4-yl]pyrrolidin-2-one |
| | Analytical Data: 594.3; 1.448 min (Method E) |
| 364 | 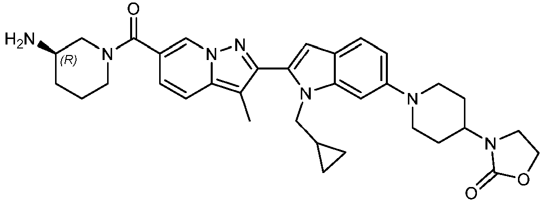 |
| | 3-[1-(2-{6-[(3R)-3-Aminopiperidine-1-carbonyl]-3-methylpyrazolo[1,5-a]pyridin-2-yl}-1-(cyclopropylmethyl)-1H-indol-6-yl)piperidin-4-yl]-1,3-oxazolidin-2-one |
| | Analytical Data: 596.3; 1.412 min (Method E) |
| 365 | 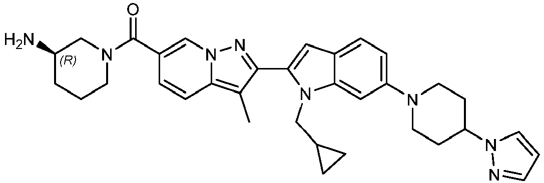 |
| | (3R)-1-{2-[1-(Cyclopropylmethyl)-6-[4-(1H-pyrazol-1-yl)piperidin-1-yl]-1H-indol-2-yl]-3-methylpyrazolo[1,5-a]pyridine-6-carbonyl}piperidin-3-amine |
| | Analytical Data: 577.3; 1.628min (Method E) |
| 366 | 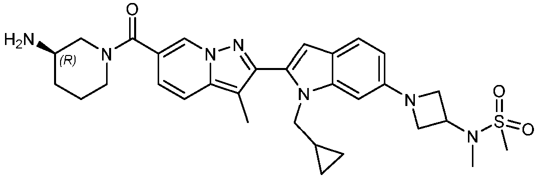 |
| | N-[1-(2-{6-[(3R)-3-Aminopiperidine-1-carbonyl]-3-methylpyrazolo[1,5-a]pyridin-2-yl }-1-(cyclopropylmethyl)-1H-indol-6-yl)azetidin-3-yl]-N-methylmethanesulfonamide |
| | Analytical Data: 590.3; 1.522min (Method E) |

Compounds of Examples 367 to 379 in Table 45 were prepared following a procedure similar to the preparation of a compound of Example 92, by Suzuki coupling of Intermediate 92A with appropriate boronic acid or boronate ester.

**Table 45**

| Example | Structure |
|---|---|
| | Name |
| | Analytical Data: LC-MS m/z [M+H]⁺; HPLC retention time (HPLC method) |
| 367 | 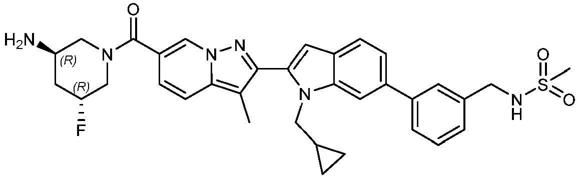 N-{[3-(2-{6-[(3R,5R)-3-Amino-5-fluoropiperidine-1-carbonyl]-3-methylpyrazolo[1,5-a]pyridin-2-yl}-1-(cyclopropylmethyl)-1H-indol-6-yl)phenyl]methyl}methanesulfonamide |
| | Analytical Data: 629.2; 1.775min (Method E) |
| 368 | 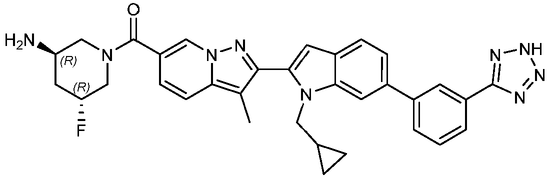 (3R,5R)-1-{2-[1-(Cyclopropylmethyl)-6-[3-(2H-1,2,3,4-tetrazol-5-yl)phenyl]-1H-indol-2-yl]-3-methylpyrazolo[1,5-a]pyridine-6-carbonyl}-5-fluoropiperidin-3-amine |
| | Analytical Data: 590.3; 1.369min (Method E) |
| 369 | 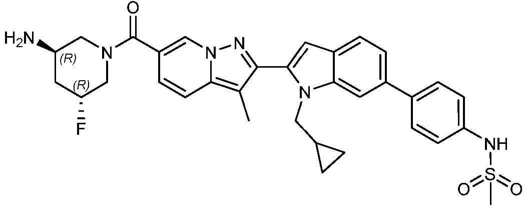 N-[4-(2-{6-[(3R,5R)-3-Amino-5-fluoropiperidine-1-carbonyl]-3-methylpyrazolo[1,5-a]pyridin-2-yl}-1-(cyclopropylmethyl)-1H-indol-6-yl)phenyl]methanesulfonamide |
| | Analytical Data: 615.2; 1.713min (Method E) |
| 370 | 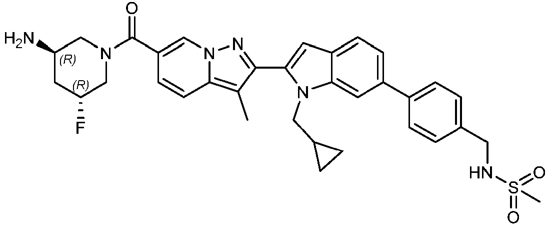 N-{[4-(2-{6-[(3R,5R)-3-Amino-5-fluoropiperidine-1-carbonyl]-3-methylpyrazolo[1,5-a]pyridin-2-yl}-1-(cyclopropylmethyl)-1H-indol-6-yl)phenyl]methyl}methanesulfonamide |
| | Analytical Data: 629.3; 1.738 min (Method E) |
| 371 | 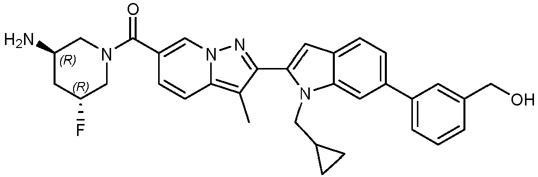 [3-(2-{6-[(3R,5R)-3-Amino-5-fluoropiperidine-1-carbonyl]-3-methylpyrazolo[1,5-a]pyridin-2-yl}-1-(cyclopropylmethyl)-1H-indol-6-yl)phenyl]methanol |
| | Analytical Data: 552.3; 1.742 min (Method E) |
| 372 | 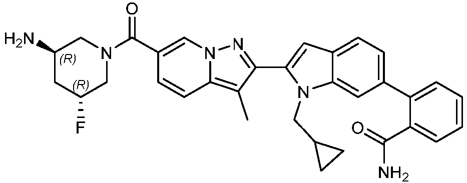 2-(2-{6-[(3R,5R)-3-Amino-5-fluoropiperidine-1-carbonyl]-3-methylpyrazolo[1,5-a]pyridin-2-yl}-1-(cyclopropylmethyl)-1H-indol-6-yl)benzamide |
| | Analytical Data: 565.3; 1.536 min (Method E) |
| 373 | 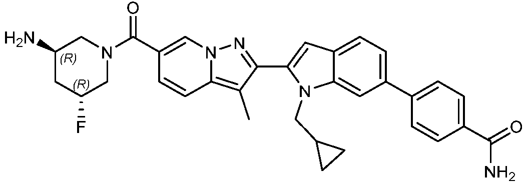 4-(2-{ 6-[(3R, SR)-3-Amino-5-fluoropiperidine-1-carbonyl]-3-methylpyrazolo[1,5-a]pyridin-2-yl}-1-(cyclopropylmethyl)-1H-indol-6-yl)benzamide |
| | Analytical Data: 565.2; 1.531 min (Method E) |
| 374 | 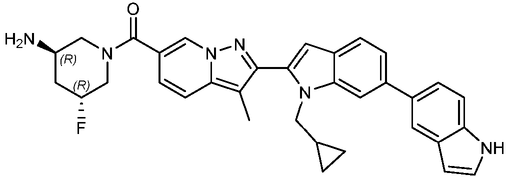 (3R,5R)-1-{2-[1'-(Cyclopropylmethyl)-1H,1'H-[5,6'-biindole]-2'-yl]-3-methylpyrazolo[1,5-a]pyridine-6-carbonyl}-5-fluoropiperidin-3-amine |
| | Analytical Data: 561.2; 1.943min (Method E) |
| 375 | 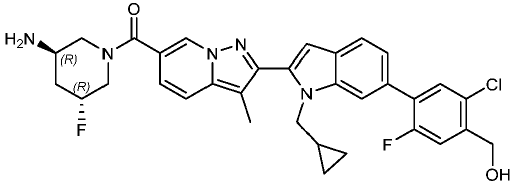 [4-(2-{ 6-[(3R, SR)-3-Amino-5-fluoropiperidine-1-carbonyl]-3-methylpyrazolo[1,5-a]pyridin-2-yl}-1-(cyclopropylmethyl)-1H-indol-6-yl)-2-chloro-5-fluorophenyl]methanol |
| | Analytical Data:604.3; 1.915 min (Method E) |
| 376 | 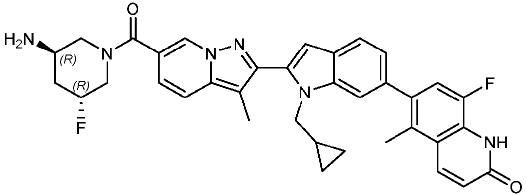 6-(2-{6-[(3R,5R)-3-Amino-5-fluoropiperidine-1-carbonyl]-3-methylpyrazolo[1,5-a]pyridin-2-yl}-1-(cyclopropylmethyl)-1H-indol-6-yl)-8-fluoro-5-methyl-1,2-dihydroquinolin-2-one |
| | Analytical Data: 621.3; 1.557 min (Method E) |
| 377 | 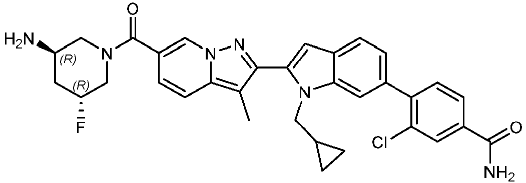 4-(2-{ 6-[(3R, SR)-3-Amino-5-fluoropiperidine-1-carbonyl]-3-methylpyrazolo[1,5-a]pyridin-2-yl}-1-(cyclopropylmethyl)-1H-indol-6-yl)-3-chlorobenzamide |
| | Analytical Data: 599.3; 1.676 min (Method E) |
| 378 | 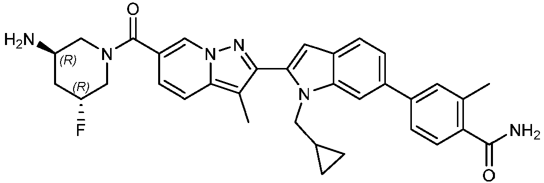 4-(2-{ 6-[(3R, SR)-3-Amino-5-fluoropiperidine-1-carbonyl]-3-methylpyrazolo[1,5-a]pyridin-2-yl}-1-(cyclopropylmethyl)-1H-indol-6-yl)-2-methylbenzamide |
| | Analytical Data: 579.4; 1.523min (Method E) |
| 379 | 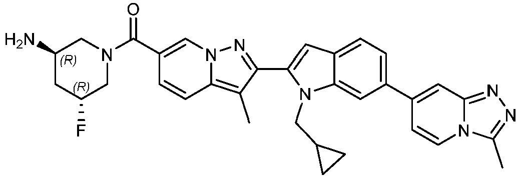 (3R,5R)-1-{2-[1-(Cyclopropylmethyl)-6-{3-methyl-[1,2,4]triazolo[4,3-a]pyridin-7-yl}-1H-indol-2-yl]-3-methylpyrazolo[1,5-a]pyridine-6-carbonyl}-5-fluoropiperidin-3-amine |
| | Analytical Data: 577.3; 1.501min (Method E) |

### Example 380

### 2-(2-{6-[(3R)-3-Aminopiperidine-1-carbonyl]-3-methylpyrazolo[1,5-a]pyridin-2-yl}-1-(cyclopropylmethyl)-1H-pyrrolo[2,3-b]pyridin-6-yl)propan-2-ol (Homochiral)

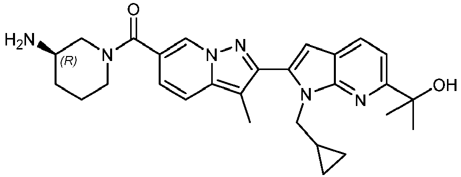

Compound of Example 380 was obtained by amide formation of Intermediate 64D with tert-butyl (R)-piperidin-3-ylcarbamate followed the similar preparation of a compound of Example 100. LC-MS m/z: 487.4 [M+H]⁺; HPLC retention time: 1.453min (Method E).

### Example 381

### 2-(2-{6-[(7R)-7-Amino-2-azabicyclo[2.2.1]heptane-2-carbonyl]-3-methylpyrazolo[1,5-a]pyridin-2-yl}-1-(cyclopropylmethyl)-1H-pyrrolo[2,3-b]pyridin-6-yl)-1,1-difluoropropan-2-ol (Diastereomeric mixture)

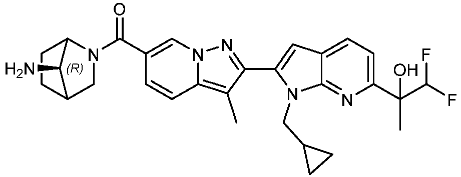

Compound of Example 381 was prepared following a procedure similar to the preparation of a compound of Example 101, by using Intermediate 100A with (Difluoromethyl)trimethylsilane reagent. LC-MS m/z: 535.2 [M+H]⁺; HPLC retention time: 1.503min (Method E).

### Example 382

### 2-(2-{6-[(3R)-3-Aminopiperidine-1-carbonyl]-3-methylpyrazolo[1,5-a]pyridin-2-yl}-1-(cyclopropylmethyl)-1H-pyrrolo[2,3-b]pyridin-6-yl)-1,1, 1-trifluoropropan-2-ol (Diastereomeric mixture)

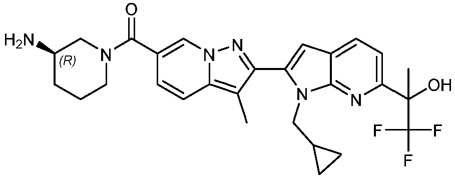

Compound of Example 382 was obtained by amide formation of Intermediate 64D with tert-butyl (R)-piperidin-3-ylcarbamate followed with the similar preparation of a compound of Example 101. LC-MS m/z: 541.3 [M+H]⁺; HPLC retention time: 1.746min (Method E).

### Example 383

### 2-(2-{6-[(7R)-7-Amino-2-azabicyclo[2.2.1]heptane-2-carbonyl]-4-fluoro-3-methylpyrazolo[1,5-a]pyridin-2-yl}-1-(cyclopropylmethyl)-1H-pyrrolo[2,3-b]pyridin-6-yl)-1,1,1-trifluoropropan-2-ol

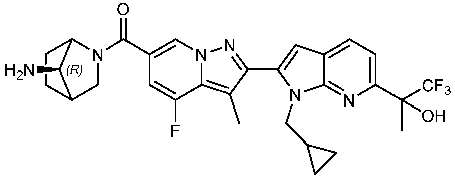

Compound of Example 383 obtained following a procedure similar to the preparation of Example 101, from Intermediate 39E. Analytical Data: LC-MS m/z: 571.3 [M+H]⁺; HPLC retention time: 2.147 min (Method E).

### Example 384

### 1-(2-{6-[(7R)-7-Amino-2-azabicyclo[2.2.1]heptane-2-carbonyl]-4-fluoro-3-methylpyrazolo[1,5-a]pyridin-2-yl}-1-(cyclopropylmethyl)-1H-pyrrolo[2,3-b]pyridin-6-yl)-1-cyclopropylethan-1-ol

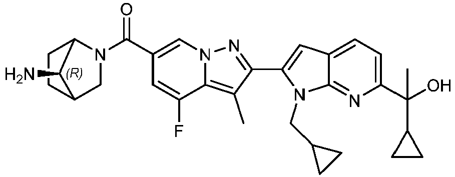

Compound of Example 384 was prepared following a procedure similar to the preparation of Example 102, from Intermediate 39E and using cyclopropyl magnesium bromide in Grignard addition reaction. LC-MS m/z: 543.2 [M+H]⁺; HPLC retention time: 1.812 min (Method E).

### Example 385

### 1-(2-{6-[(3R)-3-Aminopiperidine-1-carbonyl]-3-methylpyrazolo[1,5-a]pyridin-2-yl}-1-(cyclopropylmethyl)-1H-pyrrolo[2,3-b]pyridin-6-yl)ethan-1-ol (Diastereomeric mixture)

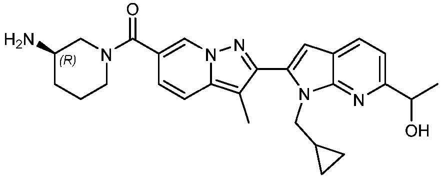

Compound of Example 385 was obtained by amide formation of Intermediate 64D with tert-butyl (R)-piperidin-3-ylcarbamate following the procedure to the preparation of Example 104. LC-MS m/z: 473.3 [M+H]⁺; HPLC retention time: 1.259min (Method E).

### Examples 386 and 387

### ((R)-3-Aminopiperidin-1-yl)(2-(1-(cyclopropylmethyl)-7-(1-(tetrahydro-2H-pyran-2-carbonyl)piperidin-4-yl)-1H-indol-2-yl)-4-methoxy-3-methylpyrazolo[1,5-a]pyridin-6-yl)methanone (isomer 1)

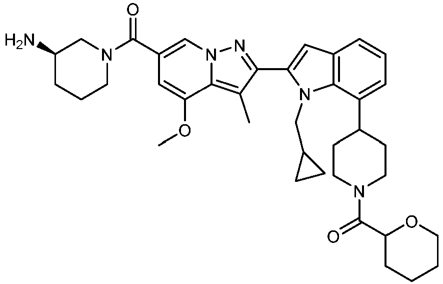

### Intermediate 386A

### Methyl 2-(7-(1-(tert-butoxycarbonyl)piperidin-4-yl)-1-(cyclopropylmethyl)-1H-indol-2-yl)-4-methoxy-3-methylpyrazolo[1,5-a]pyridine-6-carboxylate

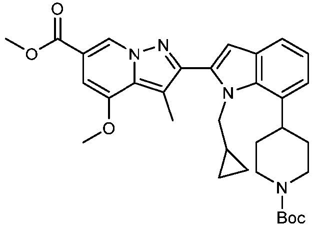

Intermediate 1 (1.042 g, 5.69 mmol) and copper(I) iodide (0.542 g, 2.84 mmol) were added to a stirred solution of Intermediate 30 (2.5 g, 5.69 mmol) in DMF (5 ml). The reaction mixture was heated to 80°C for 48 hours, allowed to cool to a room temperature, diluted with water (100 m), and extracted with ethyl acetate (2×100 ml). The combined organic layers were dried over sodium sulphate, filtered, and concentrated under reduced pressure to obtain a crude product, which was purified using silica a gel column (0-20% ethyl acetate in hexane) to afford methyl 2-(7-(1-(tert-butoxycarbonyl)piperidin-4-yl)-1-(cyclopropylmethyl)-1H-indol-2-yl)-4-methoxy-3-methylpyrazolo[1,5-a]pyridine-6-carboxylate (1.6 g, 49%). LC-MS 573.3 [M+H]⁺; ¹H NMR (400 MHz, chloroform-*d*) δ ppm: 8.84-8.86 (s, 1H), 7.53-7.61 (m, 1H), 7.14-7.19 (m, 2H), 6.87 (s, 1H), 6.77 (s, 1H), 4.44 (d, *J*=6.50 Hz, 2H), 4.04 (s, 3H), 4.01-3.97 (m, 4H), 3.93 (s, 3H), 2.89-3.00 (m, 1H), 2.55 (s, 3H), 2.08 - 2.05 (m, 2H), 1.76-1.94 (m, 2H), 1.53 (s, 9 H), 0.88-1.03 (m, 1H), 0.17-0.33 (m, 2H), 0.18-0.06 (m, 2H).

### Intermediate 386B

### Methyl 2-(1-(cyclopropylmethyl)-7-(piperidin-4-yl)-1H-indol-2-yl)-4-methoxy-3-methylpyrazolo[1,5-a]pyridine-6-carboxylate

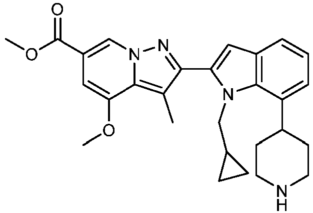

TFA (0.210 ml, 2.73 mmol) was added to a stirred solution of Intermediate 386A (1.6 g, 2.73 mmol) in DCM (15 ml) at 0°C. The reaction mixture was stirred for 3 hours at a room temperature, and concentrated under reduced pressure to obtain methyl 2-(1-(cyclopropylmethyl)-7-(piperidin-4-yl)-1H-indol-2-yl)-4-methoxy-3-methylpyrazolo[1,5-a]pyridine-6-carboxylate (1.1 g, 85%). LC-MS m/z: 473.4 [M+H] ⁺.

### Intermediate 386C

### Methyl 2-(1-(cyclopropylmethyl)-7-(1-(tetrahydro-2H-pyran-2-carbonyl)piperidin-4-yl)-1H-indol-2-yl)-4-methoxy-3-methylpyrazolo[1,5-a]pyridine-6-carboxylate

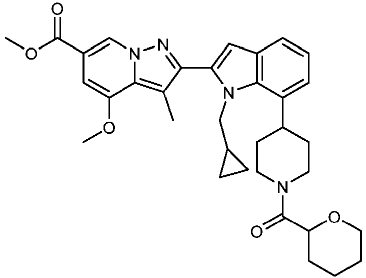

Racemic tetrahydro-2H-pyran-2-carboxylic acid (55.1 mg, 0.423 mmol), DIPEA (0.222 ml, 1.270 mmol) and HATU (241 mg, 0.635 mmol) were added to a stirred solution of Intermediate 386B (200 mg, 0.423 mmol) in DMF (2 ml). The reaction mixture was stirred for 3 hours, quenched with water, the solids obtained were filtered and dried in vacuo to obtain methyl 2-(1-(cyclopropylmethyl)-7-(1-(tetrahydro-2H-pyran-2-carbonyl) piperidin-4-yl)-1H-indol-2-yl)-4-methoxy-3-methylpyrazolo[1,5-a] pyridine-6-carboxylate (180 mg, 73%). LC-MS m/z: 585.4 [M+H]⁺.

### Intermediate 386D

### 2-(1-(Cyclopropylmethyl)-7-(1-(tetrahydro-2H-pyran-2-carbonyl)piperidin-4-yl)-1H-indol-2-yl)-4-methoxy-3-methylpyrazolo[1,5-a]pyridine-6-carboxylic acid

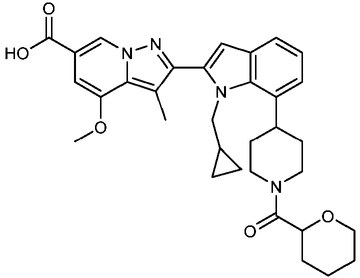

LiOH (7.37 mg, 0.308 mmol) was added to a stirred solution of Intermediate 386C (180 mg, 0.308 mmol) in a mixture of solvents [THF (1 ml), methanol (1.0 ml) and water (1 ml)]. The reaction mixture was stirred for 4 hours, concentrated under reduced pressure to obtain crude salts, which were acidified with 1 N HCl. The solids thus obtained were filtered through Buchner funnel and dried under vacuo to obtain 2-(1-(cyclopropylmethyl)-7-(1-(tetrahydro-2H-pyran-2-carbonyl)piperidin-4-yl)-1H-indol-2-yl)-4-methoxy-3-methylpyrazolo[1,5-a]pyridine-6-carboxylic acid (160 mg, 91%). LC-MS m/z: 571.2 [M+H]⁺.

### Intermediate 386E & Intermediate 386F

### tert-Butyl ((3R)-1-(2-(1-(cyclopropylmethyl)-7-(1-(tetrahydro-2H-pyran-2-carbonyl)piperidin-4-yl)-1H-indol-2-yl)-4-methoxy-3-methylpyrazolo[1,5-a]pyridine-6-carbonyl)piperidin-3-yl)carbamate

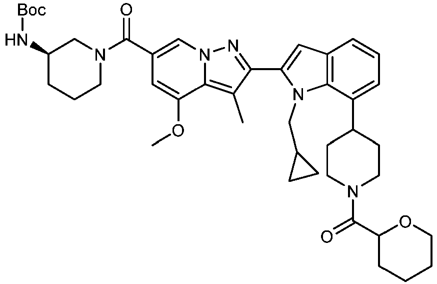

tert-Butyl (R)-piperidin-3-ylcarbamate (35.1 mg, 0.175 mmol), DIPEA (0.092 ml, 0.526 mmol) and HATU (100 mg, 0.263 mmol) were added to a stirred solution of Intermediate 386D (100 mg, 0.175 mmol) in DMF (1 ml). The reaction mixture was stirred for 3 hours, quenched with water (50 ml), and the solids obtained were filtered and dried to obtain a crude product, which was purified by SFC chiral purification to afford corresponding homochiral isomers. (*Preparative SFC Conditions* Column/dimensions: Whelk (R, R) (250×30) mm, 5u% CO₂: 60%, Co solvent: 40% of 5 mM Ammonium Acetate in [MeOH + ACN(1:1)], total flow: 170.0 g/min, back Pressure: 100 bar, temperature: 40°C, UV: 225 nm.

*Isomer 1* (Intermediate 386E): tert-butyl ((3R)-1-(2-(1-(cyclopropylmethyl)-7-(1-(tetrahydro-2H-pyran-2-carbonyl)piperidin-4-yl)-1H-indol-2-yl)-4-methoxy-3-methylpyrazolo[1,5-a]pyridine-6-carbonyl)piperidin-3-yl)carbamate (40 mg, 61%). LC-MS m/z: 753.7 [M+H]⁺.

*Isomer 2* (Intermediate 386F): tert-butyl ((3R)-1-(2-(1-(cyclopropylmethyl)-7-(1-(tetrahydro-2H-pyran-2-carbonyl)piperidin-4-yl)-1H-indol-2-yl)-4-methoxy-3-methylpyrazolo[1,5-a]pyridine-6-carbonyl)piperidin-3-yl)carbamate (40 mg, 61%). LC-MS m/z: 753.7 [M+H]⁺.

### Example 386

### ((R)-3-Aminopiperidin-1-yl)(2-(1-(cyclopropylmethyl)-7-(1-(tetrahydro-2H-pyran-2-carbonyl)piperidin-4-yl)-1H-indol-2-yl)-4-methoxy-3-methylpyrazolo[1,5-a]pyridin-6-yl)methanone (isomer 1)

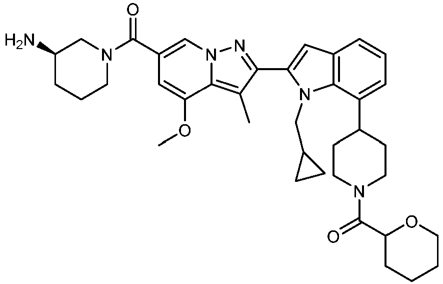

TFA (0.053 mmol) was added to a stirred solution of 386E (40 mg, 0.053 mmol) in DCM (1 ml) at 0°C. The reaction mixture was allowed to warm to a room temperature and stirred for 3 hours, concentrated in vacuo to obtain a crude product, which was purified using preparative LC/MS with the following conditions: Column: Waters X Bridge C18, 150 mm×19 mm, 5-µm particles; mobile phase A: 5:95 acetonitrile: water with 0.1% trifluoroacetic acid; mobile phase B: 95:5 acetonitrile: water with 0.1% trifluoroacetic acid; gradient: a 0-minute hold at 20% B, 20-40% B over 20 minutes, then a 5-minute hold at 100% B; flow rate: 20 ml/min, column temperature: 25°C. Fraction collection was triggered by signals. Fractions containing the desired product were combined and dried via centrifugal evaporation. The purified material was diluted with a 1:1 mixture of ethylene dichloride and methanol (EDM), treated with Si-Pyridine and shaken for a minimum of 2 hours, the resulting mixture was filtered and dried via centrifugal evaporation.to afford ((R)-3-aminopiperidin-1-yl) (2-(1-(cyclopropylmethyl)-7-(1-(tetrahydro-2H-pyran-2-carbonyl)piperidin-4-yl)-1H-indol-2-yl)-4-methoxy-3-methylpyrazolo[1,5-a]pyridin-6-yl)methanone (isomer 1, 3 mg, 8%). LC-MS m/z: 653.5 [M+H]⁺. LCMS retention time: 1.825 min (method E). ¹H NMR (400 MHz, DMSO-*d*₆) δ ppm: 8.46 (s, 1H), 7.49 (d, *J*=6.69 Hz, 1H), 7.08 (m, 2H), 6.72 (s, 1H), 6.62 (s, 1H), 4.62 - 4.57 (m, 1H), 4.41 (d, *J*=6.11 Hz, 2H), 4.24 - 4.14 (m, 2H), 3.99 (s, 3H), 3.93 - 3.85 (m, 2H), 3.68 - 3.58 (m, 1H), 3.55 - 3.45 (m, 1H), 3.30 - 3.20 (m, 4H), 2.53-2.56 (m, 2H), 2.47 (s, 3H), 2.05 - 1.95 (m, 4H), 1.87 - 1.72 (m, 4H), 1.70 - 1.55 (m, 6H), 1.53 - 1.44 (m, 2H), 1.0 - 0.9 (m, 1H), 0.23 (d, *J*=8.31 Hz, 2H), -0.18 (m, 2H).

### Example 387

### ((R)-3-Aminopiperidin-1-yl)(2-(1-(cyclopropylmethyl)-7-(1-(tetrahydro-2H-pyran-2-carbonyl)piperidin-4-yl)-1H-indol-2-yl)-4-methoxy-3-methylpyrazolo[1,5-a]pyridin-6-yl)methanone (isomer 2)

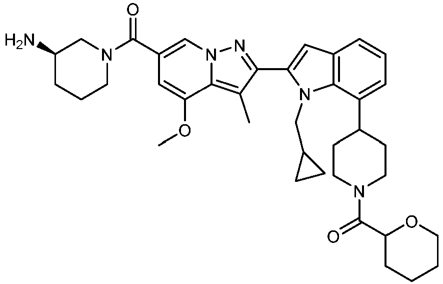

Compound of Example 387 was prepared in a similar fashion like Example 386 using the intermediate 386F.

Compounds of Examples 388 to 412 in Table 46 were prepared following a procedure similar to the preparation of a compound of Example 386, by acid amine coupling of Intermediate 386B with appropriate carboxylic acid and amines.

**Table 46**

| Example | Structure |
|---|---|
| | Name |
| | Analytical Data: LC-MS m/z [M+H]⁺; HPLC retention time (HPLC method) |
| 388 | 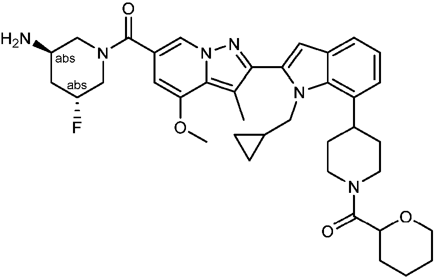 ((3R,5R)-3-Amino-5-fluoropiperidin-1-yl)(2-(1-(cyclopropylmethyl)-7-(1-(tetrahydro-2H-pyran-2-carbonyl)piperidin-4-yl)-1H-indol-2-yl)-4-methoxy-3-methylpyrazolo[1,5-a]pyridin-6-yl)methanone (isomer 1) |
| | Analytical Data: 671.; 1.953 min (Method E) |
| 389 | 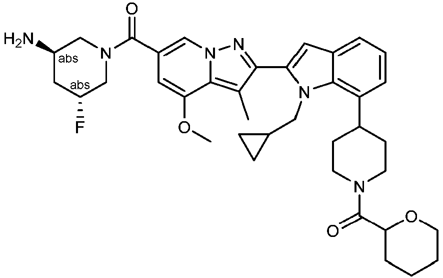 ((3R,5R)-3-Amino-5-fluoropiperidin-1-yl)(2-(1-(cyclopropylmethyl)-7-(1-(tetrahydro-2H-pyran-2-carbonyl)piperidin-4-yl)-1H-indol-2-yl)-4-methoxy-3-methylpyrazolo[1,5-a]pyridin-6-yl)methanone (isomer 2) |
| | Analytical Data: 671.4; 1.951 min (Method E) |
| 390 | 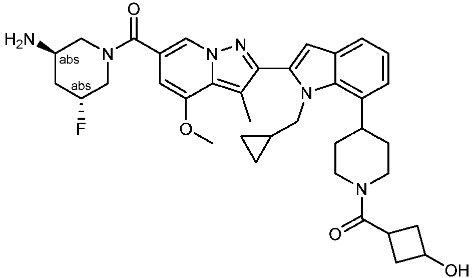 ((3R,5R)-3-Amino-5-fluoropiperidin-1-yl)(2-(1-(cyclopropylmethyl)-7-(1-(3-hydroxycyclobutane-1-carbonyl)piperidin-4-yl)-1H-indol-2-yl)-4-methoxy-3-methylpyrazolo[1,5-a]pyridin-6-yl)methanone (isomer 1) |
| | Analytical Data: 657.3; 1.502 min (Method E) |
| 391 | 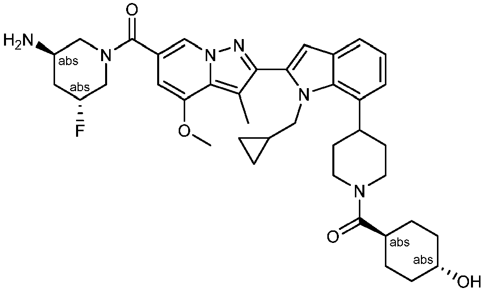 ((3R,5R)-3-Amino-5-fluoropiperidin-1-yl)(2-(1-(cyclopropylmethyl)-7-(1-((1r,4r)-4-hydroxycyclohexane-1-carbonyl)piperidin-4-yl)-1H-indol-2-yl)-4-methoxy-3-methylpyrazolo[1,5-a]pyridin-6-yl)methanone |
| | Analytical Data: 685.3; 1.625 min (Method E) |
| 392 | 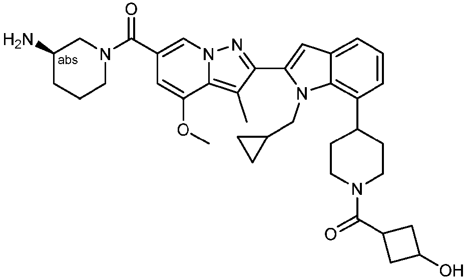 (R)-(3-Aminopiperidin-1-yl)(2-(1-(cyclopropylmethyl)-7-(1-(3-hydroxycyclobutane-1-carbonyl)piperidin-4-yl)-1H-indol-2-yl)-4-methoxy-3-methylpyrazolo[1,5-a]pyridin-6-yl)methanone (isomer 1) |
| | Analytical Data: 639.3; 1.512 min (Method E) |
| 393 | 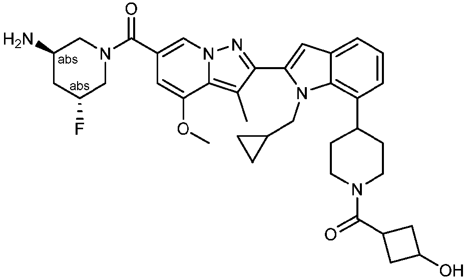 ((3R,5R)-3-Amino-5-fluoropiperidin-1-yl)(2-(1-(cyclopropylmethyl)-7-(1-(3-hydroxycyclobutane-1-carbonyl)piperidin-4-yl)-1H-indol-2-yl)-4-methoxy-3-methylpyrazolo[1,5-a]pyridin-6-yl)methanone (isomer 2) |
| | Analytical Data: 657.3; 1.534 min (Method E) |
| 394 | 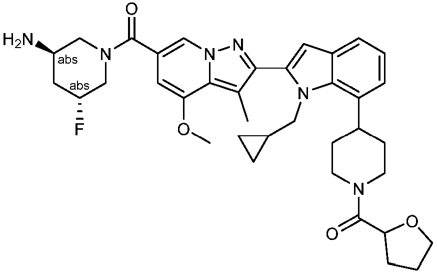 ((3R,5R)-3-Amino-5-fluoropiperidin-1-yl)(2-(1-(cyclopropylmethyl)-7-(1-(tetrahydrofuran-2-carbonyl)piperidin-4-yl)-1H-indol-2-yl)-4-methoxy-3-methylpyrazolo[1,5-a]pyridin-6-yl)methanone (isomer 1) |
| | Analytical Data: 657.3; 1.664 min (Method E) |
| 395 | 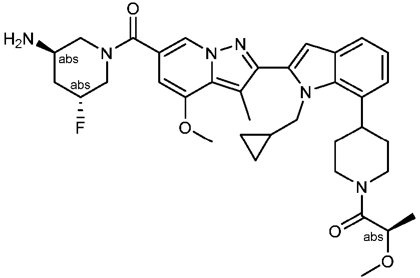 (R)-1-(4-(2-(6-((3R,5R)-3-Amino-5-fluoropiperidine-1-carbonyl)-4-methoxy-3-methylpyrazolo[1,5-a]pyridin-2-yl)-1-(cyclopropylmethyl)-1H-indol-7-yl)piperidin-1-yl)-2-methoxypropan-1-one |
| | Analytical Data: 645.3; 1.735 min (Method E) |
| 396 | 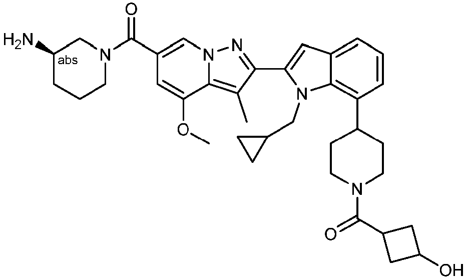 (R)-(3-Aminopiperidin-1-yl)(2-(1-(cyclopropylmethyl)-7-(1-(3-hydroxycyclobutane-1-carbonyl)piperidin-4-yl)-1H-indol-2-yl)-4-methoxy-3-methylpyrazolo[1,5-a]pyridin-6-yl)methanone (isomer 1) |
| | Analytical Data: 639.3; 1.520 min (Method E) |
| 397 | 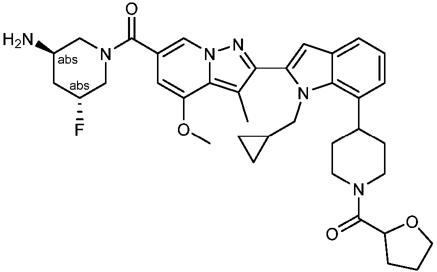 ((3R,5R)-3-Amino-5-fluoropiperidin-1-yl)(2-(1-(cyclopropylmethyl)-7-(1-(tetrahydrofuran-2-carbonyl)piperidin-4-yl)-1H-indol-2-yl)-4-methoxy-3-methylpyrazolo[1,5-a]pyridin-6-yl)methanone (isomer 2) |
| | Analytical Data: 657.3; 1.676 min (Method E) |
| 398 | 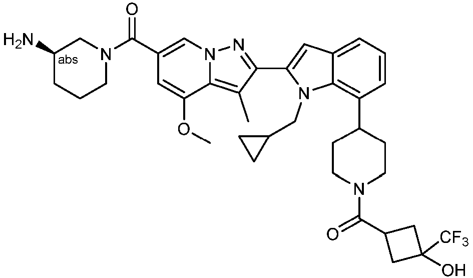 (R)-(3-Aminopiperidin-1-yl)(2-(1-(cyclopropylmethyl)-7-(1-(3-hydroxy-3-(trifluoromethyl)cyclobutane-1-carbonyl)piperidin-4-yl)-1H-indol-2-yl)-4-methoxy-3-methylpyrazolo[1,5-a]pyridin-6-yl)methanone (mixture of diastereomers) |
| | Analytical Data: 707.3; 1.774 min (Method E) |
| 399 | 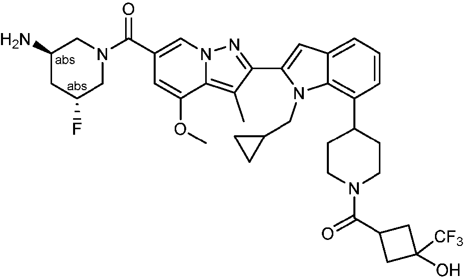 ((3R,5R)-3-Amino-5-fluoropiperidin-1-yl)(2-(1-(cyclopropylmethyl)-7-(1-(3-hydroxy-3-(trifluoromethyl)cyclobutane-1-carbonyl)piperidin-4-yl)-1H-indol-2-yl)-4-methoxy-3-methylpyrazolo[1,5-a]pyridin-6-yl)methanone (mixture of diastereomers) |
| | Analytical Data: 725.3; 1.793 min (Method E) |
| 400 | 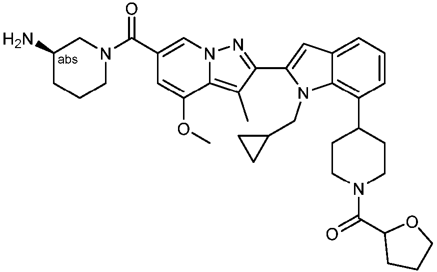 ((R)-3-Aminopiperidin-1-yl)(2-(1-(cyclopropylmethyl)-7-(1-(tetrahydrofuran-2-carbonyl)piperidin-4-yl)-1H-indol-2-yl)-4-methoxy-3-methylpyrazolo[1,5-a]pyridin-6-yl)methanone (mixture of diastereomers) |
| | Analytical Data: 639.3; 1.657 min (Method E) |
| 401 | 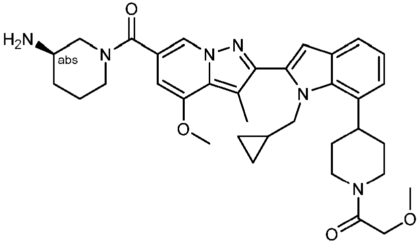 (R)-1-(4-(2-(6-(3-Aminopiperidine-1-carbonyl)-4-methoxy-3-methylpyrazolo[1,5-a]pyridin-2-yl)-1-(cyclopropylmethyl)-1H-indol-7-yl)piperidin-1-yl)-2-methoxyethan-1-one |
| | Analytical Data: 613.3; 1.565 min (Method E) |
| 402 | 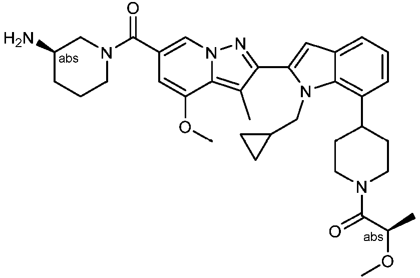 (R)-1-(4-(2-(6-((R)-3-Aminopiperidine-1-carbonyl)-4-methoxy-3-methylpyrazolo[1,5-a]pyridin-2-yl)-1-(cyclopropylmethyl)-1H-indol-7-yl)piperidin-1-yl)-2-methoxypropan-1-one |
| | Analytical Data: 627.3; 1.603 min (Method E) |
| 403 | 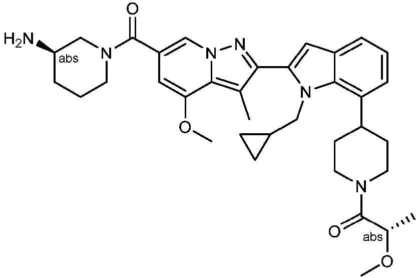 (S)-1-(4-(2-(6-((R)-3-Aminopiperidine-1-carbonyl)-4-methoxy-3-methylpyrazolo[1,5-a]pyridin-2-yl)-1-(cyclopropylmethyl)-1H-indol-7-yl)piperidin-1-yl)-2-methoxypropan-1-one |
| | Analytical Data: 627.3; 1.698 min (Method E) |
| 404 | 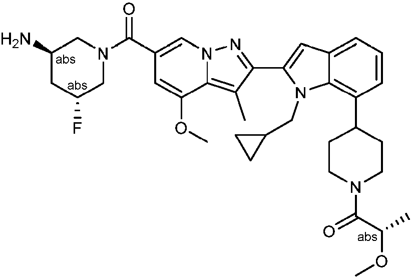 (S)-1-(4-(2-(6-((3R,5R)-3-Amino-5-fluoropiperidine-1-carbonyl)-4-methoxy-3-methylpyrazolo[1,5-a]pyridin-2-yl)-1-(cyclopropylmethyl)-1H-indol-7-yl)piperidin-1-yl)-2-methoxypropan-1-one |
| | Analytical Data: 645.3; 1.751min (Method E) |
| 405 | 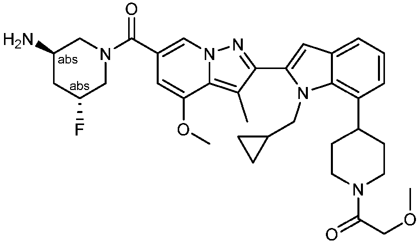 1-(4-(2-(6-((3R,5R)-3-Amino-5-fluoropiperidine-1-carbonyl)-4-methoxy-3-methylpyrazolo[1,5-a]pyridin-2-yl)-1-(cyclopropylmethyl)-1H-indol-7-yl)piperidin-1-yl)-2-methoxyethan-1-one |
| | Analytical Data: 631.3; 1.681 min (Method E) |
| 406 | 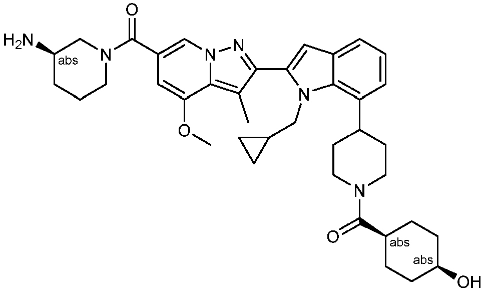 ((R)-3-Aminopiperidin-1-yl)(2-(1-(cyclopropylmethyl)-7-(1-((1s,4s)-4-hydroxycyclohexane-1-carbonyl)piperidin-4-yl)-1H-indol-2-yl)-4-methoxy-3-methylpyrazolo[1,5-a]pyridin-6-yl)methanone |
| | Analytical Data: 667.3; 1.672 min (Method E) |
| 407 | 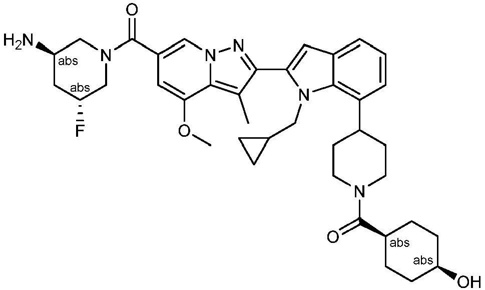 ((3R,5R)-3-Amino-5-fluoropiperidin-1-yl)(2-(1-(cyclopropylmethyl)-7-(1-((1s,4s)-4-hydroxycyclohexane-1-carbonyl)piperidin-4-yl)-1H-indol-2-yl)-4-methoxy-3-methylpyrazolo[1,5-a]pyridin-6-yl)methanone |
| | Analytical Data: 685.3; 1.721 min (Method E) |
| 408 | 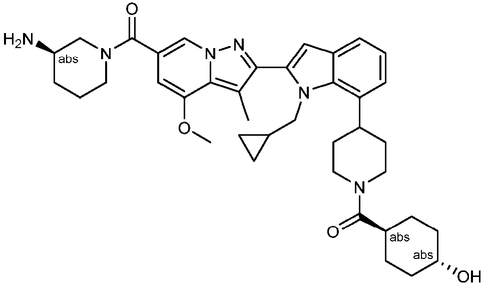 ((R)-3-Aminopiperidin-1-yl)(2-(1-(cyclopropylmethyl)-7-(1-((1r,4r)-4-hydroxycyclohexane-1-carbonyl)piperidin-4-yl)-1H-indol-2-yl)-4-methoxy-3-methylpyrazolo[1,5-a]pyridin-6-yl)methanone |
| | Analytical Data: 667.3; 1.583 min (Method E) |
| 409 | 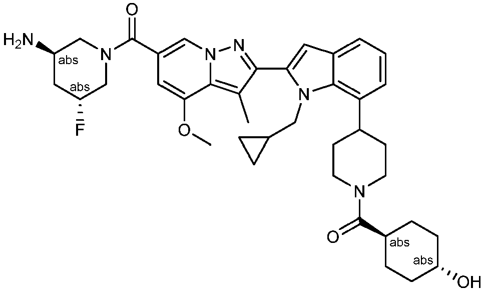 ((3R,5R)-3-Amino-5-fluoropiperidin-1-yl)(2-(1-(cyclopropylmethyl)-7-(1-((1r,4r)-4-hydroxycyclohexane-1-carbonyl)piperidin-4-yl)-1H-indol-2-yl)-4-methoxy-3-methylpyrazolo[1,5-a]pyridin-6-yl)methanone |
| | Analytical Data: 685.3; 1.625 min (Method E) |
| 410 | 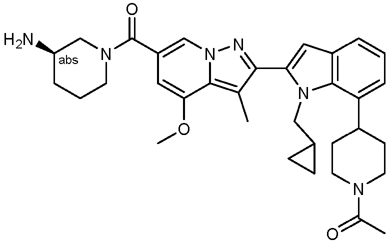 (R)-1-(4-(2-(6-(3-Aminopiperidine-1-carbonyl)-4-methoxy-3-methylpyrazolo[1,5-a]pyridin-2-yl)-1-(cyclopropylmethyl)-1H-indol-7-yl)piperidin-1-yl)ethan-1-one |
| | Analytical Data: 583.4; 1.641 min (Method E) |
| 411 | 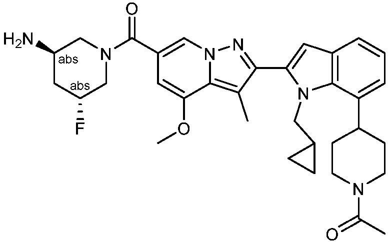 1-(4-(2-(6-((3R,5R)-3-Amino-5-fluoropiperidine-1-carbonyl)-4-methoxy-3-methylpyrazolo[1,5-a]pyridin-2-yl)-1-(cyclopropylmethyl)-1H-indol-7-yl)piperidin-1-yl)ethan-1-one |
| | Analytical Data: 601.4; 1.696 min (Method E) |
| 412 | 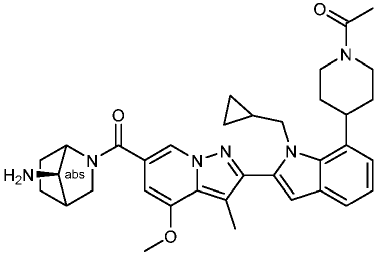 1-(4-(2-(6-((7R)-7-Amino-2-azabicyclo[2.2.1]heptane-2-carbonyl)-4-methoxy-3-methylpyrazolo[1,5-a]pyridin-2-yl)-1-(cyclopropylmethyl)-1H-indol-7-yl)piperidin-1-yl)ethan-1-one |
| | Analytical Data: 595.4; 8.079 min (Method D) |

### Example 413

### (R)-1-(4-(2-(6-(3-Aminopiperidine-1-carbonyl)-4-fluoro-3-methylpyrazolo[1,5-a]pyridin-2-yl)-1-(cyclopropylmethyl)-1H-indol-7-yl)piperidin-1-yl)ethan-1-one

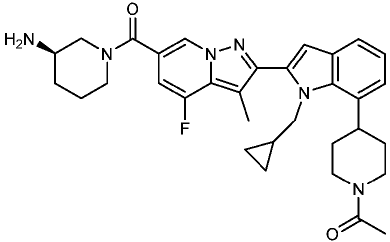

### Intermediate 413A

### Methyl 2-(7-(1-(tert-butoxycarbonyl)piperidin-4-yl)-1-(cyclopropylmethyl)-1H-indol-2-yl)-4-fluoro-3-methylpyrazolo[1,5-a]pyridine-6-carboxylate

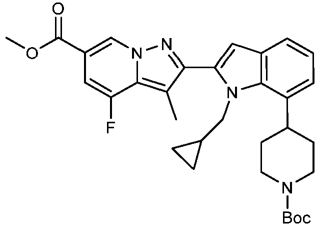

Copper(I) iodide (0.260 g, 1.365 mmol) was added to a stirred solution of Intermediate 9 (3.11 g, 18.20 mmol) and Intermediate 30 (2.0 g, 4.55 mmol) in DMF (40 ml). The reaction mixture was heated to 80°C for 16 hours, filtered through a celite bed, and washed with ethyl acetate. The combined filtrate was washed with water (2×100 ml), followed by 10% sodium bicarbonate solution. The organic layer was dried over anhydrous sodium sulphate and evaporated under reduced pressure to obtain a crude product, which was purified using a silica gel column (0-15% ethyl acetate in pet ether) to afford methyl 2-(7-(1-(tert-butoxycarbonyl)piperidin-4-yl)-1-(cyclopropylmethyl)-1H-indol-2-yl)-4-fluoro-3-methylpyrazolo[1,5-a]pyridine-6-carboxylate (0.38 g, 15%) as a gummy liquid. LC-MS m/z: 561.4 [M+H]⁺. ¹H NMR (400 MHz, DMSO-*d₆*) δ ppm: 9.14 (s, 1H), 9.15-9.13 (m, 1H), 7.52 (dd, *J*=1.3, 7.8 Hz, 1H), 7.42-7.35 (m, 1H), 7.22-7.04 (m, 3H), 6.85 (s, 1H), 4.44 (d, *J*=6.5 Hz, 2H), 4.15 (br, s, 2H), 3.94-3.90 (m, 3H), 2.49-2.46 (m, 4H), 2.41 (s, 1H), 2.00-1.83 (m, 3H), 1.78-1.56 (m, 3H), 1.53-1.35 (m, 9H), 1.26 (br, d, *J*=8.5 Hz, 2H), 0.99-0.80 (m, 1H), 0.25-0.21 (m, 2H), -0.01--0.18 (m, 2H).

### Intermediate 413B

### Methyl 2-(1-(cyclopropylmethyl)-7-(piperidin-4-yl)-1H-indol-2-yl)-4-fluoro-3-methylpyrazolo[1,5-a]pyridine-6-carboxylate

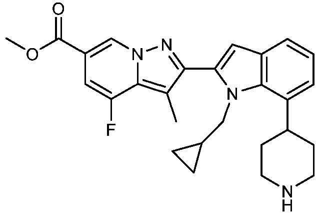

TFA (0.254 ml, 3.30 mmol) was added to a stirred solution of Intermediate 413A (0.37g, 0.660 mmol) in DCM (2 ml). The reaction mixture was stirred for 1 hour, and concentrated under reduced pressure to obtain a crude product, which was neutralized using 10% sodium bicarbonate solution and the contents were extracted with ethyl acetate (4×25 ml). The combined organic layers were washed with water and brine solution, dried over anhydrous sodium sulphate, and evaporated under reduced pressure to obtain methyl 2-(1-(cyclopropylmethyl)-7-(piperidin-4-yl)-1H-indol-2-yl)-4-fluoro-3-methylpyrazolo[1,5-a]pyridine-6-carboxylate (0.29 g, 95%). LC-MS m/z: 461.3 [M+H]⁺.

### Intermediate 413C

### Methyl 2-(7-(1-acetylpiperidin-4-yl)-1-(cyclopropylmethyl)-1H-indol-2-yl)-4-fluoro-3-methylpyrazolo[1,5-a]pyridine-6-carboxylate

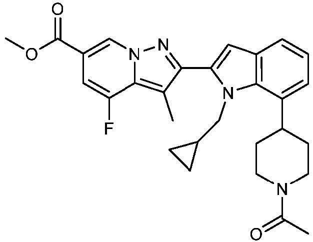

Triethylamine (0.039 ml, 0.282 mmol) was added to a stirred solution of Intermediate 413B (0.13 g, 0.282 mmol) in DCM (3 ml), followed by the addition of acetic anhydride (0.029 g, 0.282 mmol) at 0°C. The reaction mixture was allowed to warm to a room temperature and stirred for1 hour, diluted with water, and extracted with DCM (3×15 ml). The combined organic layers were washed with water and brine solution, dried over anhydrous sodium sulphate, and evaporated under reduced pressure to obtain methyl 2-(7-(1-acetylpiperidin-4-yl)-1-(cyclopropylmethyl)-1H-indol-2-yl)-4-fluoro-3-methylpyrazolo[1,5-a]pyridine-6-carboxylate (0.12 g, 85%) as gummy liquid, which was used further without purification. LC-MS m/z: 503.4 [M+H]⁺.

### Intermediate 413D

### 2-(7-(1-Acetylpiperidin-4-yl)-1-(cyclopropylmethyl)-1H-indol-2-yl)-4-fluoro-3-methylpyrazolo[1,5-a]pyridine-6-carboxylic acid

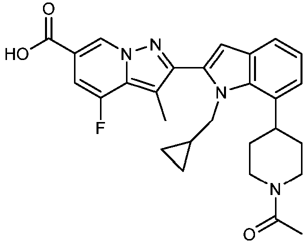

Intermediate 413D (0.1 g, 94%) was prepared following a procedure similar to the preparation of Intermediate 386D. LC-MS m/z: 489.4 [M+H]⁺

### Intermediate 413E

### tert-butyl (R)-(1-(2-(7-(1-acetylpiperidin-4-yl)-1-(cyclopropylmethyl)-1H-indol-2-yl)-4-fluoro-3-methylpyrazolo[1,5-a]pyridine-6-carbonyl)piperidin-3-yl)carbamate

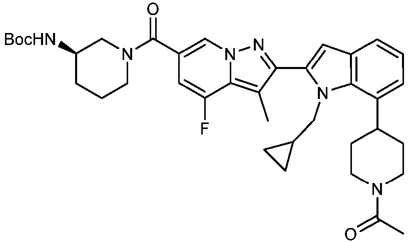

Intermediate 413E was prepared following a procedure similar to the preparation of Intermediate 386E (0.07 g, 85%) as a gummy liquid. LC-MS m/z: 671.5[M+H]⁺

### Example 413

### (R)-1-(4-(2-(6-(3-aminopiperidine-1-carbonyl)-4-fluoro-3-methylpyrazolo[1,5-a]pyridin-2-yl)-1-(cyclopropylmethyl)-1H-indol-7-yl)piperidin-1-yl)ethan-1-one

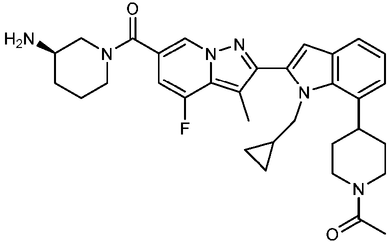

(R)-1-(4-(2-(6-(3-aminopiperidine-1-carbonyl)-4-fluoro-3-methylpyrazolo[1,5-a]pyridin-2-yl)-1-(cyclopropylmethyl)-1H-indol-7-yl)piperidin-1-yl)ethan-1-one was prepared starting from intermediate 413 E following a procedure similar to the preparation of Example 386. LC-MS m/z: 571.3 [M+H]⁺. LCMS retention time: 1.64 min (method E). ¹H NMR (400 MHz, DMSO-*d₆*) δ ppm: 8.74 (s, 1H), 7.51 (dd, *J*=7.50, 1.00 Hz, 1H), 7.04-7.22 (m, 3H), 6.79 (s, 1H), 4.43 (d, *J*=6.00 Hz, 2H), 4.1 - 3.9 (m, 2H), 3.70-3.55 (m, 2H), 3.20-3.07 (m, 2H), 3.07 - 2.86 (m, 2H), 2.85-2.62 (m, 2H), 2.07 (s, 3H), 2.05 - 1.95 (m, 2H), 1.92 (s, 3H), 1.90 - 1.77 (m, 2H), 1.75 - 1.35 (m, 4H), 1.24-1.22 (m, 2H), 0.97 - 0.93 (m, 1H), 0.26 - 0.22 (m, 2H), -0.17--0.13 (m, 2H).

Compounds of Examples 414 to 429 in Table 47 were preapred following a procedure similar to the preparation of a compound of Example 413, by acid amine coupling of Intermediate 413B with appropriate carboxylic acid followed by ester hydrolysis and the coupling of resulting carboxylic acid with appropriate amines

**Table 47**

| Example | Structure |
|---|---|
| | Name |
| | Analytical Data: LC-MS m/z [M+H]⁺; HPLC retention time (HPLC method 1) |
| 414 | 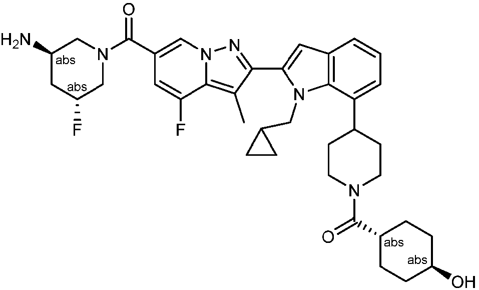 |
| | ((3R,5R)-3-Amino-5-fluoropiperidin-1-yl)(2-(1-(cyclopropylmethyl)-7-(1-((1r,4r)-4-hydroxycyclohexane-1-carbonyl)piperidin-4-yl)-1H-indol-2-yl)-4-fluoro-3-methylpyrazolo[1,5-a]pyridin-6-yl)methanone |
| | Analytical data: 673.3; 1.70 min (Method E) |
| 415 | 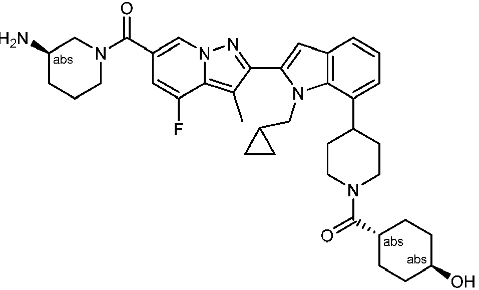 |
| | ((R)-3-Aminopiperidin-1-yl)(2-(1-(cyclopropylmethyl)-7-(1-((1r,4r)-4-hydroxycyclohexane-1-carbonyl)piperidin-4-yl)-1H-indol-2-yl)-4-fluoro-3-methylpyrazolo[1,5-a]pyridin-6-yl)methanone |
| | Analytical data: 655.4; 1.63 min (Method E) |
| 416 | 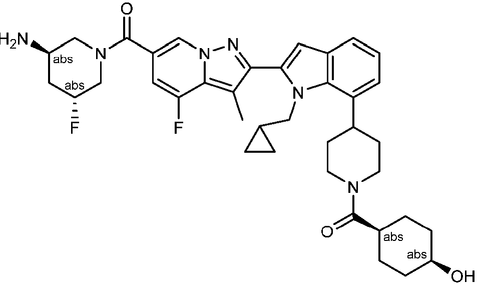 |
| | ((3R,5R)-3-Amino-5-fluoropiperidin-1-yl)(2-(1-(cyclopropylmethyl)-7-(1-((1s,4s)-4-hydroxycyclohexane-1-carbonyl)piperidin-4-yl)-1H-indol-2-yl)-4-fluoro-3-methylpyrazolo[1,5-a]pyridin-6-yl)methanone |
| | Analytical data: 673.3; 1.78 min (Method E) |
| 417 | 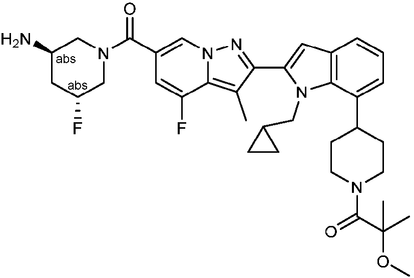 |
| | 1-(4-(2-(6-((3R, SR)-3-Amino-5-fluoropiperidine-1-carbonyl)-4-fluoro-3-methylpyrazolo[1, 5-a]pyridin-2-yl)-1-(cyclopropylmethyl)-1H-indol-7-yl)piperidin-1-yl)-2-methoxy-2-methylpropan-1-one |
| | Analytical data: 647.3; 2.0 min (Method E) |
| 418 | 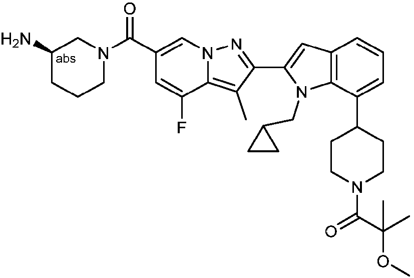 |
| | (R)-1-(4-(2-(6-(3-Aminopiperidine-1-carbonyl)-4-fluoro-3-methylpyrazolo[1,5-a]pyridin-2-yl)-1-(cyclopropylmethyl)-1H-indol-7-yl)piperidin-1-yl)-2-methoxy-2-methylpropan-1-one |
| | Analytical data: 629.4; 1.96 min (Method E) |
| 419 | 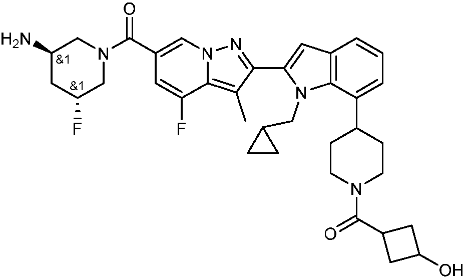 |
| | ((3R,5R)-3-Amino-5-fluoropiperidin-1-yl)(2-(1-(cyclopropylmethyl)-7-(1-(3-hydroxycyclobutane-1-carbonyl)piperidin-4-yl)-1H-indol-2-yl)-4-fluoro-3-methylpyrazolo[1,5-a]pyridin-6-yl)methanone (mixture of diastereomers) |
| | Analytical data: 645.4; 1.51 min (Method E) |
| 420 | 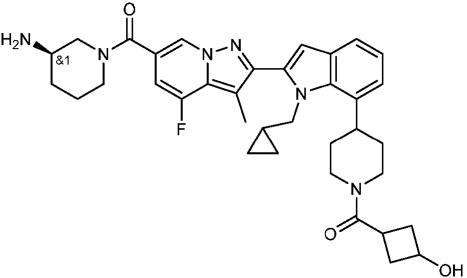 |
| | (R)-(3-Aminopiperidin-1-yl)(2-(1-(cyclopropylmethyl)-7-(1-(3-hydroxycyclobutane-1-carbonyl)piperidin-4-yl)-1H-indol-2-yl)-4-fluoro-3-methylpyrazolo[1,5-a]pyridin-6-yl)methanone (mixture of diastereomers) |
| | Analytical data: 627.4; 1.58 min (Method E) |
| 421 | 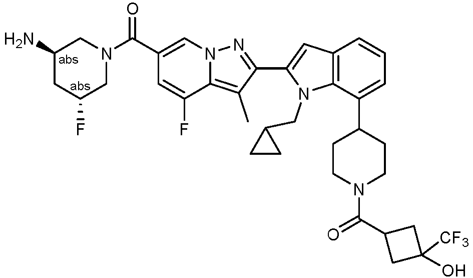 ((3R,5R)-3-Amino-5-fluoropiperidin-1-yl)(2-(1-(cyclopropylmethyl)-7-(1-(3-hydroxy-3-(trifluoromethyl)cyclobutane-1-carbonyl)piperidin-4-yl)-1H-indol-2-yl)-4-fluoro-3-methylpyrazolo[1,5-a]pyridin-6-yl)methanone (mixture of diastereomers) |
| | Analytical data: 713.3; 1.93 min (Method E) |
| 422 | 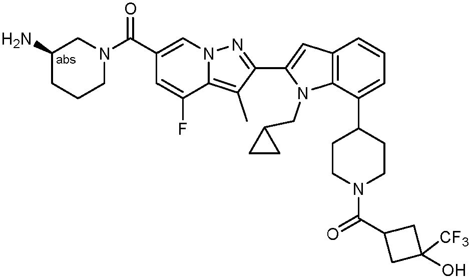 |
| | (R)-(3-Aminopiperidin-1-yl)(2-(1-(cyclopropylmethyl)-7-(1-(3-hydroxy-3-(trifluoromethyl)cyclobutane-1-carbonyl)piperidin-4-yl)-1H-indol-2-yl)-4-fluoro-3-methylpyrazolo[1,5-a]pyridin-6-yl)methanone (mixture of diastereomers) |
| | Analytical data: 695.3; 1.89 min (Method E) |
| 423 | 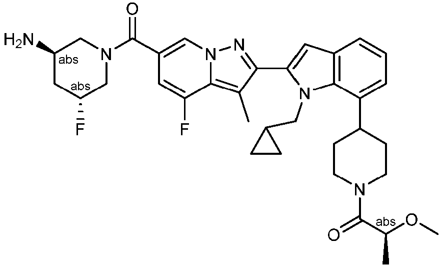 |
| | (S)-1-(4-(2-(6-((3R,5R)-3-Amino-5-fluoropiperidine-1-carbonyl)-4-fluoro-3-methylpyrazolo[1, 5-a]pyridin-2-yl)-1-(cyclopropylmethyl)-1H-indol-7-yl)piperidin-1-yl)-2-methoxypropan-1-one |
| | Analytical data: 633.3; 1.78 min (Method E) |
| 424 | 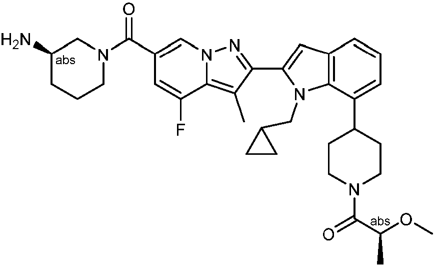 |
| | (S)-1-(4-(2-(6-((R)-3-Aminopiperidine-1-carbonyl)-4-fluoro-3-methylpyrazolo[1, 5-a]pyridin-2-yl)-1-(cyclopropylmethyl)-1H-indol-7-yl)piperidin-1-yl)-2-methoxypropan-1-one |
| | Analytical data: 615.4; 1.71 min (Method E) |
| 425 | 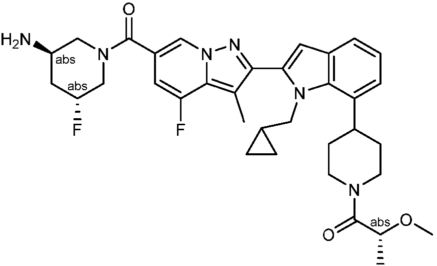 |
| | (R)-1-(4-(2-(6-((3R,5R)-3-Amino-5-fluoropiperidine-1-carbonyl)-4-fluoro-3-methylpyrazolo[1, 5-a]pyridin-2-yl)-1-(cyclopropylmethyl)-1H-indol-7-yl)piperidin-1-yl)-2-methoxypropan-1-one |
| | Analytical data: 633.4; 1.64 min (Method E) |
| 426 | 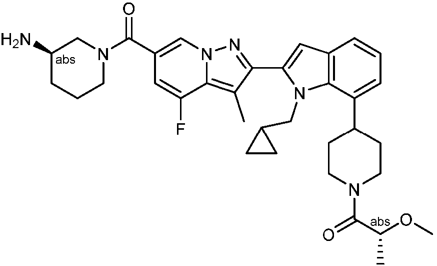 |
| | (R)-1-(4-(2-(6-((R)-3-Aminopiperidine-1-carbonyl)-4-fluoro-3-methylpyrazolo[1, 5-a]pyridin-2-yl)-1-(cyclopropylmethyl)-1H-indol-7-yl)piperidin-1-yl)-2-methoxypropan-1-one |
| | Analytical data: 615.4; 1.72 min (Method E) |
| 427 | 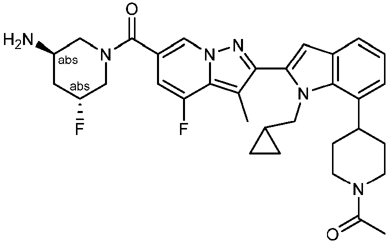 |
| | 1-(4-(2-(6-((3R, SR)-3-Amino-5-fluoropiperidine-1-carbonyl)-4-fluoro-3-methylpyrazolo[1, 5-a]pyridin-2-yl)-1-(cyclopropylmethyl)-1H-indol-7-yl)piperidin-1-yl)ethan-1-one |
| | Analytical data: 589.3; 1.70 min (Method E) |
| 428 | 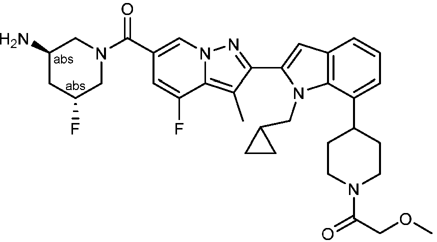 |
| | 1-(4-(2-(6-((3R, SR)-3-Amino-5-fluoropiperidine-1-carbonyl)-4-fluoro-3-methylpyrazolo[1, 5-a]pyridin-2-yl)-1-(cyclopropylmethyl)-1H-indol-7-yl)piperidin-1-yl)-2-methoxyethan-1-one |
| | Analytical data: 619.3; 1.69 min (Method E) |
| 429 | 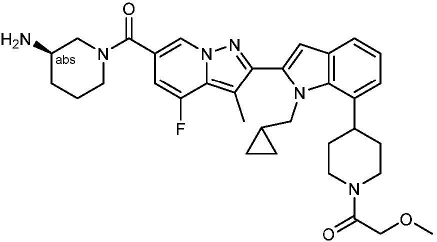 |
| | (R)-1-(4-(2-(6-(3-Aminopiperidine-1-carbonyl)-4-fluoro-3-methylpyrazolo[1,5-a]pyridin-2-yl)-1-(cyclopropylmethyl)-1H-indol-7-yl)piperidin-1-yl)-2-methoxyethan-1-one |
| | Analytical data: 601.3; 1.62 min (Method E) |

### Example 430

### Ethyl 2-(7-(1-(tert-butoxycarbonyl)piperidin-4-yl)-1-(cyclopropylmethyl)-1H-indol-2-yl)-3-methylpyrazolo[1,5-a]pyridine-6-carboxylate

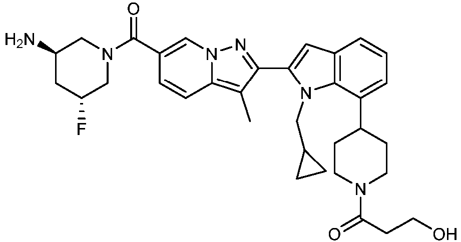

### Intermediate 430A

### Ethyl 2-(7-(1-(tert-butoxycarbonyl)piperidin-4-yl)-1-(cyclopropylmethyl)-1H-indol-2-yl)-3-methylpyrazolo[1,5-a]pyridine-6-carboxylate

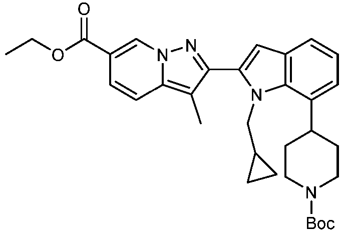

Intermediate 430A (2.1 g, 47%) was prepared following a procedure similar to the preparation of Intermediate 386A. LC-MS m/z: 557.3 [M+H]⁺

### Intermediate 430B

### Ethyl 2-(1-(cyclopropylmethyl)-7-(piperidin-4-yl)-1H-indol-2-yl)-3-methylpyrazolo[1,5-a]pyridine-6-carboxylate

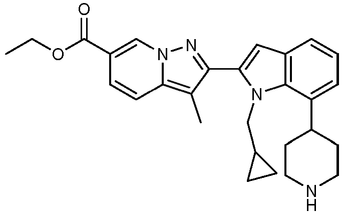

Intermediate 430B (1.4 g, 81%) was prepared starting from Intermediate 430A following a procedure similar to the preparation of Intermediate 386B. LC-MS m/z: 457.3 [M+H]⁺

### Intermediate 430C

### 1-(4-(2-(6-((3R,5R)-3-amino-5-fluoropiperidine-1-carbonyl)-3-methylpyrazolo[1,5-a]pyridin-2-yl)-1-(cyclopropylmethyl)-1H-indol-7-yl)piperidin-1-yl)-3-hydroxypropan-1-one

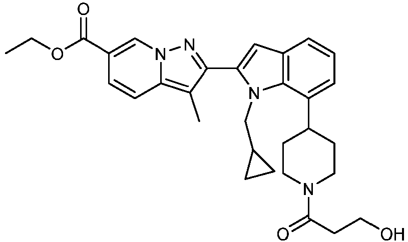

3-Hydroxypropanoic acid (59.2 mg, 0.657 mmol), DIPEA (0.344 ml, 1.971 mmol) and HATU (375 mg, 0.986 mmol) were added to a stirred solution of Intermediate 430B (300 mg, 0.657 mmol) in DMF (1 ml). The reaction was continued for 3 hours. The reaction mixture was cooled to 0°C, and quenched with ice cold water to obtain a solid, which was filtered through Buchner funnel and dried under reduced vacuum to afford the title compound ethyl 2-(1-(cyclopropylmethyl)-7-(1-(3-hydroxypropanoyl)piperidin-4-yl)-1H-indol-2-yl)-3-methylpyrazolo[1,5-a]pyridine-6-carboxylate (172 mg, 50%). LC-MS m/z: 529.3 [M+H]⁺.

### Intermediate 430D

### 2-(1-(cyclopropylmethyl)-7-(1-(3-hydroxypropanoyl)piperidin-4-yl)-1H-indol-2-yl)-3-methylpyrazolo[1,5-a]pyridine-6-carboxylic acid

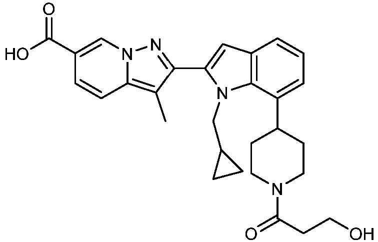

Intermediate 430D (140 mg, 92%) was prepared starting from Intermediate 430C following a procedure similar to the preparation of Intermediate 386D. LC-MS m/z: 501.4 [M+H]⁺.

### Intermediate 430E

### tert-Butyl ((3R,5R)-1-(2-(1-(cyclopropylmethyl)-7-(1-(3-hydroxypropanoyl)piperidin-4-yl)-1H-indol-2-yl)-3-methylpyrazolo[1,5-a]pyridine-6-carbonyl)-5-fluoropiperidin-3-yl)carbamate.

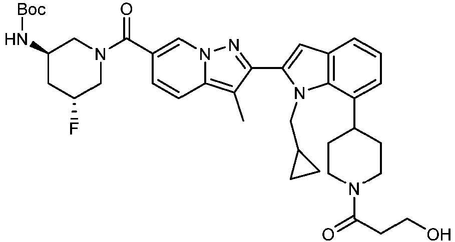

Intermediate 430E (60 mg, 86%) was prepared starting from Intermediate 430D following a procedure similar to the preparation of Intermediate 386E. LC-MS m/z: 645.4 [M-tBu]⁺.

### Example 430

### 1-(4-(2-(6-((3R,5R)-3-amino-5-fluoropiperidine-1-carbonyl)-3-methylpyrazolo[1,5-a]pyridin-2-yl)-1-(cyclopropylmethyl)-1H-indol-7-yl)piperidin-1-yl)-3-hydroxypropan-1-one

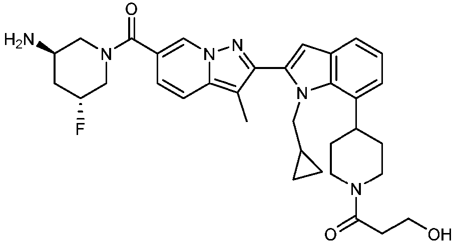

HCl (4 M in dioxane) (0.043 ml, 0.171 mmol) was added to a stirred solution of Intermediate 430E (60 mg, 0.086 mmol) in 1,4-dioxane (1 ml) at 0°C. The reaction was continued for 3 hours. The reaction mixture was concentrated in vacuo to obtain a crude compound, which was purified using preparative LC/MS with the following conditions: Column: Waters X Bridge C18, 150 mm×19 mm, 5-µm particles; mobile phase A: 5:95 acetonitrile: water with 0.1% trifluoroacetic acid; mobile phase B: 95:5 acetonitrile: water with 0.1% trifluoroacetic acid; gradient: a 0-minute hold at 15% B, 15-30% B over 20 minutes, then a 5-minute hold at 100% B; flow rate: 20 ml/min; column temperature: 25°C. Fraction collection was triggered by signals. Fractions containing the desired product were combined and dried using centrifugal evaporation. The purified material was then diluted with a 1:1 mixture of ethylene dichloride and methanol (EDM), treated with Si-Pyridine and shaken for a minimum of 2 hours. The resulting mixture was filtered, and dried via centrifugal evaporation to afford 1-(4-(2-(6-((3R,5R)-3-amino-5-fluoropiperidine-1-carbonyl)-3-methylpyrazolo[1,5-a]pyridin-2-yl)-1-(cyclopropylmethyl)-1H-indol-7-yl)piperidin-1-yl)-3-hydroxypropan-1-one (6.9 mg, 11%). LC-MS m/z: 601.3 [M+H]⁺. LCMS retention time: 1.504 min (method E). ¹H NMR (400 MHz, DMSO-*d₆*) δ ppm: 8.76 (s, 1H), 7.80 (d, *J*=9.29 Hz, 1H), 7.50 (d, *J*=7.58 Hz, 1H), 7.23 (d, *J*=9.15 Hz, 1H), 7.07-7.14 (m, 2H), 6.78 (s, 1H), 6.32 (br, s, 1H), 4.50 (d, *J*=6.36 Hz, 2H), 3.67 (m, 4H), 3.39-3.54 (m, 4H), 3.25 (m, 3H), 2.80 - 2.60 (m, 2H), 2.38 (s, 3H), 2.25 - 2.10 (m, 2H), 2.08 - 1.90 (m, 2H), 1.85 - 1.75 (m, 2H), 1.70 - 1.50 (m, 2H),1.26 - 1.22 (m, 2H), 0.98 - 0.90 (m, 1H), 0.22 (d, *J*=8.07 Hz, 2H), -0.17 (d, *J*=5.14 Hz, 2H).

Compounds of Examples 431 to 479 in Table 48 were prepared following a procedure similar to the preparation of a compound of Example 430, by acid amine coupling of Intermediate 430B with appropriate carboxylic acid followed by ester hydrolysis and amide coupling of resulting carboxylic acid with appropriate amines.

**Table 48**

| Example | Structure |
|---|---|
| | Name |
| | Analytical Data: LC-MS m/z [M+H]⁺; HPLC retention time (HPLC method) |
| 431 | 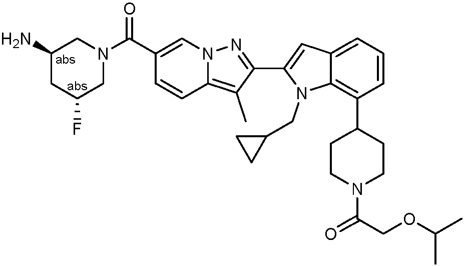 1-(4-(2-(6-((3R,5R)-3-amino-5-fluoropiperidine-1-carbonyl)-3-methylpyrazolo[1,5-a]pyridin-2-yl)-1-(cyclopropylmethyl)-1H-indol-7-yl)piperidin-1-yl)-2-isopropoxyethan-1-one |
| | Analytical Data: 629.4; 1.842min (Method E) |
| 432 | 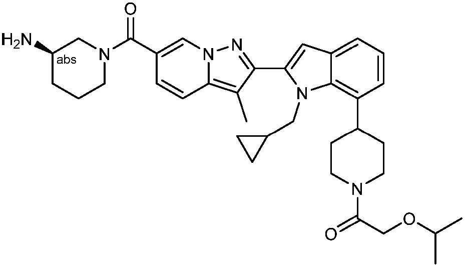 (R)-1-(4-(2-(6-(3-aminopiperidine-1-carbonyl)-3-methylpyrazolo[1,5-a]pyridin-2-yl)-1-(cyclopropylmethyl)-1H-indol-7-yl)piperidin-1-yl)-2-isopropoxyethan-1-one |
| | Analytical Data: 611.5; 1.760 min (Method E) |
| 433 | 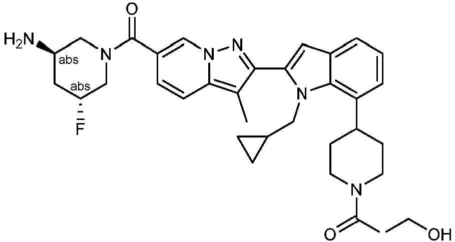 1-(4-(2-(6-((3R,5R)-3-amino-5-fluoropiperidine-1-carbonyl)-3-methylpyrazolo[1,5-a]pyridin-2-yl)-1-(cyclopropylmethyl)-1H-indol-7-yl)piperidin-1-yl)-3-hydroxy-2,2-dimethylpropan-1-one |
| | Analytical Data: 629.3; 1.748 min (Method E) |
| 434 | 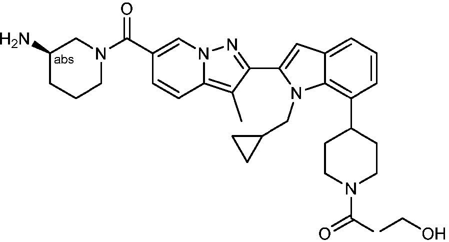 (R)-1-(4-(2-(6-(3-aminopiperidine-1-carbonyl)-3-methylpyrazolo[1,5-a]pyridin-2-yl)-1-(cyclopropylmethyl)-1H-indol-7-yl)piperidin-1-yl)-3-hydroxy-2,2-dimethylpropan-1-one |
| | Analytical Data: 611.4; 1.663 min (Method E) |
| 435 | 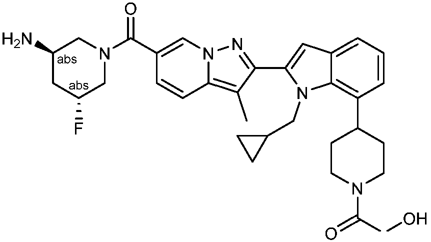 1-(4-(2-(6-((3R,5R)-3-amino-5-fluoropiperidine-1-carbonyl)-3-methylpyrazolo[1,5-a]pyridin-2-yl)-1-(cyclopropylmethyl)-1H-indol-7-yl)piperidin-1-yl)-2-hydroxy-2-methylpropan-1-one |
| | Analytical Data: 615.3; 1.694 min (Method E) |
| 436 | 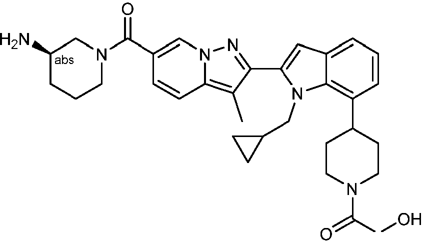 (R)-1-(4-(2-(6-(3-aminopiperidine-1-carbonyl)-3-methylpyrazolo[1,5-a]pyridin-2-yl)-1-(cyclopropylmethyl)-1H-indol-7-yl)piperidin-1-yl)-2-hydroxy-2-methylpropan-1-one |
| | Analytical Data: 597.3; 1.597min (Method E) |
| 437 | 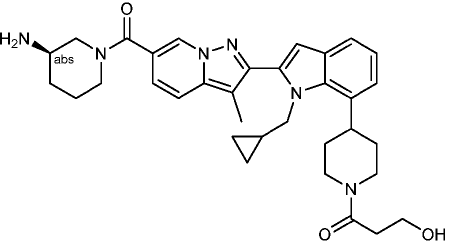 (R)-1-(4-(2-(6-(3-aminopiperidine-1-carbonyl)-3-methylpyrazolo[1,5-a]pyridin-2-yl)-1-(cyclopropylmethyl)-1H-indol-7-yl)piperidin-1-yl)-3-hydroxypropan-1-one |
| | Analytical Data: 583.4; 1.377 min (Method E) |
| 438 | 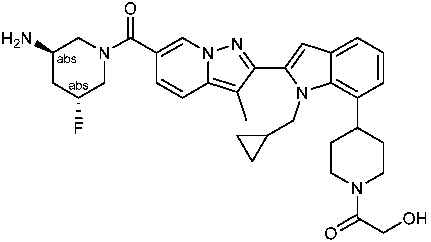 1-(4-(2-(6-((3R,5R)-3-amino-5-fluoropiperidine-1-carbonyl)-3-methylpyrazolo[1,5-a]pyridin-2-yl)-1-(cyclopropylmethyl)-1H-indol-7-yl)piperidin-1-yl)-2-hydroxyethan-1-one |
| | Analytical Data: 587.3; 1.544 min (Method E) |
| 439 | 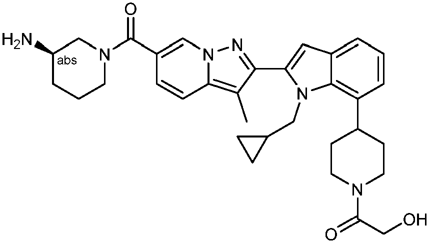 (R)-1-(4-(2-(6-(3-aminopiperidine-1-carbonyl)-3-methylpyrazolo[1,5-a]pyridin-2-yl)-1-(cycloropylmethyl)-1H-indol-7-yl)piperidin-1-yl)-2-hydroxyethan-1-one |
| | Analytical Data: 569.3; 1.464 min (Method E) |
| 440 | 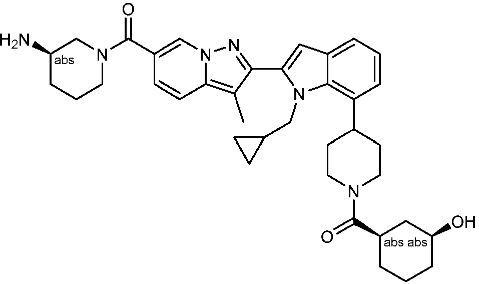 ((R)-3-aminopiperidin-1-yl)(2-(1-(cyclopropylmethyl)-7-(1-((1R,3S)-3-hydroxycyclohexane-1-carbonyl)piperidin-4-yl)-1H-indol-2-yl)-3-methylpyrazolo[1, 5-a]pyridin-6-yl)methanone |
| | Analytical Data: 637.4; 1.521 min (Method E) |
| 441 | 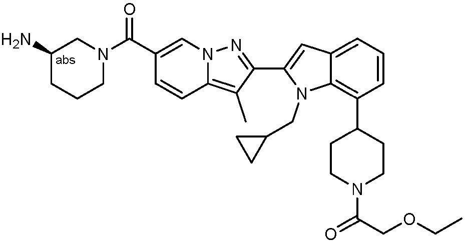 (R)-1-(4-(2-(6-(3-aminopiperidine-1-carbonyl)-3-methylpyrazolo[1,5-a]pyridin-2-yl)-1-(cyclopropylmethyl)-1H-indol-7-yl)piperidin-1-yl)-2-ethoxyethan-1-one |
| | Analytical Data: 597.3; 1.561 min (Method E) |
| 442 | 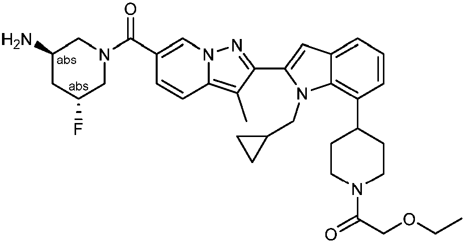 1-(4-(2-(6-((3R,5R)-3-amino-5-fluoropiperidine-1-carbonyl)-3-methylpyrazolo[1,5-a]pyridin-2-yl)-1-(cyclopropylmethyl)-1H-indol-7-yl)piperidin-1-yl)-2-ethoxyethan-1-one |
| | Analytical Data: 615.3; 1.576 min (Method E) |
| 443 | 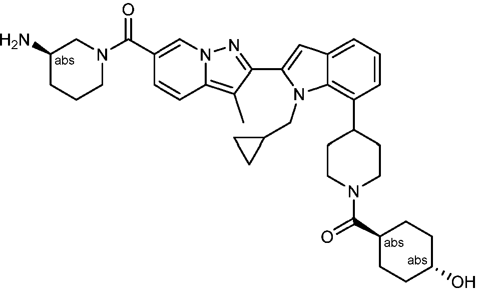 ((R)-3-aminopiperidin-1-yl)(2-(1-(cyclopropylmethyl)-7-(1-((1r,4r)-4-hydroxycyclohexane-1-carbonyl)piperidin-4-yl)-1H-indol-2-yl)-3-methylpyrazolo[1, 5-a]pyridin-6-yl)methanone |
| | Analytical Data: 637.3; 1.497 min (Method E) |
| 444 | 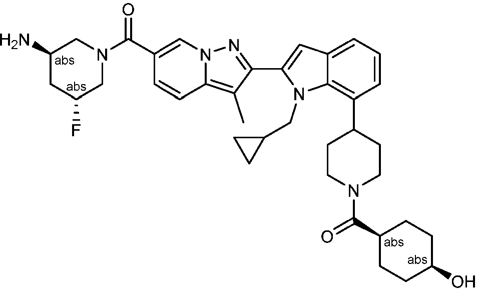 ((3R,5R)-3-amino-5-fluoropiperidin-1-yl)(2-(1-(cyclopropylmethyl)-7-(1-((1s,4s)-4-hydroxycyclohexane-1-carbonyl)piperidin-4-yl)-1H-indol-2-yl)-3-methylpyrazolo[1, 5-a]pyridin-6-yl)methanone |
| | Analytical Data: 655.4; 1.637 min (Method E) |
| 445 | 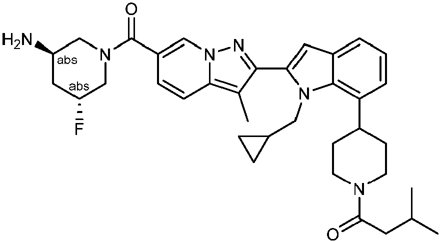 1-(4-(2-(6-((3R,5R)-3-amino-5-fluoropiperidine-1-carbonyl)-3-methylpyrazolo[1,5-a]pyridin-2-yl)-1-(cyclopropylmethyl)-1H-indol-7-yl)piperidin-1-yl)-3-methylbutan-1-one |
| | Analytical Data: 613.4; 1.918 min (Method E) |
| 446 | 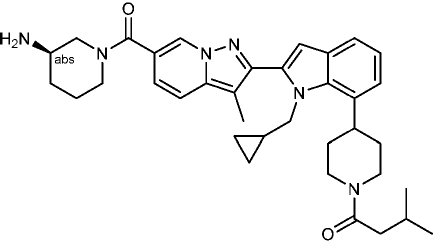 (R)-1-(4-(2-(6-(3-aminopiperidine-1-carbonyl)-3-methylpyrazolo[1,5-a]pyridin-2-yl)-1-(cyclopropylmethyl)-1H-indol-7-yl)piperidin-1-yl)-3-methylbutan-1-one |
| | Analytical Data: 595.4; 1.861 min (Method E) |
| 447 | 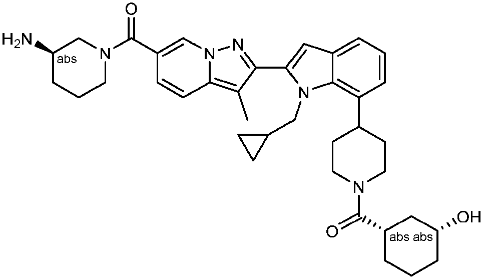 ((R)-3-aminopiperidin-1-yl)(2-(1-(cyclopropylmethyl)-7-(1-((1S,3R)-3-hydroxycyclohexane-1-carbonyl)piperidin-4-yl)-1H-indol-2-yl)-3-methylpyrazolo[1,5-a]pyridin-6-yl)methanone |
| | Analytical Data: 637.4; 1.58 min (Method E) |
| 448 | 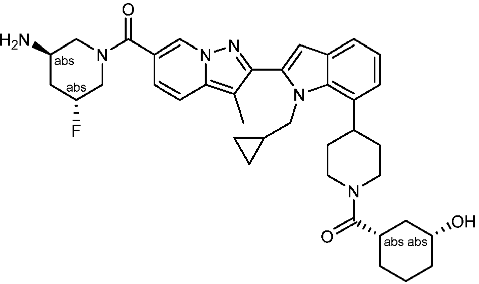 ((3R,5R)-3-amino-5-fluoropiperidin-1-yl)(2-(1-(cyclopropylmethyl)-7-(1-((1S,3R)-3-hydroxycyclohexane-1-carbonyl)piperidin-4-yl)-1H-indol-2-yl)-3-methylpyrazolo[1,5-a]pyridin-6-yl)methanone |
| | Analytical Data: 655.4; 1.492 min (Method E) |
| 449 | 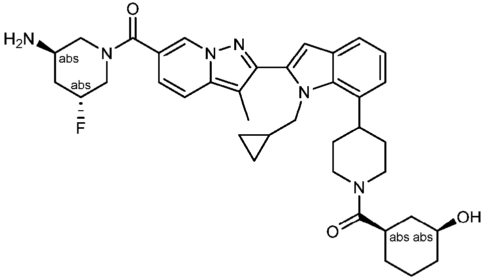 ((3R,5R)-3-amino-5-fluoropiperidin-1-yl)(2-(1-(cyclopropylmethyl)-7-(1-((1R,3S)-3-hydroxycyclohexane-1-carbonyl)piperidin-4-yl)-1H-indol-2-yl)-3-methylpyrazolo[1,5-a]pyridin-6-yl)methanone |
| | Analytical Data: 655.4; 1.494 min (Method E) |
| 450 | 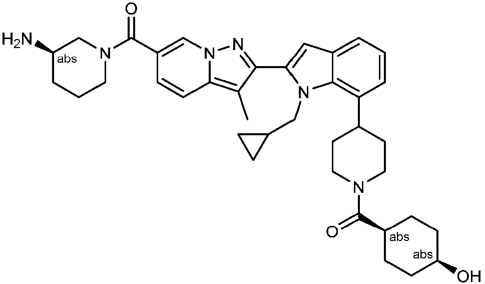 ((R)-3-aminopiperidin-1-yl)(2-(1-(cyclopropylmethyl)-7-(1-((1s,4s)-4-hydroxycyclohexane-1-carbonyl)piperidin-4-yl)-1H-indol-2-yl)-3-methylpyrazolo[1,5-a]pyridin-6-yl)methanone |
| | Analytical Data: 637.5; 1.499 min (Method E) |
| 451 | 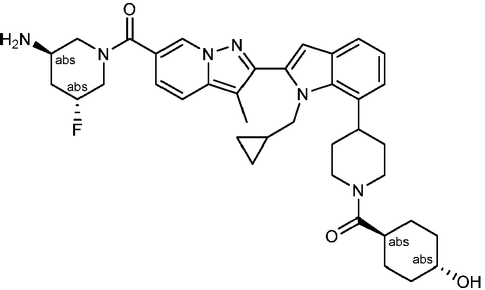 ((3R,5R)-3-amino-5-fluoropiperidin-1-yl)(2-(1-(cyclopropylmethyl)-7-(1-((1r,4r)-4-hydroxycyclohexane-1-carbonyl)piperidin-4-yl)-1H-indol-2-yl)-3-methylpyrazolo[1,5-a]pyridin-6-yl)methanone |
| | Analytical Data: 655.4; 1.546 min (Method) |
| 452 | 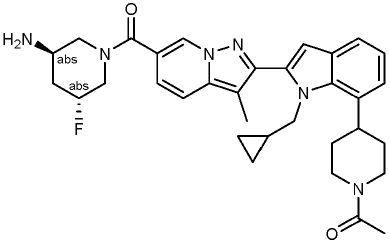 1-(4-(2-(6-((3R,5R)-3-amino-5-fluoropiperidine-1-carbonyl)-3-methylpyrazolo[1,5-a]pyridin-2-yl)-1-(cyclopropylmethyl)-1H-indol-7-yl)piperidin-1-yl)ethan-1-one |
| | Analytical Data: 571.4; 1.907 min (Method E) |
| 453 | 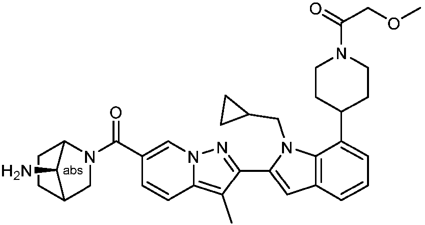 1-(4-(2-(6-((7R)-7-amino-2-azabicyclo[2.2.1]heptane-2-carbonyl)-3-methylpyrazolo[1,5-a]pyridin-2-yl)-1-(cyclopropylmethyl)-1H-indol-7-yl)piperidin-1-yl)-2-methoxyethan-1-one |
| | Analytical Data: 595.4; 8.066 min (Method D) |
| 454 | 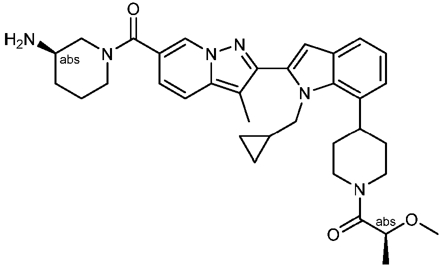 (S)-1-(4-(2-(6-((R)-3-aminopiperidine-1-carbonyl)-3-methylpyrazolo[1,5-a]pyridin-2-yl)-1-(cyclopropylmethyl)-1H-indol-7-yl)piperidin-1-yl)-2-methoxypropan-1-one |
| | Analytical Data: 597.3; 1.520 min (Method E) |
| 455 | 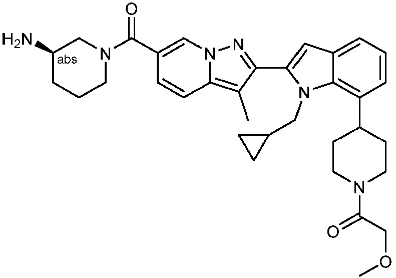 (R)-1-(4-(2-(6-(3-aminopiperidine-1-carbonyl)-3-methylpyrazolo[1,5-a]pyridin-2-yl)-1-(cyclpropylmethyl)-1H-indol-7-yl)piperidin-1-yl)-2-methoxyethan-1-one |
| | Analytical Data: 583.3; 1.505 min (Method E) |
| 456 | 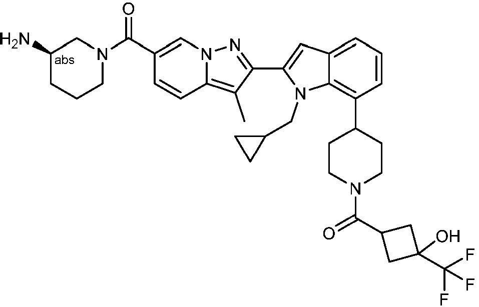 (R)-(3-aminopiperidin-1-yl)(2-(1-(cyclopropylmethyl)-7-(1-(3-hydroxy-3-(trifluoromethyl)cyclobutane-1-carbonyl)piperidin-4-yl)-1H-indol-2-yl)-3-methylpyrazolo[1,5-a]pyridin-6-yl)methanone (mixture of diastereomers) |
| | Analytical Data: 677.4; 1.779 min (Method E) |
| 457 | 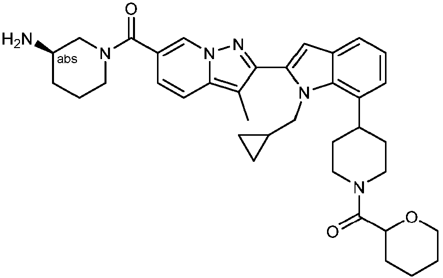 ((R)-3-aminopiperidin-1-yl)(2-(1-(cyclopropylmethyl)-7-(1-(tetrahydro-2H-pyran-2-carbonyl)piperidin-4-yl)-1H-indol-2-yl)-3-methylpyrazolo[1,5-a]pyridin-6-yl)methanone (mixture of diastereomers) |
| | Analytical Data: 623.3; 1.735 min (Method) |
| 458 | 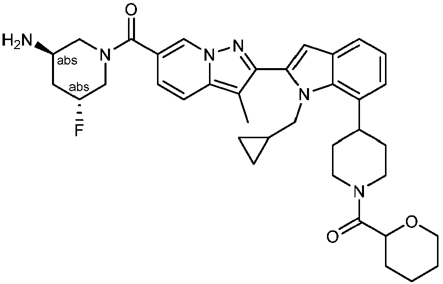 ((3R,5R)-3-amino-5-fluoropiperidin-1-yl)(2-(1-(cyclopropylmethyl)-7-(1-(tetrahydro-2H-pyran-2-carbonyl)piperidin-4-yl)-1H-indol-2-yl)-3-methylpyrazolo[1,5-a]pyridin-6-yl)methanone (mixture of diastereomers) |
| | Analytical Data: 641.5; 1.792 min (Method E) |
| 459 | 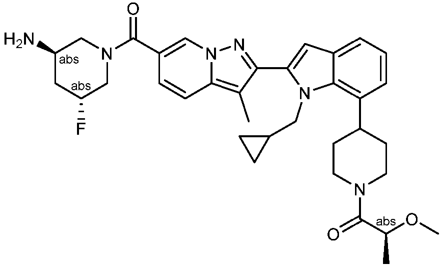 (S)-1-(4-(2-(6-((3R,5R)-3-amino-5-fluoropiperidine-1-carbonyl)-3-methylpyrazolo[1,5-a]pyridin-2-yl)-1-(cyclopropylmethyl)-1H-indol-7-yl)piperidin-1-yl)-2-methoxypropan-1-one |
| | Analytical Data: 615.3; 1.649 min (Method E) |
| 460 | 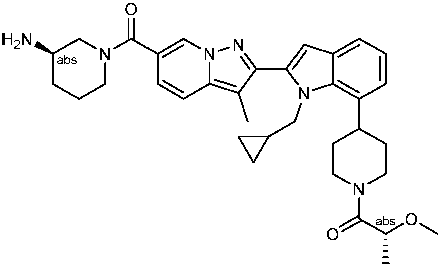 (R)-1-(4-(2-(6-((R)-3-aminopiperidine-1-carbonyl)-3-methylpyrazolo[1,5-a]pyridin-2-yl)-1-(cyclopropylmethyl)-1H-indol-7-yl)piperidin-1-yl)-2-methoxypropan-1-one |
| | Analytical Data: 597.4; 1.521 min (Method E) |
| 461 | 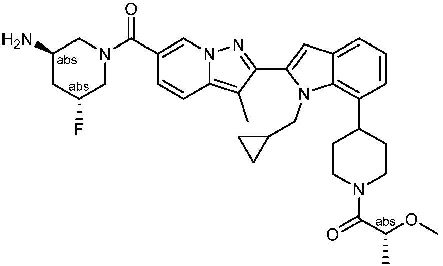 (R)-1-(4-(2-(6-((3R,5R)-3-amino-5-fluoropiperidine-1-carbonyl)-3-methylpyrazolo[1,5-a]pyridin-2-yl)-1-(cyclopropylmethyl)-1H-indol-7-yl)piperidin-1-yl)-2-methoxypropan-1-one |
| | Analytical Data: 615.3; 1.538 min (Method E) |
| 462 | 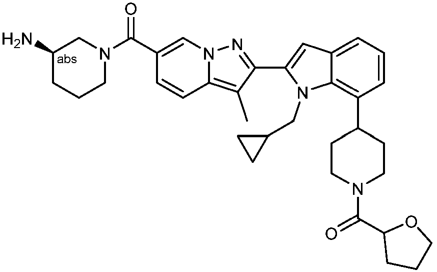 ((R)-3-aminopiperidin-1-yl)(2-(1-(cyclopropylmethyl)-7-(1-(tetrahydrofuran-2-carbonyl)piperidin-4-yl)-1H-indol-2-yl)-3-methylpyrazolo[1,5-a]pyridin-6-yl)methanone (mixture of diastereomers) |
| | Analytical Data: 609.3; 1.708 min (Method E) |
| 463 | 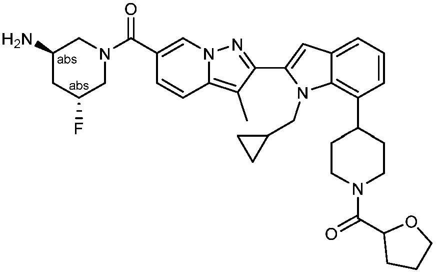 ((3R,5R)-3-amino-5-fluoropiperidin-1-yl)(2-(1-(cyclopropylmethyl)-7-(1-(tetrahydrofuran-2-carbonyl)piperidin-4-yl)-1H-indol-2-yl)-3-methylpyrazolo[1,5-a]pyridin-6-yl)methanone (mixture of diastereomers) |
| | Analytical Data: 627.3; 1.770 min (Method E) |
| 464 | 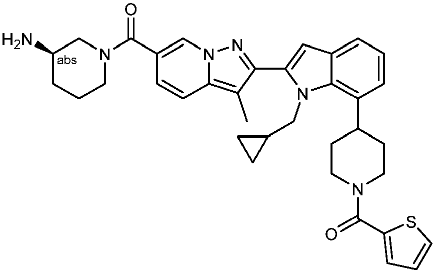 (R)-(3-aminopiperidin-1-yl)(2-(1-(cyclopropylmethyl)-7-(1-(thiophene-2-carbonyl)piperidin-4-yl)-1H-indol-2-yl)-3-methylpyrazolo[1,5-a]pyridin-6-yl)methanone |
| | Analytical Data: 621.3; 1.819 min (Method E) |
| 465 | 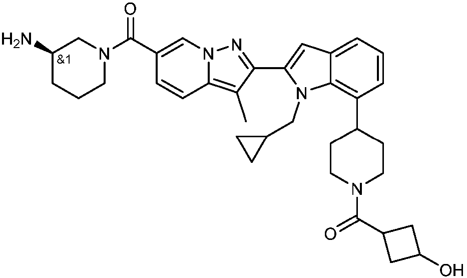 (R)-(3-aminopiperidin-1-yl)(2-(1-(cyclopropylmethyl)-7-(1-(3-hydroxycyclobutane-1-carbonyl)piperidin-4-yl)-1H-indol-2-yl)-3-methylpyrazolo[1,5-a]pyridin-6-yl)methanone (mixture of diastereomers) |
| | Analytical Data: 609.3; 1.390 min (Method E) |
| 466 | 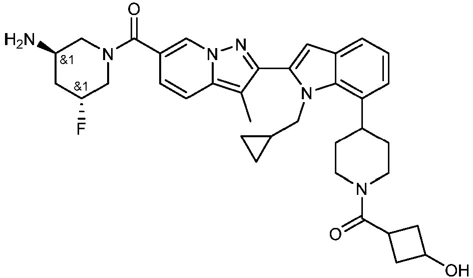 ((3R,5R)-3-amino-5-fluoropiperidin-1-yl)(2-(1-(cyclopropylmethyl)-7-(1-(3-hydroxycyclobutane-1-carbonyl)piperidin-4-yl)-1H-indol-2-yl)-3-methylpyrazolo[1,5-a]pyridin-6-yl)methanone (mixture of diastereomers) |
| | Analytical Data: 627.3; 1.406 min (Method E) |
| 467 | 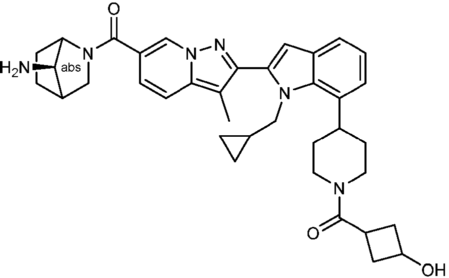 (4-(2-(6-((7R)-7-amino-2-azabicyclo[2.2.1]heptane-2-carbonyl)-3-methylpyrazolo[1,5-a]pyridin-2-yl)-1-(cyclopropylmethyl)-1H-indol-7-yl)piperidin-1-yl)(3-hydroxycyclobutyl)methanone (mixture of diastereomers) |
| | Analytical Data: 621.3; 1.371 min (Method E) |
| 468 | 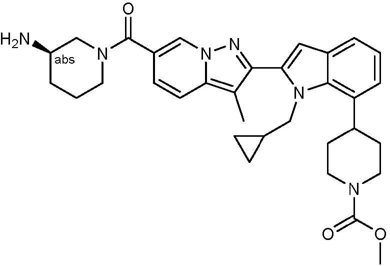 Methyl (R)-4-(2-(6-(3-aminopiperidine-1-carbonyl)-3-methylpyrazolo[1,5-a]pyridin-2-yl)-1-(cyclopropylmethyl)-1H-indol-7-yl)piperidine-1-carboxylate |
| | Analytical Data: 569.3; 1.671 min (Method E) |
| 469 | 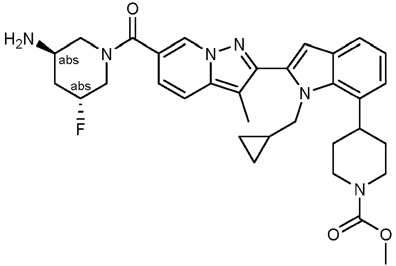 Methyl 4-(2-(6-((3R,5R)-3-amino-5-fluoropiperidine-1-carbonyl)-3-methylpyrazolo[1,5-a]pyridin-2-yl)-1-(cyclopropylmethyl)-1H-indol-7-yl)piperidine-1-carboxylate |
| | Analytical Data: 587.3; 1.699 min (Method E) |
| 470 | 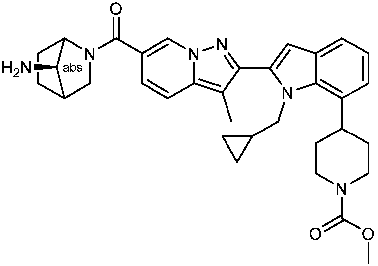 Methyl 4-(2-(6-((7R)-7-amino-2-azabicyclo[2.2.1]heptane-2-carbonyl)-3-methylpyrazolo[1,5-a]pyridin-2-yl)-1-(cyclopropylmethyl)-1H-indol-7-yl)piperidine-1-carboxylate |
| | Analytical Data: 581.3; 1.646 min (Method E) |
| 471 | 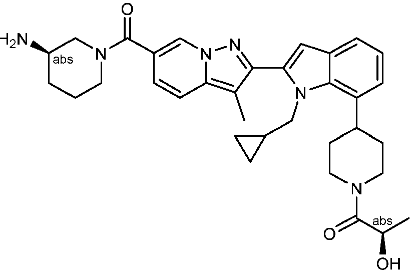 (R)-1-(4-(2-(6-((R)-3-aminopiperidine-1-carbonyl)-3-methylpyrazolo[1,5-a]pyridin-2-yl)-1-(cyclopropylmethyl)-1H-indol-7-yl)piperidin-1-yl)-2-hydroxypropan-1-one |
| | Analytical Data: 583.3; 1.428 min (Method E) |
| 472 | 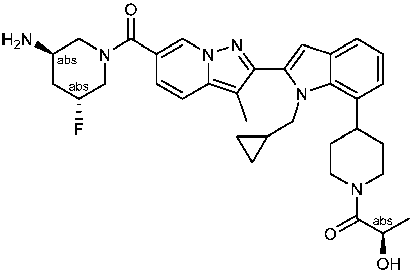 (R)-1-(4-(2-(6-((3R,5R)-3-amino-5-fluoropiperidine-1-carbonyl)-3-methylpyrazolo[1,5-a]pyridin-2-yl)-1-(cyclopropylmethyl)-1H-indol-7-yl)piperidin-1-yl)-2-hydroxypropan-1-one |
| | Analytical Data: 601.3; 1.554 min (Method E) |
| 473 | 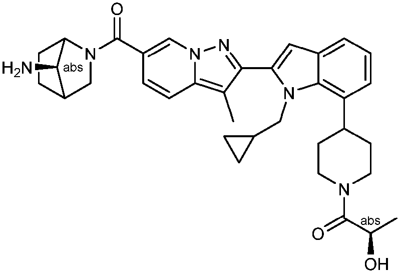 (2R)-1-(4-(2-(6-((7R)-7-Amino-2-azabicyclo[2.2.1]heptane-2-carbonyl)-3-methylpyrazolo[1,5-a]pyridin-2-yl)-1-(cyclopropylmethyl)-1H-indol-7-yl)piperidin-1-yl)-2-hydroxypropan-1-one |
| | Analytical Data: 595.3; 1.406 min (Method E) |
| 474 | 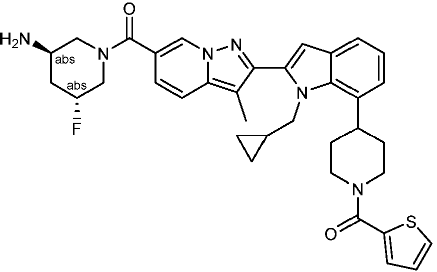 ((3R,5R)-3-Amino-5-fluoropiperidin-1-yl)(2-(1-(cyclopropylmethyl)-7-(1-(thiophene-2-carbonyl)piperidin-4-yl)-1H-indol-2-yl)-3-methylpyrazolo[1,5-a]pyridin-6-yl)methanone |
| | Analytical Data: 639.2; 1.749 min (Method E) |
| 475 | 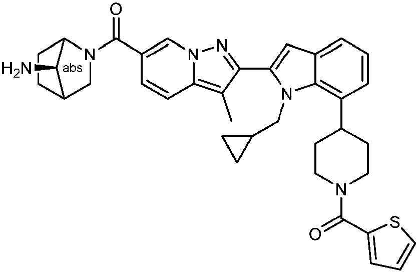 (4-(2-(6-((7R)-7-Amino-2-azabicyclo[2.2.1]heptane-2-carbonyl)-3-methylpyrazolo[1,5-a]pyridin-2-yl)-1-(cyclopropylmethyl)-1H-indol-7-yl)piperidin-1-yl)(thiophen-2-yl)methanone |
| | Analytical Data: 633.2; 1.911 min(Method E) |
| 476 | 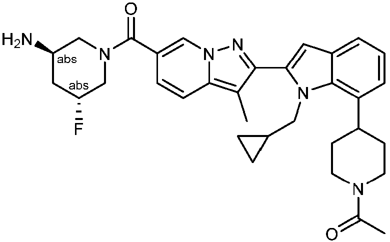 1-(4-(2-(6-((3R,SR)-3-Amino-5-fluoropiperidine-1-carbonyl)-3-methylpyrazolo[1,5-a]pyridin-2-yl)-1-(cyclopropylmethyl)-1H-indol-7-yl)piperidin-1-yl)ethan-1-one |
| | Analytical Data: 571.4; 1.60 min(Method E) |
| 477 | 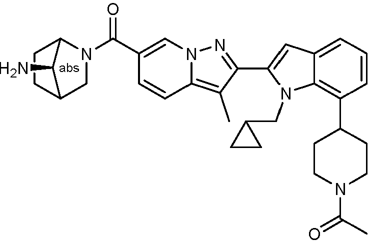 1-(4-(2-(6-((7R)-7-Amino-2-azabicyclo[2.2.1]heptane-2-carbonyl)-3-methylpyrazolo[1,5-a]pyridin-2-yl)-1-(cyclopropylmethyl)-1H-indol-7-yl)piperidin-1-yl)ethan-1-one |
| | Analytical Data: 565.4; 1.640 min (Method E) |
| 478 | 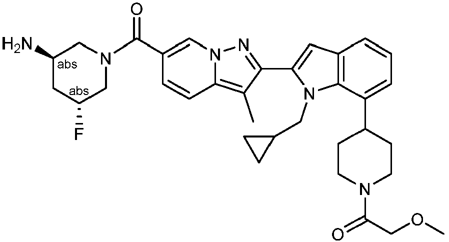 1-(4-(2-(6-((3R,5R)-3-Amino-5-fluoropiperidine-1-carbonyl)-3-methylpyrazolo[1,5-a]pyridin-2-yl)-1-(cyclopropylmethyl)-1H-indol-7-yl)piperidin-1-yl)-2-methoxyethan-1-one |
| | Analytical Data: 601.4; 5.680 min (Method D) |
| 479 | 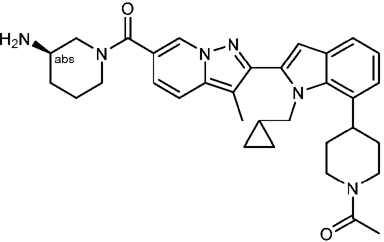 (R)-1-(4-(2-(6-(3-Aminopiperidine-1-carbonyl)-3-methylpyrazolo[1,5-a]pyridin-2-yl)-1-(cyclopropylmethyl)-1H-indol-7-yl)piperidin-1-yl)ethan-1-one |
| | Analytical Data: 553.4; 1.484 min (Method E) |

### Example 480

### (R)-1-(4-(2-(6-(3-aminopiperidine-1-carbonyl)-4-methoxy-3-methylpyrazolo[1,5-alpyridin-2-yl)-1-(cyclopropylmethyl)-5-fluoro-1H-indol-7-yl)piperidin-1-yl)-2-methoxyethan-1-one

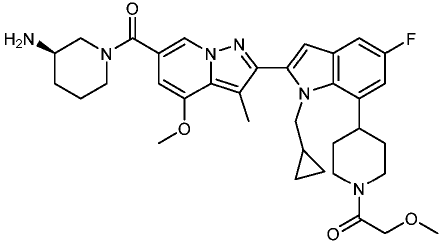

### Intermediate 480A

### methyl 2-(7-(1-(tert-butoxycarbonyl)piperidin-4-yl)-1-(cyclopropylmethyl)-5-fluoro-1H-indol-2-yl)-4-methoxy-3-methylpyrazolo[1,5-a]pyridine-6-carboxylate

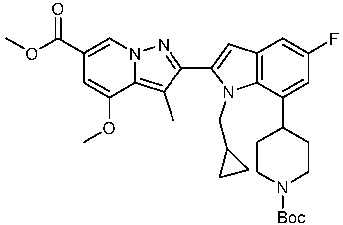

To a stirred solution of Intermediate 36 (900 mg, 1.967 mmol) in DMF (5 ml) was added Intermediate 1 (360 mg, 1.967 mmol) and copper(I) iodide (187 mg, 0.984 mmol) and heated to 80 °C for 48 hours The reaction mixture was quenched with NaHCO₃ (300ml) and extracted with ethyl acetate (2 x 300 ml). The combined organic layers were dried over sodium sulphate, filtered and concentrated under reduced vacuum pressure to give crude product. Purified using a silica gel column using 0-20% ethyl acetate in hexane to afford methyl 2-(7-(1-(tert-butoxycarbonyl)piperidin-4-yl)-1-(cyclopropylmethyl)-5-fluoro-1H-indol-2-yl)-4-methoxy-3-methylpyrazolo[1,5-a]pyridine-6-carboxylate (600 mg, 52%). LC-MS m/z: 591.2 [M+H]⁺

### Intermediate 480B

### Methyl 2-(1-(cyclopropylmethyl)-5-fluoro-7-(piperidin-4-yl)-1H-indol-2-yl)-4-methoxy-3-methylpyrazolo[1,5-a]pyridine-6-carboxylate

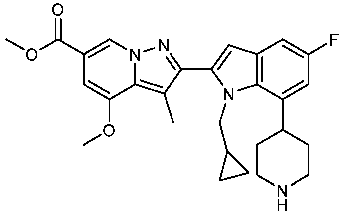

TFA (0.078 ml, 1.016 mmol) was added to stirred solution of Intermediate 480A (600 mg, 1.016 mmol) in DCM (5 ml) at 0°C. The reaction mixture was allowed to warm to a room temperature and stirred for 3 hours, concentrated under reduced pressure to obtain a crude title compound methyl 2-(1-(cyclopropylmethyl)-5-fluoro-7-(piperidin-4-yl)-1H-indol-2-yl)-4-methoxy-3-methylpyrazolo[1,5-a]pyridine-6-carboxylate (470 mg, 94%). LC-MS m/z: 491.3 [M+H]⁺.

### Intermediate 480C

### Methyl 2-(1-(cyclopropylmethyl)-5-fluoro-7-(1-(2-methoxyacetyl)piperidin-4-yl)-1H-indol-2-yl)-4-methoxy-3-methylpyrazolo[1,5-a]pyridine-6-carboxylate

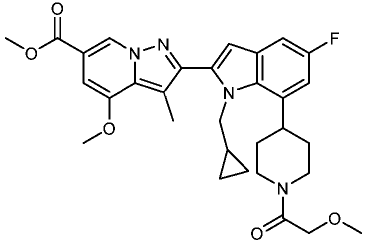

DIPEA (0.128 ml, 0.734 mmol) and HATU (140 mg, 0.367 mmol) were added to a stirred solution of Intermediate 480B (24.24 mg, 0.269 mmol). The reaction was continued for 3 hours. The reaction mixture was quenched with water (50 ml), and the solids obtained were filtered and dried in vacuo to obtain the title compound methyl 2-(1-(cyclopropylmethyl)-5-fluoro-7-(1-(2-methoxyacetyl)piperidin-4-yl)-1H-indol-2-yl)-4-methoxy-3-methylpyrazolo[1,5-a]pyridine-6-carboxylate (110 mg, 80%). LC-MS m/z: 563.3 [M+H]⁺.

### Intermediate 480D

### 2-(1-(Cyclopropylmethyl)-5-fluoro-7-(1-(2-methoxyacetyl)piperidin-4-yl)-1H-indol-2-yl)-4-methoxy-3-methylpyrazolo[1,5-a]pyridine-6-carboxylic acid

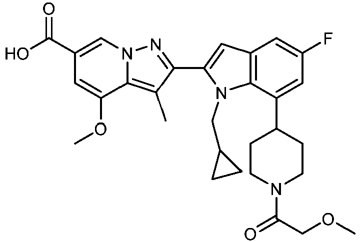

LiOH (13.83 mg, 0.578 mmol) was added to a stirred solution of Intermediate 480C (130 mg, 0.231 mmol) in a mixture of solvents: THF (1 ml), MeOH (1 ml) and water (1 ml). The reaction mixture was stirred for 3 hours, concentrated under reduced pressure to obtain a crude residue, which was acidified with 1 N HCl. The solids precipitated were filtered through Buchner funnel, and dried in vacuum to afford the title compound 2-(1-(cyclopropylmethyl)-5-fluoro-7-(1-(2-methoxyacetyl)piperidin-4-yl)-1H-indol-2-yl)-4-methoxy-3-methylpyrazolo[1,5-a]pyridine-6-carboxylic acid (105 mg, 83%). LC-MS m/z: 549.2 [M+H]⁺.

### Intermediate 480E

### tert-Butyl (R)-(1-(2-(1-(cyclopropylmethyl)-5-fluoro-7-(1-(2-methoxyacetyl)piperidin-4-yl)-1H-indol-2-yl)-4-methoxy-3-methylpyrazolo[1,5-a]pyridine-6-carbonyl)piperidin-3-yl)carbamate

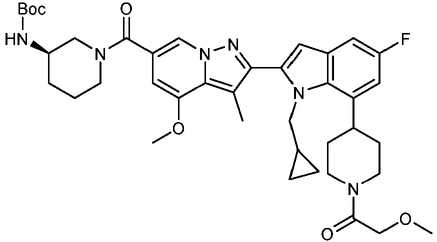

tert-Butyl (R)-piperidin-3-ylcarbamate (18.25 mg, 0.091 mmol), DIPEA (0.048 ml, 0.273 mmol) and HATU (52.0 mg, 0.137 mmol) were added to a stirred solution of Intermediate 480D (50 mg, 0.091 mmol) in DMF (1 ml). The reaction mixture was stirred for 3 hours, quenched with ice cold water, and the solids obtained were filtered through Buchner funnel and dried under reduced pressure to afford the title compound tert-butyl (R)-(1-(2-(1-(cyclopropylmethyl)-5-fluoro-7-(1-(2-methoxyacetyl)piperidin-4-yl)-1H-indol-2-yl)-4-methoxy-3-methylpyrazolo[1,5-a]pyridine-6-carbonyl)piperidin-3-yl)carbamate (43 mg, 65%). LC-MS m/z: 731.2 [M+H]⁺.

### Example 480

### (R)-1-(4-(2-(6-(3-Aminopiperidine-1-carbonyl)-4-methoxy-3-methylpyrazolo[1,5-a]pyridin-2-yl)-1-(cyclopropylmethyl)-5-fluoro-1H-indol-7-yl)piperidin-1-yl)-2-methoxyethan-1-one

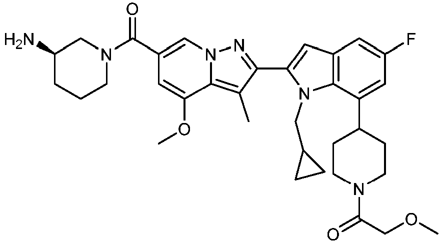

TFA (10.54 µl, 0.137 mmol) was added to a solution of Intermediate 480E (50 mg, 0.068 mmol) in DCM (1 ml) at 0°C. The reaction mixture was stirred for 3 hours, and concentrated in vacuum to obtain a crude compound, which was purified using preparative LC/MS with the following conditions: Column: Waters XBridge C18, 150 mm × 19 mm, 5-µm particles; mobile phase A: 5:95 acetonitrile: water with 0.1% trifluoroacetic acid; mobile phase B: 95:5 acetonitrile: water with 0.1% trifluoroacetic acid; gradient: a 0-minute hold at 15% B, 15-30% B over 20 minutes, then a 5-minute hold at 100% B; flow rate: 20 ml/min; column temperature: 25°C. Fraction collection was triggered by signals. Fractions containing the desired product were combined and dried using centrifugal evaporation. The purified material was then diluted with a 1:1 mixture of ethylene dichloride and methanol (EDM), treated with Si-Pyridine and shaken for a minimum of 2 hours. The resulting mixture was filtered and dried using centrifugal evaporation to afford (R)-1-(4-(2-(6-(3-aminopiperidine-1-carbonyl)-4-methoxy-3-methylpyrazolo[1,5-a]pyridin-2-yl)-1-(cyclopropylmethyl)-5-fluoro-1H-indol-7-yl)piperidin-1-yl)-2-methoxyethan-1-one.

Compounds of Examples 481 to 483 in Table 49 were prepared following a procedure similar to the preparation of a compound of Example 480, by acid amine coupling of Intermediate 480B with appropriate carboxylic acid followed by ester hydrolysis and the resulting carboxylic acid with appropriate amines.

**Table 49**

| Example | Structure |
|---|---|
| | Name |
| | Analytical Data: LC-MS m/z [M+H]⁺; HPLC retention time (HPLC method 1) |
| 481 | 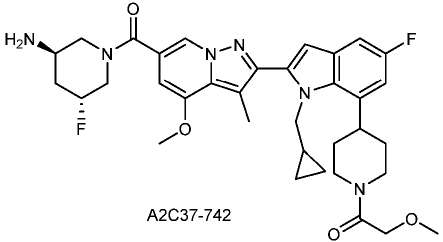 1-(4-(2-(6-((3R,5R)-3-Amino-5-fluoropiperidine-1-carbonyl)-4-methoxy-3-methylpyrazolo[1, 5-a]pyridin-2-yl)-1-(cyclopropylmethyl)-5-fluoro-1H-indol-7-yl)piperidin-1-yl)-2-methoxyethan-1-one |
| | Analytical Data: 633.2; 1.911 min(Method E) |
| 482 | 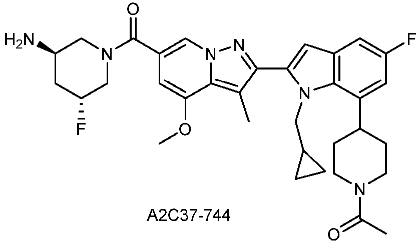 1-(4-(2-(6-((3R, SR)-3-Amino-5-fluoropiperidine-1-carbonyl)-4-methoxy-3-methylpyrazolo[1, 5-a]pyridin-2-yl)-1-(cyclopropylmethyl)-5-fluoro-1H-indol-7-yl)piperidin-1-yl)ethan-1-one |
| | Analytical Data: 619.3; 1.703 min(Method E) |
| 483 | 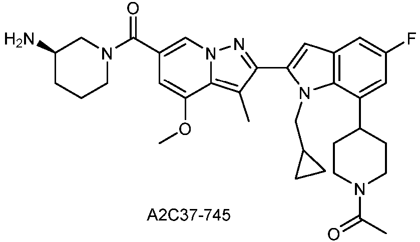 (R)-1-(4-(2-(6-(3-Aminopiperidine-1-carbonyl)-4-methoxy-3-methylpyrazolo[1,5-a]pyridin-2-yl)-1-(cyclopropylmethyl)-5-fluoro-1H-indol-7-yl)piperidin-1-yl)ethan-1-one |
| | Analytical Data: 601.3; 1.629 min(Method E) |

### Example 484

### ((R)-3-Aminopiperidin-1-yl)(2-(1-(cyclopropylmethyl)-5-fluoro-7-(1-((1r,4r)-4-hydroxycyclohexane-1-carbonyl)piperidin-4-yl)-1H-indol-2-yl)-3-methylpyrazolo[1,5-a]pyridin-6-yl)methanone

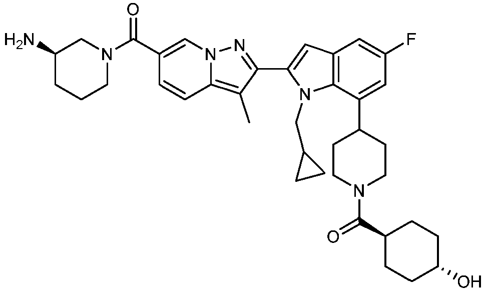

### Intermediate 484A

### Ethyl 2-(7-(1-(tert-butoxycarbonyl)piperidin-4-yl)-1-(cyclopropylmethyl)-5-fluoro-1H-indol-2-yl)-3-methylpyrazolo[1,5-a]pyridine-6-carboxylate

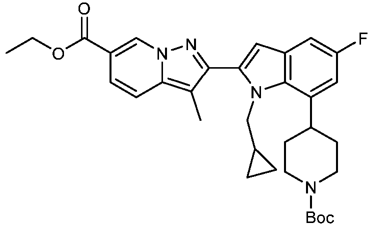

Intermediate 484A (3.0 g, 68%) was prepared following a procedure similar to the preparation of Intermediate 386A. LC-MS m/z: 575.5 [M+H]⁺.

### Intermediate 484B

### Ethyl 2-(1-(cyclopropylmethyl)-5-fluoro-7-(piperidin-4-yl)-1H-indol-2-yl)-3-methylpyrazolo[1,5-a]pyridine-6-carboxylate

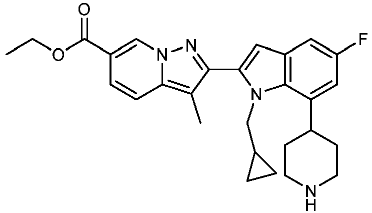

Intermediate 484B (500 mg, 76%) was obtained from intermediate 484A following a procedure similar to the preparation of Intermediate 386B. LC-MS m/z: 475.4 [M+H]⁺.

### Intermediate 484C

### Ethyl 2-(1-(cyclopropylmethyl)-5-fluoro-7-(1-((1r,4r)-4-hydroxycyclohexane-1-carbonyl)piperidin-4-yl)-1H-indol-2-yl)-3-methylpyrazolo[1,5-a]pyridine-6-carboxylate

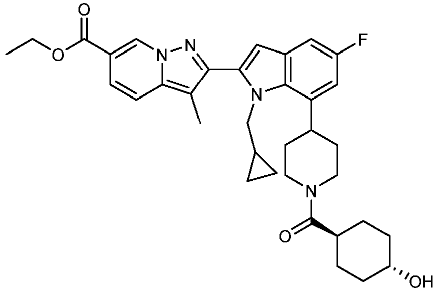

(1s,4s)-4-Hydroxycyclohexane-1-carboxylic acid (30.4 mg, 0.211 mmol), DIPEA (0.110 ml, 0.632 mmol) and HATU (120 mg, 0.316 mmol) were added to a stirred solution of Intermediate 484B (100 mg, 0.211 mmol) in DMF (2 ml). The reaction mixture was stirred 3 hours, quenched with water (50 ml), and extracted with ethyl acetate (2×50 ml). The combined organic layers were dried over sodium sulphate, filtered, and concentrated under reduced pressure to give afford crude ethyl 2-(1-(cyclopropylmethyl)-5-fluoro-7-(1-((1s,4s)-4-hydroxycyclohexane-1-carbonyl)piperidin-4-yl)-1H-indol-2-yl)-3-methylpyrazolo[1,5-a]pyridine-6-carboxylate (100 mg, 79%). LC-MS m/z: 601.8 [M+H]⁺.

### Intermediate 484D

### 2-(1-(cyclopropylmethyl)-5-fluoro-7-(1-((1r,4r)-4-hydroxycyclohexane-1-carbonyl)piperidin-4-yl)-1H-indol-2-yl)-3-methylpyrazolo[1,5-a]pyridine-6-carboxylic acid

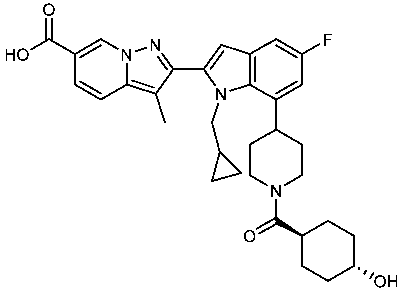

LiOH (3.99 mg, 0.166 mmol) was added to a stirred solution of Intermediate 484C (100 mg, 0.166 mmol) in a mixture of solvents: THF (1 ml), methanol (1 ml) and water (1 ml).The reaction mixture was stirred for 4 hours, concentrated in vacuo and the salts obtained were neutralized using 1 N HCl. The solids obtained was filtered through a Buchner funnel, and dried under reduced pressure to afford 2-(1-(cyclopropylmethyl)-5-fluoro-7-(1-((1r,4r)-4-hydroxycyclohexane-1-carbonyl)piperidin-4-yl)-1H-indol-2-yl)-3-methylpyrazolo[1,5-a]pyridine-6-carboxylic acid (90 mg, 94%). LC-MS m/z: 573.5 [M+H]⁺.

### Intermediate 484E

### tert-butyl ((R)-1-(2-(1-(cyclopropylmethyl)-5-fluoro-7-(1-((1r,4r)-4-hydroxycyclohexane-1-carbonyl)piperidin-4-yl)-1H-indol-2-yl)-3-methylpyrazolo[1,5-a]pyridine-6-carbonyl)piperidin-3-yl)carbamate

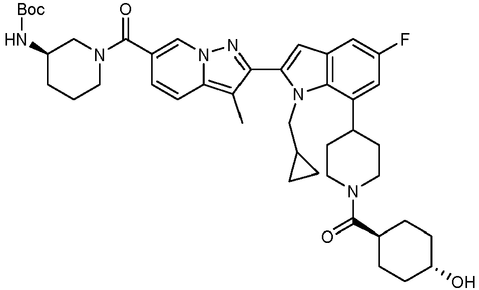

Intermediate 484E (100 mg, 76%) was prepared starting from Intermediate 484D following a procedure similar to the preparation of Intermediate 386E. LC-MS m/z: 755.6 [M+H]⁺.

### Example 484

### ((R)-3-Aminopiperidin-1-yl)(2-(1-(cyclopropylmethyl)-5-fluoro-7-(1-((1r,4r)-4-hydroxycyclohexane-1-carbonyl)piperidin-4-yl)-1H-indol-2-yl)-3-methylpyrazolo[1,5-a]pyridin-6-yl)methanone

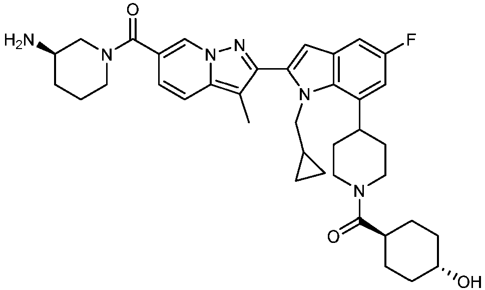

TFA (0.106 mmol) was added to a stirred solution of Intermediate 484E (80 mg, 0.106 mmol) in DCM (1 ml) at 0°C. The reaction mixture was stirred for 3 hours, concentrated in vacuo to obtain a crude compound, which was purified using preparative LC/MS with the following conditions: Column: Waters X Bridge C18, 19×150 mm, 5 µm particles; mobile phase A: 10 mM ammonium acetate; mobile phase B: acetonitrile; gradient: 12-47% B over 25 minutes, then a 5-minute hold at 100% B; flow rate: 15 ml/min. Fractions containing the desired product were combined, and dried using centrifugal evaporation. The purified material was then diluted with a 1:1 mixture of ethylene dichloride and methanol (EDM), treated with Si-Pyridine, and shaken for a minimum of 2 hours, filtered, and dried via centrifugal evaporation to afford ((R)-3-aminopiperidin-1-yl)(2-(1-(cyclopropylmethyl)-5-fluoro-7-(1-((1s,4s)-4-hydroxycyclohexane-1-carbonyl)piperidin-4-yl)-1H-indol-2-yl)-3-methylpyrazolo[1,5-a]pyridin-6-yl)methanone (7.3 mg, 10%). LCMS m/z: 655.4 [M+H]⁺. LCMS retention time : 1.52 min (method E); 1H NMR (400 MHz, DMSO-*d₆*) δ ppm: 8.86 (s, 1H), 8.13-7.90 (m, 2H), 7.82 (d, J=9.3 Hz, 1H), 7.33-7.10 (m, 3H), 7.04-6.93 (m, 2H), 6.78 (s,1H), 4.70-4.62 (m, 1H), 4.50-4.26 (m, 3H), 4.17-3.94 (m, 3H), 3.84-3.79 (m, 1H), 3.64-3.59 (m, 1H), 3.31-3.16 (m, 5H), 2.95-2.61 (m,4H), 2.37 (s, 3H), 2.06-1.94 (m, 3H), 1.87-1.35 (m, 7H), 0.98-0.88 (m, 1H), 0.23 (br, d, J=8.8 Hz, 2H), 0.12-0.28 (m, 2H).

Compounds of Examples 485 to 509 in Table 50 were prepared following a procedure similar to the preparation of a compound of Example 484, by acid amine coupling of Intermediate 484B with appropriate carboxylic acid followed by ester hydrolysis and coupling with appropriate amines

**Table 50**

| Example | Structure |
|---|---|
| | Name |
| | Analytical Data: LC-MS m/z [M+H]⁺; HPLC retention time (HPLC method) |
| 485 | 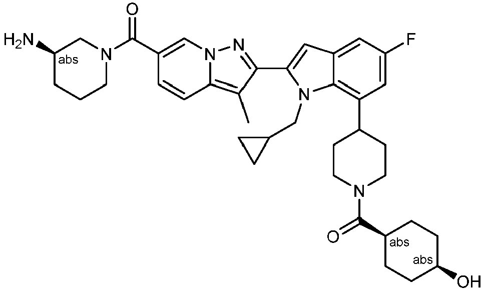 ((R)-3-Aminopiperidin-1-yl)(2-(1-(cyclopropylmethyl)-5-fluoro-7-(1-((1s,4s)-4-hydroxycyclohexane-1-carbonyl)piperidin-4-yl)-1H-indol-2-yl)-3-methylpyrazolo[1, 5-a]pyridin-6-yl)methanone |
| | Analytical Data: 655.4; 1.650 min (Method E) |
| 486 | 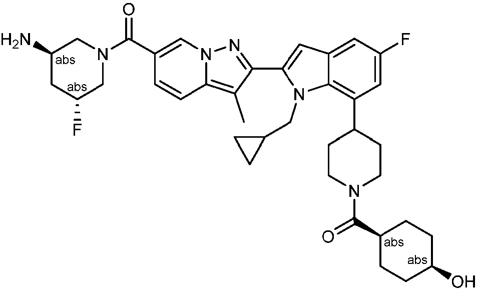 ((3R,5R)-3-Amino-5-fluoropiperidin-1-yl)(2-(1-(cyclopropylmethyl)-5-fluoro-7-(1-((1s,4s)-4-hydroxycyclohexane-1-carbonyl)piperidin-4-yl)-1H-indol-2-yl)-3-methylpyrazolo[1,5-a]pyridin-6-yl)methanone |
| | Analytical Data: 673.4; 1.623 min (Method E) |
| 487 | 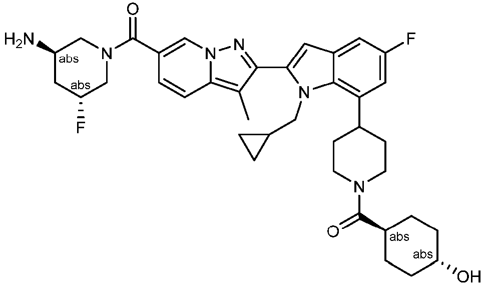 ((3R,5R)-3-Amino-5-fluoropiperidin-1-yl)(2-(1-(cyclopropylmethyl)-5-fluoro-7-(1-((1r,4r)-4-hydroxycyclohexane-1-carbonyl)piperidin-4-yl)-1H-indol-2-yl)-3-methylpyrazolo[1,5-a]pyridin-6-yl)methanone |
| | Analytical Data: 673.4; 1.627 min (Method E) |
| 488 | 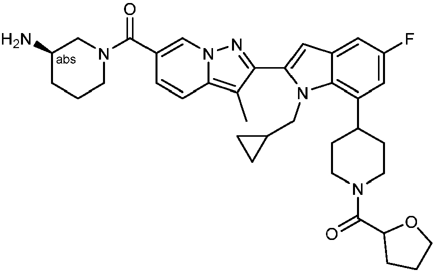 ((R)-3-Aminopiperidin-1-yl)(2-(1-(cyclopropylmethyl)-5-fluoro-7-(1-(tetrahydrofuran-2-carbonyl)piperidin-4-yl)-1H-indol-2-yl)-3-methylpyrazolo[1,5-a]pyridin-6-yl)methanone (isomer 1) |
| | Analytical Data: 627.4; 1.942 min (Method E) |
| 489 | 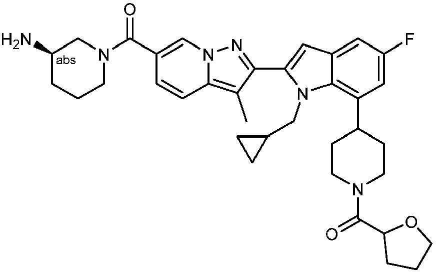 ((R)-3-Aminopiperidin-1-yl)(2-(1-(cyclopropylmethyl)-5-fluoro-7-(1-(tetrahydrofuran-2-carbonyl)piperidin-4-yl)-1H-indol-2-yl)-3-methylpyrazolo[1,5-a]pyridin-6-yl)methanone (isomer 2) |
| | Analytical Data: 627.4; 1.942 min (Method E) |
| 490 | 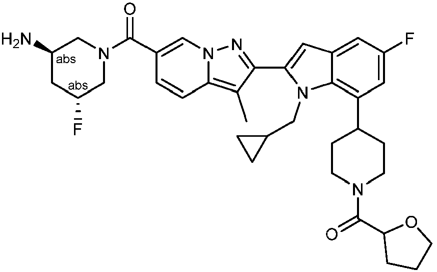 ((3R,5R)-3-Amino-5-fluoropiperidin-1-yl)(2-(1-(cyclopropylmethyl)-5-fluoro-7-(1-(tetrahydrofuran-2-carbonyl)piperidin-4-yl)-1H-indol-2-yl)-3-methylpyrazolo[1,5-a]pyridin-6-yl)methanone (isomer 1) |
| | Analytical Data: 645.45; 1.979 min (Method E) |
| 491 | 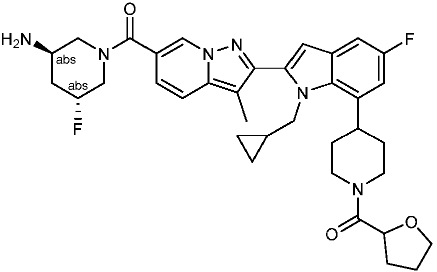 ((3R,5R)-3-Amino-5-fluoropiperidin-1-yl)(2-(1-(cyclopropylmethyl)-5-fluoro-7-(1-(tetrahydrofuran-2-carbonyl)piperidin-4-yl)-1H-indol-2-yl)-3-methylpyrazolo[1,5-a]pyridin-6-yl)methanone (isomer 2) |
| | Analytical Data: 645.45; 1.979 min (Method E) |
| 492 | 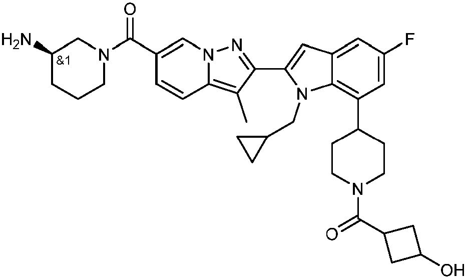 (R)-(3-Aminopiperidin-1-yl)(2-(1-(cyclopropylmethyl)-5-fluoro-7-(1-(3-hydroxycyclobutane-1-carbonyl)piperidin-4-yl)-1H-indol-2-yl)-3-methylpyrazolo[1,5-a]pyridin-6-yl)methanone (mixture of diastereomers) |
| | Analytical Data: 627.3; 1.509 min (Method E) |
| 493 | 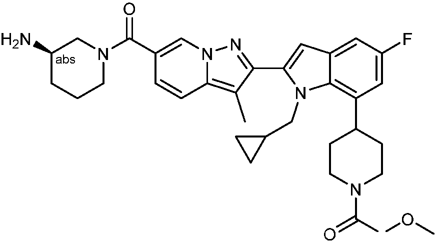 (R)-1-(4-(2-(6-(3-Aminopiperidine-1-carbonyl)-3-methylpyrazolo[1,5-a]pyridin-2-yl)-1-(cyclopropylmethyl)-5-fluoro-1H-indol-7-yl)piperidin-1-yl)-2-methoxy-2-methylpropan-1-one |
| | Analytical Data: 629.3; 1.868 min (Method E) |
| 494 | 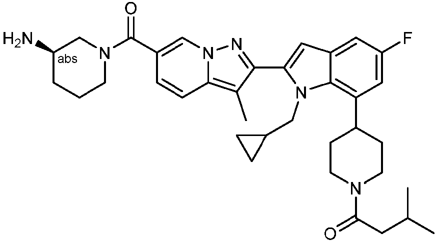 1-(4-(2-(6-((3R,5R)-3-Amino-5-fluoropiperidine-1-carbonyl)-3-methylpyrazolo[1, 5-a]pyridin-2-yl)-1-(cyclopropylmethyl)-5-fluoro-1H-indol-7-yl)piperidin-1-yl)-3-methylbutan-1-one |
| | Analytical Data: 613.3; 1.780 min (Method E) |
| 495 | 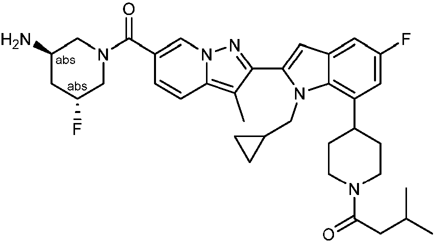 1-(4-(2-(6-((3R,5R)-3-Amino-5-fluoropiperidine-1-carbonyl)-3-methylpyrazolo[1, 5-a]pyridin-2-yl)-1-(cyclopropylmethyl)-5-fluoro-1H-indol-7-yl)piperidin-1-yl)-3-methylbutan-1-one |
| | Analytical Data: 631.3; 1.810 min (Method E) |
| 496 | 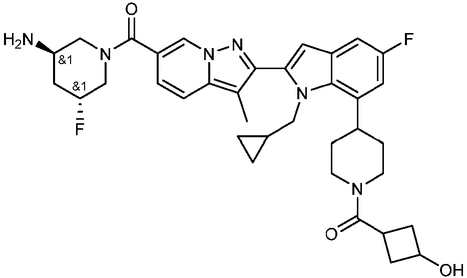 ((3R,5R)-3-Amino-5-fluoropiperidin-1-yl)(2-(1-(cyclopropylmethyl)-5-fluoro-7-(1-(3-hydroxycyclobutane-1-carbonyl)piperidin-4-yl)-1H-indol-2-yl)-3-methylpyrazolo[1,5-a]pyridin-6-yl)methanone (mixture of diastereomers) |
| | Analytical Data: 645.3; 1.447 min (Method E) |
| 497 | 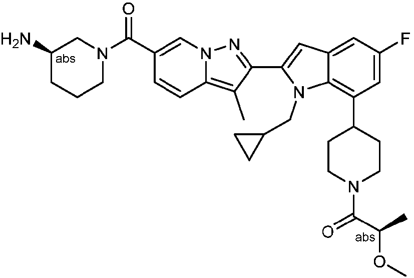 (R)-1-(4-(2-(6-((R)-3-Aminopiperidine-1-carbonyl)-3-methylpyrazolo[1,5-a]pyridin-2-yl)-1-(cyclopropylmethyl)-5-fluoro-1H-indol-7-yl)piperidin-1-yl)-2-methoxypropan-1-one |
| | Analytical Data: 615.3; 1.551 min (Method E) |
| 498 | 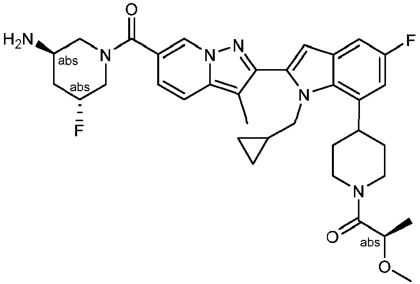 (R)-1-(4-(2-(6-((3R,5R)-3-Amino-5-fluoropiperidine-1-carbonyl)-3-methylpyrazolo[1,5-a]pyridin-2-yl)-1-(cyclopropylmethyl)-5-fluoro-1H-indol-7-yl)piperidin-1-yl)-2-methoxypropan-1-one |
| | Analytical Data: 633.3; 1.693 min (Method E) |
| 499 | 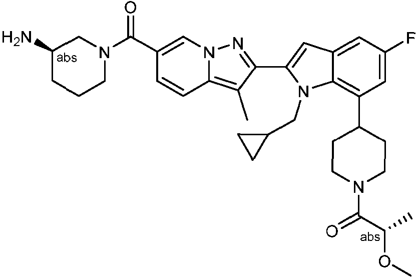 (S)-1-(4-(2-(6-((R)-3-Aminopiperidine-1-carbonyl)-3-methylpyrazolo[1,5-a]pyridin-2-yl)-1-(cyclopropylmethyl)-5-fluoro-1H-indol-7-yl)piperidin-1-yl)-2-methoxypropan-1-one |
| | Analytical Data: 615.3; 1.545 min (Method E) |
| 500 | 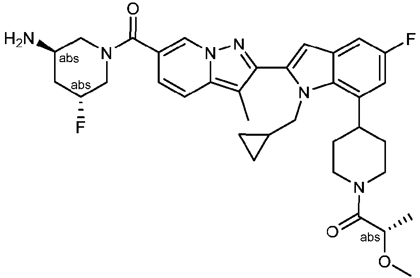 (S)-1-(4-(2-(6-((3R,5R)-3-Amino-5-fluoropiperidine-1-carbonyl)-3-methylpyrazolo[1,5-a]pyridin-2-yl)-1-(cyclopropylmethyl)-5-fluoro-1H-indol-7-yl)piperidin-1-yl)-2-methoxypropan-1-one |
| | Analytical Data: 633.3; 1.564 min (Method E) |
| 501 | 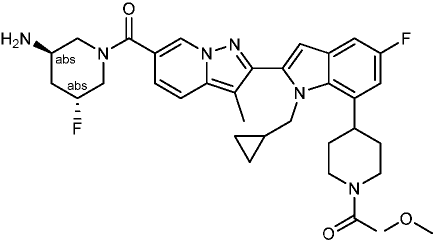 1-(4-(2-(6-((3R,5R)-3-Amino-5-fluoropiperidine-1-carbonyl)-3-methylpyrazolo[1, 5-a]pyridin-2-yl)-1-(cyclopropylmethyl)-5-fluoro-1H-indol-7-yl)piperidin-1-yl)-2-methoxy-2-methylpropan-1-one |
| | Analytical Data: 647.3; 1.908 min (Method ) |
| 502 | 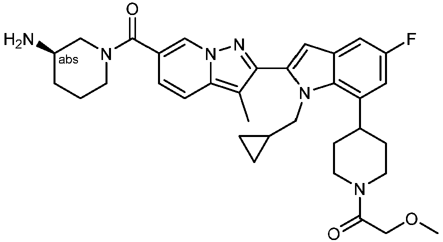 (R)-1-(4-(2-(6-(3-Aminopiperidine-1-carbonyl)-3-methylpyrazolo[1,5-a]pyridin-2-yl)-1-(cyclopropylmethyl)-5-fluoro-1H-indol-7-yl)piperidin-1-yl)-2-methoxyethan-1-one |
| | Analytical Data: 601.3; 1.513 min (Method E) |
| 503 | 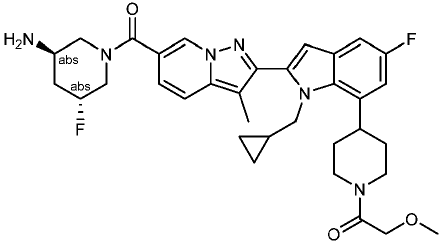 1-(4-(2-(6-((3R,5R)-3-Amino-5-fluoropiperidine-1-carbonyl)-3-methylpyrazolo[1, 5-a]pyridin-2-yl)-1-(cyclopropylmethyl)-5-fluoro-1H-indol-7-yl)piperidin-1-yl)-2-methoxyethan-1-one |
| | Analytical Data: 619.3; 1.529 min (Method E) |
| 504 | 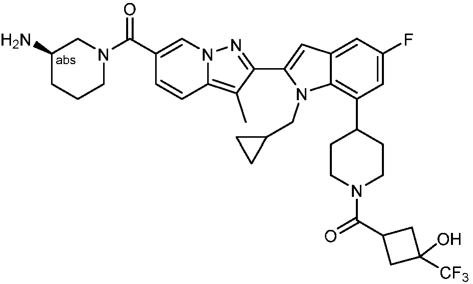 (R)-(3-Aminopiperidin-1-yl)(2-(1-(cyclopropylmethyl)-5-fluoro-7-(1-(3-hydroxy-3-(trifluoromethyl)cyclobutane-1-carbonyl)piperidin-4-yl)-1H-indol-2-yl)-3-methylpyrazolo[1,5-a]pyridin-6-yl)methanone |
| | Analytical Data: 695.3; 1.712 min (Method E) |
| 505 | 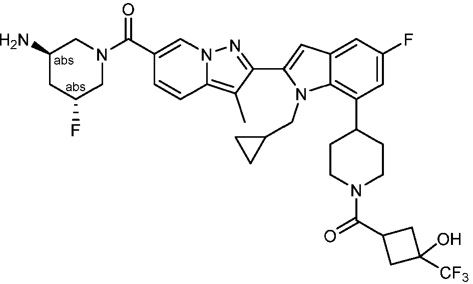 ((3R,5R)-3-Amino-5-fluoropiperidin-1-yl)(2-(1-(cyclopropylmethyl)-5-fluoro-7-(1-(3-hydroxy-3-(trifluoromethyl)cyclobutane-1-carbonyl)piperidin-4-yl)-1H-indol-2-yl)-3-methylpyrazolo[1,5-a]pyridin-6-yl)methanone (mixture of diastereomers) |
| | Analytical Data: 713.3; 1.726 min (Method E) |
| 506 | 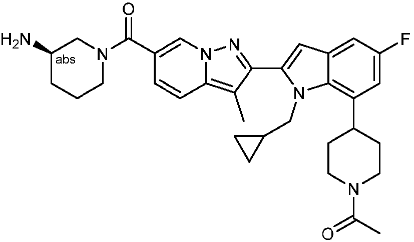 (R)-1-(4-(2-(6-(3-Aminopiperidine-1-carbonyl)-3-methylpyrazolo[1,5-a]pyridin-2-yl)-1-(cyclopropylmethyl)-5-fluoro-1H-indol-7-yl)piperidin-1-yl)ethan-1-one |
| | Analytical Data: 571.3; 1.508 min (Method E) |
| 507 | 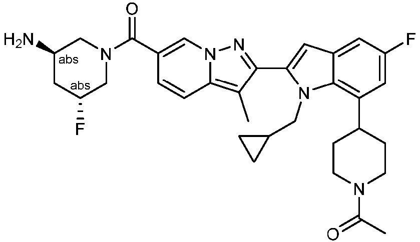 1-(4-(2-(6-((3R,5R)-3-Amino-5-fluoropiperidine-1-carbonyl)-3-methylpyrazolo[1, 5-a]pyridin-2-yl)-1-(cyclopropylmethyl)-5-fluoro-1H-indol-7-yl)piperidin-1-yl)ethan-1-one |
| | Analytical Data: 589.2; 1.573 min (Method E) |
| 508 | 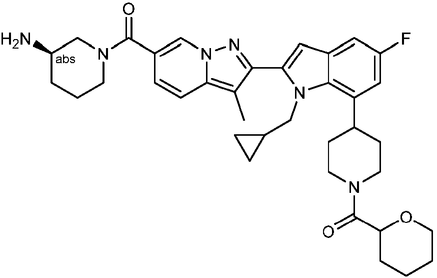 ((R)-3-Aminopiperidin-1-yl)(2-(1-(cyclopropylmethyl)-5-fluoro-7-(1-(tetrahydro-2H-pyran-2-carbonyl)piperidin-4-yl)-1H-indol-2-yl)-3-methylpyrazolo[1,5-a]pyridin-6-yl)methanone (mixture of diastereomers) |
| | Analytical Data: 641.4; 1.732 min (Method E) |
| 509 | 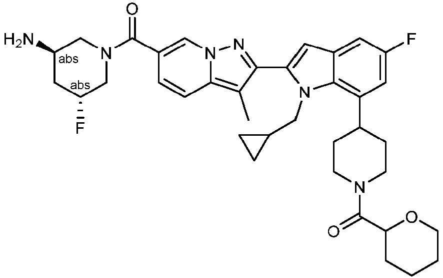 ((3R,5R)-3-Amino-5-fluoropiperidin-1-yl)(2-(1-(cyclopropylmethyl)-5-fluoro-7-(1-(tetrahydro-2H-pyran-2-carbonyl)piperidin-4-yl)-1H-indol-2-yl)-3-methylpyrazolo[1,5-a]pyridin-6-yl)methanone (mixture of diastereomers) |
| | Analytical Data: 659.3; 1.711 min (Method E) |

### Example 510

### (R)-1-(4-(2-(6-(3-Aminopiperidine-1-carbonyl)-4-methoxy-3-methylpyrazolo[1,5-a]pyridin-2-yl)-1-(cyclopropylmethyl)-1H-pyrrolo[2,3-c]pyridin-7-yl)piperidin-1-yl)-2-methoxyethan-1-one

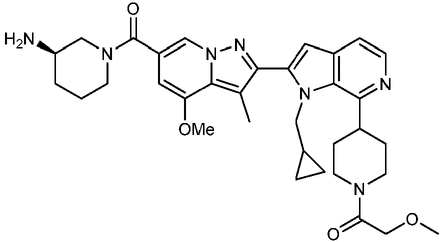

### Intermediate 510A

### methyl 2-(7-(1-(tert-butoxycarbonyl)piperidin-4-yl)-1-(cyclopropylmethyl)-1H-pyrrolo[2,3-c]pyridin-2-yl)-4-methoxy-3-methylpyrazolo[1,5-a]pyridine-6-carboxylate

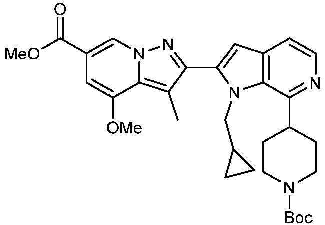

1-Amino-3-methoxy-5-(methoxycarbonyl)pyridin-1-ium 2,4 dinitro phenolate salt (2.120 g, 5.79 mmol) and copper(I) iodide (0.110 g, 0.579 mmol) were added to a stirred solution of Intermediate 44 (0.85 g, 1.929 mmol) in DMF (3 ml). The reaction mixture was stirred at 80°C for 30 hours, allowed to cool to a room temperature, diluted with water (20 ml), quenched with saturated NaHCO₃ solution (10 ml), and extracted with ethyl acetate (3×20 ml). The combined organic layers were washed with brine, dried over sodium sulfate, filtered, and concentrated in vacuum to obtain a crude product, which was purified using a silica gel column, eluting with 0-70% ethyl acetate in hexane to afford methyl 2-(7-(1-(tert-butoxycarbonyl)piperidin-4-yl)-1-(cyclopropylmethyl)-1H-pyrrolo[2,3-c]pyridin-2-yl)-4-methoxy-3-methylpyrazolo[1,5-a]pyridine-6-carboxylate (520 mg, 47%). LC-MS m/z: 574.3 [M+H]⁺.

### Intermediate 510B

### Methyl 2-(1-(cyclopropylmethyl)-7-(piperidin-4-yl)-1H-pyrrolo[2,3-c]pyridin-2-yl)-4-methoxy-3-methylpyrazolo[1,5-a]pyridine-6-carboxylate

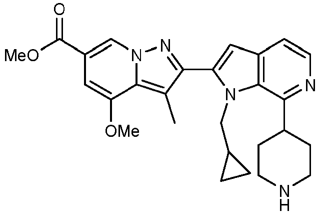

TFA (0.698 ml, 9.06 mmol) was added to a stirred solution of Intermediate 510A (520 mg, 0.906 mmol) in dichloromethane (10 ml). The reaction mixture was stirred at a room temperature for 16 hours, the salts thus obtained were treated with NaHCO₃ solution, and extracted with ethyl acetate (2×25 ml). The combined organic layers were washed with brine, dried over sodium sulfate, filtered, and concentrated in vacuum to afford methyl 2-(1-(cyclopropylmethyl)-7-(piperidin-4-yl)-1H-pyrrolo[2,3-c]pyridin-2-yl)-4-methoxy-3-methylpyrazolo[1,5-a]pyridine-6-carboxylate (410 mg, 96%). LC-MS m/z: 474.2 [M+H]⁺.

### Intermediate 510C

### Methyl 2-(1-(cyclopropylmethyl)-7-(1-(2-methoxyacetyl)piperidin-4-yl)-1H-pyrrolo[2,3-c]pyridin-2-yl)-4-methoxy-3-methylpyrazolo[1,5-a]pyridine-6-carboxylate

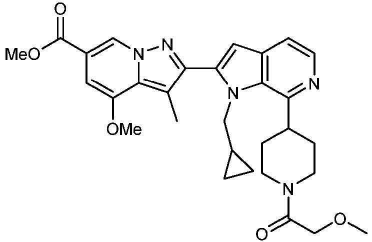

HATU (181 mg, 0.475 mmol) and 2-methoxyacetic acid (42.8 mg, 0.475 mmol) followed by DIPEA (0.138 ml, 0.792 mmol) were added to a stirred solution of Intermediate 510B (150 mg, 0.317 mmol) in DMF (2.0 ml). The reaction mixture was stirred for 2 hours, diluted with water (5 ml), quenched with saturated NaHCO₃ solution (10 ml), and extracted with ethyl acetate (3×25 ml). The combined organic layers were washed with brine, dried over sodium sulfate, filtered, and concentrated in vacuum to afford methyl 2-(1-(cyclopropylmethyl)-7-(1-(2-methoxyacetyl)piperidin-4-yl)-1H-pyrrolo[2,3-c]pyridin-2-yl)-4-methoxy-3-methylpyrazolo[1,5-a]pyridine-6-carboxylate (130 mg, 75%) LC-MS m/z: 546.3 [M+H]⁺.

### Intermediate 510D

### 2-(1-(Cyclopropylmethyl)-7-(1-(2-methoxyacetyl)piperidin-4-yl)-1H-pyrrolo[2,3-c]pyridin-2-yl)-4-methoxy-3-methylpyrazolo[1,5-a]pyridine-6-carboxylic acid

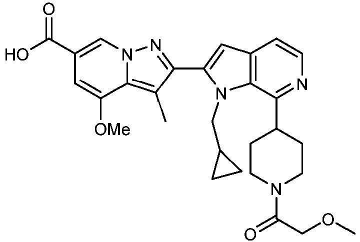

LiOH (30.7 mg, 1.283 mmol) was added to a stirred solution of Intermediate 510C (140 mg, 0.257 mmol) in THF (5 ml), MeOH (1 ml), water (2 ml). The reaction mixture stirred for 4 hours, diluted with water (5 ml) and 1N HCl (2 ml), and extracted with ethyl acetate (2×25ml). The combined organic layers were washed with brine, dried over sodium sulfate, filtered, and concentrated in vacuum to afford 2-(1-(cyclopropylmethyl)-7-(1-(2-methoxyacetyl)piperidin-4-yl)-1H-pyrrolo[2,3-c]pyridin-2-yl)-4-methoxy-3-methylpyrazolo[1,5-a]pyridine-6-carboxylic acid (90 mg, 66%). LC-MS m/z: 532.2 [M+H]⁺.

### Intermediate 510E

### (R)-1-(4-(2-(6-(3-Aminopiperidine-1-carbonyl)-4-methoxy-3-methylpyrazolo[1,5-a]pyridin-2-yl)-1-(cyclopropylmethyl)-1H-pyrrolo[2,3-c]pyridin-7-yl)piperidin-1-yl)-2-methoxyethan-1-one

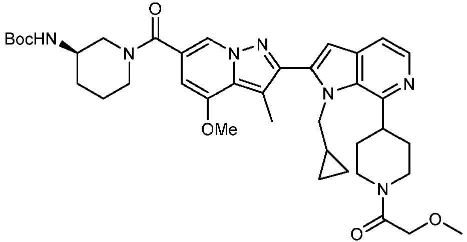

tert-Butyl (R)-piperidin-3-ylcarbamate (25.4 mg, 0.127 mmol), HATU (48.3 mg, 0.127 mmol) followed by DIPEA (0.037 ml, 0.212 mmol) were added to a stirred solution of Intermediate 510D (45 mg, 0.085 mmol) in DMF (1.0 ml). The reaction mixture was stirred 16 hours, diluted with water (5 ml), and extracted with ethyl acetate (2×10ml). The combined organic layers were washed with brine, dried over sodium sulfate, filtered, and concentrated in vacuum to afford tert-butyl (R)-(1-(2-(1-(cyclopropylmethyl)-7-(1-(2-methoxyacetyl)piperidin-4-yl)-1H-pyrrolo[2,3-c]pyridin-2-yl)-4-methoxy-3-methylpyrazolo[1,5-a]pyridine-6-carbonyl)piperidin-3-yl)carbamate (40 mg, 66%). LC-MS m/z: 714.3 [M+H]⁺.

### Example 510

### (R)-1-(4-(2-(6-(3-Aminopiperidine-1-carbonyl)-4-methoxy-3-methylpyrazolo[1,5-a]pyridin-2-yl)-1-(cyclopropylmethyl)-1H-pyrrolo[2,3-c]pyridin-7-yl)piperidin-1-yl)-2-methoxyethan-1-one

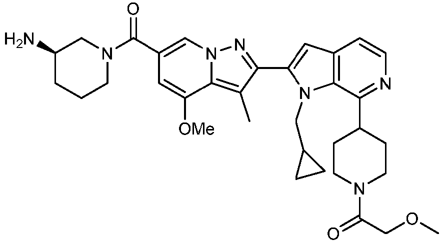

TFA (0.043 ml, 0.560 mmol) was added to a stirred solution of Intermediate 510E (40 mg, 0.056 mmol) in dichloromethane (5 ml) at 0°C. The reaction mixture was allowed to warm to room a temperature and stirred for 3 hours, concentrated in vacuum to obtain a crude compound, which was purified using preparative LC/MS with the following conditions: Column: Waters X Bridge C18, 150×19 mm, 5 µm particles; mobile phase A: 5:95 acetonitrile: water with 0.1% trifluoroacetic acid; mobile phase B: 95:5 acetonitrile: water with 0.1% trifluoroacetic acid; gradient: a 0-minute hold at 20% B, 20-40% B over 20 minutes, then a 5-minute hold at 100% B; flow rate: 20 ml/min; column temperature: 25°C. Fraction collection was triggered by signals. Fractions containing the desired product were combined and dried via centrifugal evaporation. The purified material was then diluted with a 1:1 mixture of ethylene dichloride and methanol (EDM), treated with Si-Pyridine, and shaken for a minimum of 2 hours, filtered and dried using centrifugal evaporation to afford (R)-1-(4-(2-(6-(3-aminopiperidine-1-carbonyl)-4-methoxy-3-methylpyrazolo[1,5-a]pyridin-2-yl)-1-(cyclopropylmethyl)-1H-pyrrolo[2,3-c]pyridin-7-yl)piperidin-1-yl)-2-methoxyethan-1-one. LC-MS m/z: 614.3 [M+H]⁺. Retention time: 1.188 min (Method E).

### Example 511

### 1-(4-(2-(6-((3R,5R)-3-Amino-5-fluoropiperidine-1-carbonyl)-4-methoxy-3-methylpyrazolo[1,5-a]pyridin-2-yl)-1-(cyclopropylmethyl)-1H-pyrrolo[2,3-c]pyridin-7-yl)piperidin-1-yl)-2-methoxyethan-1-one

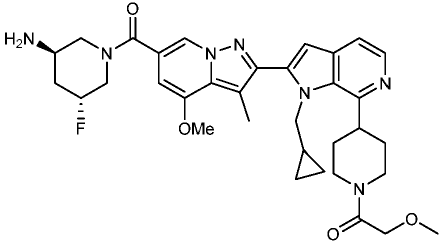

Compound of Example 511 was prepared following a procedure similar to the preparation of a compound of Example 510, by acid amine coupling of Intermediate 510B with appropriate carboxylic acid followed by ester hydrolysis and coupling with appropriate amine. LC-MS m/z [M+H]⁺: 632.3. HPLC retention time: 1.309 min (Method E)

### Example 512

### (R)-1-(4-(2-(6-(3-Aminopiperidine-1-carbonyl)-3-methylpyrazolo[1,5-a]pyridin-2-yl)-1-(cyclopropylmethyl)-1H-pyrrolo[2,3-c]pyridin-7-yl)piperidin-1-yl)-2-methoxyethan-1-one

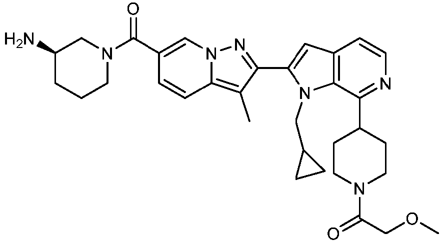

### Intermediate 512A

### Ethyl 2-(7-(1-(tert-butoxycarbonyl)piperidin-4-yl)-1-(cyclopropylmethyl)-1H-pyrrolo[2,3-c]pyridin-2-yl)-3-methylpyrazolo[1,5-a]pyridine-6-carboxylate

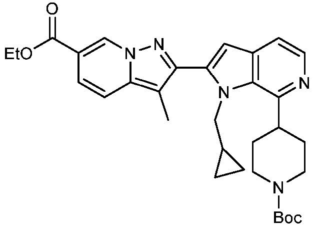

Intermediate 512A (480 mg, 45%) was prepared by following a similar procedure depicted for Intermediate 386A using the intermediates 44 and 8. LC-MS m/z: 558.3 [M+H]⁺; ¹H NMR (300 MHz, DMSO-d₆) δ ppm:9.21 (s, 1H), 8.18-8.12 (m, 1H), 7.87 (d, J=9.4 Hz, 1H), 7.64 (d, J=9.4 Hz, 1H), 7.48 (d, J=5.3 Hz, 1H), 6.89 (s, 1H), 4.62-4.52 (m, 2H), 4.46-4.29 (m, 2H), 4.21-4.01 (m, 2H), 3.11-2.81 (m, 2H), 2.38 (s, 3H), 1.93-1.79 (m, 4H), 1.59-1.50 (m, 1H), 1.44 (s, 9H), 1.39-1.34 (m, 3H),1.09-0.95 (m, 1H), 0.32-0.21 (m, 2H), -0.11--0.18 (m, 2H).

### Intermediate 512B

### Ethyl 2-(1-(cyclopropylmethyl)-7-(piperidin-4-yl)-1H-pyrrolo[2,3-c]pyridin-2-yl)-3-methylpyrazolo[1,5-a]pyridine-6-carboxylate

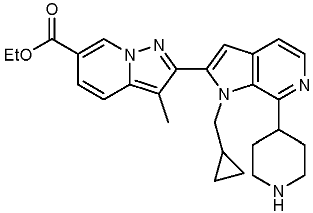

Intermediate 512B (320 mg, 81%) was prepared in similar fashion to Intermediate 510B. LC-MS m/z: 458.3 [M+H]⁺.

### Intermediate 512C

### ethyl 2-(1-(cyclopropylmethyl)-7-(1-(2-methoxyacetyl)piperidin-4-yl)-1H-pyrrolo[2,3-c]pyridin-2-yl)-3-methylpyrazolo[1,5-a]pyridine-6-carboxylate

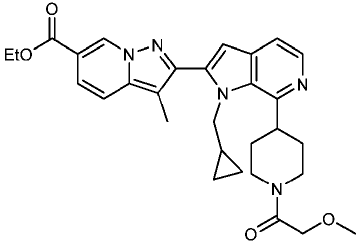

Intermediate 512C (130 mg, 0, 75%) was prepared in similar fashion to intermediate 510C. LC-MS m/z: 530.3 [M+H]⁺.

### Intermediate 512D

### 2-(1-(cyclopropylmethyl)-7-(1-(2-methoxyacetyl)piperidin-4-yl)-1H-pyrrolo[2,3-c]pyridin-2-yl)-3-methylpyrazolo[1,5-a]pyridine-6-carboxylic acid

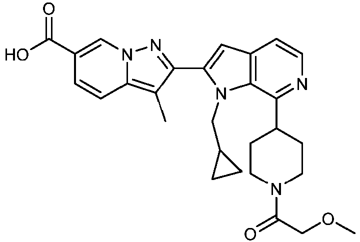

Intermediate 512D (90 mg, 68%) was prepared in similar fashion to intermediate 510D. LC-MS m/z: 502.2 [M+H]⁺

### Intermediate 512E

### tert-butyl (R)-(1-(2-(1-(cyclopropylmethyl)-7-(1-(2-methoxyacetyl)piperidin-4-yl)-1H-pyrrolo[2,3-c]pyridin-2-yl)-3-methylpyrazolo[1,5-a]pyridine-6-carbonyl)piperidin-3-yl)carbamate

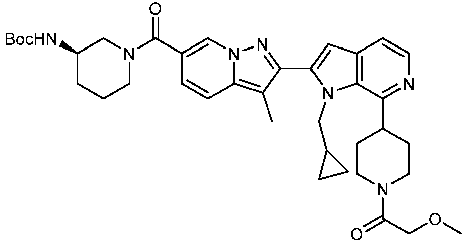

Intermediate 512E (40 mg, 65%). was prepared in similar fashion to intermediate 510E. LC-MS m/z: 684.3 [M+H]⁺

### Example 512

### (R)-1-(4-(2-(6-(3-Aminopiperidine-1-carbonyl)-3-methylpyrazolo[1,5-a]pyridin-2-yl)-1-(cyclopropylmethyl)-1H-pyrrolo[2,3-c]pyridin-7-yl)piperidin-1-yl)-2-methoxyethan-1-one

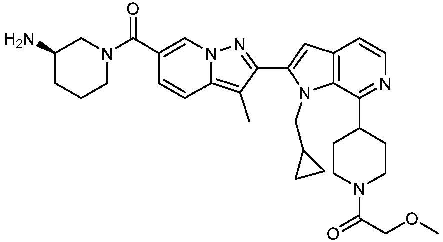

Prepared in similar fashion to Example 510. LC-MS m/z: 584.3 [M+H]⁺ 1.128 min (Method E)

### Example 513

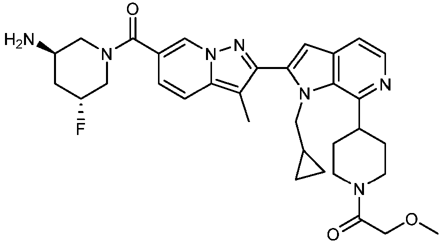

### 1-(4-(2-(6-((3R,5R)-3-Amino-5-fluoropiperidine-1-carbonyl)-3-methylpyrazolo[1,5-a]pyridin-2-yl)-1-(cyclopropylmethyl)-1H-pyrrolo[2,3-c]pyridin-7-yl)piperidin-1-yl)-2-methoxyethan-1-one

Compounds of Example 513 in Table 52 was prepared following a procedure similar to the preparation of a compound of Example 512, by acid amine coupling of Intermediate 512B with appropriate carboxylic acid followed by ester hydrolysis and coupling with appropriate amines
Analytical Data: LC-MS m/z [M+H]+; HPLC retention time (HPLC method
Analytical Data:602.3, 1.188 min (Method E)

### Example 514

### ((3R,5R)-3-Amino-5-fluoropiperidin-1-yl)(2-(1-(cyclopropylmethyl)-7-(1-((1R,4R)-4-hydroxycyclohexane-1-carbonyl)azetidin-3-yl)-1H-indol-2-yl)-4-methoxy-3-methylpyrazolo[1,5-a]pyridin-6-yl)methanone

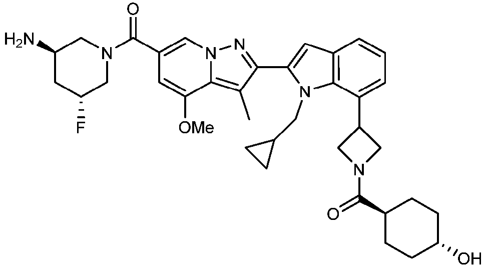

### Intermediate 514A

### Methyl 2-(7-(1-(tert-butoxycarbonyl)azetidin-3-yl)-1-(cyclopropylmethyl)-1H-indol-2-yl)-4-methoxy-3-methylpyrazolo[1,5-a]pyridine-6-carboxylate

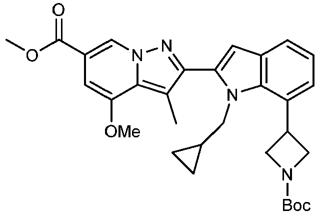

Intermediate 1 (3.20 g, 8.75 mmol) was added to a stirred solution of Intermediate 49 (1.2 g, 2.92 mmol) and CuI (0.167 g, 0.875 mmol) in DMF (10 ml). The reaction mixture was stirred at 80°C for 30 hours, diluted with water (20 ml), and extracted with ethyl acetate (3×30 ml). The combined organic layers were washed with brine, dried over sodium sulfate, filtered, and concentrated in vacuum. The crude compound thus obtained was purified using a silica gel column, eluting with 0-20% ethyl acetate in hexane to afford methyl 2-(7-(1-(tert-butoxycarbonyl)azetidin-3-yl)-1-(cyclopropylmethyl)-1H-indol-2-yl)-4-methoxy-3-methylpyrazolo[1,5-a]pyridine-6-carboxylate (1.3 g, 82%). LC-MS m/z: 545.4 [M+H]⁺.

### Intermediate 514B

### Methyl 2-(7-(azetidin-3-yl)-1-(cyclopropylmethyl)-1H-indol-2-yl)-4-methoxy-3-methylpyrazolo[1,5-a]pyridine-6-carboxylate

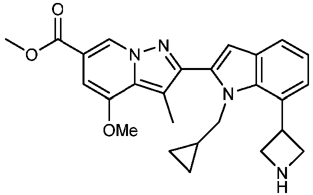

TFA (1.839 ml, 23.87 mmol) was added to a stirred solution of Intermediate 514A (1.3 g, 2.387 mmol) in dichloromethane (20 ml). The reaction mixture was stirred at a room temperature for 16 hours, diluted with water (10 ml), and saturated sodium bicarbonate solution (10 ml), and extracted with ethyl acetate (3×20 ml). The combined organic layers were washed with brine, dried over sodium sulfate, filtered, and concentrated in vacuum to afford methyl 2-(7-(azetidin-3-yl)-1-(cyclopropylmethyl)-1H-indol-2-yl)-4-methoxy-3-methylpyrazolo[1,5-a]pyridine-6-carboxylate (1 g, 94%). LC-MS m/z: 445.4 [M+H] ⁺.

### Intermediate 514C

### Methyl 2-(1-(cyclopropylmethyl)-7-(1-((1R, 4R)-4-hydroxycyclohexane-1-carbonyl)azetidin-3-yl)-1H-indol-2-yl)-4-methoxy-3-methylpyrazolo[1,5-a]pyridine-6-carboxylate

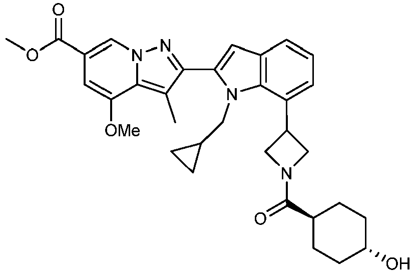

(1R,4R)-4-Hydroxycyclohexane-1-carboxylic acid (63.2 mg, 0.439 mmol) was added to a stirred solution of Intermediate 514B (150 mg, 0.337 mmol) in DMF (3 ml), followed by HATU (192 mg, 0.506 mmol) and DIPEA (0.118 ml, 0.675 mmol). The reaction mixture was stirred at a room temperature for 16 hours, diluted with water (20 ml), and extracted with ethyl acetate (3×30 ml). The combined organic layers were washed with brine, dried over sodium sulfate, filtered, and concentrated in vacuum. The crude product was purified using a silica gel column, eluting with 0-5% methanol in dichloromethane to afford methyl 2-(1-(cyclopropylmethyl)-7-(1-((1R,4R)-4-hydroxycyclohexane-1-carbonyl)azetidin-3-yl)-1H-indol-2-yl)-4-methoxy-3-methylpyrazolo[1,5-a]pyridine-6-carboxylate (110 mg, 57%). LC-MS m/z: 571.4 [M+H]⁺.

### Intermediate 514D

### 2-(1-(cyclopropylmethyl)-7-(1-((1r,4r)-4-hydroxycyclohexane-1-carbonyl)azetidin-3-yl)-1H-indol-2-yl)-4-methoxy-3-methylpyrazolo[1,5-a]pyridine-6-carboxylic acid

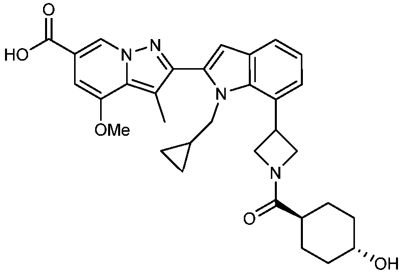

LiOH (25.2 mg, 1.051 mmol) was added to a stirred solution of Intermediate 514C (120 mg, 0.210 mmol) in THF (3 ml), water (0.5 ml), and MeOH (1 ml). The reaction mixture was stirred at a room temperature for 16 hours, diluted with water (5 ml) and 1N HCl (5 ml), and extracted with ethyl acetate (3×30 ml). The combined organic layers were washed with brine, dried over sodium sulfate, filtered, and concentrated in vacuum to get 2-(1-(cyclopropylmethyl)-7-(1-((1R, 4R)-4-hydroxycyclohexane-1-carbonyl)azetidin-3-yl)-1H-indol-2-yl)-4-methoxy-3-methylpyrazolo[1,5-a]pyridine-6-carboxylic acid (90 mg, 77%). LC-MS m/z: 557.4 [M+H]⁺.

### Intermediate 514E

### tert-Butyl ((3R,5R)-1-(2-(1-(cyclopropylmethyl)-7-(1-((1R,4R)-4-hydroxycyclohexane-1-carbonyl)azetidin-3-yl)-1H-indol-2-yl)-4-methoxy-3-methylpyrazolo[1,5-a]pyridine-6-carbonyl)-5-fluoropiperidin-3-yl)carbamate

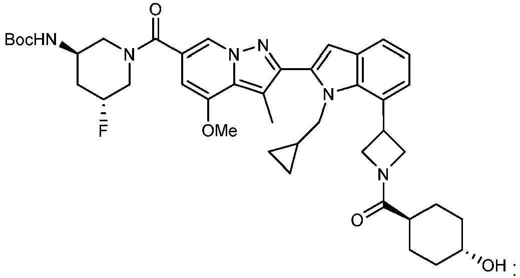

tert-Butyl ((3R,5R)-5-fluoropiperidin-3-yl)carbamate (21.17 mg, 0.097 mmol), HATU (46.1 mg, 0.121 mmol) followed by DIPEA (0.042 ml, 0.243 mmol) were added to a stirred solution of Intermediate 514D (45 mg, 0.081 mmol) in DMF (0.5 ml). The reaction mixture was stirred at a room temperature for 16 hours, diluted with water (5 ml) and 1 N HCl (5 ml), and extracted with ethyl acetate (3×30 ml). The combined organic layers were washed with brine, dried over sodium sulfate, filtered, and concentrated in vacuum to afford tert-butyl ((3R,5R)-1-(2-(1-(cyclopropylmethyl)-7-(1-((1R,4R)-4-hydroxycyclohexane-1-carbonyl)azetidin-3-yl)-1H-indol-2-yl)-4-methoxy-3-methylpyrazolo[1,5-a]pyridine-6-carbonyl)-5-fluoropiperidin-3-yl)carbamate (40 mg, 65%). LC-MS m/z: 757.6 [M+H]⁺.

### Example 514

### ((3R,5R)-3-Amino-5-fluoropiperidin-1-yl)(2-(1-(cyclopropylmethyl)-7-(1-((1R,4R)-4-hydroxycyclohexane-1-carbonyl)azetidin-3-yl)-1H-indol-2-yl)-4-methoxy-3-methylpyrazolo[1,5-a]pyridin-6-yl)methanone

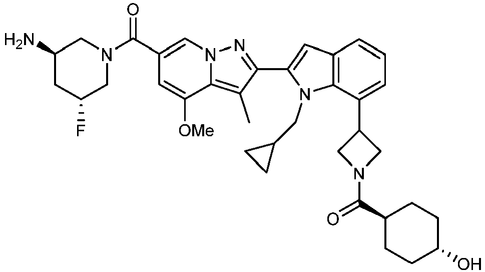

TFA (0.041 ml, 0.528 mmol) was added to a stirred solution of Intermediate 514E (40 mg, 0.053 mmol) in CH₂Cl₂ (3.0 ml). The reaction mixture was stirred at a room temperature for 6 hours, concentrated, and the crude material was purified using preparative LC/MS with the following conditions: Column: Waters X Bridge C18, 19×150 mm, 5 µm particles; mobile phase A: 10 mM ammonium acetate; mobile phase B: acetonitrile; gradient: 12-48% B over 25 minutes, then a 5-minute hold at 100% B; flow rate: 15 ml/min. Fractions containing the desired product were combined and dried using centrifugal evaporation. The purified material was then diluted with a 1:1 mixture of DCM and methanol, treated with Si-Pyridine, and shaken for a minimum of 2 hours. The resulting mixture was filtered and dried using centrifugal evaporation to afford ((3R,5R)-3-amino-5-fluoropiperidin-1-yl)(2-(1-(cyclopropylmethyl)-7-(1-((1r,4r)-4-hydroxycyclohexane-1-carbonyl)azetidin-3-yl)-1H-indol-2-yl)-4-methoxy-3-methylpyrazolo[1,5-a]pyridin-6-yl)methanone (11.2 mg, 32%). LC-MS m/z: 657.4 [M+H]⁺. LCMS retention time: 1.537 min (method E). ¹H NMR (400 MHz, DMSO-*d₆*) δ ppm: 8.36 (s, 1H), 7.58-7.49 (m, 1H), 7.37 (d, J=7.3 Hz, 1H), 7.16 (t, J=7.6 Hz, 1H), 6.76 (s, 1H), 6.54 (d, J =0.8 Hz, 1H), 5.02-4.88 (m, 1H), 4.74-4.68 (m, 1H), 4.68-4.59 (m, 1H), 4.54 (br, d, J=3.0 Hz, 1H), 4.41-4.31 (m, 5H), 4.01 (br, dd, J=6.1, 9.4Hz, 2H), 3.97 (s, 3H), 3.10-3.03 (m, 2H), 2.48 (s, 3H), 2.20 (tt, J=3.4, 11.6 Hz, 2H), 1.89-1.80 (m, 3H), 1.76-1.49 (m, 3H), 1.43-1.30 (m,2H), 1.23-1.11 (m, 3H), 0.83-0.72 (m, 1H), 0.16 (d, J=8.3 Hz, 2H), -0.16--0.34 (m, 2H)

Compounds of Examples 515 to 529 in Table 51 were prepared following a procedure similar to the preparation of a compound of Example 514, by acid amine coupling of Intermediate 514B with appropriate carboxylic acid followed by ester hydrolysis and coupling with appropriate amines

**Table 51**

| Example | Structure |
|---|---|
| | Name |
| | Analytical Data: LC-MS m/z [M+H]+; HPLC retention time (HPLC method) |
| 515 | 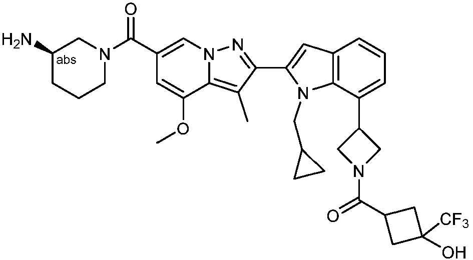 |
| | (R)-(3-Aminopiperidin-1-yl)(2-(1-(cyclopropylmethyl)-7-(1-(3-hydroxy-3-(trifluoromethyl)cyclobutane-1-carbonyl)azetidin-3-yl)-1H-indol-2-yl)-4-methoxy-3-methylpyrazolo[1,5-a]pyridin-6-yl)methanone (mixture of diastereomers) |
| | Analytical Data: 679.3, 1.754 min (Method E) |
| 516 | 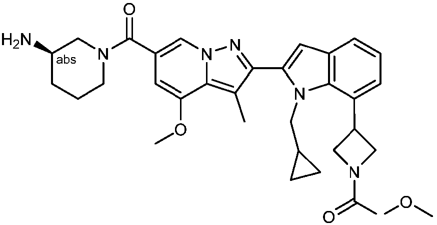 |
| | (R)-1-(3-(2-(6-(3-Aminopiperidine-1-carbonyl)-4-methoxy-3-methylpyrazolo[1, 5-a]pyridin-2-yl)-1-(cyclopropylmethyl)-1H-indol-7-yl)azetidin-1-yl)-2-methoxy-2-methylpropan-1-one |
| | Analytical Data: 613.3, 1.733 min (Method E) |
| 517 | 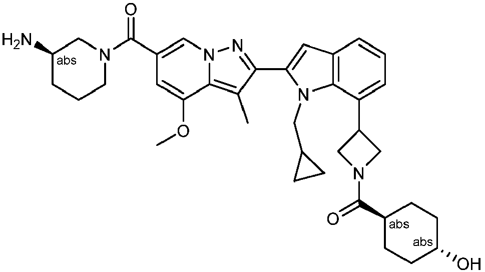 |
| | ((R)-3-Aminopiperidin-1-yl)(2-(1-(cyclopropylmethyl)-7-(1-((1r,4r)-4-hydroxycyclohexane-1-carbonyl)azetidin-3-yl)-1H-indol-2-yl)-4-methoxy-3-methylpyrazolo[1, 5-a]pyridin-6-yl)methanone |
| | Analytical Data: 639.4, 1.489 min (Method E) |
| 518 | 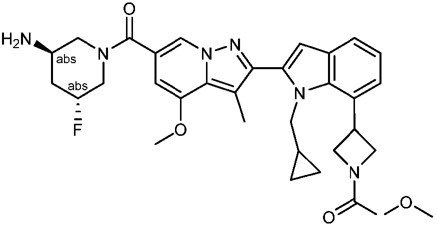 |
| | 1-(3-(2-(6-((3R,5R)-3-Amino-5-fluoropiperidine-1-carbonyl)-4-methoxy-3-methylpyrazolo[1, 5-a]pyridin-2-yl)-1-(cyclopropylmethyl)-1H-indol-7-yl)azetidin-1-yl)-2-methoxy-2-methylpropan-1-one |
| | Analytical Data: 631.3, 1.788 min (Method E) |
| 519 | 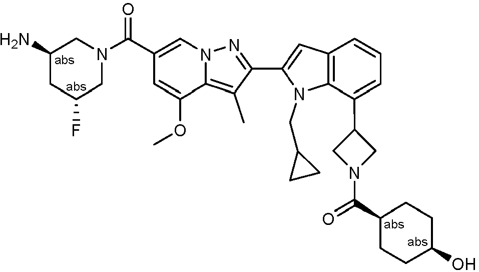 |
| | ((3R,5R)-3-Amino-5-fluoropiperidin-1-yl)(2-(1-(cyclopropylmethyl)-7-(1-((1s,4s)-4-hydroxycyclohexane-1-carbonyl)azetidin-3-yl)-1H-indol-2-yl)-4-methoxy-3-methylpyrazolo[1,5-a]pyridin-6-yl)methanone |
| | Analytical Data: 657.4, 1.630 min (Method E) |
| 520 | 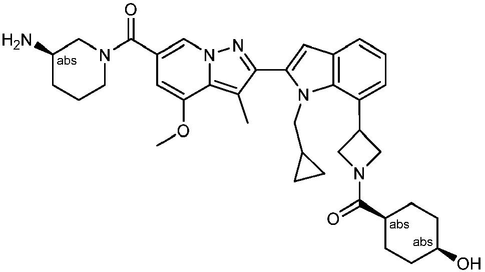 |
| | ((R)-3-Aminopiperidin-1-yl)(2-(1-(cyclopropylmethyl)-7-(1-((1s,4s)-4-hydroxycyclohexane-1-carbonyl)azetidin-3-yl)-1H-indol-2-yl)-4-methoxy-3-methylpyrazolo[1,5-a]pyridin-6-yl)methanone |
| | Analytical Data: 639.4, 1.581 min (Method E) |
| 521 | 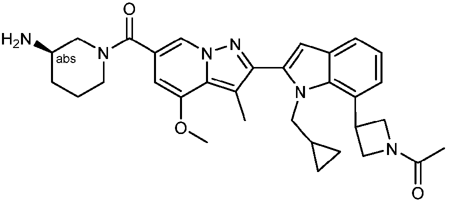 |
| | (R)-1-(3-(2-(6-(3-Aminopiperidine-1-carbonyl)-4-methoxy-3-methylpyrazolo[1, 5-a]pyridin-2-yl)-1-(cyclopropylmethyl)-1H-indol-7-yl)azetidin-1-yl)ethan-1-one |
| | Analytical Data: 555.3, 1.506 min (Method E) |
| 522 | 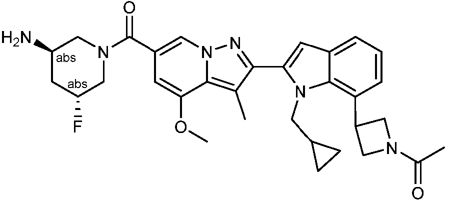 |
| | 1-(3-(2-(6-((3R,5R)-3-Amino-5-fluoropiperidine-1-carbonyl)-4-methoxy-3-methylpyrazolo[1, 5-a]pyridin-2-yl)-1-(cyclopropylmethyl)-1H-indol-7-yl)azetidin-1-yl)ethan-1-one |
| | Analytical Data: 573.3, 1.593 min (Method E) |
| 523 | 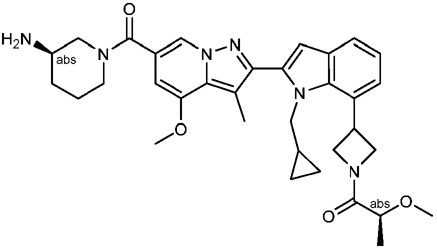 |
| | (S)-1-(3-(2-(6-((R)-3-Aminopiperidine-1-carbonyl)-4-methoxy-3-methylpyrazolo[1, 5-a]pyridin-2-yl)-1-(cyclopropylmethyl)-1H-indol-7-yl)azetidin-1-yl)-2-methoxypropan-1-one |
| | Analytical Data: 599.3, 1.605 min (Method E) |
| 524 | 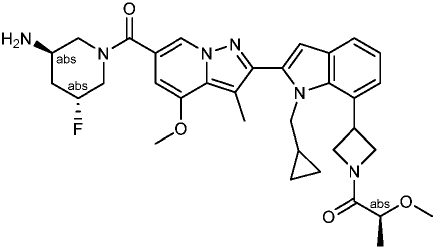 |
| | (S)-1-(3-(2-(6-((3R,5R)-3-Amino-5-fluoropiperidine-1-carbonyl)-4-methoxy-3-methylpyrazolo[1, 5-a]pyridin-2-yl)-1-(cyclopropylmethyl)-1H-indol-7-yl)azetidin-1-yl)-2-methoxypropan-1-one |
| | Analytical Data: 617.3, 1.638 min (Method E) |
| 525 | 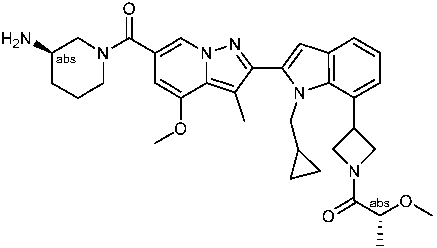 |
| | (R)-1-(3-(2-(6-((R)-3-Aminopiperidine-1-carbonyl)-4-methoxy-3-methylpyrazolo[1, 5-a]pyridin-2-yl)-1-(cyclopropylmethyl)-1H-indol-7-yl)azetidin-1-yl)-2-methoxypropan-1-one |
| | Analytical Data: 599.3, 1.613 min (Method E) |
| 526 | 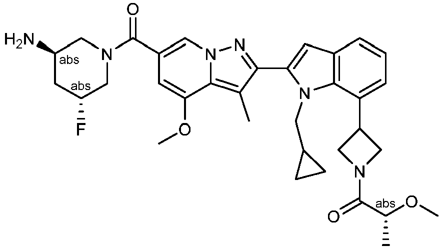 |
| | (R)-1-(3-(2-(6-((3R,5R)-3-Amino-5-fluoropiperidine-1-carbonyl)-4-methoxy-3-methylpyrazolo[1, 5-a]pyridin-2-yl)-1-(cyclopropylmethyl)-1H-indol-7-yl)azetidin-1-yl)-2-methoxypropan-1-one |
| | Analytical Data: 617.3, 1.533 min (Method F) |
| 527 | 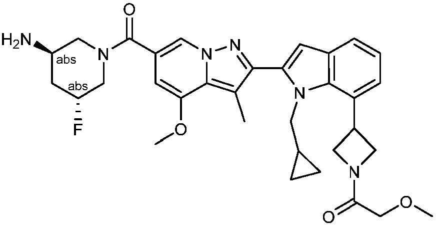 |
| | 1-(3-(2-(6-((3R,5R)-3-Amino-5-fluoropiperidine-1-carbonyl)-4-methoxy-3-methylpyrazolo[1, 5-a]pyridin-2-yl)-1-(cyclopropylmethyl)-1H-indol-7-yl)azetidin-1-yl)-2-methoxyethan-1-one |
| | Analytical Data: 603.2, 1.597 min (Method E) |
| 528 | 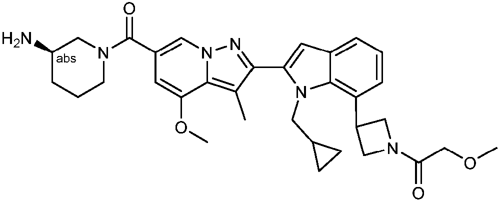 |
| | (R)-1-(3-(2-(6-(3-Aminopiperidine-1-carbonyl)-4-methoxy-3-methylpyrazolo[1, 5-a]pyridin-2-yl)-1-(cyclopropylmethyl)-1H-indol-7-yl)azetidin-1-yl)-2-methoxyethan-1-one |
| | Analytical Data: 585.3, 1.607 min (Method E) |
| 529 | 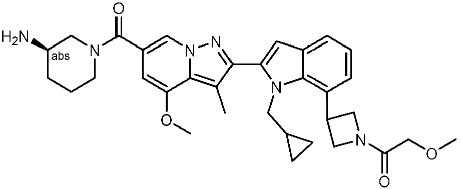 |
| | (R)-1-(3-(2-(6-(3-Aminopiperidine-1-carbonyl)-4-methoxy-3-methylpyrazolo[1, 5-a]pyridin-2-yl)-1-(cyclopropylmethyl)-1H-indol-7-yl)azetidin-1-yl)-2-methoxyethan-1-one |
| | Analytical Data: 585.3, 1.540 min (Method E) |

### Example 530

### ((R)-3-Aminopiperidin-1-yl)(2-(1-(cyclopropylmethyl)-7-(1-((1r,4r)-4-hydroxycyclohexane-1-carbonyl)azetidin-3-yl)-1H-indol-2-yl)-3-methylpyrazolo[1,5-a]pyridin-6-yl)methanone

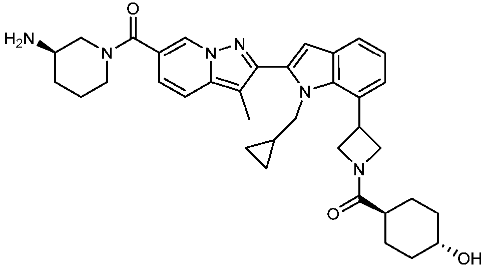

### Intermediate 530A

### Ethyl 2-(7-(1-(tert-butoxycarbonyl)azetidin-3-yl)-1-(cyclopropylmethyl)-1H-indol-2-yl)-3-methylpyrazolo[1,5-a]pyridine-6-carboxylate

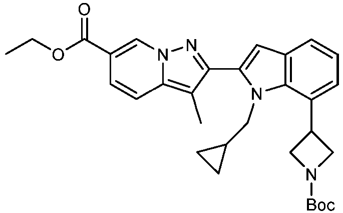

Intermediate 8 (3.83 g, 10.94 mmol) and copper(I) iodide (0.208 g, 1.08 mmol) were added to a stirred solution of Intermediate 49 (1.5 g, 3.65 mmol) in (10 ml). The reaction mixture was stirred at 80°C for 30 hours, diluted with water (15 ml), and extracted with ethyl acetate (3×30 ml). The combined organic layers were washed with brine, dried over sodium sulphate, filtered, and concentrated in vacuum. The crude product was purified using a silica gel column, eluting 20% ethyl acetate in hexane to afford (110 mg, 57%) ethyl 2-(7-(1-(tert-butoxycarbonyl)azetidin-3-yl)-1-(cyclopropylmethyl)-1H-indol-2-yl)-3-methylpyrazolo[1,5-a]pyridine-6-carboxylate. LC-MS m/z: 529.3 [M+H]⁺.

### Intermediate 530B

### Ethyl 2-(7-(azetidin-3-yl)-1-(cyclopropylmethyl)-1H-indol-2-yl)-3-methylpyrazolo[1,5-a]pyridine-6-carboxylate

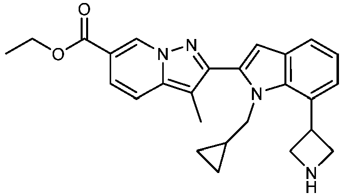

TFA (1.749 ml, 22.70 mmol) was added to a stirred solution of Intermediate 530A (1.2 g, 2.270 mmol) in dichloromethane (5 ml). The reaction mixture was stirred at a room temperature for 16 hours, concentrated, diluted with water (10 ml), washed with saturated NaHCO₃ solution (10 ml), and extracted with dichloromethane (3×20 ml). The combined organic layers were washed with brine, dried over sodium sulphate, filtered, and concentrated in vacuum to afford ethyl 2-(7-(azetidin-3-yl)-1-(cyclopropylmethyl)-1H-indol-2-yl)-3-methylpyrazolo[1,5-a]pyridine-6-carboxylate (crude 1.0 g). LC-MS m/z: 429.4 [M+H]⁺.

### Intermediate 530C

### Ethyl 2-(1-(cyclopropylmethyl)-7-(1-((1R,4R)-4-hydroxycyclohexane-1-carbonyl)azetidin-3-yl)-1H-indol-2-yl)-3-methylpyrazolo[1,5-a]pyridine-6-carboxylate

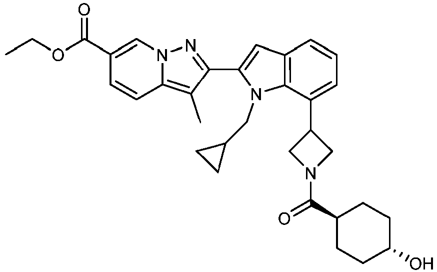

(1R,4R)-4-Hydroxycyclohexane-1-carboxylic acid (87 mg, 0.607 mmol), HATU (266 mg, 0.700 mmol) followed by DIPEA (0.245 ml, 1.400 mmol) were added to a stirred solution of Intermediate 530B (200 mg, 0.467 mmol) in DMF (5 ml). The reaction mixture was stirred at a room temperature for 1 hour, diluted with water (5 ml), and extracted with ethyl acetate (3×10 ml). The combined organic layers were washed with brine, dried over sodium sulphate, filtered, and concentrated in vacuum. The crude product thus obtained was purified using a silica gel column, eluting 2% methanol in dichloromethane to afford (130 mg, 50%) ethyl 2-(1-(cyclopropylmethyl)-7-(1-((1R,4R)-4-hydroxycyclohexane-1-carbonyl)azetidin-3-yl)-1H-indol-2-yl)-3-methylpyrazolo[1,5-a]pyridine-6-carboxylate. LC-MS m/z: 555.4 [M+H]⁺.

### Intermediate 530D

### 2-(1-(cyclopropylmethyl)-7-(1-((1R,4R)-4-hydroxycyclohexane-1-carbonyl)azetidin-3-yl)-1H-indol-2-yl)-3-methylpyrazolo[1,5-a]pyridine-6-carboxylic acid

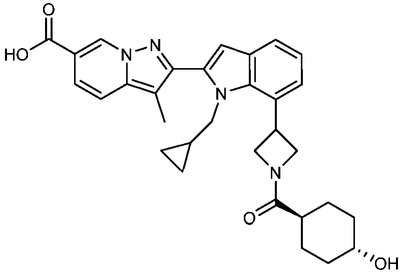

LiOH (17.27 mg, 0.721 mmol) was added to a stirred solution of Intermediate 530C (80 mg, 0.144 mmol) in THF (3 ml), methanol (1 ml), and water (0.5 ml). The reaction mixture was stirred at a room temperature for 16 hours, diluted with water (5 ml) and 1 N HCl (5 ml), and extracted with ethyl acetate (3×10 ml). The combined organic layers were washed with brine, dried over sodium sulphate, filtered, and concentrated in vacuum to afford (crude 80 mg) 2-(1-(cyclopropylmethyl)-7-(1-((1r,4r)-4-hydroxycyclohexane-1-carbonyl)azetidin-3-yl)-1H-indol-2-yl)-3-methylpyrazolo[1,5-a]pyridine-6-carboxylic acid. LC-MS m/z: 527.3 [M+H]⁺.

### Intermediate 530E

### tert-Butyl ((R)-1-(2-(1-(cyclopropylmethyl)-7-(1-((1R,4R)-4-hydroxycyclohexane-1-carbonyl)azetidin-3-yl)-1H-indol-2-yl)-3-methylpyrazolo[1,5-a]pyridine-6-carbonyl)piperidin-3-yl)carbamate

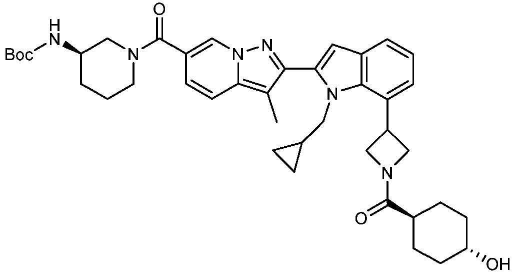

tert-Butyl (R)-piperidin-3-ylcarbamate (18.25 mg, 0.091 mmol), HATU (57.8 mg, 0.152 mmol), DIPEA (0.040 ml, 0.228 mmol) was added to a stirred solution of Intermediate 530D (40 mg, 0.076 mmol) in DMF (1 ml). The reaction mixture was stirred at a room temperature for 3 hours, diluted with water (5 ml) and 1N HCl (5 ml), and extracted with ethyl acetate (3×10 ml). The combined organic layers were washed with brine, dried over sodium sulphate, filtered, and concentrated in vacuum to afford tert-butyl ((R)-1-(2-(1-(cyclopropylmethyl)-7-(1-((1R,4R)-4-hydroxycyclohexane-1-carbonyl)azetidin-3-yl)-1H-indol-2-yl)-3-methylpyrazolo[1,5-a]pyridine-6-carbonyl)piperidin-3-yl)carbamate (40 mg, 74%). LC-MS m/z: 709.6 [M+H]⁺.

### Example 530

### ((R)-3-Aminopiperidin-1-yl)(2-(1-(cyclopropylmethyl)-7-(1-((1R,4R)-4-hydroxycyclohexane-1-carbonyl)azetidin-3-yl)-1H-indol-2-yl)-3-methylpyrazolo[1,5-a]pyridin-6-yl)methanone

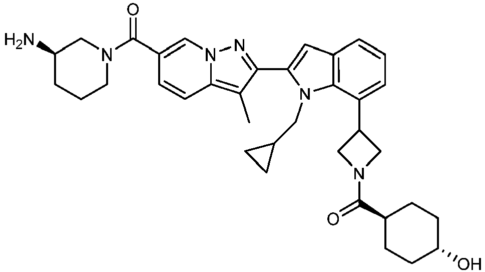

TFA (0.043 ml, 0.564 mmol) was added to a stirred solution of Intermediate 530E (40 mg, 0.056 mmol) in DCM (3 ml). The reaction mixture was stirred for 16 hours, concentrated in vacuum to obtain a crude compound, which was purified using a preparative LC/MS with the following conditions: Column: Waters XBridge C18, 150×19 mm, 5 µm particles; mobile phase A: 5:95 acetonitrile: water with 0.1% trifluoroacetic acid; mobile phase B: 95:5 acetonitrile: water with 0.1% trifluoroacetic acid; gradient: a 0-minute hold at 15% B, 15-30% B over 20 minutes, then a 5-minute hold at 100% B; flow rate: 20 ml/min; column temperature: 25°C. Fraction collection was triggered by signals. Fractions containing the desired product were combined and dried using centrifugal evaporation. The purified material was then diluted with a 1:1 mixture of ethylene dichloride and methanol (EDM), treated with Si-Pyridine, and shaken for a minimum of 2 hours. The resulting mixture was filtered and dried using centrifugal evaporation to afford ((R)-3-aminopiperidin-1-yl)(2-(1-(cyclopropylmethyl)-7-(1-((1R,4R)-4-hydroxycyclohexane-1-carbonyl)azetidin-3-yl)-1H-indol-2-yl)-3-methylpyrazolo[1,5-a]pyridin-6-yl)methanone (6.9 mg, 11%). LC-MS m/z: 609.3 [M+H]⁺. LCMS retention time: 1.411 min (method E). ¹H NMR (400 MHz, DMSO-*d₆*) δ ppm: 8.86 (s, 1H), 7.99 (br, s, 2H), 7.81 (dd, J=0.7, 9.0 Hz, 1H), 7.57 (d, J=7.8 Hz, 1H), 7.38 (d, J=7.6 Hz, 1H),7.28 (dd, J=1.5, 9.0 Hz, 1H), 7.17 (t, J=7.6 Hz, 1H),7.16-6.98 (m, 1H), 6.83 (s, 1H), 4.75-4.61 (m, 2H), 4.58-4.52 (m, 1H), 4.47-4.31 (m, 4H), 4.02 (br, s, 2H),3.82-3.74 (m,1H), 3.29-3.19 (m, 3H), 2.39 (s, 3H), 2.24-2.16 (m, 1H), 2.06-1.95 (m, 1H), 1.90-1.55 (m, 7H), 1.43-1.29 (m, 2H), 1.23-1.06 (m, 2H),0.84-0.74 (m, 1H), 0.17 (br, d, J=8.1 Hz, 2H), 0.16-0.32 (m, 2H).

Compounds of Examples 531 to 565 in Table 52 were prepared following a procedure similar to the preparation of a compound of Example 530, by acid amine coupling of Intermediate 530B with appropriate carboxylic acid followed by ester hydrolysis and coupling with appropriate amines

**Table 52**

| Example | Structure |
|---|---|
| | Name |
| | Analytical Data: LC-MS m/z [M+H]⁺; HPLC retention time (HPLC method) |
| 531 | 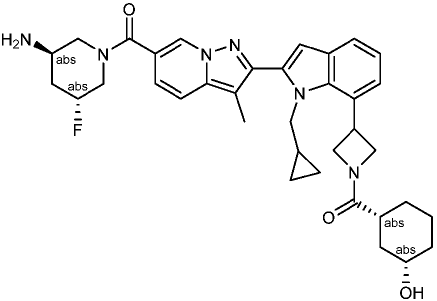 ((3R,5R)-3-Amino-5-fluoropiperidin-1-yl)(2-(1-(cyclopropylmethyl)-7-(1-((1R,3 S)-3-hydroxycyclohexane-1-carbonyl)azetidin-3-yl)-1H-indol-2-yl)-3-methylpyrazolo[1, 5-a]pyridin-6-yl)methanone |
| | Analytical Data: 627.3, 1.510min (Method E) |
| 532 | 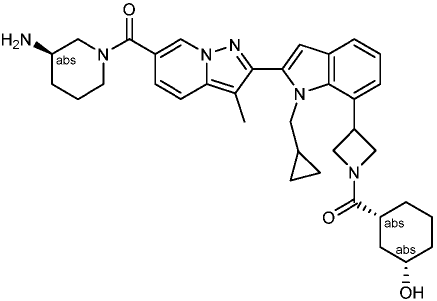 ((R)-3-Aminopiperidin-1-yl)(2-(1-(cyclopropylmethyl)-7-(1-((1R,3S)-3-hydroxycyclohexane-1-carbonyl)azetidin-3-yl)-1H-indol-2-yl)-3-methylpyrazolo[1, 5-a]pyridin-6-yl)methanone |
| | Analytical Data: 609.3, 1.446 min (Method E) |
| 533 | 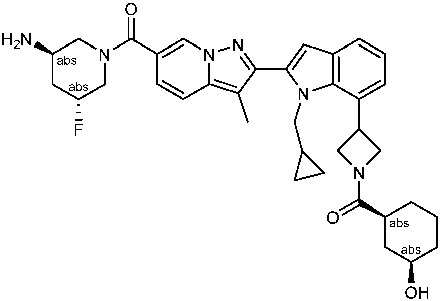 ((3R,5R)-3-Amino-5-fluoropiperidin-1-yl)(2-(1-(cyclopropylmethyl)-7-(1-((1S,3R)-3-hydroxycyclohexane-1-carbonyl)azetidin-3-yl)-1H-indol-2-yl)-3-methylpyrazolo[1, 5-a]pyridin-6-yl)methanone |
| | Analytical Data: 627.3, 1.499 min (Method E) |
| 534 | 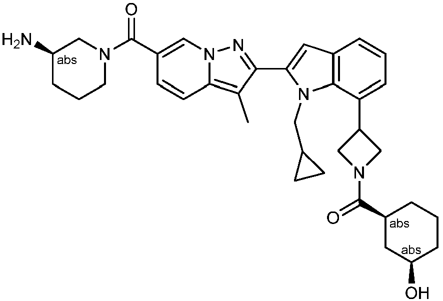 ((R)-3-Aminopiperidin-1-yl)(2-(1-(cyclopropylmethyl)-7-(1-((1S,3R)-3-hydroxycyclohexane-1-carbonyl)azetidin-3-yl)-1H-indol-2-yl)-3-methylpyrazolo[1, 5-a]pyridin-6-yl)methanone |
| | Analytical Data: 609.3, 1.446 min (Method E) |
| 535 | 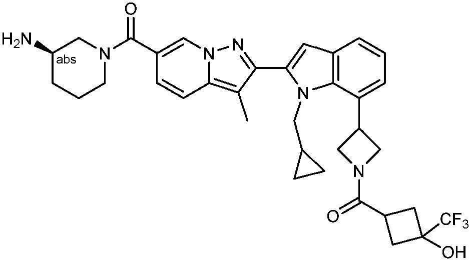 (R)-(3-Aminopiperidin-1-yl)(2-(1-(cyclopropylmethyl)-7-(1-(3-hydroxy-3-(trifluoromethyl)cyclobutane-1-carbonyl)azetidin-3-yl)-1H-indol-2-yl)-3-methylpyrazolo[1,5-a]pyridin-6-yl)methanone (mixture of diastereomers) |
| | Analytical Data: 649.3, 1.656 min (Method E) |
| 536 | 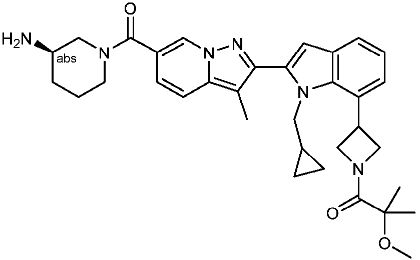 (R)-1-(3-(2-(6-(3-Aminopiperidine-1-carbonyl)-3-methylpyrazolo[1,5-a]pyridin-2-yl)-1-(cyclopropylmethyl)-1H-indol-7-yl)azetidin-1-yl)-2-methoxy-2-methylpropan-1-one |
| | Analytical Data: 583.3, 1.616 min (Method E) |
| 537 | 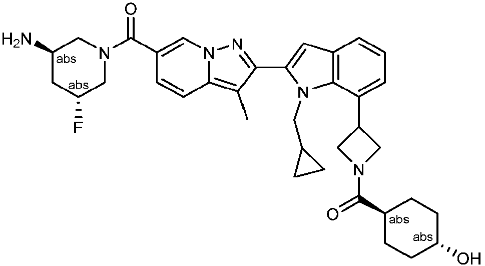 ((3R,5R)-3-Amino-5-fluoropiperidin-1-yl)(2-(1-(cyclopropylmethyl)-7-(1-((1r,4r)-4-hydroxycyclohexane-1-carbonyl)azetidin-3-yl)-1H-indol-2-yl)-3-methylpyrazolo[1, 5-a]pyridin-6-yl)methanone |
| | Analytical Data: 627.3, 1.458 min (Method E) |
| 538 | 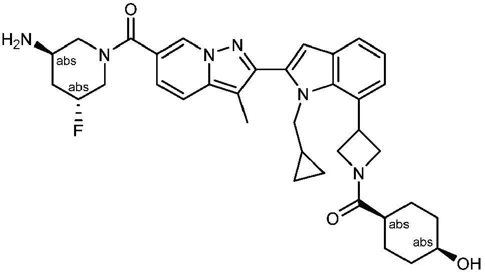 ((3R,5R)-3-Amino-5-fluoropiperidin-1-yl)(2-(1-(cyclopropylmethyl)-7-(1-((1s,4s)-4-hydroxycyclohexane-1-carbonyl)azetidin-3-yl)-1H-indol-2-yl)-3-methylpyrazolo[1, 5-a]pyridin-6-yl)methanone |
| | Analytical Data: 627.3, 1.498 min (Method E) |
| 539 | 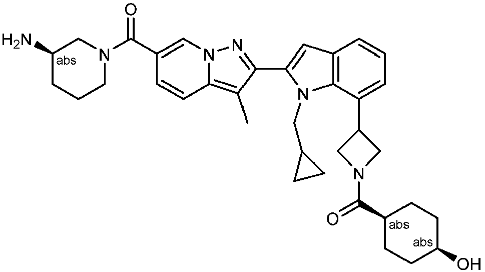 ((R)-3-Aminopiperidin-1-yl)(2-(1-(cyclopropylmethyl)-7-(1-((1s,4s)-4-hydroxycyclohexane-1-carbonyl)azetidin-3-yl)-1H-indol-2-yl)-3-methylpyrazolo[1, 5-a]pyridin-6-yl)methanone |
| | Analytical Data: 609.3, 1.440 min (Method E) |
| 540 | 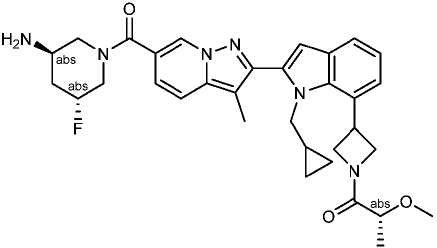 (R)-1-(3-(2-(6-((3R,5R)-3-Amino-5-fluoropiperidine-1-carbonyl)-3-methylpyrazolo[1, 5-a]pyridin-2-yl)-1-(cyclopropylmethyl)-1H-indol-7-yl)azetidin-1-yl)-2-methoxypropan-1-one |
| | Analytical Data: 587.2, 1.488 min (Method E) |
| 541 | 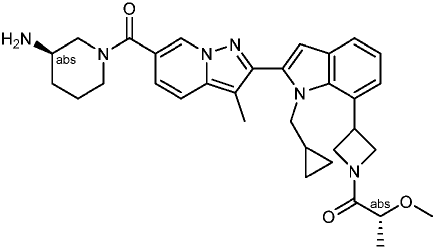 (R)-1-(3-(2-(6-((R)-3-Aminopiperidine-1-carbonyl)-3-methylpyrazolo[1,5-a]pyridin-2-yl)-1-(cyclopropylmethyl)-1H-indol-7-yl)azetidin-1-yl)-2-methoxypropan-1-one |
| | Analytical Data: 569.3, 1.443 min (Method E) |
| 542 | 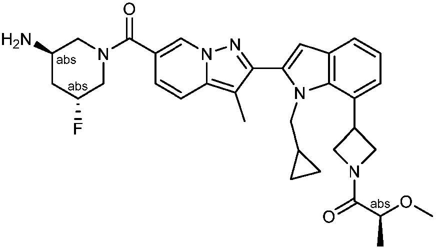 (S)-1-(3-(2-(6-((3R,5R)-3-Amino-5-fluoropiperidine-1-carbonyl)-3-methylpyrazolo[1, 5-a]pyridin-2-yl)-1-(cyclopropylmethyl)-1H-indol-7-yl)azetidin-1-yl)-2-methoxypropan-1-one |
| | Analytical Data: 587.3, 1.486 min (Method E) |
| 543 | 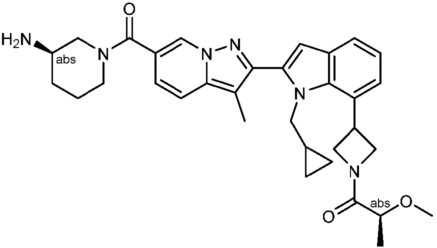 (S)-1-(3-(2-(6-((R)-3-aminopiperidine-1-carbonyl)-3-methylpyrazolo[1,5-a]pyridin-2-yl)-1-(cyclopropylmethyl)-1H-indol-7-yl)azetidin-1-yl)-2-methoxypropan-1-one |
| | Analytical Data: 569.3, 1.441 min (Method E) |
| 544 | 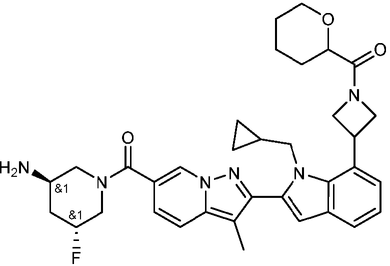 ((3R,5R)-3-Amino-5-fluoropiperidin-1-yl)(2-(1-(cyclopropylmethyl)-7-(1-(tetrahydro-2H-pyran-2-carbonyl)azetidin-3-yl)-1H-indol-2-yl)-3-methylpyrazolo[1,5-a]pyridin-6-yl)methanone (mixture of diastereomers) |
| | Analytical Data: 613.3, 1.653 min (Method E) |
| 545 | 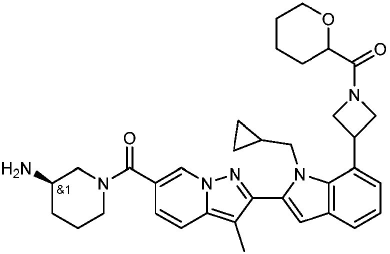 ((R)-3-Aminopiperidin-1-yl)(2-(1-(cyclopropylmethyl)-7-(1-(tetrahydro-2H-pyran-2-carbonyl)azetidin-3-yl)-1H-indol-2-yl)-3-methylpyrazolo[1,5-a]pyridin-6-yl)methanone (mixture of diastereomers) |
| | Analytical Data: 595.3, 1.732 min (Method E) |
| 546 | 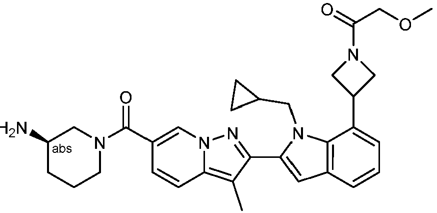 I (R)-1-(3-(2-(6-(3-Aminopiperidine-1-carbonyl)-3-methylpyrazolo[1,5-a]pyridin-2-yl)-1-(cyclopropylmethyl)-1H-indol-7-yl)azetidin-1-yl)-2-methoxyethan-1-one |
| | Analytical Data: 555.4, 6.864 min (Method D) |
| 547 | 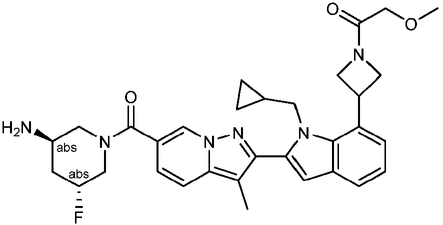 1-(3-(2-(6-((3R,5R)-3-Amino-5-fluoropiperidine-1-carbonyl)-3-methylpyrazolo[1, 5-a]pyridin-2-yl)-1-(cyclopropylmethyl)-1H-indol-7-yl)azetidin-1-yl)-2-methoxyethan-1-one |
| | Analytical Data: 573.2, 6.996 min (Method C) |
| 548 | 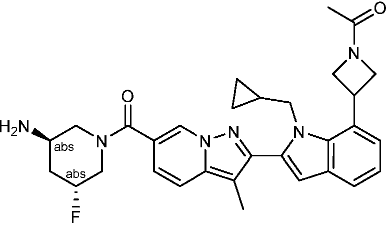 1-(3-(2-(6-((3R,5R)-3-Amino-5-fluoropiperidine-1-carbonyl)-3-methylpyrazolo[1, 5-a]pyridin-2-yl)-1-(cyclopropylmethyl)-1H-indol-7-yl)azetidin-1-yl)ethan-1-one |
| | Analytical Data: 544.2, 7.161 min (Method D) |
| 549 | 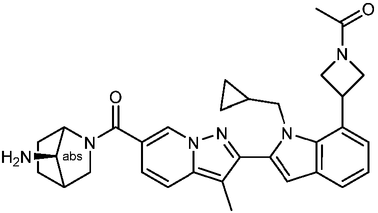 1-(3-(2-(6-((7R)-7-Amino-2-azabicyclo[2.2.1]heptane-2-carbonyl)-3-methylpyrazolo[1,5-a]pyridin-2-yl)-1-(cyclopropylmethyl)-1H-indol-7-yl)azetidin-1-yl)ethan-1-one |
| | Analytical Data: 537.4 ,6.854 min (Method D) |

### Example 550

### (R)-5-(((2-(6-((3R,5R)-3-amino-5-fluoropiperidine-1-carbonyl)-4-methoxy-3-methylpyrazolo[1,5-a]pyridin-2-yl)-1-(cyclopropylmethyl)-1H-indol-7-yl)oxy)methyl)pyrrolidin-2-one

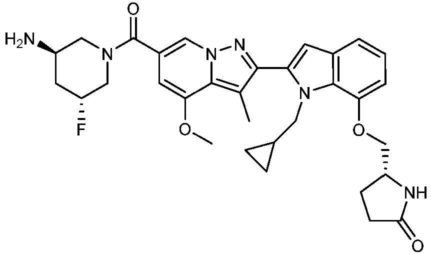

### Intermediate 550A

### Methyl 2-(7-(benzyloxy)-1-(cyclopropylmethyl)-1H-indol-2-yl)-4-methoxy-3-methylpyrazolo[1,5-a]pyridine-6-carboxylate

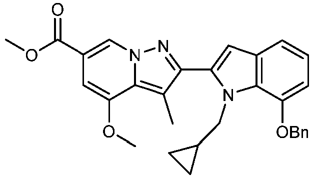

Intermediate 50 (1.9g, 5.24 mmol was added to a stirred solution of Intermediate 1 (2.28 g, 5.24 mmol) and copper(I) iodide (0.263 g, 1.380 mmol) in DMF (10 ml). The reaction mixture was heated to 80°C for 48 hours, quenched with water (100 ml) and extracted with ethyl acetate (2×100 ml). The combined organic layer was dried over sodium sulphate, filtered, and concentrated under reduced vacuum to obtain a crude product, which was purified using a silica gel column (10-20% of ethyl acetate in hexane) to afford methyl 2-(7-(benzyloxy)-1-(cyclopropylmethyl)-1H-indol-2-yl)-4-methoxy-3-methylpyrazolo[1,5-a]pyridine-6-carboxylate (500 mg, 37%). LC-MS m/z: 496.2 [M+H]⁺

### Intermediate 550B

### Methyl 2-(1-(cyclopropylmethyl)-7-hydroxy-1H-indol-2-yl)-4-methoxy-3-methylpyrazolo[1,5-a]pyridine-6-carboxylate

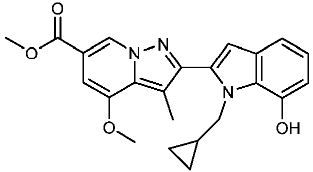

A solution of Intermediate 550A (500 mg, 1.009 mmol) in ethyl acetate (3 ml) was degassed with nitrogen for 5 min, followed by the addition of Pd/C (107 mg, 0.101 mmol). The reaction mixture was stirred under H₂ atmosphere for 4 hours, filtered through a celite bed, and washed with methanol. The combined filtrate was concentrated under reduced pressure to afford crude methyl 2-(1-(cyclopropylmethyl)-7-hydroxy-1H-indol-2-yl)-4-methoxy-3-methylpyrazolo[1,5-a]pyridine-6-carboxylate (350 mg, 86%) LC-MS m/z: 406.3 [M+H]⁺. ¹H NMR (300 MHz, DMSO-*d*₆) δ ppm: 9.77 (s, 1H), 8.85 (s, 1H), 7.05 (d, J=7.6 Hz, 1H), 6.90-6.81 (m, 2H), 6.67 (s, 1H), 6.60 (d, J=7.2 Hz, 1H), 4.56 (br, d, J=6.4 Hz, 2H), 4.09-3.94 (m, 3H), 3.90 (s, 3H), 3.32-3.17 (m, 3H), 1.02 (br, s, 1H), 0.26-0.12 (m, 2H),0.05-0.01 (m, 2H)

### Intermediate 550C

### Indol-2-yl)-4-methoxy-3-methylpyrazolo[1,5-a]pyridine-6-carboxylate AR NO- A2C37-163

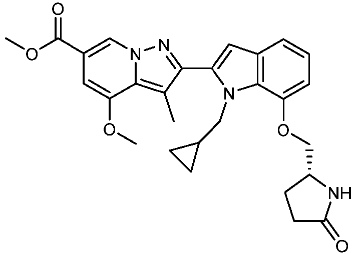

Cesium carbonate (241 mg, 0.740 mmol), tetrabutylammonium iodide (27.3 mg, 0.074 mmol) and (R)-(5-oxopyrrolidin-2-yl)methyl 4-methylbenzenesulfonate (66.4 mg, 0.247 mmol) were added to a stirred solution of Intermediate 550B (100 mg, 0.247 mmol) in DMF (1 ml). The reaction mixture was heated to 60°C for 16 hours, quenched with water (100 ml), and extracted with ethyl acetate (2×100 ml). The combined organic layers were dried over sodium sulphate, filtered, and concentrated under reduced pressure to afford methyl (R)-2-(1-(cyclopropylmethyl)-7-((5-oxopyrrolidin-2-yl)methoxy)-1H-indol-2-yl)-4-methoxy-3-methylpyrazolo[1,5-a]pyridine-6-carboxylate (105 mg, 85%). LC-MS m/z: 503.4 [M+H]⁺.

### Intermediate 550D

### (R)-2-(1-(Cyclopropylmethyl)-7-((5-oxopyrrolidin-2-yl)methoxy)-1H-indol-2-yl)-4-methoxy-3-methylpyrazolo[1,5-a]pyridine-6-carboxylic acid

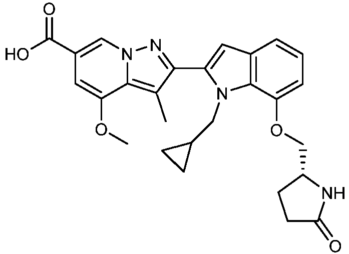

LiOH (4.76 mg, 0.199 mmol) was added to a stirred solution of Intermediate 550C (100 mg, 0.199 mmol) in THF (1 ml), methanol (1.0 ml) and water (1 ml). The reaction mixture was stirred for 16 hours, concentrated under reduced pressure, then acidified with 1 N HCl, and the solids thus obtained were filtered through a Buchner funnel and dried under reduced pressure to afford (R)-2-(1-(cyclopropylmethyl)-7-((5-oxopyrrolidin-2-yl)methoxy)-1H-indol-2-yl)-4-methoxy-3-methylpyrazolo[1,5-a]pyridine-6-carboxylic acid (90 mg, 93%). LC-MS m/z: 489.3 [M+H]⁺

### Intermediate 550E

### tert-butyl ((3R,5R)-1-(2-(1-(cyclopropylmethyl)-7-(((R)-5-oxopyrrolidin-2-yl)methoxy)-1H-indol-2-yl)-4-methoxy-3-methylpyrazolo[1,5-a]pyridine-6-carbonyl)-5-fluoropiperidin-3-yl)carbamate

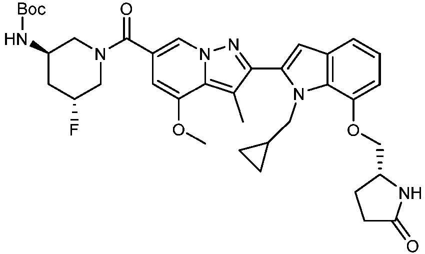

tert-Butyl ((3R,5R)-5-fluoropiperidin-3-yl)carbamate (22.34 mg, 0.102 mmol), DIPEA (0.054 ml, 0.307 mmol), HATU (58.4 mg, 0.154 mmol) were added to a stirred solution of Intermediate 550D (50 mg, 0.102 mmol) in DMF (1 ml). The reaction mixture was stirred 3 hours, quenched with water (50 ml), and extracted with ethyl acetate (2×50 ml). The combined organic layers were dried over sodium sulphate, filtered, and concentrated under reduced pressure to afford tert-butyl ((3R,5R)-1-(2-(1-(cyclopropylmethyl)-7-(((R)-5-oxopyrrolidin-2-yl)methoxy)-1H-indol-2-yl)-4-methoxy-3-methylpyrazolo[1,5-a]pyridine-6-carbonyl)-5-fluoropiperidin-3-yl)carbamate (50 mg, 71%). LC-MS m/z: 689.3 [M+H]⁺.

### Example 550

### (R)-5-(((2-(6-((3R,5R)-3-Amino-5-fluoropiperidine-1-carbonyl)-4-methoxy-3-methylpyrazolo[1,5-a]pyridin-2-yl)-1-(cyclopropylmethyl)-1H-indol-7-yl)oxy)methyl)pyrrolidin-2-one

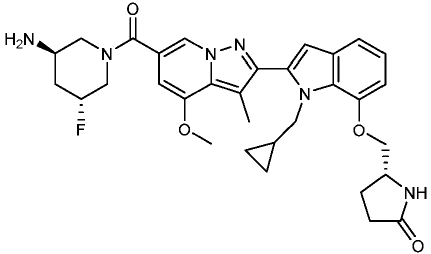

TFA (0.087 mmol) was added to a stirred solution of tert-butyl ((3R,5R)-1-(2-(1-(cyclopropylmethyl)-7-(((R)-5-oxopyrrolidin-2-yl)methoxy)-1H-indol-2-yl)-4-methoxy-3-methylpyrazolo[1,5-a]pyridine-6-carbonyl)-5-fluoropiperidin-3-yl)carbamate (60 mg, 0.087 mmol) in DCM (1 ml) at 0°C. The reaction mixture was allowed it to warm to a room temperature and stirred for 3 hours, concentrated in vacuo to afford a crude compound, which was purified using preparative HPLC to afford (R)-5-(((2-(6-((3R,5R)-3-Amino-5-fluoropiperidine-1-carbonyl)-4-methoxy-3-methylpyrazolo[1,5-a]pyridin-2-yl)-1-(cyclopropylmethyl)-1H-indol-7-yl)oxy)methyl)pyrrolidin-2-one (11.2 mg, 19%). LC-MS m/z: 589.2 [M+H]⁺. LCMS retention time: 1.498 min (method E). ¹H NMR (400 MHz, DMSO-*d₆*) δ ppm: 8.36 (s, 1H), 7.87 (s, 1H), 7.21 (d, *J*=8.0 Hz, 1H), 6.99 (t, *J*=8.0 Hz, 1H), 6.76 (d, *J*=8.0 Hz, 1H), 6.67 (s, 1H), 6.54 (s, 1H), 5.05-4.86 (m, 1H), 4.52 (br, dd, *J*=3.1, 7.0 Hz, 2H), 4.15-4.00 (m, 4H), 3.98 (s, 3H), 3.09-3.01 (m, 3H), 2.47 (s, 3H), 2.31-2.14 (m, 4H), 2.07-1.99 (m, 1H), 1.68-1.49 (m, 1H), 1.07-0.98 (m, 1H), 0.22-0.13 (m, 2H), -0.05 (q, *J*=4.8 Hz, 2H).

Compounds of Examples 551 to 565 in Table 53 were prepared following a procedure similar to the preparation of a compound of Example 550 in Table 55 , by displacement reaction of Intermediate 550B with appropriate tosylate followed by ester hydrolysis and coupling with appropriate amines

**Table 53**

| Example | Structure |
|---|---|
| | Name |
| | Analytical Data: LC-MS m/z [M+H]+; HPLC retention time (HPLC method) |
| 551 | 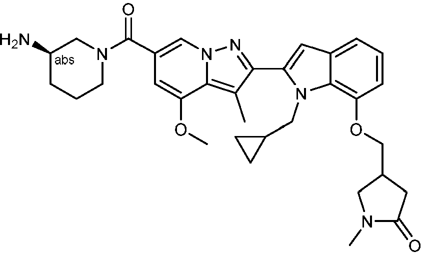 4-(((2-(6-((R)-3-Aminopiperidine-1-carbonyl)-4-methoxy-3-methylpyrazolo[1, 5-a]pyridin-2-yl)-1-(cyclopropylmethyl)-1H-indol-7-yl)oxy)methyl)-1-methylpyrrolidin-2-one (isomer 1) |
| | Analytical Data: 585.3, 1.624 min (Method E) |
| 552 | 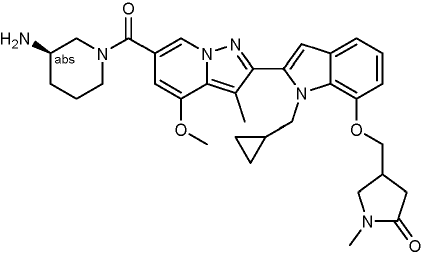 4-(((2-(6-((R)-3-Aminopiperidine-1-carbonyl)-4-methoxy-3-methylpyrazolo[1, 5-a]pyridin-2-yl)-1-(cyclopropylmethyl)-1H-indol-7-yl)oxy)methyl)-1-methylpyrrolidin-2-one (isomer 1) |
| | Analytical Data: 585.3, 1.629 min (Method E) |
| 553 | 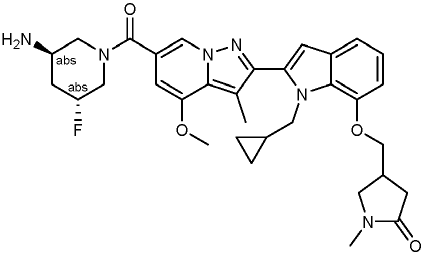 4-(((2-(6-((3R,5R)-3-Amino-5-fluoropiperidine-1-carbonyl)-4-methoxy-3-methylpyrazolo[1, 5-a]pyridin-2-yl)-1-(cyclopropylmethyl)-1H-indol-7-yl)oxy)methyl)-1-methylpyrrolidin-2-one (isomer 1) |
| | Analytical Data: 603.3, 1.645 min (Method E ) |
| 554 | 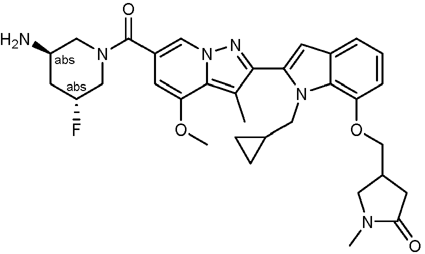 4-(((2-(6-((3R,5R)-3-Amino-5-fluoropiperidine-1-carbonyl)-4-methoxy-3-methylpyrazolo[1, 5-a]pyridin-2-yl)-1-(cyclopropylmethyl)-1H-indol-7-yl)oxy)methyl)-1-methylpyrrolidin-2-one (isomer 2) |
| | Analytical Data: 603.3, 1.640 min (Method E) |
| 555 | 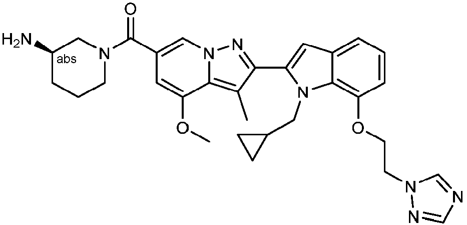 (R)-(2-(7-(2-(1H-1,2,4-Triazol-1-yl)ethoxy)-1-(cyclopropylmethyl)-1H-indol-2-yl)-4-methoxy-3-methylpyrazolo[1,5-a]pyridin-6-yl)(3-aminopiperidin-1-yl)methanone |
| | Analytical Data: 569.2, 1.482 min (Method E) |
| 556 | 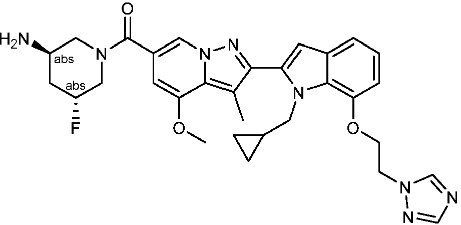 (2-(7-(2-(1H-1,2,4-Triazol-1-yl)ethoxy)-1-(cyclopropylmethyl)-1H-indol-2-yl)-4-methoxy-3-methylpyrazolo[1,5-a]pyridin-6-yl)((3R,5R)-3-amino-5-fluoropiperidin-1-yl)methanone |
| | Analytical Data: 587.3, 1.629 min (Method E) |
| 557 | 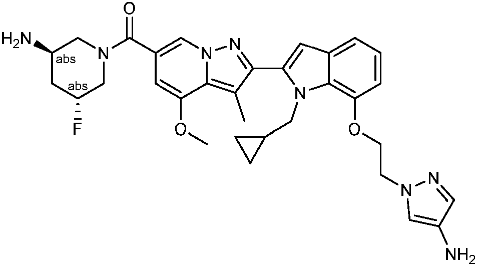 (2-(7-(2-(4-Amino-1H-pyrazol-1-yl)ethoxy)-1-(cyclopropylmethyl)-1H-indol-2-yl)-4-methoxy-3-methylpyrazolo[1,5-a]pyridin-6-yl)((3R,5R)-3-amino-5-fluoropiperidin-1-yl)methanone |
| | Analytical Data: 601.2, 1.558 min |
| | (Method E) |
| 558 | 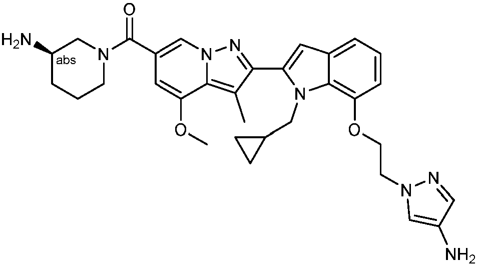 (R)-(2-(7-(2-(4-Amino-1H-pyrazol-1-yl)ethoxy)-1-(cyclopropylmethyl)-1H-indol-2-yl)-4-methoxy-3-methylpyrazolo[1,5-a]pyridin-6-yl)(3-aminopiperidin-1-yl)methanone |
| | Analytical Data: 583.2, 1.264 min |
| | (Method E) |
| 559 | 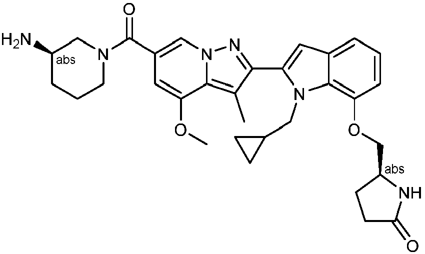 (S)-5-(((2-(6-((R)-3-Aminopiperidine-1-carbonyl)-4-methoxy-3-methylpyrazolo[1, 5-a]pyridin-2-yl)-1-(cyclopropylmethyl)-1H-indol-7-yl)oxy)methyl)pyrrolidin-2-one |
| | Analytical Data: 571.3, 1.470 min (Method E) |
| 560 | 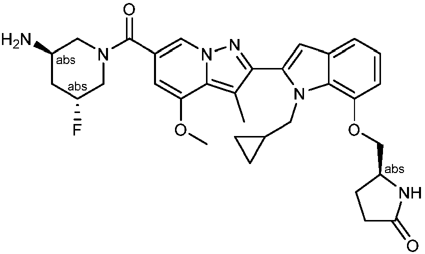 (S)-5-(((2-(6-((3R,5R)-3-Amino-5-fluoropiperidine-1-carbonyl)-4-methoxy-3-methylpyrazolo[1,5-a]pyridin-2-yl)-1-(cyclopropylmethyl)-1H-indol-7-yl)oxy)methyl)pyrrolidin-2-one |
| | Analytical Data: 589.2, 1.489 min |
| | (Method E ) |
| 561 | 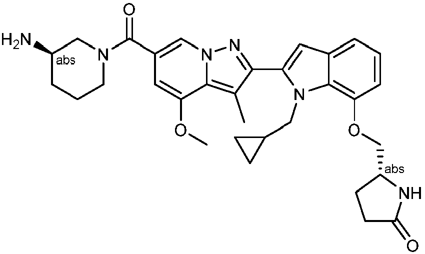 (R)-5-(((2-(6-((R)-3-Aminopiperidine-1-carbonyl)-4-methoxy-3-methylpyrazolo[1, 5-a]pyridin-2-yl)-1-(cyclopropylmethyl)-1H-indol-7-yl)oxy)methyl)pyrrolidin-2-one |
| | Analytical Data: 571.2, 1.465 min (Method E ) |
| 562 | 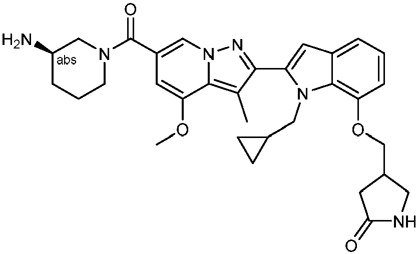 4-(((2-(6-((R)-3-Aminopiperidine-1-carbonyl)-4-methoxy-3-methylpyrazolo[1, 5-a]pyridin-2-yl)-1-(cyclopropylmethyl)-1H-indol-7-yl)oxy)methyl)pyrrolidin-2-one (isomer 1) Analytical Data: 571.2, 1.471 min (Method E ) |
| 563 | 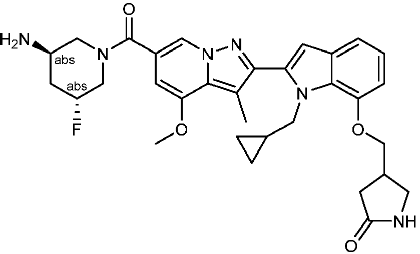 4-(((2-(6-((3R,5R)-3-Amino-5-fluoropiperidine-1-carbonyl)-4-methoxy-3-methylpyrazolo[1, 5-a]pyridin-2-yl)-1-(cyclopropylmethyl)-1H-indol-7-yl)oxy)methyl)pyrrolidin-2-one (isomer 1) |
| | Analytical Data: 589.3, 1.492 min (Method E) |
| 564 | 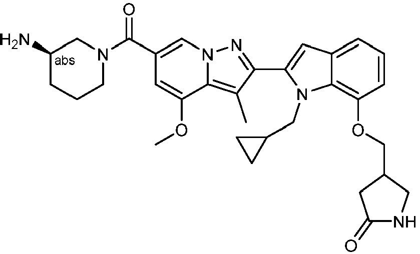 4-(((2-(6-((R)-3-Aminopiperidine-1-carbonyl)-4-methoxy-3-methylpyrazolo[1, 5-a]pyridin-2-yl)-1-(cyclopropylmethyl)-1H-indol-7-yl)oxy)methyl)pyrrolidin-2-one (isomer 2) |
| | Analytical Data: 571.2, 1.476 min |
| | (Method E) |
| 565 | 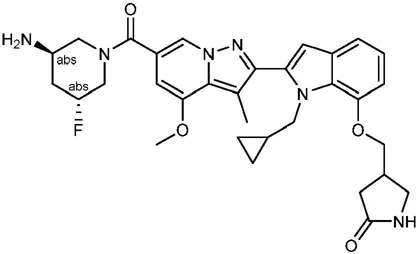 4-(((2-(6-((3R,5R)-3-Amino-5-fluoropiperidine-1-carbonyl)-4-methoxy-3-methylpyrazolo[1, 5-a]pyridin-2-yl)-1-(cyclopropylmethyl)-1H-indol-7-yl)oxy)methyl)pyrrolidin-2-one (isomer 2) |
| | Analytical Data: 589.3, 1.484 min (Method E) |

### Example 566

### 4-(((2-(6-((R)-3-Aminopiperidine-1-carbonyl)-4-fluoro-3-methylpyrazolo[1,5-a]pyridin-2-yl)-1-(cyclopropylmethyl)-1H-indol-7-yl)oxy)methyl)pyrrolidin-2-one (isomer 1)

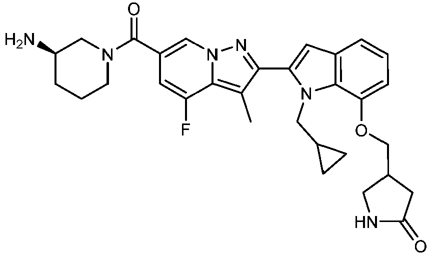

### Intermediate 566A

### methyl 2-(7-(benzyloxy)-1-(cyclopropylmethyl)-1H-indol-2-yl)-4-fluoro-3-methylpyrazolo[1,5-a]pyridine-6-carboxylate

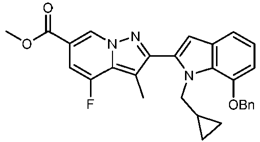

Intermediate 566A (0.7 g, 26%) was prepared following a procedure similar to the preparation of Intermediate 530A.as a gummy liquid. LC-MS m/z: 484.3 [M+H]⁺

### Intermediate 566B

### Methyl 2-(1-(cyclopropylmethyl)-7-hydroxy-1H-indol-2-yl)-4-fluoro-3-methylpyrazolo[1,5-a]pyridine-6-carboxylate

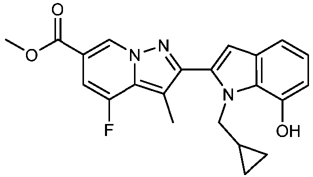

Intermediate 566B (0.31 g, 85%) was prepared following a procedure similar to the preparation of Intermediate 530B. LC-MS m/z: 394.3 [M+H]⁺; ¹H NMR (400 MHz, DMSO-d₆) δ ppm:9.13 (d, *J*=1.0 Hz, 1H), 7.38 (d, *J*=11.5 Hz, 1H), 7.07 (d, *J*=8.0 Hz, 1H), 6.87 (t, *J*=7.8 Hz, 1H), 6.75 (s, 1H), 6.62 (d, *J*=7.5 Hz, 1H), 4.61 (d, *J*=6.5 Hz, 2H), 3.91 (s, 3H), 2.49-2.47 (m, 3H), 1.05 -1.02(m, 1H), 0.28-0.14 (m, 2H), 0.10-0.03 (m, 2H).

### Intermediate 566C

### Methyl 2-(1-(cyclopropylmethyl)-7-((5-oxopyrrolidin-3-yl)methoxy)-1H-indol-2-yl)-4-fluoro-3-methylpyrazolo[1,5-a]pyridine-6-carboxylate

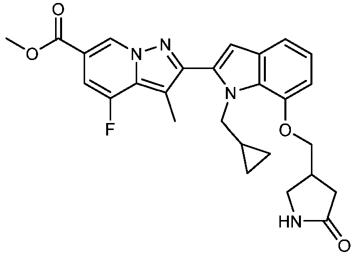

Cesium carbonate (0.124 g, 0.381 mmol) and (5-oxopyrrolidin-3-yl)methyl 4-methylbenzenesulfonate (0.068 g, 0.254 mmol) were added to a stirred solution of Intermediate 566B (0.1g, 0.254 mmol) in DMF (3 ml). The reaction mixture was stirred at 60°C for 2 hours, diluted with water, and the solids obtained were filtered and dried under reduced pressure to afford crude methyl 2-(1-(cyclopropylmethyl)-7-((5-oxopyrrolidin-3-yl)methoxy)-1H-indol-2-yl)-4-fluoro-3-methylpyrazolo[1,5-a]pyridine-6-carboxylate (0.12 g, 96%) as a brown solid. LC-MS m/z: 491.3 [M+H]⁺.

### Intermediate 566D

### 2-(1-(Cyclopropylmethyl)-7-((5-oxopyrrolidin-3-yl)methoxy)-1H-indol-2-yl)-4-fluoro-3-methylpyrazolo[1,5-a]pyridine-6-carboxylic acid

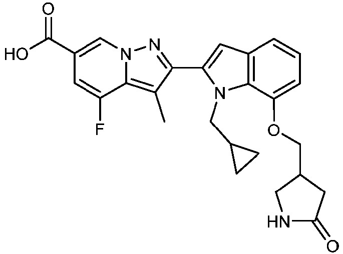

LiOH (0.029 g, 1.223 mmol) was added to a stirred solution of Intermediate 566C (0.12 g, 0.245 mmol) in ethanol (1ml), THF (1 ml), and water (1 ml). The reaction mixture was stirred for 2 hours, concentrated, and the residue obtained was neutralized using 1.5 N HCl solution. The solids thus obtained were filtered and dried under high vacuum to afford 2-(1-(cyclopropylmethyl)-7-((5-oxopyrrolidin-3-yl)methoxy)-1H-indol-2-yl)-4-fluoro-3-methylpyrazolo[1,5-a]pyridine-6-carboxylic acid (0.11 g, 94%) as a white solid. LC-MS m/z: 477.3 [M+H]⁺

### Intermediate 566E

### tert-Butyl ((3R)-1-(2-(1-(cyclopropylmethyl)-7-((5-oxopyrrolidin-3-yl)methoxy)-1H-indol-2-yl)-4-fluoro-3-methylpyrazolo[1,5-a]pyridine-6-carbonyl)piperidin-3-yl)carbamate

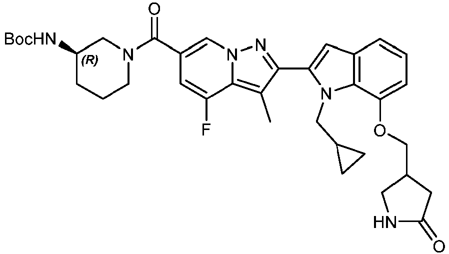

Intermediate 566E (0.07 g, 84%) was prepared following a procedure similar to the preparation of Intermediate 530E. LC-MS m/z: 659.5 [M+H]⁺

### Example 566

4-(((2-(6-((R)-3-aminopiperidine-1-carbonyl)-4-fluoro-3-methylpyrazolo[1,5-a]pyridin-2-yl)-1-(cyclopropylmethyl)-1H-indol-7-yl)oxy)methyl)pyrrolidin-2-one. (isomer 1) LC-MS m/z: 559.2 [M+H]^{+.} LCMS retention time: 1.52 min (method E); ¹H NMR (400 MHz, DMSO-*d₆*) δ ppm: 8.78 (s, 1H), 8.13 (br.s, 1H), 7.62 (s,1H), 7.26-7.12 (m, 3H), 7.00 (t, *J*=8.0 Hz, 1H), 6.79 (d, *J*=8.0 Hz, 1H), 6.75 (s, 1H), 4.52 (d, *J*=6.8 Hz, 2H), 4.17 (d, *J*=6.3 Hz, 2H), 4.10-4.00 (m, 1H), 3.54-3.49 (m, 1H), 3.25-3.10 (m, 3H), 3.03-2.94 (m, 1H), 2.53-2.50 (m, 1H), 2.46 (s, 3H), 2.44-2.38 (m, 2H), 2.19 (dd, *J*=7.3, 16.8 Hz, 1H), 2.06-1.97 (m, 2H), 1.84-1.72 (m, 1H), 1.69-1.51 (m, 1H),1.07-0.95 (m, 1H), 0.27-0.13 (m, 2H), 0.00--0.07 (m, 2H).

Compounds of Examples 567 to 572 in Table 54 were prepared following a procedure similar to the preparation of a compound of Example 566, by displacement reaction of Intermediate 566B with appropriate tosylate followed by ester hydrolysis and coupling with appropriate amines.

**Table 54**

| Example | Structure |
|---|---|
| | Name |
| | Analytical Data: LC-MS m/z [M+H]⁺; HPLC retention time (HPLC method) |
| 567 | 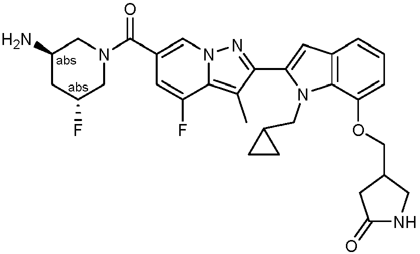 4-(((2-(6-((3R,5R)-3-Amino-5-fluoropiperidine-1-carbonyl)-4-fluoro-3-methylpyrazolo[1, 5-a]pyridin-2-yl)-1-(cyclopropylmethyl)-1H-indol-7-yl)oxy)methyl)pyrrolidin-2-one (isomer 1) |
| | Analytical Data: 577.3; 1.493 min(Method E) |
| 568 | 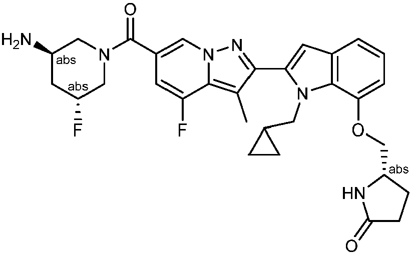 (S)-5-(((2-(6-((3R,5R)-3-Amino-5-fluoropiperidine-1-carbonyl)-4-fluoro-3-methylpyrazolo[1, 5-a]pyridin-2-yl)-1-(cyclopropylmethyl)-1H-indol-7-yl)oxy)methyl)pyrrolidin-2-one |
| | Analytical Data: 577.2; 1.650 min (Method E) |
| 569 | 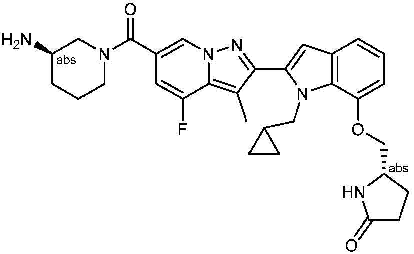 (S)-5-(((2-(6-((R)-3-Aminopiperidine-1-carbonyl)-4-fluoro-3-methylpyrazolo[1, 5-a]pyridin-2-yl)-1-(cyclopropylmethyl)-1H-indol-7-yl)oxy)methyl)pyrrolidin-2-one |
| | Analytical Data: 559.2; 1.588 min (Method E) |
| 570 | 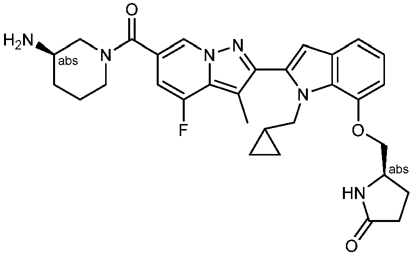 (R)-5-(((2-(6-((R)-3-Aminopiperidine-1-carbonyl)-4-fluoro-3-methylpyrazolo[1, 5-a]pyridin-2-yl)-1-(cyclopropylmethyl)-1H-indol-7-yl)oxy)methyl)pyrrolidin-2-one |
| | Analytical Data:559.2, 1.588 min (Method E) |
| 571 | 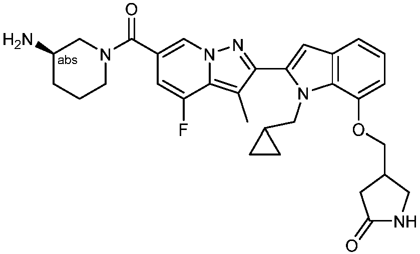 4-(((2-(6-((R)-3-Aminopiperidine-1-carbonyl)-4-fluoro-3-methylpyrazolo[1,5-a]pyridin-2-yl)-1-(cyclopropylmethyl)-1H-indol-7-yl)oxy)methyl)pyrrolidin-2-one (isomer 2) |
| | Analytical Data: 559.2, 1.52 min (Method E) |
| 572 | 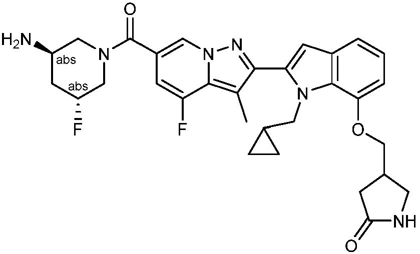 4-(((2-(6-((3R,5R)-3-Amino-5-fluoropiperidine-1-carbonyl)-4-fluoro-3-methylpyrazolo[1, 5-a]pyridin-2-yl)-1-(cyclopropylmethyl)-1H-indol-7-yl)oxy)methyl)pyrrolidin-2-one (isomer 2) |
| | Analytical Data: 577.2, 1.58 min (Method E) |

### Example 573

### 4-(((2-(6-((3R,5R)-3-Amino-5-fluoropiperidine-1-carbonyl)-3-methylpyrazolo[1,5-a]pyridin-2-yl)-1-(cyclopropylmethyl)-1H-indol-7-yl)oxy)methyl)pyrrolidin-2-one (isomer 1)

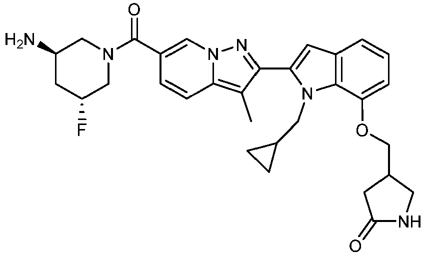

### Intermediate 573A

### Ethyl 2-(7-(benzyloxy)-1-(cyclopropylmethyl)-1H-indol-2-yl)-3-methylpyrazolo[1,5-a]pyridine-6-carboxylate

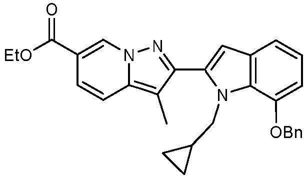

Intermediate 573A (850 mg, 32%) was prepared following a procedure similar to the preparation of Intermediate 550A. LC-MS m/z: 480.2 [M+H]⁺

### Intermediate 573B

### Ethyl 2-(1-(cyclopropylmethyl)-7-hydroxy-1H-indol-2-yl)-3-methylpyrazolo[1,5-a]pyridine-6-carboxylate

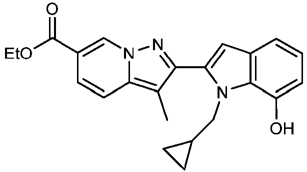

Intermediate 573B (450 mg, 65%) was prepared following a procedure similar to the preparation of Intermediate 550B. LC-MS m/z: 390.4 [M+H]⁺

### Intermediate 573C

### Ethyl 2-(1-(cyclopropylmethyl)-7-((5-oxopyrrolidin-3-yl)methoxy)-1H-indol-2-yl)-3-methylpyrazolo[1,5-a]pyridine-6-carboxylate

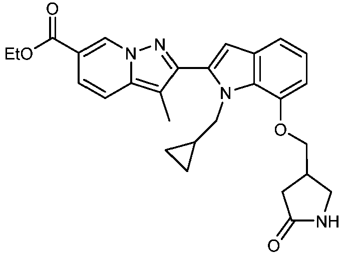

Cesium carbonate (251 mg, 0.770 mmol) and tetrabutylammonium iodide (28.5 mg, 0.077 mmol) and (5-oxopyrrolidin-3-yl)methyl 4-methylbenzenesulfonate (69.2 mg, 0.257 mmol) were added to a stirred solution of 573B (100 mg, 0.257 mmol) in DMF (1 ml). The reaction mixture was heated for at 60°C 16 hours, quenched with water (100 ml), and extracted with ethyl acetate (2×100ml). The combined the organic layers were dried over sodium sulphate, filtered, and concentrated under reduced pressure to afford crude ethyl 2-(1-(cyclopropylmethyl)-7-((5-oxopyrrolidin-3-yl)methoxy)-1H-indol-2-yl)-3-methylpyrazolo[1,5-a]pyridine-6-carboxylate (110 mg, 88%). LC-MS m/z: 487.3 [M+H]⁺

### Intermediate 573D

### 2-(1-(Cyclopropylmethyl)-7-((5-oxopyrrolidin-3-yl)methoxy)-1H-indol-2-yl)-3-methylpyrazolo[1,5-a]pyridine-6-carboxylic acid

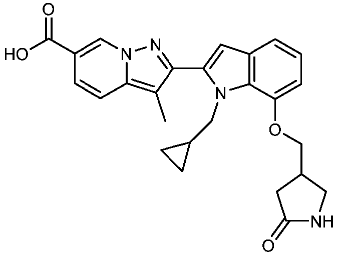

LiOH (5.91 mg, 0.247 mmol) was added to a stirred solution of Intermediate 573D (120 mg, 0.247 mmol) in THF (1 ml), methanol (1 ml), and water (1 ml). The reaction mixture was stirred for 16 hours, concentrated under reduced pressure to afford the crude salts, which were acidified with 1 N HCl. The solids obtained were filtered through a Buchner funnel and dried in vacuo to afford 2-(1-(cyclopropylmethyl)-7-((5-oxopyrrolidin-3-yl)methoxy)-1H-indol-2-yl)-3-methylpyrazolo[1,5-a]pyridine-6-carboxylic acid (100 mg, 88%). LC-MS m/z: 459.2 [M+H]⁺

### Intermediate 573E

### tert-Butyl ((3R,5R)-1-(2-(1-(cyclopropylmethyl)-7-((5-oxopyrrolidin-3-yl)methoxy)-1H-indol-2-yl)-3-methylpyrazolo[1,5-a]pyridine-6-carbonyl)-5-fluoropiperidin-3-yl)carbamate

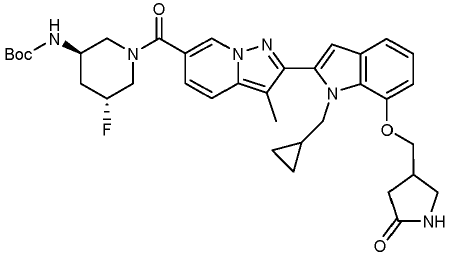

tert-Butyl ((3R,5R)-5-fluoropiperidin-3-yl)carbamate (23.80 mg, 0.109 mmol), DIPEA (0.057 ml, 0.327 mmol) and HATU (62.2 mg, 0.164 mmol) were added to a stirred solution of Intermediate 573D (50 mg, 0.109 mmol) in DMF (1 ml). The reaction mixture was stirred for 3 hours, diluted with water (50 ml), and extracted with ethyl acetate (2×50 ml). The combined organic layers were dried over sodium sulphate, filtered, and concentrated under reduced pressure to afford crude tert-butyl ((3R,5R)-1-(2-(1-(cyclopropylmethyl)-7-((5-oxopyrrolidin-3-yl)methoxy)-1H-indol-2-yl)-3-methylpyrazolo[1,5-a]pyridine-6-carbonyl)-5-fluoropiperidin-3-yl)carbamate (60 mg, 84%). LC-MS m/z: 659.3 [M+H]⁺.

### Example 573

### 4-(((2-(6-((3R,5R)-3-Amino-5-fluoropiperidine-1-carbonyl)-3-methylpyrazolo[1,5-a]pyridin-2-yl)-1-(cyclopropylmethyl)-1H-indol-7-yl)oxy)methyl)pyrrolidin-2-one (isomer 1)

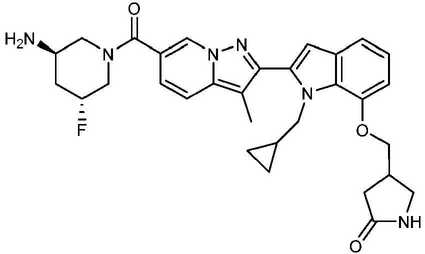

4-(((2-(6-((3R,5R)-3-amino-5-fluoropiperidine-1-carbonyl)-3-methylpyrazolo[1,5-a]pyridin-2-yl)-1-(cyclopropylmethyl)-1H-indol-7-yl)oxy)methyl)pyrrolidin-2-one (isomer 1) (21.7 mg, 42%). LC-MS m/z: 559.2 [M+H]⁺. LCMS retention time: 1.384 min (method E). ¹H NMR (400 MHz, DMSO-*d₆*) δ ppm: 8.75 (s, 1H), 7.79 (d, *J*=9.0 Hz, 1H), 7.62 (s, 1H), 7.23- 7.17 (m, 2H), 6.99 (t, *J*=8.0 Hz, 1H), 6.77 (d, *J* =8.0 Hz, 1H), 6.73 (s, 1H), 5.03-4.98 (m, 1H), 4.60 (d, *J*=8.0 Hz, 2H), 4.16 (d, *J*=4.0 Hz, 2H), 3.50 (br, d, *J*=6.4 Hz, 1H), 3.23-2.94 (m, 5H), 2.44-2.39 (m, 1H), 2.37(s, 3H), 2.23-2.11 (m, 2H), 1.60-1.42 (m, 1H), 1.07-0.97 (m, 1H), 0.22-0.14 (m, 2H), 0.00--0.06 (m, 2H).

Compounds of Examples 574 to 590 in Table 55 were obtained following a procedure similar to the preparation of a compound of Example 573, by displacement reaction of Intermediate 573B with appropriate tosylate followed by ester hydrolysis and coupling with appropriate amines.

**Table 55**

| Example | Structure |
|---|---|
| | Name |
| | Analytical Data: LC-MS m/z [M+H]⁺; HPLC retention time (HPLC method) |
| 574 | 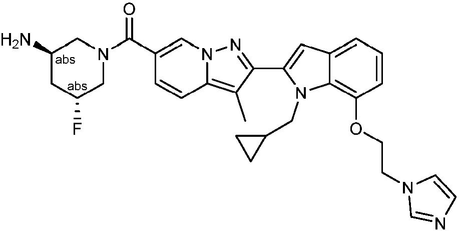 (2-(7-(2-(1H-Imidazol-1-yl)ethoxy)-1-(cyclopropylmethyl)-1H-indol-2-yl)-3-methylpyrazolo[1,5-a]pyridin-6-yl)((3R,5R)-3-amino-5-fluoropiperidin-1-yl)methanone |
| | Analytical Data: 556.3, 1.272 min (Method E) |
| 575 | 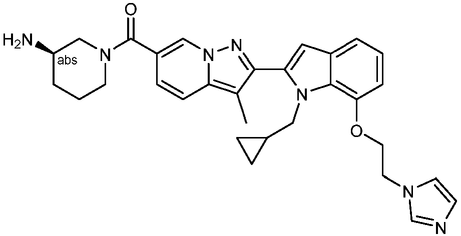 (R)-(2-(7-(2-(1H-Imidazol-1-yl)ethoxy)-1-(cyclopropylmethyl)-1H-indol-2-yl)-3-methylpyrazolo[1,5-a]pyridin-6-yl)(3-aminopiperidin-1-yl)methanone |
| | Analytical Data: 538.3 ,1.278 min (Method E) |
| 576 | 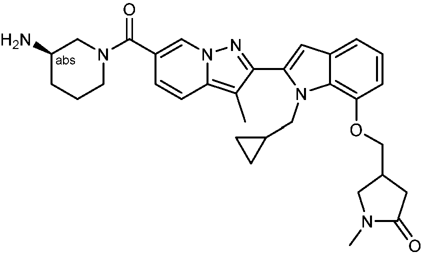 4-(((2-(6-((R)-3-Aminopiperidine-1-carbonyl)-3-methylpyrazolo[1,5-a]pyridin-2-yl)-1-(cyclopropylmethyl)-1H-indol-7-yl)oxy)methyl)-1-methylpyrrolidin-2-one (isomer 1) |
| | Analytical Data: 555.3, 1.531 min (Method E) |
| 577 | 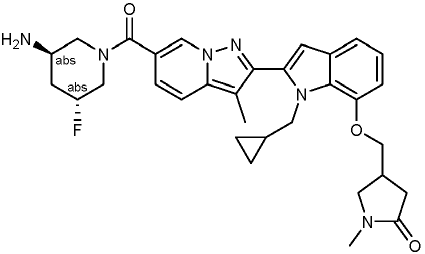 4-(((2-(6-((3R,5R)-3-Amino-5-fluoropiperidine-1-carbonyl)-3-methylpyrazolo[1,5-a]pyridin-2-yl)-1-(cyclopropylmethyl)-1H-indol-7-yl)oxy)methyl)-1-methylpyrrolidin-2-one (isomer 1) |
| | Analytical Data: 573.2, 1.544 min (Method E) |
| 578 | 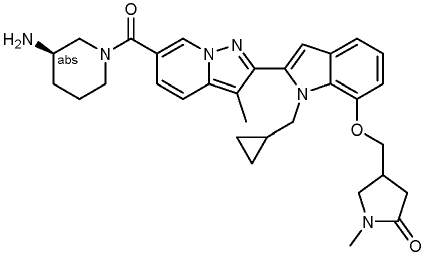 4-(((2-(6-((R)-3-Aminopiperidine-1-carbonyl)-3-methylpyrazolo[1,5-a]pyridin-2-yl)-1-(cyclopropylmethyl)-1H-indol-7-yl)oxy)methyl)-1-methylpyrrolidin-2-one (isomer 2) |
| | Analytical Data: 555.3 ,1.524 min (Method E) |
| 579 | 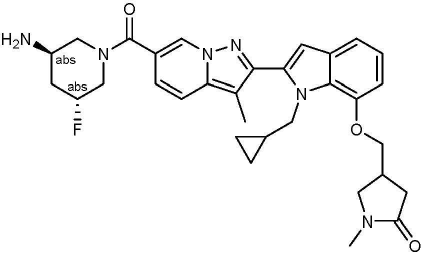 4-(((2-(6-((3R,5R)-3-Amino-5-fluoropiperidine-1-carbonyl)-3-methylpyrazolo[1, 5-a]pyridin-2-yl)-1-(cyclopropylmethyl)-1H-indol-7-yl)oxy)methyl)-1-methylpyrrolidin-2-one (isomer 2) |
| | Analytical Data: 573.2, 1.546 min (Method E) |
| 580 | 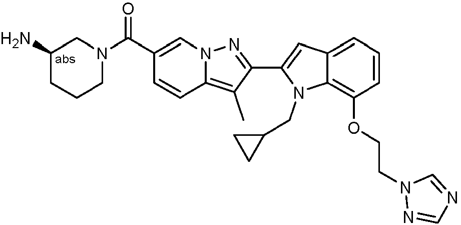 (R)-(2-(7-(2-(1H-1,2,4-Triazol-1-yl)ethoxy)-1-(cyclopropylmethyl)-1H-indol-2-yl)-3-methylpyrazolo[1,5-a]pyridin-6-yl)(3-aminopiperidin-1-yl)methanone |
| | Analytical Data: 539.3, 1.384 min (Method E) |
| 581 | 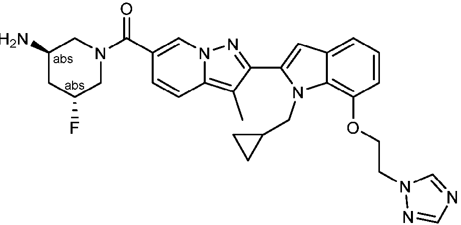 (2-(7-(2-(1H-1,2,4-Triazol-1-yl)ethoxy)-1-(cyclopropylmethyl)-1H-indol-2-yl)-3-methylpyrazolo[1,5-a]pyridin-6-yl)((3R,5R)-3-amino-5-fluoropiperidin-1-yl)methanone |
| | Analytical Data: 557.2 ,1.516 min (Method E) |
| 582 | 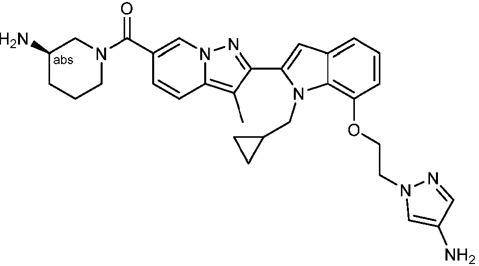 (R)-(2-(7-(2-(4-Amino-1H-pyrazol-1-yl)ethoxy)-1-(cyclopropylmethyl)-1H-indol-2-yl)-3-methylpyrazolo[1,5-a]pyridin-6-yl)(3-aminopiperidin-1-yl)methanone |
| | Analytical Data: 553.3, 1.410 min (Method E) |
| 583 | 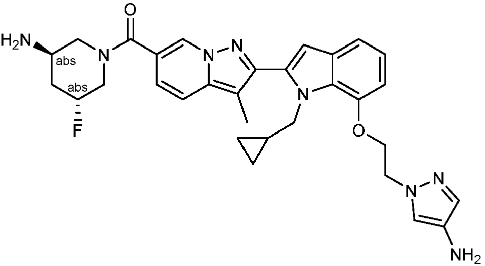 (2-(7-(2-(4-Amino-1H-pyrazol-1-yl)ethoxy)-1-(cyclopropylmethyl)-1H-indol-2-yl)-3-methylpyrazolo[1,5-a]pyridin-6-yl)((3R,5R)-3-amino-5-fluoropiperidin-1-yl)methanone |
| | Analytical Data: 571.3, 1.473 min (Method E) |
| 584 | 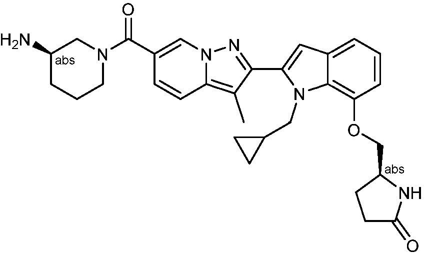 (S)-5-(((2-(6-((R)-3-Aminopiperidine-1-carbonyl)-3-methylpyrazolo[1,5-a]pyridin-2-yl)-1-(cyclopropylmethyl)-1H-indol-7-yl)oxy)methyl)pyrrolidin-2-one |
| | Analytical Data: 541.3, 1.402 min (Method E) |
| 585 | 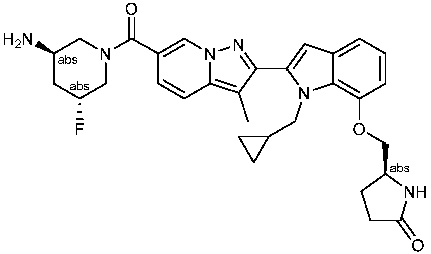 (S)-5-(((2-(6-((3R,5R)-3-Amino-5-fluoropiperidine-1-carbonyl)-3-methylpyrazolo[1, 5-a]pyridin-2-yl)-1-(cyclopropylmethyl)-1H-indol-7-yl)oxy)methyl)pyrrolidin-2-one |
| | Analytical Data: 559.3 , 1.386 min (Method E) |
| 586 | 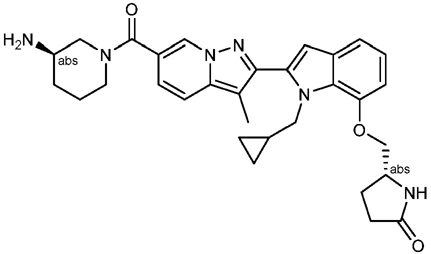 (R)-5-(((2-(6-((R)-3-Aminopiperidine-1-carbonyl)-3-methylpyrazolo[1,5-a]pyridin-2-yl)-1-(cyclopropylmethyl)-1H-indol-7-yl)oxy)methyl)pyrrolidin-2-one |
| | Analytical Data: 541.3, 1.373 min (Method E) |
| 587 | 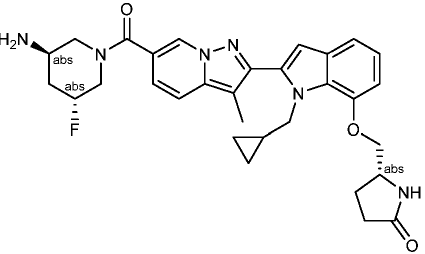 (R)-5-(((2-(6-((3R,5R)-3-Amino-5-fluoropiperidine-1-carbonyl)-3-methylpyrazolo[1, 5-a]pyridin-2-yl)-1-(cyclopropylmethyl)-1H-indol-7-yl)oxy)methyl)pyrrolidin-2-one |
| | Analytical Data: 559.2 , 1.388 min (Method E) |
| 588 | 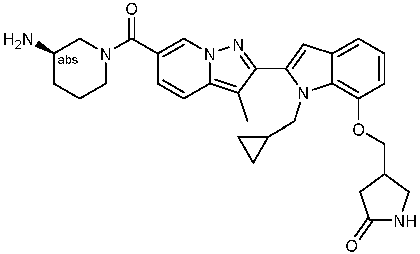 4-(((2-(6-((R)-3-Aminopiperidine-1-carbonyl)-3-methylpyrazolo[1,5-a]pyridin-2-yl)-1-(cyclopropylmethyl)-1H-indol-7-yl)oxy)methyl)pyrrolidin-2-one (isomer 1) |
| | Analytical Data: 541.2 , 1.375 min (Method E) |
| 589 | 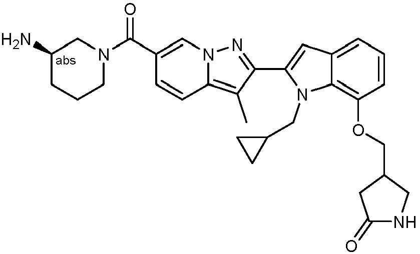 4-(((2-(6-((R)-3-Aminopiperidine-1-carbonyl)-3-methylpyrazolo[1,5-a]pyridin-2-yl)-1-(cyclopropylmethyl)-1H-indol-7-yl)oxy)methyl)pyrrolidin-2-one (isomer 2) |
| | Analytical Data: 541.3, 1.372 min (Method E) |
| 590 | 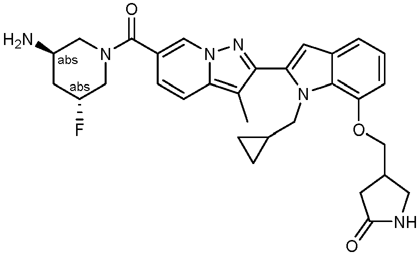 4-(((2-(6-((3R,5R)-3-Amino-5-fluoropiperidine-1-carbonyl)-3-methylpyrazolo[1,5-a]pyridin-2-yl)-1-(cyclopropylmethyl)-1H-indol-7-yl)oxy)methyl)pyrrolidin-2-one (isomer 2) |
| | Analytical Data: 559.2,1.382 min. Method E) |

### Example 591

### 4-(((2-(6-((R)-3-Aminopiperidine-1-carbonyl)-4-methoxy-3-methylpyrazolo[1,5-a]pyridin-2-yl)-1-(cyclopropylmethyl)-1H-pyrrolo[2,3-c]pyridin-7-yl)oxy)methyl)pyrrolidin-2-one (isomer 1)

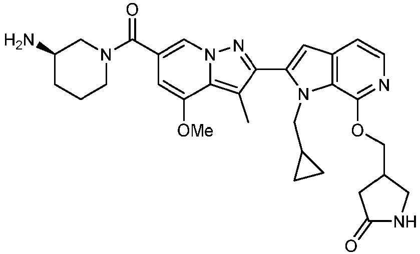

### Intermediate 591A

### Methyl 2-(7-chloro-1-(cyclopropylmethyl)-1H-pyrrolo[2,3-c]pyridin-2-yl)-4-methoxy-3-methylpyrazolo[1,5-a]pyridine-6-carboxylate

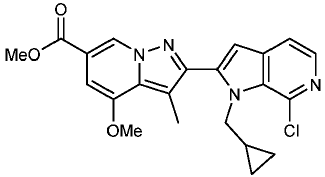

1-Amino-3-methoxy-5-(methoxycarbonyl)pyridin-1-ium (2.261 g, 12.34 mmol) was added to a stirred solution of (E)-7-chloro-1-(cyclopropylmethyl)-2-(2-nitroprop-1-en-1-yl)-1H-pyrrolo[2,3-c]pyridine (1.2 g, 4.11 mmol) in DMF (10 ml). The reaction mixture was stirred at 80°C for 30 hours, diluted with water (20 ml), and extracted with ethyl acetate (3×30 ml). The combined organic layers were washed with brine, dried over sodium sulfate, filtered, and concentrated in vacuum. The crude product thus obtained was purified using a silica gel column, eluting with 0-20% ethyl acetate in n-hexane to afford (820 mg, 47%) of methyl 2-(7-chloro-1-(cyclopropylmethyl)-1H-pyrrolo[2,3-c]pyridin-2-yl)-4-methoxy-3-methylpyrazolo[1,5-a]pyridine-6-carboxylate as brown solid. LC-MS m/z: 425.2 [M+H]⁺.

### Intermediate 591B

### 2-(7-chloro-1-(cyclopropylmethyl)-1H-pyrrolo[2,3-c]pyridin-2-yl)-4-methoxy-3-methylpyrazolo[1,5-a]pyridine-6-carboxylic acid

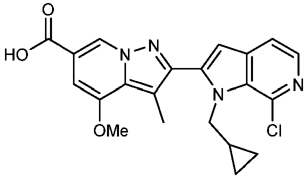

Intermediate 591B (0.62 g, 92%) was prepared following a similar procedure depicted for example 550A. LC-MS m/z: 411.1 [M+H]⁺; ¹H NMR (400 MHz, DMSO-d₆) δ ppm:8.82 (s, 1H), 8.02 (d, *J*=5.5 Hz, 1H), 7.68 (d, *J*=5.5 Hz, 1H), 6.99 (s, 1H), 6.91 (s, 1H), 4.69 (d, J=7.0 Hz, 2H), 4.02 (s, 3H), 2.47 (s, 3H), 1.06-1.03 (m, 1H), 0.29-0.22 (m, 2H), -0.02--0.05(m, 2H).

### Intermediate 591C

### tert-butyl (R)-(1-(2-(7-chloro-1-(cyclopropylmethyl)-1H-pyrrolo[2,3-c]pyridin-2-yl)-4-methoxy-3-methylpyrazolo[1,5-a]pyridine-6-carbonyl)piperidin-3-yl)carbamate

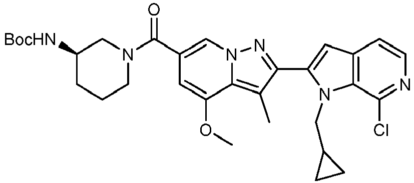

tert-Butyl (R)-piperidin-3-ylcarbamate (175 mg, 0.876 mmol), HATU (416 mg, 1.095 mmol), DIPEA (0.383 ml, 2.191 mmol) was added to a stirred solution of 2-(7-chloro-1-(cyclopropylmethyl)-1H-pyrrolo[2,3-c]pyridin-2-yl)-4-methoxy-3-methylpyrazolo[1,5-a]pyridine-6-carboxylic acid (300 mg, 0.730 mmol) in DMF (5 ml). The reaction mixture was stirred at a room temperature for 6 hours, diluted with water (10 ml), and extracted with ethyl acetate (20 ml). The combined organic layers were washed with brine, dried over sodium sulfate, filtered, and concentrated in vacuum. The crude product thus obtained was purified using comb flash chromatography (12 g, Redisep^{®} SiO₂ column, eluting with 0-10% methanol in dichloromethane to afford tert-butyl (R)-(1-(2-(7-chloro-1-(cyclopropylmethyl)-1H-pyrrolo[2,3-c]pyridin-2-yl)-4-methoxy-3-methylpyrazolo[1,5-a]pyridine-6-carbonyl)piperidin-3-yl)carbamate (380 mg ,88%) as brown solid. LC-MS m/z: 593.5 [M+H]⁺

### Intermediate 591D

### tert-Butyl ((3R)-1-(2-(1-(cyclopropylmethyl)-7-((5-oxopyrrolidin-3-yl)methoxy)-1H-pyrrolo[2,3-c]pyridin-2-yl)-4-methoxy-3-methylpyrazolo[1,5-a]pyridine-6-carbonyl)piperidin-3-yl)carbamate

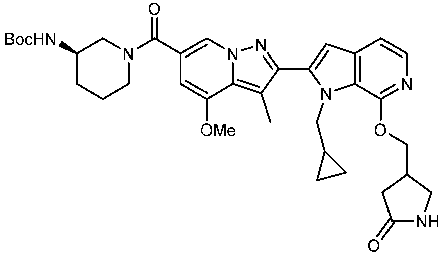

Racemic 4-(hydroxymethyl)pyrrolidin-2-one (88 mg, 0.674 mmol), Cs₂CO₃ (412 mg, 1.264 mmol), S-BINAP (52.5 mg, 0.084 mmol) and Pd₂(dba)₃ (38.6 mg, 0.042 mmol) were added to a stirred solution of tert-butyl (R)-(1-(2-(7-chloro-1-(cyclopropylmethyl)-1H-pyrrolo[2,3-c]pyridin-2-yl)-4-methoxy-3-methylpyrazolo[1,5-a]pyridine-6-carbonyl)piperidin-3-yl)carbamate (250 mg, 0.421 mmol) in Toluene (3 ml). The reaction mixture was stirred at 95°C for 16 hours, diluted with water (10 ml), and extracted with ethyl acetate (2×20 ml). The combined organic layers were washed with brine, dried over sodium sulfate, filtered, and concentrated in vacuum. The crude product thus obtained was purified using a silica gel column, eluting with 0-8% Methanol in dichloromethane) to afford racemic compound, which was separated using a chiral SFC. Isomer 01: tert-butyl ((3R)-1-(2-(1-(cyclopropylmethyl)-7-((5-oxopyrrolidin-3-yl)methoxy)-1H-pyrrolo[2,3-c]pyridin-2-yl)-4-methoxy-3-methylpyrazolo[1,5-a]pyridine-6-carbonyl)piperidin-3-yl)carbamate (30 mg, 10%); isomer 02: tert-butyl ((3R)-1-(2-(1-(cyclopropylmethyl)-7-((5-oxopyrrolidin-3-yl)methoxy)-1H-pyrrolo[2,3-c]pyridin-2-yl)-4-methoxy-3-methylpyrazolo[1,5-a]pyridine-6-carbonyl)piperidin-3-yl)carbamate (30mg, 10%). LC-MS m/z: 672.4 [M+H]⁺ (Method H).

### Example 591

### 4-(((2-(6-((R)-3-Aminopiperidine-1-carbonyl)-4-methoxy-3-methylpyrazolo[1,5-a]pyridin-2-yl)-1-(cyclopropylmethyl)-1H-pyrrolo[2,3-c]pyridin-7-yl)oxy)methyl)pyrrolidin-2-one (isomer 1)

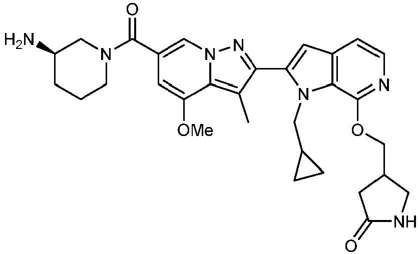

TFA (0.034 ml, 0.447 mmol) was added to a stirred solution of tert-butyl ((3R)-1-(2-(1-(cyclopropylmethyl)-7-((5-oxopyrrolidin-3-yl)methoxy)-1H-pyrrolo[2,3-c]pyridin-2-yl)-4-methoxy-3-methylpyrazolo[1,5-a]pyridine-6-carbonyl)piperidin-3-yl)carbamate (30 mg, 0.045 mmol) in dichloromethane(5 ml). The reaction mixture was stirred at a room temperature for 16 hours, concentrated, and the crude product thus obtained was purified using a preparative LC/MS with the following conditions: Column: Waters XBridge C18, 19×150 mm, 5 µm particles, mobile phase A: 10 mM ammonium acetate; mobile phase B: acetonitrile; gradient: 12-48% B over 25 minutes, then a 5-minute hold at 100% B; flow rate: 15 ml/min. Fractions containing the desired product were combined and dried using centrifugal evaporation. The purified material was then diluted with a 1:1 mixture of DCM and methanol, treated with Si-Pyridine, and shaken for a minimum of 2 hours. The resulting mixture was filtered and dried using centrifugal evaporation to afford 4-(((2-(6-((R)-3-aminopiperidine-1-carbonyl)-4-methoxy-3-methylpyrazolo[1,5-a]pyridin-2-yl)-1-(cyclopropylmethyl)-1H-pyrrolo[2,3-c]pyridin-7-yl)oxy)methyl)pyrrolidin-2-one (isomer 1) (18.8 mg, 74%). LC-MS m/z: 572.2 [M+H]⁺. Retention time: 1.309 min (Method E). ¹H NMR (400 MHz, DMSO-*d₆*) δ ppm: 8.40 (s, 1H), 7.69 (d, *J*=5.4 Hz, 1H), 7.60 (s, 1H), 7.23 (d, *J*=5.4 Hz, 1H), 6.75 (s, 1H), 6.59 (s, 1H), 4.53-4.45 (m,4H), 3.98 (s, 3H), 3.49-3.47 (m, 1H), 3.22-3.19 (m, 2H), 3.06-2.95 (m, 3H), 2.80-2.73 (m, 1H), 2.46 (s, 3H), 2.41-2.35 (m, 1H), 2.21-2.13 (m, 1H), 1.72-1.70 (m, 1H), 1.50-1.48 (m, 1H), 1.33-1.26 (m, 1H), 1.09-1.01 (m, 1H), 0.28-0.20 (m, 2H), 0.06-0.01 (m, 2H).

Compounds of Examples 592 to 608 in Table 56 were prepared following a procedure similar to the preparation of a compound of Example 591, by Buchwald etherification reaction of Intermediate 591C with appropriate alcohols

**Table 56**

| Example | Structure |
|---|---|
| | Name |
| | Analytical Data: LC-MS m/z [M+H]+; HPLC retention time (HPLC method) |
| 592 | 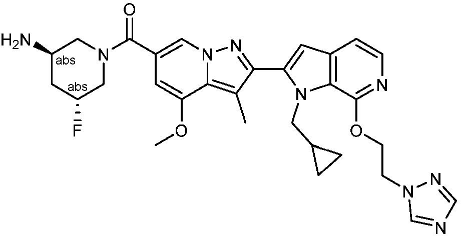 (2-(7-(2-(1H-1,2,4-Triazol-1-yl)ethoxy)-1-(cyclopropylmethyl)-1H-pyrrolo[2,3-c]pyridin-2-yl)-4-methoxy-3-methylpyrazolo[1,5-a]pyridin-6-yl)((3R,5R)-3-amino-5-fluoropiperidin-1-yl)methanone |
| | Analytical Data: 588.3, 1.476 min (Method E) |
| 593 | 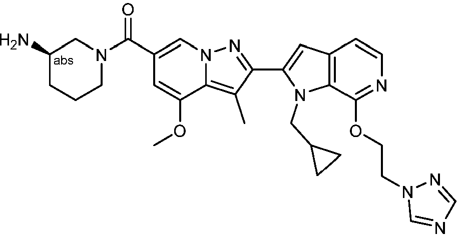 (R)-(2-(7-(2-(1H-1,2,4-Triazol-1-yl)ethoxy)-1-(cyclopropylmethyl)-1H-pyrrolo[2,3-c]pyridin-2-yl)-4-methoxy-3-methylpyrazolo[1,5-a]pyridin-6-yl)(3-aminopiperidin-1-yl)methanone |
| | Analytical Data: 570.2, 1.405 min (Method E) |
| 594 | 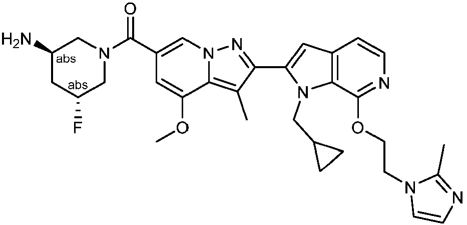 ((3R,5R)-3-Amino-5-fluoropiperidin-1-yl)(2-(1-(cyclopropylmethyl)-7-(2-(2-methyl-1H-imidazol-1-yl)ethoxy)-1H-pyrrolo[2,3-c]pyridin-2-yl)-4-methoxy-3-methylpyrazolo[1,5-a]pyridin-6-yl)methanone |
| | Analytical Data: 601.3, 1.513 min (Method E) |
| 595 | 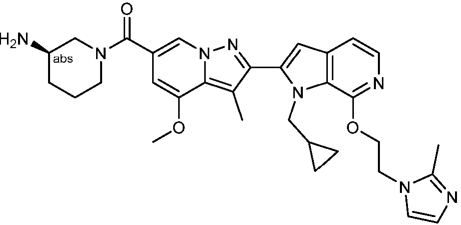 (R)-(3-Aminopiperidin-1-yl)(2-(1-(cyclopropylmethyl)-7-(2-(2-methyl-1H-imidazol-1-yl)ethoxy)-1H-pyrrolo[2,3-c]pyridin-2-yl)-4-methoxy-3-methylpyrazolo[1,5-a]pyridin-6-yl)methanone |
| | Analytical Data: 571.3, 1.398 min (Method E) |
| 596 | 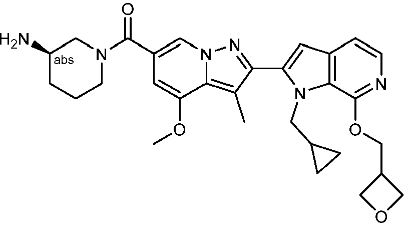 (R)-(3-Aminopiperidin-1-yl)(2-(1-(cyclopropylmethyl)-7-(oxetan-3-ylmethoxy)-1H-pyrrolo[2,3-c]pyridin-2-yl)-4-methoxy-3-methylpyrazolo[1,5-a]pyridin-6-yl)methanone |
| | Analytical Data: 545.3, 0.982 min (Method E) |
| 597 | 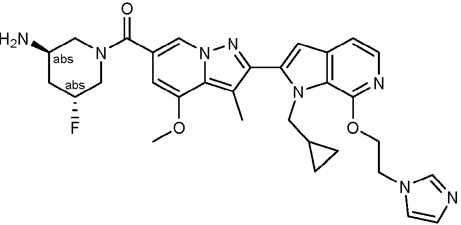 (2-(7-(2-(1H-Imidazol-1-yl)ethoxy)-1-(cyclopropylmethyl)-1H-pyrrolo[2,3-c]pyridin-2-yl)-4-methoxy-3-methylpyrazolo[1,5-a]pyridin-6-yl)((3R,5R)-3-amino-5-fluoropiperidin-1-yl)methanone |
| | Analytical Data: 587.3, 1.530min (Method E) |
| 598 | 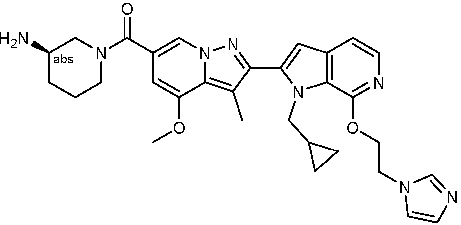 (R)-(2-(7-(2-(1H-Imidazol-1-yl)ethoxy)-1-(cyclopropylmethyl)-1H-pyrrolo[2,3-c]pyridin-2-yl)-4-methoxy-3-methylpyrazolo[1,5-a]pyridin-6-yl)(3-aminopiperidin-1-yl)methanone |
| | Analytical Data: 569.3, 1.467 min (Method E) |
| 599 | 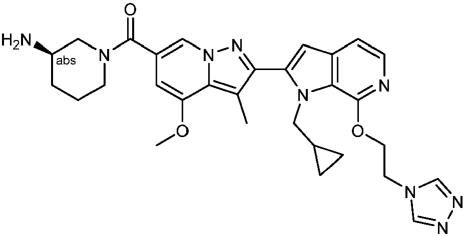 (R)-(2-(7-(2-(4H-1,2,4-Triazol-4-yl)ethoxy)-1-(cyclopropylmethyl)-1H-pyrrolo[2,3-c]pyridin-2-yl)-4-methoxy-3-methylpyrazolo[1,5-a]pyridin-6-yl)(3-aminopiperidin-1-yl)methanone |
| | Analytical Data: 570.3, 1.313 min (Method E) |
| 600 | 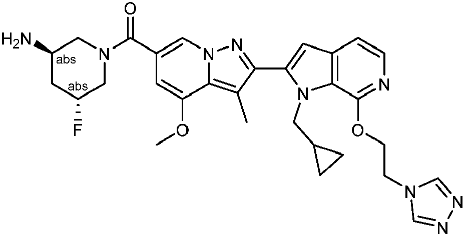 (2-(7-(2-(4H-1,2,4-Triazol-4-yl)ethoxy)-1-(cyclopropylmethyl)-1H-pyrrolo[2,3-c]pyridin-2-yl)-4-methoxy-3-methylpyrazolo[1,5-a]pyridin-6-yl)((3R,5R)-3-amino-5-fluoropiperidin-1-yl)methanone |
| | Analytical Data: 588.2, 1.369 min (Method E) |
| 601 | 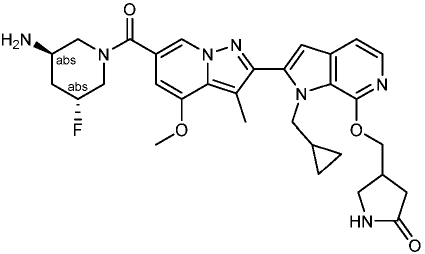 4-(((2-(6-((3R,5R)-3-Amino-5-fluoropiperidine-1-carbonyl)-4-methoxy-3-methylpyrazolo[1,5-a]pyridin-2-yl)-1-(cyclopropylmethyl)-1H-pyrrolo[2,3-c]pyridin-7-yl)oxy)methyl)pyrrolidin-2-one (isomer 1) |
| | Analytical Data: 590.3, 1.434 min (Method E) |
| 602 | 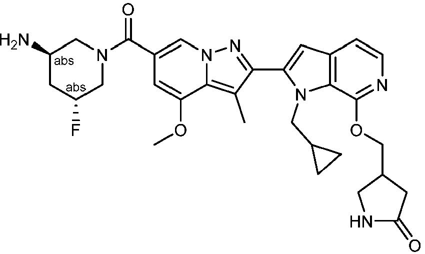 4-(((2-(6-((3R,5R)-3-Amino-5-fluoropiperidine-1-carbonyl)-4-methoxy-3-methylpyrazolo[1,5-a]pyridin-2-yl)-1-(cyclopropylmethyl)-1H-pyrrolo[2,3-c]pyridin-7-yl)oxy)methyl)pyrrolidin-2-one (isomer 2) |
| | Analytical Data: 590.2, 1.436 min (Method E) |
| 603 | 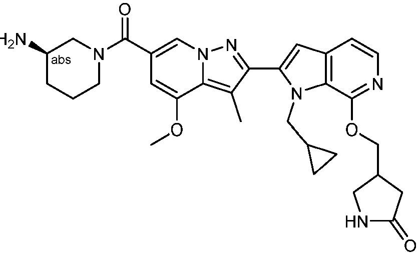 4-(((2-(6-((R)-3-Aminopiperidine-1-carbonyl)-4-methoxy-3-methylpyrazolo[1,5-a]pyridin-2-yl)-1-(cyclopropylmethyl)-1H-pyrrolo[2,3-c]pyridin-7-yl)oxy)methyl)pyrrolidin-2-one (isomer 2) |
| | Analytical Data: 572.2, 1.312 min (Method E) |
| 604 | 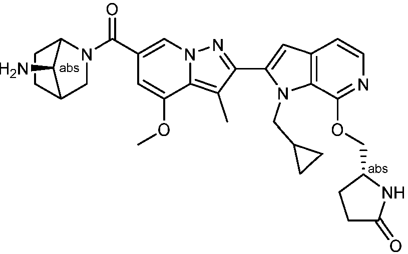 (5R)-5-(((2-(6-((7R)-7-Amino-2-azabicyclo[2.2.1]heptane-2-carbonyl)-4-methoxy-3-methylpyrazolo[1,5-a]pyridin-2-yl)-1-(cyclopropylmethyl)-1H-pyrrolo[2,3-c]pyridin-7-yl)oxy)methyl)pyrrolidin-2-one |
| | Analytical Data: 584.3, 1.438 min (Method E) |
| 605 | 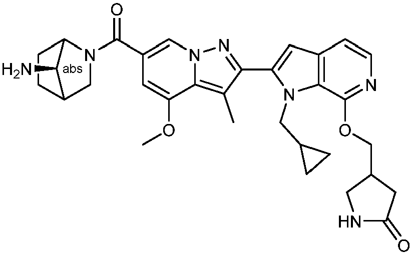 4-(((2-(6-((7R)-7-Amino-2-azabicyclo[2.2.1]heptane-2-carbonyl)-4-methoxy-3-methylpyrazolo[1,5-a]pyridin-2-yl)-1-(cyclopropylmethyl)-1H-pyrrolo[2,3-c]pyridin-7-yl)oxy)methyl)pyrrolidin-2-one (isomer 1) |
| | Analytical Data: 584.3, 8.372 min (Method C) |
| 606 | 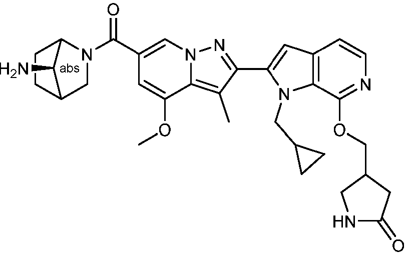 4-(((2-(6-((7R)-7-Amino-2-azabicyclo[2.2.1]heptane-2-carbonyl)-4-methoxy-3-methylpyrazolo[1,5-a]pyridin-2-yl)-1-(cyclopropylmethyl)-1H-pyrrolo[2,3-c]pyridin-7-yl)oxy)methyl)pyrrolidin-2-one (isomer 2) |
| | Analytical Data: 584.3, 8.335 min (Method C) |
| 607 | 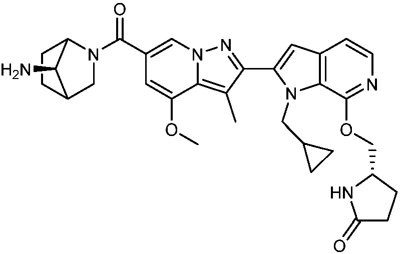 (5R)-5-(((2-(6-((7S)-7-Amino-2-azabicyclo[2.2.1]heptane-2-carbonyl)-4-methoxy-3-methylpyrazolo[1,5-a]pyridin-2-yl)-1-(cyclopropylmethyl)-1H-pyrrolo[2,3-c]pyridin-7-yl)oxy)methyl)pyrrolidin-2-one |
| | Analytical Data: 584.4, 1.505 min (Method E) |
| 608 | 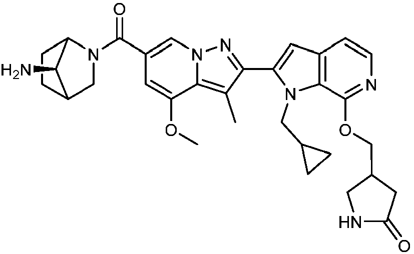 4-(((2-(6-((7R)-7-Amino-2-azabicyclo[2.2.1]heptane-2-carbonyl)-4-methoxy-3-methylpyrazolo[1,5-a]pyridin-2-yl)-1-(cyclopropylmethyl)-1H-pyrrolo[2,3-c]pyridin-7-yl)oxy)methyl)pyrrolidin-2-one (mixture of diastereomers) |
| | Analytical Data:584.3, 1.456 min (Method E) |

### Example 609

### (R)-(2-(7-(2-(4H-1,2,4-Triazol-4-yl)ethoxy)-1-(cyclopropylmethyl)-1H-pyrrolo[2,3-c]pyridin-2-yl)-3-methylpyrazolo[1,5-a]pyridin-6-yl)(3-aminopiperidin-1-yl)methanone

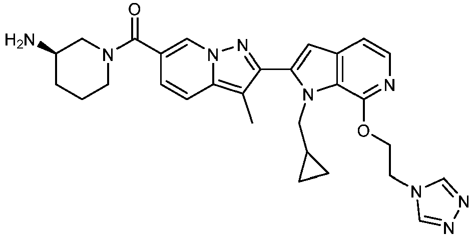

### Intermediate 609A

### Ethyl 2-(7-chloro-1-(cyclopropylmethyl)-1H-pyrrolo[2,3-c]pyridin-2-yl)-3-methylpyrazolo[1,5-a]pyridine-6-carboxylate

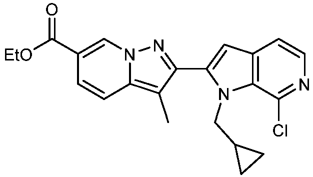

1-Amino-3-(ethoxycarbonyl)pyridin-1-ium (2.063 g, 12.34 mmol) was added to a stirred solution of (E)-7-chloro-1-(cyclopropylmethyl)-2-(2-nitroprop-1-en-1-yl)-1H-pyrrolo[2,3-c]pyridine (1.2 g, 4.11 mmol) in DMF (3 ml). The reaction mixture was stirred at 80°C for 30 hours, diluted with water (20 ml), and extracted with ethyl acetate (3×20 ml). The combined organic layers were washed with brine, dried over sodium sulfate, filtered, and concentrated in vacuum. A crude product thus obtained was purified using a silica gel column, eluting with 0-20% ethyl acetate in hexane to afford ethyl 2-(7-chloro-1-(cyclopropylmethyl)-1H-pyrrolo[2,3-c] pyridin-2-yl)-3-methylpyrazolo[1,5-a]pyridine-6-carboxylate (1.0 g, 60%) as brown solid. LC-MS m/z: 409.3 [M+H]⁺; ¹H NMR (300 MHz, DMSO-d6) δ ppm:9.24 (s, 1H), 8.30 (td, *J*=2.1, 7.9 Hz, 1H), 8.07-7.97 (m,1H), 7.88 (d, *J*=9.3 Hz, 1H), 7.71-7.55 (m, 3H), 7.05 (s, 1H), 4.78 (d, *J*=6.8 Hz, 2H), 4.42-4.31 (m, 2H), 2.38 (s, 3H), 1.40-1.30 (m, 3H), 1.08-1.03 (m, 1H), 0.40-0.19 (m, 2H), -0.01--0.04 (m, 2H).

### Intermediate 609B

### 2-(7-Chloro-1-(cyclopropylmethyl)-1H-pyrrolo[2,3-c]pyridin-2-yl)-3-methylpyrazolo[1,5-a]pyridine-6-carboxylic acid

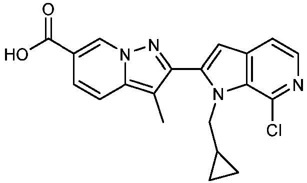

2-(7-Chloro-1-(cyclopropylmethyl)-1*H*-pyrrolo[2,3-c]pyridin-2-yl)-3-methylpyrazolo[1,5-a]pyridine-6-carboxylic acid (0.55 g, 82%) was prepared using procedure similar to the preparation of Intermediate 510D as a brown solid as a brown solid. LC-MS m/z: 381.3 [M+H]⁺; ¹H NMR (300 MHz, DMSO-d6) δ ppm:9.16 (s, 1H), 8.02 (d, *J*=5.3 Hz, 1H), 7.85 (d, *J*=9.4 Hz, 1H), 7.69 (d, *J*=5.3 Hz, 1H), 7.64 (dd, *J*=1.5, 9.4 Hz, 1H), 7.04 (s, 1H), 4.77 (d, *J*=6.8 Hz, 2H), 2.38 (s, 3H), 1.23 (s, 1H), 1.18-0.96 (m, 1H), 0.25 (br, d, *J*=7.9 Hz, 2H), -0.01--0.06 (m,2H).

### Intermediate 609C

### tert-Butyl (R)-(1-(2-(7-chloro-1-(cyclopropylmethyl)-1H-pyrrolo[2,3-c]pyridin-2-yl)-3-methylpyrazolo[1,5-a]pyridine-6-carbonyl)piperidin-3-yl)carbamate

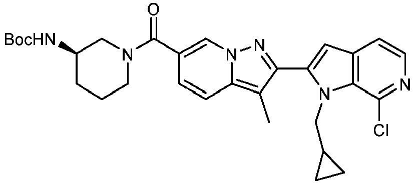

Intermediate 609C was prepared following a procedure similar to the preparation of Intermediate 386E (200 mg, 90%). LC-MS m/z: 563.3 [M+H]⁺.

### Intermediate 609D

### tert-Butyl (R)-(1-(2-(7-(2-(4H-1,2,4-triazol-4-yl)ethoxy)-1-(cyclopropylmethyl)-1H-pyrrolo[2,3-c]pyridin-2-yl)-3-methylpyrazolo[1,5-a]pyridine-6-carbonyl)piperidin-3-yl)carbamate

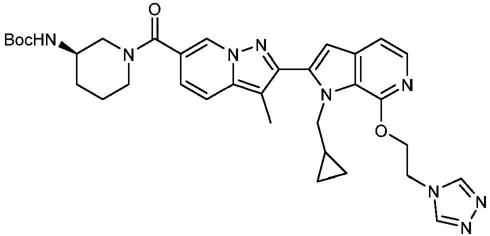

2-(4H-1,2,4-Triazol-4-yl)ethan-1-ol (30.1 mg, 0.266 mmol), Cs₂CO₃ (174 mg, 0.533 mmol), S-BINAP (22.12 mg, 0.036 mmol) and Pd₂(dba)₃ (16.26 mg, 0.018 mmol) were added to a stirred solution of tert-butyl (R)-(1-(2-(7-chloro-1-(cyclopropylmethyl)-1H-pyrrolo[2,3-c]pyridin-2-yl)-3-methylpyrazolo[1,5-a]pyridine-6-carbonyl)piperidin-3-yl)carbamate (100 mg, 0.178 mmol) in 1,4-dioxane (3 ml). The reaction mixture was degassed with argon for 5 and then stirred at 85°C for 16 hours, diluted with water (20 ml), and extracted with ethyl acetate (20 ml). The combined organic layers were washed with brine, dried over sodium sulfate, filtered, and concentrated in vacuum. A crude product thus obtained was purified using a silica gel column eluting with 0-8% MeOH in dichloromethane to afford (40 mg, 35%) tert-butyl (R)-(1-(2-(7-(2-(4H-1,2,4-triazol-4-yl)ethoxy)-1-(cyclopropylmethyl)-1H-pyrrolo[2,3-c]pyridin-2-yl)-3-methylpyrazolo[1,5-a]pyridine-6-carbonyl)piperidin-3-yl)carbamate. LCMS m/z: 640.6 [M+H]⁺

### Example 609

### (R)-(2-(7-(2-(4H-1,2,4-Triazol-4-yl)ethoxy)-1-(cyclopropylmethyl)-1H-pyrrolo[2,3-c]pyridin-2-yl)-3-methylpyrazolo[1,5-a]pyridin-6-yl)(3-aminopiperidin-1-yl)methanone

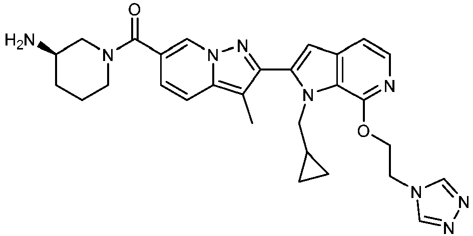

HCl in dioxane (4.0 M) (0.019 ml, 0.625 mmol) was added to a stirred solution of tert-butyl (R)-(1-(2-(7-(2-(4H-1,2,4-triazol-4-yl)ethoxy)-1-(cyclopropylmethyl)-1H-pyrrolo[2,3-c]pyridin-2-yl)-3-methylpyrazolo[1,5-a]pyridine-6-carbonyl)piperidin-3-yl)carbamate (40 mg, 0.063 mmol) in 1,4-dioxane (1 ml). The reaction mixture was stirred at a room temperature for 1 hour, concentrated, and the crude product thus obtained was purified using a preparative LC/MS with the following conditions: Column: Waters XBridge C18, 19×150 mm, 5 µm particles; mobile phase A: 10 mM ammonium acetate; mobile phase B: acetonitrile; gradient: 10-30% B over 20 minutes, then a 5-minute hold at 100% B; flow rate: 20 ml/min. Fractions containing the desired product were combined and dried using centrifugal evaporation. The purified material was then diluted with a 1:1 mixture of ethylene dichloride and methanol (EDM), treated with Si-Pyridine, and shaken for a minimum of 2 hours The resulting mixture was filtered and dried using centrifugal evaporation to afford (R)-(2-(7-(2-(4H-1,2,4-triazol-4-yl)ethoxy)-1-(cyclopropylmethyl)-1H-pyrrolo[2,3-c]pyridin-2-yl)-3-methylpyrazolo[1,5-a]pyridin-6-yl)(3-aminopiperidin-1-yl)methanone (8.9 mg, 26%). LC-MS m/z : 540.2 [M+H]⁺, LCMS retention time 1.195 min. ¹H NMR (400 MHz, DMSO-*d₆*) δ ppm: 8.84 (s, 1H), 8.64 (s, 2H), 7.82 (s, 1H), 7.70 (d, *J*=5.4 Hz, 1H), 7.27-7.24 (m, 2H), 6.79 (s, 1H), 4.85 (br, d, *J*=6.1 Hz, 2H), 4.57 (dd, *J*=3.9, 6.1 Hz, 2H), 4.43-4.41 (m, 2H), 3.29-3.03 (m, 3H), 2.89-2.84 (m, 1H), 2.53-2.53 (m, 1H), 2.36 (s, 3H), 1.92-1.89 (m, 2H), 1.24-1.00 (m, 2H), 0.82-0.80 (m, 1H), 0.17-0.14 (m, 2H), -0.10--0.13 (m, 2H)
Compounds of Examples 610 to 619 in Table 57 were obtained following a procedure similar to the preparation of a compound of Example 610, by Buchwald etherification reaction of Intermediate 609C with appropriate alcohols

**Table 57**

| Example | Structure |
|---|---|
| | Name |
| | Analytical Data: LC-MS m/z [M+H]+; HPLC retention time (HPLC method) |
| 610 | 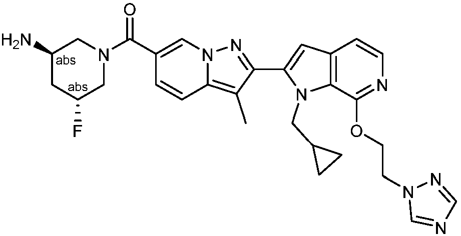 |
| | (2-(7-(2-(1H-1,2,4-Triazol-1-yl)ethoxy)-1-(cyclopropylmethyl)-1H-pyrrolo[2,3-c]pyridin-2-yl)-3-methylpyrazolo[1,5-a]pyridin-6-yl)((3R,5R)-3-amino-5-fluoropiperidin-1-yl)methanone |
| | Analytical Data: 558.3, 1.357 min (Method E) |
| 611 | 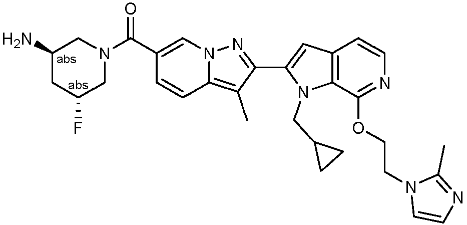 |
| | ((3R,5R)-3-Amino-5-fluoropiperidin-1-yl)(2-(1-(cyclopropylmethyl)-7-(2-(2-methyl-1H-imidazol-1-yl)ethoxy)-1H-pyrrolo[2,3-c]pyridin-2-yl)-3-methylpyrazolo[1,5-a]pyridin-6-yl)methanone |
| | Analytical Data: 571.3, 1.398 min (Method E) |
| 612 | 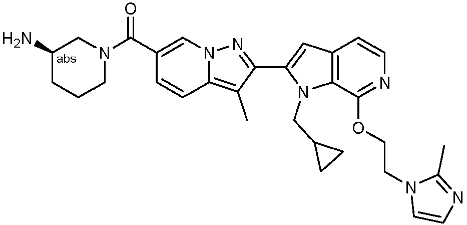 |
| | (R)-(3-Aminopiperidin-1-yl)(2-(1-(cyclopropylmethyl)-7-(2-(2-methyl-1H-imidazol-1-yl)ethoxy)-1H-pyrrolo[2,3-c]pyridin-2-yl)-3-methylpyrazolo[1,5-a]pyridin-6-yl)methanone |
| | Analytical Data: 553.3, 1.337 min (Method E) |
| 613 | 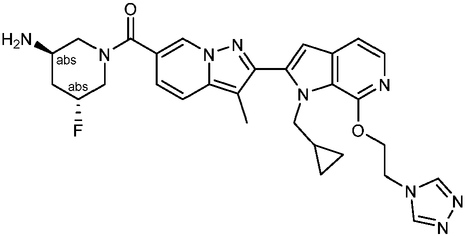 |
| | (2-(7-(2-(4H-1,2,4-Triazol-4-yl)ethoxy)-1-(cyclopropylmethyl)-1H-pyrrolo[2,3-c]pyridin-2-yl)-3-methylpyrazolo[1,5-a]pyridin-6-yl)((3R,5R)-3-amino-5-fluoropiperidin-1-yl)methanone |
| | Analytical Data: 558.2, 1.262 min (Method E) |
| 614 | 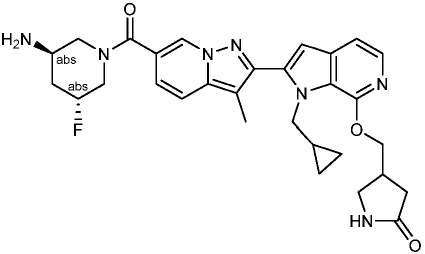 |
| | 4-(((2-(6-((3R,5R)-3-Amino-5-fluoropiperidine-1-carbonyl)-3-methylpyrazolo[1,5-a]pyridin-2-yl)-1-(cyclopropylmethyl)-1H-pyrrolo[2,3-c]pyridin-7-yl)oxy)methyl)pyrrolidin-2-one (isomer 1) |
| | Analytical Data: 560.3, 1.340 min (Method E) |
| 615 | 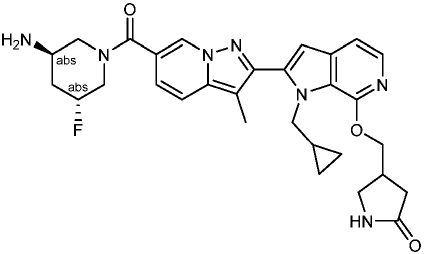 |
| | 4-(((2-(6-((3R,5R)-3-Amino-5-fluoropiperidine-1-carbonyl)-3-methylpyrazolo[1,5-a]pyridin-2-yl)-1-(cyclopropylmethyl)-1H-pyrrolo[2,3-c]pyridin-7-yl)oxy)methyl)pyrrolidin-2-one (isomer 2) |
| | Analytical Data: 560.2, 1.329 min (Method E) |
| 616 | 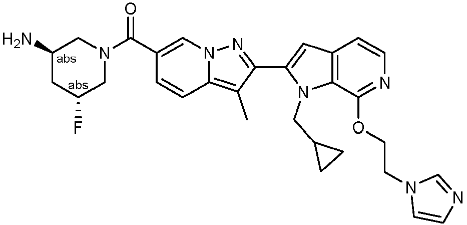 |
| | (2-(7-(2-(1H-Imidazol-1-yl)ethoxy)-1-(cyclopropylmethyl)-1H-pyrrolo[2,3-c]pyridin-2-yl)-3-methylpyrazolo[1,5-a]pyridin-6-yl)((3R,5R)-3-amino-5-fluoropiperidin-1-yl)methanone |
| | Analytical Data: 557.2, 1.414 min (Method E) |
| 617 | 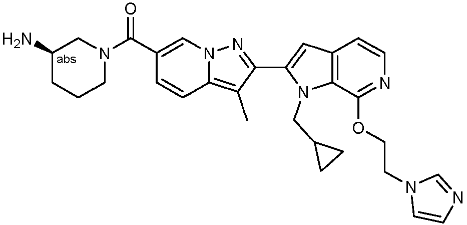 |
| | (R)-(2-(7-(2-(1H-Imidazol-1-yl)ethoxy)-1-(cyclopropylmethyl)-1H-pyrrolo[2,3-c]pyridin-2-yl)-3-methylpyrazolo[1,5-a]pyridin-6-yl)(3-aminopiperidin-1-yl)methanone |
| | Analytical Data: 539.2, 1.349 min (Method E) |
| 618 | 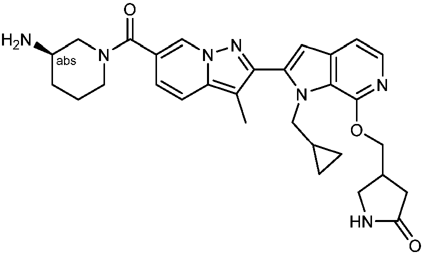 |
| | 4-(((2-(6-((R)-3-Aminopiperidine-1-carbonyl)-3-methylpyrazolo[1,5-a]pyridin-2-yl)-1-(cyclopropylmethyl)-1H-pyrrolo[2,3-c]pyridin-7-yl)oxy)methyl)pyrrolidin-2-one (isomer 1) |
| | Analytical Data: 542.2, 1.256 min (Method E) |
| 619 | 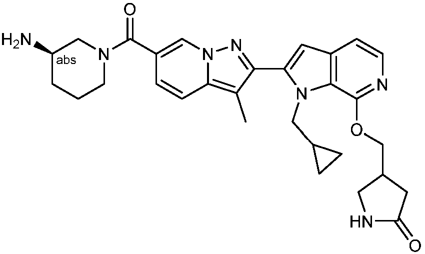 |
| | 4-(((2-(6-((R)-3-Aminopiperidine-1-carbonyl)-3-methylpyrazolo[1,5-a]pyridin-2-yl)-1-(cyclopropylmethyl)-1H-pyrrolo[2,3-c]pyridin-7-yl)oxy)methyl)pyrrolidin-2-one (isomer 2) |
| | Analytical Data: 542.2, 1.255 min (Method E) |

### Example 620

### (2-(7-(2-(4H-1,2,4-Triazol-4-yl)ethoxy)-1-methyl-1H-pyrrolo[2,3-c]pyridin-2-yl)-4-methoxy-3-methylpyrazolo[1,5-a]pyridin-6-yl)((3R,5R)-3-amino-5-fluoropiperidin-1-yl)methanone

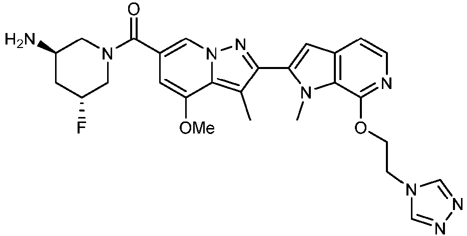

### Intermediate 620A

### Methyl 2-(7-chloro-1-methyl-1H-pyrrolo[2,3-c]pyridin-2-yl)-4-methoxy-3-methylpyrazolo[1,5-a]pyridine-6-carboxylate

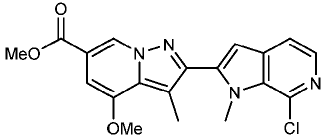

Intermediate 1 (4.37 g, 23.84 mmol) and copper(I) iodide (0.454 g, 2.384 mmol) were added to a solution of 7-chloro-1-methyl-2-(2-nitroprop-1-en-1-yl)-1H-pyrrolo[2,3-c]pyridine (2 g, 7.95 mmol) in DMF (10 ml). The reaction mixture was stirred for 16 hours, diluted with ethyl acetate (100 ml), washed with water (3×50 ml), dried over Na₂SO₄, and concentrated to afford a crude compound, which was purified using a silica gel column using 0-30% ethyl acetate in Hexane to afford methyl 2-(7-chloro-1-methyl-1H-pyrrolo[2,3-c]pyridin-2-yl)-4-methoxy-3-methylpyrazolo[1,5-a]pyridine-6-carboxylate (1.4 g, 46%) as brown solid. LC-MS m/z: 385.3 [M+H]⁺.

### Intermediate 620B

### 2-(7-Chloro-1-methyl-1H-pyrrolo[2,3-c]pyridin-2-yl)-4-methoxy-3-methylpyrazolo[1,5-a]pyridine-6-carboxylic acid

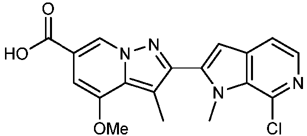

Intermediate 620B was prepared following a procedure similar to the preparation of Intermediate 519B (600 mg, 45%) as Off white solid.LC-MS m/z: 371.3 [M+H]⁺.

### Intermediate 620C

### tert-Butyl ((3R,5R)-1-(2-(7-chloro-1-methyl-1H-pyrrolo[2,3-c]pyridin-2-yl)-4-methoxy-3-methylpyrazolo[1,5-a]pyridine-6-carbonyl)-5-fluoropiperidin-3-yl)carbamate

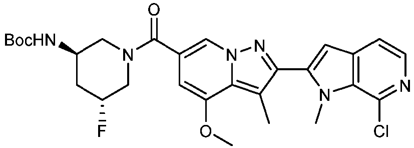

Intermediate 620C was prepared following a procedure similar to the preparation of Intermediate 519C (600 mg, 78%) as a brown solid. LC-MS m/z: 571.3 [M+H]⁺.

### Intermediate 620D

### tert-Butyl ((3R,5R)-1-(2-(7-(2-(4H-1,2,4-triazol-4-yl)ethoxy)-1-methyl-1H-pyrrolo[2,3-c]pyridin-2-yl)-4-methoxy-3-methylpyrazolo[1,5-a]pyridine-6-carbonyl)-5-fluoropiperidin-3-yl)carbamate

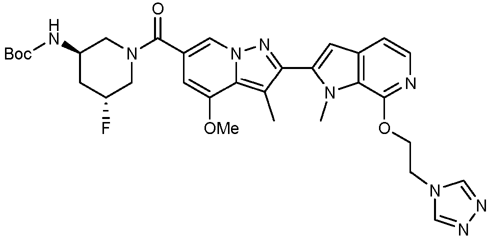

Intermediate 620D was prepared following a procedure similar to the preparation of Intermediate 519D (50 mg, 63%) as a brown solid. LC-MS m/z: 648.3 [M+H]⁺.

### Example 620

### (2-(7-(2-(4H-1,2,4-Triazol-4-yl)ethoxy)-1-methyl-1H-pyrrolo[2,3-c]pyridin-2-yl)-4-methoxy-3-methylpyrazolo[1,5-a]pyridin-6-yl)((3R,5R)-3-amino-5-fluoropiperidin-1-yl)methanone

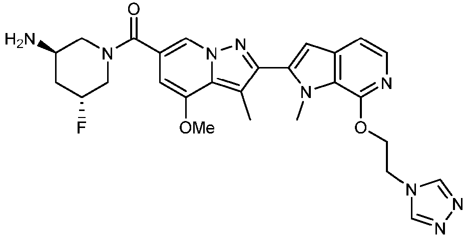

TFA (0.1 ml) was added to a solution of Intermediate 620D (20 mg, 0.031 mmol) in dichloromethane (5 ml), and the reaction mixture was stirred for 1 hour. The reaction mixture was concentrated to obtain a crude compound, which was purified using a preparative LC/MS with the following conditions: Column: Waters XBridge C18, 19×150 mm, 5 -µm particles; mobile phase A: 10 mM ammonium acetate; mobile phase B: acetonitrile; gradient: 9-32% B over 25 minutes, then a 5-minute hold at 100% B; flo rate w: 20 ml/min. Fractions containing the desired product were combined and dried via centrifugal evaporation. The purified material was then diluted with a 1:1 mixture of ethylene dichloride and methanol (EDM), treated with Si-Pyridine, and shaken for a minimum of 2 hours. The resulting mixture was filtered and dried using centrifugal evaporation to afford (2-(7-(2-(4H-1,2,4-triazol-4-yl)ethoxy)-1-methyl-1H-pyrrolo[2,3-c]pyridin-2-yl)-4-methoxy-3-methylpyrazolo[1,5-a]pyridin-6-yl)((3R,5R)-3-amino-5-fluoropiperidin-1-yl)methanone (3.2 mg,%). LC-MS m/z: 548.3 [M+H]⁺; LCMS retention time: 1.180 min [method E]

### Example 621

### ((3R,5R)-3-Amino-5-fluoropiperidin-1-yl) (2-(3-(cyclopropylmethyl)-4-(1-(2-hydroxyethyl)-1H-pyrazol-4-yl)benzo[b]thiophen-2-yl)-4-methoxy-3-methylpyrazolo[1,5-a]pyridin-6-yl)methanone

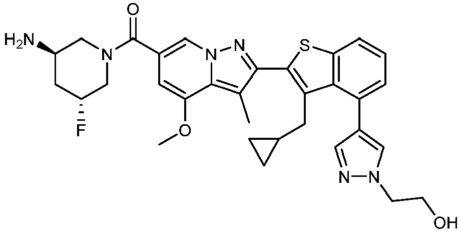

### Intermediate 621A

### Methyl 2-(4-bromo-3-(cyclopropylmethyl)benzo[b]thiophen-2-yl)-4-methoxy-3-methylpyrazolo[1,5-a]pyridine-6-carboxylate

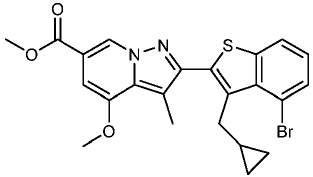

CuI (0.541 g, 2.84 mmol) was added to a stirred solution of Intermediate 62 (1 g, 2.84 mmol) and Intermediate 1(1.040 g, 5.68 mmol) in DMF (5 ml). The reaction mixture was stirred for 3 hours at 80°C, the volatiles were evaporated to dryness under reduced pressure, water (100 ml) was added, and extracted with ethyl acetate (2×100 ml). The combined organic extracts were washed with water (100 ml) and brine solution (100 ml), dried over sodium sulphate, filtered, and volatiles were evaporated to dryness under reduced pressure. Residue thus obtained was purified using a silica gel column using a 50% ethyl acetate in hexane to afford methyl 2-(4-bromo-3-(cyclopropylmethyl)benzo[b]thiophen-2-yl)-4-methoxy-3-methylpyrazolo[1,5-a]pyridine-6-carboxylate (500 mg, 36%). LC-MS m/z: 485.3 [M+H]⁺.

### Intermediate 621B

### 2-(4-Bromo-3-(cyclopropylmethyl)benzo[b]thiophen-2-yl)-4-methoxy-3-methylpyrazolo[1,5-a]pyridine-6-carboxylic acid

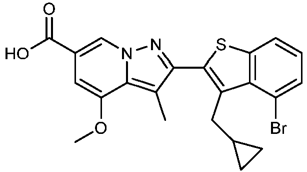

Lithium hydroxide (74.0 mg, 3.09 mmol) in water ( 1 ml) was added to a stirred solution of Intermediate 621A (500 mg, 1.030 mmol) in THF (10 ml). The reaction mixture was stirred at a room temperature for 16 hours, the volatiles were evaporated to dryness under reduced pressure. A residue thus obtained was dissolved in water (15 ml), cooled to 0°C and the pH was adjusted to pH 5 using 1.5 N HCl solution. The solids thus obtained were collected by vacuum filtration, and dried under vacuum at ambient temperature to afford 2-(4-bromo-3-(cyclopropylmethyl)benzo[b]thiophen-2-yl)-4-methoxy-3-methylpyrazolo[1,5-a]pyridine-6-carboxylic acid (400 mg, 82%). LC-MS m/z: 472.9 [M+H]⁺.

### Intermediate 621C

### tert-Butyl ((3R,5R)-1-(2-(4-bromo-3-(cyclopropylmethyl)benzo[b]thiophen-2-yl)-4-methoxy-3-methylpyrazolo[1,5-a]pyridine-6-carbonyl)-5-fluoropiperidin-3-yl)carbamate

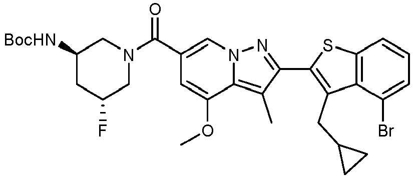

HATU (605 mg, 1.591 mmol) and DIPEA (0.556 ml, 3.18 mmol) were added to a stirred solution of Intermediate 621B (400 mg, 0.849 mmol), tert-butyl ((3R,5R)-5-fluoropiperidin-3-yl)carbamate (185 mg, 0.849 mmol) in DMF (2 ml), The reaction mixture was stirred at a room temperature for 16 hours, the volatiles were evaporated to dryness under reduced pressure, water (50 ml) was added, and extracted with ethyl acetate (2×50 ml). The combined organic extracts were washed with water (100 ml) and brine solution (100 ml), dried over sodium sulphate, filtered, and volatiles were evaporated to dryness under reduced pressure. A residue thus obtained was purified using a silica gel column (gradient of ethyl acetate in hexane), and the product was isolated at 40% ethyl acetate in hexane. Required fractions were collected and volatiles were evaporated to dryness under reduced pressure to afford tert-butyl ((3R,5R)-1-(2-(4-bromo-3-(cyclopropylmethyl)benzo[b]thiophen-2-yl)-4-methoxy-3-methylpyrazolo[1,5-a]pyridine-6-carbonyl)-5-fluoropiperidin-3-yl)carbamate (400 mg, 70%). LC-MS m/z: 673.1 [M+H]⁺.

### Intermediate 621D

### tert-Butyl ((3R,5R)-1-(2-(3-(cyclopropylmethyl)-4-(1-(2-hydroxyethyl)-1H-pyrazol-4-yl)benzo[b]thiophen-2-yl)-4-methoxy-3-methylpyrazolo[1,5-a]pyridine-6-carbonyl)-5-fluoropiperidin-3-yl)carbamate

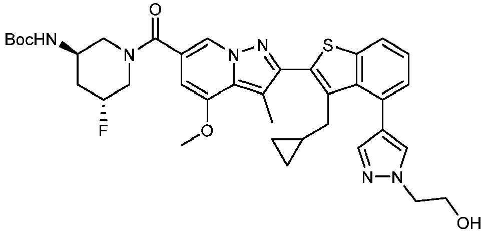

K₂CO₃ (30.9 mg, 0.223 mmol) and PdCl₂(dppf) (10.89 mg, 0.015 mmol) were added to a stirred solution Intermediate 621C (50 mg, 0.074 mmol) and 2-(4-(4,4,5,5-tetramethyl-1,3,2-dioxaborolan-2-yl)-1H-pyrazol-1-yl)ethan-1-ol (17.73 mg, 0.074 mmol) in 1,4-dioxane (2 ml) under argon atmosphere. The reaction mixture was heated at 80°C for 2 hours, the volatiles were evaporated to dryness under reduced pressure, water (10 ml) was added, and extracted with ethyl acetate (2×10 ml). The combined organic extracts were washed with water (10 ml) and brine solution (10 ml), organic layer was dried over sodium sulphate, filtered, and volatiles were evaporated to dryness under reduced pressure to afford tert-butyl ((3R,5R)-1-(2-(3-(cyclopropylmethyl)-4-(1-(2-hydroxyethyl)-1H-pyrazol-4-yl)benzo[b]thiophen-2-yl)-4-methoxy-3-methylpyrazolo[1,5-a]pyridine-6-carbonyl)-5-fluoropiperidin-3-yl)carbamate (40 mg, 76%). LC-MS m/z: 703.3 [M+H]⁺.

### Example 621

### ((3R,5R)-3-Amino-5-fluoropiperidin-1-yl) (2-(3-(cyclopropylmethyl)-4-(1-(2-hydroxyethyl)-1H-pyrazol-4-yl)benzo[b]thiophen-2-yl)-4-methoxy-3-methylpyrazolo[1,5-a]pyridin-6-yl)methanone

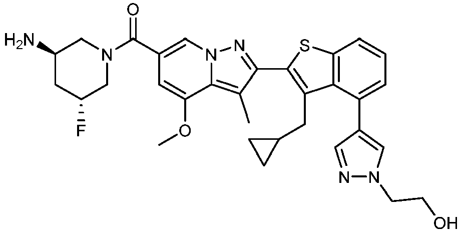

TFA (0.044 ml, 0.569 mmol) was added to a stirred solution of Intermediate 621D (40 mg, 0.057 mmol) in DCM (2 ml). The reaction mixture was stirred for 1 hour at a room temperature, the volatiles were evaporated to dryness under reduced pressure, and the crude compound thus obtained was purified using reverse phase HPLC to afford ((3R,5R)-3-amino-5-fluoropiperidin-1-yl) (2-(3-(cyclopropylmethyl)-4-(1-(2-hydroxyethyl)-1H-pyrazol-4-yl)benzo[b]thiophen-2-yl)-4-methoxy-3-methylpyrazolo[1,5-a]pyridin-6-yl)methanone (7.1 mg, 20%), LC-MS m/z: 603.3 [M+H]⁺. LCMS retention time: 1.558 min (method E).

### 6. Biological Assays

Compounds of the present invention were assayed as inhibitors of PAD4 using the assay protocols described below.

### RFMS human PAD4 Functional Assay 1:

Compounds were solubilized in 100% DMSO to achieve a10 mM compound concentration. Compound stock solutions were stored at RT. A series of dilutions were prepared in DMSO and mixed 8 times with 20 µL mixing volume. Final top concentration of compound in the assay is 50 µM. Final assay conditions were as follows:
Reaction volume: 26 µl
Assay buffer: 25 mM hepes, pH 7.5, 5 mM NaCl, 1 mM DTT, 0.2 mg/ml BSA, 0.01% CHAPS, 50 µM Calcium, and 5 µM TPEN
Final concentrations: 5 nM hPAD4 enzyme, 250 µM BAEE, and 0.5% DMSO
Total incubation time: 30 mins compound and enzyme preincubation at 37 °C, 90 min enzyme/substrate reaction, 30 min reaction with phenyl glyoxal at 37 °C
Stop solution: 40 µl 5% TCA in ACN

0.13 µL of compound solution was added to 13 µL of 10 nM PAD4 in assay buffer. After 30 min 13 µl of 500 µM of BAEE was added in 25 mM hepes, pH 7.5, 5 mM NaCl, 1 mM DTT, 0.2 mg/ml BSA, 0.01% CHAPS, 50 µM Calcium, 5 µM TPEN was added and the reaction incubated for 90 min at 37 °C. The enzymatic reaction was quenched by addition of 15 µl of 6.1N TCA, 100% Final Concentration is 20%, 35 µl of 8.5 mM phenyl glyoxal (final concentration 4 mM) is then added and the reaction is incubated for 30 min at 37°C.

After 30 minutes the plates are spun down to remove all precipitate. The enzyme reaction was quenched with an equal volume of methanol containing internal standard (modified citrulline). Samples were loaded onto the Rapid Fire RF300 system (Agilent) wherein they were first sipped for 1000 ms and then directly loaded to a C18 separations cartridge using a mixture of acetonitrile containing 0.01% formic acid for 3000 ms desalting. The flow rate of the mobile phase was 1.5 ml/min. Once the samples were eluted from the cartridge, a mobile phase of acetonitrile containing 0.01% formic acid was used to move the samples into the mass spectrometer for 4000 ms at a flow rate of 1.25 ml/min/ Sciex API5500 triple quadrupole mass spectrometer (Applied Biosystems) equipped with ESI was used to analyze the peptidyl citrulline and internal standard ions.

MRM transition of product and internal standard were monitored at m/z 424.5 to 350.4 and m/z 293 to 247 respectively. The dwell time for each transition was set at 200 ms, and the ESI voltage was used at 5500 with a source temperature of 400°C. Extracted ion peaks for each transition were integrated using the Rapid Fire Integrator software. Peak area of analyte was normalized with internal standard.).

For a given compound example, Table 59 below shows the human PAD4 (hPAD4) IC₅₀ in the rapid-fire mass spectrum (RFMS) assay.

**Table 59. PAD4 Activity**

| Example | hPAD4 RFMS IC₅₀ (µM) |
|---|---|
| 1 | 0.280 |
| 2 | 0.380 |
| 3 | 0.060 |
| 4 | 0.068 |
| 5 | 0.027 |
| 6 | 0.032 |
| 7 | 0.412 |
| 8 | 0.037 |
| 9 | 0.120 |
| 10 | 0.159 |
| 11 | 1.373 |
| 12 | 2.324 |
| 13 | 0.163 |
| 14 | 0.906 |
| 15 | 0.526 |
| 16 | 0.407 |
| 17 | 0.075 |
| 18 | 0.570 |
| 19 | 0.168 |
| 20 | 0.116 |
| 21 | 0.091 |
| 22 | 2.155 |
| 23 | 0.108 |
| 24 | 0.031 |
| 25 | 0.017 |
| 26 | 0.449 |
| 27 | 0.022 |
| 28 | NA |
| 29 | 1.020 |
| 30 | 0.084 |
| 31 | 0.065 |
| 32 | 0.414 |
| 33 | 0.534 |
| 34 | 0.042 |
| 35 | 0.139 |
| 36 | 0.782 |
| 37 | 3.614 |
| 38 | 0.158 |
| 39 | 0.087 |
| 40 | 0.025 |
| 41 | 0.085 |
| 42 | 0.009 |
| 43 | 0.150 |
| 44 | 1.485 |
| 45 | 0.101 |
| 46 | 0.041 |
| 47 | 1.482 |
| 48 | 0.326 |
| 49 | 0.036 |
| 50 | 0.074 |
| 51 | 0.109 |
| 52 | 0.882 |
| 53 | 0.446 |
| 54 | 0.358 |
| 55 | 0.543 |
| 56 | 0.468 |
| 57 | 0.781 |
| 58 | 0.083 |
| 59 | 0.291 |
| 60 | 0.083 |
| 61 | NA |
| 62 | 0.028 |
| 63 | 0.227 |
| 64 | 0.055 |
| 65 | 0.025 |
| 66 | 0.059 |
| 67 | 0.023 |
| 68 | 0.026 |
| 69 | 0.019 |
| 70 | 0.015 |
| 71 | 0.022 |
| 72 | 0.067 |
| 73 | 0.605 |
| 74 | 0.425 |
| 75 | 0.166 |
| 76 | 0.096 |
| 77 | 0.077 |
| 78 | 0.143 |
| 79 | 0.212 |
| 80 | 1.609 |
| 81 | 0.384 |
| 82 | 0.368 |
| 83 | 0.384 |
| 84 | 0.610 |
| 85 | 0.327 |
| 86 | 0.219 |
| 87 | 2.316 |
| 88 | 1.678 |
| 89 | 0.048 |
| 90 | 0.012 |
| 91 | 0.602 |
| 92 | 0.672 |
| 93 | 0.220 |
| 94 | 0.934 |
| 95 | 0.133 |
| 96 | 0.588 |
| 97 | 0.030 |
| 98 | 0.014 |
| 99 | 0.307 |
| 100 | 0.036 |
| 101 | 0.011 |
| 102 | 0.012 |
| 103 | 0.034 |
| 104 | 0.025 |
| 105 | 0.107 |
| 106 | 0.208 |
| 107 | 0.240 |
| 108 | 0.907 |
| 109 | 0.292 |
| 110 | 1.037 |
| 111 | 15.000 |
| 112 | 0.572 |
| 113 | 0.283 |
| 114 | 3.975 |
| 115 | 3.371 |
| 116 | 22.841 |
| 117 | 15.000 |
| 205 | 0.06 |
| 206 | 0.07 |
| 207 | 0.15 |
| 208 | 0.14 |
| 209 | 1.08 |
| 210 | 0.49 |
| 211 | 1.05 |
| 212 | 1.69 |
| 213 | 0.68 |
| 214 | 0.15 |
| 215 | 0.61 |
| 216 | 0.27 |
| 217 | 0.26 |
| 218 | 0.55 |
| 219 | 0.46 |
| 220 | 4.34 |
| 221 | 0.51 |
| 222 | 1.01 |
| 223 | 0.13 |
| 224 | 1.07 |
| 225 | 0.08 |
| 226 | 0.05 |
| 227 | 0.95 |
| 228 | 0.93 |
| 229 | 0.13 |
| 230 | 0.32 |
| 231 | 0.46 |
| 232 | 0.90 |
| 233 | 0.62 |
| 234 | 1.14 |
| 235 | 0.25 |
| 236 | 0.95 |
| 237 | 0.25 |
| 238 | 0.42 |
| 239 | 0.07 |
| 240 | 0.18 |
| 241 | 0.02 |
| 242 | 0.09 |
| 243 | 4.12 |
| 244 | 0.07 |
| 245 | 0.07 |
| 246 | 0.13 |
| 247 | 0.12 |
| 248 | 0.06 |
| 249 | 0.08 |
| 250 | 0.09 |
| 251 | 0.18 |
| 252 | 0.79 |
| 253 | 0.29 |
| 254 | 0.32 |
| 255 | 0.25 |
| 256 | 0.33 |
| 257 | 0.06 |
| 258 | 0.22 |
| 259 | 0.02 |
| 260 | 0.22 |
| 261 | 0.42 |
| 262 | 0.12 |
| 263 | 0.09 |
| 264 | 0.01 |
| 265 | 0.11 |
| 266 | 0.28 |
| 267 | 0.05 |
| 268 | 0.04 |
| 269 | 0.54 |
| 270 | 2.11 |
| 271 | 2.07 |
| 272 | 3.81 |
| 273 | 2.42 |
| 274 | 0.27 |
| 275 | 0.12 |
| 276 | 2.77 |
| 277 | 0.22 |
| 278 | 0.39 |
| 279 | 0.20 |
| 280 | 1.09 |
| 281 | 0.14 |
| 282 | 0.17 |
| 283 | 0.33 |
| 284 | 1.86 |
| 285 | 1.27 |
| 286 | 0.54 |
| 287 | 1.28 |
| 288 | 0.08 |
| 289 | 3.58 |
| 290 | 2.72 |
| 291 | 5.89 |
| 292 | 2.35 |
| 293 | 0.08 |
| 294 | 0.20 |
| 295 | 0.02 |
| 296 | 0.85 |
| 297 | 0.19 |
| 298 | 0.21 |
| 299 | 0.02 |
| 300 | 0.10 |
| 301 | 0.03 |
| 302 | 0.12 |
| 303 | 0.24 |
| 304 | 0.01 |
| 305 | 0.04 |
| 306 | 0.03 |
| 307 | 0.07 |
| 308 | 0.05 |
| 309 | 0.40 |
| 310 | 0.40 |
| 311 | 0.21 |
| 312 | 0.06 |
| 313 | 0.10 |
| 314 | 0.04 |
| 315 | 0.34 |
| 316 | 0.25 |
| 317 | 0.51 |
| 318 | 0.31 |
| 319 | 0.05 |
| 320 | 0.15 |
| 321 | 15.00 |
| 322 | 0.50 |
| 323 | 0.14 |
| 324 | 0.11 |
| 325 | 1.54 |
| 326 | 0.20 |
| 327 | 0.07 |
| 328 | 0.12 |
| 329 | 0.05 |
| 330 | 0.15 |
| 331 | 0.06 |
| 332 | 0.02 |
| 333 | 0.02 |
| 334 | 0.12 |
| 335 | 0.06 |
| 336 | 0.15 |
| 337 | 0.02 |
| 338 | 0.05 |
| 339 | 0.03 |
| 340 | 0.08 |
| 341 | 0.04 |
| 342 | 0.93 |
| 343 | 0.05 |
| 344 | 0.47 |
| 345 | 0.79 |
| 346 | 0.06 |
| 347 | 0.29 |
| 348 | 0.14 |
| 349 | 0.10 |
| 350 | 0.03 |
| 351 | 0.10 |
| 352 | 0.81 |
| 353 | 0.61 |
| 354 | 0.15 |
| 355 | 0.02 |
| 356 | 2.78 |
| 357 | 0.05 |
| 358 | 0.42 |
| 359 | 0.24 |
| 360 | 0.45 |
| 361 | 0.30 |
| 362 | 0.43 |
| 363 | 2.28 |
| 364 | 1.09 |
| 365 | 0.72 |
| 366 | 0.87 |
| 367 | 1.00 |
| 368 | 2.80 |
| 369 | 0.02 |
| 370 | 0.13 |
| 371 | 1.07 |
| 372 | 0.19 |
| 373 | 0.20 |
| 374 | 2.84 |
| 375 | 1.36 |
| 376 | 0.12 |
| 377 | 0.09 |
| 378 | 0.45 |
| 379 | 0.16 |
| 380 | 0.14 |
| 381 | 0.02 |
| 382 | 0.15 |
| 383 | 0.04 |
| 384 | 0.20 |
| 385 | 0.27 |
| 386 | 0.08 |
| 387 | 0.23 |
| 388 | 0.07 |
| 389 | 0.03 |
| 390 | 0.005 |
| 391 | 0.01 |
| 392 | 0.003 |
| 393 | 0.25 |
| 394 | 0.01 |
| 395 | 0.14 |
| 396 | 0.11 |
| 397 | 0.04 |
| 398 | 0.04 |
| 399 | 0.05 |
| 400 | 0.66 |
| 401 | 0.02 |
| 402 | 0.06 |
| 403 | 0.46 |
| 404 | 0.03 |
| 405 | 0.02 |
| 406 | 0.09 |
| 407 | 0.03 |
| 408 | 0.04 |
| 409 | 0.04 |
| 410 | 0.09 |
| 411 | 0.11 |
| 412 | 0.01 |
| 413 | 0.03 |
| 414 | 0.26 |
| 415 | 0.46 |
| 416 | 0.56 |
| 417 | 5.06 |
| 418 | 1.71 |
| 419 | 8.25 |
| 420 | 0.29 |
| 421 | 0.08 |
| 422 | 0.40 |
| 423 | 0.15 |
| 424 | 0.08 |
| 425 | 0.05 |
| 426 | 0.05 |
| 427 | 0.02 |
| 428 | 0.03 |
| 429 | 0.04 |
| 430 | 0.08 |
| 431 | 0.13 |
| 432 | 0.20 |
| 433 | 0.14 |
| 434 | 0.25 |
| 435 | 0.38 |
| 436 | 0.30 |
| 437 | 0.11 |
| 438 | 0.03 |
| 439 | 0.03 |
| 440 | 0.06 |
| 441 | 0.06 |
| 442 | 0.03 |
| 443 | 0.13 |
| 444 | 0.01 |
| 445 | 0.16 |
| 446 | 0.13 |
| 447 | 0.17 |
| 448 | 0.07 |
| 449 | 0.05 |
| 450 | 0.05 |
| 451 | 0.01 |
| 452 | 0.03 |
| 453 | 0.01 |
| 454 | 0.55 |
| 455 | 0.27 |
| 456 | 0.03 |
| 457 | 0.03 |
| 458 | 0.08 |
| 459 | 0.14 |
| 460 | 0.07 |
| 461 | 0.08 |
| 462 | 0.03 |
| 463 | 0.10 |
| 464 | 0.18 |
| 465 | 0.13 |
| 466 | 0.19 |
| 467 | 0.02 |
| 468 | 0.10 |
| 469 | 0.18 |
| 470 | 0.01 |
| 471 | 0.09 |
| 472 | 0.06 |
| 473 | 0.003 |
| 474 | 0.07 |
| 475 | 0.02 |
| 476 | 0.03 |
| 477 | 0.02 |
| 478 | 0.13 |
| 479 | 0.39 |
| 480 | 0.14 |
| 481 | 0.12 |
| 482 | 0.06 |
| 483 | 0.15 |
| 484 | 0.15 |
| 485 | 0.15 |
| 486 | 0.07 |
| 487 | 0.20 |
| 488 | 0.25 |
| 489 | 0.07 |
| 490 | 0.15 |
| 491 | 1.31 |
| 492 | 0.13 |
| 493 | 0.11 |
| 494 | 0.02 |
| 495 | 0.33 |
| 496 | 0.02 |
| 497 | 0.04 |
| 498 | 0.07 |
| 499 | 0.15 |
| 500 | 0.24 |
| 501 | 0.13 |
| 502 | 0.07 |
| 503 | 0.06 |
| 504 | 0.11 |
| 505 | 0.06 |
| 506 | 0.07 |
| 507 | 0.17 |
| 508 | 0.04 |
| 509 | 0.35 |
| 511 | 0.01 |
| 514 | 0.06 |
| 515 | 0.06 |
| 516 | 1.04 |
| 517 | 0.06 |
| 518 | 0.26 |
| 519 | 0.04 |
| 520 | 0.22 |
| 521 | 6.05 |
| 522 | 15.00 |
| 523 | 0.40 |
| 524 | 0.28 |
| 525 | 0.09 |
| 526 | 0.26 |
| 527 | 0.05 |
| 528 | 15.00 |
| 529 | 1.53 |
| 530 | 0.03 |
| 531 | 0.58 |
| 532 | 1.01 |
| 533 | 0.52 |
| 534 | 0.44 |
| 535 | 0.07 |
| 536 | 2.52 |
| 537 | 0.07 |
| 538 | 0.04 |
| 539 | 0.13 |
| 540 | 1.10 |
| 541 | 2.53 |
| 542 | 0.58 |
| 543 | 0.63 |
| 544 | 1.07 |
| 545 | 0.26 |
| 546 | 0.56 |
| 547 | 0.49 |
| 548 | 0.17 |
| 549 | 0.01 |
| 550 | 0.05 |
| 551 | 0.16 |
| 552 | 0.10 |
| 553 | 0.09 |
| 554 | 0.06 |
| 555 | 0.03 |
| 556 | 0.02 |
| 557 | 0.01 |
| 558 | 0.10 |
| 559 | 0.08 |
| 560 | 0.16 |
| 561 | 0.64 |
| 562 | 0.14 |
| 563 | 0.07 |
| 564 | 0.04 |
| 565 | 0.02 |
| 566 | 0.01 |
| 567 | 0.02 |
| 568 | 0.06 |
| 569 | 0.02 |
| 570 | 0.18 |
| 571 | 0.03 |
| 572 | 0.08 |
| 573 | 0.11 |
| 574 | 0.02 |
| 575 | 0.06 |
| 576 | 0.19 |
| 577 | 0.15 |
| 578 | 0.19 |
| 579 | 0.24 |
| 580 | 0.06 |
| 581 | 0.03 |
| 582 | 0.30 |
| 583 | 1.61 |
| 584 | 0.05 |
| 585 | 0.07 |
| 586 | 0.15 |
| 587 | 1.12 |
| 588 | 0.23 |
| 589 | 0.06 |
| 590 | 0.03 |
| 591 | 0.03 |
| 592 | 0.03 |
| 593 | 0.11 |
| 594 | 0.39 |
| 595 | 1.28 |
| 596 | 7.39 |
| 597 | 0.01 |
| 598 | 0.02 |
| 599 | 0.02 |
| 600 | 0.03 |
| 601 | 0.03 |
| 602 | 0.03 |
| 603 | 0.10 |
| 604 | 0.03 |
| 605 | 0.07 |
| 606 | 0.05 |
| 607 | 0.05 |
| 608 | 0.01 |
| 609 | 0.04 |
| 610 | 0.43 |
| 611 | 1.56 |
| 612 | 2.96 |
| 613 | 0.25 |
| 614 | 0.06 |
| 615 | 0.07 |
| 616 | 0.02 |
| 617 | 0.01 |
| 618 | 0.05 |
| 619 | 0.04 |
| 620 | 0.45 |
| 621 | 0.14 |

### RFMS human PAD4 Functional Assay 2:

### Compound preparation:

Stock compounds were dissolved and stored in 100% DMSO. Compound solutions were prepared via serial dilution at 3-fold intervals in DMSO with top compound concentration at 20 µM in each assay. 0.25 µl of compound solution was transferred from the compound plate to the assay plate by using an acoustic dispenser.

### Final assay conditions:

Total reaction volume: 25 µl
Assay buffer: 100 HEPES pH 7.4, 200 mM NaCl, 2 mM CaCl₂, 5 mM DTT
35 nM recombinant human PAD4
500 µM TSTGGRQGSHH peptide
1.2% DMSO
Stop solution: 10% formic acid

Reaction mixtures were incubated at room temperature for 30 minutes. 10 µl each of the reaction mixtures was then mixed with 40 µl of 10% formic acid in a microtiter plate. The plate was frozen at -80°C before shipping out on dry ice for RapidFire mass spectroscopy analysis.
Thawed samples were loaded onto the Rapid Fire 300 system (Agilent) wherein they were first sipped for 250 ms and then loaded onto an Agilent "C" (C18) cartridge using a mobile phase of water containing 0.09% formic acid/0.01% trifluoroacetic acid for 3000 ms desalting flowing at a rate of 1.5 ml/min. Once the samples were loaded and washed, a mobile phase of acetonitrile containing 0.09% formic acid/0.01% trifluoroacetic acid was used to elute the samples directly onto a Sciex API4000 triple quadrupole mass spectrometer for 3000 ms at a flow rate of 1.25ml/min.

MRM transitions for substrate and product were monitored in positive ESI mode at m/z=562.3/969.7 and m/z=562.8/541.3 respectively. The dwell time for each transition was set at 100 ms, and the ESI voltage was used at 5500 with a source temperature of 650°C. Extracted ion peaks for each transition were integrated using the Rapid Fire Integrator software.

## Claims

1. A compound of Formula (Ia): 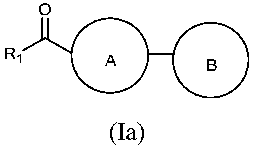 or a pharmaceutically acceptable salt thereof, wherein: 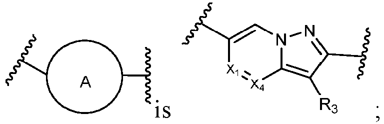 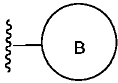 is selected from 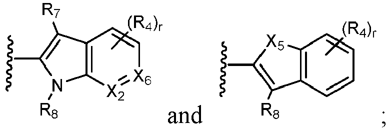
X₁ is independently selected from CR₂, and N;
X₂ is independently selected from CR₄, and N;
X₄ is independently selected from CR₂, and N; provided X₁ and X₄ are not both N;
X₅ is independently selected from O and S;
X₆ is independently selected from CR₄, and N; provided 1) X₂ and X₆ are not both N; 2) when X₂ and X₆ are both CR₄, one of R₄ is H;
R₁ is independently selected from 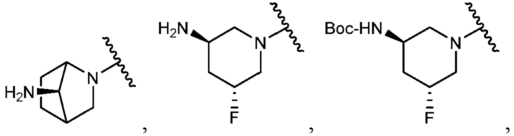 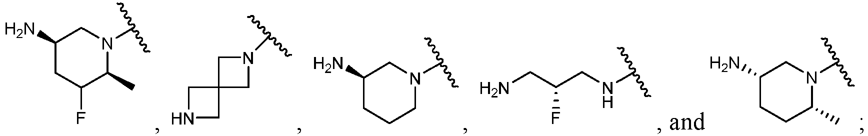
R₂ is independently selected from H, F, Cl, C₁₋₄ alkyl optionally substituted with one or more substituents selected from F, Cl, and OH, and -OC₁₋₄ alkyl;
R₃ is independently selected from H, F, Cl, CN, -C(=O)OR_{b}, and C₁₋₃ alkyl optionally substituted with one or more substituents selected from F, Cl, OH, NH₂, and N₃;
R₄ is independently selected from H, F, Cl, C₁₋₅ alkyl optionally substituted with one or more substituents selected from F, Cl, and OH, C₃₋₆ cycloalkyl, 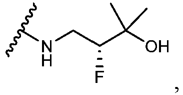 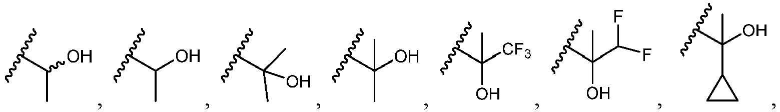 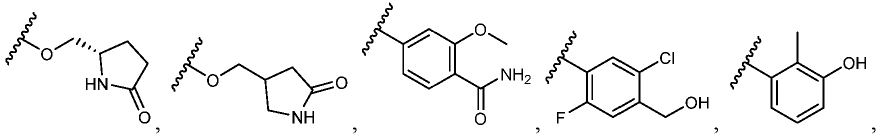 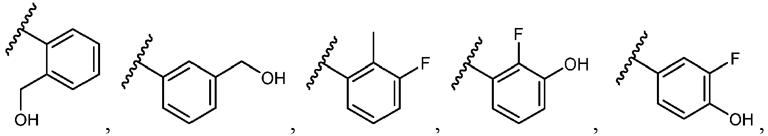 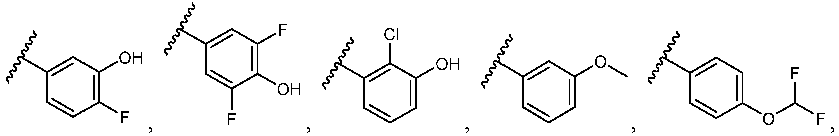 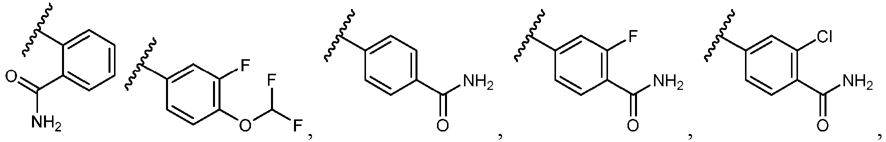 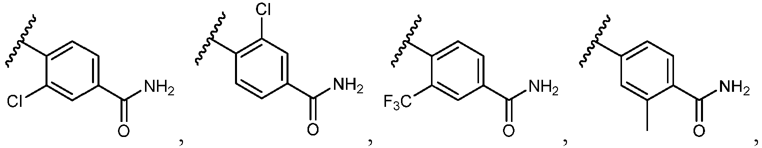 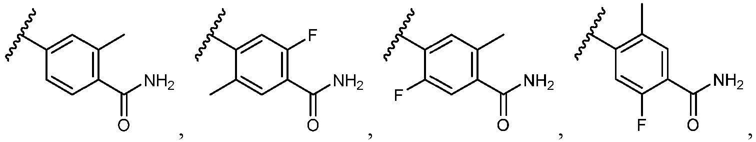 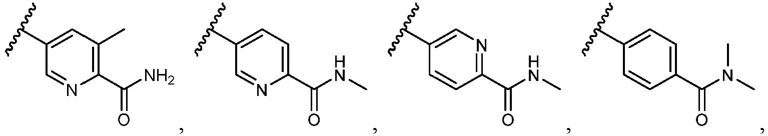 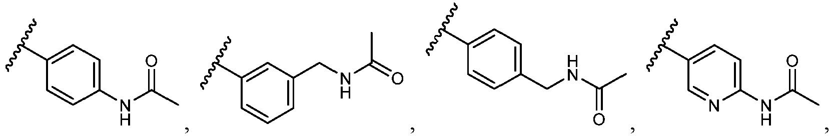 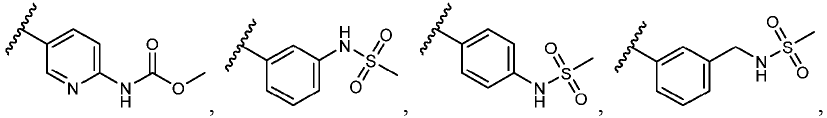 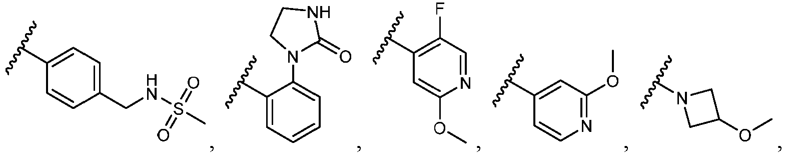 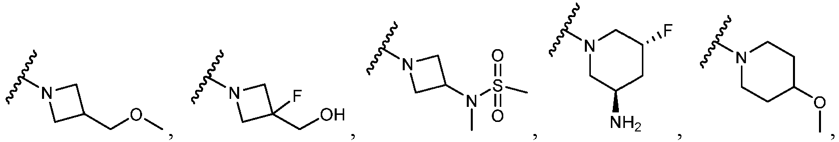 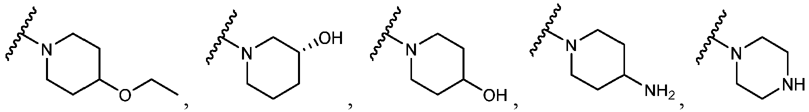 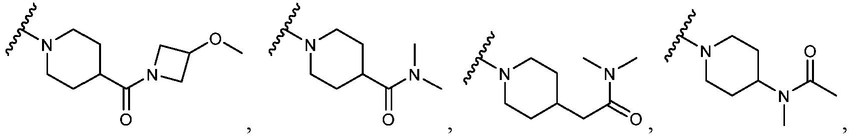 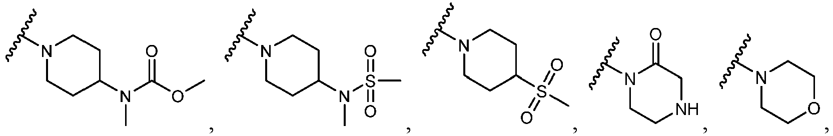 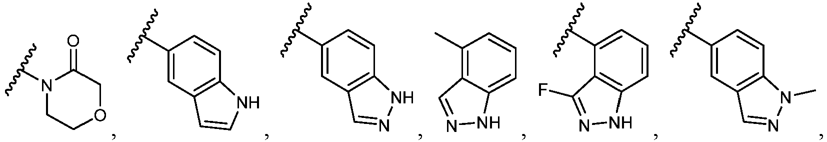 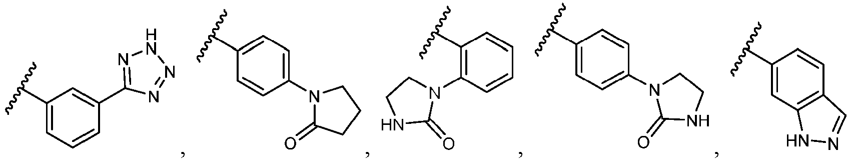 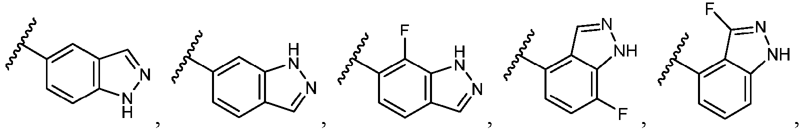 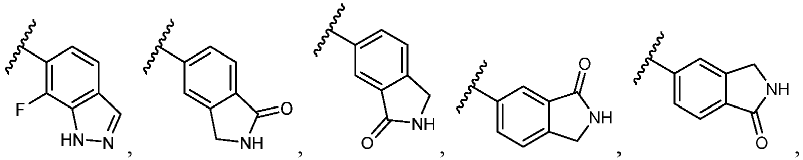 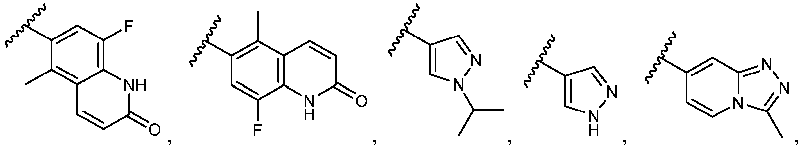 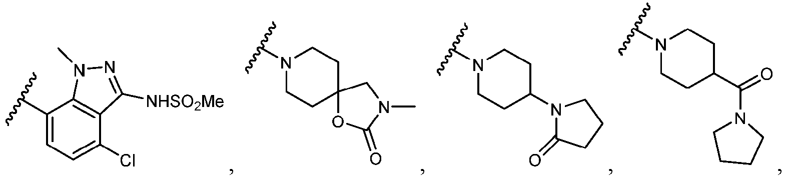 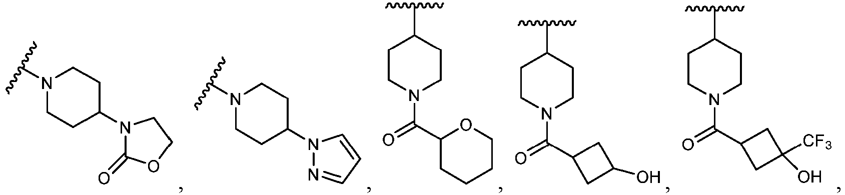 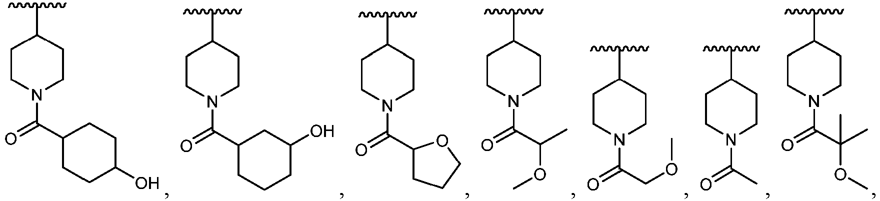 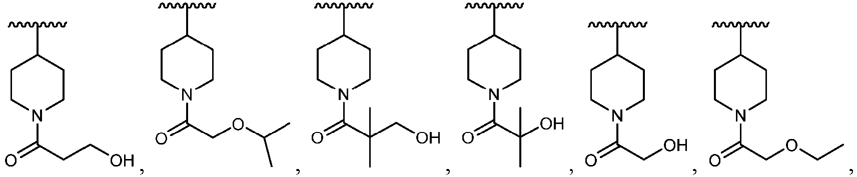 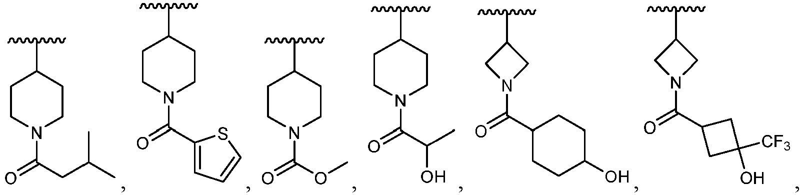 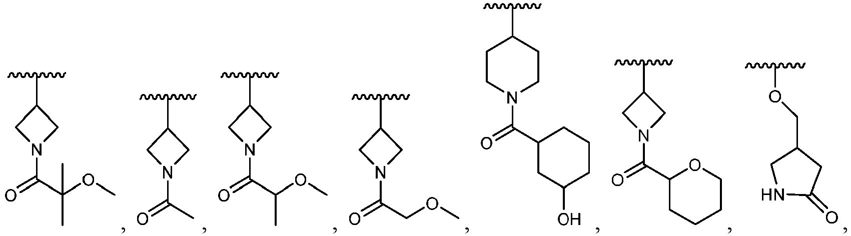 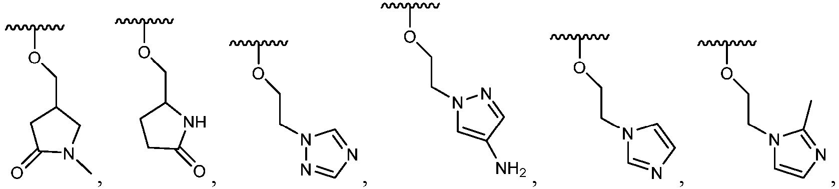 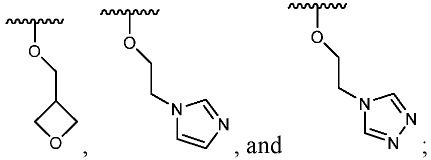
R₇ is independently selected from H, F, and Cl;
R₈ is independently selected from H, and C₁₋₆ alkyl optionally substituted with one or more substituents selected from F, Cl, and C₃₋₆ cycloalkyl;
R_{b} is independently selected from H, C₁₋₆ alkyl optionally substituted with one or more Rₑ, C₂₋₆ alkenyl optionally substituted with one or more Rₑ, C₂₋₆ alkynyl optionally substituted with one or more Rₑ, -(CH₂)ᵣ-C₃₋₁₀ carbocyclyl optionally substituted with one or more Rₑ, and -(CH₂)ᵣ-heterocyclyl optionally substituted with one or more Rₑ;
Rₑ is independently selected from F, Cl, Br, CN, NH₂, -NH-C₁₋₄ alkyl, -N(C₁₋₄ alkyl)₂, =O, OH, -OC₁₋₆ alkyl, -CO₂H, C₁₋₆ alkyl optionally substituted with one or more R_{f}, C₂₋₆ alkenyl, C₂₋₆ alkynyl, -(CH₂)ᵣ-C₃₋₆ cycloalkyl optionally substituted with one or more R_{f}, -(CH₂)ᵣ-aryl optionally substituted with one or more R_{f}, and -(CH₂)ᵣ-heterocyclyl optionally substituted with one or more R_{f},
R_{f} is independently selected from F, Cl, Br, CN, OH, OC₁₋₅ alkyl, C₁₋₅ alkyl optionally substituted with OH, C₂₋₅ alkenyl, C₂₋₅ alkynyl, C₃₋₆ cycloalkyl, and phenyl; and
r, at each occurrence, is independently selected from zero, 1, 2, 3, and 4.

2. The compound according to claim 1, having Formulae (IIa)-(XIII): 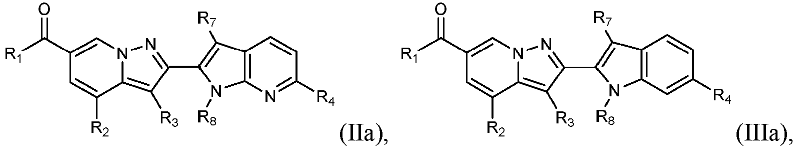 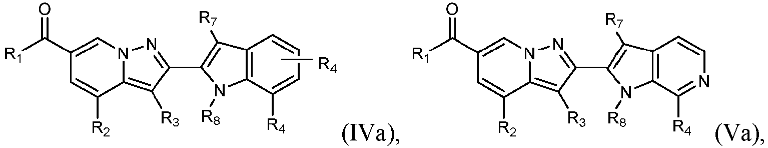 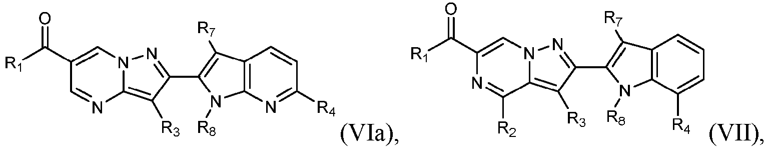 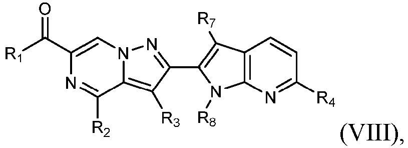 or 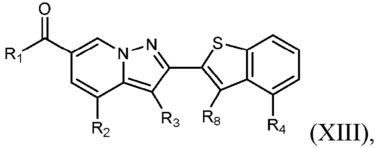 or a pharmaceutically acceptable salt thereof.

3. The compound according to claim 2, having Formula (IIa): 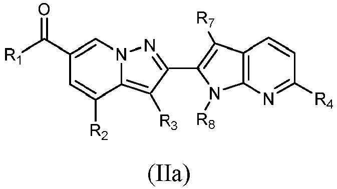 or a pharmaceutically acceptable salt thereof, wherein:
R₁ is independently selected from 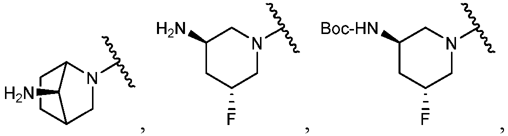 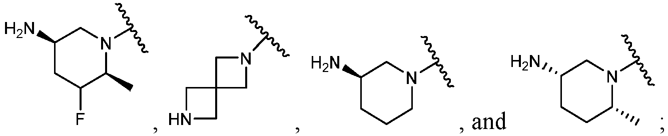
R₂ is independently selected from H, F, Cl, C₁₋₃ alkyl optionally substituted with one or more substituents selected from F, Cl, and OH, and OC₁₋₃ alkyl; and
R₃ is independently selected from H, F, Cl, and C₁₋₃ alkyl optionally substituted with one or more substituents selected from F, Cl, and OH.

4. The compound according to claim 1, having Formula (IIIa): 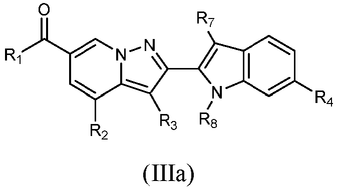 or a pharmaceutically acceptable salt thereof, wherein:
R₁ is independently selected from 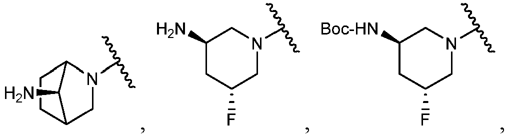 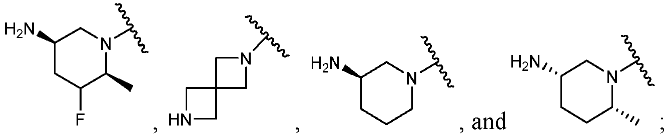
R₂ is independently selected from H, F, Cl, C₁₋₃ alkyl optionally substituted with one or more substituents selected from F, Cl, and OH, and OC₁₋₃ alkyl; and
R₃ is independently selected from H, F, Cl, and C₁₋₃ alkyl optionally substituted with one or more substituents selected from F, Cl, and OH.

5. The compound according to claim 3 or 4, or a pharmaceutically acceptable salt thereof, wherein:
R₁ is independently selected from 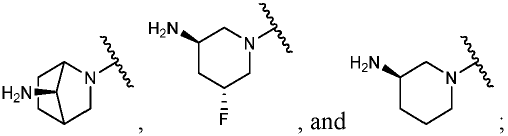
R₂ is independently selected from H, F, Cl, CH₃, and OCH₃;
R₃ is independently selected from H, F, Cl, CH₃, and CH₂OH;
R₇ is H; and
R₈ is C₁₋₃ alkyl substituted with one C₃₋₆ cycloalkyl.

6. The compound according to claim 1, having Formula (IVa): 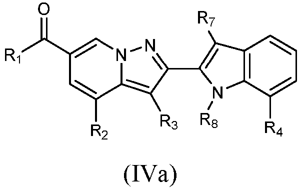 or a pharmaceutically acceptable salt thereof, wherein:
R₁ is independently selected from 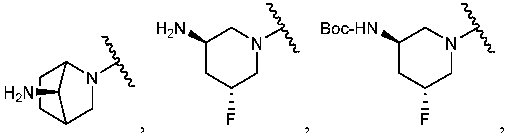 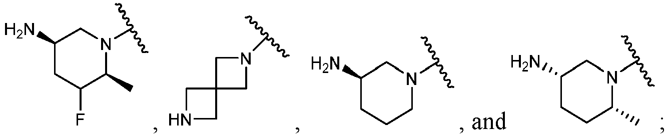
R₂ is independently selected from H, F, Cl, C₁₋₃ alkyl optionally substituted with one or more substituents selected from F, Cl, and OH, and OC₁₋₃ alkyl;
R₃ is independently selected from H, F, Cl, and C₁₋₃ alkyl optionally substituted with one or more substituents selected from F, Cl, and OH;
preferably wherein:
R₁ is independently selected from 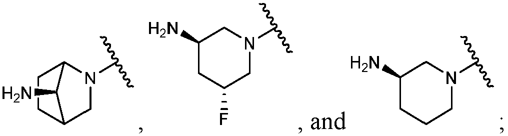
R₂ is independently selected from H, F, Cl, and OCH₃;
R₃ is independently selected from F, Cl, and CH₃;
R₇ is H; and
R₈ is C₁₋₂ alkyl optionally substituted with cyclopropyl;
more preferably wherein:
R₄ is independently selected from

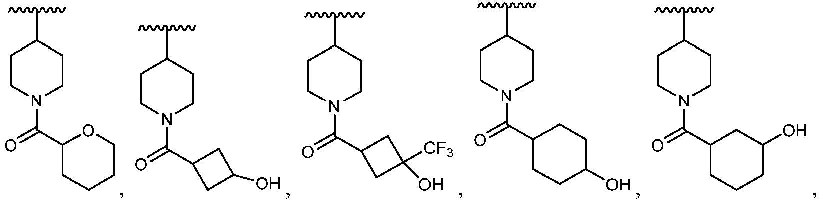

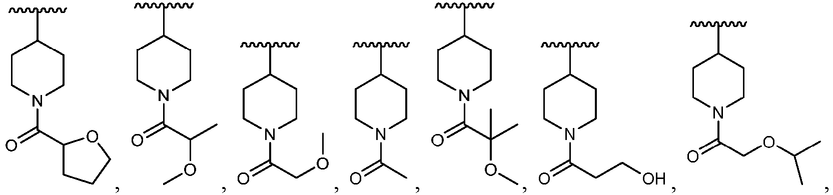

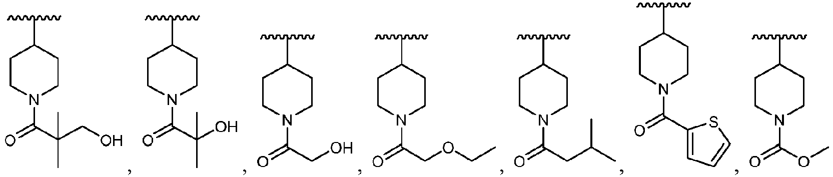

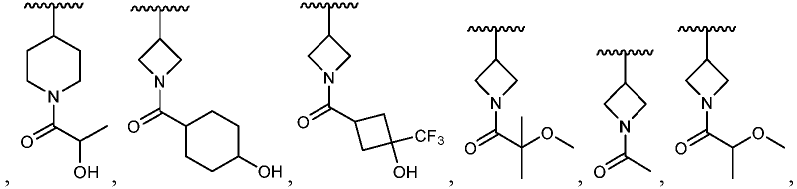

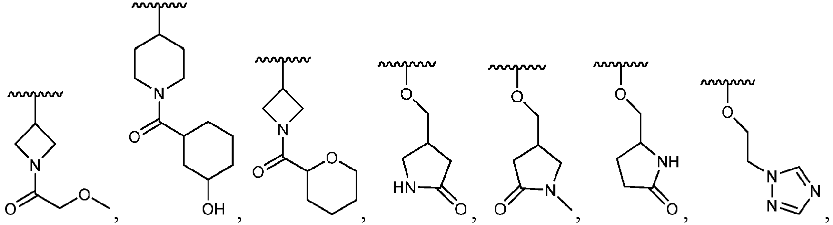

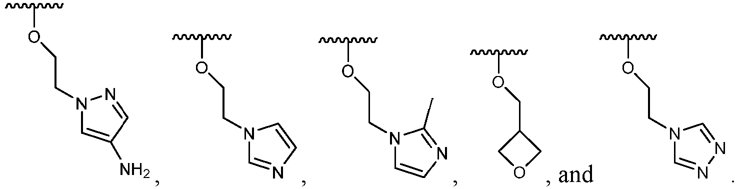

7. The compound according to claim 1, having Formula (Va): 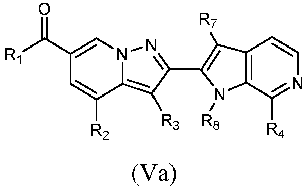 or a pharmaceutically acceptable salt thereof, wherein:
R₁ is independently selected from 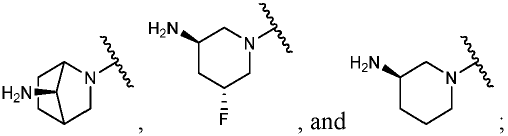
R₂ is independently selected from H, F, Cl, CH₃, and OCH₃;
R₃ is independently selected from H, F, Cl, CN, CH₃, and CH₂OH; preferably wherein:
R₁ is independently selected from 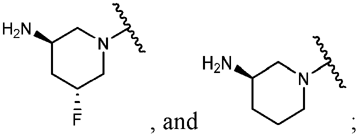
R₂ is CH₃;
R₃ is CH₃;
R₄ is independently selected from 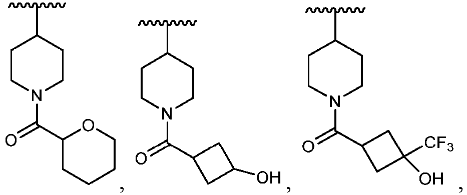 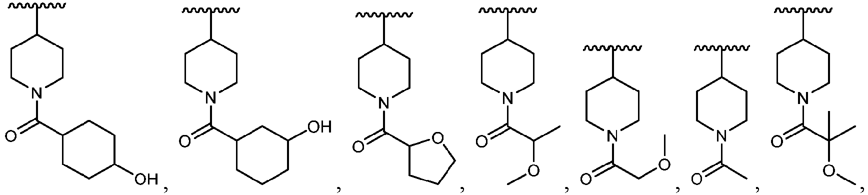 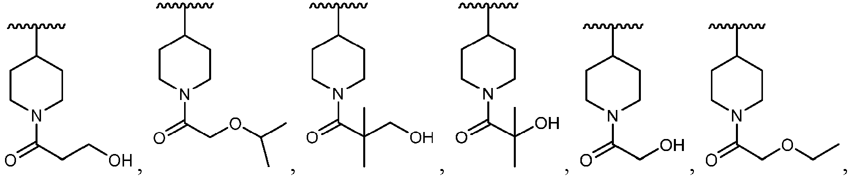 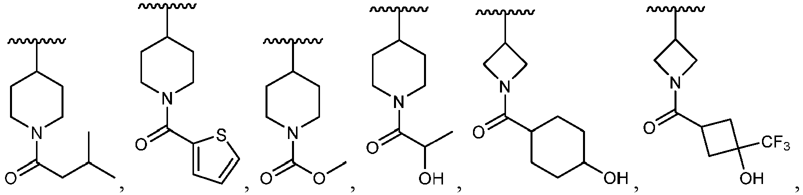 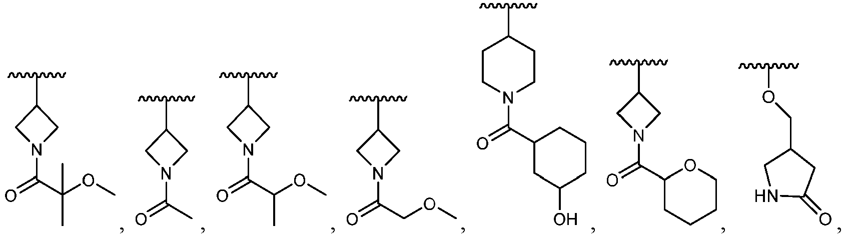 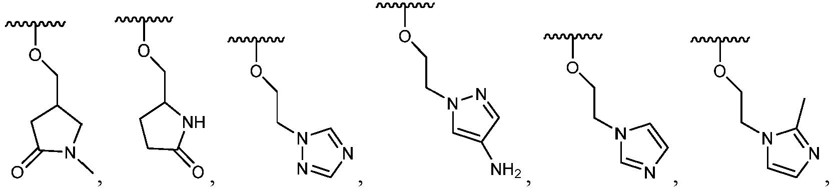 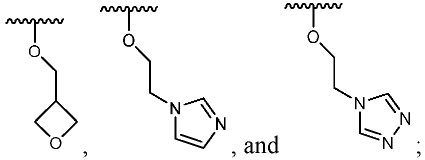
R₇ is H; and
R₈ is C₁₋₂ alkyl optionally substituted with cyclopropyl.

8. The compound according to claim 1, having Formula (VIa): 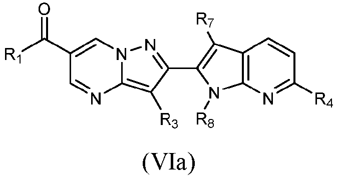 or a pharmaceutically acceptable salt thereof, wherein:
R₁ is independently selected from 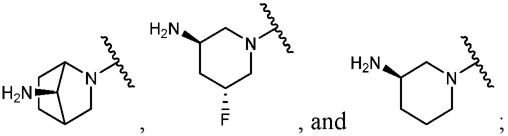 and
R₃ is independently selected from H, F, and CH₃;
preferably wherein:
R₁ is independently selected from 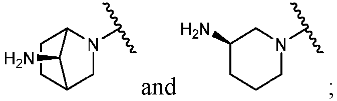
R₃ is CH₃;
R₄ is independently selected from F, Cl, 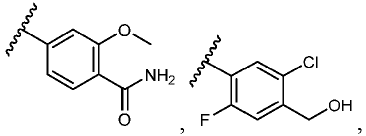 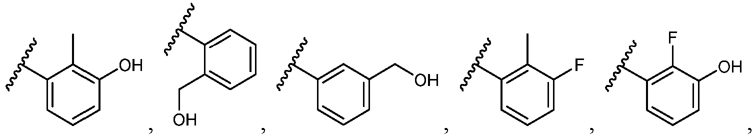 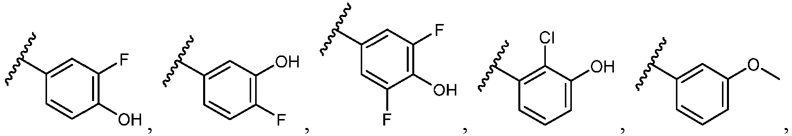 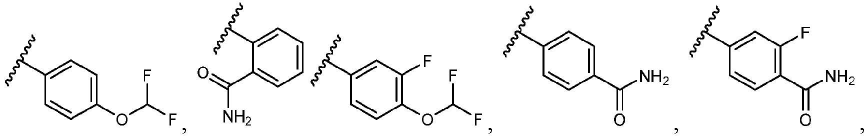 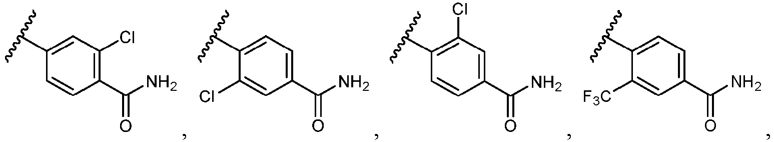 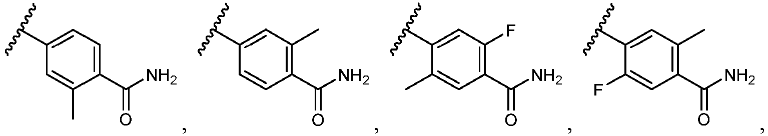 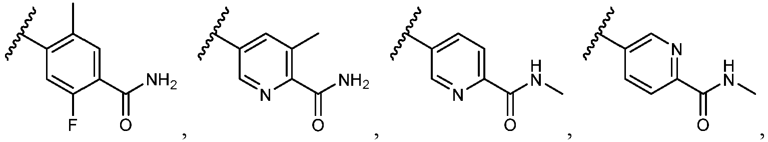 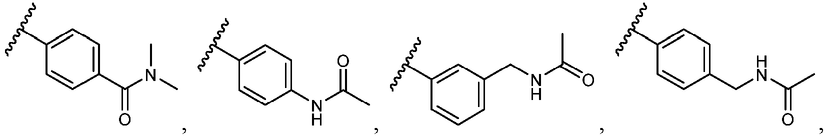 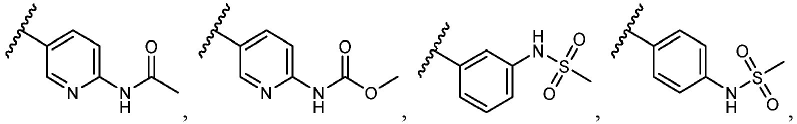 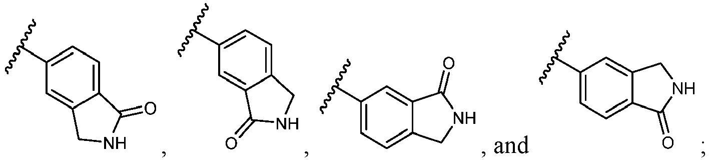
R₇ is H; and
R₈ is C₁₋₂ alkyl optionally substituted with cyclopropyl.

9. The compound according to claim 1, having Formula (VII): 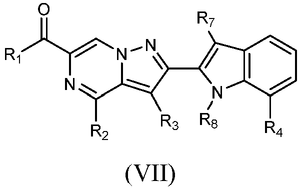 or a pharmaceutically acceptable salt thereof, wherein:
R₁ is independently selected from 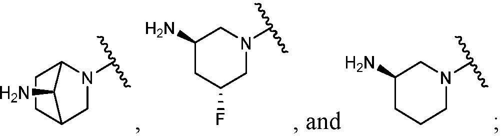
R₂ is independently selected from H, F, Cl, CH₃, and OCH₃; and
R₃ is independently selected from H, F, and CH₃.

10. The compound according to claim 1, having Formula (VIII): 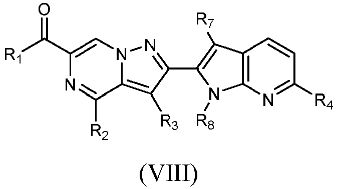 or a pharmaceutically acceptable salt thereof, wherein:
R₁ is independently selected from 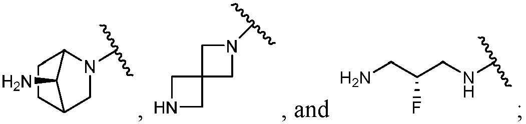
R₂ is independently selected from H, F, and OCH₃; and
R₃ is independently selected from H, F, and CH₃.

11. The compound according to claim 1, having Formula (XIII): 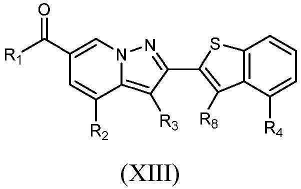 or a pharmaceutically acceptable salt thereof, wherein:
R₁ is independently selected from 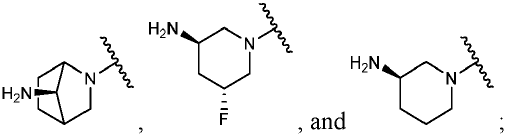
R₂ is independently selected from H, F, CH₃, and OCH₃; and
R₃ is independently selected from H, F, and CH₃.

12. The compound according to claim 1, selected from:
5-(2-(6-((7R)-7-Amino-2-azabicyclo[2.2.1]- heptane-2-carbonyl)-4-methoxy-3-methylpyrazolo[1,5-a]pyridin-2-yl)-1-(cyclopropylmethyl)-1H-pyrrolo[2,3-b]pyridin-6-yl)isoindolin-1-one;
((7R)-7-Amino-2-azabicyclo[2.2.1]heptan-2-yl)(2-(6-cyclopropyl-1-(cyclopropylmethyl)-1H-pyrrolo[2,3-b]pyridin-2-yl)-4-methoxy-3-methylpyrazolo[1,5-a]pyridin-6-yl)methanone;
5-(2-(6-((7R)-7-Amino-2-azabicyclo[2.2.1]heptane-2-carbonyl)-4-methoxy-3-methylpyrazolo[1,5-a]pyridin-2-yl)-1-(cyclopropylmethyl)-1H-indol-6-yl)isoindolin-1-one;
((7R)-7-Amino-2-azabicyclo[2.2.1]heptan-2-yl)(2-(1-(cyclopropylmethyl)-6-(3-methyl-[1,2,4]triazolo[4,3-a]pyridin-7-yl)-1H-indol-2-yl)-4-methoxy-3-methylpyrazolo[1,5-a]pyridin-6-yl)methanone;
((7R)-7-Amino-2-azabicyclo[2.2.1]heptan-2-yl)(2-(1-(cyclopropylmethyl)-6-methyl-1H-indol-2-yl)-4-methoxy-3-methylpyrazolo[1,5-a]pyridin-6-yl)methanone;
3((7R)-7-Amino-2-azabicyclo[2.2.1]heptan-2-yl)(2-(1-(cyclopropylmethyl)-6-methyl-1H-pyrrolo[2,3-b]pyridin-2-yl)-4-fluoro-3-methylpyrazolo[1,5-a]pyridin-6-yl)methanone;
5-(2-(6-((7R)-7-Amino-2-azabicyclo[2.2.1]heptane-2-carbonyl)-4-fluoro-3-ethylpyrazolo[1,5-a]pyridin-2-yl)-1-(cyclopropylmethyl)-1H-pyrrolo[2,3-b]pyridin-6-yl)isoindolin-1-one;
6-(2-(6-((7R)-7-Amino-2-azabicyclo[2.2.1]heptane-2-carbonyl)-4-fluoro-3-methylpyrazolo[1,5-a]pyridin-2-yl)-1-(cyclopropylmethyl)-1H-pyrrolo[2,3-b]pyridin-6-yl)isoindolin-1-one;
((7R)-7-Amino-2-azabicyclo[2.2.1]heptan-2-yl)(2-(1-(cyclopropylmethyl)-6-(2-fluoro-3-hydroxyphenyl)-1H-pyrrolo[2,3-b]pyridin-2-yl)-4-fluoro-3-methylpyrazolo[1,5-a]pyridin-6-yl)methanone;
((7R)-7-Amino-2-azabicyclo[2.2.1]heptan-2-yl)(2-(1-(cyclopropylmethyl)-6-(3-methyl-[1,2,4]triazolo[4,3-a]pyridin-7-yl)-1H-indol-2-yl)-4-fluoro-3-methylpyrazolo[1,5-a]pyridin-6-yl)methanone;
6-(2-(6-((7R)-7-Amino-2-azabicyclo[2.2.1]heptane-2-carbonyl)-3-methylpyrazolo[1,5-a]pyridin-2-yl)-1-(cyclopropylmethyl)-1H-pyrrolo[2,3-b]pyridin-6-yl)isoindolin-1-one;
((7R)-7-Amino-2-azabicyclo[2.2.1]heptan-2-yl)(2-(1-(cyclopropylmethyl)-6-(3-fluoro-4-hydroxyphenyl)-1H-pyrrolo[2,3-b]pyridin-2-yl)-3-methylpyrazolo[1,5-a]pyridin-6-yl)methanone;
((7R)-7-Amino-2-azabicyclo[2.2.1]heptan-2-yl)(2-(1-(cyclopropylmethyl)-6-(2-fluoro-3-hydroxyphenyl)-1H-pyrrolo[2,3-b]pyridin-2-yl)-3-methylpyrazolo[1,5-a]pyridin-6-yl)methanone;
4-(2-(6-((7R)-7-Amino-2-azabicyclo[2.2.1]heptane-2-carbonyl)-3-methylpyrazolo[1,5-a]pyridin-2-yl)-1-(cyclopropylmethyl)-1H-pyrrolo[2,3-b]pyridin-6-yl)-2-fluorobenzamide;
((7R)-7-Amino-2-azabicyclo[2.2.1]heptan-2-yl)(2-(1-(cyclopropylmethyl)-6-(3-methyl-[1,2,4]triazolo[4,3-a]pyridin-7-yl)-1H-pyrrolo[2,3-b]pyridin-2-yl)-3-methylpyrazolo[1,5-a]pyridin-6-yl)methanone;
((7R)-7-Amino-2-azabicyclo[2.2.1]heptan-2-yl)(2-(6-cyclopropyl-1-(cyclopropylmethyl)-1H-pyrrolo[2,3-b]pyridin-2-yl)-3-methylpyrazolo[1,5-a]pyridin-6-yl)methanone;
6-(2-(6-((7R)-7-Amino-2-azabicyclo[2.2.1]heptane-2-carbonyl)-3-methylpyrazolo[1,5-a]pyridin-2-yl)-1-(cyclopropylmethyl)-1H-indol-6-yl)isoindolin-1-one;
5-(2-(6-((7R)-7-Amino-2-azabicyclo[2.2.1]heptane-2-carbonyl)-3-methylpyrazolo[1,5-a]pyridin-2-yl)-1-(cyclopropylmethyl)-1H-indol-6-yl)isoindolin-1-one;
((7R)-7-Amino-2-azabicyclo[2.2.1]heptan-2-yl)(2-(1-(cyclopropylmethyl)-6-(2-hydroxypropan-2-yl)-1H-indol-2-yl)-4-methoxy-3-methylpyrazolo[1,5-a]pyridin-6-yl)methanone;
((3R,5R)-3-Amino-5-fluoropiperidin-1-yl)(2-(1-(cyclopropylmethyl)-6-(2-hydroxypropan-2-yl)-1H-indol-2-yl)-4-methoxy-3-methylpyrazolo[1,5-a]pyridin-6-yl)methanone;
((7R)-7-Amino-2-azabicyclo[2.2.1]heptan-2-yl)(2-(1-(cyclopropylmethyl)-6-(2-hydroxypropan-2-yl)-1H-indol-2-yl)-3-methylpyrazolo[1,5-a]pyridin-6-yl)methanone;
((7R)-7-Amino-2-azabicyclo[2.2.1]heptan-2-yl)(2-(1-(cyclopropylmethyl)-6-(1,1,1-trifluoro-2-hydroxypropan-2-yl)-1H-indol-2-yl)-3-methylpyrazolo[1,5-a]pyridin-6-yl)methanone;
((7R)-7-Amino-2-azabicyclo[2.2.1]heptan-2-yl)(2-(1-(cyclopropylmethyl)-6-(2-hydroxypropan-2-yl)-1H-pyrrolo[2,3-b]pyridin-2-yl)-3-methylpyrazolo[1,5-a]pyridin-6-yl)methanone;
((7R)-7-Amino-2-azabicyclo[2.2.1]heptan-2-yl)(2-(1-(cyclopropylmethyl)-6-(1,1,1-trifluoro-2-hydroxypropan-2-yl)-1H-pyrrolo[2,3-b]pyridin-2-yl)-3-methylpyrazolo[1,5-a]pyridin-6-yl)methanone;
((7R)-7-Amino-2-azabicyclo[2.2.1]heptan-2-yl)(2-(1-(cyclopropylmethyl)-6-(1-hydroxyethyl)-1H-pyrrolo[2,3-b]pyridin-2-yl)-3-methylpyrazolo[1,5-a]pyridin-6-yl)methanone;
N-[5-(2-{6-[(3R,5R)-3-Amino-5-fluoropiperidine-1-carbonyl]-4-methoxy-3-methylpyrazolo[1,5-a]pyridin-2-yl}-1-(cyclopropylmethyl)-1H-indol-6-yl)pyridin-2-yl]acetamide;
(3R,5R)-1-{2-[1-(Cyclopropylmethyl)-6-(4-methanesulfonylpiperidin-1-yl)-1H-indol-2-yl]-4-methoxy-3-methylpyrazolo[1,5-a]pyridine-6-carbonyl}-5-fluoropiperidin-3-amine;
3-(2-{ 6-[(7R)-7-Amino-2-azabicyclo[2.2.1]heptane-2-carbonyl]-4-fluoro-3-methylpyrazolo[1,5-a]pyridin-2-yl}-1-(cyclopropylmethyl)-1H-pyrrolo[2,3-b]pyridin-6-yl)-2-methylphenol;
5-(2-{ 6-[(7R)-7-Amino-2-azabicyclo[2.2.1]heptane-2-carbonyl]-4-fluoro-3-methylpyrazolo[1,5-a]pyridin-2-yl}-1-(cyclopropylmethyl)-1H-pyrrolo[2,3-b]pyridin-6-yl)-N-methylpyridine-2-carboxamide;
4-(2-{ 6-[(7R)-7-Amino-2-azabicyclo[2.2.1]heptane-2-carbonyl]-4-fluoro-3-methylpyrazolo[1,5-a]pyridin-2-yl}-1-(cyclopropylmethyl)-1H-pyrrolo[2,3-b]pyridin-6-yl)morpholin-3-one;
(7R)-2-{2-[1-(Cyclopropylmethyl)-6-(4-methanesulfonylpiperidin-1-yl)-1H-pyrrolo[2,3-b]pyridin-2-yl]-4-fluoro-3-methylpyrazolo[1,5-a]pyridine-6-carbonyl}-2-azabicyclo[2.2.1]heptan-7-amine;
(7R)-2-{2-[1-(Cyclopropylmethyl)-6-{3-methyl-[1,2,4]triazolo[4,3-a]pyridin-7-yl}-1H-1ndol-2-yl]-4-fluoro-3-methylpyrazolo[1,5-a]pyridine-6-carbonyl}-2-azabicyclo[2.2.1]heptan-7-amine;
(7R)-2-{2-[1-(Cyclopropylmethyl)-6-(1H-indazol-4-yl)-1H-pyrrolo[2,3-b]pyridin-2-yl]-3-methylpyrazolo[1,5-a]pyridine-6-carbonyl}-2-azabicyclo[2.2.1]heptan-7-amine;
3-(2-{6-[(7R)-7-Amino-2-azabicyclo[2.2.1]heptane-2-carbonyl]-3-methylpyrazolo[1,5-a]pyridin-2-yl}-1-(cyclopropylmethyl)-1H-pyrrolo[2,3-b]pyridin-6-yl)-2-methylphenol;
1-[4-(2-{6-[(7R)-7-Amino-2-azabicyclo[2.2.1]heptane-2-carbonyl]-3-methylpyrazolo[1,5-a]pyridin-2-yl}-1-(cyclopropylmethyl)-1H-pyrrolo[2,3-b]pyridin-6-yl)phenyl]pyrrolidin-2-one;
4-(2-{6-[(7R)-7-Amino-2-azabicyclo[2.2.1]heptane-2-carbonyl]-3-methylpyrazolo[1,5-a]pyridin-2-yl}-1-(cyclopropylmethyl)-1H-pyrrolo[2,3-b]pyridin-6-yl)-2-fluoro-5-methylbenzamide;
(7R)-2-{2-[1-(Cyclopropylmethyl)-6-(7-fluoro-1H-indazol-6-yl)-1H-pyrrolo[2,3-b]pyridin-2-yl]-3-methylpyrazolo[1,5-a]pyridine-6-carbonyl}-2-azabicyclo[2.2.1]heptan-7-amine;
Methyl N-[5-(2-{6-[(7R)-7-amino-2-azabicyclo[2.2.1]heptane-2-carbonyl]-3-methylpyrazolo[1,5-a]pyridin-2-yl}-1-(cyclopropylmethyl)-1H-pyrrolo[2,3-b]pyridin-6-yl)pyridin-2-yl]carbamate;
4-(2-{6-[(7R)-7-Amino-2-azabicyclo[2.2.1]heptane-2-carbonyl]-3-methylpyrazolo[1,5-a]pyridin-2-yl}-1-(cyclopropylmethyl)-1H-pyrrolo[2,3-b]pyridin-6-yl)morpholin-3-one;
1-[4-(2-{6-[(7R)-7-Amino-2-azabicyclo[2.2.1]heptane-2-carbonyl]-3-methylpyrazolo[1,5-a]pyridin-2-yl }-1-(cyclopropylmethyl)-1H-indol-6-yl)phenyl]pyrrolidin-2-one;
(7R)-2-{2-[1-(Cyclopropylmethyl)-6-(1H-indazol-6-yl)-1H-indol-2-yl]-3-methylpyrazolo[1,5-a]pyridine-6-carbonyl}-2-azabicyclo[2.2.1]heptan-7-amine;
(7R)-2-{2-[1-(Cyclopropylmethyl)-6-(1H-indazol-5-yl)-1H-indol-2-yl]-3-methylpyrazolo[1,5-a]pyridine-6-carbonyl}-2-azabicyclo[2.2.1]heptan-7-amine;
N-[7-(2-{6-[(7R)-7-Amino-2-azabicyclo[2.2.1]heptane-2-carbonyl]-3-methylpyrazolo[1,5-a]pyridin-2-yl}-1-(cyclopropylmethyl)-1H-indol-6-yl)-4-chloro-1-methyl-1H-indazol-3-yl]methanesulfonamide;
1-[2-(2-{6-[(7R)-7-Amino-2-azabicyclo[2.2.1]heptane-2-carbonyl]-3-methylpyrazolo[1,5-a]pyridin-2-yl }-1-(cyclopropylmethyl)-1H-indol-6-yl)phenyl]imidazolidin-2-one;
4-(2-{6-[(7R)-7-Amino-2-azabicyclo[2.2.1]heptane-2-carbonyl]-3-methylpyrazolo[1,5-a]pyridin-2-yl}-1-(cyclopropylmethyl)-1H-indol-6-yl)-2-chlorobenzamide;
(7R)-2-{2-[1-(Cyclopropylmethyl)-6-(1H-pyrazol-4-yl)-1H-indol-2-yl]-3-methylpyrazolo[1,5-a]pyridine-6-carbonyl}-2-azabicyclo[2.2.1]heptan-7-amine;
(7R)-2-{2-[1-(Cyclopropylmethyl)-6-(morpholin-4-yl)-1H-indol-2-yl]-3-methylpyrazolo[1,5-a]pyridine-6-carbonyl}-2-azabicyclo[2.2.1]heptan-7-amine;
(3R,5R)-1-{2-[1-(Cyclopropylmethyl)-6-[4-(pyrrolidine-1-carbonyl)piperidin-1-yl]-1H-indol-2-yl]-3-methylpyrazolo[1,5-a]pyridine-6-carbonyl}-5-fluoropiperidin-3-amine;
3-[1-(2-{6-[(3R,5R)-3-Amino-5-fluoropiperidine-1-carbonyl]-3-methylpyrazolo[1,5-a]pyridin-2-yl}-1-(cyclopropylmethyl)-1H-indol-6-yl)piperidin-4-yl]-1,3-oxazolidin-2-one;
2-(2-{6-[(7R)-7-Amino-2-azabicyclo[2.2.1]heptane-2-carbonyl]-3-methylpyrazolo[1,5-a]pyridin-2-yl}-1-(cyclopropylmethyl)-1H-pyrrolo[2,3-b]pyridin-6-yl)-1,1-difluoropropan-2-ol;
2-(2-{ 6-[(7R)-7-Amino-2-azabicyclo[2.2.1]heptane-2-carbonyl]-4-fluoro-3-methylpyrazolo[1,5-a]pyridin-2-yl}-1-(cyclopropylmethyl)-1H-pyrrolo[2,3-b]pyridin-6-yl)-1,1,1-trifluoropropan-2-ol;
((3R,5R)-3-Amino-5-fluoropiperidin-1-yl)(2-(1-(cyclopropylmethyl)-7-(1-(tetrahydro-2H-pyran-2-carbonyl)piperidin-4-yl)-1H-indol-2-yl)-4-methoxy-3-methylpyrazolo[1,5-a]pyridin-6-yl)methanone;
((3R,5R)-3-Amino-5-fluoropiperidin-1-yl)(2-(1-(cyclopropylmethyl)-7-(1-(3-hydroxycyclobutane-1-carbonyl)piperidin-4-yl)-1H-indol-2-yl)-4-methoxy-3-methylpyrazolo[1,5-a]pyridin-6-yl)methanone;
((3R,5R)-3-Amino-5-fluoropiperidin-1-yl)(2-(1-(cyclopropylmethyl)-7-(1-((1r,4r)-4-hydroxycyclohexane-1-carbonyl)piperidin-4-yl)-1H-indol-2-yl)-4-methoxy-3-methylpyrazolo[1,5-a]pyridin-6-yl)methanone;
(R)-(3-Aminopiperidin-1-yl)(2-(1-(cyclopropylmethyl)-7-(1-(3-hydroxycyclobutane-1-carbonyl)piperidin-4-yl)-1H-indol-2-yl)-4-methoxy-3-methylpyrazolo[1,5-a]pyridin-6-yl)methanone;
((3R,5R)-3-Amino-5-fluoropiperidin-1-yl)(2-(1-(cyclopropylmethyl)-7-(1-(tetrahydrofuran-2-carbonyl)piperidin-4-yl)-1H-indol-2-yl)-4-methoxy-3-methylpyrazolo[1,5-a]pyridin-6-yl)methanone;
((3R,5R)-3-Amino-5-fluoropiperidin-1-yl)(2-(1-(cyclopropylmethyl)-7-(1-(tetrahydrofuran-2-carbonyl)piperidin-4-yl)-1H-indol-2-yl)-4-methoxy-3-methylpyrazolo[1,5-a]pyridin-6-yl)methanone;
(R)-(3-Aminopiperidin-1-yl)(2-(1-(cyclopropylmethyl)-7-(1-(3-hydroxy-3-(trifluoromethyl)cyclobutane-1-carbonyl)piperidin-4-yl)-1H-indol-2-yl)-4-methoxy-3-methylpyrazolo[1,5-a]pyridin-6-yl)methanone;
((3R,5R)-3-Amino-5-fluoropiperidin-1-yl)(2-(1-(cyclopropylmethyl)-7-(1-(3-hydroxy-3-(trifluoromethyl)cyclobutane-1-carbonyl)piperidin-4-yl)-1H-indol-2-yl)-4-methoxy-3-methylpyrazolo[1,5-a]pyridin-6-yl)methanone;
(R)-1-(4-(2-(6-(3-Aminopiperidine-1-carbonyl)-4-methoxy-3-methylpyrazolo[1,5-a]pyridin-2-yl)-1-(cyclopropylmethyl)-1H-indol-7-yl)piperidin-1-yl)-2-methoxyethan-1-one;
(S)-1-(4-(2-(6-((3R,5R)-3-Amino-5-fluoropiperidine-1-carbonyl)-4-methoxy-3-methylpyrazolo[1,5-a]pyridin-2-yl)-1-(cyclopropylmethyl)-1H-indol-7-yl)piperidin-1-yl)-2-methoxypropan-1-one;
1-(4-(2-(6-((3R,5R)-3-Amino-5-fluoropiperidine-1-carbonyl)-4-methoxy-3-methylpyrazolo[1,5-a]pyridin-2-yl)-1-(cyclopropylmethyl)-1H-indol-7-yl)piperidin-1-yl)-2-methoxyethan-1-one;
((3R,5R)-3-Amino-5-fluoropiperidin-1-yl)(2-(1-(cyclopropylmethyl)-7-(1-((1s,4s)-4-hydroxycyclohexane-1-carbonyl)piperidin-4-yl)-1H-indol-2-yl)-4-methoxy-3-methylpyrazolo[1,5-a]pyridin-6-yl)methanone;
((R)-3-Aminopiperidin-1-yl)(2-(1-(cyclopropylmethyl)-7-(1-((1r,4r)-4-hydroxycyclohexane-1-carbonyl)piperidin-4-yl)-1H-indol-2-yl)-4-methoxy-3-methylpyrazolo[1,5-a]pyridin-6-yl)methanone;
((3R,5R)-3-Amino-5-fluoropiperidin-1-yl)(2-(1-(cyclopropylmethyl)-7-(1-((1r,4r)-4-hydroxycyclohexane-1-carbonyl)piperidin-4-yl)-1H-indol-2-yl)-4-methoxy-3-methylpyrazolo[1,5-a]pyridin-6-yl)methanone;
1-(4-(2-(6-((7R)-7-Amino-2-azabicyclo[2.2.1]heptane-2-carbonyl)-4-methoxy-3-methylpyrazolo[1,5-a]pyridin-2-yl)-1-(cyclopropylmethyl)-1H-indol-7-yl)piperidin-1-yl)ethan-1-one;
(R)-1-(4-(2-(6-(3-Aminopiperidine-1-carbonyl)-4-fluoro-3-methylpyrazolo[1,5-a]pyridin-2-yl)-1-(cyclopropylmethyl)-1H-indol-7-yl)piperidin-1-yl)ethan-1-one;
(R)-1-(4-(2-(6-((3R, SR)-3-Amino-5-fluoropiperidine-1-carbonyl)-4-fluoro-3-methylpyrazolo[1,5-a]pyridin-2-yl)-1-(cyclopropylmethyl)-1H-indol-7-yl)piperidin-1-yl)-2-methoxypropan-1-one;
(R)-1-(4-(2-(6-((R)-3-Aminopiperidine-1-carbonyl)-4-fluoro-3-methylpyrazolo[1,5-a]pyridin-2-yl)-1-(cyclopropylmethyl)-1H-indol-7-yl)piperidin-1-yl)-2-methoxypropan-1-one;
1-(4-(2-(6-((3R,5R)-3-Amino-5-fluoropiperidine-1-carbonyl)-4-fluoro-3-methylpyrazolo[1,5-a]pyridin-2-yl)-1-(cyclopropylmethyl)-1H-indol-7-yl)piperidin-1-yl)ethan-1-one;
1-(4-(2-(6-((3R,5R)-3-Amino-5-fluoropiperidine-1-carbonyl)-4-fluoro-3-methylpyrazolo[1,5-a]pyridin-2-yl)-1-(cyclopropylmethyl)-1H-indol-7-yl)piperidin-1-yl)-2-methoxyethan-1-one;
1-(4-(2-(6-((3R,5R)-3-amino-5-fluoropiperidine-1-carbonyl)-3-methylpyrazolo[1,5-a]pyridin-2-yl)-1-(cyclopropylmethyl)-1H-indol-7-yl)piperidin-1-yl)-2-hydroxyethan-1-one;
(R)-1-(4-(2-(6-(3-aminopiperidine-1-carbonyl)-3-methylpyrazolo[1,5-a]pyridin-2-yl)-1-(cyclopropylmethyl)-1H-indol-7-yl)piperidin-1-yl)-2-hydroxyethan-1-one;
1-(4-(2-(6-((3R,5R)-3-amino-5-fluoropiperidine-1-carbonyl)-3-methylpyrazolo[1,5-a]pyridin-2-yl)-1-(cyclopropylmethyl)-1H-indol-7-yl)piperidin-1-yl)-2-ethoxyethan-1-one;
((3R,5R)-3-amino-5-fluoropiperidin-1-yl)(2-(1-(cyclopropylmethyl)-7-(1-((1s,4s)-4-hydroxycyclohexane-1-carbonyl)piperidin-4-yl)-1H-indol-2-yl)-3-methylpyrazolo[1,5-a]pyridin-6-yl)methanone;
((3R,5R)-3-amino-5-fluoropiperidin-1-yl)(2-(1-(cyclopropylmethyl)-7-(1-((1R,3S)-3-hydroxycyclohexane-1-carbonyl)piperidin-4-yl)-1H-indol-2-yl)-3-methylpyrazolo[1,5-a]pyridin-6-yl)methanone;
((R)-3-aminopiperidin-1-yl)(2-(1-(cyclopropylmethyl)-7-(1-((1s,4s)-4-hydroxycyclohexane-1-carbonyl)piperidin-4-yl)-1H-indol-2-yl)-3-methylpyrazolo[1,5-a]pyridin-6-yl)methanone;
((3R,5R)-3-amino-5-fluoropiperidin-1-yl)(2-(1-(cyclopropylmethyl)-7-(1-((1r,4r)-4-hydroxycyclohexane-1-carbonyl)piperidin-4-yl)-1H-indol-2-yl)-3-methylpyrazolo[1,5-a]pyridin-6-yl)methanone;
1-(4-(2-(6-((3R,5R)-3-amino-5-fluoropiperidine-1-carbonyl)-3-methylpyrazolo[1,5-a]pyridin-2-yl)-1-(cyclopropylmethyl)-1H-indol-7-yl)piperidin-1-yl)ethan-1-one;
1-(4-(2-(6-((7R)-7-amino-2-azabicyclo[2.2.1]heptane-2-carbonyl)-3-methylpyrazolo[1,5-a]pyridin-2-yl)-1-(cyclopropylmethyl)-1H-indol-7-yl)piperidin-1-yl)-2-methoxyethan-1-one;
(R)-(3-aminopiperidin-1-yl)(2-(1-(cyclopropylmethyl)-7-(1-(3-hydroxy-3-(trifluoromethyl)cyclobutane-1-carbonyl)piperidin-4-yl)-1H-indol-2-yl)-3-methylpyrazolo[1,5-a]pyridin-6-yl)methanone;
((R)-3-aminopiperidin-1-yl)(2-(1-(cyclopropylmethyl)-7-(1-(tetrahydro-2H-pyran-2-carbonyl)piperidin-4-yl)-1H-indol-2-yl)-3-methylpyrazolo[1,5-a]pyridin-6-yl)methanone;
((R)-3-aminopiperidin-1-yl)(2-(1-(cyclopropylmethyl)-7-(1-(tetrahydrofuran-2-carbonyl)piperidin-4-yl)-1H-indol-2-yl)-3-methylpyrazolo[1,5-a]pyridin-6-yl)methanone;
(4-(2-(6-((7R)-7-amino-2-azabicyclo[2.2.1]heptane-2-carbonyl)-3-methylpyrazolo[1,5-a]pyridin-2-yl)-1-(cyclopropylmethyl)-1H-indol-7-yl)piperidin-1-yl)(3-hydroxycyclobutyl)methanone;
Methyl 4-(2-(6-((7R)-7-amino-2-azabicyclo[2.2.1]heptane-2-carbonyl)-3-methylpyrazolo[1,5-a]pyridin-2-yl)-1-(cyclopropylmethyl)-1H-indol-7-yl)piperidine-1-carboxylate;
(2R)-1-(4-(2-(6-((7R)-7-Amino-2-azabicyclo[2.2.1]heptane-2-carbonyl)-3-methylpyrazolo[1,5-a]pyridin-2-yl)-1-(cyclopropylmethyl)-1H-indol-7-yl)piperidin-1-yl)-2-hydroxypropan-1-one;
(4-(2-(6-((7R)-7-Amino-2-azabicyclo[2.2.1]heptane-2-carbonyl)-3-methylpyrazolo[1,5-a]pyridin-2-yl)-1-(cyclopropylmethyl)-1H-indol-7-yl)piperidin-1-yl)(thiophen-2-yl)methanone;
1-(4-(2-(6-((3R,5R)-3-Amino-5-fluoropiperidine-1-carbonyl)-3-methylpyrazolo[1,5-a]pyridin-2-yl)-1-(cyclopropylmethyl)-1H-indol-7-yl)piperidin-1-yl)ethan-1-one;
1-(4-(2-(6-((7R)-7-Amino-2-azabicyclo[2.2.1]heptane-2-carbonyl)-3-methylpyrazolo[1,5-a]pyridin-2-yl)-1-(cyclopropylmethyl)-1H-indol-7-yl)piperidin-1-yl)ethan-1-one;
1-(4-(2-(6-((3R,5R)-3-Amino-5-fluoropiperidine-1-carbonyl)-3-methylpyrazolo[1,5-a]pyridin-2-yl)-1-(cyclopropylmethyl)-5-fluoro-1H-indol-7-yl)piperidin-1-yl)-3-methylbutan-1-one;
((3R,5R)-3-Amino-5-fluoropiperidin-1-yl)(2-(1-(cyclopropylmethyl)-5-fluoro-7-(1-(3-hydroxycyclobutane-1-carbonyl)piperidin-4-yl)-1H-indol-2-yl)-3-methylpyrazolo[1,5-a]pyridin-6-yl)methanone;
(R)-1-(4-(2-(6-((R)-3-Aminopiperidine-1-carbonyl)-3-methylpyrazolo[1,5-a]pyridin-2-yl)-1-(cyclopropylmethyl)-5-fluoro-1H-indol-7-yl)piperidin-1-yl)-2-methoxypropan-1-one;
((R)-3-Aminopiperidin-1-yl)(2-(1-(cyclopropylmethyl)-5-fluoro-7-(1-(tetrahydro-2H-pyran-2-carbonyl)piperidin-4-yl)-1H-indol-2-yl)-3-methylpyrazolo[1,5-a]pyridin-6-yl)methanone;
1-(4-(2-(6-((3R,5R)-3-Amino-5-fluoropiperidine-1-carbonyl)-4-methoxy-3-methylpyrazolo[1,5-a]pyridin-2-yl)-1-(cyclopropylmethyl)-1H-pyrrolo[2,3-c]pyridin-7-yl)piperidin-1-yl)-2-methoxyethan-1-one;
((3R,5R)-3-Amino-5-fluoropiperidin-1-yl)(2-(1-(cyclopropylmethyl)-7-(1-((1s,4s)-4-hydroxycyclohexane-1-carbonyl)azetidin-3-yl)-1H-indol-2-yl)-4-methoxy-3-methylpyrazolo[1,5-a]pyridin-6-yl)methanone;
1-(3-(2-(6-((3R,5R)-3-Amino-5-fluoropiperidine-1-carbonyl)-4-methoxy-3-methylpyrazolo[1,5-a]pyridin-2-yl)-1-(cyclopropylmethyl)-1H-indol-7-yl)azetidin-1-yl)-2-methoxyethan-1-one;
((R)-3-Aminopiperidin-1-yl)(2-(1-(cyclopropylmethyl)-7-(1-((1r,4r)-4-hydroxycyclohexane-1-carbonyl)azetidin-3-yl)-1H-indol-2-yl)-3-methylpyrazolo[1,5-a]pyridin-6-yl)methanone;
1-(3-(2-(6-((7R)-7-Amino-2-azabicyclo[2.2.1]heptane-2-carbonyl)-3-methylpyrazolo[1,5-a]pyridin-2-yl)-1-(cyclopropylmethyl)-1H-indol-7-yl)azetidin-1-yl)ethan-1-one;
4-(((2-(6-((R)-3-Aminopiperidine-1-carbonyl)-4-methoxy-3-methylpyrazolo[1,5-a]pyridin-2-yl)-1-(cyclopropylmethyl)-1H-indol-7-yl)oxy)methyl)pyrrolidin-2-one;
(R)-(2-(7-(2-(1H-1,2,4-Triazol-1-yl)ethoxy)-1-(cyclopropylmethyl)-1H-indol-2-yl)-4-methoxy-3-methylpyrazolo[1,5-a]pyridin-6-yl)(3-aminopiperidin-1-yl)methanone;
(2-(7-(2-(1H-1,2,4-Triazol-1-yl)ethoxy)-1-(cyclopropylmethyl)-1H-indol-2-yl)-4-methoxy-3-methylpyrazolo[1,5-a]pyridin-6-yl)((3R,5R)-3-amino-5-fluoropiperidin-1-yl)methanone;
(2-(7-(2-(4-Amino-1H-pyrazol-1-yl)ethoxy)-1-(cyclopropylmethyl)-1H-indol-2-yl)-4-methoxy-3-methylpyrazolo[1,5-a]pyridin-6-yl)((3R,5R)-3-amino-5-fluoropiperidin-1-yl)methanone;
4-(((2-(6-((R)-3-Aminopiperidine-1-carbonyl)-4-methoxy-3-methylpyrazolo[1,5-a]pyridin-2-yl)-1-(cyclopropylmethyl)-1H-indol-7-yl)oxy)methyl)pyrrolidin-2-one;
4-(((2-(6-((3R,5R)-3-Amino-5-fluoropiperidine-1-carbonyl)-4-methoxy-3-methylpyrazolo[1,5-a]pyridin-2-yl)-1-(cyclopropylmethyl)-1H-indol-7-yl)oxy)methyl)pyrrolidin-2-one;
4-(((2-(6-((R)-3-Aminopiperidine-1-carbonyl)-4-fluoro-3-methylpyrazolo[1,5-a]pyridin-2-yl)-1-(cyclopropylmethyl)-1H-indol-7-yl)oxy)methyl)pyrrolidin-2-one;
4-(((2-(6-((3R,5R)-3-Amino-5-fluoropiperidine-1-carbonyl)-4-fluoro-3-methylpyrazolo[1,5-a]pyridin-2-yl)-1-(cyclopropylmethyl)-1H-indol-7-yl)oxy)methyl)pyrrolidin-2-one;
(S)-5-(((2-(6-((R)-3-Aminopiperidine-1-carbonyl)-4-fluoro-3-methylpyrazolo[1,5-a]pyridin-2-yl)-1-(cyclopropylmethyl)-1H-indol-7-yl)oxy)methyl)pyrrolidin-2-one;
4-(((2-(6-((R)-3-Aminopiperidine-1-carbonyl)-4-fluoro-3-methylpyrazolo[1,5-a]pyridin-2-yl)-1-(cyclopropylmethyl)-1H-indol-7-yl)oxy)methyl)pyrrolidin-2-one;
(2-(7-(2-(1H-Imidazol-1-yl)ethoxy)-1-(cyclopropylmethyl)-1H-indol-2-yl)-3-methylpyrazolo[1,5-a]pyridin-6-yl)((3R,5R)-3-amino-5-fluoropiperidin-1-yl)methanone;
(2-(7-(2-(1H-1,2,4-Triazol-1-yl)ethoxy)-1-(cyclopropylmethyl)-1H-indol-2-yl)-3-methylpyrazolo[1,5-a]pyridin-6-yl)((3R,5R)-3-amino-5-fluoropiperidin-1-yl)methanone;
(S)-5-(((2-(6-((R)-3-Aminopiperidine-1-carbonyl)-3-methylpyrazolo[1,5-a]pyridin-2-yl)-1-(cyclopropylmethyl)-1H-indol-7-yl)oxy)methyl)pyrrolidin-2-one;
4-(((2-(6-((3R,5R)-3-Amino-5-fluoropiperidine-1-carbonyl)-3-methylpyrazolo[1,5-a]pyridin-2-yl)-1-(cyclopropylmethyl)-1H-indol-7-yl)oxy)methyl)pyrrolidin-2-one;
4-(((2-(6-((R)-3-Aminopiperidine-1-carbonyl)-4-methoxy-3-methylpyrazolo[1,5-a]pyridin-2-yl)-1-(cyclopropylmethyl)-1H-pyrrolo[2,3-c]pyridin-7-yl)oxy)methyl)pyrrolidin-2-one;
(2-(7-(2-(1H-1,2,4-Triazol-1-yl)ethoxy)-1-(cyclopropylmethyl)-1H-pyrrolo[2,3-c]pyridin-2-yl)-4-methoxy-3-methylpyrazolo[1,5-a]pyridin-6-yl)((3R,5R)-3-amino-5-fluoropiperidin-1-yl)methanone;
(2-(7-(2-(1H-Imidazol-1-yl)ethoxy)-1-(cyclopropylmethyl)-1H-pyrrolo[2,3-c]pyridin-2-yl)-4-methoxy-3-methylpyrazolo[1,5-a]pyridin-6-yl)((3R,5R)-3-amino-5-fluoropiperidin-1-yl)methanone;
(R)-(2-(7-(2-(1H-Imidazol-1-yl)ethoxy)-1-(cyclopropylmethyl)-1H-pyrrolo[2,3-c]pyridin-2-yl)-4-methoxy-3-methylpyrazolo[1,5-a]pyridin-6-yl)(3-aminopiperidin-1-yl)methanone;
(R)-(2-(7-(2-(4H-1,2,4-Triazol-4-yl)ethoxy)-1-(cyclopropylmethyl)-1H-pyrrolo[2,3-c]pyridin-2-yl)-4-methoxy-3-methylpyrazolo[1,5-a]pyridin-6-yl)(3-aminopiperidin-1-yl)methanone;
(2-(7-(2-(4H-1,2,4-Triazol-4-yl)ethoxy)-1-(cyclopropylmethyl)-1H-pyrrolo[2,3-c]pyridin-2-yl)-4-methoxy-3-methylpyrazolo[1,5-a]pyridin-6-yl)((3R,5R)-3-amino-5-fluoropiperidin-1-yl)methanone;
4-(((2-(6-((3R,5R)-3-Amino-5-fluoropiperidine-1-carbonyl)-4-methoxy-3-methylpyrazolo[1,5-a]pyridin-2-yl)-1-(cyclopropylmethyl)-1H-pyrrolo[2,3-c]pyridin-7-yl)oxy)methyl)pyrrolidin-2-one;
4-(((2-(6-((3R,5R)-3-Amino-5-fluoropiperidine-1-carbonyl)-4-methoxy-3-methylpyrazolo[1,5-a]pyridin-2-yl)-1-(cyclopropylmethyl)-1H-pyrrolo[2,3-c]pyridin-7-yl)oxy)methyl)pyrrolidin-2-one;
(5R)-5-(((2-(6-((7R)-7-Amino-2-azabicyclo[2.2.1]heptane-2-carbonyl)-4-methoxy-3-methylpyrazolo[1,5-a]pyridin-2-yl)-1-(cyclopropylmethyl)-1H-pyrrolo[2,3-c]pyridin-7-yl)oxy)methyl)pyrrolidin-2-one;
4-(((2-(6-((7R)-7-Amino-2-azabicyclo[2.2.1]heptane-2-carbonyl)-4-methoxy-3-methylpyrazolo[1,5-a]pyridin-2-yl)-1-(cyclopropylmethyl)-1H-pyrrolo[2,3-c]pyridin-7-yl)oxy)methyl)pyrrolidin-2-one;
(5R)-5-(((2-(6-((7S)-7-Amino-2-azabicyclo[2.2.1]heptane-2-carbonyl)-4-methoxy-3-methylpyrazolo[1,5-a]pyridin-2-yl)-1-(cyclopropylmethyl)-1H-pyrrolo[2,3-c]pyridin-7-yl)oxy)methyl)pyrrolidin-2-one;
4-(((2-(6-((7R)-7-Amino-2-azabicyclo[2.2.1]heptane-2-carbonyl)-4-methoxy-3-methylpyrazolo[1,5-a]pyridin-2-yl)-1-(cyclopropylmethyl)-1H-pyrrolo[2,3-c]pyridin-7-yl)oxy)methyl)pyrrolidin-2-one;
(R)-(2-(7-(2-(4H-1,2,4-Triazol-4-yl)ethoxy)-1-(cyclopropylmethyl)-1H-pyrrolo[2,3-c]pyridin-2-yl)-3-methylpyrazolo[1,5-a]pyridin-6-yl)(3-aminopiperidin-1-yl)methanone;
(2-(7-(2-(1H-Imidazol-1-yl)ethoxy)-1-(cyclopropylmethyl)-1H-pyrrolo[2,3-c]pyridin-2-yl)-3-methylpyrazolo[1,5-a]pyridin-6-yl)((3R,5R)-3-amino-5-fluoropiperidin-1-yl)methanone;
(R)-(2-(7-(2-(1H-Imidazol-1-yl)ethoxy)-1-(cyclopropylmethyl)-1H-pyrrolo[2,3-c]pyridin-2-yl)-3-methylpyrazolo[1,5-a]pyridin-6-yl)(3-aminopiperidin-1-yl)methanone;
4-(((2-(6-((R)-3-Aminopiperidine-1-carbonyl)-3-methylpyrazolo[1,5-a]pyridin-2-yl)-1-(cyclopropylmethyl)-1H-pyrrolo[2,3-c]pyridin-7-yl)oxy)methyl)pyrrolidin-2-one;
4-(((2-(6-((R)-3-Aminopiperidine-1-carbonyl)-3-methylpyrazolo[1,5-a]pyridin-2-yl)-1-(cyclopropylmethyl)-1H-pyrrolo[2,3-c]pyridin-7-yl)oxy)methyl)pyrrolidin-2-one;
or a pharmaceutically acceptable salt thereof.

13. A pharmaceutical composition comprising a compound according to any one of the claims 1-12, or a stereoisomer, an enantiomer, a diastereomer, a tautomer, or a pharmaceutically acceptable salt thereof, and one or more pharmaceutically acceptable carriers.

14. A compound according to any one of claims 1-12, or a stereoisomer, an enantiomer, a diastereomer, a tautomer, or a pharmaceutically acceptable salt thereof, or a composition according to claim 13, for use in therapy.

15. A compound according to any one of claims 1-12, or a stereoisomer, an enantiomer, a diastereomer, a tautomer, or a pharmaceutically acceptable salt thereof, or a composition according to claim 13, for use in treating a disease or disorder associated with PAD4 enzyme activity selected from rheumatoid arthritis, Alzheimer's disease, multiple sclerosis, lupus, Parkinson's disease, and cancer.

## Patentansprüche

1. Verbindung der Formel (la): 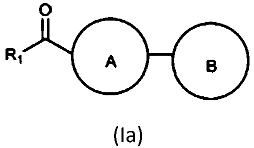 oder ein pharmazeutisch annehmbares Salz davon, wobei: 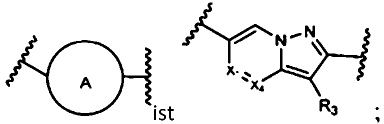 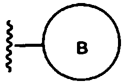 aus 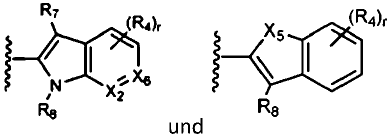 ausgewählt wird;
X₁ unabhängig aus CR₂ und N ausgewählt wird;
X₂ unabhängig aus CR₄ und N ausgewählt wird;
X₄ unabhängig aus CR₂ und N ausgewählt wird; vorausgesetzt, X₁ und X₄ sind nicht beide N;
X₅ unabhängig aus O und S ausgewählt wird;
X₆ unabhängig aus CR₄ und N ausgewählt wird; vorausgesetzt 1) X₂ und X₆ sind nicht beide N; 2) wenn X₂ und X₆ beide CR₄ sind, eines von R₄ H ist;
R₁ unabhängig aus 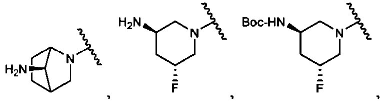 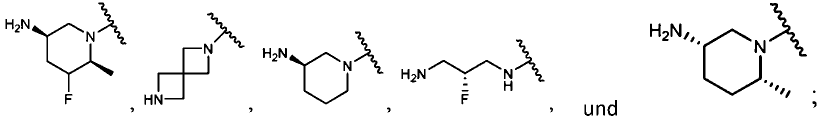 ausgewählt wird;
R₂ unabhängig aus H, F, Cl, C₁₋₄ Alkyl ausgewählt wird, optional substituiert mit einem oder mehreren Substituenten, ausgewählt aus F, CI und OH, und -OC₁₋₄ -Alkyl;
R₃ unabhängig aus H, F, Cl, CN, -C(=O)OR_{b}, und C₁₋₃ Alkyl ausgewählt wird, optional substituiert mit einem oder mehreren Substituenten, ausgewählt aus F, Cl, OH, NH₂, und N₃;
R₄ unabhängig aus H, F, Cl, C₁₋₅ Alkyl ausgewählt wird, optional substituiert mit einem oder mehreren Substituenten, ausgewählt aus F, CI und OH, C₃₋₆ Cycloalkyl, 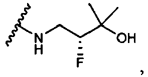 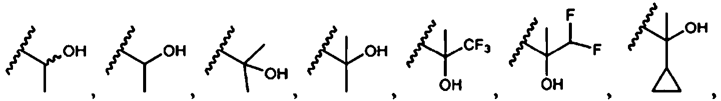 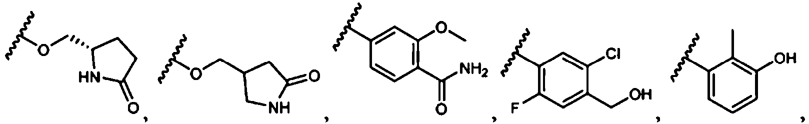 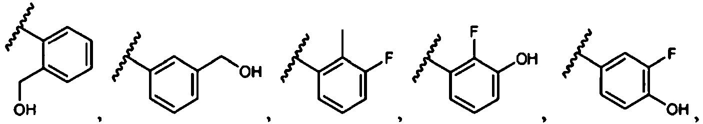 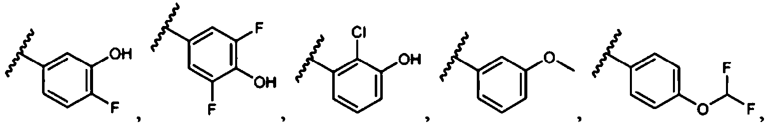 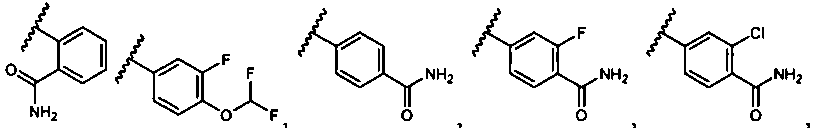 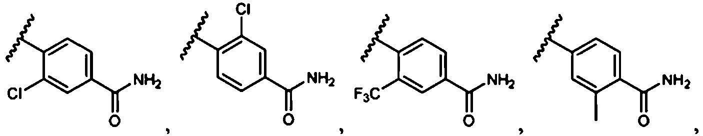 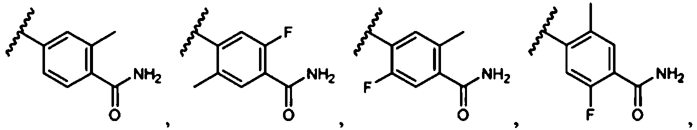 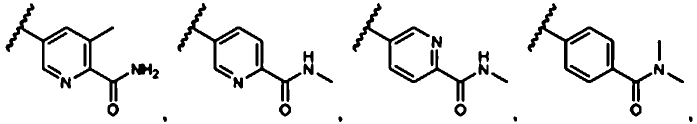 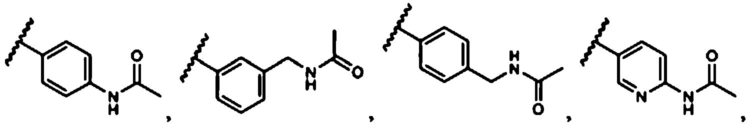 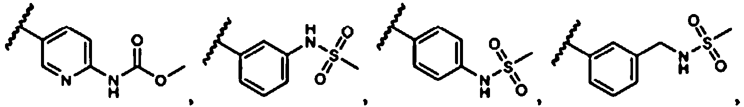 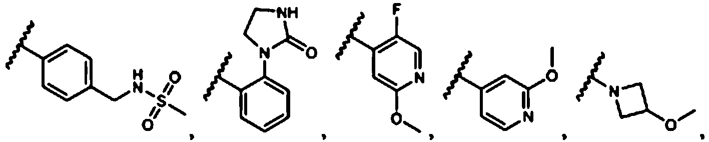 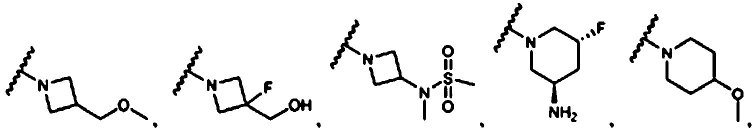 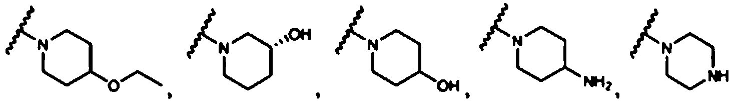 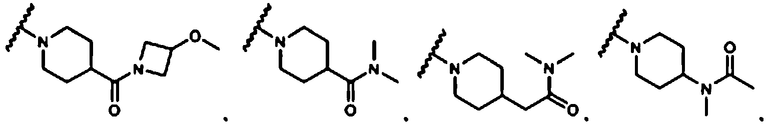 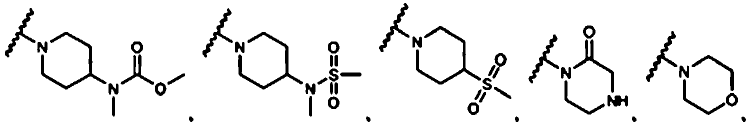 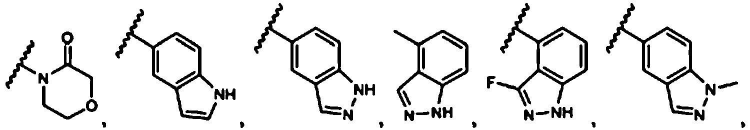 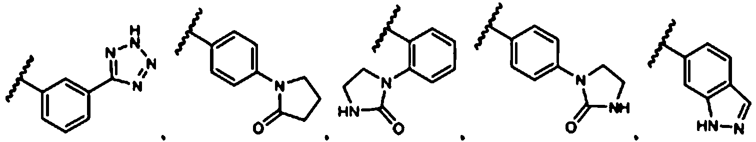 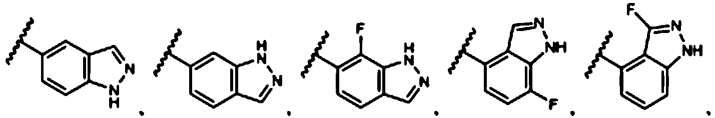 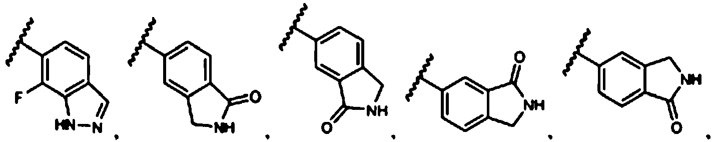 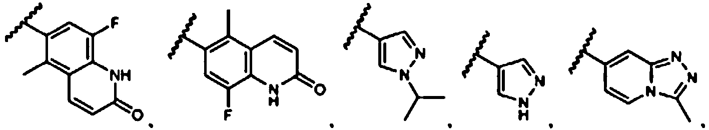 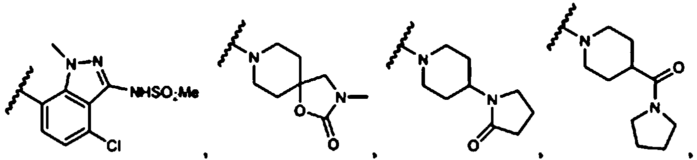 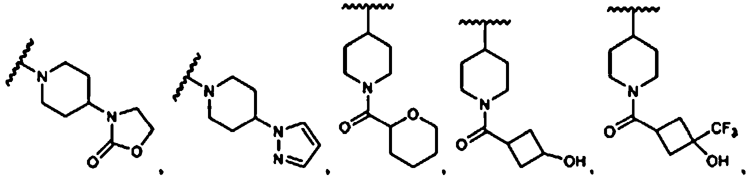 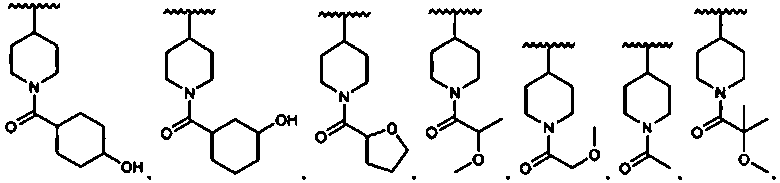 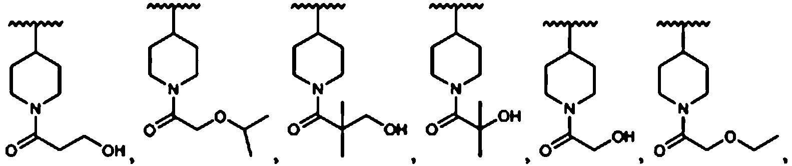 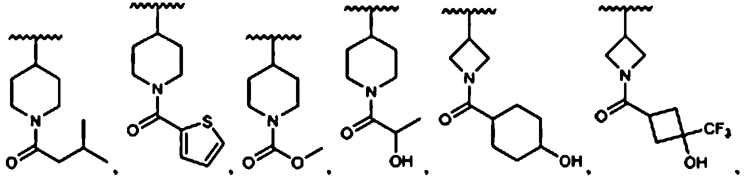 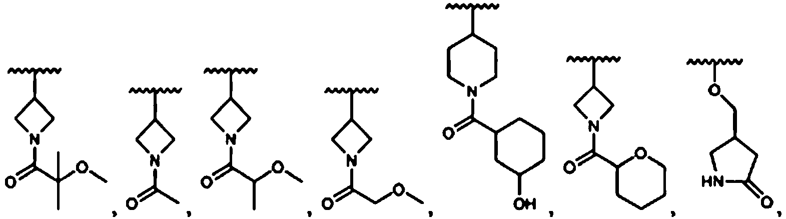 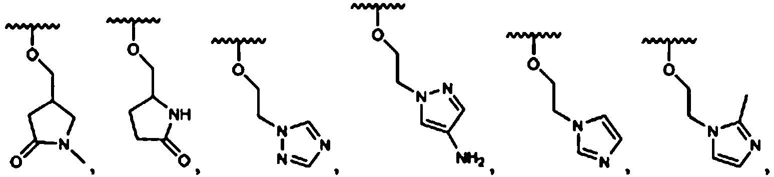 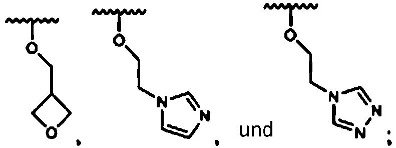
R₇ unabhängig aus H, F und CI ausgewählt wird;
R₈ unabhängig aus H und C₁₋₆ Alkyl ausgewählt wird, optional substituiert mit einem oder mehreren Substituenten, ausgewählt aus F, CI und C₃₋₆ Cycloalkyl;
R_{b} unabhängig aus H, C₁₋₆-Alkyl ausgewählt wird, optional substituiert mit einem oder mehreren Rₑ, C₂₋₆-Alkenyl, optional substituiert mit einem oder mehreren Re, C₂₋₆-Alkinyl, optional substituiert mit einem oder mehreren Rₑ, -(CH₂)ᵣ-C₃₋₁₀-Carbocyclyl, optional substituiert mit einem oder mehreren Re, und -(CH₂)ᵣ-Heterocyclyl, optional substituiert mit einem oder mehreren Rₑ;
Rₑ unabhängig aus F, Cl, Br, CN, NH₂, -NH-C₁₋₄ Alkyl, -N(C₁₋₄ Alkyl)₂, =O, OH, - OC₁₋₆-Alkyl, -CO₂H, C₁₋₆-Alkyl ausgewählt wird, optional substituiert mit einem oder mehreren R_{f}, C₂₋₆-Alkenyl, C₂₋₆-Alkinyl, -(CH₂)ᵣ -C₃₋₆-Cycloalkyl, optional substituiert mit einem oder mehreren R_{f}, -(CH₂)ᵣ-Aryl, optional substituiert mit einem oder mehreren R_{f}, und -(CH₂)ᵣ-Heterocyclyl, optional substituiert mit einem oder mehreren R_{f};
R_{f} unabhängig aus F, Cl, Br, CN, OH, OC₁₋₅ Alkyl, C₁₋₅ Alkyl ausgewählt wird, optional substituiert mit OH, C₂₋₅ Alkenyl, C₂₋₅ Alkinyl, C₃₋₆ Cycloalkyl und Phenyl; und r bei jedem Vorkommen unabhängig aus Null, 1, 2, 3 und 4 ausgewählt wird.

2. Verbindung nach Anspruch 1, welche die Formeln (IIa)-(XIII) aufweist: 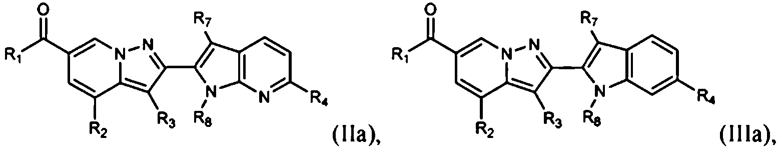 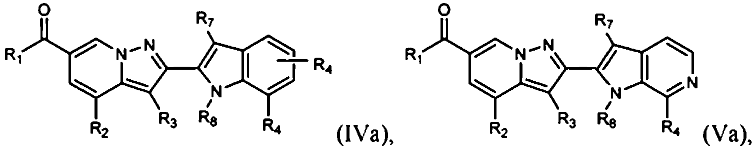 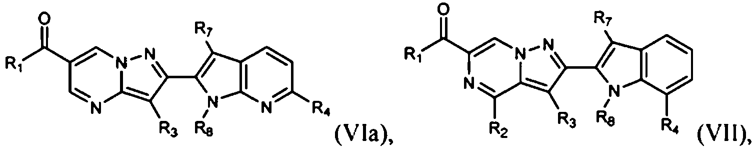 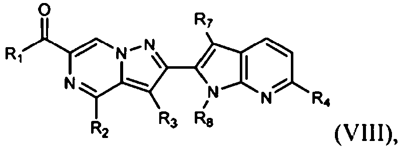 ode 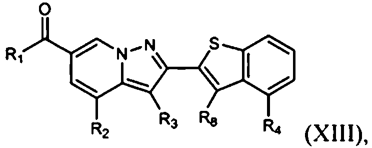 oder ein pharmazeutisch annehmbares Salz davon.

3. Verbindung nach Anspruch 2, welche die Formel (IIa) aufweist: 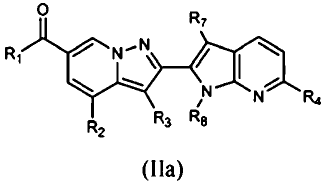 oder ein pharmazeutisch annehmbares Salz davon, wobei:
R₁ unabhängig aus 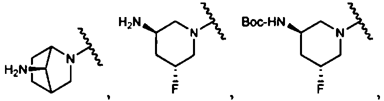 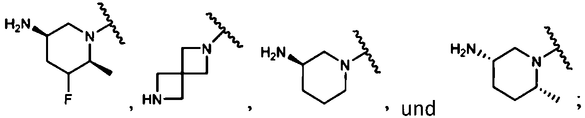 ausgewählt wird;
R₂ unabhängig aus H, F, Cl, C₁₋₃ Alkyl ausgewählt wird, optional substituiert mit einem oder mehreren Substituenten, ausgewählt aus F, CI und OH, und OC₁₋₃ Alkyl; und
R₃ unabhängig aus H, F, Cl und C₁₋₃ Alkyl ausgewählt wird, optional substituiert mit einem oder mehreren Substituenten, ausgewählt aus F, CI und OH.

4. Verbindung nach Anspruch 1, welche die Formel (IIIa) aufweist: 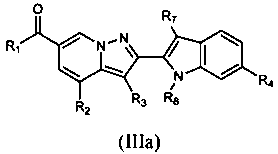 oder ein pharmazeutisch annehmbares Salz davon, wobei:
R₁ unabhängig aus 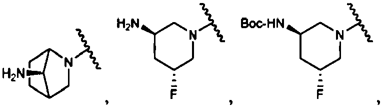 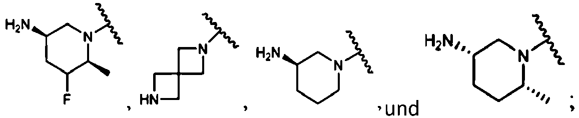 ausgewählt wird;
R₂ unabhängig aus H, F, Cl, C₁₋₃ Alkyl ausgewählt wird, optional substituiert mit einem oder mehreren Substituenten, ausgewählt aus F, CI und OH, und OC₁₋₃ Alkyl; und
R₃ unabhängig aus H, F, Cl und C₁₋₃ Alkyl ausgewählt wird, optional substituiert mit einem oder mehreren Substituenten, ausgewählt aus F, CI und OH.

5. Verbindung nach Anspruch 3 oder 4, oder ein pharmazeutisch annehmbares Salz davon, wobei:
R₁ unabhängig aus 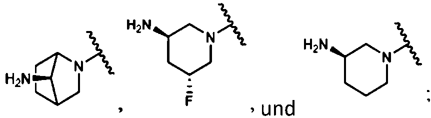 ausgewählt wird;
R₂ unabhängig aus H, F, Cl, CH₃ und OCH₃ ausgewählt wird;
R₃ unabhängig aus H, F, Cl, CH₃ und CH₂OH ausgewählt wird;
R₇ H ist; und
R₈ C₁₋₃-Alkyl ist, substituiert mit einem C₃₋₆ Cycloalkyl.

6. Verbindung nach Anspruch 1, welche die Formel (IVa) aufweist: 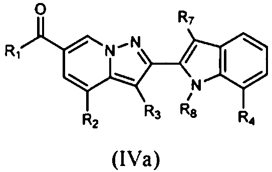 oder ein pharmazeutisch annehmbares Salz davon, wobei:
R₁ unabhängig aus 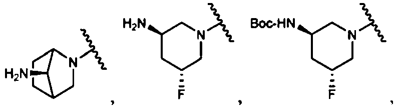 ausgewählt wird; 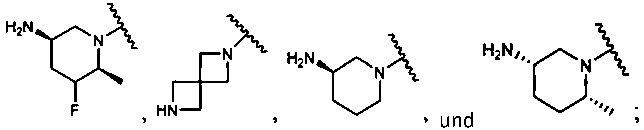
R₂ unabhängig aus H, F, Cl, C₁₋₃ Alkyl ausgewählt wird, optional substituiert mit einem oder mehreren Substituenten, ausgewählt aus F, CI und OH, und OC₁₋₃ Alkyl;
R₃ unabhängig aus H, F, Cl und C₁₋₃ Alkyl ausgewählt wird, optional substituiert mit einem oder mehreren Substituenten, ausgewählt aus F, CI und OH;
wobei vorzugsweise:
R₁ unabhängig aus 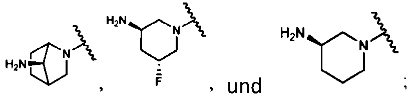 ausgewählt wird;
R₂ unabhängig aus H, F, Cl und OCH₃ ausgewählt wird;
R₃ unabhängig aus F, Cl und CH₃ ausgewählt wird;
R₇ H ist; und
R₈ C₁₋₂-Alkyl ist, optional substituiert mit Cyclopropyl;
wobei bevorzugter:
R₄ unabhängig aus 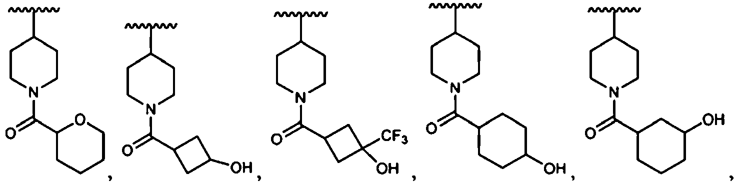 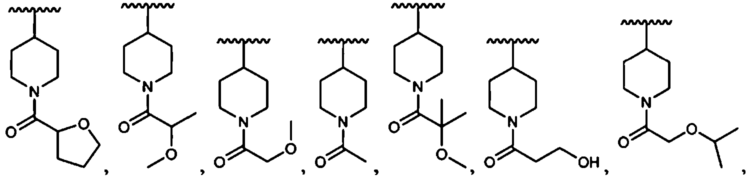 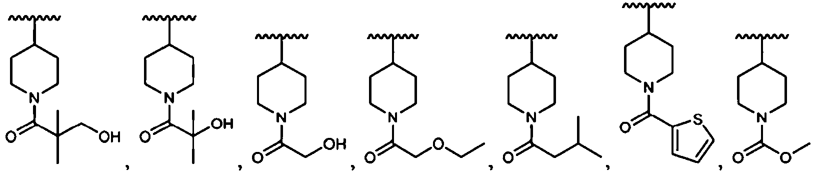 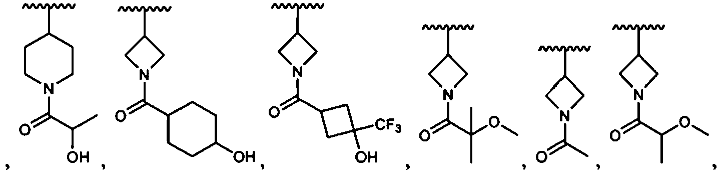 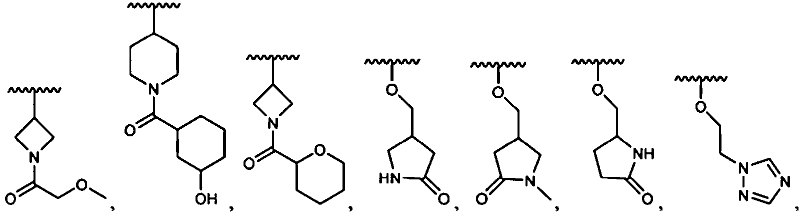 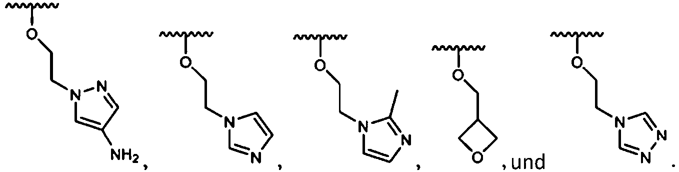 ausgewählt wird.

7. Verbindung nach Anspruch 1, welche die Formel (Va) aufweist: 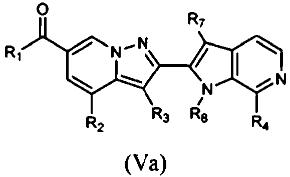 oder ein pharmazeutisch annehmbares Salz davon, wobei:
R₁ unabhängig aus 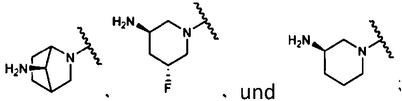 ausgewählt wird;
R₂ unabhängig aus H, F, Cl, CH₃ und OCH₃ ausgewählt wird;
R₃ unabhängig aus H, F, Cl, CN, CH₃ und CH₂OH ausgewählt wird;
wobei vorzugsweise:
R₁ unabhängig aus 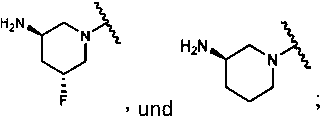 ausgewählt wird;
R₂ CH₃ ist;
R3 CH₃ ist;
R₄ unabhängig aus 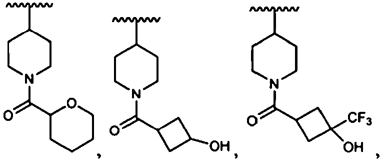 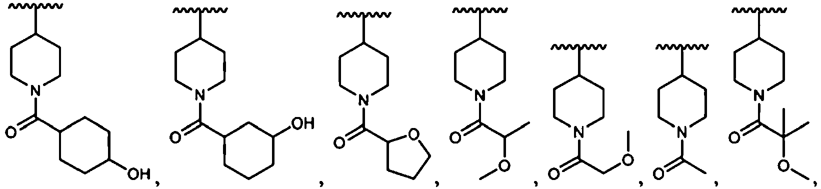 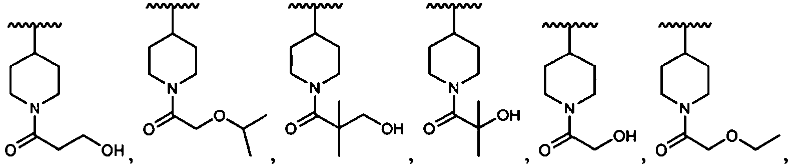 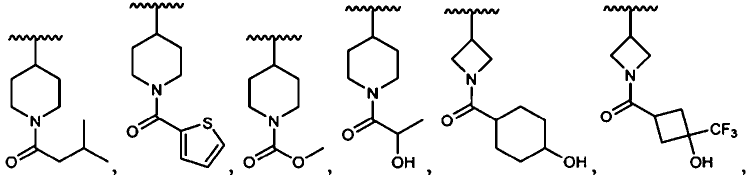 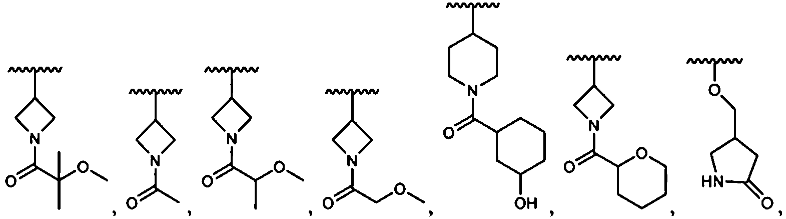 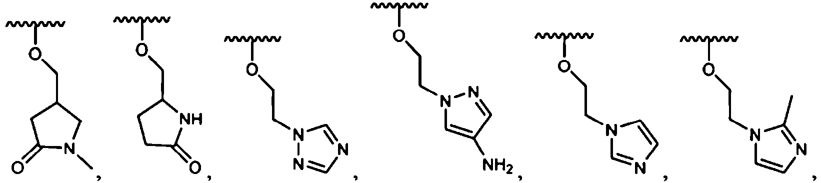 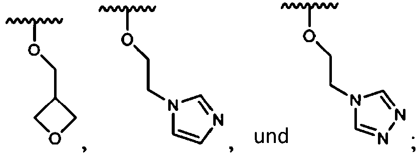 ausgewählt wird;
R₇ H ist; und
R₈ C₁₋₂-Alkyl ist, optional substituiert mit Cyclopropyl.

8. Verbindung nach Anspruch 1, welche die Formel (VIa) aufweist: 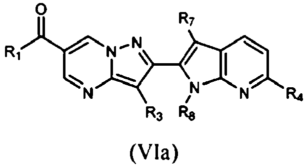 oder ein pharmazeutisch annehmbares Salz davon, wobei:
R₁ unabhängig aus 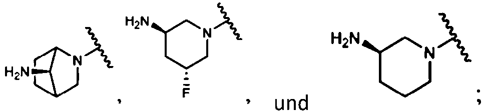 ausgewählt wird; und
R₃ unabhängig aus H, F und CH₃ ausgewählt wird;
wobei vorzugsweise:
R₁ unabhängig aus 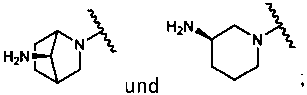 ausgewählt wird;
R3 CH₃ ist;
R₄ unabhängig aus F, Cl, 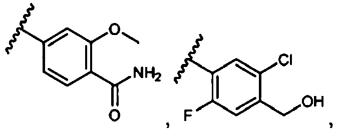 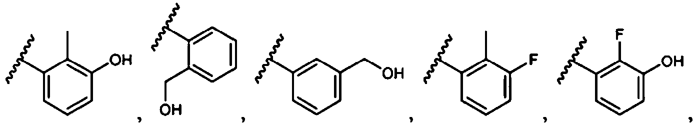 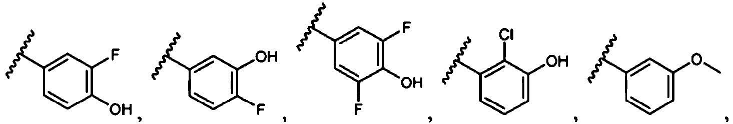 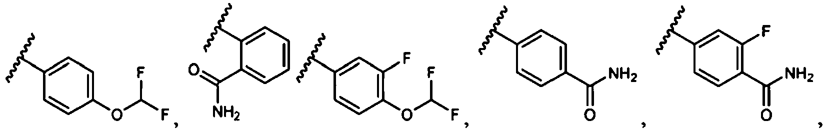 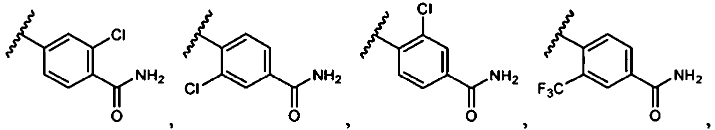 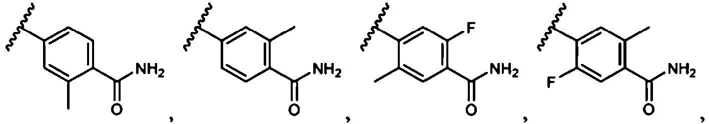 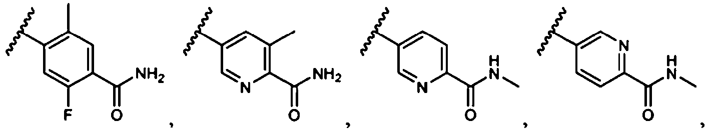 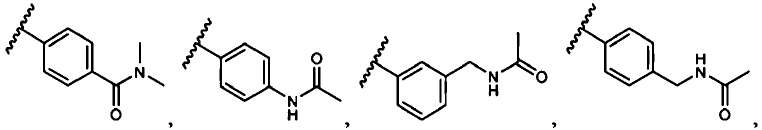 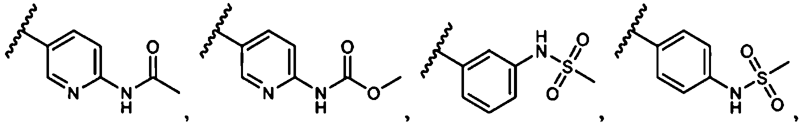 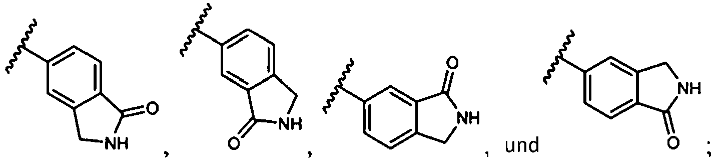 ausgewählt wird;
R₇ H ist; und
R₈ C₁₋₂-Alkyl ist, optional substituiert mit Cyclopropyl.

9. Verbindung nach Anspruch 1, welche die Formel (VII) aufweist: 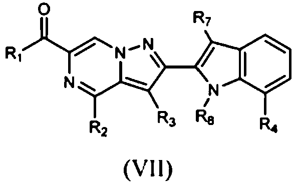 oder ein pharmazeutisch annehmbares Salz davon, wobei:
R₁ unabhängig aus 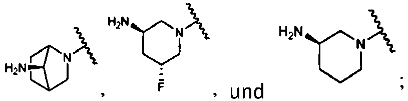 ausgewählt wird;
R₂ unabhängig aus H, F, Cl, CH₃ und OCH₃ ausgewählt wird; und
R₃ unabhängig aus H, F und CH₃ ausgewählt wird.

10. Verbindung nach Anspruch 1, welche die Formel (VIII) aufweist: 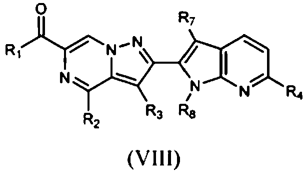 oder ein pharmazeutisch annehmbares Salz davon, wobei:
R₁ unabhängig aus 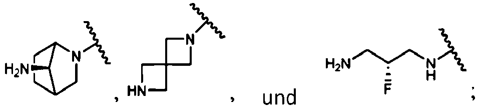 ausgewählt wird;
R₂ unabhängig aus H, F und OCH₃ ausgewählt wird; und
R₃ unabhängig aus H, F und CH₃ ausgewählt wird.

11. Verbindung nach Anspruch 1, welche die Formel (XIII) aufweist: 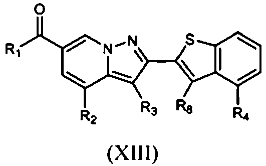 oder ein pharmazeutisch annehmbares Salz davon, wobei:
R₁ unabhängig aus 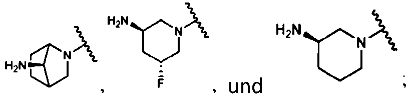 ausgewählt wird;
R₂ unabhängig aus H, F, CH₃, und OCH₃ ausgewählt wird; und
R₃ unabhängig aus H, F und CH₃ ausgewählt wird.

12. Verbindung nach Anspruch 1 ausgewählt aus:
5-(2-(6-((7R)-7-Amino-2-azabicyclo[2.2.1]-heptan-2-carbonyl)-4-methoxy-3-methylpyrazol[1,5-a]pyridin-2-yl)-1-(cyclopropylmethyl)-1H-pyrrolo[2,3-b]pyridin-6-yl)isoindolin-1-one;
((7R)-7-Amino-2-azabicyclo[2.2.1]heptan-2-yl)(2-(6-cyclopropyl-1-(cyclopropylmethyl)-1H-pyrrolo[2,3-b]pyridin-2-yl)-4-methoxy-3-methylpyrazolo[1,5-a]pyridin-6-yl)methanon;
5-(2-(6-((7R)-7-Amino-2-azabicyclo[2.2.1]heptan-2-carbonyl)-4-methoxy-3-methylpyrazolo[1,5-a]pyridin-2-yl)-1-(cyclopropylmethyl)-1H-indol-6-yl)isoindolin-1-one;
((7R)-7-Amino-2-azabicyclo[2.2.1]heptan-2-yl)(2-(1-(cyclopropylmethyl)-6-(3-methyl-[1,2,4]triazolo[4,3-a]pyridin-7-yl)-1H-indol-2-yl)-4-methoxy-3-methylpyrazolo[1,5-a]pyridin-6-yl)methanon;
((7R)-7-Amino-2-azabicyclo[2.2.1]heptan-2-yl)(2-(1-(cyclopropylmethyl)-6-methyl-1H-indol-2-yl)-4-methoxy-3-methylpyrazolo[1,5-a]pyridin-6-yl)methanon;
3((7R)-7-Amino-2-azabicyclo[2.2.1]heptan-2-yl)(2-(1-(cyclopropylmethyl)-6-methyl-1H-pyrrolo[2,3-b]pyridin-2-yl)-4-fluor-3-methylpyrazolo[1,5-a]pyridin-6-yl)methanon;
5-(2-(6-((7R)-7-Amino-2-azabicyclo[2.2.1]heptan-2-carbonyl)-4-fluor-3-methylpyrazolo[1,5-a]pyridin-2-yl)-1-(cyclopropylmethyl)-1H-pyrrolo[2,3-b]pyridin-6-yl)isoindolin-1-one;
6-(2-(6-((7R)-7-Amino-2-azabicyclo[2.2.1]heptan-2-carbonyl)-4-fluor-3-methylpyrazolo[1,5-a]pyridin-2-yl)-1-(cyclopropylmethyl)-1H-pyrrolo[2,3-b]pyridin-6-yl)isoindolin-1-one;
((7R)-7-Amino-2-azabicyclo[2.2.1]heptan-2-yl)(2-(1-(cyclopropylmethyl)-6-(2-fluor-3-hydroxyphenyl)-1H-pyrrolo[2,3-b]pyridin-2-yl)-4-fluor-3-methylpyrazolo[1,5-a]pyridin-6-yl)methanon;
((7R)-7-Amino-2-azabicyclo[2.2.1]heptan-2-yl)(2-(1-(cyclopropylmethyl)-6-(3-methyl-[1,2,4]triazolo[4,3-a]pyridin-7-yl)-1H-indol-2-yl)-4-fluor-3-methylpyrazolo[1,5-a]pyridin-6-yl)methanon;
6-(2-(6-((7R)-7-Amino-2-azabicyclo[2.2.1]heptan-2-carbonyl)-3-methylpyrazolo[1,5-a]pyridin-2-yl)-1-(cyclopropylmethyl)-1H-pyrrolo[2,3-b]pyridin-6-yl)isoindolin-1-one;
((7R)-7-Amino-2-azabicyclo[2.2.1]heptan-2-yl)(2-(1-(cyclopropylmethyl)-6-(3-fluor-4-hydroxyphenyl)-1H-pyrrolo[2,3-b]pyridin-2-yl)-3-methylpyrazolo[1,5-a]pyridin-6-yl)methanon;
((7R)-7-Amino-2-azabicyclo[2.2.1]heptan-2-yl)(2-(1-(cyclopropylmethyl)-6-(2-fluor-3-hydroxyphenyl)-1H-pyrrolo[2,3-b]pyridin-2-yl)-3-methylpyrazolo[1,5-a]pyridin-6-yl)methanon;
4-(2-(6-((7R)-7-Amino-2-azabicyclo[2.2.1]heptan-2-carbonyl)-3-methylpyrazolo[1,5-a]pyridin-2-yl)-1-(cyclopropylmethyl)-1H-pyrrolo[2,3-b]pyridin-6-yl)-2-fluorbenzamid;
((7R)-7-Amino-2-azabicyclo[2.2.1]heptan-2-yl)(2-(1-(cyclopropylmethyl)-6-(3-methyl-[1,2,4]triazolo[4,3-a]pyridin-7-yl)-1H-pyrrolo[2,3-b]pyridin-2-yl)-3-methylpyrazolo[1,5-a]pyridin-6-yl)methanon;
((7R)-7-Amino-2-azabicyclo[2.2.1]heptan-2-yl)(2-(6-cyclopropyl-1-(cyclopropylmethyl)-1H-pyrrolo[2,3-b]pyridin-2-yl)-3-methylpyrazolo[1,5-a]pyridin-6-yl)methanon;
6-(2-(6-((7R)-7-Amino-2-azabicyclo[2.2.1]heptan-2-carbonyl)-3-methylpyrazolo[1,5-a]pyridin-2-yl)-1-(cyclopropylmethyl)-1H-indol-6-yl)isoindolin-1-one;
5-(2-(6-((7R)-7-Amino-2-azabicyclo[2.2.1]heptan-2-carbonyl)-3-methylpyrazolo[1,5-a]pyridin-2-yl)-1-(cyclopropylmethyl)-1H-indol-6-yl)isoindolin-1-one;
((7R)-7-Amino-2-azabicyclo[2.2.1]heptan-2-yl)(2-(1-(cyclopropylmethyl)-6-(2-hydroxypropan-2-yl)-1H-indol-2-yl)-4-methoxy-3-methylpyrazolo[1,5-a]pyridin-6-yl)methanon;
((3R,5R)-3-Amino-5-fluorpiperidin-1-yl)(2-(1-(cyclopropylmethyl)-6-(2-hydroxypropan-2-yl)-1H-indol-2-yl)-4-methoxy-3-methylpyrazolo[1,5-a]pyridin-6-yl)methanon;
((7R)-7-Amino-2-azabicyclo[2.2.1]heptan-2-yl)(2-(1-(cyclopropylmethyl)-6-(2-hydroxypropan-2-yl)-1H-indol-2-yl)-3-methylpyrazolo[1,5-a]pyridin-6-yl)methanon;
((7R)-7-Amino-2-azabicyclo[2.2.1]heptan-2-yl)(2-(1-(cyclopropylmethyl)-6-(1,1,1-trifluor-2-hydroxypropan-2-yl)-1H-indol-2-yl)-3-methylpyrazolo[1,5-a]pyridin-6-yl)methanon;
((7R)-7-Amino-2-azabicyclo[2.2.1]heptan-2-yl)(2-(1-(cyclopropylmethyl)-6-(2-hydroxypropan-2-yl)-1H-pyrrolo[2,3-b]pyridin-2-yl)-3-methylpyrazolo[1,5-a]pyridin-6-yl)methanon;
((7R)-7-Amino-2-azabicyclo[2.2.1]heptan-2-yl)(2-(1-(cyclopropylmethyl)-6-(1,1,1-trifluor-2-hydroxypropan-2-yl)-1H-pyrrolo[2,3-b]pyridin-2-yl)-3-methylpyrazolo[1,5-a]pyridin-6-yl)methanon;
((7R)-7-Amino-2-azabicyclo[2.2.1]heptan-2-yl)(2-(1-(cyclopropylmethyl)-6-(1-hydroxyethyl)-1H-pyrrolo[2,3-b]pyridin-2-yl)-3-methylpyrazolo[1,5-a]pyridin-6-yl)methanon;
N-[5-(2-{6-[(3R,5R)-3-Amino-5-fluorpiperidin-1-carbonyl]-4-methoxy-3-methylpyrazolo[1,5-a]pyridin-2-yl}-1-(cyclopropylmethyl)-1H-indol-6-yl)pyridin-2-yl]acetamid;
(3R,5R)-1-{2-[1-(Cyclopropylmethyl)-6-(4-methanesulfonylpiperidin-1-yl)-1H-indol-2-yl]-4-methoxy-3-methylpyrazolo[1,5-a]pyridin-6-carbonyl}-5-fluorpiperidin-3-amin;
3-(2-{6-[(7R)-7-Amino-2-azabicyclo[2.2.1]heptan-2-carbonyl]-4-fluor-3-methylpyrazolo[1,5-a]pyridin-2-yl}-1-(cyclopropylmethyl)-1H-pyrrolo[2,3-b]pyridin-6-yl)-2-Methylphenol;
5-(2-{6-[(7R)-7-Amino-2-azabicyclo[2.2.1]heptan-2-carbonyl]-4-fluor-3-methylpyrazolo[1,5-a]pyridin-2-yl}-1-(cyclopropylmethyl)-1H-pyrrolo[2,3-b]pyridin-6-yl)-N-methylpyridin-2-carboxamid;
4-(2-{6-[(7R)-7-Amino-2-azabicyclo[2.2.1]heptan-2-carbonyl]-4-fluor-3-methylpyrazolo[1,5-a]pyridin-2-yl}-1-(cyclopropylmethyl)-1H-pyrrolo[2,3-b]pyridin-6-yl)morpholin-3-one;
(7R)-2-{2-[1-(Cyclopropylmethyl)-6-(4-methanesulfonylpiperidin-1-yl)-1H-pyrrolo[2,3-b]pyridin-2-yl]-4-fluor-3-methylpyrazolo[1,5-a]pyridin-6-carbonyl}-2-azabicyclo[2.2.1]heptan-7-amin;
(7R)-2-{2-[1-(Cyclopropylmethyl)-6-{3-methyl-[1,2,4]triazolo[4,3-a]pyridin-7-yl}-1H-indol-2-yl]-4-fluor-3-methylpyrazolo[1,5-a]pyridin-6-carbonyl}-2-azabicyclo[2.2.1] heptan-7-amin;
(7R)-2-{2-[1-(Cyclopropylmethyl)-6-(1H-indazol-4-yl)-1H-pyrrolo[2,3-b]pyridin-2-yl]-3-methylpyrazolo[1,5-a]pyridin-6-carbonyl}-2-azabicyclo[2.2.1]heptan-7-amin;
3-(2-{6-[(7R)-7-Amino-2-azabicyclo[2.2.1]heptan-2-carbonyl]-3-methylpyrazolo[1,5-a]pyridin-2-yl}-1-(cyclopropylmethyl)-1H-pyrrolo[2,3-b]pyridin-6-yl)-2-methylphenol;
1-[4-(2-{6-[(7R)-7-Amino-2-azabicyclo[2.2.1]heptan-2-carbonyl]-3-methylpyrazolo[1,5-a]pyridin-2-yl}-1-(cyclopropylmethyl)-1H-pyrrolo[2,3-b]pyridin-6-yl)phenyl]pyrrolidin-2-one;
4-(2-{6-[(7R)-7-Amino-2-azabicyclo[2.2.1]heptan-2-carbonyl]-3-methylpyrazolo[1,5-a]pyridin-2-yl}-1-(cyclopropylmethyl)-1H-pyrrolo[2,3-b]pyridin-6-yl)-2-fluor-5-methylbenzamid;
(7R)-2-{2-[1-(Cyclopropylmethyl)-6-(7-fluor-1H-indazol-6-yl)-1H-pyrrolo[2,3-b]pyridin-2-yl]-3-methylpyrazolo[1,5-a]pyridin-6-carbonyl}-2-azabicyclo[2.2.1]heptan-7-amin;
Methyl N-[5-(2-{6-[(7R)-7-Amino-2-azabicyclo[2.2.1]heptan-2-carbonyl]-3-methylpyrazolo[1,5-a]pyridin-2-yl}-1-(cyclopropylmethyl)-1H-pyrrolo[2,3-b]pyridin-6-yl)pyridin-2-yl]carbamat;
4-(2-{6-[(7R)-7-Amino-2-azabicyclo[2.2.1]heptan-2-carbonyl]-3-methylpyrazolo[1,5-a]pyridin-2-yl}-1-(cyclopropylmethyl)-1H-pyrrolo[2,3-b]pyridin-6-yl)morpholin-3-one;
1-[4-(2-{6-[(7R)-7-Amino-2-azabicyclo[2.2.1]heptan-2-carbonyl]-3-methylpyrazolo[1,5-a]pyridin-2-yl}-1-(cyclopropylmethyl)-1H-indol-6-yl)phenyl]pyrrolidin-2-one;
(7R)-2-{2-[1-(Cyclopropylmethyl)-6-(1H-indazol-6-yl)-1H-indol-2-yl]-3-methylpyrazolo[1,5-a]pyridin-6-carbonyl}-2-azabicyclo[2.2.1]heptan-7-amin;
(7R)-2-{2-[1-(Cyclopropylmethyl)-6-(1H-indazol-5-yl)-1H-indol-2-yl]-3-methylpyrazolo[1,5-a]pyridin-6-carbonyl}-2-azabicyclo[2.2.1]heptan-7-amin;
N-[7-(2-{6-[(7R)-7-Amino-2-azabicyclo[2.2.1]heptan-2-carbonyl]-3-methylpyrazolo[1,5-a]pyridin-2-yl}-1-(cyclopropylmethyl)-1H-indol-6-yl)-4-chloro-1-methyl-1H-indazol-3-yl]methansulfonamid;
1-[2-(2-{6-[(7R)-7-Amino-2-azabicyclo[2.2.1]heptan-2-carbonyl]-3-methylpyrazolo[1,5-a]pyridin-2-yl}-1-(cyclopropylmethyl)-1H-indol-6-yl)phenyl]imidazolidin-2-one;
4-(2-{6-[(7R)-7-Amino-2-azabicyclo[2.2.1]heptan-2-carbonyl]-3-methylpyrazolo[1,5-a]pyridin-2-yl}-1-(cyclopropylmethyl)-1H-indol-6-yl)-2-chlorbenzamid;
(7R)-2-{2-[1-(Cyclopropylmethyl)-6-(1H-pyrazol-4-yl)-1H-indol-2-yl]-3-methylpyrazolo[1,5-a]pyridin-6-carbonyl}-2-azabicyclo[2.2.1]heptan-7-amin;
(7R)-2-{2-[1-(Cyclopropylmethyl)-6-(morpholin-4-yl)-1H-indol-2-yl]-3-methylpyrazolo[1,5-a]pyridin-6-carbonyl}-2-azabicyclo[2.2.1]heptan-7-amin;
(3R,5R)-1-{2-[1-(Cyclopropylmethyl)-6-[4-(pyrrolidin-1-carbonyl)piperidin-1-yl]-1H-indol-2-yl]-3-methylpyrazolo[1,5-a]pyridin-6-carbonyl}-5-fluorpiperidin-3-amin;
3-[1-(2-{6-[(3R,5R)-3-Amino-5-fluorpiperidin-1-carbonyl]-3-methylpyrazolo[1,5-a]pyridin-2-yl}-1-(cyclopropylmethyl)-1H-indol-6-yl)piperidin-4-yl]-1,3-oxazolidin-2-one;
2-(2-{6-[(7R)-7-Amino-2-azabicyclo[2.2.1]heptan-2-carbonyl]-3-methylpyrazolo[1,5-a]pyridin-2-yl}-1-(cyclopropylmethyl)-1H-pyrrolo[2,3-b]pyridin-6-yl)-1,1-difluorpropan-2-ol;
2-(2-{6-[(7R)-7-Amino-2-azabicyclo[2.2.1]heptan-2-carbonyl]-4-fluor-3-methylpyrazolo[1,5-a]pyridin-2-yl}-1-(cyclopropylmethyl)-1H-pyrrolo[2,3-b]pyridin-6-yl)-1,1,1-trifluorpropan-2-ol;
((3R,5R)-3-Amino-5-fluorpiperidin-1-yl)(2-(1-(cyclopropylmethyl)-7-(1-(tetrahydro-2H-pyran-2-carbonyl)piperidin-4-yl)-1H-indol-2-yl)-4-methoxy-3-methylpyrazolo[1,5-a]pyridin-6-yl)methanon;
((3R,5R)-3-Amino-5-fluorpiperidin-1-yl)(2-(1-(cyclopropylmethyl)-7-(1-(3-hydroxycyclobutan-1-carbonyl)piperidin-4-yl)-1H-indol-2-yl)-4-methoxy-3-methylpyrazolo[1,5-a]pyridin-6-yl)methanon;
((3R,5R)-3-Amino-5-fluorpiperidin-1-yl)(2-(1-(cyclopropylmethyl)-7-(1-((1r,4r)-4-hydroxycyclohexan-1-carbonyl)piperidin-4-yl)-1H-indol-2-yl)-4-methoxy-3-methylpyrazolo[1,5-a]pyridin-6-yl)methanon;
(R)-(3-Aminopiperidin-1-yl)(2-(1-(cyclopropylmethyl)-7-(1-(3-hydroxycyclobutan-1-carbonyl)piperidin-4-yl)-1H-indol-2-yl)-4-methoxy-3-methylpyrazolo[1,5-a]pyridin-6-yl)methanon;
((3R,5R)-3-Amino-5-fluorpiperidin-1-yl)(2-(1-(cyclopropylmethyl)-7-(1-(tetrahydrofuran-2-carbonyl)piperidin-4-yl)-1H-indol-2-yl)-4-methoxy-3-methylpyrazolo[1,5-a]pyridin-6-yl)methanon;
((3R,5R)-3-Amino-5-fluorpiperidin-1-yl)(2-(1-(cyclopropylmethyl)-7-(1-(tetrahydrofuran-2-carbonyl)piperidin-4-yl)-1H-indol-2-yl)-4-methoxy-3-methylpyrazolo[1,5-a]pyridin-6-yl)methanon;
(R)-(3-Aminopiperidin-1-yl)(2-(1-(cyclopropylmethyl)-7-(1-(3-hydroxy-3-(trifluormethyl)cyclobutan-1-carbonyl)piperidin-4-yl)-1H-indol-2-yl)-4-methoxy-3-methylpyrazolo[1,5-a]pyridin-6-yl)methanon;
((3R,5R)-3-Amino-5-fluorpiperidin-1-yl)(2-(1-(cyclopropylmethyl)-7-(1-(3-hydroxy-3-(trifluormethyl)cyclobutan-1-carbonyl)piperidin-4-yl)-1H-indol-2-yl)-4-methoxy-3-methylpyrazolo[1,5-a]pyridin-6-yl)methanon;
(R)-1-(4-(2-(6-(3-Aminopiperidin-1-carbonyl)-4-methoxy-3-methylpyrazolo[1,5-a]pyridin-2-yl)-1-(cyclopropylmethyl)-1H-indol-7-yl)piperidin-1-yl)-2-methoxyethan-1-one;
(S)-1-(4-(2-(6-((3R,5R)-3-Amino-5-fluorpiperidin-1-carbonyl)-4-methoxy-3-methylpyrazolo[1,5-a]pyridin-2-yl)-1-(cyclopropylmethyl)-1H-indol-7-yl)piperidin-1-yl)-2-methoxypropan-1-one;
1-(4-(2-(6-((3R,5R)-3-Amino-5-fluorpiperidin-1-carbonyl)-4-methoxy-3-methylpyrazolo[1,5-a]pyridin-2-yl)-1-(cyclopropylmethyl)-1H-indol-7-yl)piperidin-1-yl)-2-methoxyethan-1-one;
((3R,5R)-3-Amino-5-fluorpiperidin-1-yl)(2-(1-(cyclopropylmethyl)-7-(1-((1s,4s)-4-hydroxycyclohexan-1-carbonyl)piperidin-4-yl)-1H-indol-2-yl)-4-methoxy-3-methylpyrazolo[1,5-a]pyridin-6-yl)methanon;
((R)-3-Aminopiperidin-1-yl)(2-(1-(cyclopropylmethyl)-7-(1-((1r,4r)-4-hydroxycyclohexan-1-carbonyl)piperidin-4-yl)-1H-indol-2-yl)-4-methoxy-3-methylpyrazolo[1,5-a]pyridin-6-yl)methanon;
((3R,5R)-3-Amino-5-fluorpiperidin-1-yl)(2-(1-(cyclopropylmethyl)-7-(1-((1r,4r)-4-hydroxycyclohexan-1-carbonyl)piperidin-4-yl)-1H-indol-2-yl)-4-methoxy-3-methylpyrazolo[1,5-a]pyridin-6-yl)methanon;
1-(4-(2-(6-((7R)-7-Amino-2-azabicyclo[2.2.1]heptan-2-carbonyl)-4-methoxy-3-methylpyrazolo[1,5-a]pyridin-2-yl)-1-(cyclopropylmethyl)-1H-indol-7-yl)piperidin-1-yl)ethan-1-one;
(R)-1-(4-(2-(6-(3-Aminopiperidin-1-carbonyl)-4-fluor-3-methylpyrazolo[1,5-a]pyridin-2-yl)-1-(cyclopropylmethyl)-1H-indol-7-yl)piperidin-1-yl)ethan-1-one;
(R)-1-(4-(2-(6-((3R,5R)-3-Amino-5-fluorpiperidin-1-carbonyl)-4-fluor-3-methylpyrazolo[1,5-a]pyridin-2-yl)-1-(cyclopropylmethyl)-1H-indol-7-yl)piperidin-1-yl)-2-methoxypropan-1-one;
(R)-1-(4-(2-(6-((R)-3-Aminopiperidin-1-carbonyl)-4-fluor-3-methylpyrazolo[1,5-a]pyridin-2-yl)-1-(cyclopropylmethyl)-1H-indol-7-yl)piperidin-1-yl)-2-methoxypropan-1-one;
1-(4-(2-(6-((3R,5R)-3-Amino-5-fluorpiperidin-1-carbonyl)-4-fluor-3-methylpyrazolo[1,5-a]pyridin-2-yl)-1-(cyclopropylmethyl)-1H-indol-7-yl)piperidin-1-yl)ethan-1-one;
1-(4-(2-(6-((3R,5R)-3-Amino-5-fluorpiperidin-1-carbonyl)-4-fluor-3-methylpyrazolo[1,5-a]pyridin-2-yl)-1-(cyclopropylmethyl)-1H-indol-7-yl)piperidin-1-yl)-2-methoxyethan-1-one;
1-(4-(2-(6-((3R,5R)-3-Amino-5-fluorpiperidin-1-carbonyl)-3-methylpyrazolo[1,5-a]pyridin-2-yl)-1-(cyclopropylmethyl)-1H-indol-7-yl)piperidin-1-yl)-2-hydroxyethan-1-one;
(R)-1-(4-(2-(6-(3-Aminopiperidin-1-carbonyl)-3-methylpyrazolo[1,5-a]pyridin-2-yl)-1-(cyclopropylmethyl)-1H-indol-7-yl)piperidin-1-yl)-2-hydroxyethan-1-one;
1-(4-(2-(6-((3R,5R)-3-Amino-5-fluorpiperidin-1-carbonyl)-3-methylpyrazolo[1,5-a]pyridin-2-yl)-1-(cyclopropylmethyl)-1H-indol-7-yl)piperidin-1-yl)-2-ethoxyethan-1-one;
((3R,5R)-3-Amino-5-fluorpiperidin-1-yl)(2-(1-(cyclopropylmethyl)-7-(1-((1s,4s)-4-hydroxycyclohexan-1-carbonyl)piperidin-4-yl)-1H-indol-2-yl)-3-methylpyrazolo[1,5-a]pyridin-6-yl)methanon;
((3R,5R)-3-Amino-5-fluorpiperidin-1-yl)(2-(1-(cyclopropylmethyl)-7-(1-((1R,3S)-3-hydroxycyclohexan-1-carbonyl)piperidin-4-yl)-1H-indol-2-yl)-3-methylpyrazolo[1,5-a]pyridin-6-yl)methanon;
((R)-3-Aminopiperidin-1-yl)(2-(1-(cyclopropylmethyl)-7-(1-((1s,4s)-4-hydroxycyclohexan-1-carbonyl)piperidin-4-yl)-1H-indol-2-yl)-3-methylpyrazolo[1,5-a]pyridin-6-yl)methanon;
((3R,5R)-3-Amino-5-fluorpiperidin-1-yl)(2-(1-(cyclopropylmethyl)-7-(1-((1r,4r)-4-hydroxycyclohexan-1-carbonyl)piperidin-4-yl)-1H-indol-2-yl)-3-methylpyrazolo[1,5-a]pyridin-6-yl)methanon;
1-(4-(2-(6-((3R,5R)-3-Amino-5-fluorpiperidin-1-carbonyl)-3-methylpyrazolo[1,5-a]pyridin-2-yl)-1-(cyclopropylmethyl)-1H-indol-7-yl)piperidin-1-yl)ethan-1-one;
1-(4-(2-(6-((7R)-7-Amino-2-azabicyclo[2.2.1]heptan-2-carbonyl)-3-methylpyrazolo[1,5-a]pyridin-2-yl)-1-(cyclopropylmethyl)-1H-indol-7-yl)piperidin-1-yl)-2-methoxyethan-1-one;
(R)-(3-Aminopiperidin-1-yl)(2-(1-(cyclopropylmethyl)-7-(1-(3-hydroxy-3-(trifluormethyl)cyclobutan-1-carbonyl) piperidin-4-yl)-1H-indol-2-yl)-3-methylpyrazolo[1,5-a]pyridin-6-yl)methanon;
((R)-3-Aminopiperidin-1-yl)(2-(1-(cyclopropylmethyl)-7-(1-(tetrahydro-2H-pyran-2-carbonyl)piperidin-4-yl)-1H-indol-2-yl)-3-methylpyrazolo[1,5-a]pyridin-6-yl)methanon;
((R)-3-Aminopiperidin-1-yl)(2-(1-(cyclopropylmethyl)-7-(1-(tetrahydrofuran-2-carbonyl)piperidin-4-yl)-1H-indol-2-yl)-3-methylpyrazolo[1,5-a]pyridin-6-yl)methanon;
(4-(2-(6-((7R)-7-Amino-2-azabicyclo[2.2.1]heptan-2-carbonyl)-3-methylpyrazolo[1,5-a]pyridin-2-yl)-1-(cyclopropylmethyl)-1H-indol-7-yl)piperidin-1-yl)(3-hydroxycyclobutyl)methanon;
Methyl 4-(2-(6-((7R)-7-Amino-2-azabicyclo[2.2.1]heptan-2-carbonyl)-3-methylpyrazolo[1,5-a]pyridin-2-yl)-1-(cyclopropylmethyl)-1H-indol-7-yl)piperidin-1-carboxylat;
(2R)-1-(4-(2-(6-((7R)-7-Amino-2-azabicyclo[2.2.1]heptan-2-carbonyl)-3-methylpyrazolo[1,5-a]pyridin-2-yl)-1-(cyclopropylmethyl)-1H-indol-7-yl)piperidin-1-yl)-2-hydroxypropan-1-one;
(4-(2-(6-((7R)-7-Amino-2-azabicyclo[2.2.1]heptan-2-carbonyl)-3-methylpyrazolo[1,5-a]pyridin-2-yl)-1-(cyclopropylmethyl)-1H-indol-7-yl)piperidin-1-yl)(thiophen-2-yl)methanon;
1-(4-(2-(6-((3R,5R)-3-Amino-5-Fluorpiperidin-1-carbonyl)-3-methylpyrazolo[1,5-a]pyridin-2-yl)-1-(cyclopropylmethyl)-1H-indol-7-yl)piperidin-1-yl)ethan-1-one;
1-(4-(2-(6-((7R)-7-Amino-2-azabicyclo[2.2.1]heptan-2-carbonyl)-3-methylpyrazolo[1,5-a]pyridin-2-yl)-1-(cyclopropylmethyl)-1H-indol-7-yl)piperidin-1-yl)ethan-1-one;
1-(4-(2-(6-((3R,5R)-3-Amino-5-fluorpiperidin-1-carbonyl)-3-methylpyrazolo[1,5-a]pyridin-2-yl)-1-(cyclopropylmethyl)-5-fluor-1H-indol-7-yl)piperidin-1-yl)-3-methylbutan-1-one;
((3R,5R)-3-Amino-5-fluorpiperidin-1-yl)(2-(1-(cyclopropylmethyl)-5-fluor-7-(1-(3-hydroxycyclobutan-1-carbonyl)piperidin-4-yl)-1H-indol-2-yl)-3-methylpyrazolo[1,5-a] pyridin-6-yl)methanon;
(R)-1-(4-(2-(6-((R)-3-Aminopiperidin-1-carbonyl)-3-methylpyrazolo[1,5-a]pyridin-2-yl)-1-(cyclopropylmethyl)-5-fluor-1H-indol-7-yl)piperidin-1-yl)-2-methoxypropan-1-one;
((R)-3-Aminopiperidin-1-yl)(2-(1-(cyclopropylmethyl)-5-fluor-7-(1-(tetrahydro-2H-pyran-2-carbonyl)piperidin-4-yl)-1H-indol-2-yl)-3-methylpyrazolo[1,5-a]pyridin-6-yl)methanon;
1-(4-(2-(6-((3R,5R)-3-Amino-5-fluorpiperidin-1-carbonyl)-4-methoxy-3-methylpyrazolo[1,5-a]pyridin-2-yl)-1-(cyclopropylmethyl)-1H-pyrrolo[2,3-c]pyridin-7-yl)piperidin-1-yl)-2-methoxyethan-1-one;
((3R,5R)-3-Amino-5-fluorpiperidin-1-yl)(2-(1-(cyclopropylmethyl)-7-(1-((1s,4s)-4-hydroxycyclohexan-1-carbonyl)azetidin-3-yl)-1H-indol-2-yl)-4-methoxy-3-methylpyrazolo[1,5-a]pyridin-6-yl)methanon;
1-(3-(2-(6-((3R,5R)-3-Amino-5-fluorpiperidin-1-carbonyl)-4-methoxy-3-methylpyrazolo[1,5-a]pyridin-2-yl)-1-(cyclopropylmethyl)-1H-indol-7-yl)azetidin-1-yl)-2-methoxyethan-1-one;
((R)-3-Aminopiperidin-1-yl)(2-(1-(cyclopropylmethyl)-7-(1-((1r,4r)-4-hydroxycyclohexan-1-carbonyl)azetidin-3-yl)-1H-indol-2-yl)-3-methylpyrazolo[1,5-a]pyridin-6-yl)methanon;
1-(3-(2-(6-((7R)-7-Amino-2-azabicyclo[2.2.1]heptan-2-carbonyl)-3-methylpyrazolo[1,5-a]pyridin-2-yl)-1-(cyclopropylmethyl)-1H-indol-7-yl)azetidin-1-yl)ethan-1-one;
4-(((2-(6-((R)-3-Aminopiperidin-1-carbonyl)-4-methoxy-3-methylpyrazolo[1,5-a]pyridin-2-yl)-1-(cyclopropylmethyl)-1H-indol-7-yl)oxy)methyl)pyrrolidin-2-one;
(R)-(2-(7-(2-(1H-1,2,4-Triazol-1-yl)ethoxy)-1-(cyclopropylmethyl)-1H-indol-2-yl)-4-methoxy-3-methylpyrazolo[1,5-a]pyridin-6-yl)(3-Aminopiperidin-1-yl)methanon;
(2-(7-(2-(1H-1,2,4-Triazol-1-yl)ethoxy)-1-(cyclopropylmethyl)-1H-indol-2-yl)-4-methoxy-3-methylpyrazolo[1,5-a]pyridin-6-yl)((3R,5R)-3-Amino-5-fluorpiperidin-1-yl)methanon;
(2-(7-(2-(4-Amino-1H-pyrazol-1-yl)ethoxy)-1-(cyclopropylmethyl)-1H-indol-2-yl)-4-methoxy-3-methylpyrazolo[1,5-a]pyridin-6-yl)((3R,5R)-3-Amino-5-fluorpiperidin-1-yl)methanon;
4-(((2-(6-((R)-3-Aminopiperidin-1-carbonyl)-4-methoxy-3-methylpyrazolo[1,5-a]pyridin-2-yl)-1-(cyclopropylmethyl)-1H-indol-7-yl)oxy)methyl)pyrrolidin-2-one;
4-(((2-(6-((3R,5R)-3-Amino-5-fluorpiperidin-1-carbonyl)-4-methoxy-3-methylpyrazolo[1,5-a]pyridin-2-yl)-1-(cyclopropylmethyl)-1H-indol-7-yl)oxy)m Ethyl)pyrrolidin-2-one;
4-(((2-(6-((R)-3-Aminopiperidin-1-carbonyl)-4-fluor-3-methylpyrazolo[1,5-a]pyridin-2-yl)-1-(cyclopropylmethyl)-1H-indol-7-yl)oxy)methyl)pyrrolidin-2-one;
4-(((2-(6-((3R,5R)-3-Amino-5-fluorpiperidin-1-carbonyl)-4-fluor-3-methylpyrazolo[1,5-a]pyridin-2-yl)-1-(cyclopropylmethyl)-1H-indol-7-yl)oxy)m Ethyl)pyrrolidin-2-one;
(S)-5-(((2-(6-((R)-3-Aminopiperidin-1-carbonyl)-4-fluor-3-methylpyrazolo[1,5-a]pyridin-2-yl)-1-(cyclopropylmethyl)-1H-indol-7-yl)oxy)methyl)pyrrolidin-2-one;
4-(((2-(6-((R)-3-Aminopiperidin-1-carbonyl)-4-fluor-3-methylpyrazolo[1,5-a]pyridin-2-yl)-1-(cyclopropylmethyl)-1H-indol-7-yl)oxy)methyl)pyrrolidin-2-one;
(2-(7-(2-(1H-imidazol-1-yl)ethoxy)-1-(cyclopropylmethyl)-1H-indol-2-yl)-3-methylpyrazolo[1,5-a]pyridin-6-yl)((3R,5R)-3-Amino-5-fluorpiperidin-1-yl)methanon;
(2-(7-(2-(1H-1,2,4-Triazol-1-yl)ethoxy)-1-(cyclopropylmethyl)-1H-indol-2-yl)-3-methylpyrazolo[1,5-a]pyridin-6-yl)((3R,5R)-3-Amino-5-fluorpiperidin-1-yl)methanon;
(S)-5-(((2-(6-((R)-3-Aminopiperidin-1-carbonyl)-3-methylpyrazolo[1,5-a]pyridin-2-yl)-1-(cyclopropylmethyl)-1H-indol-7-yl)oxy)methyl)pyrrolidin-2-one;
4-(((2-(6-((3R,5R)-3-Amino-5-fluorpiperidin-1-carbonyl)-3-methylpyrazolo[1,5-a]pyridin-2-yl)-1-(cyclopropylmethyl)-1H-indol-7-yl)oxy)m Ethyl)pyrrolidin-2-one;
4-(((2-(6-((R)-3-Aminopiperidin-1-carbonyl)-4-methoxy-3-methylpyrazolo[1,5-a]pyridin-2-yl)-1-(cyclopropylmethyl)-1H-pyrrolo[2,3-c]pyridin-7-yl)oxy)methyl)pyrrolidin-2-one;
(2-(7-(2-(1H-1,2,4-Triazol-1-yl)ethoxy)-1-(cyclopropylmethyl)-1H-pyrrolo[2,3-c]pyridin-2-yl)-4-methoxy-3-methylpyrazolo[1,5-a]pyridin-6-yl)((3R,5R)-3-Amino-5-fluorpiperidin-1-yl)methanon;
(2-(7-(2-(1H-Imidazol-1-yl)ethoxy)-1-(cyclopropylmethyl)-1H-pyrrolo[2,3-c]pyridin-2-yl)-4-methoxy-3-methylpyrazolo[1,5-a]pyridin-6-yl)((3R,5R)-3-Amino-5-fluorpiperidin-1-yl)methanon;
(R)-(2-(7-(2-(1H-Imidazol-1-yl)ethoxy)-1-(cyclopropylmethyl)-1H-pyrrolo[2,3-c]pyridin-2-yl)-4-methoxy-3-methylpyrazolo[1,5-a]pyridin-6-yl)(3-Aminopiperidin-1-yl)methanon;
(R)-(2-(7-(2-(4H-1,2,4-Triazol-4-yl)ethoxy)-1-(cyclopropylmethyl)-1H-pyrrolo[2,3-c]pyridin-2-yl)-4-methoxy-3-methylpyrazolo[1,5-a]pyridin-6-yl)(3-Aminopiperidin-1-yl)methanon;
(2-(7-(2-(4H-1,2,4-Triazol-4-yl)ethoxy)-1-(cyclopropylmethyl)-1H-pyrrolo[2,3-c]pyridin-2-yl)-4-methoxy-3-methylpyrazolo[1,5-a]pyridin-6-yl)((3R,5R)-3-Amino-5-fluorpiperidin-1-yl)methanon;
4-(((2-(6-((3R,5R)-3-Amino-5-fluorpiperidin-1-carbonyl)-4-methoxy-3-methylpyrazolo[1,5-a]pyridin-2-yl)-1-(cyclopropylmethyl)-1H-pyrrolo[2,3-c]pyridin-7-yl)oxy)m Ethyl)pyrrolidin-2-one;
4-(((2-(6-((3R,5R)-3-Amino-5-fluorpiperidin-1-carbonyl)-4-methoxy-3-methylpyrazolo[1,5-a]pyridin-2-yl)-1-(cyclopropylmethyl)-1H-pyrrolo[2,3-c]pyridin-7-yl)oxy)m Ethyl)pyrrolidin-2-one;
(5R)-5-(((2-(6-((7R)-7-Amino-2-azabicyclo[2.2.1]heptan-2-carbonyl)-4-methoxy-3-methylpyrazolo[1,5-a]pyridin-2-yl)-1-(cyclopropylmethyl)-1H-pyrrolo[2,3-c]pyridin-7-yl)oxy)m Ethyl)pyrrolidin-2-one;
4-(((2-(6-((7R)-7-Amino-2-azabicyclo[2.2.1]heptan-2-carbonyl)-4-methoxy-3-methylpyrazolo[1,5-a]pyridin-2-yl)-1-(cyclopropylmethyl)-1H-pyrrolo[2,3-c]pyridin-7-yl)oxy)m Ethyl)pyrrolidin-2-one;
(5R)-5-(((2-(6-((7S)-7-Amino-2-azabicyclo[2.2.1]heptan-2-carbonyl)-4-methoxy-3-methylpyrazolo[1,5-a]pyridin-2-yl)-1-(cyclopropylmethyl)-1H-pyrrolo[2,3-c]pyridin-7-yl)oxy)m Ethyl)pyrrolidin-2-one;
4-(((2-(6-((7R)-7-Amino-2-azabicyclo[2.2.1]heptan-2-carbonyl)-4-methoxy-3-methylpyrazolo[1,5-a]pyridin-2-yl)-1-(cyclopropylmethyl)-1H-pyrrolo[2,3-c]pyridin-7-yl)oxy)m Ethyl)pyrrolidin-2-one;
(R)-(2-(7-(2-(4H-1,2,4-Triazol-4-yl)ethoxy)-1-(cyclopropylmethyl)-1H-pyrrolo[2,3-c]pyridin-2-yl)-3-methylpyrazolo[1,5-a]pyridin-6-yl)(3-Aminopiperidin-1-yl)methanon;
(2-(7-(2-(1H-Imidazol-1-yl)ethoxy)-1-(cyclopropylmethyl)-1H-pyrrolo[2,3-c]pyridin-2-yl)-3-methylpyrazolo[1,5-a]pyridin-6-yl)((3R,5R)-3-Amino-5-fluorpiperidin-1-yl)methanon;
(R)-(2-(7-(2-(1H-Imidazol-1-yl)ethoxy)-1-(cyclopropylmethyl)-1H-pyrrolo[2,3-c]pyridin-2-yl)-3-methylpyrazolo[1,5-a]pyridin-6-yl)(3-Aminopiperidin-1-yl)methanon;
4-(((2-(6-((R)-3-Aminopiperidin-1-carbonyl)-3-methylpyrazolo[1,5-a]pyridin-2-yl)-1-(cyclopropylmethyl)-1H-pyrrolo[2,3-c]pyridin-7-yl)oxy)methyl)pyrrolidin-2-one;
4-(((2-(6-((R)-3-Aminopiperidin-1-carbonyl)-3-methylpyrazolo[1,5-a]pyridin-2-yl)-1-(cyclopropylmethyl)-1H-pyrrolo[2,3-c]pyridin-7-yl)oxy)methyl)pyrrolidin-2-one;
oder ein pharmazeutisch annehmbares Salz davon.

13. Pharmazeutische Zusammensetzung, umfassend eine Verbindung nach einem der Ansprüche 1-12 oder ein Stereoisomer, ein Enantiomer, ein Diastereomer, ein Tautomer oder ein pharmazeutisch annehmbares Salz davon, und einen oder mehrere pharmazeutisch annehmbare Träger.

14. Verbindung nach einem der Ansprüche 1-12 oder ein Stereoisomer, ein Enantiomer, ein Diastereomer, ein Tautomer oder ein pharmazeutisch annehmbares Salz davon oder eine Zusammensetzung nach Anspruch 13 zur Verwendung in der Therapie.

15. Verbindung nach einem der Ansprüche 1-12 oder ein Stereoisomer, ein Enantiomer, ein Diastereomer, ein Tautomer oder ein pharmazeutisch annehmbares Salz davon oder eine Zusammensetzung nach Anspruch 13, zur Verwendung bei der Behandlung einer Krankheit oder Störung, die mit der PAD4-Enzymaktivität in Zusammenhang steht, ausgewählt aus rheumatoider Arthritis, Alzheimer-Krankheit, Multipler Sklerose, Lupus, Parkinson-Krankheit und Krebs.

## Revendications

1. Composé de formule (la) : 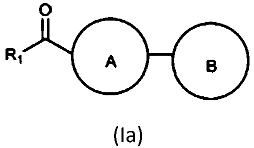 ou sel pharmaceutiquement acceptable de celui-ci, dans lequel : 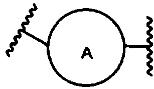 est 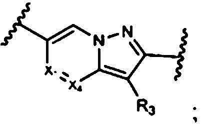 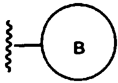 est choisi parmi 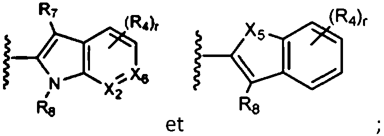
X₁ est choisi indépendamment parmi CR₂, et N ;
X₂ est choisi indépendamment parmi CR₄, et N ;
X₄ est choisi indépendamment parmi CR₂, et N ; à condition que_{X1} et_{X4} ne soient pas tous les deux N ;
X₅ est choisi indépendamment parmi O et S ;
X₆ est choisi indépendamment parmi CR₄, et N ; à condition que 1) X₂ et X₆ ne soient pas tous les deux N ; 2) lorsque X₂ et X₆ sont tous deux CR₄, l'un des R₄ est H ;
R₁ est choisi indépendamment parmi 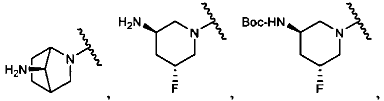 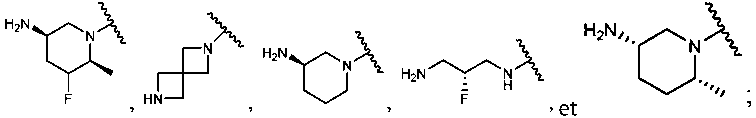
R₂ est choisi indépendamment parmi H, F, Cl, alkyle en C1-4 éventuellement substitué par un ou plusieurs substituants choisis parmi F, CI et OH, et alkyle en -OC₁₋₄ ;
R₃ est choisi indépendamment parmi H, F, Cl, CN, -C(=O)OR_{b}, et alkyle en C₁₋₃ éventuellement substitué par un ou plusieurs substituants choisis parmi F, Cl, OH, NH₂, et N₃ ;
R₄ est choisi indépendamment parmi H, F, Cl, alkyle en C₁₋₅ éventuellement substitué par un ou plusieurs substituants choisis parmi F, CI et OH, cycloalkyle en C₃₋₆, 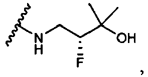 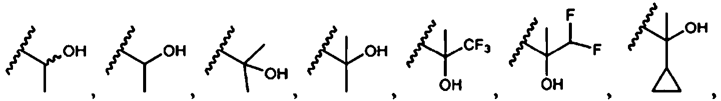 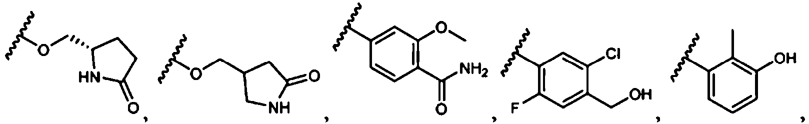 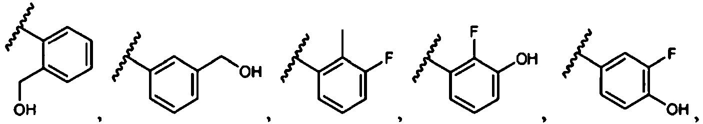 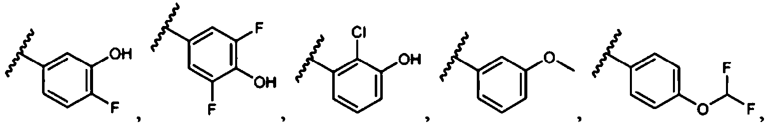 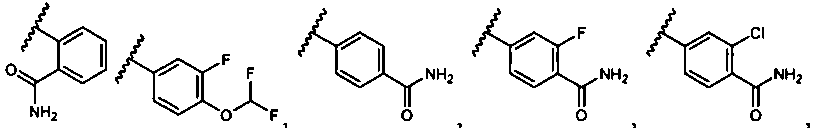 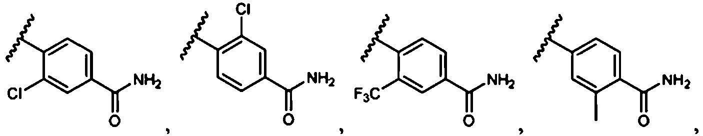 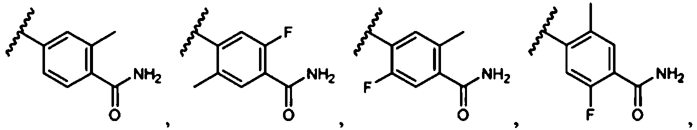 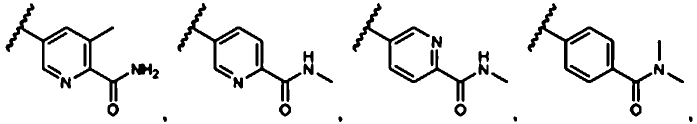 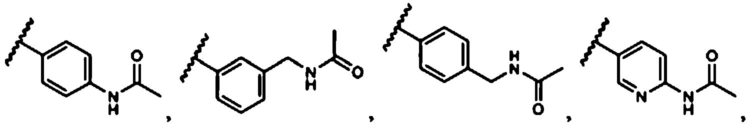 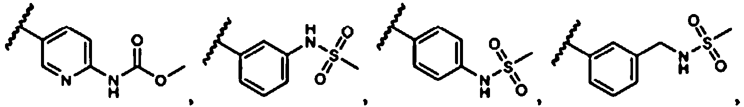 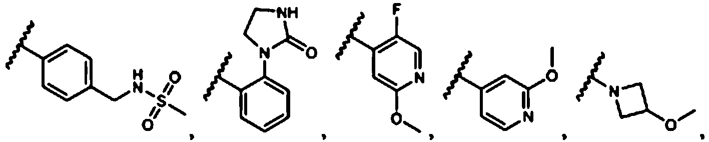 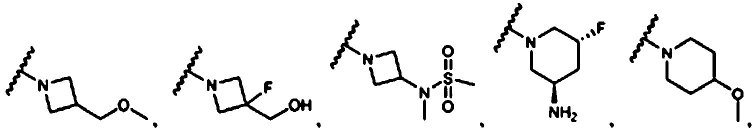 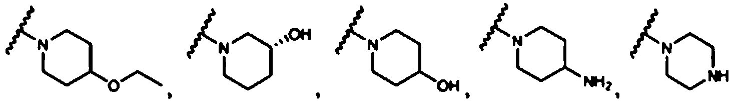 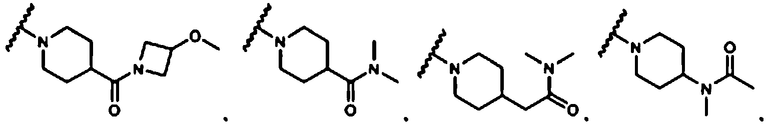 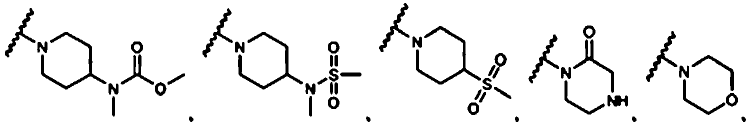 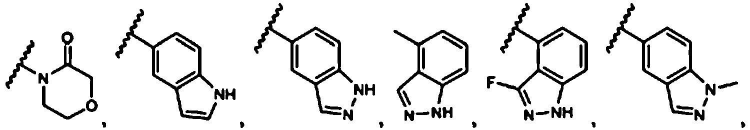 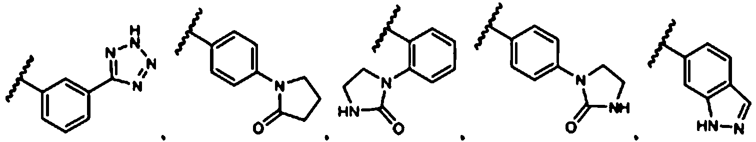 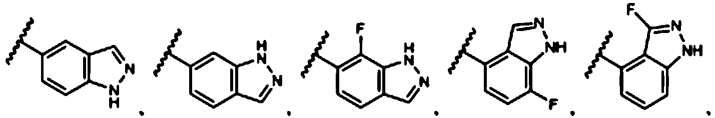 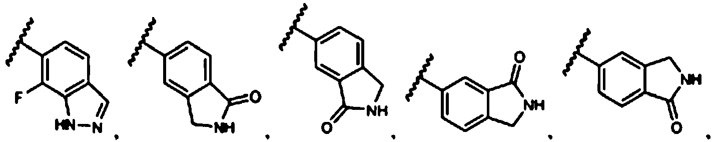 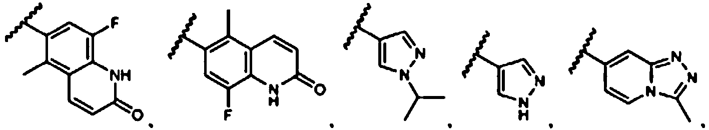 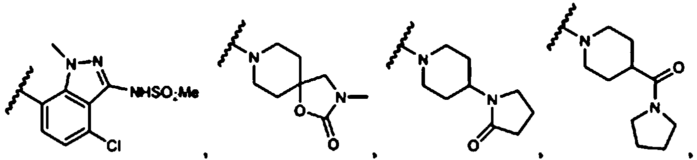 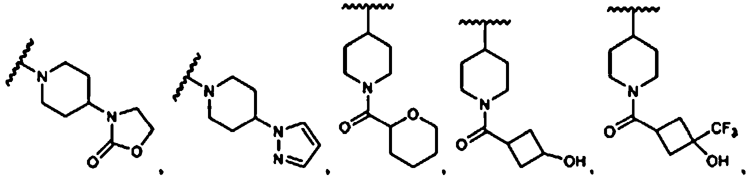 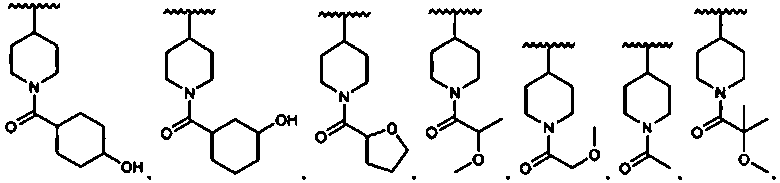 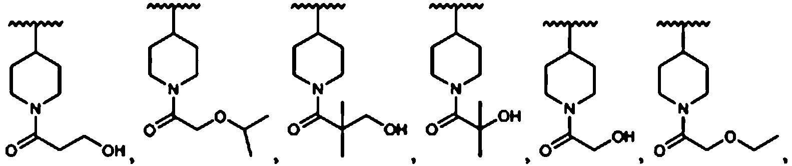 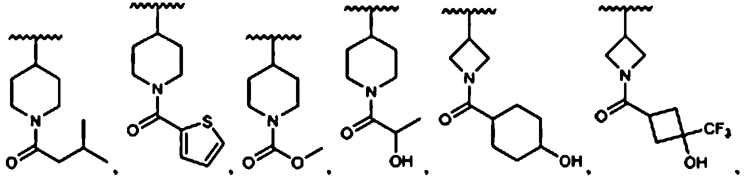 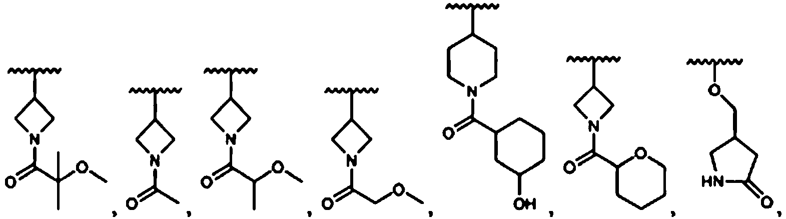 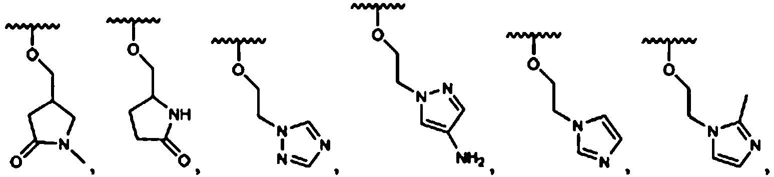 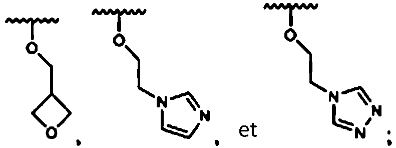
R₇ est choisi indépendamment parmi H, F et Cl ;
R₈ est choisi indépendamment parmi H, et alkyle en C₁₋₆ éventuellement substitué par un ou plusieurs substituants choisis parmi F, CI et cycloalkyle en C₃₋₆ ;
R_{b} est choisi indépendamment parmi H, alkyle en C₁₋₆ éventuellement substitué par un ou plusieurs Rₑ, alcényle en C₂₋₆ éventuellement substitué par un ou plusieurs Rₑ, alcynyle en C₂₋₆ éventuellement substitué par un ou plusieurs Rₑ, carbocyclyle en -(CH₂)ᵣ-C₃₋₁₀ éventuellement substitué par un ou plusieurs Rₑ, et hétérocyclyles en -(CH₂)ᵣéventuellement substitués par un ou plusieurs Rₑ;
Rₑ est choisi indépendamment parmi F, Cl, Br, CN, NH₂, -NH-alkyle en C₁₋₄, - N(alkyle en C₁₋₄)₂, =O, OH, alkyle en -OC₁₋₆, -CO₂H, alkyle en C₁₋₆ éventuellement substitué par un ou plusieurs R_{f}, C₂₋₆ alcényle, alcynyle en C₂₋₆, -(CH₂)ᵣ-cycloalkyle en C₃₋₆ éventuellement substitué par un ou plusieurs R_{f}, -(CH₂)ᵣ-aryle éventuellement substitué par un ou plusieurs R_{f}, et -(CH₂)ᵣ-hétérocyclyle éventuellement substitué par un ou plusieurs R_{f};
R_{f} est choisi indépendamment parmi F, Cl, Br, CN, OH, alkyle en OC₁₋₅, alkyle en C₁₋₅ éventuellement substitué par OH, alcényle en C₂₋₅, alcynyle en C₂₋₅, cycloalkyle en C₃₋₆ et phényle ; et r, à chaque occurrence, est choisi indépendamment parmi zéro, 1, 2, 3 et 4.

2. Composé selon la revendication 1, présentant les formules (Ila)-(XIII) : 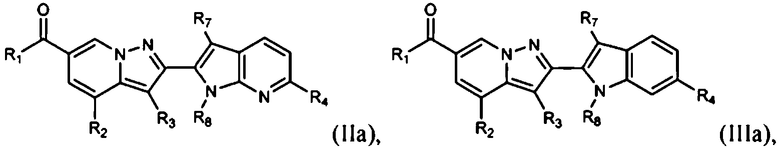 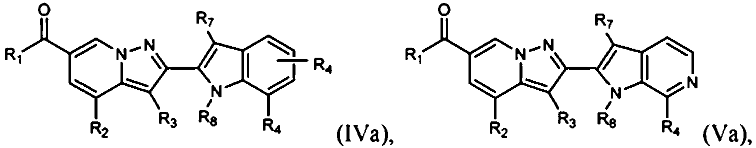 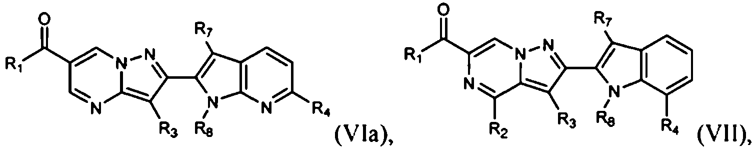 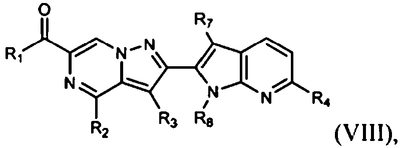 ou 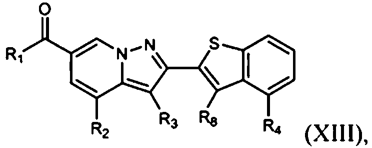 ou sel pharmaceutiquement acceptable de celui-ci.

3. Composé selon la revendication 2, présentant la formule (IIa) : 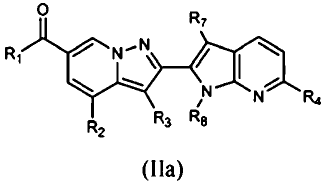 ou sel pharmaceutiquement acceptable de celui-ci, dans lequel :
R₁ est choisi indépendamment parmi 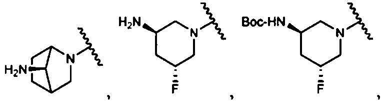 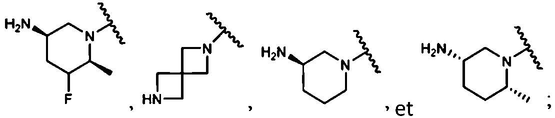
R₂ est choisi indépendamment parmi H, F, Cl, alkyle en C₁₋₃ éventuellement substitué par un ou plusieurs substituants choisis parmi F, CI et OH, et alkyle en OC₁₋₃ ; et
R₃ est choisi indépendamment parmi H, F, CI et alkyle en C₁₋₃ éventuellement substitué par un ou plusieurs substituants choisis parmi F, CI et OH.

4. Composé selon la revendication 1, présentant la formule (IIIa) : 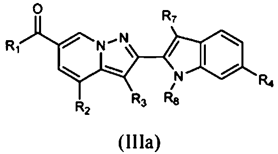 ou sel pharmaceutiquement acceptable de celui-ci, dans lequel :
R₁ est choisi indépendamment parmi 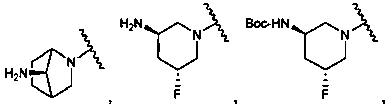 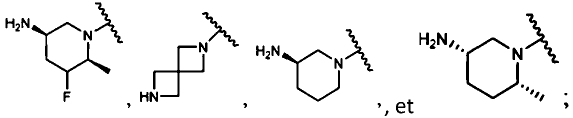
R₂ est choisi indépendamment parmi H, F, Cl, alkyle en C₁₋₃ éventuellement substitué par un ou plusieurs substituants choisis parmi F, CI et OH, et alkyle en OC₁₋₃ ; et
R₃ est choisi indépendamment parmi H, F, CI et alkyle en C₁₋₃ éventuellement substitué par un ou plusieurs substituants choisis parmi F, CI et OH.

5. Composé selon la revendication 3 ou 4, ou sel pharmaceutiquement acceptable de celui-ci, dans lequel :
R₁ est choisi indépendamment parmi 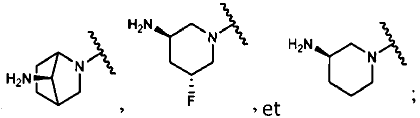
R₂ est choisi indépendamment parmi H, F, Cl, CH₃ et OCH₃ ;
R₃ est choisi indépendamment parmi H, F, Cl, CH₃ et CH₂OH ;
R₇ est H ; et
R₈ est un alkyle en C₁₋₃ substitué par un cycloalkyle en C₃₋₆.

6. Composé selon la revendication 1, présentant la formule (IVa) : 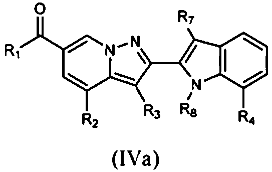 ou sel pharmaceutiquement acceptable de celui-ci, dans lequel :
R₁ est choisi indépendamment parmi 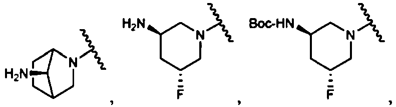 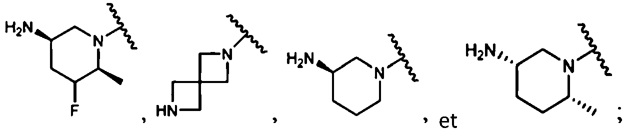
R₂ est choisi indépendamment parmi H, F, Cl, alkyle en C₁₋₃ éventuellement substitué par un ou plusieurs substituants choisis parmi F, CI et OH, et alkyle en OC₁₋₃ ;
R₃ est choisi indépendamment parmi H, F, Cl et alkyle en C₁₋₃ éventuellement substitué par un ou plusieurs substituants choisis parmi F, CI et OH ;
de préférence dans lequel :
R₁ est choisi indépendamment parmi 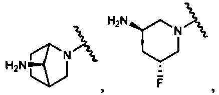 et 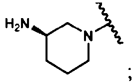
R₂ est choisi indépendamment parmi H, F, CI et OCH₃ ;
R₃ est choisi indépendamment parmi F, CI et CH₃ ;
R7 est H ; et
R₈ est un alkyle en C₁₋₂ éventuellement substitué par un cyclopropyle ;
de préférence dans lequel :
R₄ est choisi indépendamment parmi

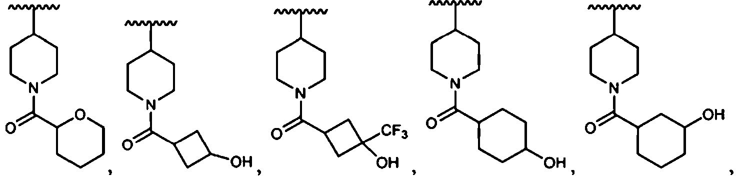

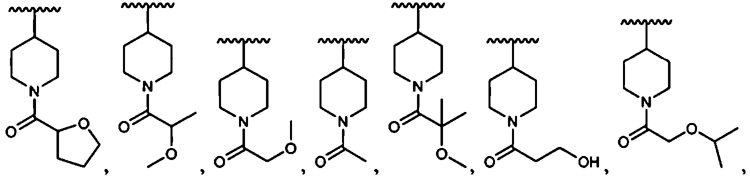

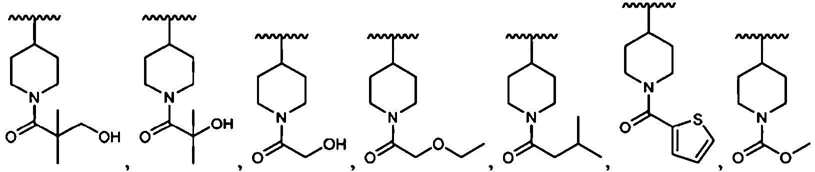

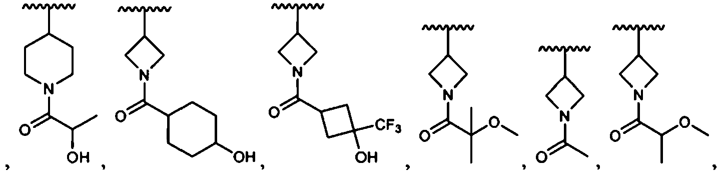

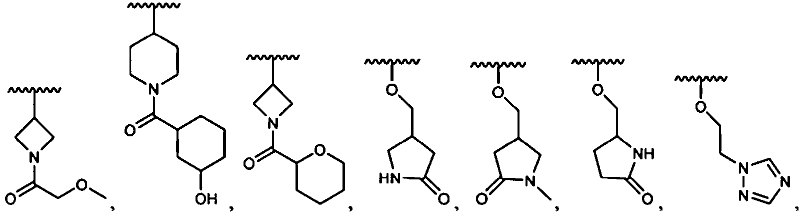

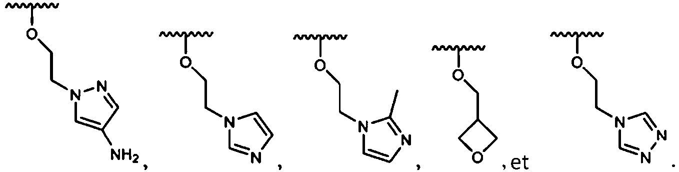

7. Composé selon la revendication 1, présentant la formule (Va) : 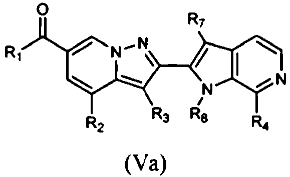 ou sel pharmaceutiquement acceptable de celui-ci, dans lequel :
R₁ est choisi indépendamment parmi 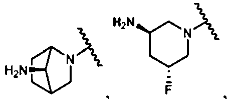 et 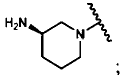
R₂ est choisi indépendamment parmi H, F, CI, CH₃ et OCH₃ ;
R₃ est choisi indépendamment parmi H, F, CI, CN, CH₃ et CH₂OH ;
de préférence dans lequel :
R₁ est choisi indépendamment parmi 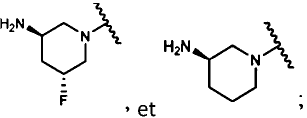
R₂ est CH₃ ;
R3 est CH3 ;
R₄ est choisi indépendamment parmi 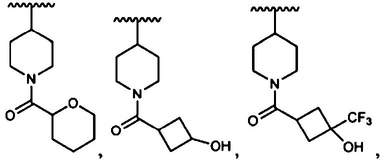 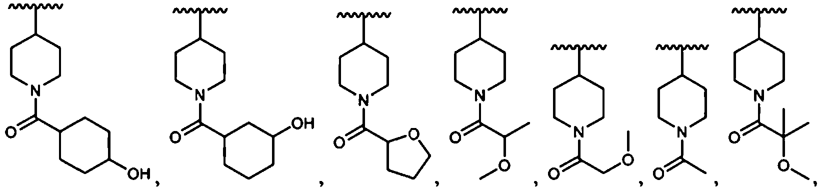 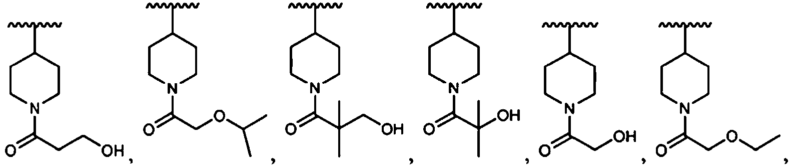 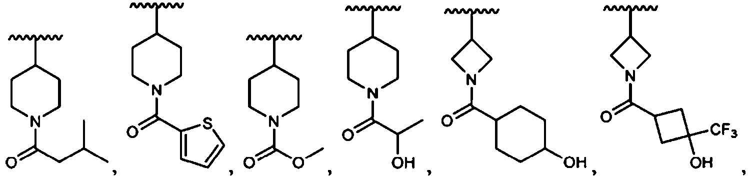 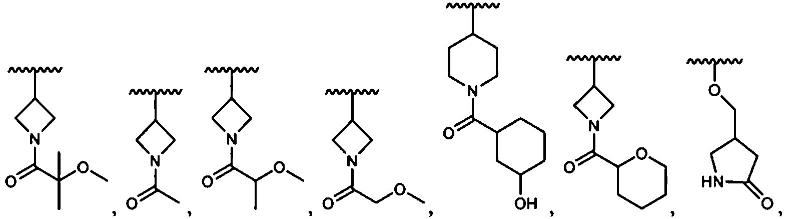 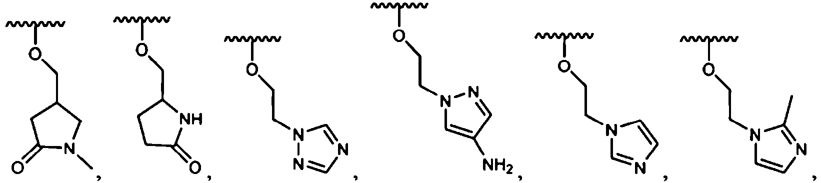 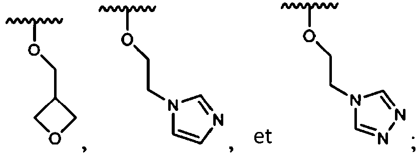
R₇ est H ; et
R₈ est un alkyle en C₁₋₂ éventuellement substitué par un cyclopropyle.

8. Composé selon la revendication 1, présentant la formule (VIa) : 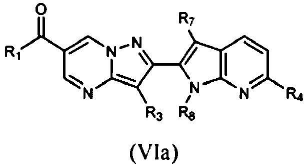 ou sel pharmaceutiquement acceptable de celui-ci, dans lequel :
R₁ est choisi indépendamment parmi 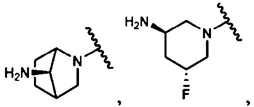 et 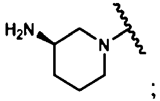 et
R₃ est choisi indépendamment parmi H, F et CH₃ ;
de préférence dans lequel :
R₁ est choisi indépendamment parmi 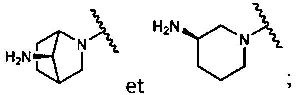
R3 est CH₃ ;
R₄ est choisi indépendamment parmi F, Cl, 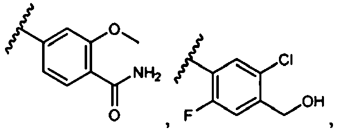 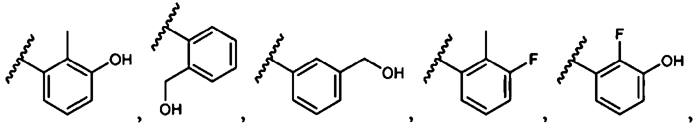 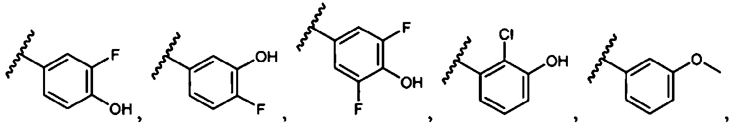 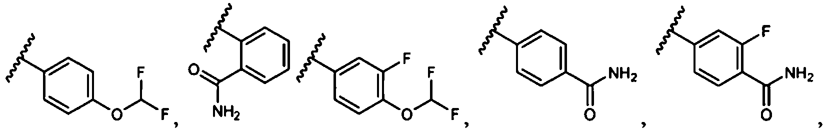 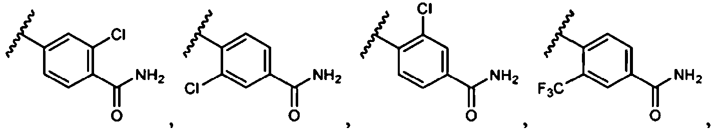 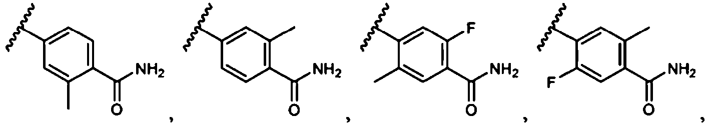 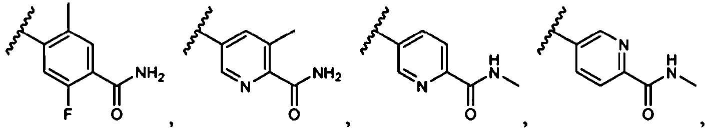 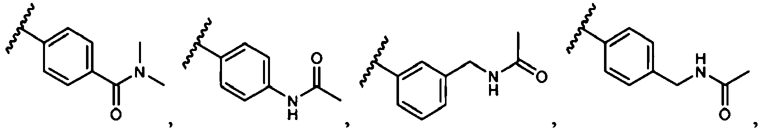 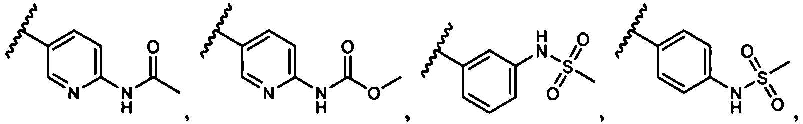 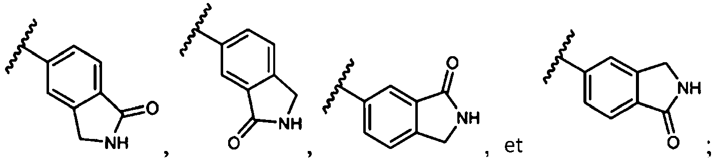
R₇ est H ; et
R₈ est un alkyle en C₁₋₂ éventuellement substitué par un cyclopropyle.

9. Composé selon la revendication 1, présentant la formule (VII) : 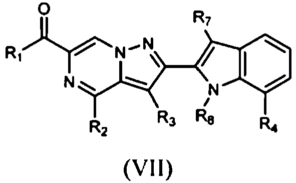 ou sel pharmaceutiquement acceptable de celui-ci, dans lequel :
R₁ est choisi indépendamment parmi 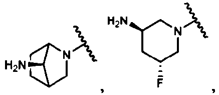 et 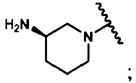
R₂ est choisi indépendamment parmi H, F, CI, CH₃ et OCH₃ ; et
R₃ est choisi indépendamment parmi H, F et CH₃.

10. Composé selon la revendication 1, présentant la formule (VIII) : 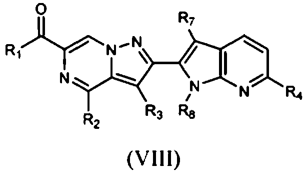 ou sel pharmaceutiquement acceptable de celui-ci, dans lequel :
R₁ est choisi indépendamment parmi 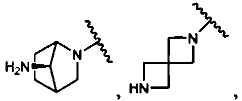 et 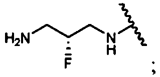
R₂ est choisi indépendamment parmi H, F et OCH₃ ; et
R₃ est choisi indépendamment parmi H, F et CH₃.

11. Le composé selon la revendication 1, présentant la formule (XIII) : 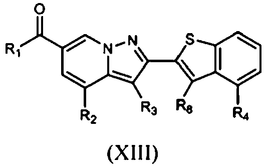 ou sel pharmaceutiquement acceptable de celui-ci, dans lequel :
R₁ est choisi indépendamment parmi 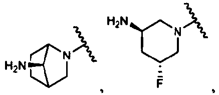 et 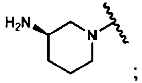
R₂ est choisi indépendamment parmi H, F, CH₃ et OCH₃ ; et
R₃ est choisi indépendamment parmi H, F et CH₃.

12. Composé selon la revendication 1, choisi parmi :
5-(2-(6-((7R)-7-Amino-2-azabicyclo[2.2.1]-heptane-2-carbonyl)-4-méthoxy-3-méthylpyrazolo[1,5-a]pyridin-2-yl)-1-(cyclopropylméthyl)-1H-pyrrolo[2,3-b]pyridin-6-yl)isoindolin-1-one ;
((7R)-7-Amino-2-azabicyclo[2.2.1]heptan-2-yl)(2-(6-cyclopropyl-1-(cyclopropylméthyl)-1H-pyrrolo[2,3-b]pyridin-2-yl)-4-méthoxy-3-méthylpyrazolo[1,5-a]pyridin-6-yl)méthanone ;
5-(2-(6-((7R)-7-Amino-2-azabicyclo[2.2.1]heptane-2-carbonyl)-4-méthoxy-3-méthylpyrazolo[1,5-a]pyridin-2-yl)-1-(cyclopropylméthyl)-1H-indol-6-yl)isoindolin-1-one ;
((7R)-7-Amino-2-azabicyclo[2.2.1]heptan-2-yl)(2-(1-(cyclopropylméthyl)-6-(3-méthyl-[1,2,4]triazolo[4,3-a]pyridin-7-yl)-1H-indol-2-yl)-4-méthoxy-3-méthylpyrazolo[1,5-a]pyridin-6-yl)méthanone ;
((7R)-7-Amino-2-azabicyclo[2.2.1]heptan-2-yl)(2-(1-(cyclopropylméthyl)-6-méthyl-1H-indol-2-yl)-4-méthoxy-3-méthylpyrazolo[1,5-a]pyridin-6-yl)méthanone ;
3((7R)-7-Amino-2-azabicyclo[2.2.1]heptan-2-yl)(2-(1-(cyclopropylméthyl)-6-méthyl-1H-pyrrolo[2,3-b]pyridin-2-yl)-4-fluoro-3-méthylpyrazolo[I,5-a]pyridin-6-yl)méthanone ;
5-(2-(6-((7R)-7-Amino-2-azabicyclo[2.2.1]heptane-2-carbonyl)-4-fluoro-3-méthylpyrazolo[1,5-a]pyridin-2-yl)-1-(cyclopropylméthyl)-1H-pyrrolo[2,3-b]pyridin-6-yl)isoindolin-1-one ;
6-(2-(6-((7R)-7-Amino-2-azabicyclo[2.2.1]heptane-2-carbonyl)-4-fluoro-3-méthylpyrazolo[1,5-a]pyridin-2-yl)-1-(cyclopropylméthyl)-IH-pyrrolo[2,3-b]pyridin-6-yl)isoindolin-1-one ;
((7R)-7-Amino-2-azabicyclo[2.2.1]heptan-2-yl)(2-(1-(cyclopropylméthyl)-6-(2-fluoro-3-hydroxyphényl)-1H-pyrrolo[2,3-b]pyridin-2-yl)-4-fluoro-3-méthylpyrazolo[1,5-a]pyridin-6-yl)méthanone ;
((7R)-7-Amino-2-azabicyclo[2.2.1]heptan-2-yl)(2-(1-(cyclopropylméthyl)-6-(3-méthyl-[1,2,4]triazolo[4,3-a]pyridin-7-yl)-1H-indol-2-yl)-4-fluoro-3-méthylpyrazolo[1,5-a]pyridin-6-yl)méthanone ;
6-(2-(6-((7R)-7-Amino-2-azabicyclo[2.2.1]heptane-2-carbonyl)-3-méthylpyrazolo[1,5-a]pyridin-2-yl)-1-(cyclopropylméthyl)-1H-pyrrolo[2,3-b]pyridin-6-yl)isoindolin-1-one ;
((7R)-7-Amino-2-azabicyclo[2.2.1]heptan-2-yl)(2-(1-(cyclopropylméthyl)-6-(3-fluoro-4-hydroxyphényl)-1H-pyrrolo[2,3-b]pyridin-2-yl)-3-méthylpyrazolo[1,5-a]pyridin-6-yl)méthanone ;
((7R)-7-Amino-2-azabicyclo[2.2.1]heptan-2-yl)(2-(1-(cyclopropylméthyl)-6-(2-fluoro-3-hydroxyphényl)-1H-pyrrolo[2,3-b]pyridin-2-yl)-3-méthylpyrazolo[1,5-a]pyridin-6-yl)méthanone ;
4-(2-(6-((7R)-7-Amino-2-azabicyclo[2.2.1]heptane-2-carbonyl)-3-méthylpyrazolo[1,5-a]pyridin-2-yl)-1-(cyclopropylméthyl)-1H-pyrrolo[2,3-b]pyridin-6-yl)-2-fluorobenzamide ;
((7R)-7-Amino-2-azabicyclo[2.2.1]heptan-2-yl)(2-(1-(cyclopropylméthyl)-6-(3-méthyl-[1,2,4]triazolo[4,3-a]pyridin-7-yl)-1H-pyrrolo[2,3-b]pyridin-2-yl)-3-méthylpyrazolo[1,5-a]pyridin-6-yl)méthanone ;
((7R)-7-Amino-2-azabicyclo[2.2.1]heptan-2-yl)(2-(6-cyclopropyl-1-(cyclopropylméthyl)-1H-pyrrolo[2,3-b]pyridin-2-yl)-3-méthylpyrazolo[1,5-a]pyridin-6-yl)méthanone ;
6-(2-(6-((7R)-7-Amino-2-azabicyclo[2.2.1]heptane-2-carbonyl)-3-méthylpyrazolo[1,5-a]pyridin-2-yl)-1-(cyclopropylméthyl)-1H-indol-6-yl)isoindolin-1-one ;
5-(2-(6-((7R)-7-Amino-2-azabicyclo[2.2.1]heptane-2-carbonyl)-3-méthylpyrazolo[1,5-a]pyridin-2-yl)-1-(cyclopropylméthyl)-1H-indol-6-yl)isoindolin-1-one ;
((7R)-7-Amino-2-azabicyclo[2.2.1]heptan-2-yl)(2-(1-(cyclopropylméthyl)-6-(2-hydroxypropan-2-yl)-1H-indol-2-yl)-4-méthoxy-3-méthylpyrazolo[1,5-a]pyridin-6-yl)méthanone ;
((3R,5R)-3-Amino-5-fluoropipéridin-1-yl)(2-(1-(cyclopropylméthyl)-6-(2-hydroxypropan-2-yl)-1H-indol-2-yl)-4-méthoxy-3-méthylpyrazolo[1,5-a]pyridin-6-yl)méthanone ;
((7R)-7-Amino-2-azabicyclo[2.2.1]heptan-2-yl)(2-(1-(cyclopropylméthyl)-6-(2-hydroxypropan-2-yl)-1H-indol-2-yl)-3-méthylpyrazolo[1,5-a]pyridin-6-yl)méthanone ;
((7R)-7-Amino-2-azabicyclo[2.2.1]heptan-2-yl)(2-(1-(cyclopropylméthyl)-6-(1,1,1-trifluoro-2-hydroxypropan-2-yl)-1H-indol-2-yl)-3-méthylpyrazolo[1,5-a]pyridin-6-yl)méthanone ;
((7R)-7-Amino-2-azabicyclo[2.2.1]heptan-2-yl)(2-(1-(cyclopropylméthyl)-6-(2-hydroxypropan-2-yl)-1H-pyrrolo[2,3-b]pyridin-2-yl)-3-méthylpyrazolo[1,5-a]pyridin-6-yl)méthanone ;
((7R)-7-Amino-2-azabicyclo[2.2.1]heptan-2-yl)(2-(1-(cyclopropylméthyl)-6-(1,1,1-trifluoro-2-hydroxypropan-2-yl)-1H-pyrrolo[2,3-b]pyridin-2-yl)-3-méthylpyrazolo[1,5-a]pyridin-6-yl)méthanone ;
((7R)-7-Amino-2-azabicyclo[2.2.1]heptan-2-yl)(2-(1-(cyclopropylméthyl)-6-(1-hydroxyéthyl)-1H-pyrrolo[2,3-b]pyridin-2-yl)-3-méthylpyrazolo[1,5-a]pyridin-6-yl)méthanone ;
N-[5-(2-{6-[(3R,5R)-3-Amino-5-fluoropipéridine-1-carbonyl]-4-méthoxy-3-méthylpyrazolo[1,5-a]pyridin-2-yl}-1-(cyclopropylméthyl)-1H-indol-6-yl)pyridin-2-yl]acétamide ;
(3R,5R)-1-{2-[1-(Cyclopropylméthyl)-6-(4-méthanesulfonylpipéridin-1-yl)-1H-indol-2-yl]-4-méthoxy-3-méthylpyrazolo[1,5-a]pyridine-6-carbonyl}-5-fluoropipéridin-3-amine ;
3-(2-{6-[(7R)-7-Amino-2-azabicyclo[2.2.1]heptane-2-carbonyl]-4-fluoro-3-méthylpyrazolo[1,5-a]pyridin-2-yl}-1-(cyclopropylméthyl)-1H-pyrrolo[2,3-b]pyridin-6-yl)-2-méthylphénol ;
5-(2-{6-[(7R)-7-Amino-2-azabicyclo[2.2.1]heptane-2-carbonyl]-4-fluoro-3-méthylpyrazolo[1,5-a]pyridin-2-yl}-1-(cyclopropylméthyl)-1H-pyrrolo[2,3-b]pyridin-6-yl)-N-méthylpyridine-2-carboxamide ;
4-(2-{6-[(7R)-7-Amino-2-azabicyclo[2.2.1]heptane-2-carbonyl]-4-fluoro-3-méthylpyrazolo[1,5-a]pyridin-2-yl}-1-(cyclopropylméthyl)-1H-pyrrolo[2,3-b]pyridin-6-yl)morpholin-3-one ;
(7R)-2-{2-[1-(Cyclopropylméthyl)-6-(4-méthanesulfonylpipéridin-1-yl)-1H-pyrrolo[2,3-b]pyridin-2-yl]-4-fluoro-3-méthylpyrazolo[1,5-a]pyridine-6-carbonyl}-2-azabicyclo[2.2.1]heptan-7-amine ;
(7R)-2-{2-[1-(Cyclopropylméthyl)-6-{3-méthyl-[1,2,4]triazolo[4,3-a]pyridin-7-yl}-1H-indol-2-yl]-4-fluoro-3-méthylpyrazolo[1,5-a]pyridine-6-carbonyl}-2-azabicyclo[2.2.1]heptan-7-amine ;
(7R)-2-{2-[1-(Cyclopropylméthyl)-6-(1H-indazol-4-yl)-1H-pyrrolo[2,3-b]pyridin-2-yl]-3-méthylpyrazolo[1,5-a]pyridine-6-carbonyl}-2-azabicyclo[2.2.1]heptan-7-amine ;
3-(2-{6-[(7R)-7-Amino-2-azabicyclo[2.2.1]heptane-2-carbonyl]-3-méthylpyrazolo[1,5-a]pyridin-2-yl}-1-(cyclopropylméthyl)-1H-pyrrolo[2,3-b]pyridin-6-yl)-2-méthylphénol ;
1-[4-(2-{6-[(7R)-7-Amino-2-azabicyclo[2.2.1]heptane-2-carbonyl]-3-méthylpyrazolo[1,5-a]pyridin-2-yl}-1-(cyclopropylméthyl)-1H-pyrrolo[2,3-b]pyridin-6-yl)phényl]pyrrolidin-2-one ;
4-(2-{6-[(7R)-7-Amino-2-azabicyclo[2.2.1]heptane-2-carbonyl]-3-méthylpyrazolo[1,5-a]pyridin-2-yl}-1-(cyclopropylméthyl)-1H-pyrrolo[2,3-b]pyridin-6-yl)-2-fluoro-5-méthylbenzamide ;
(7R)-2-{2-[1-(Cyclopropylméthyl)-6-(7-fluoro-1H-indazol-6-yl)-1H-pyrrolo[2,3-b]pyridin-2-yl]-3-méthylpyrazolo[1,5-a]pyridine-6-carbonyl}-2-azabicyclo[2.2.1]heptan-7-amine ;
Méthyle N-[5-(2-{6-[(7R)-7-amino-2-azabicyclo[2.2.1]heptane-2-carbonyl]-3-méthylpyrazolo[1,5-a]pyridin-2-yl}-1-(cyclopropylméthyl)-1H-pyrrolo[2,3-b]pyridin-6-yl)pyridin-2-yl]carbamate ;
4-(2-{6-[(7R)-7-Amino-2-azabicyclo[2.2.1]heptane-2-carbonyl]-3-méthylpyrazolo[1,5-a]pyridin-2-yl}-1-(cyclopropylméthyl)-1H-pyrrolo[2,3-b]pyridin-6-yl)morpholin-3-one ;
1-[4-(2-{6-[(7R)-7-Amino-2-azabicyclo[2.2.1]heptane-2-carbonyl]-3-méthylpyrazolo[1,5-a]pyridin-2-yl}-1-(cyclopropylméthyl)-1H-indol-6-yl)phényl]pyrrolidin-2-one ;
(7R)-2-{2-[1-(Cyclopropylméthyl)-6-(1H-indazol-6-yl)-1H-indol-2-yl]-3-méthylpyrazolo[1,5-a]pyridine-6-carbonyl}-2-azabicyclo[2.2.1]heptan-7-amine ;
(7R)-2-{2-[1-(Cyclopropylméthyl)-6-(1H-indazol-5-yl)-1H-indol-2-yl]-3-méthylpyrazolo[1,5-a]pyridine-6-carbonyl}-2-azabicyclo[2.2.1]heptan-7-amine ;
N-[7-(2-{6-[(7R)-7-Amino-2-azabicyclo[2.2.1]heptane-2-carbonyl]-3-méthylpyrazolo[1,5-a]pyridin-2-yl}-1-(cyclopropylméthyl)-1H-indol-6-yl)-4-chloro-1-méthyl-IH-indazol-3-yl]méthanesulfonamide ;
1-[2-(2-{6-[(7R)-7-Amino-2-azabicyclo[2.2.1]heptane-2-carbonyl]-3-méthylpyrazolo[1,5-a]pyridin-2-yl}-1-(cyclopropylméthyl)-1H-indol-6-yl)phényl]imidazolidin-2-one ;
4-(2-{6-[(7R)-7-Amino-2-azabicyclo[2.2.1]heptane-2-carbonyl]-3-méthylpyrazolo[1,5-a]pyridin-2-yl}-1-(cyclopropylméthyl)-1H-indol-6-yl)-2-chlorobenzamide ;
(7R)-2-{2-[1-(Cyclopropylméthyl)-6-(1H-pyrazol-4-yl)-1H-indol-2-yl]-3-méthylpyrazolo[1,5-a]pyridine-6-carbonyl}-2-azabicyclo[2.2.1]heptan-7-amine ;
(7R)-2-{2-[1-(Cyclopropylméthyl)-6-(morpholin-4-yl)-1H-indol-2-yl]-3-méthylpyrazolo[1,5-a]pyridine-6-carbonyl}-2-azabicyclo[2.2.1]heptan-7-amine ;
(3R,5R)-1-{2-[1-(Cyclopropylméthyl)-6-[4-(pyrrolidine-1-carbonyl)pipéridin-1-yl]-1H-indol-2-yl]-3-méthylpyrazolo[1,5-a]pyridine-6-carbonyl}-5-fluoropipéridin-3-amine ;
3-[1-(2-{6-[(3R,5R)-3-Amino-5-fluoropipéridine-1-carbonyl]-3-méthylpyrazolo[1,5-a]pyridin-2-yl}-1-(cyclopropylméthyl)-1H-indol-6-yl)pipéridin-4-yl]-1,3-oxazolidin-2-one ;
2-(2-{6-[(7R)-7-Amino-2-azabicyclo[2.2.1]heptane-2-carbonyl]-3-méthylpyrazolo[1,5-a]pyridin-2-yl}-1-(cyclopropylméthyl)-1H-pyrrolo[2,3-b]pyridin-6-yl)-1,1-difluoropropan-2-ol ;
2-(2-{6-[(7R)-7-Amino-2-azabicyclo[2.2.1]heptane-2-carbonyl]-4-fluoro-3-méthylpyrazolo[1,5-a]pyridin-2-yl}-1-(cyclopropylméthyl)-1H-pyrrolo[2,3-b]pyridin-6-yl)-1,1,1-trifluoropropan-2-ol ;
((3R,5R)-3-Amino-5-fluoropipéridin-1-yl)(2-(1-(cyclopropylméthyl)-7-(1-(tétrahydro-2H-pyran-2-carbonyl)pipéridin-4-yl)-1H-indol-2-yl)-4-méthoxy-3-méthylpyrazolo[1,5-a]pyridin-6-yl)méthanone ;
((3R,5R)-3-Amino-5-fluoropipéridin-1-yl)(2-(1-(cyclopropylméthyl)-7-(1-(3-hydroxycyclobutane-1-carbonyl)pipéridin-4-yl)-1H-indol-2-yl)-4-méthoxy-3-méthylpyrazolo[1,5-a]pyridin-6-yl)méthanone ;
((3R,5R)-3-Amino-5-fluoropipéridin-1-yl)(2-(1-(cyclopropylméthyl)-7-(1-((1r,4r)-4-hydroxycyclohexane-1-carbonyl)pipéridin-4-yl)-1H-indol-2-yl)-4-méthoxy-3-méthylpyrazolo[1,5-a]pyridin-6-yl)méthanone ;
(R)-(3-Aminopipéridin-1-yl)(2-(1-(cyclopropylméthyl)-7-(1-(3-hydroxycyclobutane-1-carbonyl)pipéridin-4-yl)-1H-indol-2-yl)-4-méthoxy-3-méthylpyrazolo[1,5-a]pyridin-6-yl)méthanone ;
((3R,5R)-3-Amino-5-fluoropipéridin-1-yl)(2-(1-(cyclopropylméthyl)-7-(1-(tétrahydrofuran-2-carbonyl)pipéridin-4-yl)-1H-indol-2-yl)-4-méthoxy-3-méthylpyrazolo[1,5-a]pyridin-6-yl)méthanone ;
((3R,5R)-3-Amino-5-fluoropipéridin-1-yl)(2-(1-(cyclopropylméthyl)-7-(1-(tétrahydrofuran-2-carbonyl)pipéridin-4-yl)-1H-indol-2-yl)-4-méthoxy-3-méthylpyrazolo[1,5-a]pyridin-6-yl)méthanone ;
(R)-(3-Aminopipéridin-1-yl)(2-(1-(cyclopropylméthyl)-7-(1-(3-hydroxy-3-(trifluorométhyl)cyclobutane-1-carbonyl)pipéridin-4-yl)-1H-indol-2-yl)-4-méthoxy-3-méthylpyrazolo[1,5-a]pyridin-6-yl)méthanone ;
((3R,5R)-3-Amino-5-fluoropipéridin-1-yl)(2-(1-(cyclopropylméthyl)-7-(1-(3-hydroxy-3-(trifluorométhyl)cyclobutane-1-carbonyl)pipéridin-4-yl)-1H-indol-2-yl)-4-méthoxy-3-méthylpyrazolo[1,5-a]pyridin-6-yl)méthanone ;
(R)-1-(4-(2-(6-(3-Aminopipéridine-1-carbonyl)-4-méthoxy-3-méthylpyrazolo[1,5-a]pyridin-2-yl)-1-(cyclopropylméthyl)-1H-indol-7-yl)pipéridin-1-yl)-2-méthoxyéthan-1-one ;
(S)-1-(4-(2-(6-((3R,5R)-3-Amino-5-fluoropipéridine-1-carbonyl)-4-méthoxy-3-méthylpyrazolo[1,5-a]pyridin-2-yl)-1-(cyclopropylméthyl)-1H-indol-7-yl)pipéridin-1-yl)-2-méthoxypropan-1-one ;
1-(4-(2-(6-((3R,5R)-3-Amino-5-fluoropipéridine-1-carbonyl)-4-méthoxy-3-méthylpyrazolo[1,5-a]pyridin-2-yl)-1-(cyclopropylméthyl)-1H-indol-7-yl)pipéridin-1-yl)-2-méthoxyéthan-1-one ;
((3R,5R)-3-Amino-5-fluoropipéridin-1-yl)(2-(1-(cyclopropylméthyl)-7-(1-((1s,4s)-4-hydroxycyclohexane-1-carbonyl)pipéridin-4-yl)-1H-indol-2-yl)-4-méthoxy-3-méthylpyrazolo[1,5-a]pyridin-6-yl)méthanone ;
((R)-3-Aminopipéridin-1-yl)(2-(1-(cyclopropylméthyl)-7-(1-((1r,4r)-4-hydroxycyclohexane-1-carbonyl)pipéridin-4-yl)-1H-indol-2-yl)-4-méthoxy-3-méthylpyrazolo[1,5-a]pyridin-6-yl)méthanone ;
((3R,5R)-3-Amino-5-fluoropipéridin-1-yl)(2-(1-(cyclopropylméthyl)-7-(1-((1r,4r)-4-hydroxycyclohexane-1-carbonyl)pipéridin-4-yl)-1H-indol-2-yl)-4-méthoxy-3-méthylpyrazolo[1,5-a]pyridin-6-yl)méthanone ;
1-(4-(2-(6-((7R)-7-Amino-2-azabicyclo[2.2.1]heptane-2-carbonyl)-4-méthoxy-3-méthylpyrazolo[1,5-a]pyridin-2-yl)-1-(cyclopropylméthyl)-1H-indol-7-yl)pipéridin-1-yl)éthan-1-one ;
(R)-1-(4-(2-(6-(3-Aminopipéridine-1-carbonyl)-4-fluoro-3-méthylpyrazolo[1,5-a]pyridin-2-yl)-1-(cyclopropylméthyl)-1H-indol-7-yl)pipéridin-1-yl)éthan-1-one ;
(R)-1-(4-(2-(6-((3R,5R)-3-Amino-5-fluoropipéridine-1-carbonyl)-4-fluoro-3-méthylpyrazolo[1,5-a]pyridin-2-yl)-1-(cyclopropylméthyl)-1H-indol-7-yl)pipéridin-1-yl)-2-méthoxypropan-1-one ;
(R)-1-(4-(2-(6-((R)-3-Aminopipéridine-1-carbonyl)-4-fluoro-3-méthylpyrazolo[1,5-a]pyridin-2-yl)-1-(cyclopropylméthyl)-1H-indol-7-yl)pipéridin-1-yl)-2-méthoxypropan-1-one ;
1-(4-(2-(6-((3R,5R)-3-Amino-5-fluoropipéridine-1-carbonyl)-4-fluoro-3-méthylpyrazolo[1,5-a]pyridin-2-yl)-1-(cyclopropylméthyl)-1H-indol-7-yl)pipéridin-1-yl)éthan-1-one ;
1-(4-(2-(6-((3R,5R)-3-Amino-5-fluoropipéridine-1-carbonyl)-4-fluoro-3-méthylpyrazolo[1,5-a]pyridin-2-yl)-1-(cyclopropylméthyl)-1H-indol-7-yl)pipéridin-1-yl)-2-méthoxyéthan-1-one ;
1-(4-(2-(6-((3R,5R)-3-amino-5-fluoropipéridine-1-carbonyl)-3-méthylpyrazolo[1,5-a]pyridin-2-yl)-1-(cyclopropylméthyl)-1H-indol-7-yl)pipéridin-1-yl)-2-hydroxyéthan-1-one ;
(R)-1-(4-(2-(6-(3-aminopipéridine-1-carbonyl)-3-méthylpyrazolo[1,5-a]pyridin-2-yl)-1-(cyclopropylméthyl)-1H-indol-7-yl)pipéridin-1-yl)-2-hydroxyéthan-1-one ;
1-(4-(2-(6-((3R,5R)-3-amino-5-fluoropipéridine-1-carbonyl)-3-méthylpyrazolo[1,5-a]pyridin-2-yl)-1-(cyclopropylméthyl)-1H-indol-7-yl)pipéridin-1-yl)-2-éthoxyéthan-1-one ;
((3R,5R)-3-amino-5-fluoropipéridin-1-yl)(2-(1-(cyclopropylméthyl)-7-(1-((1s,4s)-4-hydroxycyclohexane-1-carbonyl)pipéridin-4-yl)-1H-indol-2-yl)-3-méthylpyrazolo[1,5-a]pyridin-6-yl)méthanone ;
((3R,5R)-3-amino-5-fluoropipéridin-1-yl)(2-(1-(cyclopropylméthyl)-7-(1-((1R,3S)-3-hydroxycyclohexane-1-carbonyl)pipéridin-4-yl)-1H-indol-2-yl)-3-méthylpyrazolo[1,5-a]pyridin-6-yl)méthanone ;
((R)-3-aminopipéridin-1-yl)(2-(1-(cyclopropylméthyl)-7-(1-((1s,4s)-4-hydroxycyclohexane-1-carbonyl)pipéridin-4-yl)-1H-indol-2-yl)-3-méthylpyrazolo[1,5-a]pyridin-6-yl)méthanone ;
((3R,5R)-3-amino-5-fluoropipéridin-1-yl)(2-(1-(cyclopropylméthyl)-7-(1-((1r,4r)-4-hydroxycyclohexane-1-carbonyl)pipéridin-4-yl)-1H-indol-2-yl)-3-méthylpyrazolo[1,5-a]pyridin-6-yl)méthanone ;
1-(4-(2-(6-((3R,5R)-3-amino-5-fluoropipéridine-1-carbonyl)-3-méthylpyrazolo[1,5-a]pyridin-2-yl)-1-(cyclopropylméthyl)-1H-indol-7-yl)pipéridin-1-yl)éthan-1-one ;
1-(4-(2-(6-((7R)-7-amino-2-azabicyclo[2.2.1]heptane-2-carbonyl)-3-méthylpyrazolo[1,5-a]pyridin-2-yl)-1-(cyclopropylméthyl)-1H-indol-7-yl)pipéridin-1-yl)-2-méthoxyéthan-I-one ;
(R)-(3-aminopipéridin-1-yl)(2-(1-(cyclopropylméthyl)-7-(1-(3-hydroxy-3-(trifluorométhyl)cyclobutane-1-carbonyl)pipéridin-4-yl)-1H-indol-2-yl)-3-méthylpyrazolo[1,5-a]pyridin-6-yl)méthanone ;
((R)-3-aminopipéridin-1-yl)(2-(1-(cyclopropylméthyl)-7-(1-(tétrahydro-2H-pyran-2-carbonyl)pipéridin-4-yl)-1H-indol-2-yl)-3-méthylpyrazolo[1,5-a]pyridin-6-yl)méthanone ;
((R)-3-aminopipéridin-1-yl)(2-(1-(cyclopropylméthyl)-7-(1-(tétrahydrofuran-2-carbonyl)pipéridin-4-yl)-1H-indol-2-yl)-3-méthylpyrazolo[1,5-a]pyridin-6-yl)méthanone ;
(4-(2-(6-((7R)-7-amino-2-azabicyclo[2.2.1]heptane-2-carbonyl)-3-méthylpyrazolo[1,5-a]pyridin-2-yl)-1-(cyclopropylméthyl)-1H-indol-7-yl)pipéridin-1-yl)(3-hydroxycyclobutyl)méthanone ;
Méthyle 4-(2-(6-((7R)-7-amino-2-azabicyclo[2.2.1]heptane-2-carbonyl)-3-méthylpyrazolo[1,5-a]pyridin-2-yl)-1-(cyclopropylméthyl)-1H-indol-7-yl)pipéridine-1-carboxylate ;
(2R)-1-(4-(2-(6-((7R)-7-Amino-2-azabicyclo[2.2.1]heptane-2-carbonyl)-3-méthylpyrazolo[1,5-a]pyridin-2-yl)-1-(cyclopropylméthyl)-1H-indol-7-yl)pipéridin-1-yl)-2-hydroxypropan-1-one ;
(4-(2-(6-((7R)-7-Amino-2-azabicyclo[2.2.1]heptane-2-carbonyl)-3-méthylpyrazolo[1,5-a]pyridin-2-yl)-1-(cyclopropylméthyl)-1H-indol-7-yl)pipéridin-1-yl)(thiophén-2-yl)méthanone ;
1-(4-(2-(6-((3R,5R)-3-Amino-5-fluoropipéridine-1-carbonyl)-3-méthylpyrazolo[1,5-a]pyridin-2-yl)-1-(cyclopropylméthyl)-1H-indol-7-yl)pipéridin-1-yl)éthan-1-one ;
1-(4-(2-(6-((7R)-7-Amino-2-azabicyclo[2.2.1]heptane-2-carbonyl)-3-méthylpyrazolo[1,5-a]pyridin-2-yl)-1-(cyclopropylméthyl)-1H-indol-7-yl)pipéridin-1-yl)éthan-1-one ;
1-(4-(2-(6-((3R,5R)-3-Amino-5-fluoropipéridine-1-carbonyl)-3-méthylpyrazolo[1,5-a]pyridin-2-yl)-1-(cyclopropylméthyl)-5-fluoro-1H-indol-7-yl)pipéridin-1-yl)-3-méthylbutan-1-one ;
((3R,5R)-3-Amino-5-fluoropipéridin-1-yl)(2-(1-(cyclopropylméthyl)-5-fluoro-7-(1-(3-hydroxycyclobutane-1-carbonyl)pipéridin-4-yl)-1H-indol-2-yl)-3-méthylpyrazolo[1,5-a]pyridin-6-yl)méthanone ;
(R)-1-(4-(2-(6-((R)-3-Aminopipéridine-1-carbonyl)-3-méthylpyrazolo[1,5-a]pyridin-2-yl)-1-(cyclopropylméthyl)-5-fluoro-1H-indol-7-yl)pipéridin-1-yl)-2-méthoxypropan-1-one ;
((R)-3-Aminopipéridin-1-yl)(2-(1-(cyclopropylméthyl)-5-fluoro-7-(1-(tétrahydro-2H-pyran-2-carbonyl)pipéridin-4-yl)-1H-indol-2-yl)-3-méthylpyrazolo[1,5-a]pyridin-6-yl)méthanone ;
1-(4-(2-(6-((3R,5R)-3-Amino-5-fluoropipéridine-1-carbonyl)-4-méthoxy-3-méthylpyrazolo[1,5-a]pyridin-2-yl)-1-(cyclopropylméthyl)-1H-pyrrolo[2,3-c]pyridin-7-yl)pipéridin-1-yl)-2-méthoxyéthan-1-one ;
((3R,5R)-3-Amino-5-fluoropipéridin-1-yl)(2-(1-(cyclopropylméthyl)-7-(1-((1s,4s)-4-hydroxycyclohexane-1-carbonyl)azetidin-3-yl)-1H-indol-2-yl)-4-méthoxy-3-méthylpyrazolo[1,5-a]pyridin-6-yl)méthanone ;
1-(3-(2-(6-((3R,5R)-3-Amino-5-fluoropipéridine-1-carbonyl)-4-méthoxy-3-méthylpyrazolo[1,5-a]pyridin-2-yl)-1-(cyclopropylméthyl)-1H-indol-7-yl)azetidin-1-yl)-2-méthoxyéthan-1-one ;
((R)-3-Aminopipéridin-1-yl)(2-(1-(cyclopropylméthyl)-7-(1-((1r,4r)-4-hydroxycyclohexane-1-carbonyl)azetidin-3-yl)-1H-indol-2-yl)-3-méthylpyrazolo[1,5-a]pyridin-6-yl)méthanone ;
1-(3-(2-(6-((7R)-7-Amino-2-azabicyclo[2.2.1]heptane-2-carbonyl)-3-méthylpyrazolo[1,5-a]pyridin-2-yl)-1-(cyclopropylméthyl)-1H-indol-7-yl)azetidin-1-yl)éthan-1-one ;
4-(((2-(6-((R)-3-Aminopipéridine-1-carbonyl)-4-méthoxy-3-méthylpyrazolo[1,5-a]pyridin-2-yl)-1-(cyclopropylméthyl)-1H-indol-7-yl)oxy)méthyl)pyrrolidin-2-one ;
(R)-(2-(7-(2-(1H-1,2,4-Triazol-1-yl)éthoxy)-1-(cyclopropylméthyl)-IH-indol-2-yl)-4-méthoxy-3-méthylpyrazolo[1,5-a]pyridin-6-yl)(3-aminopipéridin-1-yl)méthanone ;
(2-(7-(2-(1H-1,2,4-Triazol-1-yl)éthoxy)-1-(cyclopropylméthyl)-1H-indol-2-yl)-4-méthoxy-3-méthylpyrazolo[1,5-a]pyridin-6-yl)((3R,5R)-3-amino-5-fluoropipéridin-1-yl)méthanone ;
(2-(7-(2-(4-Amino-1H-pyrazol-1-yl)éthoxy)-1-(cyclopropylméthyl)-1H-indol-2-yl)-4-méthoxy-3-méthylpyrazolo[1,5-a]pyridin-6-yl)((3R,5R)-3-amino-5-fluoropipéridin-1-yl)méthanone ;
4-(((2-(6-((R)-3-Aminopipéridine-1-carbonyl)-4-méthoxy-3-méthylpyrazolo[1,5-a]pyridin-2-yl)-1-(cyclopropylméthyl)-1H-indol-7-yl)oxy)méthyl)pyrrolidin-2-one ;
4-(((2-(6-((3R,5R)-3-Amino-5-fluoropipéridine-1-carbonyl)-4-méthoxy-3-méthylpyrazolo[1,5-a]pyridin-2-yl)-1-(cyclopropylméthyl)-1H-indol-7-yl)oxy)m éthyl)pyrrolidin-2-one ;
4-(((2-(6-((R)-3-Aminopipéridine-1-carbonyl)-4-fluoro-3-méthylpyrazolo[1,5-a]pyridin-2-yl)-1-(cyclopropylméthyl)-1H-indol-7-yl)oxy)méthyl)pyrrolidin-2-one ;
4-(((2-(6-((3R,5R)-3-Amino-5-fluoropipéridine-1-carbonyl)-4-fluoro-3-méthylpyrazolo[1,5-a]pyridin-2-yl)-1-(cyclopropylméthyl)-1H-indol-7-yl)oxy)m éthyl)pyrrolidin-2-one ;
(S)-5-(((2-(6-((R)-3-Aminopipéridine-1-carbonyl)-4-fluoro-3-méthylpyrazolo[1,5-a]pyridin-2-yl)-1-(cyclopropylméthyl)-1H-indol-7-yl)oxy)méthyl)pyrrolidin-2-one ;
4-(((2-(6-((R)-3-Aminopipéridine-1-carbonyl)-4-fluoro-3-méthylpyrazolo[1,5-a]pyridin-2-yl)-1-(cyclopropylméthyl)-1H-indol-7-yl)oxy)méthyl)pyrrolidin-2-one ;
(2-(7-(2-(1H-Imidazol-1-yl)éthoxy)-1-(cyclopropylméthyl)-1H-indol-2-yl)-3-méthylpyrazolo[1,5-a]pyridin-6-yl)((3R,5R)-3-amino-5-fluoropipéridin-1-yl)méthanone ;
(2-(7-(2-(1H-1,2,4-Triazol-1-yl)éthoxy)-1-(cyclopropylméthyl)-1H-indol-2-yl)-3-méthylpyrazolo[1,5-a]pyridin-6-yl)((3R,5R)-3-amino-5-fluoropipéridin-1-yl)méthanone ;
(S)-5-(((2-(6-((R)-3-Aminopipéridine-1-carbonyl)-3-méthylpyrazolo[1,5-a]pyridin-2-yl)-1-(cyclopropylméthyl)-1H-indol-7-yl)oxy)méthyl)pyrrolidin-2-one ;
4-(((2-(6-((3R,5R)-3-Amino-5-fluoropipéridine-1-carbonyl)-3-méthylpyrazolo[1,5-a]pyridin-2-yl)-1-(cyclopropylméthyl)-1H-indol-7-yl)oxy)m éthyl)pyrrolidin-2-one ;
4-(((2-(6-((R)-3-Aminopipéridine-1-carbonyl)-4-méthoxy-3-méthylpyrazolo[1,5-a]pyridin-2-yl)-1-(cyclopropylméthyl)-1H-pyrrolo[2,3-c]pyridin-7-yl)oxy)méthyl)pyrrolidin-2-one ;
(2-(7-(2-(1H-1,2,4-Triazol-1-yl)éthoxy)-1-(cyclopropylméthyl)-1H-pyrrolo[2,3-c]pyridin-2-yl)-4-méthoxy-3-méthylpyrazolo[1,5-a]pyridin-6-yl)((3R,5R)-3-amino-5-fluoropipéridin-1-yl)méthanone ;
(2-(7-(2-(1H-Imidazol-1-yl)éthoxy)-1-(cyclopropylméthyl)-1H-pyrrolo[2,3-c]pyridin-2-yl)-4-méthoxy-3-méthylpyrazolo[1,5-a]pyridin-6-yl)((3R,5R)-3-amino-5-fluoropipéridin-1-yl)méthanone ;
(R)-(2-(7-(2-(1H-Imidazol-1-yl)éthoxy)-1-(cyclopropylméthyl)-1H-pyrrolo[2,3-c]pyridin-2-yl)-4-méthoxy-3-méthylpyrazolo[1,5-a]pyridin-6-yl)(3-aminopipéridin-1-yl)méthanone ;
(R)-(2-(7-(2-(4H-1,2,4-Triazol-4-yl)éthoxy)-1-(cyclopropylméthyl)-1H-pyrrolo[2,3-c]pyridin-2-yl)-4-méthoxy-3-méthylpyrazolo[1,5-a]pyridin-6-yl)(3-aminopipéridin-1-yl)méthanone ;
(2-(7-(2-(4H-1,2,4-Triazol-4-yl)éthoxy)-1-(cyclopropylméthyl)-1H-pyrrolo[2,3-c]pyridin-2-yl)-4-méthoxy-3-méthylpyrazolo[1,5-a]pyridin-6-yl)((3R,5R)-3-amino-5-fluoropipéridin-1-yl)méthanone ;
4-(((2-(6-((3R,5R)-3-Amino-5-fluoropipéridine-1-carbonyl)-4-méthoxy-3-méthylpyrazolo[1,5-a]pyridin-2-yl)-1-(cyclopropylméthyl)-1H-pyrrolo[2,3-c]pyridin-7-yl)oxy)m éthyl)pyrrolidin-2-one ;
4-(((2-(6-((3R,5R)-3-Amino-5-fluoropipéridine-1-carbonyl)-4-méthoxy-3-méthylpyrazolo[1,5-a]pyridin-2-yl)-1-(cyclopropylméthyl)-1H-pyrrolo[2,3-c]pyridin-7-yl)oxy)m éthyl)pyrrolidin-2-one ;
(5R)-5-(((2-(6-((7R)-7-Amino-2-azabicyclo[2.2.1]heptane-2-carbonyl)-4-méthoxy-3-méthylpyrazolo[1,5-a]pyridin-2-yl)-1-(cyclopropylméthyl)-1H-pyrrolo[2,3-c]pyridin-7-yl)oxy)m éthyl)pyrrolidin-2-one ;
4-(((2-(6-((7R)-7-Amino-2-azabicyclo[2.2.1]heptane-2-carbonyl)-4-méthoxy-3-méthylpyrazolo[1,5-a]pyridin-2-yl)-1-(cyclopropylméthyl)-1H-pyrrolo[2,3-c]pyridin-7-yl)oxy)m éthyl)pyrrolidin-2-one ;
(5R)-5-(((2-(6-((75)-7-Amino-2-azabicyclo[2.2.1]heptane-2-carbonyl)-4-méthoxy-3-méthylpyrazolo[1,5-a]pyridin-2-yl)-1-(cyclopropylméthyl)-1H-pyrrolo[2,3-c]pyridin-7-yl)oxy)m éthyl)pyrrolidin-2-one ;
4-(((2-(6-((7R)-7-Amino-2-azabicyclo[2.2.1]heptane-2-carbonyl)-4-méthoxy-3-méthylpyrazolo[1,5-a]pyridin-2-yl)-1-(cyclopropylméthyl)-1H-pyrrolo[2,3-c]pyridin-7-yl)oxy)m éthyl)pyrrolidin-2-one ;
(R)-(2-(7-(2-(4H-1,2,4-Triazol-4-yl)éthoxy)-1-(cyclopropylméthyl)-1H-pyrrolo[2,3-c]pyridin-2-yl)-3-méthylpyrazolo[1,5-a]pyridin-6-yl)(3-aminopipéridin-1-yl)méthanone ;
(2-(7-(2-(1H-Imidazol-1-yl)éthoxy)-1-(cyclopropylméthyl)-1H-pyrrolo[2,3-c]pyridin-2-yl)-3-méthylpyrazolo[1,5-a]pyridin-6-yl)((3R,5R)-3-amino-5-fluoropipéridin-1-yl)méthanone ;
(R)-(2-(7-(2-(1H-Imidazol-1-yl)éthoxy)-1-(cyclopropylméthyl)-1H-pyrrolo[2,3-c]pyridin-2-yl)-3-méthylpyrazolo[1,5-a]pyridin-6-yl)(3-aminopipéridin-1-yl)méthanone ;
4-(((2-(6-((R)-3-Aminopipéridine-1-carbonyl)-3-méthylpyrazolo[1,5-a]pyridin-2-yl)-1-(cyclopropylméthyl)-1H-pyrrolo[2,3-c]pyridin-7-yl)oxy)méthyl)pyrrolidin-2-one ;
4-(((2-(6-((R)-3-Aminopipéridine-1-carbonyl)-3-méthylpyrazolo[1,5-a]pyridin-2-yl)-1-(cyclopropylméthyl)-1H-pyrrolo[2,3-c]pyridin-7-yl)oxy)méthyl)pyrrolidin-2-one ;
ou sel pharmaceutiquement acceptable de celui-ci.

13. Composition pharmaceutique comprenant un composé selon l'une quelconque des revendications 1-12, ou un stéréoisomère, un énantiomère, un diastéréoisomère, un tautomère ou un sel pharmaceutiquement acceptable de celui-ci, et un ou plusieurs supports pharmaceutiquement acceptables.

14. Composé selon l'une quelconque des revendications 1-12, ou un stéréoisomère, un énantiomère, un diastéréoisomère, un tautomère ou un sel pharmaceutiquement acceptable de celui-ci, ou une composition selon la revendication 13, pour une utilisation en thérapie.

15. Composé selon l'une quelconque des revendications 1 à 12, ou un stéréoisomère, un énantiomère, un diastéréoisomère, un tautomère ou un sel pharmaceutiquement acceptable de celui-ci, ou une composition selon la revendication 13, pour être utilisé dans le traitement d'une maladie ou d'un trouble associé à l'activité de l'enzyme PAD4 choisi parmi la polyarthrite rhumatoïde, la maladie d'Alzheimer, la sclérose en plaques, le lupus, la maladie de Parkinson et le cancer.
